# EUROPEAN PATENT SPECIFICATION

(11) **EP 0 850 227 B1**
(45) Date of publication and mention of the grant of the patent: **09.01.2008**
(21) Application number: 96927192.3
(22) Date of filing: 19.08.1996
(51) Int. Cl.: C07D 237/14, C07D 413/04, C07D 405/04, C07D 403/04, C07D 265/36, C07C 251/76, A01N 43/58

(54) **PYRIDAZIN-3-ONE DERIVATIVES, THEIR USE, AND INTERMEDIATES FOR THEIR PRODUCTION**
PYRIDAZIN-3-ON DERIVATE, DEREN VERWENDUNG UND ZWISCHENPRODUKTE FÜR DEREN HERSTELLUNG
DERIVES DE PYRIDAZIN-3-ONE, LEUR UTILISATION ET INTERMEDIAIRES DESTINES A LEUR PRODUCTION

(30) Priority: 21.08.1995 JP 23609895; 21.02.1996 JP 6023296; 01.04.1996 JP 10461896
(43) Date of publication of application: 01.07.1998
(73) Proprietor: Sumitomo Chemical Company, Limited, Tokyo 104-8260 (JP)
(72) Inventor: KATAYAMA, Tadashi, Hyogo 665 (JP); KAWAMURA, Shinichi, Osaka 540 (JP); SANEMITSU, Yuzuru, Hyogo 655 (JP); MINE, Yoko, Tokyo 188 (JP)
(74) Representative: Vossius & Partner
(86) International application number: PCT/JP1996/002311
(87) International publication number: WO 1997/007104

(56) References cited:
- EP-A- 0 029 123

## Description

The present invention relates to pyridazin-3-one derivatives, their use as herbicides, and intermediates for their production.

EP-A-0029123 discloses a process for the preparation of substituted anilines and novel substituted anilines. In particular, it teaches that certain substituted anilines obtained by this process are useful as important starting materials for the preparation of novel substituted pyridazin-3-one derivatives having herbicidal activity in postemergence application. These pyridazin-3-one derivatives are, however, different from those of the present invention in that they have a substituted phenoxyphenyl group as an essential structural element at position 2 of the pyridazinone ring and further have quite distinct substituents on the pyridazinone ring.

The present inventors have intensively studied to find a compound having excellent herbicidal activity. As a result, they have found that pyridazin-3-one derivatives represented by formula [1] as depicted below have excellent herbicidal activity, thereby completing the present invention.

Thus the present invention provides a compound of the formula: 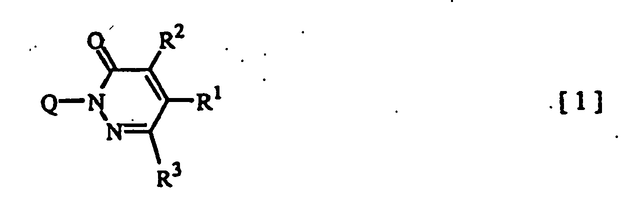 wherein R¹ is C₁-C₃ haloalkyl; R² and R³ are the same or different and are hydrogen, C₁-C₃ alkyl, C₁-C₃ haloalkyl, or C₁-C₃ alkoxy C₁-C₃ alkyl; and Q is [Q-1], [Q-2], [Q-3], or [Q-4] of the formula: 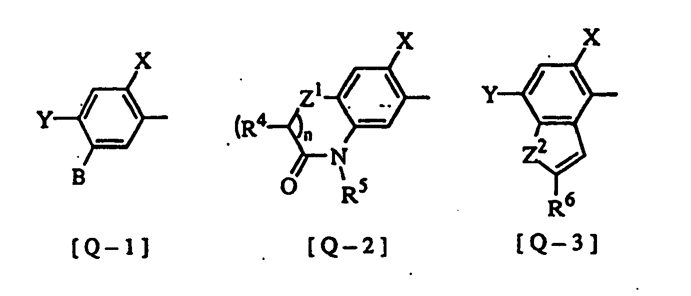 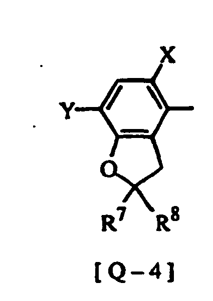 wherein X is hydrogen or halogen;
Y is halogen, nitro, cyano, or trifluoromethyl;
Z¹ is oxygen, sulfur, or NH;
Z² is oxygen or sulfur;
n is 0 or 1;
B is hydrogen, halogen, nitro, cyano, chlorosulfonyl, OR¹⁰, SR¹⁰, SO₂-OR¹⁰, N(R¹¹)R¹², SO₂N(R¹¹)R¹², NR¹¹(COR¹³), NR¹¹(SO₂R¹⁴), N(SO₂R¹⁴)-(SO₂R¹⁵), N(SO₂R¹⁴)(COR¹³), NHCOOR¹³, COOR¹⁰, CON(R¹¹)R¹², CSN(R¹¹)R¹², COR¹⁶, CR¹⁷=CR¹⁸COR¹⁶, CR¹⁷=CR¹⁸COOR¹³, CR¹⁷=CR¹⁸CON(R¹¹)R¹², CH₂CH-WCOOR¹³, CH₂CHWCON(R¹¹)R¹², CR¹⁷=NOR³³, CR¹⁷=NN(R¹¹)R¹², CR¹⁷(Z²-R³⁴)₂, OCO₂R¹⁹, or OCOR¹⁹;
R⁴ is hydrogen or C₁-C₃ alkyl;
R⁵ is hydrogen, C₁-C₆ alkyl, C₁-C₆ haloalkyl, C₃-C₈ cycloalkylalkyl, C₃-C₆ alkenyl, C₃-C₆ haloalkenyl, C₃-C₆ alkynyl, C₃-C₆ haloalkynyl, cyano C₁-C₆ alkyl, C₂-C₈ alkoxyalkyl, C₃-C₈ alkoxyalkoxyalkyl, carboxy C₁-C₆ alkyl, (C₁-C₆ alkoxy)-carbonyl C₁-C₆ alkyl, {(C₁-C₄ alkoxy) C₁-C₄ alkoxy}carbonyl C₁-C₆ alkyl, (C₃-C₈ cycloalkoxy)carbonyl C₁-C₆ alkyl, CH₂CON(R¹¹)R¹², CH₂COON(R¹¹)R¹², CH(C₁-C₄ alkyl)CON(R¹¹)R¹², CH(C₁-C₄ alkyl)COON(R¹¹)R¹², C₂-C₈ alkylthioalkyl, or hydroxy C₁-C₆ alkyl;
R⁶ is C₁-C₆ alkyl, C₁-C₆ haloalkyl, formyl, cyano, carboxyl, hydroxy C₁-C₆ alkyl, C₁-C₆ alkoxy C₁-C₆ alkyl, C₁-C₆ alkoxy C₁-C₆ alkoxy C₁-C₆ alkyl, (C₁-C₆ alkyl)carbonyloxy C₁-C₆ alkyl, (C₁-C₆ haloalkyl)carbonyloxy C₁-C₆ alkyl, (C₁-C₆ alkoxy)carbonyl, or (C₁-C₆ alkyl)carbonyl;
R⁷ is hydrogen or C₁-C₆ alkyl; and
R⁸ is hydrogen, C₁-C₆ alkyl, C₁-C₆ haloalkyl, hydroxy C₁-C₆ alkyl, C₂-C₈ alkoxyalkyl, C₃-C₁₀ alkoxyalkoxyalkyl, (C₁-C₅ alkyl)carbonyloxy C₁-C₆ alkyl, (C₁-C₆ haloalkyl)carbonyloxy C₁-C₆ alkyl, carboxyl, carboxy C₁-C₆ alkyl, (C₁-C₈ alkoxy)-carbonyl, (C₁-C₆ haloalkoxy)carbonyl, (C₃-C₁₀ cycloalkoxy)carbonyl, (C₃-C₈ alkenyloxy)carbonyl, (C₃-C₈ alkynyloxy)carbonyl, aminocarbonyl, (C₁-C₆ alkyl)aminocarbonyl, di(C₁-C₆ alkyl)aminocarbonyl, (C₁-C₆ alkyl)aminocarbonyloxy C₁-C₆ alkyl, or di(C₁-C₆ alkyl)aminocarbonyloxy C₁-C₆ alkyl;
wherein R¹⁰ is hydrogen, C₁-C₆ alkyl, C₁-C₆ haloalkyl, C₃-C₈ cycloalkyl, benzyl, C₃-C₆ alkenyl, C₃-C₆ haloalkenyl, C₃-C₆ alkynyl, C₃-C₆ haloalkynyl, cyano C₁-C₆ alkyl, C₂-C₈ alkoxyalkyl, C₂-C₈ alkylthioalkyl, carboxy C₁-C₆ alkyl, (C₁-C₈ alkoxy)carbonyl C₁-C₆ alkyl, (C₁-C₆ haloalkoxy)carbonyl C₁-C₆ alkyl, {(C₁-C₄ alkoxy) C₁-C₄ alkoxy}carbonyl C₁-C₆ alkyl, (C₃-C₈ cycloalkoxy)carbonyl C₁-C₆ alkyl, (C₁-C₆ alkyl)carbonyl C₁-C₆ alkyl, (C₁-C₆ haloalkyl)carbonyl C₁-C₆ alkyl, {(C₁-C₄ alkoxy) C₁-C₄ alkyl}carbonyl C₁-C₆ alkyl, (C₃-C₈ cycloalkyl)carbonyl C₁-C₆ alkyl, CH₂CON-(R¹¹)R¹², CH₂COON(R¹¹)R¹², CH(C₁-C₄ alkyl)CON(R¹¹)R¹², CH(C₁-C₄ alkyl)-COON(R¹¹)R¹², {(C₁-C₆ alkoxy)carbonyl C₁-C₆ alkyl}oxycarbonyl C₁-C₆ alkyl, or hydroxy C₁-C₆ alkyl:
R¹¹ and R ¹² are independently hydrogen, C₁-C₆ alkyl, C₁-C₆ haloalkyl, C₃-C₆ alkenyl, C₃-C₆ alkynyl, cyano C₁-C₆ alkyl, C₂-C₈ alkoxyalkyl, C₂-C₈ alkylthioalkyl, carboxy C₁-C₆ alkyl, (C₁-C₆ alkoxy)carbonyl C₁-C₆ alkyl, (C₃-C₈ cycloalkoxy)-carbonyl C₁-C₆ alkyl, {(C₁-C₄ alkoxy) C₁-C₄ alkoxy)carbonyl C₁-C₆ alkyl, or R¹¹ and R¹² are combined together to form tetramethylene, pentamethylene, or ethyleneoxyethylene;
R¹³ is hydrogen. C₁-C₆ alkyl, C₁-C₆ haloalkyl, C₃-C₈ cycloalkyl, or C₃-C₆ alkenyl;
R¹⁴ and R¹⁵ are independently C₁-C₆ alkyl, C₁-C₆ haloalkyl, or phenyl optionally substituted with methyl or nitro;
R ¹⁶ is hydrogen, C₁-C₆ alkyl, C₁-C₆ haloalkyl, C₂-C₆ alkenyl, C₂-C₆ haloalkenyl, C₂-C₆ alkynyl, C₂-C₆ haloalkynyl, C₂-C₈ alkoxyalkyl, or hydroxy C₁-C₆ alkyl;
R¹⁷ and R¹⁸ are independently hydrogen or C₁-C₆alkyl;
R¹⁹ is C₁-C₆ alkyl;
R³³ is hydrogen, C₁-C₆ alkyl, C₁-C₆ haloalkyl, C₃-C₈ cycloalkyl, C₃-C₆ alkenyl, C₃-C₆ haloalkenyl, C₃-C₆ alkynyl, C₃-C₆ haloalkynyl, cyano C₁-C₆ alkyl, or (C₁-C₆ alkoxy)carbonyl C₁-C₆ alkyl;
R³⁴ is C₁-C₆ alkyl, or two R³⁴'s are combined together to form (CH₂)₂ or (CH₂)₃: and
W is hydrogen, chlorine, or bromine,
(hereinafter referred to as the present compound(s)); and a herbicide containing it as an active ingredient.

The present invention also provides a compound of the formula: 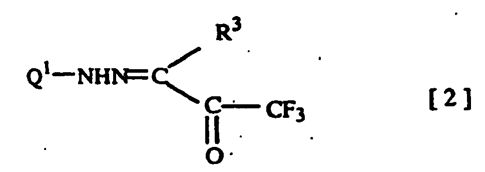 wherein R³ is as defined above, and Q¹ is [Q¹-1], [Q-2], [Q¹-3], or [Q-4] of the formula: 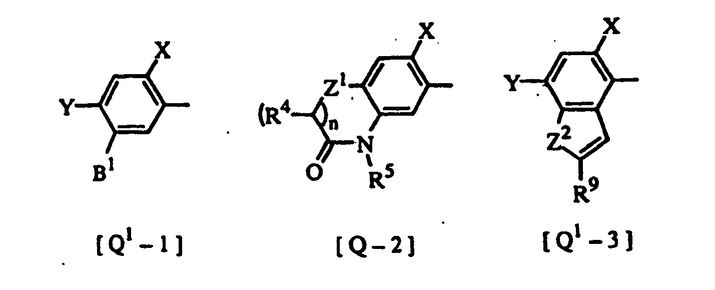 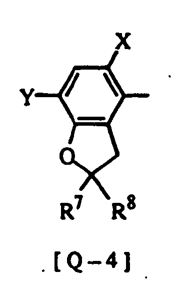 wherein X, Y, Z¹, Z², n, R⁴, R⁵, R⁷, and R⁸ are as defined above; B¹ is hydrogen, halogen, nitro, cyano, OR²⁷, SR²⁷, SO₂OR²⁷, NR¹¹(R¹²), SO₂NR¹¹(R¹²), NR¹¹-(COR¹³), NR¹¹(SO₂R¹⁴), N(SO₂R¹⁴)(SO₂R¹⁵), N(SO₂R¹⁴)(COR¹³), NHCOOR¹³, COOR²⁷, CONR¹¹(R¹²), CSNR¹¹(R¹²), CR¹⁷=COOR¹³, CR¹⁷=CR¹⁸CONR¹¹(R¹²), CH₂CHWCOOR¹³, CH₂CHWCONR¹¹(R¹²), CR¹⁷=NOR³³, CR¹⁷=NNR¹¹(R¹²), CR¹⁷(Z²R³⁴)₂, OCO₂R¹⁹, or OCOR¹⁹; R⁹ is C₁-C₆ alkyl, C₁-C₆ haloalkyl, cyano, carboxyl, hydroxy C₁-C₆ alkyl, C₁-C₆ alkoxy C₁-C₆ alkyl, C₁-C₆ alkoxy C₁-C₆ alkoxy C₁-C₆ alkyl, (C₁-C₆ alkyl)carbonyloxy C₁-C₆ alkyl, (C₁-C₆ haloalkyl)carbonyloxy C₁-C₆ alkyl, (C₁-C₆ alkoxy)carbonyl, or (C₁-C₆ alkyl)carbonyl; wherein R¹⁹ is as defined above; R²⁷ is hydrogen, C₁-C₆ alkyl, C₁-C₆ haloalkyl, C₃-C₈ cycloalkyl, benzyl, C₃-C₆ alkenyl, C₃-C₆ haloalkenyl, C₃-C₆ alkynyl, C₃-C₆ haloalkynyl, cyano C₁-C₆ alkyl, C₂-C₈ alkoxyalkyl, C₂-C₈ alkylthioalkyl, carboxy C₁-C₆ alkyl, (C₁-C₈ alkoxy)carbonyl C₁-C₆ alkyl, (C₁-C₆ haloalkoxy)carbonyl C₁-C₆ alkyl, ((C₁-C₄ alkoxy) C₁-C₄ alkoxy)carbonyl C₁-C₆ alkyl, (C₃-C₈ cycloalkoxy)caibonyl C₁-C₆ alkyl, CH₂CON(R¹¹)R¹², CH₂COON(R¹¹)R¹², CH(C₁-C₄ alkyl)CON(R¹¹)R¹². CH(C₁-C₄ alkyl)COON(R¹¹)R¹², {(C₁-C₆ alkoxy)carbonyl C₁-C₆ alkyl}oxycarbonyl C₁-C₆ alkyl, or hydroxy C₁-C₆ alkyl; and R¹¹, R¹², R¹³, R¹⁴, R¹⁵, R¹⁷, R¹⁸, R¹⁹, R³³, R³⁴, and Z² are as defined above.

In the above definition of the present compounds, the respective substituents are exemplified as follows:

Examples of the C₁-C₃ haloalkyl represented by R¹ include trifluoromethyl and chlorodifluoromethyl.

Examples of the C₁-C₃ alkyl represented by R² and R³ include methyl, ethyl, and isopropyl.

Examples of the C₁-C₃ haloalkyl represented by R² and R³ include trichloromethyl, trifluoromethyl, difluoromethyl, chlorodifluoromethyl, and pentafluoroethyl.

Examples of the C₁-C₃ alkoxy C₁-C₃ alkyl represented by R² and R³ include methoxymethyl.

Examples of the halogen represented by X, Y, and B include chlorine, fluorine, bromine, or iodine.

Examples of the C₁-C₆ alkyl represented by R¹⁰ include methyl, ethyl, isopropyl, propyl, isobutyl, butyl, t-butyl, amyl, isoamyl, and t-amyl.

Examples of the C₁-C₆ haloalkyl represented by R¹⁰ include 2-chloroethyl, 3-chloropropyl, and 2,2,2-trifluoroethyl.

Examples of the C₃-C₈ cycloalkyl represented by R¹⁰ include cyclopropyl, cyclobutyl, cyclopentyl, and cyclohexyl.

Examples of the C₃-C₆ alkenyl represented by R¹⁰ include allyl, 1-methyl-2-propenyl, 3-butenyl, 2-butenyl, 3-methyl-2-butenyl, and 2-methyl-3-butenyl.

Examples of the C₃-C₆ haloalkenyl represented by R¹⁰ include 2-chloro-2-propenyl and 3,3-dichloro-2-propenyl.

Examples of the C₃-C₆ alkynyl represented by R¹⁰ include propargyl, 1-methyl-2-propynyl, 2-butynyl, and 1,1-dimethyl-2-propynyl.

Examples of the C₃-C₆ haloalkynyl represented by R¹⁰ include 4-bromo-2-butynyl.

Examples of the cyano C₁-C₆ alkyl represented by R¹⁰ include cyanomethyl.

Examples of the C₂-C₈ alkoxyalkyl represented by R¹⁰ include methoxymethyl, methoxyethyl, ethoxymethyl, and ethoxyethyl.

Examples of the C₂-C₈ alkylthioalkyl represented by R¹⁰ include methylthiomethyl.

Examples of the carboxy C₁-C₆ alkyl represented by R¹⁰ include carboxymethyl, 1-carboxyethyl, and 2-carboxyethyl.

Examples of the (C₁-C₈ alkoxy)carbonyl C₁-C₆ alkyl represented by R¹⁰ include methoxycarbonylmethyl, ethoxycarbonylmethyl, propoxycarbonylmethyl, isopropoxycarbonylmethyl, butoxycarbonylmethyl, isobutoxycarbonylmethyl, t-butoxycarbonylmethyl, amyloxycarbonylmethyl, isoamyloxycarbonylmethyl, t-amyloxycarbonylmethyl, 1-methoxycarbonylethyl, 1-ethoxycarbonylethyl, 1-propoxycarbonylethyl, 1-isopropoxycarbonylethyl, 1-butoxycarbonylethyl, 1-isobutoxycarbonylethyl, 1-t-butoxycarbonylethyl, 1-amyloxycarbonylethyl, 1-isoamyloxycarbonylethyl, and 1-t-amyloxycarbonylethyl.

Examples of the (C₁-C₆ haloalkoxy)carbonyl C₁-C₆ alkyl represented by R¹⁰ include 2-chloroethoxycarbonylmethyl.

Examples of the {(C₁-C₄ alkoxy) C₁-C₄ alkoxy}carbonyl C₁-C₆ alkyl represented by R¹⁰ include methoxymethoxycarbonylmethyl and 1-methoxymethoxycarbonylethyl.

Examples of the (C₃-C₈ cycloalkoxy)carbonyl C₁-C₆ alkyl represented by R¹⁰ include cyclobutyloxycarbonylmethyl, cyclopentyloxycarbonylmethyl, cyclohexyloxycarbonylmethyl, 1-cyclobutyloxycarbonylethy, 1-cyclopentyloxycarbonylethyl, and 1-cyclohexyloxycarbonylethyl.

Examples of the (C₁-C₆ alkyl)carbonyl C₁-C₆ alkyl represented by R¹⁰ include methylcarbonylmethyl.

Examples of the (C₁-C₆ haloalkyl)carbonyl C₁-C₆ alkyl represented by R¹⁰ include chloromethylcarbonylmethyl.

Examples of the {(C₁-C₄ alkoxy) C₁-C₄ alkyl}carbonyl C₁-C₆ alkyl represented by R¹⁰ include 2-methoxyethylcarbonylmethyl.

Examples of the (C₃-C₈ cycloalkyl)carbonyl C₁-C₆ alkyl represented by R¹⁰ include cyclopentylcarbonylmethyl.

Examples of the {(C₁-C₆ alkoxy)carbonyl C₁-C₆ alkyl}oxycarbonyl C₁-C₆ alkyl represented by R¹⁰ include (ethoxycarbonyl)methoxycarbonylmethyl.

Examples of the C₁-C₆ alkyl represented by R¹¹ and R¹² include methyl, ethyl, propyl, butyl, isopropyl, and isobutyl.

Examples of the C₁-C₆ haloalkyl represented by R¹¹ and R¹² include chloroethyl and bromoethyl.

Examples of the C₃-C₆ alkenyl represented by R¹¹ and R¹² include allyl, 1-methyl-2-propenyl, and 3-butenyl.

Examples of the C₃-C₆ alkynyl represented by R¹¹ and R¹² include propargyl and 1-methyl-2-propynyl.

Examples of the cyano C₁-C₆ alkyl represented by R¹¹ and R¹² include cyanomethyl.

Examples of the C₂-C₈ alkoxyalkyl represented by R¹¹ and R¹² include methoxymethyl and ethoxyethyl.

Examples of the C₂-C₈ alkylthioalkyl represented by R¹¹ and R¹² include methylthiomethyl and methylthioethyl.

Examples of the carboxy C₁-C₆ alkyl represented by R¹¹ and R¹² include carboxymethyl and 1-carboxyethyl.

Examples of the (C₁-C₆ alkoxy)carbonyl C₁-C₆ alkyl represented by R¹¹ and R¹² include methoxycarbonylmethyl, ethoxycarbonylmethyl, propoxycarbonylmethyl, isopropoxycarbonylmethyl, butoxycarbonylmethyl, isobutoxycarbonylmethyl, t-butoxycarbonylmethyl, amyloxycarbonylmethyl, isoamyloxycarbonylmethyl, t-amyloxycarbonylmethyl, 1-methoxycarbonylethyl, 1-ethoxycarbonylethyl, 1-propoxycarbonylethyl, 1-isopropoxycarbonylethyl, 1-butoxycarbonylethyl, 1-isobutoxycarbonylethyl, 1-t-butoxycarbonylethyl, 1-amyloxycarbonylethyl, 1-isoamyloxycarbonylethyl, and 1-t-amyloxycarbonylethyl.

Examples of the (C₃-C₈ cycloalkoxy)carbonyl C₁-C₆ alkyl represented by R¹¹ and R¹² include cyclopentyloxycarbonylmethyl.

Examples of the {(C₁-C₄ alkoxy) C₁-C₄ alkoxy}carbonyl C₁-C₆ alkyl represented by R¹¹ and R ¹² include methoxymethoxycarbonylmethyl and 1-methoxymethoxycarbonylethyl.

Examples of the C₁-C₆ alkyl represented by R¹³ include methyl, ethyl, propyl, butyl, amyl, isopropyl, isobutyl, and isoamyl.

Examples of the C₁-C₆ haloalkyl represented by R¹³ include 2,2,2-trifluoroethyl.

Examples of the C₃-C₈ cycloalkyl represented by R¹³ include cyclopropyl, cyclobutyl, cyclopentyl, and cyclohexyl.

Examples of the C₃-C₆ alkenyl represented by R¹³ include allyl.

Examples of the C₁-C₆ alkyl represented by R¹⁴ and R¹⁵ include methyl, ethyl, propyl, butyl, and isopropyl.

Examples of the C₁-C₆ haloalkyl represented by R¹⁴ and R¹⁵ include trifluoromethyl, 2,2,2-trifluoroethyl, 2-chloroethyl, chloromethyl, and trichloromethyl.

Examples of the phenyl optionally substituted by methyl or nitro, which is represented by R¹⁴ and R¹⁵, include phenyl, p-methylphenyl, 2-nitrophenyl, 3-nitrophenyl, and 4-nitrophenyl.

Examples of the C₁-C₆ alkyl represented by R¹⁶ include methyl, ethyl, propyl, butyl, amyl, isopropyl, isobutyl, t-butyl, isoamyl, and t-amyl.

Examples of the C₁-C₆ haloalkyl represented by R¹⁶ include chloromethyl, dichloromethyl, bromomethyl, dibromomethyl, 1-chloroethyl, 1,1-dichloroethyl, 1-bromoethyl, and 1,1-dibromoethyl.

Examples of the C₂-C₆ alkenyl represented by R¹⁶ include vinyl, allyl, 1-propenyl, and 1-methyl-2-propenyl.

Examples of the C₂-C₆ haloalkenyl represented by R¹⁶ include 3,3-dichloro-2-propenyl and 3,3-dibromo-2-propenyl.

Examples of the C₂-C₆ alkynyl represented by R¹⁶ include ethynyl and 2-butynyl.

Examples of the C₂-C₆ haloalkynyl represented by R¹⁶ include 3-bromo-2-propynyl.

Examples of the C₂-C₈ alkoxyalkyl represented by R¹⁶ include methoxymethyl, ethoxymethyl, and isopropoxymethyl.

Examples of the hydroxy C₁-C₆ alkyl represented by R¹⁶ include hydroxymethyl.

Examples of the C₁-C₆ alkyl represented by R¹⁷ and R¹⁸ include methyl.

Examples of the C₁-C₆, alkyl represented by R¹⁹ include methyl and ethyl.

Examples of the C₁-C₆ alkyl represented by R³³ include methyl and ethyl.

Examples of the C₁-C₆ haloalkyl represented by R³³ include 2-chloroethyl.

Examples of the C₃-C₈ cycloalkyl represented by R³³ include cyclopentyl.

Examples of the C₃-C₆ alkenyl represented by R³³ include allyl.

Examples of the C₃-C₆ haloalkenyl represented by R³³ include 2-chloro-2-propenyl.

Examples of the C₃-C₆ alkynyl represented by R³³ include propargyl.

Examples of the C₃-C₆ haloalkynyl represented by R³³ include 4-chloro-2-butynyl.

Examples of the cyano C₁-C₆ alkyl represented by R³³ include 2-cyanoethyl and cyanomethyl.

Examples of the (C₁-C₆ alkoxy)carbonyl C₁-C₆ alkyl represented by R³³ include ethoxycarbonylmethyl.

Examples of the C₁-C₆ alkyl represented by R³⁴ include methyl and ethyl.

Examples of the C₁-C₃ alkyl represented by R⁴ include methyl.

Examples of the C₁-C₆ alkyl represented by R⁵ include methyl, ethyl, propyl, butyl, amyl, isopropyl, isobutyl, and isoamyl.

Examples of the C₁-C₆ haloalkyl represented by R⁵ include 2-chloroethyl, 2-bromoethyl, 3-chlorobutyl, 3-bromobutyl, difluoromethyl, and bromodifluoromethyl.

Examples of the C₃-C₈ cycloalkylalkyl represented by R⁵ include cyclopentylmethyl.

Examples of the C₃-C₆ alkenyl represented by R⁵ include allyl, 1-methyl-2-propenyl, 3-butenyl, 2-butenyl, 3-methyl-2-butenyl, and 2-methyl-3-butenyl.

Examples of the C₃-C₆ haloalkenyl represented by R⁵ include 2-chloro-2-propenyl and 3,3-dichloro-2-propenyl.

Examples of the C₃-C₆ alkynyl represented by R⁵ include propargyl, 1-methyl-2-propynyl, 2-butynyl, and 1,1-dimethyl-2-propynyl.

Examples of the C₃-C₆ haloalkynyl represented by R⁵ include 3-iodo-2-propynyl and 3-bromo-2-propynyl.

Examples of the cyano C₁-C₆ alkyl represented by R⁵ include cyanomethyl.

Examples of the C₂-C₈ alkoxyalkyl represented by R⁵ include methoxymethyl, ethoxymethyl, and 1-methoxyethyl.

Examples of the C₃-C₈ alkoxyalkoxyalkyl represented by R⁵ include methoxyethoxymethyl.

Examples of the carboxy C₁-C₆ alkyl represented by R⁵ include carboxymethyl, 1-carboxyethyl, and 2-carboxyethyl.

Examples of the (C₁-C₆ alkoxy)carbonyl C₁-C₆ alkyl represented by R⁵ include methoxycarbonylmethyl, ethoxycarbonylmethyl, propoxycarbonylmethyl, isopropoxycarbonylmethyl, butoxycarbonylmethyl, isobutoxycarbonylmethyl, t-butoxycarbonylmethyl, amyloxycarbonylmethyl, isoamyloxycarbonylmethyl, t-amyloxycarbonylmethyl, 1-methoxycarbonylethyl, 1-ethoxycarbonylethyl, 1-propoxycarbonylethyl, 1-isopropoxycarbonylethyl. 1-butoxycarbonylethyl, 1-isobutoxycarbonylethyl, 1-t-butoxycarbonylethyl. 1-amyloxycarbonylethyl, I-isoamyloxycarbonylethyl, and 1-t-amyloxycarbonylethyl.

Examples of the {(C₁-C₄ alkoxy) C₁-C₄ alkoxy}carbonyl C₁-C₆ alkyl represented by R⁵ include methoxymethoxycarbonylmethyl and 1-methoxymethoxycarbonylethyl.

Examples of the (C₃-C₈ cycloalkoxy)carbonyl C₁-C₆ alkyl represented by R⁵ include cyclobutyloxycarbonylmethyl, cyclopentyloxycarbonylmethyl, cyclohexyloxycarbonylmethyl, 1-cyclobutyloxycarbonylethyl, 1-cyclopentyloxycarbonylethyl, and 1-cyclohexyloxycarbonylethyl.

Examples of the C₂-C₈ alkylthioalkyl represented by R⁵ include methylthiomethyl.

Examples of the hydroxy C₁-C₆ alkyl represented by R⁵ include hydroxymethyl, hydroxyethyl, and hydroxypropyl.

Examples of the C₁-C₆ alkyl represented by R⁶ include methyl and ethyl.

Examples of the C₁-C₆ haloalkyl represented by R⁶ include bromomethyl, dibromomethyl, tribromomethyl, 1-bromoethyl, chloromethyl, dichloromethyl, and trichloromethyl.

Examples of the hydroxy C₁-C₆ alkyl represented by R⁶ include hydroxymethyl.

Examples of the C₁-C₆ alkoxy C₁-C₆ alkyl represented by R⁶ include methoxymethyl, ethoxymethyl, propoxymethyl, and isopropoxymethyl.

Examples of the C₁-C₆ alkoxy C₁-C₆ alkoxy C₁-C₆ alkyl represented by R⁶ include methoxymethoxymethyl, methoxyethoxymethyl, and ethoxymethoxymethyl.

Examples of the (C₁-C₆ alkyl)carbonyloxy C₁-C₆ alkyl represented by R⁶ include acetyloxymethyl, ethylcarbonyloxymethyl, and isopropylcarbonyloxymethyl.

Examples of the (C₁-C₆ haloalkyl)carbonyloxy C₁-C₆ alkyl represented by R⁶ include trifluoroacetyloxymethyl, chloroacetyloxymethyl, and trichloroacetyloxymethyl.

Examples of the (C₁-C₆ alkoxy)carbonyl represented by R⁶ include methoxycarbonyl, ethoxycarbonyl, propoxycarbonyl, butoxycarbonyl, amyloxycarbonyl, isopropoxycarbonyl, isobutoxycarbonyl, and isoamyloxycarbonyl.

Examples of the (C₁-C₆ alkyl)carbonyl represented by R⁶ include methylcarbonyl, ethylcarbonyl, and isopropylcarbonyl.

Examples of the C₁-C₆ alkyl represented by R⁷ include methyl.

Examples of the C₁-C₆ alkyl represented by R⁸ include methyl and ethyl.

Examples of the C₁-C₆ haloalkyl represented by R⁸ include chloromethyl, bromomethyl, and fluoromethyl.

Examples of the C₁-C₆ hydroxyalkyl represented by R⁸ include hydroxymethyl.

Examples of the C₂-C₈ alkoxyalkyl represented by R⁸ include methoxymethyl, ethoxymethyl, isopropoxymethyl, butoxymethyl, and isobutoxymethyl.

Examples of the C₃-C₁₀ alkoxyalkoxyalkyl represented by R⁸ include methoxymethoxymethyl, methoxyethoxymethyl, and ethoxymethoxymethyl.

Examples of the (C₁-C₅ alkyl)carbonyloxy C₁-C₆ alkyl represented by R⁸ include acetyloxymethyl, ethylcarbonyloxymethyl, and isopropylcarbonyloxymethyl.

Examples of the (C₁-C₆ haloalkyl)carbonyloxy C₁-C₆ alkyl represented by R⁸ include 2-chloroethylcarbonyloxymethyl.

Examples of the carboxy C₁-C₆ alkyl represented by R⁸ include carboxymethyl.

Examples of the (C₁-C₈)alkoxycarbonyl represented by R⁸ include methoxycarbonyl, ethoxycarbonyl, propoxycarbonyl, butoxycarbonyl, amyloxycarbonyl, isopropoxycarbonyl, isobutoxycarbonyl, and isoamyloxycarbonyl.

Examples of the (C₁-C₆ haloalkoxy)carbonyl represented by R⁸ include 2-chloroethoxycarbonyl; 2-bromoethoxycarbonyl, 3-chlorobutoxycarbonyl, 1-chloro-2-propoxycarbonyl, 1,3-dichloro-2-propoxycarbonyl, 2,2-dichloroethoxycarbonyl, 2,2,2-trifluoroethoxycarbonyl, 2,2.2-trichloroethoxycarbonyl, and 2,2,2-tribromoethoxycarbonyl.

Examples of the (C₃-C₁₀ cycloalkoxy)carbonyl represented by R⁸ include cyclobutyloxycarbonyl, cyclopentyloxycarbonyl, and cyclohexyloxycarbonyl.

Examples of the (C₃-C₈ alkenyloxy)carbonyl represented by R⁸ include allyloxycarbonyl and 3-butenyloxycarbonyl.

Examples of the (C₃-C₈ alkynyloxy)carbonyl represented by R⁸ include propargyloxycarbonyl, 3-butynyloxycarbonyl, and 1-methyl-2-propynyloxycarbonyl.

Examples of the (C₁-C₆ alkyl)aminocarbonyl represented by R⁸ include methylaminocarbonyl, ethylaminocarbonyl, and propylaminocarbonyl.

Examples of the di(C₁-C₆ alkyl)aminocarbonyl represented by R⁸ include dimethylaminocarbonyl, diethylaminocarbonyl, and diisopropylaminocarbonyl.

Examples of the (C₁-C₆ alkyl)aminocarbonyloxy C₁-C₆ alkyl represented by R⁸ include methylaminocarbonyloxymethyl, ethylaminocarbonyloxymethyl, and propylaminocarbonyloxymethyl.

Examples of the di(C₁-C₆ alkyl)aminocarbonyloxy C₁-C₆ alkyl represented by R⁸ include dimethylaminocarbonyloxyalkyl and diethylaminocarbonyloxyalkyl.

In the present compounds, preferred substituents from the viewpoint of their herbicidal activity are as follows:
R¹ is preferably methyl substituted with one or more fluorine atoms, such as trifluoromethyl or chlorodifluoromethyl, or ethyl substituted with one or more fluorine atoms, such as pentafluoroethyl, and more preferably trifluoromethyl;
R² is preferably C₁-C₃ alkyl such as methyl or ethyl, or hydrogen, and more preferably methyl or hydrogen; and
R³ is preferably C₁-C₃ alkyl such as methyl or ethyl, or hydrogen, and more preferably methyl or hydrogen.

Preferred examples of the present compounds from the viewpoint of their herbicidal activity are those which contain the above preferred substituents in combination.

When Q is [Q-1], more preferred compounds are those wherein X is hydrogen or fluorine and Y is chlorine. Among these compounds are more preferred ones wherein B is OR¹⁰, SR¹⁰, N(R¹¹)R¹², NR¹¹(SO₂R¹⁴), or COOR¹⁰. Among these compounds are more preferred ones wherein R¹⁰ is C₁-C₆ alkyl, C₃-C₆ alkynyl, (C₁-C₈ alkoxy)carbonyl C₁-C₆ alkyl, or (C₃-C₈ cycloalkoxy)carbonyl C₁-C₆ alkyl; R¹ is hydrogen; R¹² is (C₁-C₆ alkoxy)carbonyl C₁-C₆ alkyl; and R¹⁴ is C₁-C₆ alkyl or C₁-C₆ haloalkyl.

When Q is [Q-2], more preferred compounds are those wherein X is fluorine or hydrogen; Z¹ is oxygen; R⁴ is hydrogen; and n is 1. Among these compounds are more preferred ones wherein R⁵ is C₃-C₆ alkynyl.

Typical examples of the preferred compounds are as follows:
7-Fluoro-6-(5-trifluoromethyl-3-pyridazinon-2-yl)-4-propargyl-2H-1,4-benzoxazin-3-one;
7-Fluoro-6-(4-methyl-5-trifluoromethyl-3-pyndazinon-2-yl)-4-propargyl-2H-1,4-benzoxazin-3-one;
6-(5-Trifluoromethyl-3-pyridazinon-2-yl)-4-propargyl-2H-1,4-benzoxazin-3-one;
6-(4-Methyl-5-trifluoromethyl-3-pyridazinon-2-yl)-4-propargyl-2H-1,4-benzoxazin-3-one;
2-(4-Chloro-2-fluoro-5-isopropoxyphenyl)-4-methyl-5-trifluoromethylpyridazin-3-one;
2-(4-Chloro-2-fluoro-5-methoxyphenyl)-4-methyl-5-trifluoromethylpyridazin-3-one;
2-(4-Chloro-2-fluoro-5-ethoxyphenyl)-4-methyl-5-trifluoromethylpyridazin-3-one;
2-(4-Chloro-2-fluoro-5-propargyloxyphenyl)-4-methyl-5-trifluoromethylpyridazin-3-one:
Methyl 2-chloro-4-fluoro-5-(4-methyl-5-trifluoromethyl-3-pyridazinon-2-yl)-phenoxyacetate;
Ethyl 2-chloro-4-fluoro-5-(4-methyl-5-trifluoromethyl-3-pyridazinon-2-yl)-phenoxyacetate;
Propyl 2-chloro-4-fluoro-5-(4-methyl-5-trifluoromethyl-3-pyridazinon-2-yl)-phenoxyacetate;
lsopropyl 2-chloro-4-fluoro-5-(4-methyl-5-trifluoromethyl-3-pyridazinon-2-yl)phenoxyacetate;
Butyl 2-chloro-4-fluoro-5-(4-methyl-5-trifluoromethyl-3-pyridazinon-2-yl)-phenoxyacetate;
Pentyl 2-chloro-4-fluoro-5-(4-methyl-5-trifluoromethyl-3-pyridazinon-2-yl)-phenoxyacetate;
Cyclopentyl 2-chloro-4-fluoro-5-(4-methyl-5-trifluoromethyl-3-pyridazinon-2-yl)phenoxyacetate;
Ethyl 2-{2-chloro-4-fluoro-5-(4-methyl-5-trifluoromethyl-3-pyridazinon-2-yl)phenoxy}propionate;
Methyl 2-{2-chloro-4-fluoro-5-(4-methyl-5-trifluoromethyl-3-pyridazinon-2-yl)phenoxy}propionate;
Ethyl 2-chloro-4-fluoro-5-(4-methyl-5-trifluoromethyl-3-pyridazinon-2-yl)-phenylthioacetate;
Methyl 2-chloro-4-fluoro-5-(4-methyl-5-trifluoromethyl-3-pyridazinon-2-yl)-phenylthioacetate;
Ethyl 2-{2-chloro-4-fluoro-5-(4-methyl-5-trifluoromethyl-3-pyridazinon-2-yl)phenylthio}propionate;
Methyl 2- {2-chloro-4-fluoro-5-(4-methyl-5-trifluoromethyl-3-pyridazinon-2-yl)phenylthio}propionate;
Methyl 2-{2-chloro-4-fluoro-5-(5-trifluoromethyl-3-pyridazinon-2-yl)phenoxy} propionate;
Ethyl 2- (2-chloro-4-fluoro-5-(5-trifluoromethyl-3-pyridazinon-2-yl)phenoxy}propionate;
Ethyl 2-chloro-4-fluoro-5-(5-trifluoromethyl-3-pyridazinon-2-yl)phenylthioacetate;
Methyl 2-chloro-4-fluoro-5-(5-trifluoromethyl-3-pyridazinon-2-yl)phenylthioacetate;
Ethyl 2-{2-chloro-4-fluoro-5-(5-trifluoromethyl-3-pyridazinon-2-yl)phenylthio}propionate;
Methyl 2-{2-chloro-4-fluoro-5-(5-trifluoromethyl-3-pyridazinon-2-yl)phenylthio}propionate;
Isopropyl 2-{2-chloro-4-fluoro-5-(5-trifluoromethyl-3-pyridazinon-2-yl)-phenylthio }propionate;
Ethyl 2-{2-chloro-4-fluoro-5-(4-methyl-5-trifluoromethyl-3-pyridazinon-2-yl)phenylamino }propionate;
Ethyl 2-{2-chloro-4-fluoro-5-(5-trifluoromethyl-3-pyridazinon-2-yl)phenylamino}propionate;
N-{2-chloro-4-fluoro-5-(4-methyl-5-trifluoromethyl-3-pyridazinon-2-yl)-phenyl }methanesulfonamide;
N-{2-chloro-4-fluoro-5-(4-methyl-5-trifluoromethyl-3-pyridazinon-2-yl)-phenyl }chloromethanesulfonamide;
N-{2-chloro-5-(4-methyl-5-trifluoromethyl-3-pyridazinon-2-yl)phenyl}-methanesulfonamide;
Methyl 2-chloro-4-fluoro-5-(4-methyl-5-trifluoromethyl-3-pyridazinon-2-yl)-benzoate;
Ethyl 2-chloro-4-fluoro-5-(4-methyl-5-trifluoromethyl-3-pyridazinon-2-yl)-benzoate;
Ethyl 2-chloro-5-(4-methyl-5-trifluoromethyl-3-pyridazinon-2-yl)benzoate;
Isopropyl 2-chloro-5-(4-methyl-5-trifluoromethyl-3-pyridazinon-2-yl)benzoate; and
2-(4-Chloro-2-fluoro-5-propargyloxyphenyl)-6-methyl-5-trifluoromethylpyridazin-3-one.

Among these compounds, more preferred ones from the viewpoint of their herbicidal activity are as follows:
7-Fluoro-6-(5-trifluoromethyl-3-pyridazinon-2-yl)-4-proparygyl-2H-1,4-benzoxazin-3-one;
7-Fluoro-6-(4-methyl-5-trifluoromethyl-3-pyridazinon-2-yl)-4-propargyl-2H-1,4-benzoxazin-3-one;
6-(4-Methyl-5-trifluoromethyl-3-pyridazinon-2-yl)-4-propargyl-2H-1,4-benzoxazin-3-one;
Ethyl 2-(2-chloro-4-fluoro-5-(5-trifluoromethyl-3-pyrizazinon-2-yl)phenoxy)propionate; and
Ethyl 2-{2-chloro-4-fluoro-5-(5-trifluoromethyl-3-pyridazinon-2-yl)phenylthio}propionate.

In addition, more preferred ones from the viewpoint of their selectivity between crop plants and undesired weeds are as follows:
2-(4-Chloro-2-fluoro-5-propargyloxyphenyl)-4-methyl-5-trifluoromethylpyrizazin-3-one;
Ethyl 2-chloro-4-fluoro-5-(4-methyl-5-trifluoromethyl-3-pyridazinon-2-yl)-phenoxyacetate;
N-{2-chloro-4-fluoro-5-(4-methyl-5-trifluoromethyl-3-pyridazinon-2-yl)phenyl}methanesulfonamide; and
Ethyl 2-chloro-4-fluoro-5-(4-methyl-5-trifluoromethyl-3-pyridazinon-2-yl)-benzoate.

The present compounds can be produced, for example, according to the production processes described below.

### (Production Process 1)

This is the production process in which among the present compounds, a compound of the formula: 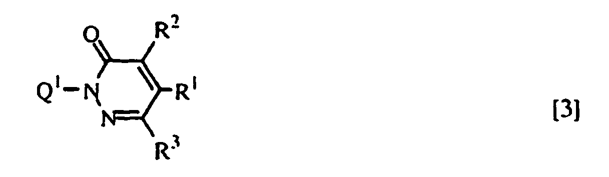 wherein R¹, R², R³, and Q¹ are as defined above, is produced by reacting a hydrazone derivative of the formula: 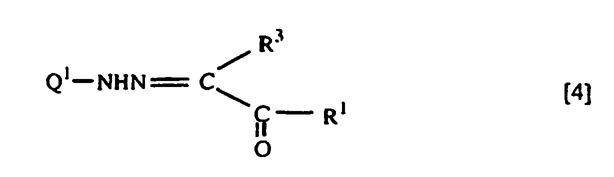 wherein R¹, R³, and Q¹ are as defined above, with a compound of the formula: 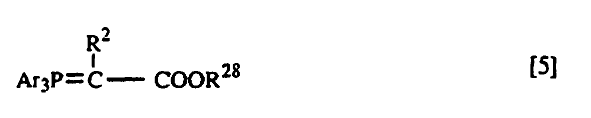 wherein R² is as defined above: R²⁸ is C₁-C₆ alkyl such as methyl or ethyl; and Ar is an optionally substituted phenyl such as phenyl.

The reaction is usually effected in a solvent. The reaction temperature is usually in the range of -20° to 150°C, preferably 0° to 100°C. The reaction time is usually in the range of a moment to 72 hours. The amounts of the reagents to be used in the reaction, although the proportion of I mole of compound [5] to 1 mole of compound [4] is ideal, can be freely changed depending upon the reaction conditions.

Examples of the solvent which can be used include aliphatic hydrocarbons such as hexane, heptane, ligroin, cyclohexane, and petroleum ether; aromatic hydrocarbons such as benzene, toluene, and xylene; ethers such as diethyl ether, diisopropyl ether, dioxane, tetrahydrofuran (THF), and ethylene glycol dimethyl ether; nitro compounds such as nitromethane and nitrobenzene; acid amides such as formamide, N,N-dimethylformamide, and acetamide; tertiary amines such as pyridine, triethylamine, diisopropylethylamine, N,N-dimethylaniline, N,N-diethylaniline, and N-methylmorpholine; sulfur compounds such as dimethylsulfoxide and sulforane; alcohols such as methanol, ethanol, ethylene glycol, and isopropanol; water; and mixtures thereof.

After completion of the reaction, the reaction solvent is distilled out from the reaction mixture and the residue is subjected to chromatography, or the reaction mixture is subjected to ordinary post-treatments such as extraction with an organic solvent and concentration, followed by, if necessary, subsequent purification by a technique such as chromatography or recrystallization. Thus the desired compound of the present invention can be isolated.

The above reaction is effected through a compound of the formula: 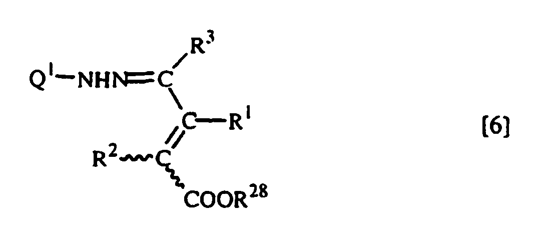 wherein R¹, R², R³, R²⁸, and Q¹ are as defined above.

This production process can also be conducted by isolating compound [6] and effecting intramolecular cyclization of compound [6]. The cyclization can usually be effected in a solvent. The reaction temperature is usually in the range of -20° to 150°C, preferably 50° to 150°C. The reaction time is usually in the range of a moment to 72 hours.

Examples of the solvent which can be used include aliphatic hydrocarbons such as hexane, heptane, ligroin, cyclohexane, and petroleum ether; aromatic hydrocarbons such as benzene, toluene, and xylene; halogenated hydrocarbons such as chloroform, carbon tetrachloride, dichloromethane, dichloroethane, chlorobenzene, and dichlorobenzene; ethers such as diethyl ether, diisopropyl ether, dioxane, tetrahydrofuran, and ethylene glycol dimethyl ether; ketones such as acetone, methyl ethyl ketone, methyl isobutyl ketone, isophorone, and cyclohexanone; esters such as ethyl formate, ethyl acetate, butyl acetate, and diethyl carbonate; nitro compounds such as nitromethane and nitrobenzene; nitriles such as acetonitrile and isobutyronitrile: acid amides such as formamide. N,N-dimethylformamide, and acetamide; tertiary amines such as pyridine, triethylamine, diisopropylethylamine, N,N-dimethylaniline, N,N-diethylaniline, and N-methylmorpholine: sulfur compounds such as dimethylsulfoxide and sulforane; fatty acids such as formic acid, acetic acid, and propionic acid; alcohols such as methanol, ethanol, ethylene glycol, and isopropanol; water; and mixtures thereof.

As the reaction catalyst, acids such as sulfuric acid or bases such as sodium methylate can be used.

### (Production Process 2)

This is the production process according to the following scheme: 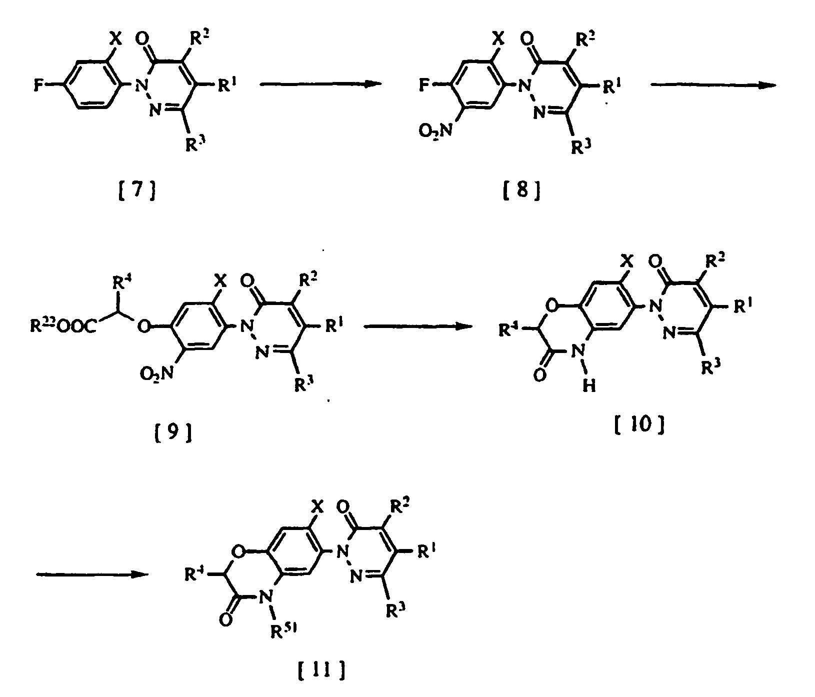 wherein R⁵¹ is a substituent other than hydrogen, which is included in the definition of R⁵; R¹, R², R³, R⁴, and X are as defined above; R²² is C₁-C₆ alkyl; and D is chlorine, bromine, iodine, methanesulfonyloxy, trifluoromethanesulfonyloxy, or p-toluenesulfonyloxy.

### Process for Producing Compound [8] from Compound [7]

Compound [8] can be produced by reacting compound [7] with a nitrating agent in a solvent.
Nitrating agent: nitric acid or the like
Amount of nitrating agent: 1 to 10 moles per mole of compound [7]
Solvent: sulfuric acid
Temperature: -10°C to room temperature
Time: a moment to 24 hours

### Process for Producing Compound [9] from Compound [8]

Compound [9] can be produced by reacting compound [8] with a compound of the formula: 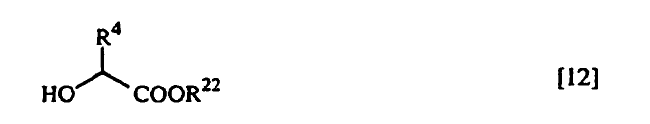 wherein R⁴ and R²² are as defined above, in the presence of potassium fluoride in a solvent.
Amount of compound [12]: 1 to 50 moles per mole of compound [8]
Amount of potassium fluoride: 1 to 50 moles per mole of compound [8]
Solvent: 1,4-dioxane or the like
Temperature: room temperature to refluxing temperature under heating
Time: a moment to 96 hours

### Process for Producing Compound [10] from Compound [9]

Compound [10] can be produced by reducing compound [9] with iron powder or the like in the presence of an acid in a solvent.
Amount of iron powder: 3 moles to an excess per mole of compound [9]
Acid: acetic acid or the like
Amount of acid: 1 to 10 moles
Solvent: water, ethyl acetate, or the like
Temperature: room temperature to refluxing temperature under heating
Time: a moment to 24 hours

### Process for Producing Compound [11] from Compound [10]

Compound [11] can be produced by reacting compound [10] with a compound of the formula:

R⁵¹-D [13]

wherein R⁵¹ and D are as defined above.

The reaction is usually effected in the presence of a base in a solvent. The reaction temperature is usually in the range of -20° to 150°C, preferably 0° to 50°C. The reaction time is usually in the range of a moment to 48 hours. The amounts of the reagents to be used in the reaction are usually 1 to 3 moles of compound [13] and usually 1 to 2 moles of the base, per mole of compound [10].

Examples of the base which can be used include inorganic bases such as sodium hydride, potassium hydride, sodium hydroxide, potassium hydroxide, potassium carbonate, and sodium carbonate: and organic bases such as triethylamine, diisopropylethylamine, pyridine, 4-dimethylaminopyridine, N,N-dimethylaniline, and N,N-diethylaniline.

Examples of the solvent which can be used include aliphatic hydrocarbons such as hexane, heptane, ligroin, cyclohexane, and petroleum ether; aromatic hydrocarbons such as benzene, toluene, and xylene; halogenated hydrocarbons such as chlorobenzene and dichlorobenzene; ethers such as diethyl ether, diisopropyl ether, dioxane, tetrahydrofuran, and ethylene glycol dimethyl ether; nitro compounds such as nitrobenzene; acid amides such as formamide, N,N-dimethylformamide, and acetamide; tertiary amines such as pyridine, triethylamine, diisopropylethylamine, N,N-dimethylamine, N,N-diethylaniline, and N-methylmorpholine; and mixtures thereof.

After completion of the reaction, the reaction mixture is poured into water, if necessary and subjected to ordinary post-treatments such as extraction with an organic solvent and concentration, followed by, if necessary, subsequent purification by a technique such as column chromatography or recrystallization. Thus the present compound [11] can be obtained.

The above compound [9] can also be produced according to the following scheme: 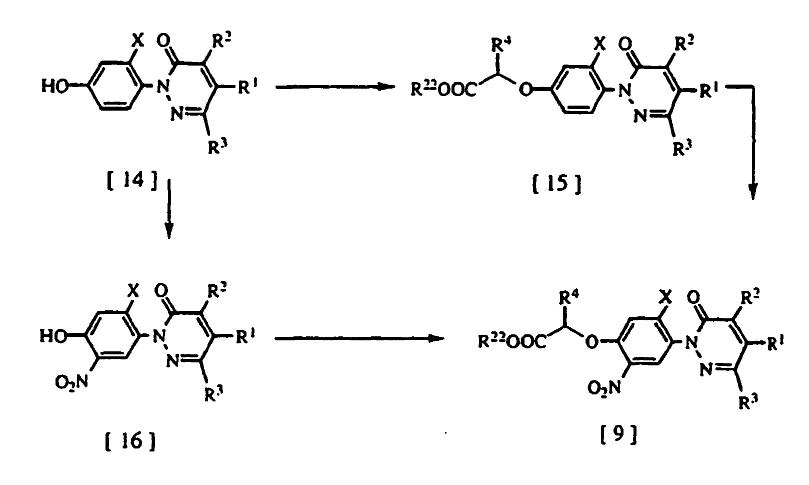 wherein R¹, R², R³, R⁴, R²², and X are as defined above.

### Process for Producing Compound [15] from Compound [14]

Compound [15] can be produced by reacting compound [14] with a compound of the formula: 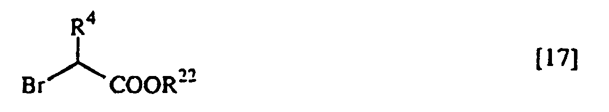 wherein R⁴ and R²² are as defined above, in the presence of a base in a solvent.
Amount of compound [17]: 1 to 2 moles per mole of compound [14]
Base: sodium hydride, sodium carbonate, or the like
Amount of base: 1 to 2 moles per mole of compound [14]
Solvent: 1,4-dioxane, N,N-dimethylformamide, or the like
Temperature: 0° to 100°C
Time: a moment to 24 hours

### Process for Producing Compound [9] from Compound [15]

Compound [9] can be produced by reacting compound [15] with a nitrating agent in a solvent.
Nitrating agent: nitric acid or the like
Amount of nitrating agent: I to 10 moles per mole of compound [15]
Solvent: sulfuric acid, acetic acid, or the like
Temperature: -10°C to room temperature
Time: a moment to 24 hours

### Process for Producing Compound [16] from Compound [14]

Compound [16] can be produced by reacting compound [14] with a nitrating agent in a solvent.
Nitrating agent: nitric acid or the like
Amount of nitrating agent: 1 to 10 moles per mole of compound [14]
Solvent: sulfuric acid, acetic acid, or the like
Temperature: -10°C to room temperature
Time: a moment to 24 hours

### Process for Producing Compound [9] from Compound [16]

Compound [9] can be produced by reacting compound [16] with compound[17] in the presence of a base in a solvent.
Amount of compound [17]: 1 to 2 moles per mole of compound [16]
Base: sodium hydride, potassium carbonate, or the like
Amount of base: 1 to 2 moles per mole of compound [16]
Solvent: 1,4-dioxane, N,N-dimethylformamide, or the like
Temperature: 0° to 100°C
Time: a moment to 24 hours

### (Production Process 3)

This is the production process according to the following scheme: 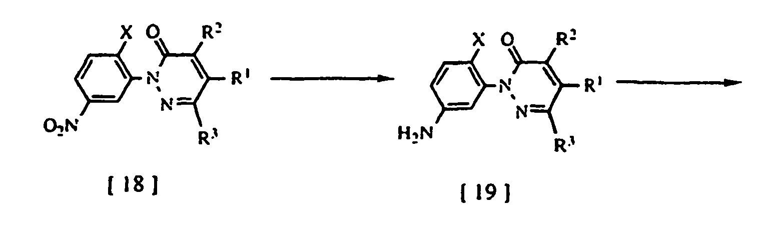 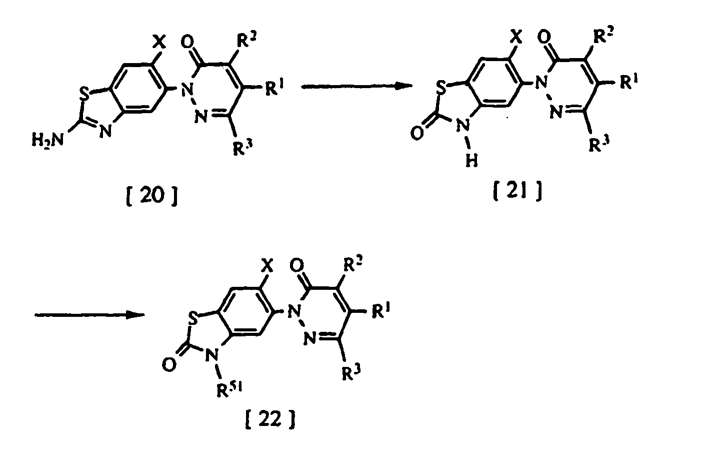 wherein X, R¹, R², R³, R⁵¹, and D are as defined above.

### Process for Producing Compound [19] from Compound [18]

Compound [19] can be produced by reducing compound [18] with iron powder or the like in the presence of an acid in a solvent.
Amount of iron powder: 3 moles to an excess per mole of compound [18]
Acid: acetic acid or the like
Amount of acid: 1 to 10 moles per mole of compound [18]
Solvent: water, ethyl acetate, or the like
Temperature: room temperature to refluxing temperature under heating
Time: a moment to 24 hours

### Process for Producing Compound [21] from Compound [20]

Compound [20] can be produced by reacting compound [19] with sodium thiocyanate, potassium thiocyanate, or the like in a solvent, and then reacting it with bromine or chlorine in a solvent.

Amount of sodium thiocyanate, potassium thiocyanate, or the like: 1 to 10 moles per mole of compound [19]
Amount of bromine or chlorine: I to 10 moles per mole of compound [19]
Solvent: aqueous hydrochloric acid, aqueous acetic acid, aqueous sulfuric acid, or the like
Temperature: 0° to 50°C
Time: a moment to 150 hours

### Process for Producing Compound [21] from Compound [20]

Compound [21] can be produced by 1) reacting compound [20] with sodium nitrite, potassium nitrite, or the like in a solvent, and then 2) heating it in an acidic solution.

### <Reaction 1)>

Amount of sodium nitrite, potassium nitrite, or the like: 1 to 2 moles per mole of compound [20]
Solvent: aqueous hydrochloric acid or aqueous sulfuric acid
Temperature: -10° to 10°C
Time: a moment to 5 hours

### <Reaction 2)>

Acidic solution: aqueous hydrochloric acid, aqueous sulfuric acid, or the like
Temperature: 70°C to refluxing temperature under heating
Time: a moment to 24 hours

### Process for Producing Compound [22] from Compound [21]

Compound [22] can be produced by reacting compound [21] with compound [13] in the presence of a base in a solvent.
Amount of compound [13]: 1 to 3 moles per mole of compound [21]
Base: sodium hydride, potassium carbonate, or the like
Amount of base: 1 to 2 moles per mole of compound [21]
Solvent: 1,4-dioxane, N,N-dimethylformamide, or the like
Temperature: 0° to 100°C
Time: a moment to 48 hours

### (Production Process 4)

This is the production process according to the following scheme: 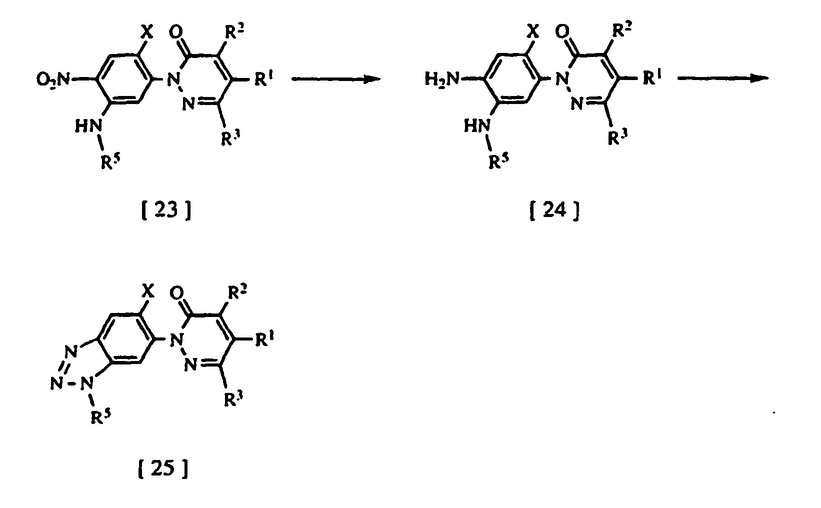 wherein X, R¹, R², R³, and R⁵ are as defined above.

### Process for Producing Compound [24] from Compound [23]

Compound [24] can be produced by reducing compound [23] with iron powder or the like in the presence of an acid in a solvent.
Amount of iron powder: 3 moles to an excess per mole of compound [23]
Acid: acetic acid or the like
Amount of acid: 1 to 10 moles per mole of compound [23]
Solvent: water, ethyl acetate, or the like
Temperature: room temperature to refluxing temperature under heating
Time: a moment to 24 hours

### Process for Producing Compound [25] from Compound [24]

Compound [25] can be produced by 1) reacting compound [24] with a nitrite salt in a solvent to form a diazonium salt, and then 2) raising the temperature to cause the cyclization of the diazonium salt in a solvent.

### <Reaction 1)>

Nitrite salt: sodium nitrite, potassium nitrite, or the like
Amount of nitrite salt: I to 2 moles per mole of compound [24]
Solvent: aqueous hydrochloric acid, aqueous sulfuric acid, or the like
Temperature: -10° to 10°C
Time: a moment to 5 hours

### <Reaction 2)>

Solvent: aqueous hydrochloric acid, aqueous sulfuric acid, or the like
Temperature: room temperature to 80°C
Time: a moment to 24 hours

### (Production Process 5)

This is the production process according to the following scheme: 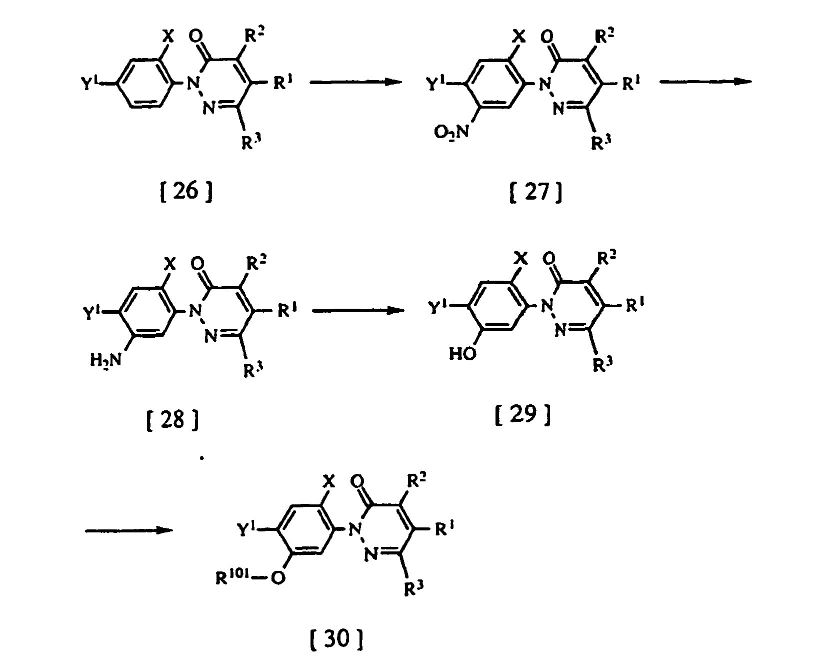 wherein Y¹ is a substituent other than nitro, which is included in the definition of Y; R¹⁰¹ is a substituent other than hydrogen, which is included in the definition of R¹⁰; and X, R¹, R², and R³ are as defined above.

### Process for Producing Compound [27] from compound [26]

Compound [27] can be produced by adding nitric acid to compound [26] in a solvent (see Organic Synthesis Collective, Vol. 1, p. 372).

The reaction temperature is usually in the range of 0° to 100°C. The reaction time is usually in the range of a moment to 24 hours. The amounts of the reagents to be used in the reaction, although the proportion of 1 mole of nitric acid to 1 mole of compound [26] is ideal, can be freely changed depending upon the reaction conditions.

Examples of the solvent which can be used include acidic solvents such as sulfuric acid.

### Process for Producing Compound [28] from compound [27]

Compound [28] can be produced by reducing compound [27] in a solvent (see Organic Synthesis Collective, Vol. 2. p. 471, and *ibid.,* Vol. 5, p. 829).

For example, the production can be achieved by adding compound [27], which is neat or dissolved in a solvent such as ethyl acetate, to a mixture of acetic acid, iron powder, and water. The reaction temperature is usually in the range of 0° to 100°C. The reaction time is usually in the range of a moment to 24 hours.

After completion of the reaction, the reaction mixture is filtered to collect the crystals, which may be precipitated by the addition of water, if necessary, or the reaction mixture is subjected to ordinary post-treatments such as extraction with an organic solvent and concentration, followed by, if necessary, subsequent purification by a technique such as column chromatography or recrystallization. Thus the desired product can be isolated.

### Process for Producing Compound [29]from compound [28]

Compound [29] can be produced by 1) reacting compound [28] with a nitrite salt in a solvent, and then 2) heating it in an acidic solvent.

### <Reaction 1)>

Nitrite salt: sodium nitrite, potassium nitrite, or the like
Amount of nitrite salt: 1 to 2 moles per mole of compound [28]
Solvent: aqueous hydrochloric acid, aqueous sulfuric acid, or the like
Temperature: -10° to 10°C
Time: a moment to 5 hours

### <Reaction 2)>

Acidic solvent: aqueous hydrochloric acid or aqueous sulfuric acid
Temperature: 70°C to refluxing temperature under heating
Time: a moment to 24 hours.

### Process for Producing Compound [30] from Compound [29]

Compound [30] can be produced by reacting compound [29] with a compound of the formula:

R¹⁰¹-D [31]

wherein R¹⁰¹ and D are as defined above, in the presence of a base in a solvent.

The reaction is usually effected in a solvent. The reaction temperature is usually in the range of -20° to 150°C, preferably 0° to 100°C. The reaction time is usually in the range of a moment to 72 hours. The amounts of the reagents to be used in the reaction, although the proportion of 1 mole of compound [31] and 1 mole of a base to 1 mole of compound [29] is ideal, can be freely changed depending upon the reaction conditions.

Examples of the base which can be used include organic bases and inorganic bases such as potassium carbonate, sodium hydroxide, and sodium hydride.

Examples of the solvent which can be used include aliphatic hydrocarbons such as hexane, heptane, ligroin, cyclohexane, and petroleum ether; aromatic hydrocarbons such as benzene, toluene, and xylene; halogenated hydrocarbons such as chloroform, carbon tetrachloride, dichloromethane, dichloroethane, chlorobenzene, and dichlorobenzene; ethers such as diethyl ether, diisopropyl ether, dioxane, tetrahydrofuran, and ethylene glycol dimethyl ether; ketones such as acetone, methyl ethyl ketone, methyl isobutyl ketone, isophorone, and cyclohexanone; esters such as ethyl formate, ethyl acetate, butyl acetate, and diethyl carbonate; nitro compounds such as nitromethane and nitrobenzene: nitriles such as acetonitrile and isobutyronitrile; acid amides such as formamide, N,N-dimethylformamide, and acetamide; tertiary amines such as pyridine, triethylamine, diisopropylethylamine, N,N-dimethylaniline. N,N-diethylaniline, and N-methylmorpholine; sulfur compounds such as dimethylsulfoxide and sulforane; and mixtures thereof.

After completion of the reaction, the reaction mixture is filtered to collect the crystals, which may be precipitated by the addition of water, if necessary, or the reaction mixture is subjected to ordinary post-treatments such as extraction with an organic solvent and concentration, followed by, if necessary, subsequent purification by a technique such as column chromatography or recrystallization. Thus the desired product can be isolated.

### (Production Process 6)

This is the production process according to the following scheme: 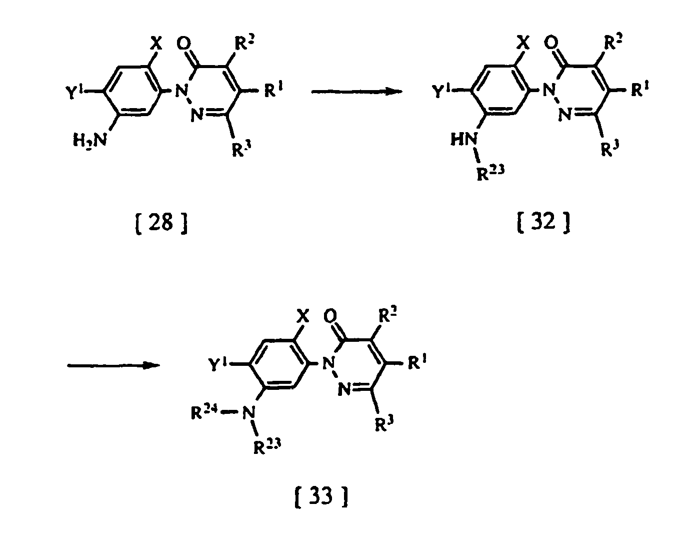 wherein R²³ and R²⁴ are independently a substituent other than hydrogen, which is included in the definition of R¹¹ and R¹²: or COR¹³, SO₂R¹⁴, SO²R¹⁵, or COOR¹⁰,
wherein R¹⁰, R¹³, R¹⁴, and R¹⁵ are as defined above; X, Y¹, R¹, R², and R³ are as defined above.

### Process for Producing compound [32] from Compound [28]

Compound [32] can be produced by reacting compound [28] with a compound of the formula:

R²⁰-D [34]

wherein R²⁰ is a substituent other than hydrogen, which is included in the definition of R¹¹ or R¹²; or COR¹³, SO₂R¹⁴, SO₂R¹⁵, or COOR¹⁰, wherein R¹⁰, R¹³, R¹⁴, and R¹⁵ are as defined above; and D is as defined above; or with a compound of the formula:

(R²¹)₂O [35]

wherein R²¹ is COR¹³, SO₂R¹⁴, SO₂R¹⁵, or COOR¹⁰, wherein R¹⁰, R¹³, R¹⁴, and R¹⁵ are as defined above, usually in the presence of a base and usually in a solvent.

The reaction temperature is usually in the range of-20° to 200°C, preferably 0° to 180°C. The reaction time is usually in the range of a moment to 72 hours. The amounts of the reagents to be used in the reaction, although the proportion of 1 mole of compound [34] or [35] to 1 mole of compound [28] is ideal, can be freely changed depending upon the reaction conditions.

Examples of the base which can be used include organic bases such as pyridine and triethylamine, and inorganic bases.

Examples of the solvent which can be used include aliphatic hydrocarbons such as hexane, heptane, ligroin, cyclohexane, and petroleum ether; aromatic hydrocarbons such as benzene, toluene, and xylene; halogenated hydrocarbons such as chloroform, carbon tetrachloride, dichloromethane, dichloroethane, chlorobenzene, and dichlorobenzene; ethers such as diethyl ether, diisopropyl ether, dioxane, tetrahydrofuran, and ethylene glycol dimethyl ether; ketones such as acetone, methyl ethyl ketone, methyl isobutyl ketone, isophorone, and cyclohexanone; esters such as ethyl formate, ethyl acetate, butyl acetate, and diethyl carbonate; nitro compounds such as nitromethane and nitrobenzene; nitriles such as acetonitrile and isobutyronitrile; acid amides such as formamide. N,N-dimethylformamide, and acetamide; tertiary amines such as pyridine, triethylamine, diisopropylethylamine, N,N-dimethylaniline, N,N-diethylaniline, and N-methylmorpholine; sulfur compounds such as dimethylsulfoxide and sulforane; and mixtures thereof.

After completion of the reaction, the reaction mixture is filtered to collect the precipitated crystals, or the reaction mixture is subjected to ordinary post-treatments such as extraction with an organic solvent and concentration, followed by, if necessary, subsequent purification by a technique such as column chromatography or recrystallization. Thus the desired product can be isolated.

### Process for Producing compound [33] from Compound [32]

Compound [33] can be produced by reacting compound [32] with compound [34] or [35]. This production process is based on the process for producing compound [32] from compound [28].

### (Production Process 7)

This is the production process according to the following scheme: 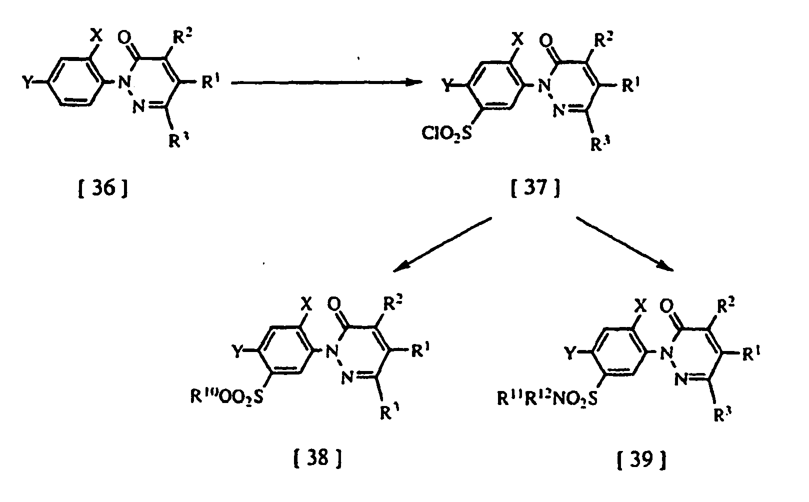 wherein X, Y, R¹, R², R³, R¹⁰, R¹¹, and R¹² are as defined above.

### Process for Producing compound [37] from compound [36]

Compound [37] can be produced by reacting compound [36] with chlorosulfonic acid without any solvent or in a solvent.

Amount of chlorosulfonic acid: 1 mole to an excess per mole of compound [36]
Solvent: sulfuric acid
Temperature: 0° to 70°C
Time: a moment to 24 hours
   (see Org. Syn. Coll., Vol. 1, 8 (1941))

### Process for Producing Compound [38] from Compound [37]

Compound [38] can be produced by reacting compound [37] with a compound of the formula:

R¹⁰-OH [40]

wherein R¹⁰ is as defined above, in the presence of a base without any solvent or in a solvent.

Amount of compound [24]: 1 mole to an excess per mole of compound [37]
Base: organic bases such as triethylamine or inorganic bases such as potassium carbonate
Amount of base: 1 to 2 moles per mole of compound [37]
Solvent: N,N-dimethylformamide, 1,4-dioxane, or the like
Temperature: 0° to 100°C
Time: a moment to 24 hours

### Process for Producing Compound [39] from Compound [37]

Compound [39] can be produced by reacting compound [37] with a compound of the formula:

R¹¹R¹²NH [41]

wherein R¹¹ and R¹² are as defined above, in the presence or absence of a base without any solvent or in a solvent.
Amount of compound [41]: 1 mole to an excess per mole of compound [37]
Base: organic bases such as triethylamine or inorganic bases such as potassium carbonate
Amount of base: 1 to 2 moles per mole of compound [37]
Solvent: 1,4-dioxane, N,N-dimethylformamide, or the like
Temperature: 0° to 100°C
Time: a moment to 24 hours

### (Production Process 8)

This is the production process according to the following scheme: 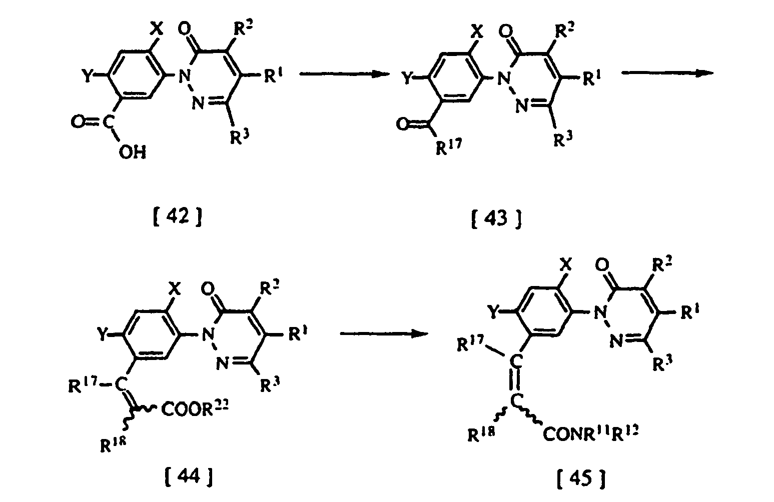 wherein X, Y, R¹, R², R³, R¹¹, R¹², R¹⁷, R¹⁸, and R²² are as defined above.

### Process for Producing Compound [43] from Compound [42]

Compound [43] can be produced from compound [42] according to the method described in JP-A 5-294920/1993, pp. 15-16.

### Process for Producing Compound [44] from Compound [43]

Compound [44] can be produced by reacting compound [43] with a compound of the formula:

(C₆H₅)₃P=CR¹⁸COOR²² [45]

or

(C₂H₅O)₂P(O)CHR¹⁸COOR²² [46]

wherein R¹⁸ and R²² are as defined above, in a solvent, and when compound [46] is used, in the presence of a base.
Amount of compound [45] or [46]: 1 to 2 moles per mole of compound [43]
Solvent: tetrahydrofuran, toluene, or the like
Base: sodium hydride or the like
Amount of base: 1 to 2 moles per mole of compound [43]
Temperature: 0° to 50°C
Time: a moment to 24 hours

### Process for Producing Compound [45] from Compound [44]

Compound[45] can be produced by reacting compound [44] with compound [41].

### (Production Process 9)

This is the production process according to the following scheme: 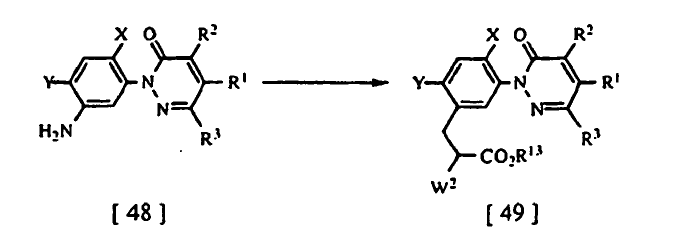 wherein W² is chlorine or bromine; and X, Y, R¹, R², R³, and R¹³ are as defined above.

The reaction conditions are described, for example, in USP 5,208,212.

The production can be achieved by converting compound [48] into a diazonium salt in a solution of hydrochloric acid, hydrobromic acid, or the like according to the ordinary method, and then reacting it with a compound of the formula:

CH₂=CHCO₂R¹³ ] [50

wherein R¹³ is as defined above, in the presence of a copper salt, such as copper (II) chloride or copper (II) bromide, in a solvent such as acetonitrile.

The reaction temperature is usually in the range of -20° to 150°C, preferably 0° to 60°C. The reaction time is usually in the range of a moment to 72 hours.

After completion of the reaction, the reaction mixture is filtered to collect the crystals, which may be precipitated by the addition of water, if necessary, or the reaction mixture is subjected to ordinary post-treatments such as extraction with an organic solvent and concentration, followed by, if necessary, subsequent purification by a technique such as column chromatography or recrystallization. Thus the desired product can be isolated.

### (Production Process 10)

This is the production process according to the following scheme: 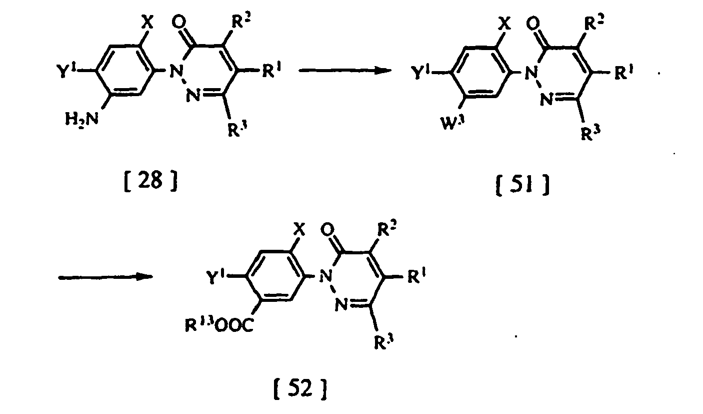 wherein W³ is bromine or iodine: and X, Y¹, R¹, R², R³, and R¹³ are as defined above.

### Process for Producing Compound [51] from compound [28]

Compound [51] can be produced by 1) making a diazonium salt from compound [28] in a solvent and then 2) reacting it with potassium iodide or copper (1) bromide in a solvent.

### <Reaction 1)>

Diazotizing agent: sodium nitrite, potassium nitrite, or the like.
Amount of diazotizing agent: 1 to 2 moles per mole of compound [28]
Solvent: aqueous hydrogen bromide, aqueous sulfuric acid, or the like.
Temperature: -10° to 10°C
Time: a moment to 5 hours

### <Reaction 2)>

Amount of potassium iodide or copper (1) bromide: 1 mole to an excess per mole of compound [28]
Solvent: aqueous hydrogen bromide, aqueous sulfuric acid, or the like
Temperature: 0° to 80°C
Time: a moment to 24 hours
   (see Org. Syn. Coll., Vol. 2, 604 (1943), and *ibid.,* Vol. 1. 136 (1941))

### Process for Producing Compound [52] from Compound [51]

Compound [52] can be produced by reacting compound [51] with a compound of the formula:

R¹³-OH [53]

wherein R¹³ is as defined above, in the presence of a transition metal catalyst and a base in a solvent under an atmosphere of carbon monoxide.
Catalyst: PdCl₂(PPh₃)₂ or the like
Amount of catalyst: a catalytic amount to 0.5 mole per mole of compound [51]
Amount of compound [53]: 1 mole to an excess per mole of compound [51]
Base: organic bases such as diethylamine
Amount of base: 1 to 2 moles per mole of compound [51]
Solvent: N,N-dimethylformamide or the like
Pressure of carbon monoxide: I to 150 atm.
Temperature: 0° to 100°C
Time: a moment to 72 hours
   (see Bull. Chem. Soc. Jpn., 48 (7) 2075 (1975))

### (Production Process 11)

This is the production process according to the following scheme: 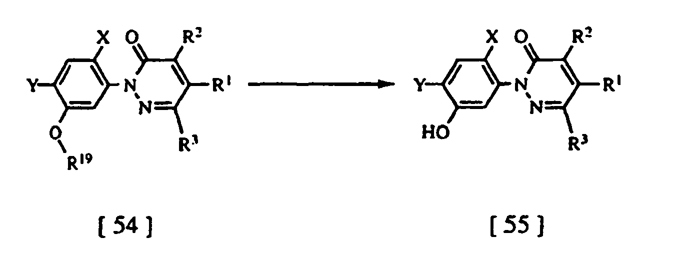 wherein X, Y, R¹, R², R³, and R¹⁹ are as defined above.

Compound [55] can be produced by hydrolyzing compound [54] in an acid solvent such as sulfuric acid, or in the presence of an acid such as boron tribromide in a solvent such as methylene chloride.

The reaction temperature is usually in the range of -20° to 150°C, preferably 0° to 100°C. The reaction time is usually in the range of a moment to 72 hours.

The amount of the acid to be used in the reaction, although the proportion of I mole of the acid to 1 mole of compound [54] is ideal, can be freely changed depending upon the reaction conditions.

After completion of the reaction, the reaction mixture is filtered to collect the crystals, which may be precipitated by the addition of water, if necessary, or the reaction mixture is subjected to ordinary post-treatments such as extraction with an organic solvent and concentration, followed by, if necessary, subsequent purification by a technique such as column chromatography or recrystallization. Thus the desired product can be isolated.

### (Production Process 12)

This is the production process according to the following scheme: 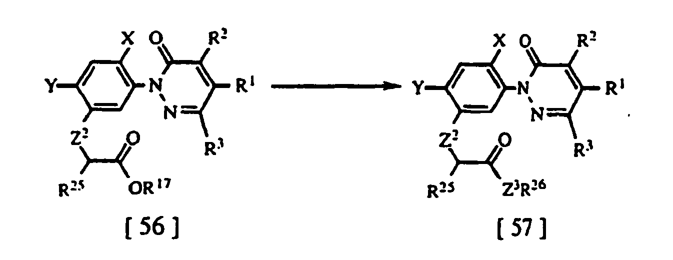 wherein X, Y, R¹, R², R³, Z², and R¹⁷ are as defined above; Z³ is oxygen or sulfur; R²⁵ is hydrogen or C₁-C₅ alkyl; and R²⁶ is C₁-C₆ alkyl, C₃-C₆ alkenyl, or C₃-C₆ alkynyl.

Compound [57] can be produced by reacting compound [56] with a compound of the formula:

R²⁶Z³H [58]

wherein R²⁶ and Z³ are as defined above, in the presence or absence of a catalyst and usually in a solvent.

The amount of compound [58] to be used in the reaction, although the proportion of I mole of compound [58] to 1 mole of compound [56] is ideal, can be freely changed depending upon the reaction conditions.

Examples of the catalyst which can be used include p-toluenesulfonic acid. Examples of the solvent which can be used include toluene, xylene or the like, or compound [56].

The reaction temperature is usually in the range of 0° to 200°C, preferably 50° to 150°C. The reaction time is usually in the range of a moment to 72 hours.

After completion of the reaction, the reaction mixture is filtered to collect the crystals, which may be precipitated by the addition of water, if necessary, or the reaction mixture is subjected to ordinary post-treatments such as extraction with an organic solvent and concentration, followed by, if necessary, subsequent purification by a technique such as column chromatography or recrystallization. Thus the desired product can be isolated.

### (Production Process 13)

This is the production process according to the following scheme: 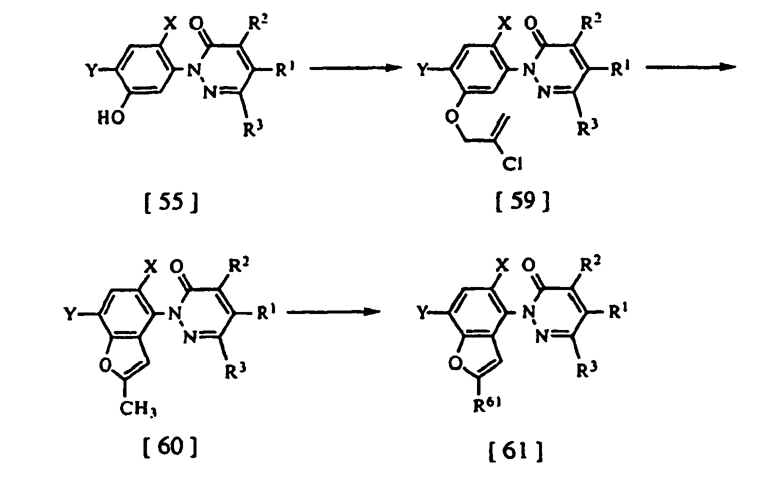 wherein R⁶¹ is a substituent other than methyl, which is included in the definition of R⁶; and X. Y, R¹, R², and R³ are as defined above.

### Process for Producing Compound [59] from Compound [55]

Compound [59] can be produced by reacting compound [55] with 2,3-dichloropropene in the presence of a base in a solvent.
Amount of 2,3-dichloropropene: 1 to 3 moles per mole compound [55]
Base: inorganic bases such as potassium carbonate
Amount of base: 1 to 2 moles per mole of compound [55]
Solvent: N,N-dimethylformamide or the like
Temperature: 0° to 70°C
Time: a moment to 24 hours

### Process for Producing Compound [60] from Compound [59]

Compound [60] can be produced by heating compound [59] in a solvent.

Solvent: N,N-dimethylformamide, N,N-dimethylaniline, N,N-diethylaniline, p-diisopropylbenzene, or the like
Temperature: 70° to 200°C
Time: a moment to 24 hours

### Process for Producing Compound [61] from Compound [60]

Compound [61] can be produced from compound [62] according to the method in which the methyl group in position 2 on the benzofuran ring is replaced with another substituent, as described in USP 5,308,829, columns 2-11.

### (Production Process 14)

This is the production process according to the following scheme: 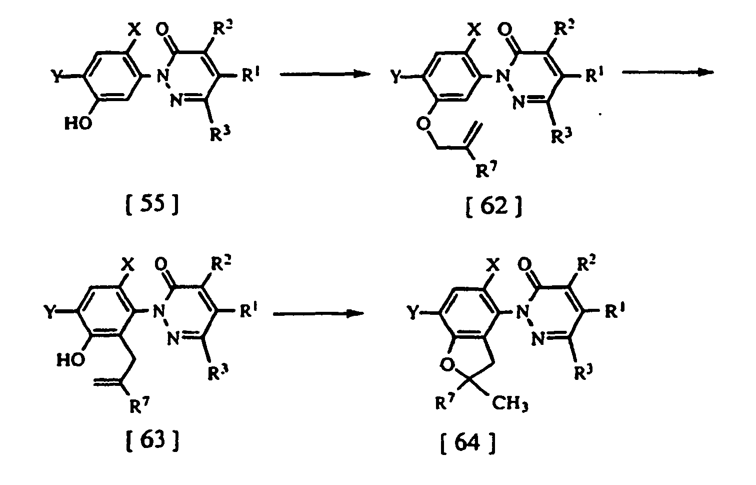 wherein X, Y, R¹, R², R³, and R⁷ are as defined above.

### Process for Producing Compound [62] from compound [55]

Compound [62] can be produced by reacting compound [55] with a compound of the formula:

CH₂=CR⁷CH₂W² [65]

wherein W² and R⁷ are as defined above, in the presence of a base in a solvent.
Amount of compound [65]: 1 to 5 moles per mole of compound [55]
Base: inorganic bases such as potassium carbonate
Amount of base: I to 2 moles per mole of compound [55]
Solvent: N,N-dimethylforman-dde, 1,4-dioxane, or the like
Temperature: 0° to 70°C
Time: a moment to 24 hours

### Process for Producing Compound [63] from Compound [62]

Compound [64] can be produced by heating compound [62] in a solvent.

Solvent: N,N-dimethylaniline, N,N-diethylaniline, p-diisopropylbenzene, or the like
Temperature: 100° to 200°C
Time: a moment to 24 hours

### Process for Producing Compound [64] from Compound [63]

Compound [64] can be produced by heating compound [63] in the presence of an acid in a solvent.

Acid: organic acids such as p-toluenesulfonic acid; and inorganic acids such as sulfuric acid
Amount of acid: a catalytic amount to 1 mole per mole of compound [63]
Solvent: toluene, xylene, or the like
Temperature: 100° to 250°C
Time: a moment to 24 hours

### (Production Process 15)

This is the production process according to the following scheme: 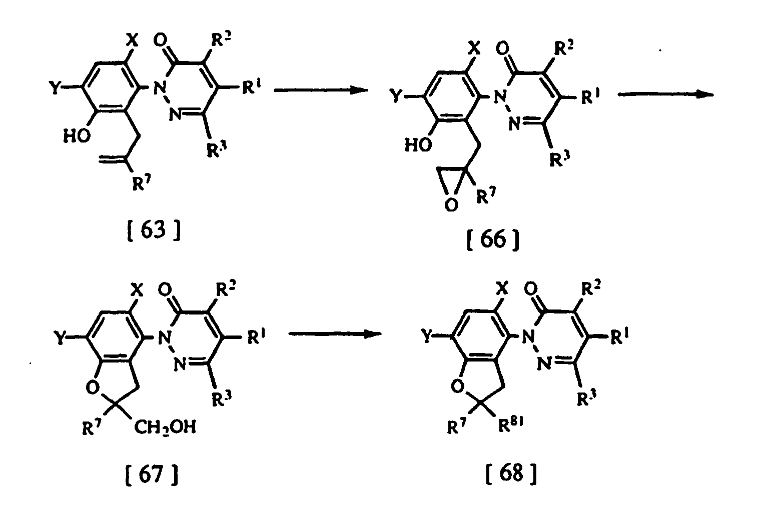 wherein R⁸¹ is a substituent other than methyl and hydroxymethyl, which is included in the definition of R⁸; and X, Y, R¹, R², R³, and R⁷ are as defined above.

### Process for Producing Compound [66] from Compound [63]

Compound [66] can be produced by reacting compound [63] with a peracid in a solvent.
Peracid: m-chloroperbenzoic acid or peracetic acid
Amount of peracid: 1 mole to an excess per mole of compound [63]
Solvent: halogenated hydrocarbons such as dichloromethane; and organic acids such as acetic acid
Temperature: -20°C to room temperature
Time: a moment to 24 hours

### Process for Producing Compound [67] from Compound [66]

Compound [67] can be produced by reacting compound [66] in the presence of a base in a solvent.
Base: potassium carbonate or the like
Amount of base: I to 2 moles per mole of compound [66]
Solvent: methanol, ethanol, or the like
Temperature: 0° to 50°C
Time: a moment to 5 hours

### Process for Producing Compound [68] from Compound [67]

Compound [68] can be produced from compound [67] according to the method in which the hydroxyalkyl group in position 2 on the dihydrobenzofuran ring is replaced with another substituent, as described in USP 5,411,935, columns 5-10.

### (Production Process 16)

This is the production process according to the following scheme: 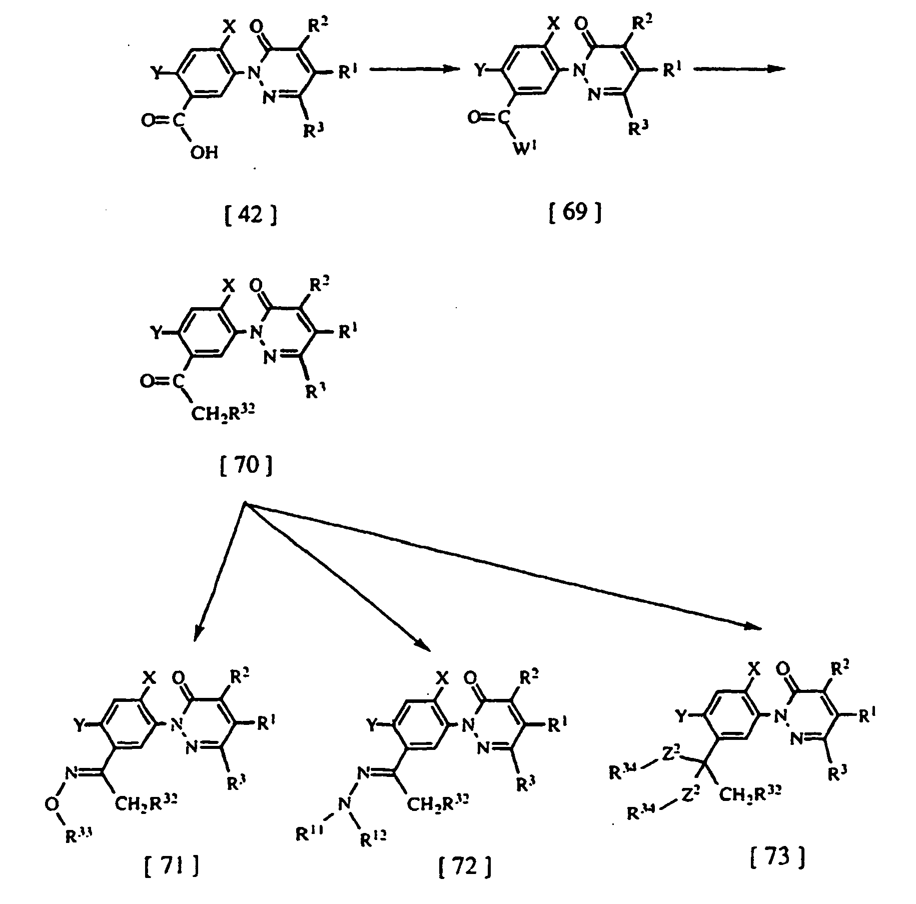 wherein W¹ is halogen, preferably chlorine; R³² is hydrogen or C₁-C₅ alkyl; and X, Y, Z², R¹, R², R³, R¹¹, R¹², R³³, and R³⁴ are as defined above.

### Process for Producing Compound [69] from compound [42]

Compound [69] can be produced by reacting compound [42] with a halogenating agent such as thionyl chloride in a solvent according to the ordinary method.

### Process for Producing Compound [70] from Compound [69]

Compound [70] can be produced by reacting compound [69] with a compound of the formula: 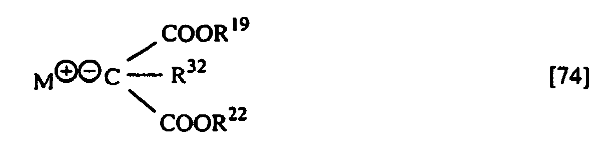 wherein M^{⊕} is an alkali metal cation, preferably lithium cation or sodium cation; and R¹⁹, R²², and R³² are as defined above, to give a compound of the formula: 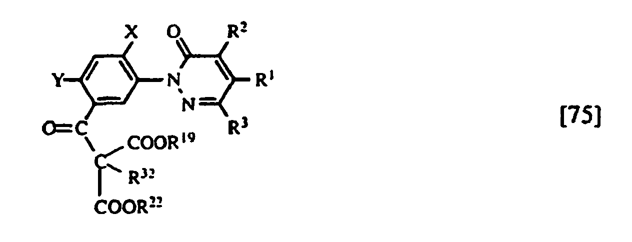 wherein X, Y, R¹, R², R³, R¹⁹, R²², and R³² are as defined above, and then hydrolyzing and decarboxylating compound [75].

The first reaction is usually effected in a solvent. The reaction temperature is usually in the range of -20° to 50°C, preferably room temperature. The reaction time is usually in the range of a moment to 72 hours.

Examples of the solvent which can be used include aliphatic hydrocarbons such as hexane, heptane, ligroin, cyclohexane, and petroleum ether; ethers such as diethyl ether, diisopropyl ether, dioxane, tetrahydrofuran, and ethylene glycol dimethyl ether; acid amides such as formamide, N.N-dimethylformamide, and acetamide: sulfur compounds such as dimethylsulfoxide and sulforane; and mixtures thereof.

The second reaction is effected in the presence of sulfuric acid, hydrobromic acid, or the like in a solvent such as a lower carboxylic acid, e.g., acetic acid, or without any solvent. The reaction temperature is usually in the range of 80° to 140°C, preferably 100° to 120°C. The reaction time is usually in the range of a moment to 72 hours.

### Process for Producing Compound [71] from Compound [70]

Compound [71] can be produced by reacting compound [70] with a compound of the formula:

H₂N-O-R³³ [76]

wherein R³³ is as defined above.

The reaction is effected in a lower alcohol such as methanol, ethanol or isopropanol, or in a mixed solution of such a lower alcohol and water. The reaction temperature is in the range of 0° to 80°C. The reaction time is in the range of a moment to 72 hours.

Compound [76] can be used in the form of a free base or an acid addition salt such as a hydrochloride salt or a sulfate salt.

The above reaction can also be effected with the addition of a basic catalyst such as an organic base, e.g., pyridine; an alkali metal carbonate, e.g., sodium carbonate, potassium carbonate or the like; alkali metal hydrogencarbonate; or alkaline earth metal carbonate.

Compound [71] can also be produced by reacting a compound of the formula: 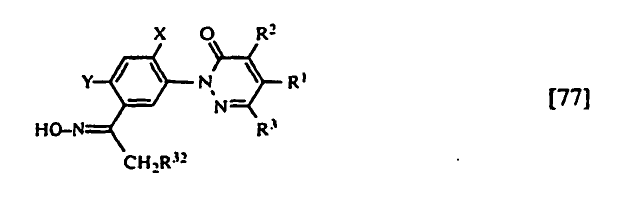 wherein X, Y, R¹, R², R³, and R³² are as defined above, with a compound of the formula:

R³³-D [78]

wherein R³³ and D are as defined above, in the presence of a base, usually in a solvent.

Examples of the base which can be used include alkali metal alcoholates and alkali metal hydrides such as sodium hydride.

The amounts of the reagents to be used in the reaction, although the proportion of about 1 mole of compound [78] and I to 2 moles of the base to 1 mole of compound [77] is ideal, can be free changed depending upon the reaction conditions.

Examples of the solvent which can be used include ethers such as diethyl ether, diisopropyl ether, dioxane, tetrahydrofuran, and ethylene glycol dimethyl ether; acid amides such as formamide, N,N-dimethylformamide, and acetamide; sulfur compounds such as dimethylsulfoxide and sulforane; alcohols such as methanol, ethanol, ethylene glycol, and isopropanol; and mixtures thereof.

The reaction temperature in the above reaction is in the range of -10° to 100°C, preferably 0° to 80°C. The reaction time is in the range of a moment to 72 hours.

### Process for Producing Compound [72] from compound [70]

Compound [72] can be produced by reacting compound [70] with a compound of the formula: 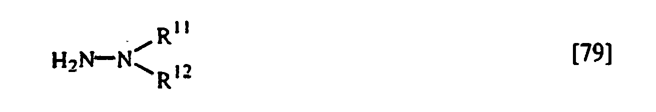 wherein R¹¹ and R¹² are as defined above.

The reaction is effected in a lower alcohol such as methanol, ethanol or isopropanol, or in a mixed solution of such a lower alcohol and water. The reaction temperature is in the range of 0° to 80°C. The reaction time is in the range of a moment to 72 hours.

Compound [79] can be used in the form of a free base or an acid addition salt such as a hydrochloride salt or a sulfate salt.

The above reaction can also be effected with the addition of a basic catalyst such as an organic base, e.g., pyridine; an alkali metal carbonate, e.g., sodium carbonate, potassium carbonate or the like; alkali metal hydrogencarbonate; or alkaline earth metal carbonate.

### Process for Producing Compound [73] from Compound [70]

Compound [73] can be produced by reacting compound [70] with a compound of the formula:

R³⁴-Z²H [80]

wherein Z² and R³⁴ are as defined above, usually in the presence of a catalytic amount to an excess of an acid such as p-toluenesulfonic acid, hydrochloric acid or sulfuric acid, in an organic solvent such as benzene or chloroform.

The reaction temperature is in the range of -30°C to the boiling temperature of the reaction mixture. The reaction time is in the range of a moment to 72 hours.

### (Production Process 17)

This is the production process according to the following scheme: 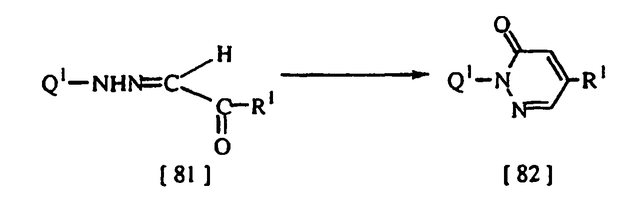 wherein Q¹ and R¹ are as defined above.

Compound [82] can be produced by reacting compound [81] with a compound of the formula: 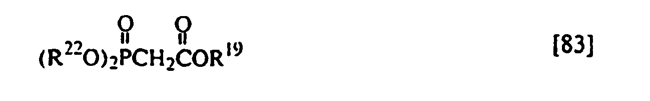 wherein R 19 and R²² are as defined above, in a solvent.

The reaction temperature is usually in the range of 30° to 120°C, preferably 40° to 80°C. The reaction time is usually in the range of 5 to 72 hours. The amounts of the reagents to be used in the reaction, although the proportion of 1 mole of compound [83] to 1 mole of compound [81] is ideal, can be changed depending upon the reaction conditions.

Examples the solvent which can be used include tertiary amines such as triethylamine.

After completion of the reaction, the reaction solvent is distilled out from the reaction mixture and the residue is subjected to chromatography, or the reaction mixture is subjected to ordinary post-treatments such as extraction with an organic solvent and concentration, followed by, if necessary, subsequent purification by a technique such as column chromatography or recrystallization. Thus the desired compound of the present invention can be isolated.

### (Production Process 18)

This is the production process according to the following scheme: 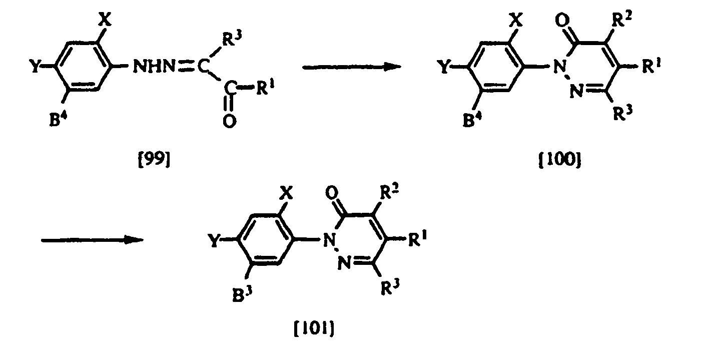 wherein B³ is OR³⁵, SR³⁵, COOR³⁵, COR ¹⁶, or CR¹⁷=CR¹⁸COR¹⁶ (wherein R³⁵ is (C₁-C₆ alkyl)carbonyl C₁-C₆ alkyl (C₁-C₆ haloalkyl)carbonyl C₁-C₆ alkyl, {(C₁-C₄ alkoxy) C₁-C₄ alkyl carbonyl C₁-C₆ alkyl, or (C₃-C₈ cycloalkyl)carbonyl C₁-C₆ alkyl; and R¹⁶, R¹⁷, and R¹⁸ are as defined above); B⁴ is a substituent derived from B³ by protecting its ketone or aldehyde moiety with an alcohol; and X, Y, R¹, R², and R³ are as defined above.

### Process for Producing Compound [100] from Compound [99]

Compound [100] can be produced in the same manner as described in Production Process 1, except that compound [99] is used in place of compound [4].

### Process for Producing Compound [101] from Compound [100]

Compound [101] can be produced by deprotecting the ketal or acetal moiety of compound [100] by the ordinary method.

Compound [99] can be produced in the same manner as described below in the production process for compound [4], except that the ketone or aldehyde moiety in the substituent B³ of a compound of the formula: 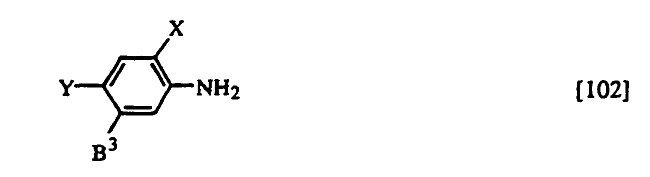 wherein X, Y, and B³ are as defined above, is protected with an alcohol such as methanol to give a compound of the formula: 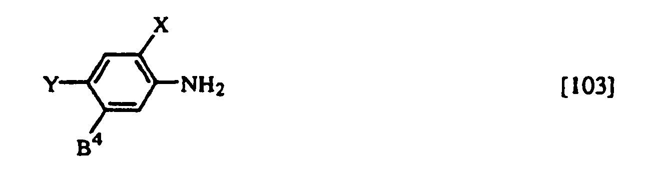 wherein X, Y, and B⁴ are as defined above, and compound [103] is used in place of compound [91] as described below.

Compound [5], which is one of the starting compounds in the production of the present compounds by production process 1, can be obtained from commercial sources or can be produced, for example, according to the method described in Jikken Kagaku Kouza (Maruzen K.K.), 4th ed., Vol. 24, pp. 259-260.

Compound [4], which is the other starting compound used in production process 1, can be produced by reacting a compound of the formula: 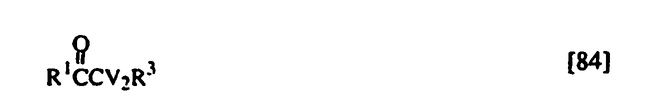 wherein R¹ and R³ are as defined above; and V is iodine, bromine, or chlorine, with water in the presence of a base to give a compound of the formula: 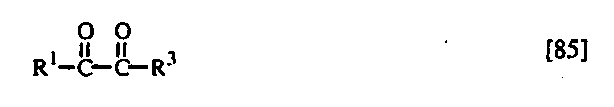 wherein R¹ and R³ are as defined above (hereinafter referred to as reaction 1), and then reacting compound [85] with a compound of the formula:

Q¹-NHNH₂ [86]

wherein Q¹ is as defined above (hereinafter referred to as reaction 2).

Compound [85] can also be reacted as its hydrate or acetal derivative in water or an alcohol.

Reaction 1 is usually effected in a solvent. The reaction temperature is usually in the range of 20° to 100°C. The reaction time is usually in the range of a moment to 24 hours. The amounts of the reagents to be used in the reaction, although the proportion of 2 moles of water and 2 moles of a base to 1 mole of compound [84] is ideal, can be changed, if necessary.

Examples of the base which can be used include organic bases and inorganic bases such as sodium acetate and potassium acetate.

Examples of the solvent which can be used include aliphatic hydrocarbons such as hexane, heptane, ligroin, cyclohexane, and petroleum ether; aromatic hydrocarbons such as benzene, toluene, and xylene; halogenated hydrocarbons such as chlorobenzene and dichlorobenzene; ethers such as diethyl ether, diisopropyl ether, dioxane, tetrahydrofuran, and ethylene glycol dimethyl ether; esters such as ethyl formate, ethyl acetate, butyl acetate, and diethyl carbonate; nitro compounds such as nitromethane and nitrobenzene: nitriles such as acetonitrile and isobutyronitrile; acid amides such as N,N-dimethylformamide; tertiary amines such as pyridine, triethylamine, diisopropylethylamine, N,N-dimethylaniline. N,N-diethylaniline, and N-methylmorpholine; sulfur compounds such as dimethylsulfoxide and sulforane; alcohols such as methanol, ethanol, ethylene glycol, and isopropanol; water; and mixtures thereof.

Reaction 2 is usually effected in a solvent. The reaction temperature is usually in the range of -20° to 200°C. The reaction time is usually in the range of a moment to 72 hours. The amounts of the reagents to be used in the reaction, although the proportion of 1 mole of compound [86] to 1 mole of compound [84] used in reaction 1 is ideal, can be freely changed depending upon the reaction conditions. If necessary, the hydrochloride salt or sulfate salt of compound [86] can also be used.

Examples of the solvent which can be used include aliphatic hydrocarbons such as hexane, heptane, ligroin, cyclohexane, and petroleum ether; aromatic hydrocarbons such as benzene, toluene, and xylene; halogenated hydrocarbons such as chloroform, carbon tetrachloride, dichloromethane, dichloroethane, chlorobenzene, and dichlorobenzene; ethers such as diethyl ether, diisopropyl ether, dioxane, tetrahydrofuran, and ethylene glycol dimethyl ether; esters such as ethyl formate, ethyl acetate, butyl acetate, and diethyl carbonate; nitro compounds such as nitromethane and nitrobenzene; nitriles such as acetonitrile and isobutyronitrile; acid amides such as formamide, N,N-dimethylformamide, and acetamide; tertiary amines such as pyridine, triethylamine, diisopropylethylamine, N,N-dimethylaniline, N,N-diethylaniline, and N-methylmorpholine; sulfur compounds such as dimethylsulfoxide and sulforane; fatty acids such as formic acid, acetic acid, and propionic acid; alcohols such as methanol, ethanol, ethylene glycol, and isopropanol; water; and mixtures thereof.

After completion of the reaction, the reaction mixture is filtered to collect the crystals, which may be precipitated by the addition of water, if necessary, or the reaction mixture is subjected to ordinary post-treatments such as extraction with an organic solvent and concentration, followed by, if necessary, subsequent purification by a technique such as column chromatography or recrystallization. Thus the desired product can be isolated.

Among the examples of compound [4], a compound of the formula: 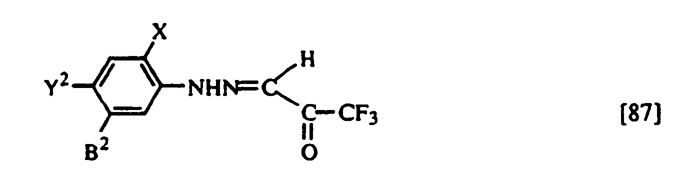 wherein X is as defined above: Y² is halogen; and B² is hydrogen, halogen, C₁-C₆ alkoxy, or C₁-C₆ alkylthio, can also be produced according to the following scheme: 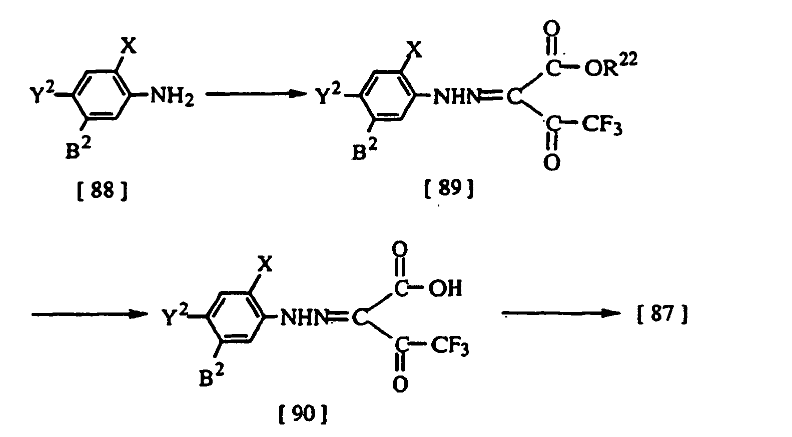 wherein X, Y², B², and R²² are as defined above.

### Process for Producing Compound [89] from Compound [88]

Compound [89] can be produced by reacting compound [88] with a nitrite salt in hydrochloric acid or sulfuric acid to convert it into a diazonium salt, and then reacting the diazonium salt with a compound of the formula: 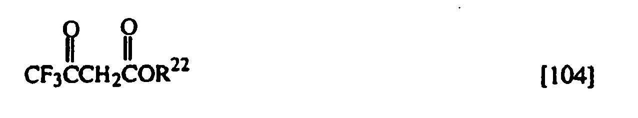 wherein R²² is as defined above, in the presence of a base such as sodium acetate or pyridine.
(see, e.g., Tetrahedron, Vol. 35, p. 2013 (1979))

### Process for Producing Compound [90] from Compound [89]

Compound [90] can be produced by hydrolyzing compound [89] usually in the presence of a base in a solvent.

The reaction temperature is in the range of 0° to 150°C, preferably 20° to 100°C. The reaction time is in the range of 1 to 24 hours, preferably I to 10 hours. The amounts of the reagents to be used in the reaction, although the proportion of 1 mole of a base to 1 mole of compound [89] is ideal, can be changed, if necessary.

Examples of the base which can be used include inorganic bases such as potassium hydroxide, lithium hydroxide, barium hydroxide, and sodium hydroxide.

Examples of the solvent which can be used include aliphatic hydrocarbons such as hexane, heptane, ligroin, cyclohexane, and petroleum ether; aromatic hydrocarbons such as benzene, toluene, and xylene; halogenated hydrocarbons such as chlorobenzene and dichlorobenzene; ethers such as diethyl ether, diisopropyl ether, dioxane, tetrahydrofuran, and ethylene glycol dimethyl ether; ketones such as acetone, methyl ethyl ketone, methyl isobutyl ketone, isophorone, and cyclohexanone; nitro compounds such as nitromethane and nitrobenzene; acid amides such as N,N-dimethylformamide; tertiary amines such as pyridine, triethylamine, diisopropylethylamine, N,N-dimethylaniline, N,N-diethylaniline, and N-methylmorpholine; sulfur compounds such as dimethylsulfoxide and sulforane; alcohols such as methanol, ethanol, ethylene glycol, and isopropanol; water; and mixtures thereof.

### Process for Producing Compound [87] from Compound [90]

Compound [87] can be produced by heating compound [90] in a solvent.

The reaction temperature is in the range of 50° to 200°C. preferably 50° to 150°C. The reaction time is in the range of a moment to 72 hours.

Examples of the solvent which can be used include aliphatic hydrocarbons such as hexane, heptane, ligroin, cyclohexane, and petroleum ether; aromatic hydrocarbons such as benzene, toluene, and xylene; halogenated hydrocarbons such as chloroform, carbon tetrachloride, dichloromethane, dichloroethane, chlorobenzene, and dichlorobenzene; ethers such as diethyl ether, diisopropyl ether, dioxane, tetrahydrofuran, and ethylene glycol dimethyl ether; ketones such as acetone, methyl ethyl ketone, methyl isobutyl ketone, isophorone, and cyclohexanone; esters such as ethyl formate, ethyl acetate, butyl acetate, and diethyl carbonate; nitro compounds such as nitromethane and nitrobenzene; nitriles such as acetonitrile and isobutyronitrile; acid amides such as formamide, N,N-dimethylformamide, and acetamide; tertiary amines such as pyridine, triethylamine, diisopropylethylamine, N,N-dimethylaniline, N,N-diethylaniline, and N-methylmorpholine; sulfur compounds such as dimethylsulfoxide and sulforane; fatty acids such as formic acid, acetic acid, and propionic acid; alcohols such as methanol, ethanol, ethylene glycol, and isopropanol; water; and mixtures thereof.

The above reaction can also be effected with the use of a metal, e.g., copper, as a catalyst.

After completion of the reaction, the reaction mixture is filtered to collect the precipitated crystals, or the reaction mixture is subjected to ordinary post-treatments such as extraction with an organic solvent and concentration, followed by, if necessary, subsequent purification by a technique such as column chromatography or recrystallization. Thus the desired compound can be isolated. Compound [86] can also be produced by the following scheme: 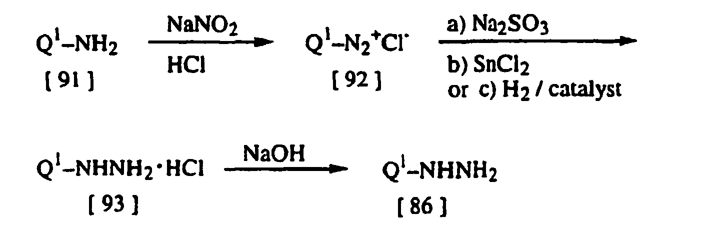 wherein Q¹ is as defined above.
(see Organic Synthesis Collective, Vol. 1, p. 442)

Compound [91] is known in, or can be produced according to the method as described in, EP-61741-A; USP 4,670,046, USP 4,770,695, USP 4,709,049, USP 4,640,707, USP 4,720,297, USP 5,169,431; and JP-A 63-156787/1988.

Some examples of compound [91] can also be produced according to the following scheme: 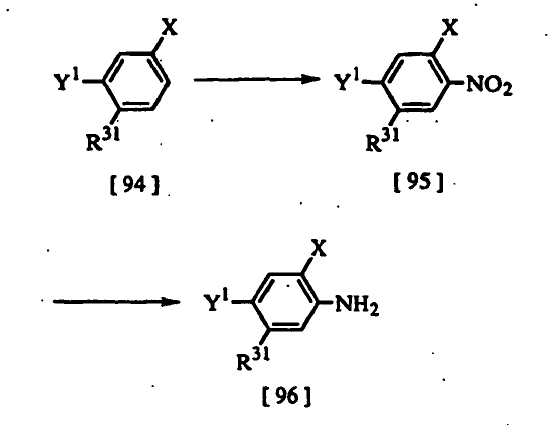 wherein R³¹ is COR¹⁶ or COOR¹⁰.

### Process for Producing Compound [95] from Compound [94]

Compound [95] can be produced by reacting compound [94] with nitric acid in a solvent.

The reaction temperature is usually in the range of 0° to 100°C. The reaction time is usually in the range of a moment to 24 hours. The amounts of the reagents to be used in the reaction, although the proportion of 1 mole of nitric acid to 1 mole of compound [94] is ideal, can be freely changed depending upon the reaction conditions.

Examples of the solvent which can be used include acidic solvents such as mixtures of nitric acid and sulfuric acid.
(see Organic Synthesis Collective, Vol. 1, p. 372)

### Process for Producing Compound [96] from Compound [95]

Compound [96] can be produced by reducing compound [95] in a mixture of acetic acid, iron powder, and water.

The reaction temperature is usually in the range of 0° to 100°C. The reaction time is usually in the range of a moment to 24 hours.

After completion of the reaction, the reaction mixture is subjected to ordinary post-treatments such as extraction with an organic solvent and concentration, followed by, if necessary, subsequent purification by a technique such as column chromatography or recrystallization. Thus the desired compound can be isolated.
(see Organic Synthesis Collective, Vol. 2, p. 471, and *ibid.,* Vol. 5, p. 829)

The present compounds have excellent herbicidal activity, and some of them exhibit excellent selectivity between crop plants and unfavorable weeds. In particular, the present compounds have herbicidal activity against various unfavorable weeds as recited below, which may cause trouble in the foliar treatment and soil treatment on upland fields.

### Polygonaceae:

wild buckwheat *(Polygonum convolvulus),* pale smartweed *(Polygonum lapathifolium),* Pennsylvania smanweed *(Polygonum pensylvanicum),* ladysthumb *(Polygonum persicaria),* curly dock *(Rumex crispus),* broadleaf dock *(Rumex obtusifolius),* Japanese knotweed *(Polygonum cuspidatum)*
Portulacaceae:
   common purslane *(Portulaca oleracea)*
Caryophyllaceae:
   common chickweed (*Stellaria media)*
Chenopodiaceae:
   common lambsquarters *(Chenopodium album),* kochia *(Kochia scoparia)*
Amaranthaceae:
   redroot pigweed *(Amaranthus retroflexus),* smooth pigweed *(Amaranthus hybridus)*
Crusiferae:
   wild radish *(Raphanus raphanistrum),* wild mustard *(Sinapis arvensis),* shepherdspurse *(Capsella bursa-pastoris)*
Leguminosae:
   hemp sesbania (*Sesbania exaltata),* sicklepod *(Cassia obtusifolia),* Florida beggarweed *(Desmodium tortuosum),* white clover *(Trifolium repens)*
Malvaceae:
   velvetleaf *(Abutilon theophrasti),* prickly sida *(Sida spinosa)*
Violaceae:
   field pansy *(Viola arvensis),* wild pansy *(Viola tricolor)*
Rubiaceae:
   catchweed bedstraw (cleavers) *(Galium aparine)*
Convolvulaceae:
   ivyleaf morningglory *(Ipomoea hederacea),* tall morningglory *(Ipomoea purpurea),* entireleaf morningglory *(Ipomoea hederacea* var. *integriuscula),* pitted morningglory *(Ipomoea lacunosa),* field bindweed *(Convolvulus arvensis)*
Labiatae:
   red deadnettle *(Lamium purpureum),* henbit *(Lamium amplexicaule)*
Solanaceae:
   jimsonweed *(Datura stramonium),* black nightshade *(Solanum nigrum)*
Scrophulariaceae:
   birdseye speedwell *(Veronica persica),* ivyleaf speedwell *(Veronica hederae-folia)*
Compositae:
   common cocklebur *(Xanthium pensylvanicum),* common sunflower *(Helianthus annuus),* scentless chamomile *(Matricaria perforata* or *inodora),* corn marigold *(Chrysanthemum segetum),* pineappleweed *(Matricaria matricarioides),* common ragweed *(Ambrosia artemisiifolia),* giant ragweed *(Ambrosia trifida),* horseweed *(Erigeron canadensis),* Japanese mugwort *(Artemisia princeps),* tall goldenrod *(Solidago altissima)*
Boraginaceae:
   field forget-me-not *(Myosotis arvensis)*
Asclepiadaceae:
   common milkweed *(Asclepias syriaca)*
Euphorbiaceae:
   sun spurge *(Euphorbia helioscopia),* spotted spurge *(Euphorbia maculata*)
Gramineae:
   barnyardgrass *(Echinochloa crus-galli),* green foxtail (*Setaria viridis),* giant foxtail *(Setaria faberi),* large crabgrass (*Digitaria sanguinalis),* goosegrass *(Eleusine indica*)*,* annual bluegrass (*Poa annua),* blackgrass *(Alopecurus myosuroides),* wild oat *(Avena fatua*)*,* johnsongrass *(Sorghum halepense),* quackgrass *(Agropyron repens),* downy brome *(Bromus tectorum*)*,* bermudagrass *(Cynodon dactylon),* fall panicum *(Panicum dichotomiflorum),* Texas panicum *(Panicum texanum),* shattercane *(Sorghum vulgare*)
Commelinaceae:
   common dayflower *(Commelina communis)*
Equisetaceae:
   field horsetail *(Equisetum arvense)*
Cyperaceae:
   rice flatsedge *(Cyperus iria),* purple nutsedge *(Cyperus rotundus),* yellow nutsedge *(Cyperus esculentus)*

Furthermore, some of the present compounds have no problematic phytotoxicity on main crops such as corn *(Zea mays),* wheat *(Triticum aestivum),* barley *(Hordeum vulgare),* rice *(Oryza sativa),* sorghum *(Sorghum bicolor),* soybean *(Glycine max*), cotton *(Gossypium* spp.), sugar beet *(Beta vulgaris),* peanut *(Arachis hypogaea),* sunflower *(Helianthus annuus)* and canola *(Brassica napus);* garden crops such as flowers and ornamental plants; and vegetable crops.

The present compounds can attain effective control of unfavorable weeds in the no-tillage cultivation of soybean *(Glycine max),* corn *(Zea mays),* and wheat *(Triticum aestivum).* Furthermore, some of them exhibit no problematic phytotoxicity on crop plants.

The present compounds have herbicidal activity against various unfavorable weeds as recited below under the flooding treatment on paddy fields.
Gramineae:
   barnyardgrass *(Echinochloa oryzicola)*
Scrophulatiaceae:
   common falsepimpemel *(Lindernia procumbens)*
Lythraceae:
   *Rotala indica, Ammannia multiflora*
Elatinaceae:
   *Elatine triandra*
Cyperaceae:
   smallflower umbrellaplant *(Cyperus difformis),* hardstem bulrush (*Scirpus juncoides),* needle spikerush *(Eleocharis acicularis*)*, Cyperus serotinus, Eleocharis kuroguwai*
Pontederiaceae:
   *Monochoria vaginalis*
Alismataceae:
   *Sagittaria pygmaea, Sagittaria trifolia, Alisma canaliculatum*
Potamogetonaoeae:
   roundleaf pondweed (*Potamogeton distinctus)*
Umbelliferae:
   *Oenanthe javanica*

Furthermore, some of the present compounds have no problematic phytotoxicity on transplanted paddy rice.

The present compounds can attain effective control of various unfavorable weeds in orchards, grasslands, lawns, forests, waterways, canals, or other non-cultivated lands.

The present compounds also have herbicidal activity against various aquatic plants such as water hyacinth *(Eichhornia crassipes),* which will grow in waterways, canals, or the like.

The present compounds have substantially the same characteristics as those of the herbicidal compounds described in the publication of International Patent Application, WO95/34659. In the case where crop plants with tolerance imparted by introducing a herbicide tolerance gene described in the publication are cultivated, the present compounds can be used at greater doses than those used when ordinary crop plants without tolerance are cultivated, and it is, therefore, possible to attain effective control of other unfavorable plants.

When the present compounds are used as active ingredients of herbicides, they are usually mixed with solid or liquid carriers or diluents, surfactants, and other auxiliary agents to give formulations such as emulsifiable concentrates, wettable powders, flowables, granules, concentrated emulsions, and water-dispersible granules.

These formulations may contain any of the present compounds as an active ingredient at an amount of 0.001 % to 80% by weight, preferably 0.005% to 70% by weight, based on the total weight of the formulation.

Examples of the solid carrier or diluent may include fine powders or granules of the following materials: mineral matters such as kaolin clay, attapulgite clay, bentonite, terra alba, pyrophyllite, talc, diatomaceous earth, and calcite; organic substances such as walnut shell powder; water-soluble organic substances such as urea; inorganic salts such as ammonium sulfate; and synthetic hydrated silicon oxide. Examples of the liquid carrier or diluent may include aromatic hydrocarbons such as methylnaphthalene, phenylxylylethane, and alkylbenzenes (e.g., xylene): alcohols such as isopropanol, ethylene glycol, and 2-ethoxyethanol; esters such as phthalic acid dialkyl esters; ketones such as acetone, cyclohexanone, and isophorone; mineral oils such as machine oil; vegetable oils such as soybean oil and cotton seed oil; dimethylsulfoxide, N,N-dimethylformamide, acetonitrile, N-methylpyrrolidone, water, and the like.

Examples of the surfactant used for emulsification, dispersing, or spreading may include surfactants of the anionic type, such as alkylsulfates, alkylsulfonates, alkylarylsulfonates, dialkylsulfosuccinates, and phosphates of polyoxyethylene alkyl aryl ethers; and surfactants of the nonionic type, such as polyoxyethylene alkyl ethers, polyoxyethylene alkyl aryl ethers, polyoxyethylene polyoxypropylene block copolymers, sorbitan fatty acid esters, and polyoxyethylene sorbitan fatty acid esters.

Examples of the auxiliary agent used for formulation may include ligninsulfonates, alginates, polyvinyl alcohol, gum arabic, carboxymethyl cellulose (CMC), and isopropyl acid phosphate (PAP).

The present compounds are usually formulated as described above and then used for the pre- or post-emergence soil, foliar, or flooding treatment of unfavorable weeds. The soil treatment may include soil surface treatment and soil incorporation. The foliar treatment may include application over the plants and directed application in which a chemical is applied only to the unfavorable weeds so as to keep off the crop plants.

The present compounds can be used, if necessary, in combination with other compounds having herbicidal activity. Examples of the compounds which can be used in combination with the present compounds may include various compounds described in Catalog 1995 Edition of Farm Chemicals Handbook (Meister Publishing Company); AG CHEM NEW COMPOUND REVIEW, VOL: 13, 1995 (AG CHEM INFORMATION SERVICE); or JOSOUZAI KENKYU SOURAN (Hakuyu-sha). Typical examples of such compounds are as follows: atrazin, cyanazine, dimethametryn, metribuzin, prometryn, simazine, simetryn, chlorotoluron, diuron, dymuron, fluometuron, isoproturon, linuron, methabenzthiazuron, bromoxynil, ioxynil, ethalfluralin, pendimethalin, trifluralin, acifluorfen, acifluorfen-sodium, bifenox, chlomethoxynil, fomesafen, lactofen, oxadiazon, oxyfluorfen, carfentrazone, flumiclorac-pentyl, flumioxazine, fluthiacet-methyl, sulfentrazone, thidiazimin, difenzoquat, diquat, paraquat, 2,4-D, 2,4-DB, DCPA, MCPA, MCPB, clomeprop, clopyralid, dicamba, dithiopyr, fluroxypyr, mecoprop, naploanilide, phenothiol, quinclorac, triclopyr, acetochlor, alachlor, butachlor, diethatylethyl, metolachlor, pretilachlor, propachlor, bensulfuron-methyl, chlorsulfuron, chlorimuron-ethyl, halosulfuron-methyl, metsulfuron-methyl, nicosulfuron, primisulfuron, pyrazosulfuron-ethyl, sulfometuron-methyl, thifensulfuron-methyl, triasulfuron, tribenuron-methyl; azimsulfuron, cloransulam-methyl, cyclosulfamuron, flumeturam, flupyrsulfuron, flazasulfuron, imazosulfuron, metosulam, prosulfuron, rimsulfuron, triflusulfuron-methyl, imazamethabenz-methyl, imazapyr, imazaquin, imazethapyr, imazameth, imazamox, bispyribac-sodium, pyriminobac-methyl, pyrithiobac-sodium, alloxydimsodium, clethodim, sethoxydim, tralkoxydim, diclofop-methyl, fenoxaprop-ethyl, fenoxaprop-p-ethyl, fluazifop-butyl, fluazifop-p-butyl, haloxyfop-methyl, quizalofop-p-ethyl, cyhalofop-butyl, clodinafop-propargyl, benzofenap, clomazone, diflufenican, norflurazon, pyrazolate, pyrazoxyfen, isoxaflutole, sulcotrione, glufosinate-ammonium, glyphosate, bentazon, benthiocarb, bromobutide, butarnifos, butylate, dimepiperate, dimethenamid, DSMA, EPTC, esprocarb, isoxaben, mefenacet, molinate, MSMA, piperophos, pributycarb, propanil, pyridate, triallate, cafenstrol, flupoxam, and thiafluamide.

The following will describe typical examples of such a combination, where the present compounds are designated by their compound numbers shown in Tables 1 to 4.
1. A mixture of one compound selected from the group consisting of compounds 1-495, 1-496, 1-499, 1-503 and 1-577, and one compound selected from the group consisting of atrazin, cyanazine, bromoxynil and bentazon at a weight ratio of 1 : 1 to 100.
2. A mixture of one compound selected from the group consisting of compounds 1-495, 1-496, 1-499, 1-503 and 1-577, and one compound selected from the group consisting of clethodim, sethoxydim, dichlofop-methyl, quizalofop-p-ethyl, lactofen, acifluorfen, acifluorfen-sodium, fomesafen, flumiclorac-pentyl and dicamba at a weight ratio of 1 : 0.5 to 50.
3. A mixture of one compound selected from the group consisting of compounds 1-495, 1-496, 1-499, 1-503 and 1-577, and one compound selected from the group consisting of nicosulfuron, primisulfuron, prosulfuron, chlorimuran-ethyl, thifensulfuron, rimsulfuron, halosulfuron, oxasulfuron, isoxaflutole, imazethapyr and imazamox at a weight ratio of 1 : 0.1 1 to 10.
4. A mixture of one compound selected from the group consisting of compounds 1-439, 1-482, 1-486, 1-496, 1-1076, 1-1123 and 1-1441, and one compound selected from the group consisting of isoproturon and chlorotoluron at a weight ratio of 1 : 1 to 100.
5. A mixture of one compound selected from the group consisting of compounds 1-439, 1-482, 1-486, 1-496, 1-1076, 1-1123 and 1-1441, and one compound selected from the group consisting of mecoprop, fluroxypyr and ioxynil at a weight ratio of 1 : 0.5 to 50.
6. A mixture of one compound selected from the group consisting of compounds 1-439, 1-482, 1-486, 1-496, 1-1076, 1-1123 and 1-1441, and one compound selected from the group consisting of diflufenican, metsulfuron-methyl, fenoxaprop-ethyl and clodinafop-propargyl at a weight ratio of 1 : 0.1 to 10.
7. A mixture of one compound selected from the group consisting of compounds 1-1141, 1-1222 and 2-203, and one compound selected from the group consisting of glyphosate, glufosinate-ammonium and paraquat at a weight ratio of 1 : 1 to 100.

Moreover, the present compounds may also be used in admixture with insecticides, acaricides, nematocides, fungicides, plant growth regulators, fertilizers, soil improver, and the like.

When the present compounds are used as active ingredients of herbicides, the application amount is usually in the range of 0.01 to 10,000 g, preferably I to 8000 g, per hectare, although it may vary depending upon the weather conditions, formulation type, application timing, application method, soil conditions, crop plants, unfavorable weeds, and the like. In the case of emulsifiable concentrates, wettable powders, flowables, concentrated emulsions, water-dispersible granules, or the like, the formulation is usually applied at a prescribed amount after diluted with water having a volume of about 10 to 1000 liters per hectare, if necessary, with the addition of an adjuvant such as a spreading agent. In the case of granules or some types of flowables, the formulation is usually applied as such without any dilution.

Examples of the adjuvant used, if necessary, may include, in addition to the surfactants recited above, polyoxyethylene resin acids (esters), ligninsulfonates, abietates, dinaphtylmethanedisulfonates, crop oil concentrates, and vegetable oils such as soybean oil, corn oil, cotton seed oil, and sunflower oil.

The present compounds can also be used as active ingredients of harvesting aids such as defoliants and desiccating agents for cotton, and desiccating agents for potato. In these cases, the present compounds are usually formulated in the same manner as the case where they are used as active ingredients of herbicides, and used alone or in combination with other harvesting aids for foliar treatment before the harvesting of crops.

The present invention will be further illustrated by the following production examples, reference examples, formulation examples, and test examples; however, the present invention is not limited to these examples.

The following will describe production examples for the present compounds and the hydrazones of formula [2] as the intermediate compounds, where the present compounds are designated by their compound numbers shown in Tables 1 to 4.

### Production Example 1 (Production of Compound 2-631)

To a mixed solution of 8.0 g (97.2 mmol) of sodium acetate and 50 ml of water was added under ice cooling 6.6 g (24.3 mmol) of 1,1-dibromo-3,3,3-trifluoroacetone, and the reaction was allowed to proceed at 80°C for 30 minutes. Then, the reaction mixture was cooled to 0°C, to which 4.4 g (18.7 mmol) of 7-fluoro-6-hydrazino-4-propargyl-2H-1,4-benzoxazin-3-one was added, and the reaction mixture was stirred at room temperature for 2 hours. The precipitated crystals were collected by filtration, washed twice with 10 ml of water, and dried, which afforded 6.3 g (18.37 mmol) of 7-fluoro-6-trifluoroacetylmethylidenhydrazino-4-propargyl-2H-1,4-benzoxazin-3-one [another name: 3,3,3-trifluoro-2-oxopropanal 1-(7-fluoro-3-oxo-4-propargyl-2H-1,4-benzoxazin-6-ylhydrazone)], m.p. 190.6°C (decomp.).

To a mixed solution of 6.0 g (17.5 mmol) of the above compound and 50 ml of toluene was added 9.1 g (26.2 mmol) of carbethoxymethylenetriphenylphosphorane, and the mixture was heated under reflux for 1 hour. The toluene was distilled out under reduced pressure, and the residue was subjected to silica gel column chromatography, which afforded 1.3 g (3.5 mmol) of 7-fluoro-6-[5-trifluoromethyl-3-pyridazinon-2-yl]-4-propargyl-2H-1,4-benzoxazin-3-one (compound 2-631).

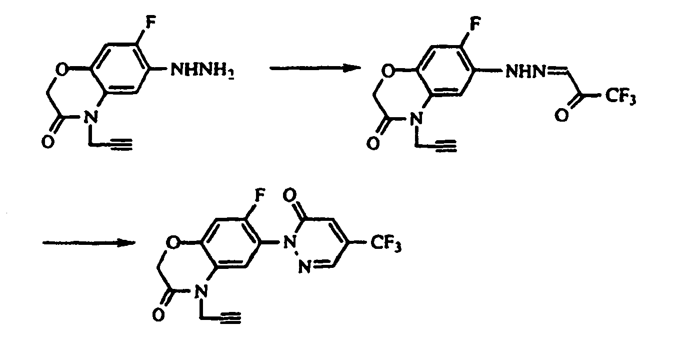

### Production Example 2 (Production of Compound 1-476)

To a mixed solution of 5.3 g (53.5 mmol) of sodium acetate and about 100 ml of water was added under ice cooling 6.6 g (24.3 mmol) of 1,1-dibromo-3,3,3-trifluoroacetone, and the reaction was allowed to proceed at 70°C for 20 minutes. Then, the reaction mixture was cooled to room temperature, to which a solution of 5.8 g (21.5 mmol) of 2-fluoro-4-chloro-5-isopropoxyphenylhydrazine dissolved in about 20 ml of diethyl ether was added, and the reaction mixture was stirred at room temperature for 1 hour. The ether layer was separated and concentrated. Then, about 60 ml of THF was added to the residue, to which 8.3 g (23.0 mmol) of carbethoxyethylidenetriphenylphosphorane was added, and the mixture was heated under reflux for 2 hours. The toluene was distilled out under reduced pressure, and the residue was subjected to silica gel column chromatography, which afforded 3.8 g (10.5 mmol) of 2-[2-fluoro-4-chloro-5-isopropoxyphenyl]-4-methyl-5-trifluoromethylpyridazin-3-one (compound 1-476).

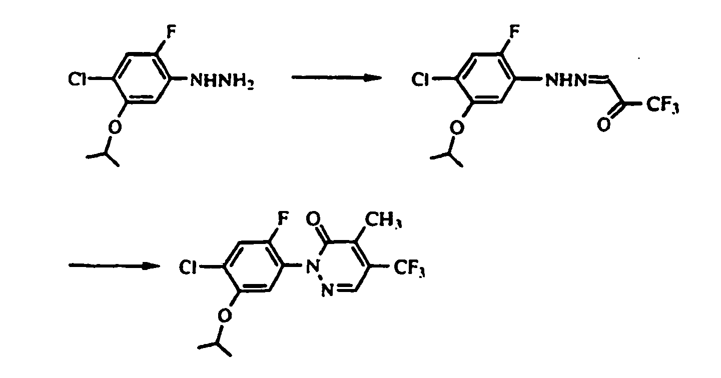

### Production Example 3 (Production of Compound 1-391)

First, 3.5 g (9.7 mmol) of 2-[2-fluoro-4-chloro-5-isopropoxyphenyl]-4-methyl-5-trifluoromethylpyridazin-3-one (compound 1-476) was dissolved in about 10 ml of concentrated sulfuric acid under ice cooling, and the solution was warmed to room temperature. After 10 minutes, about 100 ml of water was added to the reaction mixture, and the precipitated crystals were collected by filtration, and washed twice with 20 ml of water and once with 10 ml of hexane. These crystals were recrystallized from isopropanol, which afforded 3.2 g (9.0 mmol) of 2-[2-fluoro-4-chloro-5-hydroxyphenyl]-4-methyl-5-trifluoromethylpyridazin-3-one (compound 1-391).

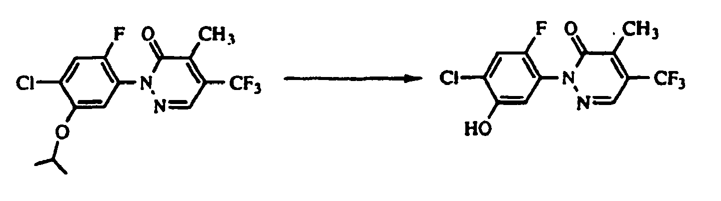

### Production Example 4 (Production of Compound 1-486)

First, 3.2 g (10 mmol) of compound 1-391 was dissolved in about 50 ml of DMF, to which 2.0 g (13 mmol) of potassium carbonate was added at room temperature and 1.3 g (1mmol) of propargyl bromide was then added, and the mixture was stirred at room temperature for 30 minutes, followed by the addition of 100 ml of water. The precipitated crystals were collected by filtration, washed with hexane, and recrystallized from isopropanol, which afforded 3.4 g (9 mmol) of compound 1-486.

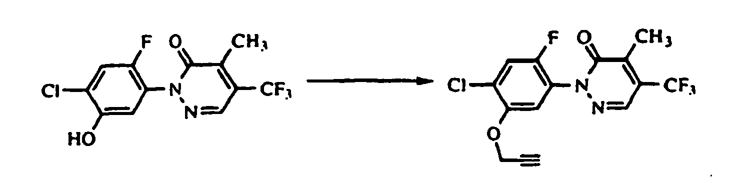

### Production Example 5 (Production of Compound 1-496)

First, 3.2 g (10 mmol) of compound 1-391 was dissolved in about 50 ml of DMF, to which 0.44 g (11 mmol) of sodium hydride (60 wt.%, oil dispersion) was added, and the mixture was allowed to stand at room temperature for 30 minutes, followed by the addition of 1.8 g (11 mmol) of ethyl bromoacetate under ice cooling. After stirring at room temperature for 1 hour, the reaction mixture was extracted with diethyl ether. The organic layer was washed with 10% aqueous HCl, aqueous sodium bicarbonate solution and then with saturated sodium chloride solution, and dried with anhydrous magnesium sulfate. The solvent was distilled out under reduced pressure, and the residue was subjected to silica gel column chromatography, which afforded 2.4 g (5.5 mmol) of compound 1-496.

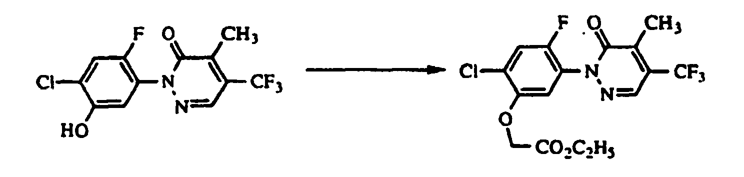

### Production Example 6 (Production of Compound 2-251)

To a mixed solution of 5.3 g (53.5 mmol) of sodium acetate and about 50 ml of water was added under ice cooling 6.6 g (24.3 mmol) of 1,1-dibromo-3,3,3-trifluoroacetone, and the reaction was allowed to proceed at 80°C for 1 hour. Then, the reaction mixture was cooled to 0°C, to which 4.4 g (18.7 mmol) of 7-fluoro-6-hydrazino-4-propargyl-2H-1,4-benzoxazin-3-one was added, and the reaction mixture was stirred at room temperature for 2 hours. The precipitated crystals were collected by filtration, washed twice with 10 ml of water and once with 10 ml of hexane, and then dissolved in 50 ml of toluene without drying. To this solution was added 8.8 g (24.3 mmol) of carbethoxyethylidenetriphenylphosphorane, and the mixture was heated under reflux for I hour, while conducting azeotropic dehydration. The toluene was distilled out under reduced pressure, and the residue was subjected to silica gel column chromatography, which afforded 3.5 g (9.01 mmol) of compound 2-251.

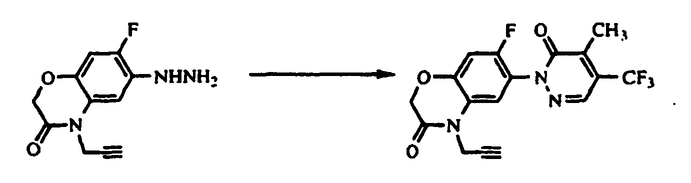

### Production Example 7 (Production of Compound 2-328)

To a mixed solution of 5.3 g (53.5 mmol) of sodium acetate and about 50 ml of water was added under ice cooling 6.6 g (24.3 mmol) of 1,1-dibromo-3,3,3-trifluoroacetone, and the reaction was allowed to proceed at 80°C for I hour. Then, the reaction mixture was cooled to 0°C, to which 4.8 g (18.7 mmol) of 6-fluoro-5-hydrazino-3-(sec-butyl)-1,3-benzothiazol-2-one was added, and the reaction mixture was stirred at room temperature for 2 hours. Then, 100 ml of ether was added to the reaction mixture, followed by stirring and phase separation, and the organic layer was concentrated. The residue was dissolved in 50 ml of THF, to which 8.8 g (24.3 mmol) of carbethoxyethylidenetriphenylphosphorane was added, and the mixture was heated under reflux for 1 hour. The THF was distilled out under reduced pressure, and the residue was subjected to silica gel column chromatography, which afforded 3.7 g (9.6 mmol) of compound 2-328.

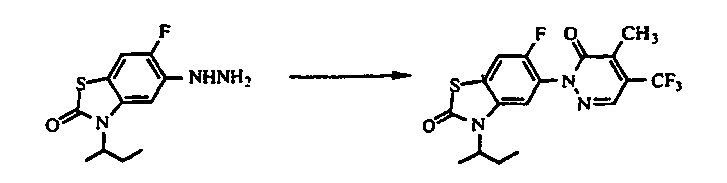

### Production Example 8 (Production of Compound 1-347)

First, 50 ml of concentrated sulfuric acid was ice cooled, in which 7.0 g (22.8 mmol) of compound 1-341 was dissolved. Then, 1.51 g (24 mmol) of fuming nitric acid was added dropwise at 5°C or lower, followed by maturation at 0° to 5°C for 1 hour. The mixture was poured into 300 ml of ice-water and extracted three times with 50 ml of ether. The combined ether layer was washed with about 100 ml of water and neutralized with 100 ml of aqueous sodium bicarbonate solution, followed by phase separation. The organic layer was dried with magnesium sulfate and concentrated to half volume, and the residue was subjected to silica gel column chromatography, which afforded 6.1 g (17.4 mmol) of compound 1-347.

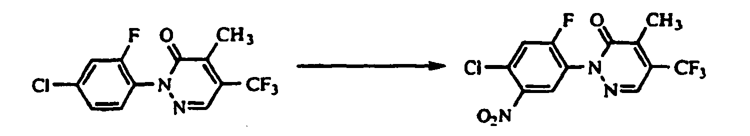

### Production Example 9 (Production of Compound 1-353)

First, 5.0 g of iron powder, 75 ml of acetic acid, and 10 ml of water were mixed, and the mixture was warmed to about 80°C, followed by maturation for about 15 minutes. Then, 6.0 g (17.1 mmol) of compound 1-347 was dissolved in 40 ml of ethyl acetate, which was added dropwise to the above mixture at 80°C or lower. After maturation at about 80°C for 1 hour, the reaction mixture was allowed to stand for cooling to room temperature, and extracted twice with 100 ml of ethyl acetate. The combined ethyl acetate layer was washed twice with 50 ml of water and neutralized with aqueous sodium bicarbonate solution, followed by phase separation. The organic layer was dried with magnesium sulfate, and the ethyl acetate was distilled out under reduced pressure. The residue was subjected to silica gel chromatography, which afforded 5.1 g (15.9 mmol) of compound 1-353.

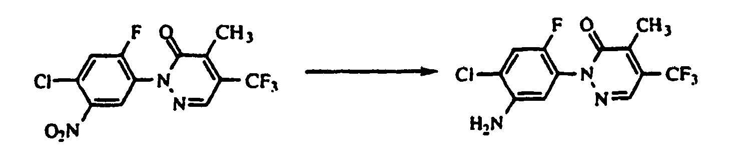

### Production Example 10 (Production of Compound 1-420)

First, 500 mg (1.6 mmol) of compound 1-353 was mixed with 10 ml (77.3 mmol) of ethyl 2-bromopropionate, and the mixture was heated under reflux at about 160°C for about 12 hours. After allowing to stand for cooling, the reaction mixture was subjected to silica gel column chromatography, which afforded 60 mg (0.6 mmol) of compound 1-420.

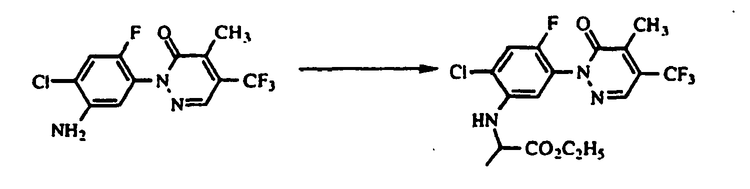

### Production Example 11 (Production of Compound 1-1622)

First, 6 ml (55.4 mmol) of ethyl acrylate, 0.5 g (4.8 mmol) of t-butyl nitrite, and 0.6 g (4.5 mmol) of copper (II) chloride were mixed together in 5 ml of acetonitrile, followed by ice cooling. Then, 1.0 g (3.1 mmol) of compound 1-353 dissolved in 5 ml of acetonitrile was added dropwise at 5°C or lower, followed by overnight maturation at room temperature. The reaction mixture was poured into ice-water and extracted twice with 100 ml of ethyl acetate. The combined ethyl acetate layer was washed with 50 ml of diluted hydrochloric acid and dried with magnesium sulfate. The solvent was distilled out under reduced pressure, and the residue was subjected to silica gel column chromatography, which afforded 0.51 g (1.2 mmol) of compound 1-1622.

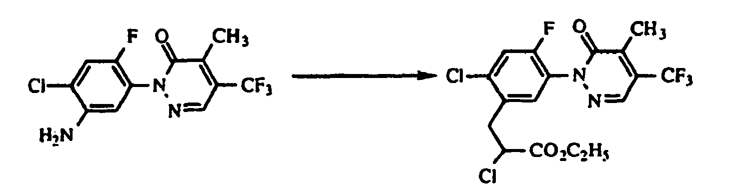

### Production Example 12 (Production of Compound 1-1221)

To a mixed solution of 5.3 g (53.5 mmol) of sodium acetate and about 50 ml of water was added under ice cooling 6.6 g (24.3 mmol) of 1,1-dibromo-3,3,3-trifluoroacetone, and the reaction was allowed to proceed at 80°C for 1 hour. Then, the reaction mixture was cooled to 0°C, to which 5.2 g (18.7 mmol) of methyl 2-(2-chloro-4-fluoro-5-hydrazinophenylthio)propionate was added, and the reaction mixture was stirred at room temperature for 2 hours. The precipitated crystals were collected by filtration, washed twice with 10 ml of water and once with 10 ml of hexane, and dried. The residue was dissolved in 50 ml of THF, to which 8.4 g (22.4 mmol) of carbethoxymethylenetriphenylphosphorane was added, and the solution was stirred at room temperature for 3 hours. The THF was distilled out under reduced pressure, and the residue was subjected to silica gel column chromatography, which afforded 3.8 g (9.0 mmol) of compound 1-1221.

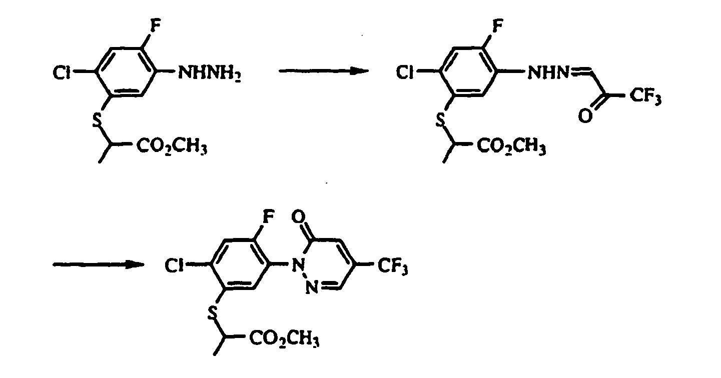

### Production Example 13 (Production of Compound 2-821)

To a mixed solution of 8.0 g (97.2 mmol) of sodium acetate and about 50 ml of water was added under ice cooling 6.9 g (24.3 mmol) of 3,3-dibromo-1,1,1-trifluorobutanone, and the reaction was allowed to proceed at 80°C for 30 minutes. Then, the reaction mixture was cooled to 0°C, to which 4.4 g (18.7 mmol) of 7-fluoro-6-hydrazino-4-propargyl-2H-1,4-benzoxazin-3-one was added, and the reaction mixture was stirred at room temperature for 2 hours. The precipitated crystals were collected by filtration, washed twice with 10 ml of water, and dried, which afforded 6.1 g (17.0 mmol) of 1,1,1-trifluoro-2,3-butandione 3-(7-fluoro-3-oxo-4-propargyl-2H-1,4-benzoxazin-6-ylhydrazone).

To a mixed solution of 6.1 g (17.0 mmol) of the above compound and 50 ml of THF was added 7.1 g (20.4 mmol) of carbethoxymethylenetriphenylphosphorane, and the mixture was heated under reflux for 1 hour. The THF was distilled out under reduced pressure, and the residue was subjected to silica gel column chromatography, which afforded 0.61 g (1.6 mmol) of 7-fluoro-6-(6-methyl-5-trifluoromethyl-3-pyridazinon-2-yl)-4-propargyl-2H-1,4-benzoxazin-3-one (compound 2-821).

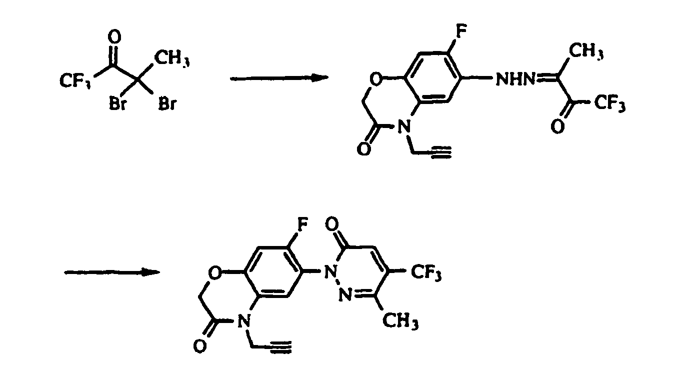

### Reference Example 1

This is a production example for 3,3-dibromo-1,1,1-trifluoro-2-butanone used in Production Example 13.

First, 34.0 g of sodium acetate was dissolved in 270 ml of acetic acid, to which 25 g (0.20 mol) of 1,1,1-trifluoro-2-butanone was added, and while keeping the temperature at 15° to 20°C, 66.3 g (0.42 mol) of bromine was added dropwise over 45 minutes. The reaction mixture was stirred for 5 hours, while keeping the temperature at 15° to 20°C, and then allowed to stand at room temperature for 68 hours. The supernatant was taken and washed with 600 ml of concentrated sulfuric acid. Further washing with 307 ml of concentrated sulfuric acid and distillation under normal pressure gave 28 g (0.10 mol) of 3,3-dibromo-1,1,1-trifluoro-2-butanone.

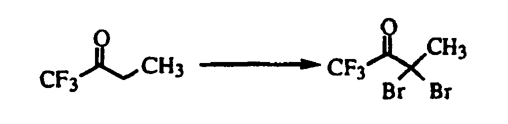

### Production Example 14 (Production of Compound 1-1346)

To a mixed solution of 5.3 g (53.5 mmol) of sodium acetate and about 50 m1 of water was added under ice cooling 6.9 g (24.3 mmol) of 3,3-dibromo-1,1,1-trifluoro-2-butanone, and the reaction was allowed to proceed at 80°C for 1 hour. Then, the reaction mixture was cooled to 0°C, to which 3.3 g (18.7 mmol) of 2-chloro-4-fluoro-5-hydrazinophenol was added, and the reaction mixture was stirred at room temperature for 2 hours. The precipitated crystals were collected by filtration, washed twice with 10 ml of water and once with 10 ml of hexane, dried, and then dissolved in 50 ml of THF. To this solution was added 8.8 g (24.3 mmol) of carbethoxymethylenetriphenylphosphorane, and the mixture was stirred at room temperature for 3 hours. The THF was distilled out under reduced pressure, and the residue was subjected to silica gel chromatography, which afforded 0.51 g (1.6 mmol) of compound 1-1346.

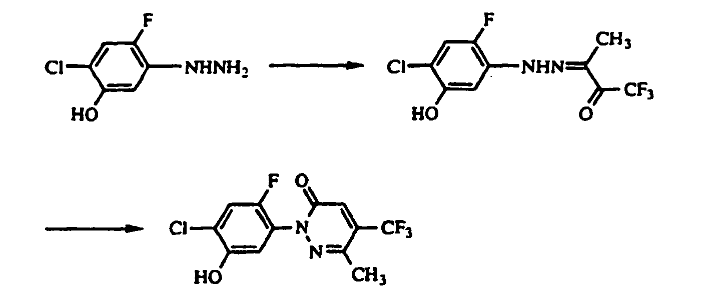

### Production Example 15 (Production of Compound 1-1441)

First, 3.2 g (10mmol) of compound 1-1346 was dissolved in about 50 ml of DMF, to which 2.0 g (13 mmol) of potassium carbonate was added at room temperature and 1.3 g (11 mmol) of propargyl bromide was then added, and the mixture was stirred at room temperature for 30 minutes, followed by the addition of 100 ml of water. The precipitated crystals were collected by filtration, washed with hexane, and recrystallized from isopropanol, which afforded 3.2 g (8.5 mmol) of compound 1-1441.

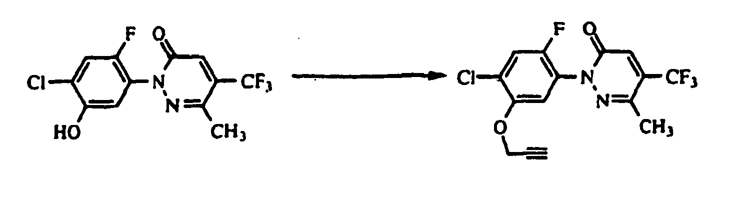

### Production Example 16 (Production of Compound 1-499)

This example followed the procedures of Production Example 5, except that 1.8 g (1.1 mmol) of n-pentyl chloroacetate was used in place of ethyl bromoacetate. After the addition of this compound, the reaction mixture was stirred at 40°C for 3 hours and then extracted with diethyl ether. The organic layer was washed with 10% HCl, aqueous sodium bicarbonate solution and saturated sodium chloride solution, and dried with anhydrous magnesium sulfate. The solvent was distilled out under reduced pressure, and the residue was subjected to silica gel chromatography, which afforded 3.8 g (8.0 mmol) of compound 1-499.

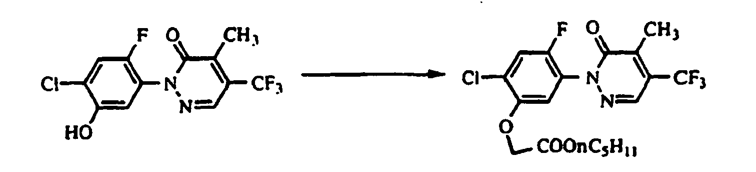

### Production Example 17 (Production of Compound 2-203)

To a mixed solution of 5.3 g (53.5 mmol) of sodium acetate and about 50 ml of water was added under ice cooling 6.6 g (24.3 mmol) of 1,1-dibromo-3,3,3-trifluoroacetone, and the reaction was allowed to proceed at 80°C for 1 hour. Then, the reaction mixture was cooled to 0°C, to which 4.0 g (18.7 mmol) of 6-hydrazino-4-propargyl-2H-1,4-benzoxazin-3-one was added, and the reaction mixture was stirred at room temperature for 2 hours. The precipitated crystals were collected by filtration, washed twice with 10 ml of water and once with 10 ml of hexane, and then dissolved in 50 ml of THF without drying. To this solution was added 8.8 g (24.3 mmol) of carbethoxyethylidenetriphenylphosphorane, and the mixture was heated under reflux for 3 hours. The THF was distilled out under reduced pressure, and the residue was subjected to silica gel column chromatography, which afforded 3.3 g (8.8 mmol) of compound 2-203.

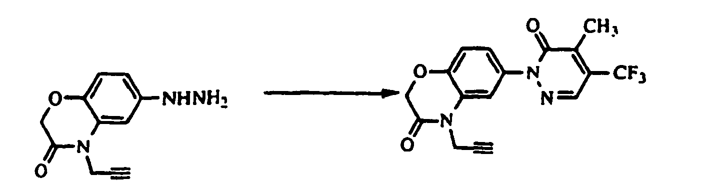

### Production Example 18 (Production of Compound 1-1222)

To a mixed solution of 5.3 g (53.5 mmol) of sodium acetate and about 50 ml of water was added under ice cooling 6.6 g (24.3 mmol) of 1,1-dibromo-3,3,3-trifluoroacetone, and the reaction was allowed to proceed at 80°C for 1 hour. Then, the reaction mixture was cooled to 0°C, to which 5.5 g (18.7 mmol) of ethyl 2-(2-chloro-4-fluoro-5-hydrazinophenylthio)propionate was added, and the reaction mixture was stirred at room temperature for 2 hours. The precipitated crystals were collected by filtration, washed twice with 10 ml of water and once with 10 ml of hexane, dried, and then dissolved in 50 ml of THF. To this solution was added 8.4 g (22.4 mmol) of carbethoxymethylenetriphenylphosphorane, and the mixture was stirred at room temperature for 3 hours. The THF was distilled out under reduced pressure, and the residue was subjected to silica gel column chromatography, which afforded 4.3 g (9.9 mmol) of compound 1-1222.

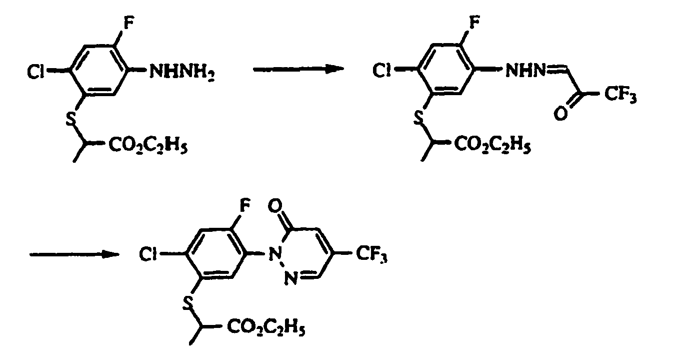

### Production Example 19 (Production of Compound 1-476)

To a mixed solution of 5.3 g (53.5 mmol) of sodium acetate and about 100 ml of water was added under ice cooling 6.6 g (24.3 mmol) of 1,1-dibromo-3,3,3-trifluoroacetone, and the reaction was allowed to proceed at 70°C for 20 minutes. Then, the reaction mixture was cooled to room temperature, to which a solution of 5.8 g (21.5 mmol) of 2-fluoro-4-chloro-5-isopropoxyphenylhydrazine dissolved in about 20 ml of diethyl ether was added, and the reaction mixture was stirred at room temperature for 1 hour. The ether layer was separated, washed once with 10 ml of saturated sodium chloride solution, and dried with magnesium sulfate. The diethyl ether was distilled out, which afforded 6.5 g (20.0 mmol) of 3,3,3-trifluoro-2-oxo-propanal 1-(4-chloro-2-fluoro-5-isopropoxyphenylhydrazone).

¹H-NMR (250 MHz, CDCl₃, TMS 8 (ppm)) 1.39 (6H, d, J. = 6.0 Hz), 4.38-4.52 (1H, m), 7.15 (1H, d, J = 10.5 Hz), 7.22 (1H, d, J = 7.3 Hz), 7.43 (1H, q, J = 1.7 Hz), 9.18 (1H, br).

This compound was dissolved in 50 ml of THF. To this solution was added 8.3 g (23.0 mmol) of carbethoxyethylidenetriphenylphosphorane, and the mixture was heated under reflux for 2 hours. The THF was distilled out under reduced pressure, and the residue was subjected to silica gel column chromatography, which afforded 3.8 g (10.5 mmol) of 2-[2-fluoro-4-chloro-5-isopropoxyphenyl]-4-methyl-5-trifluoromethylpyridazin-3-one (compound 1-476).

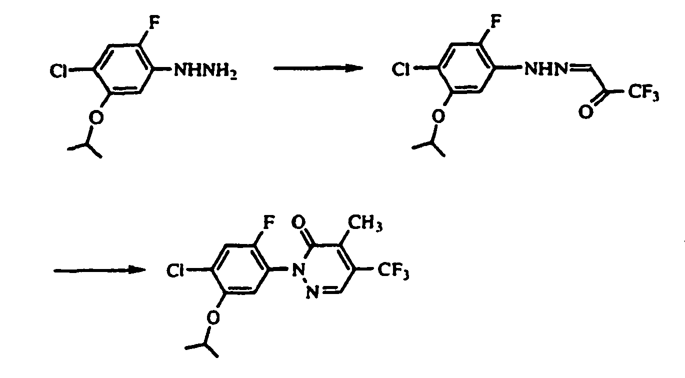

### Production Example 20 (Production of Compound 1-642)

To a mixed aqueous solution of 5.3 g (53.5 mmol) of sodium acetate and about 100 ml of water was added under ice cooling 6.6 g (24.3 mmol) of 1,1-dibromo-3,3,3-trifluoroacetone, and the reaction was allowed to proceed at 70°C for 20 minutes. Then, the reaction mixture was cooled to room temperature. Separately, 5.8 g (21.5 mmol) of crude ethyl 2-chloro-4-fluoro-5-hydrazinobenzoate hydrochloride was dissolved in 30 ml of water, to which 100 ml of diethyl ether was added, and while cooling, the mixture was neutralized by the addition of saturated sodium hydrogencarbonate solution, followed by washing with saturated sodium chloride solution, which afforded a solution of ethyl 2-chloro-4-fluoro-5-hydrazinobenzoate in diethyl ether. This solution was added to the above reaction mixture, followed by vigorous stirring at room temperature for 2 hours. The ether layer was separated, washed once with 10 ml of saturated sodium chloride solution, and dried with magnesium sulfate. The diethyl ether was distilled out. The residue was dissolved in 50 ml of THF, to which 8.3 g (23.0 mmol) of carbethoxyethylidenetriphenylphosphorane was added, and the mixture was heated under reflux for 2 hours. The THF was distilled out under reduced pressure, and the residue was subjected to silica gel column chromatography, which afforded 3.8 g (10.0 mmol) of compound 1-642.

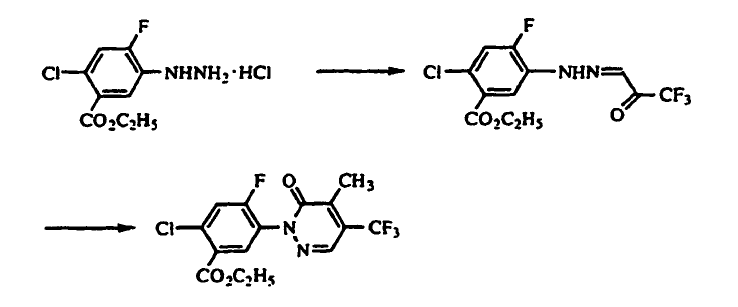

### Reference Example 2

This is a production example for ethyl 2-chloro-4-fluoro-5-hydrazinobenzoate hydrochloride used in Production Example 20.

First, 50 g (0.29 mol) of 2-chloro-4-fluorobenzoic acid was dissolved in 150 ml of hydrochloric acid at room temperature, to which a mixed acid of 28 ml (0.31 mol) of fuming nitric acid and 56 ml of concentrated sulfuric acid was added dropwise at 35° to 45°C. Then, the solution was stirred at 40°C for 1 hour and poured into 250 ml of ice-water. The precipitated crystals were collected by filtration and recrystallized from a mixed solution of hexane and ethyl acetate, which afforded 55 g (0.25 mol) of 2-chloro-4-fluoro-5-nitrobenzoic acid. Then, 55 g (0.25 mol) of 2-chloro-4-fluoro-5-nitrobenzoic acid was dissolved in 50 ml of ethyl acetate, to which 33 g (0.28 mol) of thionyl chloride was added, and the mixture was heated under reflux for 3 hours and then allowed to stand for cooling to room temperature. Then, 20 ml of ethanol and 30 g of triethylamine were added under ice cooling, and the mixture was stirred at room temperature for 2 hours. The solvent was distilled out, and the residue was purified by silica gel chromatography, which afforded 57 g (0.23 mol) of ethyl 2-chloro-4-fluoro-5-nitrobenzoate.

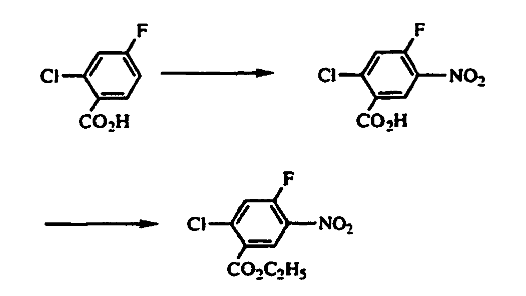

Then, 60 g of iron powder and 500 ml of 10% acetic acid were mixed, and the mixture was heated to 40°C. Separately, 50 g (0.20 mol) of ethyl 2-chloro-4-fluoro-5-nitrobenzoate was dissolved in a mixed solution of 20 ml of acetic acid and 20 ml of ethyl acetate, and added dropwise to the above iron powder-acetic acid mixed solution. Then, the reaction mixture was stirred at 50°C for 1 hour and filtered though celite. The filtrate was extracted with 100 ml of ethyl acetate. The ethyl acetate layer was washed with aqueous sodium bicarbonate solution and saturated sodium chloride solution, and dried with magnesium sulfate. The solvent was distilled out, and the residue was purified by silica gel chromatography, which afforded to 40 g (0.18 mol) of ethyl 5-amino-2-chloro-4-fluorobenzoate.

Then, 19 g (87.4 mmol) of ethyl 5-amino-2-chloro-4-fluorobenzoate was dissolved in 120 ml of hydrochloric acid, followed by cooling to 0°C, to which a solution of 6.3 g (91.7 mmol) of sodium nitrite dissolved in 10 ml of water was added dropwise at 10°C or lower. The mixture was stirred at 0°C for 30 minutes and then cooled to -30°C, into which a solution of 58 g (0.31 mol) of anhydrous tin (II) chloride dissolved in 40 ml of hydrochloric acid was poured, followed by further stirring at 0°C for 3 hours. The precipitated crystals were collected by filtration and then dried, which afforded 13.6 g (50.7 mmol) of crude ethyl 2-chloro-4-fluoro-5-hydrazinobenzoate hydrochloride.

### Production Example 21 (Production of Compound 1-1789)

First, 5.0 g (15.5 mmol) of compound 1-391 was dissolved in about 50 ml of DMF, to which 2.8 g (20.2 mmol) of potassium carbonate was added at room temperature and then 1.5 g (17.1 mmol) of 3-bromo-2-methyl-1-propene was added, and the mixture was stirred at room temperature for 30 minutes, followed by the addition of 100 ml of water. The precipitated crystals were collected by filtration, washed with hexane, and recrystallized from isopropanol, which afforded 4.4 g (13.2 mmol) of compound 1-1789.

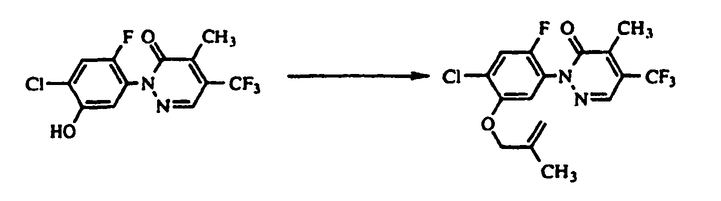

### Production Example 22 (Production of Compound 4-451)

First, 4.0 g (12.0 mmol) of compound 1-1789 was dissolved in 20 ml of N,N-dimethylaniline, and the solution was heated at 180°C for 3 hours. After cooling to room temperature, 100 ml of ethyl acetate was added, and the mixture was washed with IN aqueous hydrochloric acid and saturated sodium chloride solution, and dried with magnesium sulfate. The solvent was distilled out, and the precipitated crystals were recrystallized from isopropanol, which afforded 3.4 g (10.2 mmol) of 2-[4-chloro-6-fluoro-3-hydroxy-2-(2-methyl-2-propenyl)]-4-methyl-5-trifluoromethylpyridazin-3-one, m.p. 133.2°C.

The product was dissolved in 30 ml of xylene, to which a catalytic amount of p-toluenesulfonic acid was added, and the mixture was heated under reflux for I hour. After cooling to room temperature, 100 ml of ethyl acetate was added, and the mixture was washed with aqueous sodium bicarbonate solution and sodium chloride solution, and dried with magnesium sulfate. The solvent was distilled out, and the residue was purified by silica gel column chromatography, which afforded 3.0 g (9.0 mmol) of compound 4-451.

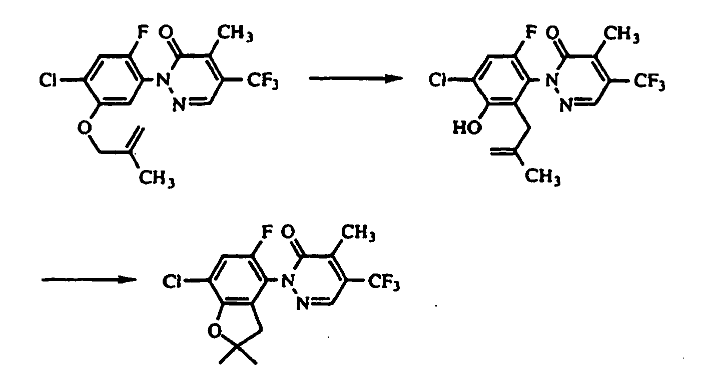

### Production Example 23 (Production of Compound 1-483)

First, 5.0 g (15.5 mmol) of compound 1-391 was dissolved in about 20 ml of DMF, to which 2.4 g (17.1 mmol) of potassium carbonate was added at room temperature. The solution was heated to about 40°C, to which 1.7 g (17.1 mmol) of 2,3-dichloropropene was added, and after 1 hour, the mixture was allowed to stand for cooling and poured into ice-water. The precipitated crystals were collected by filtration, washed with hexane, and recrystallized from isopropanol, which afforded 5.2 g (13.1 mmol) of compound 1-483.

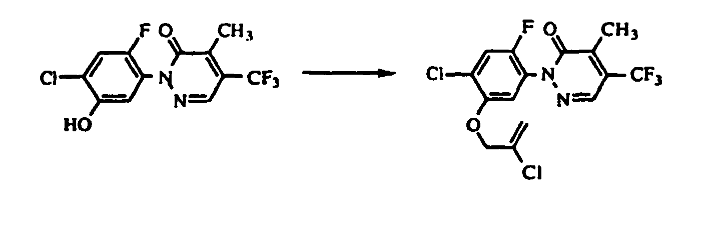

### Production Example 24 (Production of Compound 3-139)

First, 3.0 g (7.6 mmol) of compound 1-436 was dissolved in 10 ml of N,N-dimethylaniline, and the solution was heated under reflux for 3 hours. After cooling to room temperature, 50 ml of ethyl acetate was added, and the mixture was washed with I N aqueous hydrochloric acid and saturated sodium chloride solution, and dried with magnesium sulfate. The solvent was distilled out, and the precipitated crystals were recrystallized from isopropanol, which afforded 2.2 g (5.6 mmol) of 2-[4-chloro-6-fluoro-3-hydroxy-2-(2-chloro-2-propenyl)]-4-methyl-5-trifluoromethylpyridazin-3-one.

¹H-NMR (300 MHz, CDCl₃, TMS δ (ppm)) 2.41 (3H, q, J = 1.9 Hz), 3.56 (1H, d, J = 16.3 Hz), 3.72 (1H, d, J = 16.3 Hz), 4.91 (1H, q, J = 1.4 Hz), 5.12 (1H, d. J = 1.5 Hz), 5.72 (1H, s), 7.25 (1H, d, J = 8.7 Hz), 8.00 (1H, s)

The product was dissolved in 10 ml of trifluoromethanesulfonic acid cooled by ice, and the solution was stirred under ice cooling. After 30 minutes, the solution was poured into ice-water, and the precipitated crystals were collected by filtration and subjected to silica gel column chromatography, which afforded 1.9 g (5.4 mmol) of compound 3-139.

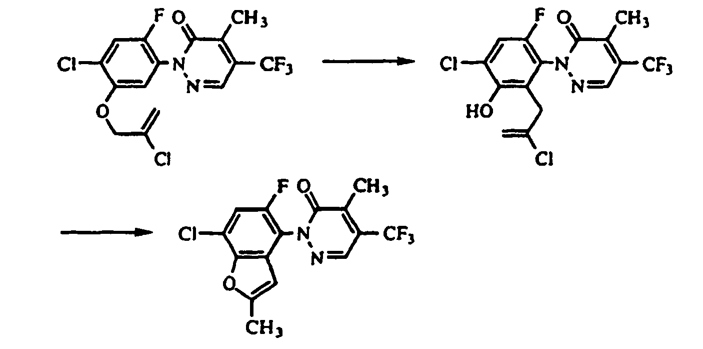

### Production Example 25 (Production of Compound 1-1744)

This example followed the procedures of Production Example 20, except that 6.1 g (21.5 mmol) of ethyl 2,4-dichloro-5-hydrazinobenzoate hydrochloride was used in place of ethyl 2-chloro-4-fluoro-5-hydrazinobenzoate hydrochloride, which afforded 4.8 g (12.2 mmol) of compound 1- 1744.

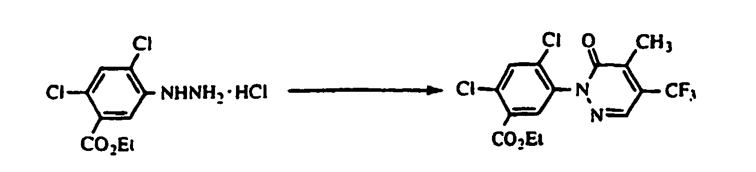

The ethyl 2,4-dichloro-5-hydrazinobenzoate hydrochloride used above was produced from 2,4-dichlorobenzoic acid by the same process as shown in Reference Example 2.

### Production Example 26 (Production of Compound 1-1279)

To a mixed aqueous solution of 5.3 g (53.5 mmol) of sodium acetate and about 100 ml of water was added under ice cooling 6.6 g (24.3 mmol) of 1,1-dibromo-3,3,3-trifluoroacetone, and the reaction was allowed to proceed at 70°C for 20 minutes. Then, the reaction mixture was cooled to room temperature. Separately, 5.8 g (21.5 mmol) of ethyl 2-chloro-4-fluoro-5-hydrazinobenzoate hydrochloride was dissolved in 30 ml of water, to which 100 ml of diethyl ether was added, and while cooling, the mixture was neutralized by the addition of saturated sodium hydrogencarbonate solution, followed by washing with saturated sodium chloride solution, which afforded a solution of ethyl 2-chloro-4-fluoro-5-hydrazinobenzoate in diethyl ether. This solution was added to the above reaction mixture, followed by vigorous stirring at room temperature for 2 hours. The ether layer was separated, washed once with 10 ml of saturated sodium chloride solution, and dried with magnesium sulfate. The diethyl ether was distilled out, and a small amount of hexane was added to give 4.3 g (12.6 mmol) of ethyl 2-chloro-4-fluoro-5-(2-oxo-3,3,3-trifluoropentylidenehydrazino)benzoate. This product was dissolved in 50 ml of THF, to which 5.0 g (14.4 mmol) of carbethoxymethylenetriphenylphosphorane was added, and the mixture was heated under reflux for 2 hours. The THF was distilled out under reduced pressure, and the residue was subjected to silica gel column chromatography, which afforded 3.6 g (9.7 mmol) of compound 1-1279.

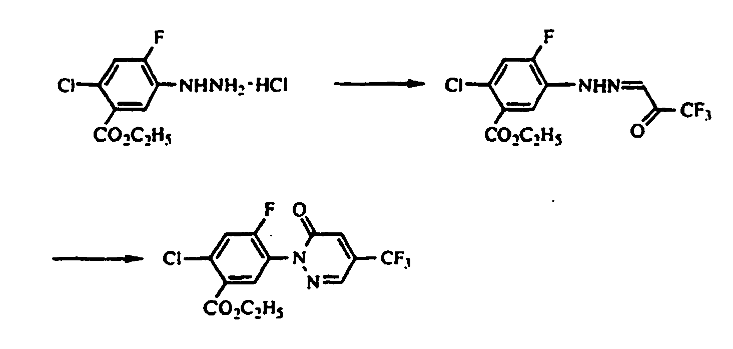

### Production Example 27 (Production of Compound 1-1780)

First, 50 g (0.61 mol) of sodium acetate and 41 g (0.14 mol) of 1,1-dibromo-3,3,3-trifluoroacetone were mixed with 500 ml of water, and the mixture was stirred at 80°C for 30 minutes and then cooled to 0°C. Then, 45 g (0.14 mol) of 4-bromo-2-fluoro-5-isopropoxyphenylhydrazine hydrochloride was added at 10°C or lower, and the mixture was stirred at 10°C or lower for 3 hours. The precipitated crystals were collected by filtration and dried, which afforded 35 g (94.3 mmol) of 3,3,3-trifluoro-2-oxopropanal 1-(4-bromo-2-fluoro-5-isopropoxyphenylhydrazone).

Then, 16 g (46.0 mmol) of carbethoxymethylenetriphenylphosphorane and 16 g (43.1 mmol) of 3.3,3-trifluoro-2-oxopropanal 1-(4-bromo-2-fluoro-5-isopropyloxyphenylhydrazone) were stirred in 100 ml of THF at room temperature for 4 hours. After completion of the reaction, the reaction mixture was concentrated, and the residue was subjected to silica gel chromatography, which afforded 9.4 g (23.8 mmol) of compound 1-1780.

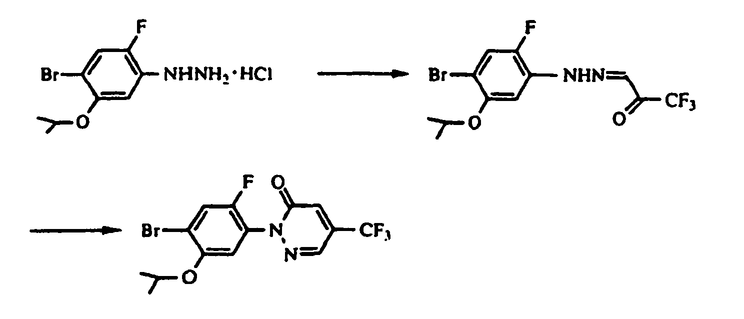

### Reference Example 3

This is a production example for 4-bromo-2-fluoro-5-isopropoxyphenylhydrazine hydrochloride used in Production Example 27.

First, 93 g (0.49 mol) of 2-bromo-4-fluorophenol was suspended in 200 ml of water, into which 55 g (0.59 mol) of methyl chloroformate and a solution of 21.5 g (0.51 mol) of sodium hydroxide in 60 ml of water were poured together at 10°C or lower, and the mixture was stirred at the same temperature for 2 hours. The precipitated crystals were collected by filtration, washed with water, and dried in a vacuum oven, which afforded 111.6 g (0.45 mol) of methyl 2-bromo-4-fluorophenoxyformate.

Then, 110 g (0.44 mol) of methyl 2-bromo-4-fluorophenoxyformate was dissolved in 250 ml of sulfuric acid, to which a mixed acid of 30 g of fuming nitric acid and 30 ml of sulfuric acid was added dropwise at 5°C or lower, and the mixture was stirred for 2 hours. The reaction mixture was poured onto ice, and the precipitated crystals were collected by filtration, washed with water, and dried, which afforded 126 g (0.43 mol) of methyl 2-bromo-4-fluoro-5-nitrophenoxyformate.

Then, 125 g (0.43 mol) of methyl 2-bromo-4-fluoro-5-nitrophenoxyformate was suspended in 200 ml of water, to which 19 g (0.47 mol) of sodium hydroxide was added, and the mixture was stirred at 50° to 60°C for 4 hours. After completion of the reaction, the reaction mixture was cooled to room temperature and washed with chloroform. The aqueous layer was acidified with aqueous hydrochloric acid and extracted with ethyl acetate. The ethyl acetate layer was dried and concentrated, which afforded 104 g (0.43 mol) of 2-bromo-4-fluoro-5-nitrophenol.

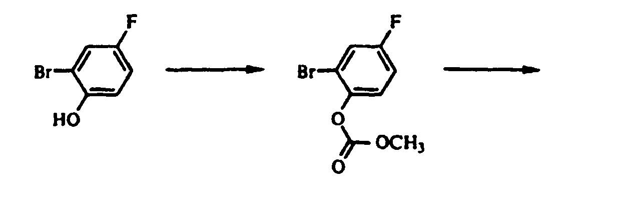

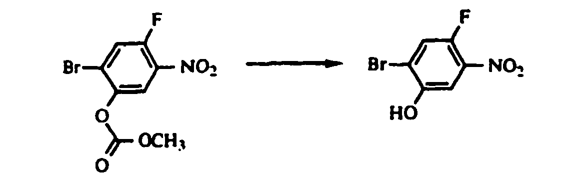

Then, 100 g (0.42 mol) of 2-bromo-4-fluoro-5-nitrophenol was dissolved in 400 ml of dimethylformamide, to which 70 g (0.50 mol) of potassium carbonate was added and after warming to 50°C, 94 g (0.55 mol) of isopropyl iodide was added dropwise, and the mixture was stirred at 45° to 50°C for I hour. After completion of the reaction, the reaction mixture was poured into water and extracted with ethyl acetate. The ethyl acetate layer was washed with water and then with diluted hydrochloric acid, dried, and concentrated. The residue was subjected to column chromatography, which afforded 99.8 g (0.36 mol) of 2-bromo-4-fluoro-5-nitrophenyl isopropyl ether.

Then, 60 g (0.22 mol) of 2-bromo-4-fluoro-5-nitrophenyl isopropyl ether was dissolved in 300 ml of ethyl acetate, to which 1.0 g of 10% palladium-carbon was added, and the hydrogenation was effected under an atmosphere of hydrogen. After completion of the reaction, the palladium-carbon was removed by filtration, and the filtrate was concentrated, which afforded 52 g (0.21 mol) of 4-bromo-2-fluoro-5-isopropoxyaniline.

Then, 108 g (0.57 mol) of tin (II) chloride was dissolved in 100 ml of concentrated hydrochloric acid, followed by cooling to -30°C, to which a diazonium solution prepared from 47 g (0.19 mol) of 4-bromo-2-fluoro-5-isopropoxyaniline, 13.5 g (0.20 mol) of sodium nitrite, and 120 ml of hydrochloric acid was added dropwise at 0°C or lower, and the mixture was stirred at room temperature for 2 hours. The precipitated crystals were collected by filtration and dried in a vacuum oven to give 45 g (0.14 mol) of the crude product, 4-bromo-2-fluoro-5-isopropoxyphenylhydrazine hydrochloride.

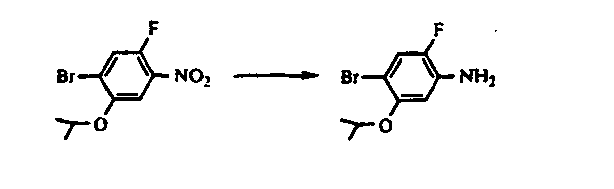

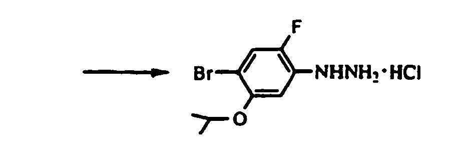

### Production Example 28 (Production of Compound 1-1783)

First, 19 g (52.4 mmol) of carbethoxyethylidenetriphenylphosphorane and 19 g (63.2 mmol) of 3,3,3-trifluoro-2-oxopropanal 1-(4-bromo-2-fluoro-5-isopropyloxyphenylhydrazone) were heated under reflux in 100 ml of THF for 5 hours. After completion of the reaction, the reaction mixture was concentrated, and the residue was subjected to silica gel chromatography, which afforded 9.1 g (22.2 mmol) of compound 1-1783.

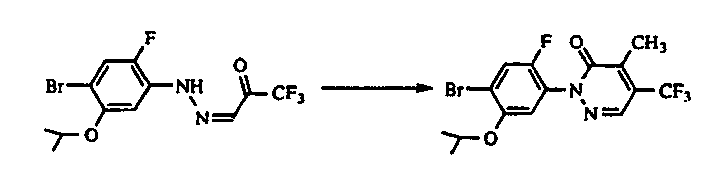

### Production Example 29 (Production of Compound 1-1748)

This example followed the procedures of Production Example 27, except that 41 g (0.41 mol) of 2,4-dichloro-5-isopropoxyphenylhydrazine hydrochloride was used in place of 4-brome-2-fluoro-5-isopropoxyphenylhydrazine hydrochloride, which afforded 31.3 g (91.3 mmol) of 3,3,3-trifluoro-2-oxopropanal 1-(2,4-dichloro-5-isopropoxyphenylhydrazone). Then, this compound and 40 g (0.11 mol) of carbethoxyethylidenetriphenylphosphorane were heated under reflux in 100 ml of THF for 5 hours. After completion of the reaction, the reaction mixture was concentrated, and the residue was subjected to silica gel column chromatography, which afforded 21 g (54.8 mmol) of compound 1-1748.

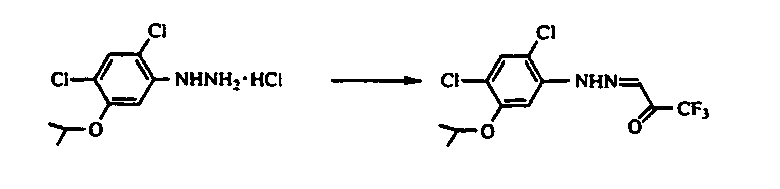

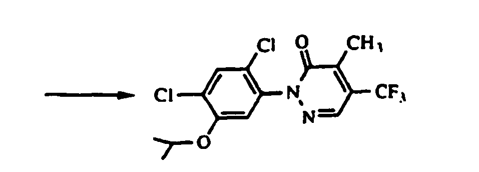

The 2,4-dichloro-5-isopropoxyphenylhydrazine hydrochloride used above was produced from 2,4-dichlorophenol by the same process as shown in Reference Example 3.

### Production Example 30 (Production of Compound 1-1029)

First, 9 g (22.8 mmol) of 2-(4-bromo-2-fluoro-5-isopropyloxyphenyl)-5-trifluoromethylpyridazin-3-one was added to 50 ml of sulfuric acid, and the mixture was stirred for I hour. After completion of the reaction, the reaction mixture was poured onto ice and extracted with ethyl acetate. The ethyl acetate layer was dried and concentrated, and the residue was subjected to column chromatography (eluent, hexane : ethyl acetate = 5 : 1), which afforded 5.9 g (16.7 mmol) of compound 1-1029.

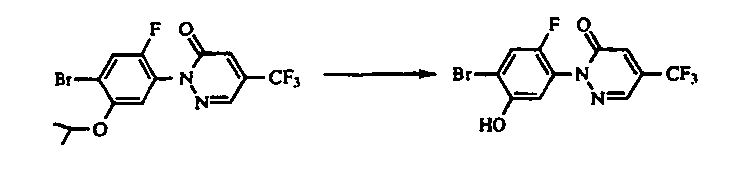

### Production Example 31 (Production of Compound 1-392)

First, 9 g (22.0 mmol) of 2-(4-bromo-2-fluoro-5-isopropyloxyphenyl)-4-methyl-5-trifluoromethylpyridazin-3-one was added to 50 ml of sulfuric acid, and the mixture was stirred for 1 hour. After completion of the reaction, the reaction mixture was poured onto ice and extracted with ethyl acetate. The ethyl acetate layer was dried and concentrated, and the residue was subjected to column chromatography (eluent, hexane : ethyl acetate = 5: 1), which afforded 4.2 g (11.5 mmol) of compound 1-392.

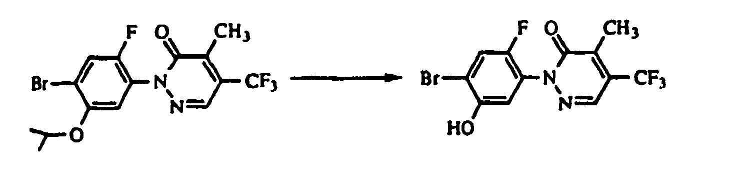

### Production Example 32 (Production of Compound 1-1274)

First, 7.4 g (90.2 mmol) of sodium acetate and 8.0 g (28.2 mmol) of 1,1-dibromo-3,3,3-trifluoroacetone were mixed with 70 ml of water, and the mixture was stirred at 80°C for 30 minutes and then cooled, to which 7.0 g of ethyl 2-chloro-5-hydrazinocinnamate was added at 10°C or lower, and the mixture was stirred for 3 hours. The precipitated crystals were collected by filtration and dried, which afforded 9.6 g (27.5 mmol) of ethyl 2-chloro-5-(3,3,3-trifluoro-2-oxopropylidenehydrazino)cinnamate.

¹H-NMR (250 MHz, CDCl₃, TMS δ (ppm)) 1.36 (3H, t, J = 6.9 Hz), 4.30 (2H, q, J = 6.9 Hz), 6.4-6.6 (1H, m), 7.2-7.5 (3H, m), 7.65 (1H, d, J = 2.5 Hz), 8.0-8.1 (1H, m)

Then, 1.0 g (2.9 mmol) of carbethoxymethylenetriphenylphosphorane and 1.0 g (2.9 mmol) of ethyl 2-chloro-5-(3,3,3-trifluoro-2-oxopropylidenehydrazino)-cinnamate were stirred in 10 ml of THF at room temperature for 1 hour. After completion of the reaction, the reaction mixture was concentrated, and the residue was subjected to column chromatography (eluent, hexane : ethyl acetate = 5 : 1), which afforded 0.43 g (11.5 mmol) of compound 1-1274.

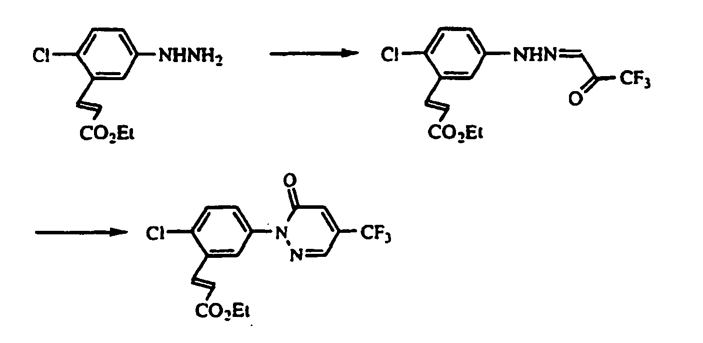

### Reference Example 4

The ethyl 2-chloro-5-hydrazinocinnamate used in Production Example 32 was produced by the following process.

First. 60 g of tin (11) chloride was dissolved in 60 ml of concentrated hydrochloric acid, and the mixture was cooled to -30°C, to which a diazonium solution prepared from 19 g of ethyl 5-amino-2-chlorocinnamate and 6.3 g of sodium nitrite was added dropwise at 0°C or lower. The reaction mixture was stirred at room temperature for 1 hour, and the precipitated crystals were collected by filtration. These crystals were added to ice-water, neutralized with 2N aqueous sodium hydroxide, and extracted with chloroform. The chloroform layer was dried and concentrated, which afforded 7.0 g of ethyl 2-chloro-5-hydrazinocinnamate.

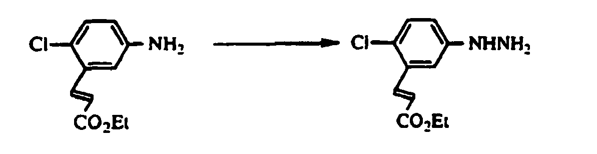

### Production Example 33 (Production of Compound 1-637)

First, 1.1 g (2.9 mmol) of carbethoxyethylidenetriphenylphosphorane and 1.0 g (2.9 mmol) of ethyl 2-chloro-5-(3,3,3-trifluoro-2-oxopropylidenehydrazino)-cinnamate were heated under reflux in 10 ml of THF for 3 hours. After completion of the reaction, the reaction mixture was concentrated, and the residue was subjected to column chromatography (eluent, hexane : ethyl acetate = 5 : 1), which afforded 0.66 g (1.7 mmol) of compound 1-637.

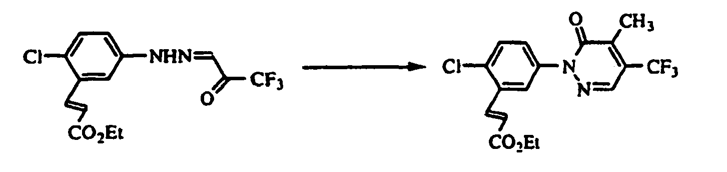

### Production Example 34 (Production of Compound 1-367)

First, 0.5 g (1.6 mmol) of compound 1-353 was dissolved in 1.5 ml of pyridine, to which 0.2 g (1.7 mmol) of methanesulfonyl chloride was added dropwise, followed by stirring for 2 hours. The reaction mixture was poured into ice-water and extracted with ethyl acetate. The ethyl acetate layer was washed with diluted hydrochloric acid, dried, and concentrated. The residue was subjected to column chromatography (eluent, hexane : ethyl acetate = 3 : 1), which afforded 0.42 g (I. 1 mmol) of compound 1-367.

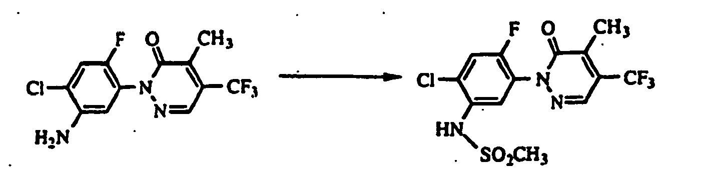

### Production Example 35 (Production of Compound 1-369)

This example followed the procedures of Production Example 34, except that 0.23 g (1.6 mmol) of chloromethylsulfonyl chloride was used in place of methanesulfonyl chloride, which afforded 0.38 g (0.91 mmol) of compound 1-369.

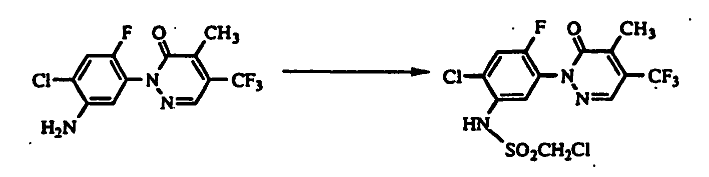

### Production Example 36 (Production of Compound 1-391)

First, 32.3 g of 5-amino-2-chloro-4-fluorophenol was mixed with 150 ml of concentrated hydrochloric acid, and the mixture was stirred at 50°C for 30 minutes, to which a solution of 15 g of sodium nitrite dissolved in 40 ml of water was added dropwise at 0°C over 10 minutes. After stirring at 0°C for 1 hour, the mixture was cooled to -50°C. Then, a solution of 132 g of tin (II) chloride dissolved in 132 g of concentrated sulfuric acid was rapidly added dropwise at -50°C, and the mixture was gradually warmed to room temperature and stirred for 1 hour. The solids formed were collected by filtration and dried at 80°C under reduced pressure to give 75 g of the crude 2-fluoro-4-chloro-5-hydroxyphenylhydrazine hydrochloride-crystals.

¹H-NMR (DMSO-d₆, TMS 8 (ppm)) 3-5 (2H, br), 6.73 (1H. d), 7.22 (1H, d), 8.20 (1H, s), 9-11 (2H, br)

Then, 49.2 g of sodium acetate and 40.5 g of 1,1-dibromo-3,3,3-trifluoroacetone were dissolved in 400 ml of water, and the solution was heated at 80° to 90°C for 40 minutes. The solution was cooled to 0°C, to which 75 g of the crude 2-fluoro-4-chloro-5-hydroxyphenylhydrazine hydrochloride crystals obtained above was added, and the mixture was stirred at room temperature for 70 minutes. The precipitated crystals were collected by filtration and dried under reduced pressure, which afforded 35.4 g of 3,3,3-trifluoro-2-oxopropanal 1-(4-chloro-2-fluoro-5-hydroxyphenylhydrazone).

¹H-NMR (300 MHz, CDCl₃, TMS δ (ppm)) 5.49 (1H, s), 7.15 (1H, d, J = 10.5 Hz), 7.25 (1H, d, J = 7.4 Hz), 7.38 (1H, q, J = 1.8 Hz), 8.75 (1H, s)

Then, 12.9 g of 3,3,3-trifluoro-2-oxopropanal 1-(4-chloro-2-fluom-5-hydroxyphenylhydrazone) and 22.3 g of carbethoxyethylidenetriphenylphosphorane were dissolved in 110 ml of tetrahydrofuran, and the solution was heated under reflux for 3 hours. The solvent was distilled out under reduced pressure, and the residue was subjected to silica gel chromatography, which afforded 8.8 g of 2-(2-fluoro-4-chloro-5-hydroxyphenyl)-4-methyl-5-trifluoromethylpyridazin-3-one (compound 1-391).

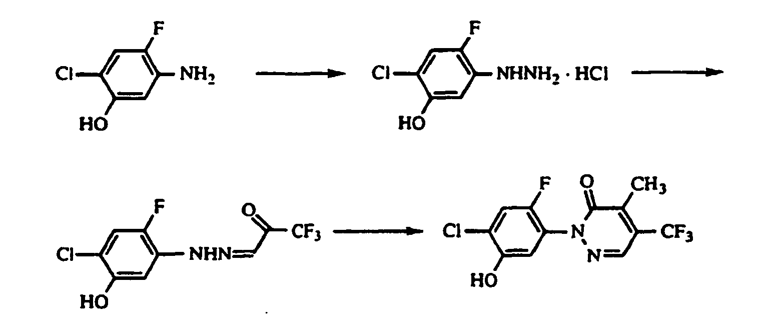

The 5-amino-2-chloro-4-fluorophenol used above can be produced by the method described in the publication of European Patent Application, EP-61741-A.

### Production Example 37 (Production of Compound 1-332)

First, 2 g of 3,3,3-trifluoro-2-oxo-1-propanal 1-(4-chlorophenylhydrazone) and 2 g of ethyl diethylphosphonoacetate were mixed with 20 ml of triethylamine, and the reaction was allowed to proceed at 50°C for 24 hours. The solvent was distilled out under reduced pressure, and the residue was subjected to column chromatography, which afforded 1.16 g of 2-(4-chlorophenyl)-5-trifluoromethylpyridazin-3-one (compound 1-332).

### Reference Example 5

The 3.3.3-trifluoro-2-oxopropanal 1-(4-chloro-2-fluoro-5-isopropoxyphenylhydrazone) produced in Production Example 19 can also be produced by the following process.

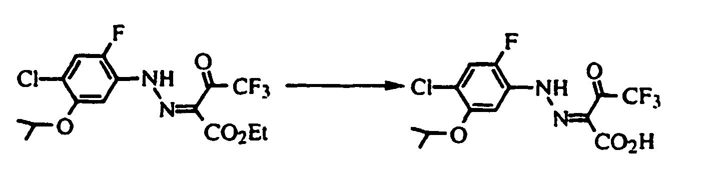

First, 20.1 g of ethyl 4,4,4-trifluoroacetoacetate and 25 g of sodium acetate were dissolved in 150 ml of water, to which a diazonium solution in hydrochloric acid prepared from 20.3 g of 4-chloro-2-fluoro-5-isopropoxyaniline, 20 ml of concentrated hydrochloric acid, 20 ml of water, and 7.35 g of sodium nitrite was added dropwise at 10°C or lower. After stirring at room temperature for 1 hour, the precipitated crystals were collected by filtration, washed with water, and dried, which afforded 34 g of the desired product as orange crystals (yield, 85%).

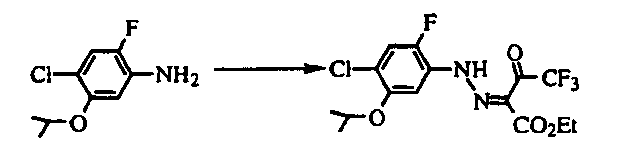

Then, 15.9 g of the ester obtained above and 1.7 g of lithium hydroxide monohydrate were added to 30 ml of 1,4-dioxane and 3 ml of water, and the mixture was heated under reflux for 6 hours. The reaction mixture was poured into ice-water, neutralized with diluted hydrochloric acid, and extracted with ethyl acetate. The ethyl acetate layer was dried and concentrated, and the precipitated crystals were washed with hexane, which afforded 11.3 g of the desired product as yellow crystals (yield, 76.3%).

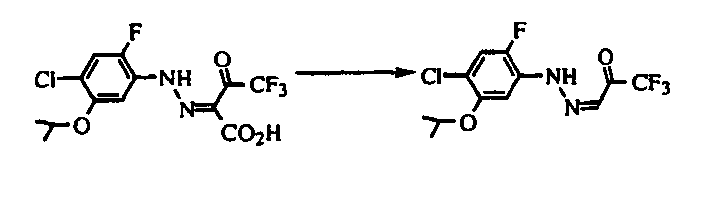

Then, 7.4 g of the carboxylic acid obtained above was dissolved in 42 ml of N,N-dimethylformamide, and the reaction solution was heated to 100°C and kept at the same temperature for 30 minutes. Thereafter, the reaction solution was cooled to room temperature, poured into water, and extracted with ethyl acetate. The ethyl acetate layer was washed with diluted hydrochloric acid, dried with magnesium sulfate, and concentrated, which afforded 5.9 g of the desired product as orange crystals (yield, 90%).

¹H-NMR (250 MHz, CDCl₃, TMS δ (ppm)) 1.39 (6H, d, J = 6.0 Hz), 4.38-4.52 (1H, m), 7.15 (1H, d, J = 10.5 Hz), 7.22 (1H, d. J = 7.3 Hz), 7.43 (1H, q, J = 1.7 Hz), 9.18 (1H, br)

### Reference Example 6

In the same manner as described in Reference Example 2, 3,3,3-trifluoro-2-oxopropanal 4-chlorophenylhydrazone was produced.

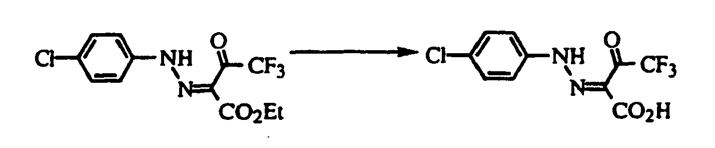

First, 5.0 g of the ester as the starting material arid 0.67 g of lithium hydroxide monohydrate were added to a mixed solution of 30 ml of 1,4-dioxane and 2 ml of water, and the mixture was heated under reflux for 1.5 hours. The reaction mixture was poured into ice-water, neutralized with diluted hydrochloric acid, and extracted with ethyl acetate. The ethyl acetate layer was dried with magnesium sulfate and concentrated, and the precipitated crystals were washed with a mixed solvent of hexane and diethyl ether (hexane : diethyl ether = 2 : 1), which afforded 3.3 g of the desired compound as yellow crystals (yield, 73%).

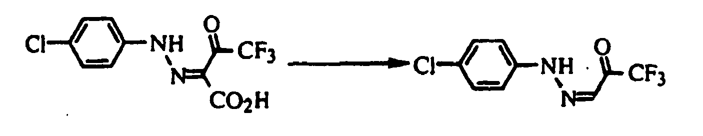

Process 1) A solution prepared by dissolving 3.3 g of the carboxylic acid obtained by the above reaction in 10 ml of dimethylsulfoxide was heated to 100°C and kept at the same temperature for 10 minutes, followed by cooling to room temperature. Thereafter, the reaction mixture was subjected to silica gel chromatography (eluent, hexane: ethyl acetate = 7 : 1), which afforded 2.55 g of the desired product (yield, 91%).

Process 2) A reaction mixture prepared by adding 5.0 g of the carboxylic acid obtained by the above reaction, 0.5 ml of quinoline, and 0.1 g of copper powder to 40 ml of toluene was heated to 100°C and kept at the same temperature for 20 minutes. After completion of the reaction, the reaction mixture was cooled to room temperature and subjected to silica gel chromatography (eluent, hexane : ethyl acetate = 8 : 1), which afforded 3.6 g of the desired product (yield, 86%).

Some of the present compounds are shown with their compound numbers in Tables 1 to 4, where the symbol "n" refers to normal-; "i", iso-; "s", secondary-; and "c", cyclo-.

**TABLE 1**

| | | | | | | |
|---|---|---|---|---|---|---|
| Compounds of the formula: | | | | | | |
| 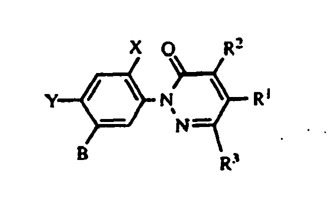 | | | | | | |

| Compound No. | X | Y | R³ | R¹ | R² | B |
|---|---|---|---|---|---|---|
| 1-1 | H | F | H | CF₂ Cl | H | H |
| 1-2 | H | Cl | H | CF₂ Cl | H | H |
| 1-3 | H | Br | H | CF₂ Cl | H | H |
| 1-4 | H | F | H | CF₂ Cl | CH₃ | H |
| 1-5 | H | Cl | H | CF₂ Cl | CH₃ | H |
| 1-6 | H | Br | H | CF₂ Cl | CH₃ | H |
| 1-7 | F | F | H | CF₂ Cl | H | H |
| 1-8 | F | Cl | H | CF₂ Cl | H | H |
| 1-9 | F | Br | H | CF₂ Cl | H | H |
| 1-10 | F | F | H | CF₂ Cl | CH₃ | H |
| 1-11 | F | Cl | H | CF₂ Cl | CH₃ | H |
| 1-12 | F | Br | H | CF₂ Cl | CH₃ | H |
| 1-13 | H | F | H | CF₂ Cl | CH₃ | NO₂ |
| 1-14 | H | Cl | H | CF₂ Cl | CH₃ | NO₂ |
| 1-15 | H | Br | H | CF₂ Cl | CH₃ | NO₂ |
| 1-16 | F | F | H | CF₂ Cl | CH₃ | NO₂ |
| 1-17 | F | Cl | H | CF₂ Cl | CH₃ | NO₂ |
| 1-18 | F | Br | H | CF₂ Cl | CH₃ | NO₂ |
| 1-19 | H | F | H | CF₂ Cl | CH₃ | NH₂ |
| 1-20 | H | Cl | H | CF₂ Cl | CH₃ | NH₂ |
| 1-21 | H | Br | H | CF₂ Cl | CH₃ | NH₂ |
| 1-22 | F | F | H | CF₂ Cl | CH₃ | NH₂ |
| 1-23 | F | Cl | H | CF₂ Cl | CH₃ | NH₂ |
| 1-24 | F | Br | H | CF₂ Cl | CH₃ | NH₂ |
| 1-25 | H | F | H | CF₂ Cl | CH₃ | OH |
| 1-26 | H | Cl | H | CF₂ Cl | CH₃ | OH |
| 1-27 | H | Br | H | CF₂ Cl | CH₃ | OH |
| 1-28 | F | F | H | CF₂ Cl | CH₃ | OH |
| 1-29 | F | Cl | H | CF₂ Cl | CH₃ | OH |
| 1-30 | F | Br | H | CF₂ Cl | CH₃ | OH |
| 1-31 | H | Cl | H | CF₂ Cl | CH₃ | NHCH₃ |
| 1-32 | H | Cl | H | CF₂ Cl | CH₃ | NHC₂ H ₅ |
| 1-33 | H | Cl | H | CF₂ Cl | CH₃ | NHCH₂ CH=CH₂ |
| 1-34 | H | Cl | H | CF₂ Cl | CH₃ | NHCH₂ C ≡ CH |
| 1-35 | H | Cl | H | CF₂ Cl | CH₃ | NHCH(CH₃)C≡CH |
| 1-36 | H | Cl | H | CF₂ Cl | CH₃ | NHSO₂ CH₃ |
| 1-37 | H | Cl | H | CF₂ Cl | CH₃ | NHSO₂ C ₂ H ₅ |
| 1-38 | H | Cl | H | CF₂ Cl | CH₃ | NHSO₂ CH₂ Cl |
| 1-39 | H | Cl | H | CF₂ Cl | CH₃ | NHSO₂ CF₃ |
| 1-40 | H | Cl | H | CF₂ Cl | CH₃ | N(CH₃)SO₂ CH₃ |
| 1-41 | H | Cl | H | CF₂ Cl | CH₃ | N(CH₂ C ≡ CH)SO₂ CH₃ |
| 1-42 | H | Cl | H | CF₂ Cl | CH₃ | NHCOOCH₃ |
| 1-43 | H | Cl | H | CF₂ Cl | CH₃ | NHCOOC₂ H s |
| 1-44 | H | Cl | H | CF₂ Cl | CH₃ | NHCOO ⁿ C₃ H₇ |
| 1-45 | H | Cl | H | CF₂ Cl | CH₃ | NHCOO ⁱ C₃ H₇ |
| 1-46 | H | Cl | H | CF₂ Cl | CH₃ | NHCOO ⁿ C₄ H₉ |
| 1-47 | H | Cl | H | CFz Cl | CH₃ | NHCOO ⁿ C ₅ H₁₁ |
| 1-48 | H | Cl | H | CF₂ Cl | CH₃ | NHCH₂ COOCH ₃ |
| 1-49 | H | Cl | H | CF₂ Cl | CH₃ | NHCH₂ COOC₂ H₅ |
| 1-50 | H | Cl | H | CF₂ Cl | CH₃ | NHCH₂ COO ⁿ C₃ H₇ |
| 1-51 | H | Cl | H | CF₂ Cl | CH₃ | NHCH₂ COO ⁿ C₄ H₉ |
| 1-52 | H | Cl | H | CF₂ Cl | CH₃ | NHCH₂ COO ⁿ C₅ H₁₁ |
| 1-53 | H | Cl | H | CF₂ Cl | CH₃ | NHCH₂ COO ⁱ C₃ H₇ |
| 1-54 | H | Cl | H | CF₂ Cl | CH₃ | NHCH₂ COO ^{c}C₅ H₉ |
| 1-55 | H | Cl | H | CF₂ Cl | CH₃ | NHCH₂ COO ^{c}C₆ H₁₁ |
| 1-56 | H | Cl | H | CF₂ Cl | CH₃ | NHCH(CH₃)COOCH₃ |
| 1-57 | H | Cl | H | CF₂ Cl | CH₃ | NHCH(CH₃)COOC₂ H₅ |
| 1-58 | H | Cl | H | CF₂ Cl | CH₃ | NHCH(CH₃)COO ⁿ C₃ H₇ |
| 1-59 | H | Cl | H | CF₂ Cl | CH₂ | NHCH(CH₃)COO ⁿ C₄ H₉ |
| 1-60 | H | Cl | H | CF₂ Cl | CH₃ | NHCH(CH₃)COO ⁿ C₅ H₁₁ |
| 1-61 | H | Cl | H | CF₂ Cl | CH₃ | NHCH(CH₃)COO ⁱ C₃ H ₇ |
| 1-62 | H | Cl | H | CF₂ Cl | CH₃ | NHCH(CH₃)COO ^{c} C₅ H₉ |
| 1-63 | H | Cl | H | CF₂ Cl | CH₃ | NHCH(CH₃)COO ^{c} C₆ H ₁₁ |
| 1-64 | F | Cl | H | CF₂ Cl | CH₃ | NHCH₃ |
| 1-65 | F | Cl | H | CF₂ Cl | CH₃ | NHC ₂ H ₅ |
| 1-66 | F | Cl | H | CF₂ Cl | CH₃ | NHCH₂ CH=CH₂ |
| 1-67 | F | Cl | H | CF₂ Cl | CH₃ | NHCH₂ C ≡⁻CH |
| 1-68 | F | Cl | H | CF₂ Cl | CH₃ | NHCH(CH ₃)C≡CH |
| 1-69 | F | Cl | H | CF₂ Cl | CH₃ | NHSO₂ CH₃ |
| 1-70 | F | Cl | H | CF₂ Cl | CH₃ | NHSO₂ C₂ H₅ |
| 1-71 | F | Cl | H | CF₂ Cl | CH₃ | NHSO₂ CH₂ Cl |
| 1-72 | F | Cl | H | CF₂ Cl | CH₃ | NHSO₂ CF₃ |
| 1-73 | F | Cl | H | CF₂ Cl | CH₃ | N(CH₃)SO₂ CH₃. |
| 1-74 | F | Cl | H | CF₂ Cl | CH₃ | N(CH₂ C ≡ CH)SO ₂ CH₃ |
| 1-75 | F | Cl | H | CF₂ Cl | CH₃ | NHCOOCH ₃ |
| 1-76 | F | Cl | H | CF₂ Cl | CH₃ | NHCOOC₂ H ₅ |
| 1-77 | F | Cl | H | CF₂ Cl | CH₃ | NHCOO ⁿ C ₃ H ₇ |
| 1-78 | F | Cl | H | CF₂ Cl | CH₃ | NHCOO ⁱ C ₃ H ₇ |
| 1-79 | F | Cl | H | CF₂ Cl | CH₃ | NHCOO ⁿ C ₄ H ₉ |
| 1-80 | F | Cl | H | CF₂ Cl | CH₃ | NHCOO ⁿ C ₅ H ₁₁ |
| 1-81 | F | Cl | H | CF₂ Cl | CH₃ | NHCH₂ COOCH₃ |
| 1-82 | F | Cl | H | CF₂ Cl | CH₃ | NHCH₂ COOC₂ H ₅ |
| 1-83 | F | Cl | H | CF₂ Cl | CH₃ | NHCH₂ COO ⁿ C₃ H₇ |
| 1-84 | F | Cl | H | CF₂ Cl | CH₃ | NHCH₂ COO ⁿ C₄ H₉ |
| 1-85 | F | Cl | H | CF₂ Cl | CH₃ | NHCH₂ COO ⁿ C₅ H₁₁ |
| 1-86 | F | Cl | H | CF₂ Cl | CH₃ | NHCH₂ COO ⁱ C₃ H₇ |
| 1-87 | F | Cl | H | CF₂ Cl | CH₃ | NHCH₂ COO ^{c} C₅ H₉ |
| 1-88 | F | Cl | H | CF₂ Cl | CH₃ | NH₂ CH₂ COO ^{c} C₅ H₁₁ |
| 1-89 | F | Cl | H | CF₂ Cl | CH₃ | NHCH(CH₃) COOCH₃ |
| 1-90 | F | Cl | H | CF₂ Cl | CH₃ | NHCH(CH₃) COOC₂ H₅ |
| 1-91 | F | Cl | H | CF₂ Cl | CH₃ | NHCH(CH₃) COO ⁿ C₃ H₇ |
| 1-92 | F | Cl | H | CF₂ Cl | CH₃ | NHCH(CH₃) COO ⁿ C₄ H₉ |
| 1-93 | F | Cl | H | CF₂ Cl | CH₃ | NHCH(CH₃ )COO ⁿ C₃ H₁₁ |
| 1-94 | F | Cl | H | CF₂ Cl | CH₃ | NHCH(CH₃)COO ⁱ C₃ H₇ |
| 1-95 | F | Cl | H | CF₂ Cl | CH₃ | NHCH(CH₃)COO ^{c}C₅ H₉ |
| 1-96 | F | Cl | H | CF₂ Cl | CH₃ | NHCH(CH₃ )COO ^{c}C₆ H₁₁ |
| 1-97 | H | Cl | H | CF₂ Cl | CH₃ | OCH₃ |
| 1-98 | H | Cl | H | CF₂ Cl | CH₃ | OC₂H₅ |
| 1-99 | H | Cl | H | CF₂ Cl | CH₃ | Oⁱ C₃ H ₇ |
| 1-100 | H | Cl | H | CF₂ Cl | CH₃ | Oⁿ C ₃ H₇ |
| 1-101 | H | Cl | H | CF₂ Cl | CH₃ | OCH₂ CH₂ Cl |
| 1-102 | H | Cl | H | CF₂ Cl | CH₃ | OCF₂ CF₂ H |
| 1-103 | H | Cl | H | CF₂ Cl | CH₃ | O^{c} C₅ H₉ |
| 1-104 | H | Cl | H | CF₂ Cl | CH₃ | O^{c} C₆ H₁₁ |
| 1-105 | H | Cl | H | CF₂ Cl | CH₃ | OCH₂ CH= CH₂ |
| 1-106 | H | Cl | H | CF₂ Cl | CH₃ | OCH₂ CCl = CH₂ |
| 1-107 | H | Cl | H | CF₂ Cl | CH₃ | OCH₂ CCl =CHCl |
| 1-108 | H | Cl | H | CF₂ Cl | CH₃ | OCH(CH ₃)CH = CH₂ |
| 1-109 | H | Cl | H | CF₂ Cl | CH₃ | OCH₂ C ≡ CH |
| 1-110 | H | Cl | H | CF₂ Cl | CH₃ | OCH(CH ₃)C ≡ CH |
| 1-111 | H | Cl | H | CF₂ Cl | CH₃ | OCH₂ C ≡ CBr |
| 1-112 | H | Cl | H | CF₂ Cl | CH₃ | OCH₂ C ≡CCl |
| 1-113 | H | Cl | H | CF₂ Cl | CH₃ | OCH₂ C ≡ CCH ₂ Cl |
| 1-114 | H | Cl | H | CF₂ Cl | CH₃ | OCH₂ CN |
| 1-115 | H | Cl | H | CF₂ Cl | CH₃ | OCH₂ OCH ₃ |
| 1-116 | H | Cl | H | CF₂ Cl | CH₃ | OCH₂ OC₂ H ₅ |
| 1-117 | H | Cl | H | CF₂ Cl | CH₃ | OCH₂ SCH ₃ |
| 1-118 | H | Cl | H | CF₂ Cl | CH₃ | OCH₂ COOCH ₃ |
| 1-119 | H | Cl | H | CF₂ Cl | CH₃ | OCH₂ COOC₂ H ₅ |
| 1-120 | H | Cl | H | CF₂ Cl | CH₃ | OCH₂ COO ⁿ C ₉ H ₇ |
| 1-121 | H | Cl | H | CF₂ Cl | CH₃ | OCH₂ COO ⁿ C₄ H₉ |
| 1-122 | H | Cl | H | CF₂ Cl | CH₃ | OCH₂ COO ⁿ C₅ H₁₁ |
| 1-123 | H | Cl | H | CF₂ Cl | CH₃ | OCH₂ COO ⁱ C₃ H₇ |
| 1-124 | H | Cl | H | CF₂ Cl | CH₃ | OCH₂ COO ^{c} C₅ H₉ |
| 1-125 | H | Cl | H | CF₂ Cl | CH₃ | OCH₂ COO ^{c}C₆ H₁₁ |
| 1-126 | H | Cl | H | CF₂ Cl | CH₃ | OCN(CH ₃)COOCH₃ |
| 1-127 | H | Cl | H | CF₂ Cl | CH₃ | OCH(CH ₃)COOC₂ H₅ |
| 1-128 | H | Cl | H | CF₂ Cl | CH₃ | OCH(CH₃ )COO ⁿ C₃ H ₇ |
| 1-129 | H | Cl | H | CF₂ Cl | CH₃ | OCH(CH₃ )COO ⁿ C₄ H₉ |
| 1-130 | H | Cl | H | CF₂ Cl | CH₃ | OCH(CH₃ )COO ⁿ C₅ H₁₁ |
| 1-131 | H | Cl | H | CF₂ Cl | CH₃ | OCH(CH₃ )COO ⁱ C₃ H ₇ |
| 1-132 | H | Cl | H | CF₂ Cl | CH₃ | OCH(CH₃ )COO ^{c} C₅ H₉ |
| 1-133 | H | Cl | H | CF₂ Cl | CH₃ | OCH(CH ₃)COO ^{c} C₆ H₁₁ |
| 1-134 | H | Cl | H | CF₂ Cl | CH₃ | OCH₂ CON(CH₃ )₂ |
| 1-135 | H | Cl | H | CF₂ Cl | CH₃ | OCH₂ CONC(C₂ H₅)₂ |
| 1-136 | H | Cl | H | CF₂ Cl | CH₃ | OCH₂ CON(CH₃ ) C₂ H₅ |
| 1-137 | H | Cl | H | CF₂ Cl | CH₃ | OCH(CH ₃ )CON(CH ₃)₂ |
| 1-138 | H | Cl | H | CF₂ Cl | CH₃ | OCH(CH₃ )CON(C ₂ H₅)₂ |
| 1-139 | H | Cl | H | CF₂ Cl | CH₃ | OCH(CH₃ )CON(CH ₃)C₂ H₅ |
| 1-140 | H | Cl | H | CF₂ Cl | CH₃ | OCH ₂COON(CH ₃) ₂ |
| 1-141 | H | Cl | H | CF₂ Cl | CH₃ | OCH ₂COON(C₂ H₅)₂ |
| 1-142 | H | Cl | H | CF₂ Cl | CH₃ | OCH(CH₃ )COON(CH₃) ₂ |
| 1-143 | H | Cl | H | CF₂ Cl | CH₃ | OCH(CH₃ )COON(C₂ H₅) ₂ |
| 1-144 | F | Cl | H | CF₂ Cl | CH₃ | OCH₃ |
| 1-145 | F | Cl | H | CF₂ Cl | CH₃ | OC ₂ H ₅ |
| 1-146 | F | Cl | H | CF₂ Cl | CH₃ | Oⁱ C₃ H₇ |
| 1-147 | F | Cl | H | CF₂ Cl | CH₃ | Oⁿ C₃ H ₇ |
| 1-148 | F | Cl | H | CF₂ Cl | CH₃ | OCH₂ CH₂ Cl |
| 1-149 | F | Cl | H | CF₂ Cl | CH₃ | OCF₂ CF₂ H |
| 1-150 | F | Cl | H | CF₂ Cl | CH₃ | O^{c} C₆ H₉ |
| 1-151 | F | Cl | H | CF₂ Cl | CH₃ | O^{c} C ₆ H ₁₁ |
| 1-152 | F | Cl | H | CF₂ Cl | CH₃ | OCH₂ CH₂ = CH₂ |
| 1-153 | F | Cl | H | CF₂ Cl | CH₃ | OCH₂ CCl = CH₂ |
| 1-154 | F | Cl | H | CF₂ Cl | CH₃ | OCH₂ CCl = CHCl |
| 1-155 | F | Cl | H | CF₂ Cl | CH₃ | OCH(CH ₃ )CH = CH₂ |
| 1-156 | F | Cl | H | CF₂ Cl | CH₃ | OCH₂ C ≡ CH |
| 1-157 | F | Cl | H | CF₂ Cl | CH₃ | OCH(CH ₃ )C ≡ CH |
| 1-158 | F | Cl | H | CF₂ Cl | CH₃ | OCH₂ C ≡ CBr |
| 1-159 | F | Cl | H | CF₂ Cl | CH₃ | OCH₂ C ≡CCl |
| 1-160 | F | Cl | H | CF₂ Cl | CH₃ | OCH₂ C ≡ CCH ₂ Cl |
| 1-161 | F | Cl | H | CF₂ Cl | CH₃ | OCH₂ CN |
| 1-162 | F | Cl | H | CF₂ Cl | CH₃ | OCH₂ OCH₃ |
| 1-163 | F | Cl | H | CF₂ Cl | CH₃ | OCH₂ OC₂ H₅ |
| 1-164 | F | Cl | H | CF₂ Cl | CH₃ | OCH₂ SCH ₃ |
| 1-165 | F | Cl | H | CF₂ Cl | CH₃ | OCH₂ COOCH₃ |
| 1-166 | F | Cl | H | CF₂ Cl | CH₃ | OCH₂ COOC₂ H ₅ |
| 1-167 | F | Cl | H | CF₂ Cl | CH₃ | OCH₂ COO ⁿ C ₃ H ₇ |
| 1-168 | F | Cl | H | CF₂ Cl | CH₃ | OCH₂ COO ⁿ C ₄ H₉ |
| 1-169 | F | Cl | H | CF₂ Cl | CH₃ | OCH₂ COO ⁿ C ₅ H ₁₁ |
| 1-170 | F | Cl | H | CF₂ Cl | CH₃ | OCH₂ COO ⁱ C ₃ H ₇ |
| 1-171 | F | Cl | H | CF₂ Cl | CH₃ | OCH₂ COO^{c} C ₅ H₉ |
| 1-172 | F | Cl | H | CF₂ Cl | CH₃ | OCH₂ COO ^{c} C₆ H₁₁ |
| 1-173 | F | Cl | H | CF₂ Cl | CH₃ | OCH(CH₃ )COOCH₃ |
| 1-174 | F | Cl | H | CF₂ Cl | CH₃ | OCH(CH₃)COOC ₂ H ₅ |
| 1-175 | F | Cl | H | CF₂ Cl | CH₃ | OCH(CH₃)COO ⁿ C₃ H₇ |
| 1-176 | F | Cl | H | CF₂ Cl | CH₃ | OCH(CH₃)COO ⁿ C₄ H₉ |
| 1-177 | F | Cl | H | CF₂ Cl | CH₃ | OCH(CH₃)COO ⁿ C₅ H₁₁ |
| 1-178 | F | Cl | H | CF₂ Cl | CH₃ | OCH(CH₃)COO ⁱ C₃ H ₇ |
| 1-179 | F | Cl | H | CF₂ Cl | CH₃ | OCH(CH₃)COO ^{c} C₅ H ₉ |
| 1-180 | F | Cl | H | CF₂ Cl | CH₃ | OCH(CH₃)COO^{c} C₆ H₁₁ |
| 1-181 | F | Cl | H | CF₂ Cl | CH₂ | OCH₂ CON(CH₃)₂ |
| 1-182 | F | Cl | H | CF₂ Cl | CH₃ | OCH₂ CON(C₂ H₅) ₂ |
| 1-183 | F | Cl | H | CF₂ Cl | CH₃ | OCH₂ CON(CH₃ ) C ₂ H ₅ |
| 1-184 | F | Cl | H | CF₂ Cl | CH₃ | OCH(CH₃ )CON(CH₃) ₂ |
| 1-185 | F | Cl | H | CF₂ Cl | CH₃ | OCH(CH₃ )CON(C ₂ H ₅ )₂ |
| 1-186 | F | Cl | H | CF₂ Cl | CH₃ | OCH(CH₃ )CON(CH₃ )C₂ H ₅ |
| 1-187 | F | Cl | H | CF₂ Cl | CH₃ | OCH ₂ COON(CH ₃) ₂ |
| 1-188 | F | Cl | H | CF₂ Cl | CH₃ | OCH ₂ COON(C ₂ H ₅)₂ |
| 1-189 | F | Cl | H | CF₂ Cl | CH₃ | OCH(CH₃ )COON(CH₃ ) ₂ |
| 1-190 | F | Cl | H | CF₂ Cl | CH₃ | OCH(CH₃ )COON(C ₂ H ₅ ) ₂ |
| 1-191 | H | F | H | CF₂ Cl | CH₃ | SH |
| 1-192 | H | Cl | H | CF₂ Cl | CH₃ | SH |
| 1-193 | H | Br | H | CF₂ Cl | CH₃ | SH |
| 1-194 | F | F | H | CF₂ Cl | CH₃ | SH |
| 1-195 | F | Cl | H | CF₂ Cl | CH₃ | SH |
| 1-196 | F | Br | H | CF₂ Cl | CH₃ | SH |
| 1-197 | H | Cl | H | CF₂ Cl | CH₃ | SCH₃ |
| 1-198 | H | Cl | H | CF₂ Cl | CH₃ | SC₂ H ₅ |
| 1-199 | H | Cl | H | CF₂ Cl | CH₃ | Sⁱ C ₃H ₇ |
| 1-200 | H | Cl | H | CF₂ Cl | CH₃ | SCH₂ CH₂ Cl |
| 1-201 | H | Cl | H | CF₂ Cl | CH₃ | S^{c} C₅ H₉ |
| 1-202 | H | Cl | H | CF₂ Cl | CH₃ | S^{c} C₆ H₁₁ |
| 1-203 | H | Cl | H | CF₂ Cl | CH₃ | SCH₂ CH=CH₂ |
| 1-204 | H | Cl | H | CF₂ Cl | CH₃ | SCH₂ CCl = CH₂ |
| 1-205 | H | Cl | H | CF₂ Cl | CH₃ | SCH₂ CCl = CHCl |
| 1-206 | H | Cl | H | CF₂ Cl | CH₃ | SCH(CH₃ )CH = CH₂ |
| 1-207 | H | Cl | H | CF₂ Cl | CH₃ | SCH₂ C ≡ CH |
| 1-208 | H | Cl | H | CF₂ Cl | CH₃ | SCH(CH ₃ )C ≡ CH |
| 1-209 | H | Cl | H | CF₂ Cl | CH₃ | SCH₂ COOCH ₃ |
| 1-210 | H | Cl | H | CF₂ Cl | CH₃ | SCH₂ COOC₂ H ₅ |
| 1-211 | H | Cl | H | CF₂ Cl | CH₃ | SCH₂ COO ⁿ C ₃ H ₇ |
| 1-212 | H | Cl | H | CF₂ Cl | CH₃ | SCH₂ COO ⁿ C₄ H ₉ |
| 1-213 | H | Cl | H | CF₂ Cl | CH₃ | SCH₂ COO ⁿ C ₅ H ₁₁ |
| 1-214 | H | Cl | H | CF₂ Cl | CH₃ | SCH₂ COO ⁱ C ₃ H ₇ |
| 1-215 | H | Cl | H | CF₂ Cl | CH₃ | SCH₂ COO ^{c} C ₅ H ₉ |
| 1-216 | H | Cl | H | CF₂ Cl | CH₃ | SCH₂ COO ^{c} C ₆ H ₁₁ |
| 1-217 | H | Cl | H | CF₂ Cl | CH₃ | SCH(CH₃)COOCH₃ |
| 1-218 | H | Cl | H | CF₂ Cl | CH₃ | SCH(CH ₃)COOC₂ H₅ |
| 1-219 | H | Cl | H | CF₂ Cl | CH₃ | SCH(CH ₃ )COO ⁿ C₃ H ₇ |
| 1-220 | H | Cl | H | CF₂ Cl | CH ₃ | SCH(CH ₃ )COO ⁿ C₄ H ₉ |
| 1-221 | H | Cl | H | CF₂ Cl | CH₃ | SCH(CH ₃ )COO ⁿ C₅ H ₁₁ |
| 1-222 | H | Cl | H | CF₂ Cl | CH₃ | SCH(CH ₃ )COO ⁱ C₃ H ₇ |
| 1-223 | H | Cl | H | CF₂ Cl | CH₃ | SCH(CH ₃ )COO ^{c} C₅ H ₉ |
| 1-224 | H | Cl | H | CF₂ Cl | CH₃ | SCH(CH ₃ )COO ^{c} C₆ H ₁₁ |
| 1-225 | H | Cl | H | CF₂ Cl | CH₃ | SCH₂ CON(CH₃ ) ₂ |
| 1-226 | H | Cl | H | CF₂ Cl | CH₃ | SCH₂ CON(C ₂ H ₅) ₂ |
| 1-227 | H | Cl | H | CF₂ Cl | CH₃ | SCH₂CON (tetramethylene) |
| 1-228 | H | Cl | H | CF₂ Cl | CH₃ | SCH₂CON(pentamethylene) |
| 1-229 | H | Cl | H | CF₂ Cl | CH₃ | SCH₂CON (ethyleneoxyethylene) |
| 1-230 | H | Cl | H | CF₂ Cl | CH₃ | SCH(CH₃)CON(CH₃)₂ |
| 1-231 | H | Cl | H | CF₂ Cl | CH₃ | SCH(CH₃)CON(C₂ H₅)₂ |
| 1-232 | H | Cl | H | CF₂ Cl | CH₃ | SCH(CH₃)CON (tetramethylene) |
| 1-233 | H | Cl | H | CF₂ Cl | CH₃ | SCH(CH₃)CON (pentamethylene) |
| 1-234 | F | Cl | H | CF₂ Cl | CH₃ | SCH₃ |
| 1-235 | F | Cl | H | CF₂ Cl | CH₃ | SC₂ H ₅ |
| 1-236 | F | Cl | H | CF₂ Cl | CH₃ | S' C₃ H₇ |
| 1-237 | F | Cl | H | CF₂ Cl | CH₃ | SCH₂ CH₂ Cl |
| 1-238 | F | Cl | H | CF₂ Cl | CH₃ | S^{c} C ₅ H ₉ |
| 1-239 | F | Cl | H | CF₂ Cl | CH₃ | S^{c} C ₆ H₁₁ |
| 1-240 | F | Cl | H | CF₂ Cl | CH₃ | SCH₂ CH=CH₂ |
| 1-241 | F | Cl | H | CF₂ Cl | CH₃ | SCH₂ CCl =CH₂ |
| 1-242 | F | Cl | H | CF₂ Cl | CH₃ | SCH₂ CCI = CHCl |
| 1-243 | F | Cl | H | CF₂ Cl | CH₃ | SCH(CH₃)CH = CH₂ |
| 1-244 | F | Cl | H | CF₂ Cl | CH₃ | SCH₂ C ≡CH |
| 1-245 | F | Cl | H | CF₂ Cl | CH₃ | SCH(CH ₃)C≡CH |
| 1-246 | F | Cl | H | CF₂ Cl | CH₃ | SCH₂ COOCH₃ |
| 1-247 | F | Cl | H | CF₂ Cl | CH₃ | SCH₂ CODC₂ H ₅ |
| 1-248 | F | Cl | H | CF₂ Cl | CH₃ | SCH₂ COO ⁿ C₃ H₇ |
| 1-249 | F | Cl | H | CF₂ Cl | CH₃ | SCH₂ COO ⁿ C ₄ H ₉ |
| 1-250 | F | Cl | H | CF₂ Cl | CH₃ | SCH₂ COO ⁿ C ₅ H ₁₁ |
| 1-251 | F | Cl | H | CF₂ Cl | CH₃ | SCH₂ COO ⁱ C ₃ H ₇ |
| 1-252 | F | Cl | H | CF₂ Cl | CH₃ | SCH₂ COO ^{c} C ₅ H ₉ |
| 1-253 | F | Cl | H | CF₂ Cl | CH₃ | SCH₂ COO ^{c} C ₆ H ₁₁ |
| 1-254 | F | Cl | H | CF₂ Cl | CH₃ | SCH(CH ₃ )COOCH ₃ |
| 1-255 | F | Cl | H | CF₂ Cl | CH₃ | SCH(CH ₃ )COOC ₂ H ₅ |
| 1-256 | F | Cl | H | CF₂ Cl | CH₃ | SCH(CH ₃ )COO ⁿ C₃ H ₇ |
| 1-257 | F | Cl | H | CF₂ Cl | CH₃ | SCH(CH ₃ )COO ⁿ C₄ H ₉ |
| 1-258 | F | Cl | H | CF₂ Cl | CH₃ | SCH(CH ₃ )COO ⁿ C₅ H ₁₁ |
| 1-259 | F | Cl | H | CF₂ Cl | CH₃ | SCH(CH ₃ )COO ⁱ C₃ H₇ |
| 1-260 | F | Cl | H | CF₂ Cl | CH₃ | SCH(CH ₃ )COO ^{c} C₅ H ₉ |
| 1-261 | F | Cl | H | CF₂ Cl | CH₃ | SCH(CH ₃ )COO ^{c} C₆ H₁₁ |
| 1-262 | F | Cl | H | CF₂ Cl | CH₃ | SCH₂ CON(CH₃ ) ₂ |
| 1-263 | F | Cl | H | CF₂ Cl | CH₃ | SCH₂ CON(C ₂ H ₅ ) ₂ |
| 1-264 | F | Cl | H | CF₂ Cl | CH₃ | SCH₂CON(tetramethylene) |
| 1-265 | F | Cl | H | CF₂ Cl | CH₃ | SCH₂CON(pentamethylene) |
| 1-266 | F | Cl | H | CF₂ Cl | CH₃ | SCH₂CON (ethyleneoxyethylene) |
| 1-267 | F | Cl | H | CF₂ Cl | CH₃ | SCH(CH₃ )CON(CH ₃ ) ₂ |
| 1-268 | F | Cl | H | CF₂ Cl | CH₃ | SCH(CH₃ )CON(C₂ H₅ ) ₂ |
| 1-269 | F | Cl | H | CF₂ Cl | CH₃ | SCH(CH₃)CON (tetramethylene) |
| 1-270 | F | Cl | H | CF₂ Cl | CH₃ | SCH(CH₃)CON (pentamethylene) |
| 1-271 | H | F | H | CF₂ Cl | CH₃ | SO₂ Cl |
| 1-272 | H | Cl | H | CF₂ Cl | CH₃ | SO₂ Cl |
| 1-273 | H | Br | H | CF₂ Cl | CH₃ | SO₂ Cl |
| 1-274 | F | F | H | CF₂ Cl | CH₃ | SO₂ Cl |
| 1-275 | F | Cl | H | CF₂ Cl | CH₃ | SO₂ Cl |
| 1-276 | F | Br | H | CF₂ Cl | CH₃ | SO₂ Cl |
| 1-277 | H | Cl | H | CF₂ Cl | CH₃ | SO₂ OCH₃ |
| 1-278 | H | Cl | H | CF₂ Cl | CH₃ | SO₂ OC₂ H₅ |
| 1-279 | H | Cl | H | CF₂ Cl | CH₃ | SO₂ Oⁱ C₃ H₇ |
| 1-280 | H | Cl | H | CF₂ Cl | CH₃ | SO₂ OCH ₂ CH = CH₂ |
| 1-281 | F | Cl | H | CF₂ Cl | CH₃ | SO₂ OCH₃ |
| 1-282 | F | Cl | H | CF₂ Cl | CH₃ | SO₂ OC₂ H₅ |
| 1-283 | F | Cl | H | CF₂ Cl | CH₃ | SO₂ Oⁱ C₃ H₇ |
| 1-284 | F | Cl | H | CF₂ Cl | CH₃ | SO₂ OCH₂ CH = CH₂ |
| 1-285 | H | Cl | H | CF₂ Cl | CH₃ | SO₂ N(CH₃)₂ |
| 1-286 | H | Cl | H | CF₂ Cl | CH₃ | SO₂ N (C₂ H ₅)₂ |
| 1-287 | F | Cl | H | CF₂ Cl | CH₃ | SO₂ N (CH₃)₂ |
| 1-288 | F | Cl | H | CF₂ Cl | CH₃ | SO₂ N (C₂ H₅)₂ |
| 1-289 | H | Cl | H | CF₂ Cl | CH₃ | COOH |
| 1-290 | H | Cl | H | CF₂ Cl | CH₃ | COOCH₃ |
| 1-291 | H | Cl | H | CF₂ Cl | CH₃ | COOC ₂ H₅ |
| 1-292 | H | Cl | H | CF₂ Cl | CH₃ | COOⁿ C ₃ H ₇ |
| 1-293 | H | Cl | H | CF₂ Cl | CH₃ | COOⁿ C ₄ H ₉ |
| 1-294 | H | Cl | H | CF₂ Cl | CH₃ | COOⁿ C ₅ H ₁₁ |
| 1-295 | H | Cl | H | CF₂ Cl | CH₃ | COOⁱ C ₃ H ₇ |
| 1-296 | H | Cl | H | CF₂ Cl | CH₃ | COOCH₂ CH₂ Cl |
| 1-297 | H | Cl | H | CF₂ Cl | CH₃ | COOCH₂ CH₂ Br |
| 1-298 | H | Cl | H | CF₂ Cl | CH₃ | CON(CH ₃ ) ₂ |
| 1-299 | H | Cl | H | CF₂ Cl | CH₃ | CONHCH ₃ |
| 1-300 | H | Cl | H | CF₂ Cl | CH₃ | CON(C₂ H ₅ ) ₂ |
| 1-301 | H | Cl | H | CF₂ Cl | CH₃ | CONHC₂ H ₅ |
| 1-302 | H | Cl | H | CF₂ Cl | CH₃ | COCH₃ |
| 1-303 | H | Cl | H | CF₂ Cl | CH₃ | COC₂ H₅ |
| 1-304 | H | Cl | H | CF₂ Cl | CH₃ | COCH₂ Cl |
| 1-305 | H | Cl | H | CF₂ Cl | CH₃ | CHO |
| 1-306 | H | Cl | H | CF₂ Cl | CH₃ | CH=CHCOOCH₃ |
| 1-307 | H | Cl | H | CF₂ Cl | CH₃ | CH= CHCOOC₂ H ₅ |
| 1-308 | H | Cl | H | CF₂ Cl | CH₃ | CH₂ CH₂ COOCH ₃ |
| 1-309 | H | Cl | H | CF₂ Cl | CH₃ | CH₂ CH₂ COOC₂ H ₅ |
| 1-310 | F | Cl | H | CF₂ Cl | CH₃ | COOH |
| 1-311 | F | Cl | H | CF₂ Cl | CH₃ | COOCH ₃ |
| 1-312 | F | Cl | H | CF₂ Cl | CH₃ | COOC ₂H₅ |
| 1-313 | F | Cl | H | CF₂ Cl | CH₃ | COOⁿ C₃ H₇ |
| 1-314 | F | Cl | H | CF₂ Cl | CH₃ | COOⁿ C₄ H₉ |
| 1-315 | F | Cl | H | CF₂ Cl | CH₃ | COOⁿ C ₅ H ₁₁ |
| 1-316 | F | Cl | H | CF₂ Cl | CH₃ | COOⁱ C₃H₇ |
| 1-317 | F | Cl | H | CF₂ Cl | CH₃ | COOCH₂ CH₂ Cl |
| 1-318 | F | Cl | H | CF₂ Cl | CH₃ | COOCH₂ CH₂ Br |
| 1-319 | F | Cl | H | CF₂ Cl | CH₃ | CON(CH₃) ₂ |
| 1-320 | F | Cl | H | CF₂ Cl | CH₃ | CONHCH ₃ |
| 1-321 | F | Cl | H | CF₂ Cl | CH₃ | CON(C₂ H ₅) ₂ |
| 1-322 | F | Cl | H | CF₂ Cl | CH₃ | CONHC₂ H ₅ |
| 1-323 | F | Cl | H | CF₂ Cl | CH₃ | COCH ₃ |
| 1-324 | F | Cl | H | CF₂ Cl | CH₃ | COC₂ H ₅ |
| 1-325 | F | Cl | H | CF₂ Cl | CH₃ | COCH₂ Cl |
| 1-326 | F | Cl | H | CF₂ Cl | CH₃ | CHO |
| 1-327 | F | Cl | H | CF₂ Cl | CH₃ | CH=CHCOOCH ₃ |
| 1-328 | F | Cl | H | CF₂ Cl | CH₃ | CH= CHCOOC₂ H ₅ |
| 1-329 | F | Cl | H | CF₂ Cl | CH₃ | CH₂ CH₂ COOCH ₃ |
| 1-330 | F | Cl | H | CF₂ Cl | CH₃ | CH₂ CH₂ COOC₂ H ₅ |
| 1-331 | H | F | H | CF₃ | H | H |
| 1-332 | H | Cl | H | CF₃ | H | H |
| 1-333 | H | Br | H | CF₃ | H | H |
| 1-334 | H | F | H | CF₃ | CH₃ | H |
| 1-335 | H | Cl | H | CF₃ | CH₃ | H |
| 1-336 | H | Br | H | CF₃ | CH₃ | H |
| 1-337 | F | F | H | CF₃ | H | |
| 1-338 | F | Cl | H | CF₃ | H | H |
| 1-339 | F | Br | H | CF₃ | H | H |
| 1-340 | F | F | H | CF₃ | CH₃ | H |
| 1-341 | F | Cl | H | CF₃ | CH₃ | H |
| 1-342 | F | Br | H | CF₃ | CH₃ | H |
| 1-343 | H | F | H | CF₃ | CH₃ | NO₂ |
| 1-344 | H | Cl | H | CF₃ | CH₃ | NO₂ |
| 1-345 | H | Br | H | CF₃ | CH₃ | NO₂ |
| 1-346 | F | F | H | CF₃ | CH₃ | NO₂ |
| 1-347 | F | Cl | H | CF₃ | CH₃ | NO₂ |
| 1-348 | F | Br | H | CF₃ | CH₃ | NO₂ |
| 1-349 | H | F | H | CF₃ | CH₃ | NH₂ |
| 1-350 | H | Cl | H | CF₃ | CH₃ | NH₂ |
| 1-351 | H | Br | H | CF₃ | CH₃ | NH₂ |
| 1-352 | F | F | H | CF₃ | CH₃ | NH₂ |
| 1-353 | F | Cl | H | CF₃ | CH₃ | NH₂ |
| 1-354 | H | Cl | H | CF₃ | CH₃ | NHCH(CH₃ )COOCH ₃ |
| 1-355 | H | Cl | H | CF₃ | CH₃ | NHCH(CH₃ )COOC ₂ H ₅ |
| 1-356 | H | Cl | H | CF₃ | CH₃ | NHCH(CH₃ )COO ⁿ C₃ H ₇ |
| 1-357 | H | Cl | H | CF₃ | CH₃ | NHCH(CH₃ )COO ⁿ C₄ H ₉ |
| 1-358 | H | Cl | H | CF₃ | CH₃ | NHCH(CH₃ )COO ⁿ C₅ H ₁₁ |
| 1-359 | H | Cl | H | CF₃ | CH₃ | NHCH(CH₃ )COO ⁱ C₃ H ₇ |
| 1-360 | H | Cl | H | CF₃ | CH₃ | NHCH(CH₃ )COO ^{c} C₅ H ₉ |
| 1-361 | H | Cl | H | CF₃ | CH₃ | NHCH(CH₃ )COO ^{c} C₆ H₁₁ |
| 1-362 | F | Cl | H | CF₃ | CH₃ | NHCH₃ |
| 1-363 | F | Cl | H | CF₃ | CH₃ | NHC ₂ H ₅ |
| 1-364 | F | Cl | H | CF₃ | CH₃ | NHCH₂ CH=CH₂ |
| 1-365 | F | Cl | H | CF₃ | CH₃ | NHCH₂ C ≡ CH |
| 1-366 | F | Cl | H | CF₃ | CH₃ | NHCH(CH₃ )C≡CH |
| 1-367 | F | Cl | H | CF₃ | CH₃ | NHSO₂ CH₃ |
| 1-368 | F | Cl | H | CF₃ | CH₃ | NHSO₂ C ₂ H ₅ |
| 1-369 | F | Cl | H | CF₃ | CH₃ | NHSO₂ CH₂ Cl |
| 1-370 | F | Cl | H | CF₃ | CH₃ | NHSO₂ CF₃ |
| 1-371 | F | Cl | H | CF₃ | CH₃ | N(CH₃ )SO ₂ CH₃ |
| 1-372 | F | Cl | H | CF₃ | CH₃ | N(CH₂ C ≡ CH)SO ₂ CH₃ |
| 1-373 | F | Cl | H | CF₃ | CH₃ | NHCOOCH ₃ |
| 1-374 | F | Cl | H | CF₃ | CH₃ | NHCOOC₂ H ₅ |
| 1-375 | F | Cl | H | CF₃ | CH₃ | NHCOO ⁿ C ₃ H ₇ |
| 1-376 | F | Cl | H | CF₃ | CH₃ | NHCOO ⁱ C ₃ H ₇ |
| 1-377 | F | Cl | H | CF₃ | CH₃ | NHCOO ⁿ C ₄ H ₉ |
| 1-378 | F | Cl | H | CF₃ | CH₃ | NHCOO ⁿ C ₅ H ₁₁ |
| 1-379 | F | Cl | H | CF₃ | CH₃ | NHCH₂ COOCH ₃ |
| 1-380 | F | Cl | H | CF₃ | CH₃ | NHCH₂ COOC₂ H ₅ |
| 1-381 | F | Cl | H | CF₃ | CH₃ | NHCH₂ COO ⁿ C ₃ H ₇ |
| 1-382 | F | Cl | H | CF₃ | CH₃ | NHCH₂ COO ⁿ C ₄H ₉ |
| 1-383 | F | Cl | H | CF₃ | CH₃ | NHCH₂ COO ⁿ C ₅ H ₁₁ |
| 1-384 | F | Cl | H | CF₃ | CH₃ | NHCH₂ COO ⁱ C₃ H ₇ |
| 1-385 | F | Cl | H | CF₃ | CH₃ | NHCH₂ COO ^{c} C ₅ H ₉ |
| 1-386 | F | Br | H | CF₃ | CH₃ | NH₂ |
| 1-387 | H | F | H | CF₃ | CH₃ | OH |
| 1-388 | H | Cl | H | CF₃ | CH₃ | OH |
| 1-389 | H | Br | H | CF₃ | CH₃ | OH |
| 1-390 | F | F | H | CF₃ | CH₃ | OH |
| 1-391 | F | Cl | H | CF₃ | CH₃ | OH |
| 1-392 | F | Br | H | CF₃ | CH₃ | OH |
| 1-393 | H | Cl | H | CF₃ | CH₃ | NHCH₃ |
| 1-394 | H | Cl | H | CF₃ | CH₃ | NHC ₂ H ₅ |
| 1-395 | H | Cl | H | CF₃ | CH₃ | NHCH₂ CH=CH₂ |
| 1-396 | H | Cl | H | CF₃ | CH₃ | NHCH₂ C ≡ CH |
| 1-397 | H | Cl | H | CF₃ | CH₃ | NHCH(CH ₃ )C≡CH |
| 1-398 | H | Cl | H | CF₃ | CH₃ | NHSO₂ CH₃ |
| 1-399 | H | Cl | H | CF₃ | CH₃ | NHSO₂ C ₂ H ₅ |
| 1-400 | H | Cl | H | CF₃ | CH₃ | NHSO₂ CH₂ Cl |
| 1-401 | H | Cl | H | CF₃ | CH₃ | NHSO₂ CF₃ |
| 1-402 | H | Cl | H | CF₃ | CH₃ | N(CH₃ )SO ₂ CH₃ |
| 1-403 | H | Cl | H | CF₂ | CH₃ | N(CH₂ C ≡CH)SO ₂ CH₃ |
| 1-404 | H | Cl | H | CF₃ | CH₃ | NHCOOCH ₃ |
| 1-405 | H | Cl | H | CF₃ | CH₃ | NHCOOC₂ H ₅ |
| 1-406 | H | Cl | H | CF₃ | CH₃ | NHCOO ⁿ C₃ H ₇ |
| 1-407 | H | Cl | H | CF₃ | CH₃ | NHCOO ⁱ C ₃ H ₇ |
| 1-408 | H | Cl | H | CF₃ | CH₃ | NHCOO ⁿ C ₄ H ₉ |
| 1-409 | H | Cl | H | CF₃ | CH₃ | NHCOO ⁿ C ₅ H ₁₁ |
| 1-410 | H | Cl | H | CF₃ | CH₃ | NHCH₂ COOCH ₃ |
| 1-411 | H | Cl | H | CF₃ | CH₃ | NHCH₂ COOC₂ H ₅ |
| 1-412 | H | Cl | H | CF₃ | CH₃ | NHCH₂ COO ⁿ C ₃ H ₇ |
| 1-413 | H | Cl | H | CF₃ | CH₃ | NHCH₂ COO ⁿ C ₄ H ₉ |
| 1-414 | H | Cl | H | CF₃ | CH₃ | NHCH₂ COO ⁿ C ₅ H ₁₁ |
| 1-415 | H | Cl | H | CF₃ | CH₃ | NHCH₂ COO ⁱ C₃ H ₇ |
| 1-416 | H | Cl | H | CF₃ | CH₃ | NHCH₂ COO ^{c} C ₅ H ₉ |
| 1-417 | H | Cl | H | CF₃ | CH₃ | NHCH₂ COO ^{c} C ₆ H ₁₁ |
| 1-418 | F | Cl | H | CF₃ | CH₃ | NH CH₂ COO ^{c} C ₆ H ₁₁ |
| 1-419 | F | Cl | H | CF₃ | CH₃ | NHCH(CH ₃ )COOCH ₃ |
| 1-420 | F | Cl | H | CF₃ | CH₃ | NHCH(CH₃)COOC ₂ H ₅ |
| 1-421 | F | Cl | H | CF₃ | CH₃ | NHCH(CH₃ )COO ⁿ C₃ H ₇ |
| 1-422 | F | Cl | H | CF₃ | CH₃ | NHCH(CH₃ )COO ⁿ C₄ H ₉ |
| 1-423 | F | Cl | H | CF₃ | CH₃ | NHCH(CH₃ )COO ⁿ C₅ H ₁₁ |
| 1-424 | F | Cl | H | CF₃ | CH₃ | NHCH(CH₃ )COO ⁱ C₃ H ₇ |
| 1-425 | F | Cl | H | CF₃ | CH₃ | NHCH(CH₃ )COO ^{c} C₅ H ₉ |
| 1-426 | F | Cl | H | CF₃ | CH₃ | NHCH(CH₃ )COO ^{c} C₆ H ₁₁ |
| 1-427 | H | Cl | H | CF₃ | CH₃ | OCH₃ |
| 1-428 | H | Cl | H | CF₃ | CH₃ | OC ₂ H ₅ |
| 1-429 | H | Cl | H | CF₃ | CH₃ | Oⁱ C ₃ H ₇ |
| 1-430 | H | Cl | H | CF₃ | CH₃ | Oⁿ C ₃ H ₇ |
| 1-431 | H | Cl | H | CF₃ | CH₃ | OCH₂ CH₂ Cl |
| 1-432 | H | Cl | H | CF₃ | CH₃ | OCF₂ CF₂ H |
| 1-433 | H | Cl | H | CF₃ | CH₃ | O^{c} C ₅ H ₉ |
| 1-434 | H | Cl | H | CF₃ | CH₃ | O^{c} C ₆ H ₁₁ |
| 1-435 | H | Cl | H | CF₃ | CH₃ | OCH₂ CH = CH₂ |
| 1-436 | H | Cl | H | CF₃ | CH₃ | OCH₂ CCl = CH₂ |
| 1-437 | H | Cl | H | CF₃ | CH₃ | OCH₂ CCl = CHCI |
| 1-438 | H | Cl | H | CF₃ | CH₃ | OCH(CH₃ )CH =CH₂ |
| 1-439 | H | Cl | H | CF₃ | CH₃ | OCH₂ C ≡ CH |
| 1-440 | H | Cl | H | CF₃ | CH₃ | OCH(CH₃) C≡CH |
| 1-441 | H | Cl | H | CF₃ | CH₃ | OCH₂ C = CBr |
| 1-442 | H | Cl | H | CF₃ | -CH₃ | OCH₂ C ≡CCl |
| 1-443 | H | Cl | H | CF₃ | CH₃ | OCH₂ C ≡CCH₂ Cl |
| 1-444 | H | Cl | H | CF₃ | CH₃ | OCH₂ CN |
| 1-445 | H | Cl | H | CF₃ | CH₃ | OCH₂ OCH₃ |
| 1-446 | H | Cl | H | CF₃ | CH₃ | OCH₂ OC₂ H₅ |
| 1-447 | H | Cl | H | CF₃ | CH₃ | OCH₂ SCH₃ |
| 1-448 | H | Cl | H | CF₃ | CH₃ | OCH₂ COOCH₃ |
| 1-449 | H | Cl | H | CF₃ | CH₃ | OCH₂ COOC₂ H₅ |
| 1-450 | H | Cl | H | CF₃ | CH₃ | OCH₂ COO ⁿ C₃ H₇ |
| 1-451 | H | Cl | H | CF₃ | CH₃ | OCH₂ COO ⁿ C₄ H₉ |
| 1-452 | H | Cl | H | CF₃ | CH₃ | OCH₂ COO ⁿ C₆ H₁₁ |
| 1-453 | H | Cl | H | CF₃ | CH₃ | OCH₂ COOⁿ C₃ H₇ |
| 1-454 | H | Cl | H | CF₃ | CH₃ | OCH₂ COO ^{c} C₅ H₉ |
| 1-455 | H | Cl | H | CF₃ | CH₃ | OCH₂ COO ^{c} C₆ H₁₁ |
| 1-456 | H | Cl | H | CF₃ | CH₃ | OCH(CCH₃)COOCH₃ |
| 1-457 | H | Cl | H | CF₃ | CH₃ | OCH(CH₃)COOC ₂ H ₅ |
| 1-458 | H | Cl | H | CF₃ | CH₃ | OCH(CH ₃)COOⁿ C₃ H₇ |
| 1-459 | H | Cl | H | CF₃ | CH₃ | OCH(CH₃)COOⁿ C₄ H ₉ |
| 1-460 | H | Cl | H | CF₃ | CH₃ | OCH(CH₃)COO ⁿ C₅ H₁₁ |
| 1-461 | H | Cl | H | CF₃ | CH₃ | OCH(CH ₃ )COO ⁱ C₃ H₇ |
| 1-462 | H | Cl | H | CF₃ | CH₃ | OCH(CH₃ )COO ^{c} C₅ H ₉ |
| 1-463 | H | Cl | H | CF₃ | CH₃ | OCH(CH ₃ )COO ^{c} C₆ H ₁₁ |
| 1-464 | H | Cl | H | CF₃ | CH₃ | OCH₂ CON(CH₃ ) ₂ |
| 1-465 | H | Cl | H | CF₃ | CH₃ | OCH₂ CON(C ₂ H ₅ ) ₂ |
| 1-466 | H | Cl | H | CF₃ | CH₃ | OCH₂ CON(CH₃ ) C ₂ H ₅ |
| 1-467 | H | Cl | H | CF₃ | CH₃ | OCH(CH ₃ )CON(CH ₃ ) ₂ |
| 1-468 | H | Cl | H | CF₃ | CH₃ | OCH(CH₃ )CON(C ₂ H ₅ )₂ |
| 1-469 | H | Cl | H | CF₃ | CH₃ | OCH(CH₃)CON(CH₃ )C₂ H₅ |
| 1-470 | H | Cl | H | CF₃ | CH₃ | OCH ₂ COON(CH ₃ ) ₂ |
| 1-471 | H | Cl | H | CF₃ | CH₃ | OCH ₂ COON(C₂ H₅)₂ |
| 1-472 | H | Cl | H | CF₃ | CH₃ | OCH(CH₃ )COON(CH₃ ) ₂ |
| 1-473 | H | Cl | H | CF₃ | CH₃ | OCH(CH₃ )COON(C ₂ H ₅) ₂ |
| 1-474 | F | Cl | H | CF₃ | CH₃ | OCH₃ |
| 1-475 | F | Cl | H | CF₃ | CH₃ | OC ₂H₅ |
| 1-476 | F | Cl | H | CF₃ | CH₃ | Oⁱ C ₃ H ₇ |
| 1-477 | F | Cl | H | CF₃ | CH₃ | Oⁿ C₃ H ₇ |
| 1-478 | F | Cl | H | CF₃ | CH₃ | OCH₂ CH₂ Cl |
| 1-479 | F | Cl | H | CF₃ | CH₃ | OCF₂ CF₂ H |
| 1-480 | F | Cl | H | CF₃ | CH₃ | O^{c} C ₅ H ₉ |
| 1-481 | F | Cl | H | CF₃ | CH₃ | O^{c} C ₆ H ₁₁ |
| 1-482 | F | Cl | H | CF₃ | CH₃ | OCH₂ CH= CH₂ |
| 1-483 | F | Cl | H | CF₃ | CH₃ | OCH₂ CCl =CH₂ |
| 1-484 | F | Cl | H | CF₃ | CH₃ | OCH₂ CCl = CHCl |
| 1-485 | F | Cl | H | CF₃ | CH₃ | OCH(CH₃) CH =CH₂ |
| 1-486 | F | Cl | H | CF₃ | CH₃ | OCH₂ C ≡ CH |
| 1-487 | F | Cl | H | CF₃ | CH₃ | OCH(CH ₃)C≡CH |
| 1-488 | F | Cl | H | CF₃ | CH₃ | OCH₂ C ≡CBr |
| 1-489 | F | Cl | H | CF₃ | CH₃ | OCH₂ C ≡CCl |
| 1-490 | F | Cl | H | CF₃ | CH₃ | OCH₂ C ≡ CCH ₂Cl |
| 1-491 | F | Cl | H | CF₃ | CH₃ | OCH₂ CN |
| 1-492 | F | Cl | H | CF₃ | CH₃ | OCH₂ OCH₃ |
| 1-493 | F | Cl | H | CF₃ | CH₃ | OCH₂ OC₂ H₅ |
| 1-494 | F | Cl | H | CF₃ | CH₃ | OCH₂ SCH ₃ |
| 1-495 | F | Cl | H | CF₃ | CH₃ | OCH₂ COOCH ₃ |
| 1-496 | F | Cl | H | CF₃ | CH₃ | OCH₂ COOC₂ H₅ |
| 1-497 | F | Cl | H | CF₃ | CH₃ | OCH₂ COO ⁿ C ₂ H ₇ |
| 1-498 | F | Cl | H | CF₃ | CH₃ | OCH₂ COO ⁿ C ₄ H ₉ |
| 1-499 | F | Cl | H | CF₃ | CH₃ | OCH₂ COO ⁿ C ₅ H ₁₁ |
| 1-500 | F | Cl | H | CF₃ | CH₃ | OCH₂ COO ⁱ C ₃ H ₇ |
| 1-501 | F | Cl | H | CF₃ | CH₃ | OCH₂ COO ^{c} C ₅ H ₉ |
| 1-502 | F | Cl | H | CF₃ | CH₃ | OCH₂ COO ^{c} C ₆ H ₁₁ |
| 1-503 | F | Cl | H | CF₃ | CH₃ | OCH(CH ₃ )COOCH ₃ |
| 1-504 | F | Cl | H | CF₃ | CH₃ | OCN(CH ₃ )COOC ₂ H ₅ |
| 1-505 | F | Cl | H | CF₃ | CH₃ | OCH(CH ₃ )COO C₃ H ₇ |
| 1-506 | F | Cl | H | CF₃ | CH₃ | OCH(CH ₃ )COO ⁿ C₄ H ₉ |
| 1-507 | F | Cl | H | CF₃ | CH₃ | OCH(CH ₃ )COO ⁿ C₅ H ₁₁ |
| 1-508 | F | Cl | H | CF₃ | CH₃ | OCH(CH ₃ ) COO ⁱ C₃ H ₇ |
| 1-509 | F | Cl | H | CF₃ | CH₃ | OCH(CH ₃ )COO ^{c} C₅ H ₉ |
| 1-510 | F | Cl | H | CF₃ | CH₃ | OCH(CH ₃ )COO ^{c} C₆ H ₁₁ |
| 1-511 | F | Cl | H | CF₃ | CH₃ | OCH₂ CON(CH₃ ) ₂ |
| 1-5I2 | F | Cl | H | CF₃ | CH₃ | OCH₂ CON(C ₂ H ₅ ) ₂ |
| 1-513 | F | Cl | H | CF₃ | CH₃ | OCH₂ CON(CH₃) C ₂ H ₅ |
| 1-514 | F | Cl | H | CF₃ | CH₃ | OCH(CH ₃ )CON(CH ₃ ) ₂ |
| 1-515 | F | Cl | H | CF₃ | CH₃ | OCH(CH₃ )CON(C ₂ H ₅ ) ₂ |
| 1-516 | F | Cl | H | CF₃ | CH₃ | OCH(CH₃ )CON(CH ₃ )C₂ H ₅ |
| 1-517 | F | Cl | H | CF₃ | CH₃ | OCH ₂ COON(CH ₃ ) ₂ |
| 1-518 | F | Cl | H | CF₃ | CH₃ | OCH ₂ COON(C ₂ H ₅ ) ₂ |
| 1-519 | F | Cl | H | CF₃ | CH₃ | OCH(CH₃ )COON(CH₃ ) ₂ |
| 1-520 | F | Cl | H | CF₃ | CH₃ | OCH(CH₃ )COON(C ₂ H ₅ ) ₂ |
| 1-521 | H | F | H | CF₃ | CH₃ | SH |
| 1-522 | H | Cl | H | CF₃ | CH₃ | SH |
| 1-523 | H | Br | H | CF₃ | CH₃ | SH |
| 1-524 | F | F | H | CF₃ | CH₃ | SH |
| 1-525 | F | Cl | H | CF₃ | CH₃ | SH |
| 1-526 | F | Br | H | CF₃ | CH₃ | SH |
| 1-527 | H | Cl | H | CF₃ | CH₃ | SCH ₃ |
| 1-528 | H | Cl | H | CF₃ | CH₃ | SC₂ H ₅ |
| 1-529 | H | Cl | H | CF₃ | CH₃ | Sⁱ C ₃ H ₇ |
| 1-530 | H | Cl | H | CF₃ | CH₃ | SCH₂ CH₂ Cl |
| 1-531 | H | Cl | H | CF₃ | CH₃ | S^{c} C ₅ H ₈ |
| 1-532 | H | Cl | H | CF₃ | CH₃ | S^{c} C ₆ H ₁₁ |
| 1-533 | H | Cl | H | CF₃ | CH₃ | SCH₂ CH=CH₂ |
| 1-534 | H | Cl | H | CF₃ | CH₃ | SCH₂ CCl = CH₂ |
| 1-535 | H | Cl | H | CF₃ | CH₃ | SCH₂ CCI = CHCl |
| 1-536 | H | Cl | H | CF₃ | CH₃ | SCH(CH ₃)CH = CH₂ |
| 1-537 | H | Cl | H | CF₃ | CH₃ | SCH₂ C =CH |
| 1-538 | H | Cl | H | CF₃ | CH₃ | SCH(CH ₃)C=CH |
| 1-539 | H | Cl | H | CF₃ | CH₃ | SCH₂ COOCH ₃ |
| 1-540 | H | Cl | H | CF₃ | CH₃ | SCH₂ COOC₂ H⁻₅ |
| 1-541 | H | Cl | H | CF₃ | CH₃ | SCH₂ COO ⁿ C ₃ H ₇ |
| 1-542 | H | Cl | H | CF₃ | CH₃ | SCH₂ COO ⁿ C ₄ H ₉ |
| 1-543 | H | Cl | H | CF₃ | CH₃ | SCH₂ COO ⁿ C ₅ H ₁₁ |
| 1-544 | H | Cl | H | CF₃ | CH₃ | SCH₂ COO ⁱ C ₃ H ₇ |
| 1-545 | H | Cl | H | CF₃ | CH₃ | SCH₂ COO ^{c} C ₅ H ₉ |
| 1-546 | H | Cl | H | CF₃ | CH₃ | SCH₂ COO ^{c} C ₆ H ₁₁ |
| 1-547 | H | Cl | H | CF ₃ | CH₃ | SCH(CH ₃ )COOCH ₃ |
| 1-548 | H | Cl | H | CF₃ | CH₃ | SCH(CH ₃ )COOC ₂ H ₅ |
| 1-549 | H | Cl | H | CF₃ | CH₃ | SCH(CH ₃ )COO ⁿ C₃ H ₇ |
| 1-550 | H | Cl | H | CF₃ | CH₃ | SCH(CH ₃ )COO ⁿ C₄ H ₉ |
| 1-551 | H | Cl | H | CF₃ | CH₃ | SCH(CH ₃ )COO ⁿ C₅ H ₁₁ |
| 1-552 | H | Cl | H | CF₃ | CH₃ | SCH(CH ₃ )COO ⁱ C₃ H ₇ |
| 1-553 | H | Cl | H | CF₃ | CH₃ | SCH(CH ₃)COO ^{c} C₅ H ₉ |
| 1-554 | H | Cl | H | CF₃ | CH₃ | SCH(CH ₃)COO ^{c} C₆ H₁₁ |
| 1-555 | H | Cl | H | CF₃ | CH₃ | SCH₂ CON(CH₃)₂ |
| 1-556 | H | Cl | H | CF₃ | CH₃ | SCH₂ CON(C ₂ H ₅ ) ₂ |
| 1-557 | H | Cl | H | CF₃ | CH₃ | SCH₂CON (tetramethylene) |
| 1-558 | H | Cl | H | CF₃ | CH₃ | SCH₂CON (pentamethylene) |
| 1-559 | H | Cl | H | CF₃ | CH₃ | SCH₂CON (ethyleneoxyethylene) |
| 1-560 | H | Cl | H | CF₃ | CH₃ | SCH(CH₃ )CON(CH₃)₂ |
| 1-561 | H | Cl | H | CF₃ | CH₃ | SCH(CH₃ )CON(C₂ H₅)₂ |
| 1-562 | H | Cl | H | CF₃ | CH₃ | SCH(CH₃)CON (tetramethylene) |
| 1-563 | H | Cl | H | CF₃ | CH₃ | SCH(CH₃)CON (pentamethylene) |
| 1-564 | F | Cl | H | CF₃ | CH₃ | SCH ₃ |
| 1-565 | F | Cl | H | CF₃ | CH₃ | SC₂ H s |
| 1-566 | F | Cl | H | CF₃ | CH₃ | S' C ₃ H₇ |
| 1-567 | F | Cl | H | CF₃ | CH₃ | SCH₂ CH₂ Cl |
| 1-568 | F | Cl | H | CF₃ | CH₃ | S^{c} C₅ H₉ |
| 1-569 | F | Cl | H | CF₃ | CH₃ | S^{c} C₆ H₁₁ |
| 1-570 | F | Cl | H | CF₃ | CH₃ | SCH₂ CH=CH₂ |
| 1-571 | F | Cl | H | CF₃ | CH₃ | SCH₂ CCl = CH₂ |
| 1-572 | F | Cl. | H | CF₃ | CH₃ | SCH₂ CCl =CHCl |
| 1-573 | F | Cl | H | CF₃ | CH₃ | SCH(CH₃)CH =CH₂ |
| 1-574 | F | Cl | H | CF₃ | CH₃ | SCH₂ C ≡ CH |
| 1-575 | F | Cl | H | CF₃ | CH₃ | SCH(CH₃ )C≡CH |
| 1-576 | F | Cl | H | CF₃ | CH₃ | SCH₂ COOCH ₃ |
| 1-577 | F | Cl | H | CF₃ | CH₃ | SCH₂ COOC₂ H s |
| 1-578 | F | Cl | H | CF₃ | CH₃ | SCH₂ COO ⁿ C₃ H₇ |
| 1-579 | F | Cl | H | CF₃ | CH₃ | SCH₂ COO ⁿ C₄ H₉ |
| 1-580 | F | Cl | H | CF₃ | CH₃ | SCH₂ COO ⁿ C ₅ H₁₁ |
| 1-581 | F | Cl | H | CF₃ | CH₃ | SCH₂ COO ⁱ C₃ H₇ |
| 1-582 | F | Cl | H | CF₃ | CH₃ | SCH₂ COO ^{c} C ₅ H ₉ |
| 1-583 | F | Cl | H | CF₃ | CH₃ | SCH₂ COO ^{c} C₆ H₁₁ |
| 1-584 | F | Cl | H | CF₃ | CH₃ | SCH(CH₃)COOCH₃ |
| 1-585 | F | Cl | H | CF₃ | CH₃ | SCH(CH₃ )COOC₂ H ₅ |
| 1-586 | F | Cl | H | CF₃ | CH₃ | SCH(CH₃ )COOⁿ C₃ H ₇ |
| 1-587 | F | Cl | H | CF₃ | CH₃ | SCH(CH₃ )COO ⁿ C₄ H ₉ |
| 1-588 | F | Cl | H | CF₃ | CH₃ | SCH(CH₃ )COO ⁿ C₅ H₁₁ |
| 1-589 | F | Cl | H | CF₃ | CH₃ | SCH(CH₃ )COOⁱ C₃ H ₇ |
| 1-590 | F | Cl | H | CF₃ | CH₃ | SCH(CH₃ )COO ^{c}C₅ H ₉ |
| 1-591 | F | Cl | H | CF₃ | CH₃ | SCH(CH₃ )COO ^{c} C₆ H₁₁ |
| 1-592 | F | Cl | H | CF₃ | CH₃ | SCH₂ CON(CH₃)₂ |
| 1-593 | F | Cl | H | CF₃ | CH₃ | SCH₂ CON(C₂ H ₅) ₂ |
| 1-599 | F | Cl | H | CF₃ | CH₃ | SCH₂CON (tetramethylene) |
| 1-595 | F | Cl | H | CF₃ | CH₃ | SCH₂CON (pentamethylene) |
| 1-596 | F | Cl | H | CF₃ | CH₃ | SCH₂CON (ethyleneoxyethylene) |
| 1-597 | F | Cl | H | CF₃ | CH₃ | SCH(CH₃ )CON(CH₃) ₂ |
| 1-598 | F | Cl | H | CF₃ | CH₃ | SCH(CH₃ )CON(C₂ H₅) ₂ |
| 1-599 | F | Cl | H | CF₃ | CH₃ | SCH(CH₃)CON (tetramethylene) |
| 1-600 | F | Cl | H | CF₃ | CH₃ | SCH(CH₃)CON (pentamethylene) |
| 1-601 | H | F | H | CF₃ | CH₃ | SO₂ Cl |
| 1-602 | H | Cl | H | CF₃ | CH₃ | SO₂ Cl |
| 1-603 | H | Br | H | CF₃ | CH₃ | SO₂ Cl |
| 1-604 | F | F | H | CF₃ | CH₃ | SO₂ Cl |
| 1-605 | F | Cl | H | CF₃ | CH₃ | SO₂ Cl |
| 1-606 | F | Br | H | CF₃ | CH₃ | SO₂ Cl |
| 1-607 | H | Cl | H | CF₃ | CH₃ | SO₂ OCH ₃ |
| 1-608 | H | Cl | H | CF₃ | CH₃ | SO₂ OC₂ H ₅ |
| 1-609 | H | Cl | H | CF₃ | CH₃ | SO₂ O ⁱ C₃ H₇ |
| 1-610 | H | Cl | H | CF₃ | CH₃ | SO₂ OCH ₂ CH=CH₂ |
| 1-611 | F | Cl | H | CF₃ | CH₃ | SO₂ OCH ₃ |
| 1-612 | F | Cl | H | CF₃ | CH₃ | SO₂ OC₂ H ₅ |
| 1-613 | F | Cl | H | CF₃ | CH₃ | SO₂ O ⁱ C ₃ H ₇ |
| 1-614 | F | Cl | H | CF₃ | CH₃ | SO₂ OCH ₂ CH=CH₂ |
| 1-615 | H | Cl | H | CF₃ | CH₃ | SO₂ N(CH₃ ) ₂ |
| 1-616 | H | Cl | H | CF₃ | CH₃ | SO₂ N (C₂ H ₅ ) ₂ |
| 1-617 | F | Cl | H | CF₃ | CH₃ | SO₂ N (CH ₃ ) ₂ |
| 1-618 | F | Cl | H | CF₃ | CH₃ | SO₂ N (C₂ H ₅ ) ₂ |
| 1-619 | H | Cl | H | CF₃ | CH₃ | COOH |
| 1-620 | H | Cl | H | CF₃ | CH₃ | COOCH ₃ |
| 1-621 | H | Cl | H | CF₃ | CH₃ | COOC ₂ H ₅ |
| 1-622 | H | Cl | H | CF₃ | CH₃ | COOⁿ C ₃ H ₇ |
| 1-623 | H | Cl | H | CF₃ | CH₃ | COOⁿ C ₄ H ₉ |
| 1-624 | H | Cl | H | CF₃ | CH₃ | COOⁿ C s H ₁₁ |
| 1-625 | H | Cl | H | CF₃ | CH₃ | COO' C ₃ H ₇ |
| 1-626 | H | Cl | H | CF₃ | CH₃ | COOCH₂ CH₂ Cl |
| 1-627 | H | Cl | H | CF₃ | CH₃ | COOCH₂ CH₂ Br |
| 1-628 | H | Cl | H | CF₃ | CH₃ | CON(CH ₃ ) ₂ |
| 1-629 | H | Cl | H | CF₃ | CH₃ | CONHCH₃ |
| 1-630 | H | Cl | H | CF₃ | CH₃ | CON(C₂ H ₅ ) ₂ |
| 1-631 | H | Cl | H | CF₃ | CH₃ | CONHC₂ H ₅ |
| 1-632 | H | Cl | H | CF₃ | CH₃ | COCH , ₃ |
| 1-633 | H | Cl | H | CF₃ | CH₃ | COC₂ H s |
| 1-634 | H | Cl | H | CF₃ | CH₃ | COCH₂ Cl |
| 1-635 | H | Cl | H | CF₃ | CH₃ | CHO |
| 1-636 | H | Cl | H | CF₃ | CH₃ | CH = CHCOOCH₃ |
| 1-637 | H | Cl | H | CF₃ | CH₃ | CH=CHCOOC₂ x H ₅ |
| 1-638 | H | Cl | H | CF₃ | CH₃ | CH₂ CH₂ COOCH₃ |
| 1-639 | H | Cl | H | CF₃ | CH₃ | CH₂ CH₂ COOC₂ H₆ |
| 1-640 | F | Cl | H | CF₃ | CH₃ | COOH |
| 1-641 | F | Cl | H | CF₃ | CH₃ | COOCH₃ |
| 1-642 | F | Cl | H | CF₃ | CH₃ | COOC₂ H ₅ |
| 1-643 | F | Cl | H | CF₃ | CH₃ | COOⁿ C₃ H₇ |
| 1-644 | F | Cl | H | CF₃ | CH₃ | COOⁿ C₄ H₉ |
| 1-645 | F | Cl | H | CF₃ | CH₃ | COOⁿ C₅ H₁₁ |
| 1-646 | F | Cl | H | CF₃ | CH₃ | COOⁱ C₃ H₇ |
| 1-647 | F | Cl | H | CF₃ | CH₃ | COOCH₂ CH₂ Cl |
| 1-648 | F | Cl | H | CF₃ | CH₃ | COOCH₂ CH₂ Br |
| 1-649 | F | Cl | H | CF₃ | CH₃ | CON(CH₃)₂ |
| 1-650 | F | Cl | H | CF₃ | CH₃ | CONHCH₃ |
| 1-651 | F | Cl | H | CF₃ | CH₃ | CON(C₂ H ₅ ) ₂ |
| 1-652 | F | Cl | H | CF₃ | CH₃ | CONHC₂ H ₅ |
| 1-653 | F | Cl | H | CF₃ | CH₃ | COCH ₃ |
| 1-654 | F | Cl | H | CF₃ | CH₃ | COC₂ H ₅ |
| 1-655 | F | Cl | H | CF₃ | CH₃ | COCH₂ Cl |
| 1-656 | F | Cl | H | CF₃ | CH₃ | CHO |
| 1-657 | F | Cl | H | CF₃ | CH₃ | CH = CHCOOCH ₃ |
| 1-658 | F | Cl | H | CF₃ | CH₃ | CH=CHCOOC ₂ H ₅ |
| 1-659 | F | Cl | H | CF₃ | CH₃ | CH₂ CH₂ COOCH ₃ |
| 1-660 | F | Cl | H | CF₂ Cl | CH₃ | CH₂ CH₂ COOC₂ H ₅ |
| 1-661 | Cl | Cl | H | CF₂ Cl | H | H |
| 1-662 | H | F | H | CF₂ Cl | H | NO₂ |
| 1-663 | H | Cl | H | CF₂ Cl | H | NO₂ |
| 1-664 | H | Br | H | CF₂ Cl | H | NO₂ |
| 1-665 | F | F | H | CF₂ Cl | H | NO₂ |
| 1-666 | F | Cl | H | CF₂ Cl | H | NO₂ |
| 1-667 | F | Br | H | CF₂ Cl | H | NO₂ |
| 1-668 | H | F | H | CF₂ Cl | H | NH₂ |
| 1-669 | H | Cl | H | CF₂ Cl | H | NH₂ |
| 1-670 | H | Br | H | CF₂ Cl | H | NH₂ |
| 1-671 | F | F | H | CF₂ Cl | H | NH₂ |
| 1-672 | F | Cl | H | CF₂ Cl | H | NH₂ |
| 1-673 | F | Br | H | CF₂ Cl | H | NH₂ |
| 1-674 | H | F | H | CF₂ Cl | H | OH |
| 1-675 | H | Cl | H | CF₂ Cl | H | OH |
| 1-676 | H | Br | H | CF₂ Cl | H | OH |
| 1-677 | F | F | H | CF₂ Cl | H | OH |
| 1-678 | F | Cl | H | CF₂ Cl | H | OH |
| 1-679 | F | Br | H | CF₂ Cl | H | OH |
| 1-680 | H | Cl | H | CF₂ Cl | H | NHCH₃ |
| 1-681 | H | Cl | H | CF₂ Cl | H | NHC₂ H ₅ |
| 1-682 | H | Cl | H | CF₂ Cl | H | NHCH₂ CH=CH₂ |
| 1-683 | H | Cl | H | CF₂ Cl | H | NHCH₂ C ≡ CH |
| 1-684 | H | Cl | H | CF₂ Cl | H | NHCH(CH ₃)C ≡ CH |
| 1-685 | H | Cl | H | CF₂ Cl | H | NHSO₂ CH₃ |
| 1-686 | H | Cl | H | CF₂ Cl | H | NHSO₂ C ₂ H ₅ |
| 1-687 | H | Cl | H | CF₂ Cl | H | NHSO₂ CH₂ Cl |
| 1-688 | H | Cl | H | CF₂ Cl | H | NHSO₂ CF₃ |
| 1-689 | H | Cl | H | CF₂ Cl | H | N(CH₃ )SO ₂ CH₃ |
| 1-690 | H | Cl | H | CF₂ Cl | H | N(CH₂ C ≡ CH)SO ₂ CH₃ |
| 1-691 | H | Cl | H | CF₂ Cl | H | NHCOOCH₃ |
| 1-692 | H | Cl | H | CF₂ Cl | H | NHCOOC₂ H ₅ |
| 1-693 | H | Cl | H | CF₂ Cl | H | NHCOO ⁿ C ₃ H ₇ |
| 1-694 | H | Cl | H | CF₂ Cl | H | NHCOO ⁱ C ₃ H ₇ |
| 1-695 | H | Cl | H | CF₂ Cl | H | NHCOO ⁿ C₄ H ₉ |
| 1-696 | H | Cl | H | CF₂ Cl | H | NHCOO ⁿ C ₅ H ₁₁ |
| 1-697 | H | Cl | H | CF₂ Cl | H | NHCH₂ COOCH ₃ |
| 1-698 | H | Cl | H | CF₂ Cl | H | NHCH₂ COOC₂ H ₅ |
| 1-699 | H | Cl | H | CF₂ Cl | H | NHCH₂ COO ⁿ C ₃ H ₇ |
| 1-700 | H | Cl | H | CF₂ Cl | H | NHCH₂ COO ⁿ C ₄ H ₉ |
| 1-701 | H | Cl | H | CF₂ Cl | H | NHCH₂ COO ⁿ C ₅ H ₁₁ |
| 1-702 | H | Cl | H | CF₂ Cl | H | NHCH₂ COO ⁱ C ₃ H ₇ |
| 1-703 | H | Cl | H | CF₂ Cl | H | NHCH₂ COO ^{c} C ₅ H ₉ |
| 1-704 | H | Cl | H | CF₂ Cl | H | NHCH₂ COO ^{c} C ₆ H ₁₁ |
| 1-705 | H | Cl | H | CF₂ Cl | H | NHCH(CH ₃ )COOCH ₃ |
| 1-706 | H | Cl | H | CF₂ Cl | H | NHCH(CH₃ )COOC₂ H₅ |
| 1-707 | H | Cl | H | CF₂ Cl | H | NHCH(CH₃ )COO ⁿ C₃ H ₇ |
| 1-708 | H | Cl | H | CF₂ Cl | H | NHCH(CH₃ )COO ⁿ C₄ H ₉ |
| 1-709 | H | Cl | H | CF₂ Cl | H | NHCH(CH₃ )COO ⁿ C₆ H ₁₁ |
| 1-710 | H | Cl | H | CF₂ Cl | H | NHCH(CH₃)COO³ C₃ H ₇ |
| 1-711 | H | Cl | H | CF₂ Cl | H | NHCH(CH₃ )COO ^{c} C₅ H ₉ |
| 1-712 | H | Cl | H | CF₂ Cl | H | NHCH(CH₃ )COO ^{c} C₆ H ₁₁ |
| 1-713 | F | Cl | H | CF₂ Cl | H | NHCH₃ |
| 1-714 | F | Cl | H | CF₂ Cl | H | NHC ₂ H₅ |
| 1-715 | F | Cl | H | CFz Cl | H | NHCH₂ CH=CH₂ |
| 1-716 | F | Cl | H | CF₂ Cl | H | NHCH₂ C ≡⁻CH |
| 1-717 | F | Cl | H | CF₂ Cl | H | NHCH(CH ₃ )C≡CH |
| 1-718 | F | Cl | H | CF₂ Cl | H | NHSO₂ CH₃ |
| 1-719 | F | Cl | H | CF₂ Cl | H | NHSO₂ C ₂ H ₅ |
| 1-720 | F | Cl | H | CF₂ Cl | H | NHSO₂ CH₂ Cl |
| 1-721 | F | Cl | H | CF₂ Cl | H | NHSO₂ CF₃ |
| 1-722 | F | Cl | H | CF₂ Cl | H | N(CH₃ )SO ₂ CH₃ |
| 1-723 | F | Cl | H | CF₂ Cl | H | N(CH₂ C ≡CH)SO ₂ CH₃ |
| 1-724 | F | Cl | H | CF₂ Cl | H | NHCOOCH ₃ |
| 1-725 | F | Cl | H | CF₂ Cl | H | NHCOOC₂ H ₅ |
| 1-726 | F | Cl | H | CF₂ Cl | H | NHCOO ⁿ C ₃ H₇ |
| 1-727 | F | Cl | H | CF₂ Cl | H | NHCOO ⁱ C ₃ H ₇ |
| 1-728 | F | Cl | H | CF₂ Cl | H | HCOO ⁿ C ₄ H ₉ |
| 1-729 | F | Cl | H | CF₂ Cl | H | NHCOOⁿ C₅ H₁₁ |
| 1-730 | F | Cl | H | CF₂ Cl | H | NHCH₂ COOCH ₃ |
| 1-731 | F | Cl | H | CF₂ Cl | H | NHCH₂ COOC₂ H ₅ |
| 1-732 | F | Cl | H | CF₂ Cl | H | NHCH₂ COO ⁿ C₃H₇ |
| 1-733 | F | Cl | H | CF₂ Cl | H | NNCH₂ COO ⁿ C₄ H ₉ |
| 1-734 | F | Cl | H | CF₂ Cl | H | NHCH₂ COO ⁿ C ₅ H₁₁ |
| 1-735 | F | Cl | H | CF₂ Cl | H | NHCH₂ COO ⁱ C ₃ H ₇ |
| 1-736 | F | Cl | H | CF₂ Cl | H | NHCH₂ COO ^{c} C₅ H₉ |
| 1-737 | F | Cl | H | CF₂ Cl | H | NH₂ CH₂ COO ^{c} C ₆ H ₁₁ |
| 1-738 | F | Cl | H | CF₂ Cl | H | NHCH(CH₃ )COOCH ₃ |
| 1-739 | F | Cl | H | CF₂ Cl | H | NHCH(CH₃ )COOC ₂ H ₅ |
| 1-740 | F | Cl | H | CF₂ Cl | H | NHCH(CH₃ )COO ⁿ C₃ H ₇ |
| 1-741 | F | Cl | H | CF₂ Cl | H | NHCH(CH₃ )COO ⁿ C₄ H ₉ |
| 1-742 | F | Cl | H | CF₂ Cl | H | NHCH(CH₃ )COO ⁿ C₅ H₁₁ |
| 1-743 | F | Cl | H | CF₂ Cl | H | NHCH(CH₃ )COO ⁱ C₃ H₇ |
| 1-744 | F | Cl | H | CF₂ Cl | H | NHCH(CH₃ )COO ^{c} C₅ H₉ |
| 1-745 | F | Cl | H | CF₂ Cl | H | NHCH(CH₃ )COO ^{c} C₆ H₁₁ |
| 1-746 | H | Cl | H | CF₂ Cl | H | OCH₃ |
| 1-747 | H | Cl | H | CF₂ Cl | H | OC₂ H₅ |
| 1-748 | H | Cl | H | CF₂ Cl | H | Oⁱ C₃ H₇ |
| 1-749 | H | Cl | H | CF₂ Cl | H | Oⁿ C₃ H₇ |
| 1-750 | H | Cl | H | CF₂ Cl | H | OCH₂ CH₂ Cl |
| 1-751 | H | Cl | H | CF₂ Cl | H | OCF₂ CF₂ H |
| 1-752 | H | Cl | H | CF₂ Cl | H | O^{c} C ₅ H ₉ |
| 1-753 | H | Cl | H | CF₂ Cl | H | O^{c} C ₆ H₁₁ |
| 1-754 | H | Cl | H | CF₂ Cl | H | OCH₂ CH=CH₂ |
| 1-755 | H | Cl | H | CF₂ Cl | H | OCH₂ CCl =CH₂ |
| 1-756 | H | Cl | H | CF₂ Cl | H | OCH₂ CCl = CHCl |
| 1-757 | H | Cl | H | CF₂ Cl | H | OCH(CH₃ )CH =CH₂ |
| 1-758 | H | Cl | H | CF₂ Cl | H | OCH₂ C ≡CH |
| 1-759 | H | Cl | H | CF₂ Cl | H | OCH(CH₃ )C≡CH |
| 1-760 | H | Cl | H | CF₂ Cl | H | OCH₂ C ≡CBr |
| 1-761 | H | Cl | H | CF₂ Cl | H | OCH₂ C ≡ CCl |
| 1-762 | H | Cl | H | CF₂ Cl | H | OCH₂ C ≡CCH ₂ Cl |
| 1-763 | H | Cl | H | CF₂ Cl | H | OCH₂ CN |
| 1-764 | H | Cl | H | CF₂ Cl | H | OCH₂ OCH ₃ |
| 1-765 | H | Cl | H | CF₂ Cl | H | OCH₂ OC₂ H ₅ |
| 1-766 | H | Cl | H | CF₂ Cl | H | OCH₂ SCH ₃ |
| 1-767 | H | Cl | H | CF₂ Cl | H | OCH₂ COOCH ₃ |
| 1-768 | H | Cl | H | CF₂ Cl | H | OCH₂ COOC₂ H ₅ |
| 1-769 | H | Cl | H | CF₂ Cl | H | OCH₂ COO ⁿ C₃ H₇ |
| 1-770 | H | Cl | H | CF₂ Cl | H | OCH₂ COO ⁿ C H ₉ |
| 1-771 | H | Cl | H | CF₂ Cl | H | OCH₂ COO ⁿ C ₅ H₁₁ |
| 1-772 | H | Cl | H | CF₂ Cl | H | OCH₂ COO ⁱ C₃ H₇ |
| 1-773 | H | Cl | H | CF₂ Cl | H | OCH₂COO ^{c} C₅ H₉ |
| 1-774 | H | Cl | H | CF₂ Cl | H | OCH₂ COO ^{c} C ₆ H₁₁ |
| 1-775 | H | Cl | H | CF₂ Cl | H | OCH(CH ₃ )COOCH ₂ |
| 1-776 | H | Cl | H | CF₂ Cl | H | OCH(CH ₃ )COOC ₂ H ₅ |
| 1-777 | H | Cl | H | CF₂ Cl | H | OCH(OH ₃ )COO ⁿ C₃ H ₇ |
| 1-778 | H | Cl | H | CF₂ Cl | H | OCH(CH ₃ )COO ⁿ C₄ H ₉ |
| 1-779 | H | Cl | H | CF₂ Cl | H | OCH(CH ₃ )COO ⁿ C₅ H₁₁ |
| 1-780 | H | Cl | H | CF₂ Cl | H | OCH(CH₃ )COO ⁱ C₃ H₇ |
| 1-781 | H | Cl | H | CF₂ Cl | H | OCH(CH ₃ )COO ^{c} C₅ H₉ |
| 1-782 | H | Cl | H | CF₂ Cl | H | OCH(CH ₃ )COO^{c} C₆ H ₁₁ |
| 1-783 | H | Cl | H | CF₂ Cl | H | OCH₂ CON(CH₃ ) ₂ |
| 1-784 | H | Cl | H | CF₂ Cl | H | OCH₂ CON(C ₂H₅)₂ |
| 1-785 | H | Cl | H | CF₂ Cl | H | OCH₂ CON(CH₃) C ₂ H ₅ |
| 1-786 | H | Cl | H | CF₂ Cl | H | OCH (CH₃ )CON(CH₃) ₂ |
| 1-787 | H | Cl | H | CF₂ Cl | H | OCH(CH₃) CON(C₂ H₅)₂ |
| 1-788 | H | Cl | H | CF₂ Cl | H | OCH(CH₃) CONCCH₃) C₂ H₅ |
| 1-789 | H | Cl | H | CF₂ Cl | H | OCH₂ COON(CH₃)₂ |
| 1-790 | H | Cl | H | CF₂ Cl | H | OCH₂ COON (C₂ H₅)₂ |
| 1-791 | H | Cl | H | CF₂ Cl | H | OCH(CH₃) COON(CH₃)₂ |
| 1-792 | H | Cl | H | CF₂ Cl | H | OCH(CH₃) COON(C₂ H₅)₂ |
| 1-793 | F | Cl | H | CF₂ Cl | H | OCH₃ |
| 1-794 | F | Cl | H | CF₂ Cl | H | OC₂ H₅ |
| 1-795 | F | Cl | H | CF₂ Cl | H | Oⁱ C₃ H₇ |
| 1-796 | F | Cl | H | CF₂ Cl | H | Oⁿ C₃ H₇ |
| 1-797 | F | Cl | H | CF₂ Cl | H | OCH₂ CH₂ Cl |
| 1-798 | F | Cl | H | CF₂ Cl | H | OCF₂ CF₂ H |
| 1-799 | F | Cl | H | CF₂ Cl | H | O^{c} C ₅ H ₉ |
| 1-800 | F | Cl | H | CF₂ Cl | H | O^{c} C ₆ H₁₁ |
| 1-801 | F | Cl | H | CF₂ Cl | H | OCH₂ CH = CH₂ |
| 1-802 | F | Cl | H | CF₂ Cl | H | OCH₂ CCl = CH₂ |
| 1-803 | F | Cl | H | CF₂ Cl | H | OCH₂ CCl = CHCl |
| 1-804 | F | Cl | H | CF₂ Cl | H | OCH(CH ₃ )CH = CH₂ |
| 1-805 | F | Cl | H | CF₂ Cl | H | OCH₂ C ≡ CH |
| 1-806 | F | Cl | H | CF₂ Cl | H | OCH(CH ₃ )C ≡ CH |
| 1-807 | F | Cl | H | CF₂ Cl | H | OCH₂ C ≡ CBr |
| 1-808 | F | Cl | H | CF₂ Cl | H | OCH₂ C ≡ CCl |
| 1-809 | F | Cl | H | CF₂ Cl | H | OCH₂ C ≡ CCH ₂ Cl |
| 1-810 | F | Cl | H | CF₂ Cl | H | OCH₂ CN |
| 1-811 | F | Cl | H | CF₂ Cl | H | OCH₂ OCH ₃ |
| 1-812 | F | Cl | H | CF₂ Cl | H | OCH₂ OC₂ H ₅ |
| 1-813 | F | Cl | H | CF₂ Cl | H | OCH₂ SCH ₃ |
| 1-814 | F | Cl | H | CF₂ Cl | H | OCH₂ COOCH ₃ |
| 1-815 | F | Cl | H | CF₂ Cl | H | OCH ₂ COOC₂ H ₅ |
| 1-816 | F | Cl | H | CF₂ Cl | H | OCH₂ COO ⁿ C ₃ H ₇ |
| 1-817 | F | Cl | H | CF₂ Cl | H | OCH₂ COO ⁿ C₄ H ₉ |
| 1-818 | F | Cl | H | CF₂ Cl | H | OCH₂ COO ⁿ C ₅ H ₁₁ |
| 1-819 | F | Cl | H | CF₂ Cl | H | OCH₂ COO ⁱ C ₃ H ₇ |
| 1-820 | F | Cl | H | CF₂ Cl | H | OCH₂ COO ^{c} C ₅ H ₉ |
| 1-821 | F | Cl | H | CF₂ Cl | H | OCH₂ COO ^{c} C ₆ H ₁₁ |
| 1-822 | F | Cl | H | CF₂ Cl | H | OCH(CH ₃ )COOCH ₃ |
| 1-823 | F | Cl | H | CF₂ Cl | H | OCH(CH ₃ )COOC ₂ H ₅ |
| 1-824 | F | Cl | H | CF₂ Cl | H | OCH(CH ₃ )COO ⁿ C₃ H ₇ |
| 1-825 | F | Cl | H | CF₂ Cl | H | OCH(CH ₃ )COO ⁿ C₄ H ₉ |
| 1-826 | F | Cl | H | CF₂ Cl | H | OCH(CH ₃ )COO ⁿ C₅ H₁₁ |
| 1-827 | F | Cl | H | CF₂ Cl | H | OCH(CH ₃ )COO ⁱ C₃ H ₇ |
| 1-828 | F | Cl | H | CF₂ Cl | H | OCH(CH ₃ )COO ^{c} C₅ H ₉ |
| 1-829 | F | Cl | H | CF₂ Cl | H | OCH(CH ₃ )COO ^{c} C₆ H ₁₁ |
| 1-830 | F | Cl | H | CF₂ Cl | H | OCH₂ CON(CH₃ ) ₂ |
| 1-831 | F | Cl | H | CF₂ Cl | H | OCH₂ CON(C ₂ H ₅ ) ₂ |
| 1-832 | F | Cl | H | CF₂ Cl | H | OCH₂ CON(CH₃ ) C ₂ H ₅ |
| 1-833 | F | Cl | H | CF₂ Cl | H | OCH(CH₃)CON(CH₃)₂ |
| 1-834 | F | Cl | H | CF₂ Cl | H | OCH(CH₃ )CON(C ₂ H ₅ )₂ |
| 1-835 | F | Cl | H | CF₂ Cl | H | OCH(CH₃)CON(CH₃)C₂ H ₅ |
| 1-836 | F | Cl | H | CF₂ Cl | H | OCH ₂ COON(CH₃)₂ |
| 1-837 | F | Cl | H | CF₂ Cl | H | OCH ₂ COON(C ₂ H ₅ )₂ |
| 1-838 | F | Cl | H | CF₂ Cl | H | OCH(CH₃ )COON(CH₃)₂ |
| 1-839 | F | Cl | H | CF₂ Cl | H | OCH(CH₃ )COON(C₂H₅)₂ |
| 1-840 | H | F | H | CF₂ Cl | H | SH |
| 1-841 | H | Cl | H | CF₂ Cl | H | SH |
| 1-842 | H | Br | H | CF₂ Cl | H | SH |
| 1-843 | F | F | H | CF₂ Cl | H | SH |
| 1-844 | F | Cl | H | CF₂ Cl | H | SH |
| 1-845 | F | Br | H | CF₂ Cl | H | SH |
| 1-846 | H | Cl | H | CF₂ Cl | H | SCH ₃ |
| 1-847 | H | Cl | H | CF₂ Cl | H | SC₂ H ₅ |
| 1-848 | H | Cl | H | CF₂ Cl | H | Sⁱ C ₃ H₇ |
| 1-849 | H | Cl | H | CF₂ Cl | H | SCH₂ CH₂ Cl |
| 1-850 | H | Cl | H | CF₂ Cl | H | S^{c} C ₅ H ₉ |
| 1-851 | H | Cl | H | CF₂ Cl | H | S^{c} C ₆ H ₁₁ |
| 1-852 | H | Cl | H | CF₂ Cl | H | SCH₂ CH=CH₂ |
| 1-853 | H | Cl | H | CF₂ Cl | H | SCH₂ CCl = CH₂ |
| 1-854 | H | Cl | H | CF₂ Cl | H | SCH₂ CCl =CHCl |
| 1-855 | H | Cl | H | CF₂ Cl | H | SCH(CH ₃)CH =CH₂ |
| 1-856 | H | Cl | H | CF₂ Cl | H | SCH₂ C ≡ CH |
| 1-857 | H | Cl | H | CF₂ Cl | H | SCH(CH₃ )C≡CH |
| 1-858 | H | Cl | H | CF₂ Cl | H | SCH₂ COOCH ₃ |
| 1-859 | H | Cl | H | CF₂ Cl | H | SCH₂ COOC₂ H ₅ |
| 1-860 | H | Cl | H | CF₂ Cl | H | SCH₂ COO ⁿ C ₃H₇ |
| 1-861 | H | Cl | H | CF₂ Cl | H | SCH₂ COO ⁿ C₄ H₉ |
| 1-862 | H | Cl | H | CF₂ Cl | H | SCH₂ COO ⁿ C ₅ H₁₁ |
| 1-863 | H | Cl | H | CF₂ Cl | H | SCH₂ COO ⁱ C₃ H ₇ |
| 1-864 | H | Cl | H | CF₂ Cl | H | SCH₂ COO ^{c} C₅ H₉ |
| 1-865 | H | Cl | H | CF₂ Cl | H | SCH₂ COO ^{c} C ₆ H₁₁ |
| 1-866 | H | Cl | H | CF₂ Cl | H | SCH(CH ₃ )COOCH ₃ |
| 1-867 | H | Cl | H | CF₂ Cl | H | SCH(CH ₃ )COOC ₂ H ₅ |
| 1-868 | H | Cl | H | CF₂ Cl | H | SCH(CH ₃ )COO ⁿ C₃ H ₇ |
| 1-869 | H | Cl | H | CF₂ Cl | H | SCH(CH ₃)COO ⁿ C₄ H ₉ |
| 1-870 | H | Cl | H | CF₂ Cl | H | SCH(CH ₃ )COO ⁿ C₅ H ₁₁ |
| 1-871 | H | Cl | H | CF₂ Cl | H | SCH(CH ₃ )COO ⁱ C₃ H ₇ |
| 1-872 | H | Cl | H | CF₂ Cl | H | SCH(CH ₃ )COO ^{c} C₅ H ₉ |
| 1-873 | H | Cl | H | CF₂ Cl | H | SCH(CH ₃ )COO ^{c} C₆ H ₁₁ |
| 1-874 | H | Cl | H | CF₂ Cl | H | SCH₂ CON(CH₃ ) ₂ |
| 1-875 | H | Cl | H | CF₂ Cl | H | SCH₂ CON(C ₂ H ₅ ) ₂ |
| 1-876 | H | Cl | H | CF₂ Cl | H | SCH₂CON (tetramethylene) |
| 1-877 | H | Cl | H | CF₂ Cl | H | SCH₂CON (pentamethylene) |
| 1-878 | H | Cl | H | CF₂ Cl | H | SCH₂CON (ethyleneoxyethylene) |
| 1-879 | H | Cl | H | CF₂ Cl | H | SCH(CH₃ )CON(CH ₃ ) ₂ |
| 1-880 | H | Cl | H | CF₂ Cl | H | SCH(CH₃ )CON(C₂ H ₅ ) ₂ |
| 1-881 | H | Cl | H | CF₂ Cl | H | SCH(CH₃)CON (tetramethylene) |
| 1-882 | H | Cl | H | CF₂ Cl | H | SCH( CH₃)CON (pentamethylene) |
| 1-883 | F | Cl | H | CF₂ Cl | H | SCH ₃ |
| 1-884 | F | Cl | H | CF₂ Cl | H | SC₂ H₅ |
| 1-885 | F | Cl | H | CF₂ Cl | H | Sⁱ C₃ H₇ |
| 1-886 | F | Cl | H | CF₂ Cl | H | SCH₂ CH₂ Cl |
| 1-887 | F | Cl | H | CF₂ Cl | H | S^{c} C₅ H₉ |
| 1-888 | F | Cl | H | CF₂ Cl | H | S^{c} C₆H₁₁ |
| 1-889 | F | Cl | H | CF₂ Cl | H | SCH₂ CH=CH₂ |
| 1-890 | F | Cl | H | CF₂ Cl | H | SCH₂ CCl = CH₂ |
| 1-891 | F | Cl | H | CF₂ Cl | H | SCH₂ CCl = CHCl |
| 1-892 | F | Cl | H | CF₂ Cl | H | SCH(CH₃)CH = CH₂ |
| 1-893 | F | Cl | H | CF₂ Cl | H | SCH₂ C ≡ CH |
| 1-894 | F | Cl | H | CF₂ Cl | H | SCH(CH₃)C≡CH |
| 1-895 | F | Cl | H | CF₂ Cl | H | SCH₂ COOCH₃ |
| 1-896 | F | Cl | H | CF₂ Cl | H | SCH₂ COOC₂ H₅ |
| 1-897 | F | Cl | H | CF₂ Cl | H | SCH₂ COO ⁿ C ₃ H ₇ |
| 1-898 | F | Cl | H | CF₂ Cl | H | SCH₂ COO ⁿ C ₄ H ₉ |
| 1-899 | F | Cl | H | CF₂ Cl | H | SCH₂ COO ⁿ C ₅ H ₁₁ |
| 1-900 | F | Cl | H | CF₂ Cl | H | SCH₂ COO ⁱ C ₃ H ₇ |
| 1-901 | F | Cl | H | CF₂ Cl | H | SCH₂ COO ^{c} C ₅ H ₉ |
| 1-902 | F | Cl | H | CF₂ Cl | H | SCH₂ COO ^{c} C ₆ H ₁₁ |
| 1-903 | F | Cl | H | CF₂ Cl | H | SCH(CH ₃ )COOCH ₃ |
| 1-904 | F | Cl | H | CF₂ Cl | H | SCH(CH ₃ )COOC ₂ H ₅ |
| 1-905 | F | Cl | H | CF₂ Cl | H | SCH(CH ₃ )COO ⁿ C₃ H ₇ |
| 1-906 | F | Cl | H | CF₂ Cl | H | SCH(CH ₃ )COO ⁿ C₄ H ₉ |
| 1-907 | F | Cl | H | CF₂ Cl | H | SCH(CH₃)COO ⁿ C₅ H₁₁ |
| 1-908 | F | Cl | H | CF₂ Cl | H | SCH(CH₃ )COO ⁱ C₃ H₇ |
| 1-909 | F | Cl | H | CF₂ Cl | H | SCH(CH₃ )COO^{c} C₅ H ₉ |
| 1-910 | F | Cl | H | CF₂ Cl | H | SCH(CH₃) COO ^{c} C₆ H₁₁ |
| 1-911 | F | Cl | H | CF₂ Cl | H | SCH₂ CON(CH₃)₂ |
| 1-912 | F | Cl | H | CF₂ Cl | H | SCH₂ CON(C₂ H₅)₂ |
| 1-913 | F | Cl | H | CF₂ Cl | H | SCH₂CON (tetramethylene) |
| 1-914 | F | Cl | H | CF₂ Cl | H | SCH₂CON (pentamethylene) |
| 1-915 | F | Cl | H | CF₂ Cl | H | SCH₂CON (ethyleneoxyethylene) |
| 1-916 | F | Cl | H | CF₂ Cl | H | SCH(CH₃) CON(CH₂)₂ |
| 1-917 | F | Cl | H | CF₂ Cl | H | SCH(CH₃) CON(C₂ H₅)₂ |
| 1-918 | F | Cl | H | CF₂ Cl | H | SCH(CH₃)CON (tetramethylene) |
| 1-919 | F | Cl | H | CF₂ Cl | H | SCH(CH₃)CON (pentamethylene) |
| 1-920 | H | F | H | CF₂ Cl | H | SO₂ Cl |
| 1-921 | H | Cl | H | CF₂ Cl | H | SO₂ Cl |
| 1-922 | H | Br | H | CF₂ Cl | H | SO₂ Cl |
| 1-923 | F | F | H | CF₂ Cl | H | SO₂ Cl |
| 1-924 | F | Cl | H | CF₂ Cl | H | SO₂ Cl |
| 1-925 | F | Br | H | CF₂ Cl | H | SO₂ Cl |
| 1-926 | H | Cl | H | CF₂ Cl | H | SO₂ OCH ₃ |
| 1-927 | H | Cl | H | CF₂ Cl | H | SO₂ OC₂ H ₅ |
| 1-928 | H | Cl | H | CF₂ Cl | H | SO₂ O ⁱ C₃ H₇ |
| 1-929 | H | Cl | H | CF₂ Cl | H | SO₂ OCH ₂ CH=CH₂ |
| 1-930 | F | Cl | H | CF₂ Cl | H | SO₂ OCH ₃ |
| 1-931 | F | Cl | H | CF₂ Cl | H | SO₂ OC₂ H₅ |
| 1-932 | F | Cl | H | CF₂ Cl | H | SO₂ O ⁱ C₃ H ₇ |
| 1-933 | F | Cl | H | CF₂ Cl | H | SO₂ OCH₂ CH=CH₂ |
| 1-934 | H | Cl | H | CF₂ Cl | H | SO₂ N(CH₃ ) ₂ |
| 1-935 | H | Cl | H | CF₂ Cl | H | SO₂ N (C₂ H₅) ₂ |
| 1-936 | F | Cl | H | CF₂ Cl | H | SO₂ N (CH₃) ₂ |
| 1-937 | F | Cl | H | CF₂ Cl | H | SO₂ N (C₂ H ₅ ) ₂ |
| 1-938 | H | Cl | H | CF₂ Cl | H | COOH |
| 1-939 | H | Cl | H | CF₂ Cl | H | COOCH₃ |
| 1-940 | H | Cl | H | CF₂ Cl | H | COOC ₂ H ₅ |
| 1-941 | H | Cl | H | CF₂ Cl | H | COO ⁿ C ₃ H ₇ |
| 1-942 | H | Cl | H | CF₂ Cl | H | COOⁿ C ₄ H ₉ |
| 1-943 | H | Cl | H | CF₂ Cl | H | COOⁿ C ₅ H ₁₁ |
| 1-944 | H | Cl | H | CF₂ Cl | H | COOⁱ C ₃ H ₇ |
| 1-945 | H | Cl | H | CF₂ Cl | H | COOCH₂ CH₂ Cl |
| 1-946 | H | Cl | H | CF₂ Cl | H | COOCH₂ CH₂ Br |
| 1-947 | H | Cl | H | CF₂ Cl | H | CON(CH ₃ ) ₂ |
| 1-948 | H | Cl | H | CF₂ Cl | H | CONHCH ₃ |
| 1-949 | H | Cl | H | CF₂ Cl | H | CON(C₂ H ₅ ) ₂ |
| 1-950 | H | Cl | H | CF₂ Cl | H | CONHC₂ H ₅ |
| 1-951 | H | Cl | H | CF₂ Cl | H | COCH ₃ |
| 1-952 | H | Cl | H | CF₂ Cl | H | COC₂ H ₅ |
| 1-953 | H | Cl | H | CF₂ Cl | H | COCH₂ Cl |
| 1-954 | H | Cl | H | CF₂ Cl | H | CHO |
| 1-955 | H | Cl | H | CF₂ Cl | H | CH = CHCOOCH ₃ |
| 1-956 | H | Cl | H | CF₂ Cl | H | CH=CHCOOC₂ H ₅ |
| 1-957 | H | Cl | H | CF₂ Cl | H | CH₂ CH₂ COOCH ₃ |
| 1-958 | H | Cl | H | CF₂ Cl | H | CH₂ CH₂ COOC₂ H₅ |
| 1-959 | F | Cl | H | CF₂ Cl | H | COOH |
| 1-960 | F | Cl | H | CF₂ Cl | H | COOCH ₃ |
| 1-961 | F | Cl | H | CF₂ Cl | H | COOC ₂ H ₅ |
| 1-962 | F | Cl | H | CF₂ Cl | H | COOⁿ C ₃ H ₇ |
| 1-963 | F | Cl | H | CF₂ Cl | H | COOⁿ C ₄ H ₉ |
| 1-964 | F | Cl | H | CF₂ Cl | H | COOⁿ C ₅ H ₁₁ |
| 1-965 | F | Cl | H | CF₂ Cl | H | COOⁱ C ₃ H₇ |
| 1-966 | F | Cl | H | CF₂ Cl | H | COOCH₂ CH₂ Cl |
| 1-967 | F | Cl | H | CF₂ Cl | H | COOCH₂ CH₂ Br |
| 1-968 | F | Cl | H | CF₂ Cl | H | CON(CH₃)₂ |
| 1-969 | F | Cl | H | CF₂ Cl | H | CONHCH₃ |
| 1-970 | F | Cl | H | CF₂ Cl | H | CON(C₂ H₅) ₂ |
| 1-971 | F | Cl | H | CF₂ Cl | H | CONHC₂ H ₅ |
| 1-972 | F | Cl | H | CF₂ Cl | H | COCH ₃ |
| 1-973 | F | Cl | H | CF₂ Cl | H | COC₂ H ₅ |
| 1-974 | F | Cl | H | CF₂ Cl | H | COCH₂ Cl |
| 1-975 | F | Cl | H | CF₂ Cl | H | CHO |
| 1-976 | F | Cl | H | CF₂ Cl | H | CH = CHCOOCH₃ |
| 1-977 | F | Cl | H | CF₂ Cl | H | CH=CHCOOC₂ H ₅ |
| 1-978 | F | Cl | H | CF₂ Cl | H | CH₂ CH₂ COOCH₃ |
| 1-979 | F | Cl | H | CF₂ Cl | H | CH₂ CH₂ COOC₂ H₅ |
| 1-980 | H | F | H | CF₃ | H | NO₂ |
| 1-981 | H | Cl | H | CF₃ | H | NO₂ |
| 1-982 | H | Br | H | CF₃ | H | NO₂ |
| 1-983 | F | F | H | CF₃ | H | NO₂ |
| 1-984 | F | Cl | H | CF₃ | H | NO₂ |
| 1-985 | F | Br | H | CF₃ | H | NO₂ |
| 1-986 | H | F | H | CF₃ | H | NH₂ |
| 1-987 | H | Cl | H | CF₃ | H | NH₂ |
| 1-988 | H | Br | H | CF₃ | H | NH₂ |
| 1-989 | F | F | H | CF₃ | H | NH₂ |
| 1-990 | F | Cl | H | CF₃ | H | NH₂ |
| 1-991 | H | Cl | H | CF₃ | H | NHCH(CH₃ )COOCH₂ |
| 1-992 | H | Cl | H | CF₃ | H | NHCH(CH₃ )COOC₂ H₅ |
| 1-993 | H | Cl | H | CF₃ | H | NHCH(CH₃ )COO ⁿ C₃ H₇ |
| 1-994 | H | Cl | H | CF₃ | H | NHCH(CH₃)COO ⁿ C₄ H₉ |
| 1-995 | H | Cl | H | CF₃ | H | NHCH(CH₃ )COO ⁿ C₅ H₁₁ |
| 1-996 | H | Cl | H | CF₃ | H | NHCH(CH₃ )COO ⁱ C₃ H ₇ |
| 1-997 | H | Cl | H | CF₃ | H | NHCH(CH₃ )COO ^{c} C₅ H ₉ |
| 1-998 | H | Cl | H | CF₃ | H | NHCH(CH₃ )COO ^{c} C₆ H ₁₁ |
| 1-999 | F | Cl | H | CF₃ | H | NHCH₃ |
| 1-1000 | F | Cl | H | CF₃ | H | NHC ₂ H ₅ |
| 1-1001 | F | Cl | H | CF₃ | H | NHCH₂ CH=CH₂ |
| 1-1002 | F | Cl | H | CF₃ | H | NHCH₂ C =CH |
| 1-1003 | F | Cl | H | CF₃ | H | NHCH(CH₃) C=CH |
| 1-1004 | F | Cl | H | CF₃ | H | NHSO₂ CH₃ |
| 1-1005 | F | Cl | H | CF₃ | H | NHSO₂ C ₂ H ₅ |
| 1-1006 | F | Cl | H | CF₃ | H | NHSO₂ CH₂ Cl |
| 1-1007 | F | Cl | H | CF₃ | H | NHSO₂ CF₃ |
| 1-1008 | F | Cl | H | CF₃ | H | N(CH₃ )SO ₂ CH₃ |
| 1-1009 | F | Cl | H | CF₃ | H | N(CH₂ C ≡ CH)SO ₂ CH ₃ |
| 1-1010 | F | Cl | H | CF₃ | H | NHCOOCH ₃ |
| 1-1011 | F | Cl | H | CF₃ | H | NHCOOC₂ H ₅ |
| 1-1012 | F | Cl | H | CF₃ | H | NHCOO ⁿ C₃ H ₇ |
| 1-1013 | F | Cl | H | CF₃ | H | NHCOO C ₃ H ₇ |
| 1-1014 | F | Cl | H | CF₃ | H | NHCOO ⁿ C ₄ H ₉ |
| 1-1015 | F | Cl | H | CF₃ | H | NHCOO ⁿ C ₅ H ₁₁ |
| 1-1016 | F | Cl | H | CF₃ | H | NHCH₂ COOCH₃ |
| 1-1017 | F | Cl | H | CF₃ | H | NHCH₂ COOC₂ H ₅ |
| 1-1018 | F | Cl | H | CF₃ | H | NHCH₂ COO ⁿ C ₃ H ₇ |
| 1-1019 | F | Cl | H | CF₃ | H | NHCH₂ COO ⁿ C ₄ H ₉ |
| 1-1020 | F | Cl | H | CF₃ | H | NHCH₂ COO ⁿ C ₅ H ₁₁ |
| 1-1021 | F | Cl | H | CF₃ | H | NHCH₂ COO ⁱ C₃ H ₇ |
| 1-1022 | F | Cl | H | CF₃ | H | NHCH₂ COO^{c} C ₅ H ₉ |
| 1-1023 | F | Br | H | CF₃ | H | NH₂ |
| 1-1024 | H | F | H | CF₃ | H | OH |
| 1-1025 | H | Cl | H | CF₃ | H | OH |
| 1-1026 | H | Br | H | CF₃ | H | OH |
| 1-1027 | F | F | H | CF₃ | H | OH |
| 1-1028 | F | Cl | H | CF₃ | H | OH |
| 1-1029 | F | Br | H | CF₃ | H | OH |
| 1-1030 | H | Cl | H | CF₃ | H | NHCH₃ |
| 1-1031 | H | Cl | H | CF₃ | H | NHC ₂ H₅ |
| 1-1032 | H | Cl | H | CF₃ | H | NHCH₂ CH=CH₂ |
| 1-1033 | H | Cl | H | CF₃ | H | NHCH₂ C ≡ CH |
| 1-1034 | H | Cl | H | CF₃ | H | NHCH(CH ₃ )C≡CH |
| 1-1035 | H | Cl | H | CF₃ | H | NHSO₂ CH₃ |
| 1-1036 | H | Cl | H | CF₃ | H | NHSO₂ C ₂ H ₅ |
| 1-1037 | H | Cl | H | CF₃ | H | NHSO₂ CH₂ Cl |
| 1-1038 | H | Cl | H | CF₃ | H | NHSO₂ CF₃ |
| 1-1039 | H | Cl | H | CF₃ | H | N(CH₃ )SO ₂ CH₃ |
| 1-1040 | H | Cl | H | CF₃ | H | N(CH₂ C ≡ CH)SO ₂ CH₃ |
| 1-1041 | H | Cl | H | CF₃ | H | NHCOOCH ₃ |
| 1-1042 | H | Cl | H | CF₃ | H | NHCOOC₂ H ₅ |
| 1-1043 | H | Cl | H | CF₃ | H | NHCOO ⁿ C ₃ H ₇ |
| 1-1044 | H | Cl | H | CF₃ | H | NHCOO ⁱ C ₃ H ₇ |
| 1-1045 | H | Cl | H | CF₃ | H | NHCOO ⁿ C₄ H ₉ |
| 1-1046 | H | Cl | H | CF₃ | H | NHCOO ⁿ C ₅ H ₁₁ |
| 1-1047 | H | Cl | H | CF₃ | H | NHCH₂ COOCH ₃ |
| 1-1048 | H | Cl | H | CF₃ | H | NHCH₂ COOC₂ H ₅ |
| 1-1049 | H | Cl | H | CF₃ | H | NHCH₂ COO ⁿ C ₃ H ₇ |
| 1-1050 | H | Cl | H | CF₃ | H | NHCH₂ COO ⁿ C ₄ H ₉ |
| 1-1051 | H | Cl | H | CF₃ | H | NHCH₂ COO ⁿ C ₅ H ₁₁ |
| 1-1052 | H | Cl | H | CF₃ | H | NHCH₂ COO ⁱ C ₃ H ₇ |
| 1-1053 | H | Cl | H | CF₃ | H | NHCH₂ COO ^{c} C ₅ H ₉ |
| 1-1054 | H | Cl | H | CF₃ | H | NHCH₂ COO ^{c} C₆ H ₁₁ |
| 1-1055 | F | Cl | H | CF₃ | H | NH CH₂ COO ^{c} C₆ H₁₁ |
| 1-1056 | F | Cl | H | CF₃ | H | NHCH(CH ₃ )COOCH ₃ |
| 1-1057 | F | Cl | H | CF₃ | H | NHCH(CH₃ )COOC ₂ H₅ |
| 1-1058 | F | Cl | H | CF₃ | H | NHCH(CH₃ )COO ⁿ C₃ H ₇ |
| 1-1059 | F | Cl | H | CF₃ | H | NHCH(CH₃ )COO ⁿ C₄ H ₉ |
| 1-1060 | F | Cl | H | CF₃ | H | NHCH(CH₃ )COO ⁿ C₅ H ₁₁ |
| 1-1061 | F | Cl | H | CF₃ | H | NHCH(CH₃ )COO ⁱ C₃ H ₇ |
| 1-1062 | F | Cl | H | CF₃ | H | NHCH(CH₃ )COO ^{c} C₅ H ₉ |
| 1-1063 | F | Cl | H | CF₃ | H | NHCH(CH₃ )COO^{c} C₆ H ₁₁ |
| 1-1064 | H | Cl | H | CF₃ | H | OCH₃ |
| 1-1065 | H | Cl | H | CF₃ | H | OC ₂ H ₅ |
| 1-1066 | H | Cl | H | CF₃ | H | Oⁱ C ₃ H ₇ |
| 1-1067 | H | Cl | H | CF₃ | H | Oⁿ C ₃ H₇ |
| 1-1068 | H | Cl | H | CF₃ | H | OCH₂ CH₂ Cl |
| 1-1069 | H | Cl | H | CF₃ | H | OCF₂ CF₂ H |
| 1-1070 | H | Cl | H | CF₃ | H | O^{c} C₅ H ₈ |
| 1-1071 | H | Cl | H | CF₃ | H | O^{c} C ₆ H ₁₁ |
| 1-1072 | H | Cl | H | CF₃ | H | OCH₂ CH=CH₂ |
| 1-1073 | H | Cl | H | CF₃ | H | OCH₂ CCl = CH₂ |
| 1-1074 | H | Cl | H | CF₃ | H | OCH₂ CCl = CHCl |
| 1-1075 | H | Cl | H | CF₃ | H | OCH (CH ₃ )CH =CH₂ |
| 1-1076 | H | Cl | H | CF₃ | H | OCH₂ C =CH |
| 1-1077 | H | Cl | H | CF₃ | H | OCH(CH ₃)C≡CH |
| 1-1078 | H | Cl | H | CF₃ | H | OCH₂ C≡CBr |
| 1-1079 | H | Cl | H | CF₃ | H | OCH₂ C ≡CCl |
| 1-1080 | H | Cl | H | CF₃ | H | OCH₂ C ≡ CCH ₂Cl |
| 1-1081 | H | Cl | H | CF₃ | H | OCH₂ CN |
| 1-1082 | H | Cl | H | CF₃ | H | OCH₂ OCH₃ |
| 1-1083 | H | Cl | H | CF₃ | H | OCH₂ OC₂ H ₅ |
| 1-1084 | H | Cl | H | CF₃ | H | OCH₂ SCH ₃ |
| 1-1085 | H | Cl | H | CF₃ | H | OCH₂ COOCH₃ |
| 1-1086 | H | Cl | H | CF₃ | H | OCH₂ COOC₂ H ₅ |
| 1-1087 | H | Cl | H | CF₃ | H | OCH₂ COO ⁿ C ₃ H₇ |
| 1-1088 | H | Cl | H | CF₃ | H | OCH₂ COO ⁿ C ₄ H₉ |
| 1-1089 | H | Cl | H | CF₃ | H | OCH₂ COO ⁿ C ₅ H₁₁ |
| 1-1090 | H | Cl | H | CF₃ | H | OCH₂ COO ⁱ C₃ H ₇ |
| 1-1091 | H | Cl | H | CF₃ | H | OCH₂ COO ^{c} C ₅ H ₉ |
| 1-1092 | H | Cl | H | CF₃ | H | OCH₂ COO ^{c} C ₆ H ₁₁ |
| 1-1093 | H | Cl | H | CF₃ | H | OCH(CH ₃ )COOCH ₃ |
| 1-1094 | H | Cl | H | CF₃ | H | OCH(CH ₃ )COOC ₂ H ₅ |
| 1-1095 | H | Cl | H | CF₃ | H | OCH(CH ₃ )COO ⁿ C₃ H ₇ |
| 1-1096 | H | Cl | H | CF₃ | H | OCH(CH ₃ )COO ⁿ C₄ H ₉ |
| 1-1097 | H | Cl | H | CF₃ | H | OCH(CH ₃ )COO ⁿ C₅ H ₁₁ |
| 1-1098 | H | Cl | H | CF₃ | H | OCHCCH ₃ )COO ⁱ C₃ H ₇ |
| 1-1099 | H | Cl | H | CF₃ | H | OCH(CH ₃ )COO ⁱ C₅ H ₉ |
| 1-1100 | H | Cl | H | CF₃ | H | OCH(CH ₃ )COO ^{c} C₆ H₁₁ |
| 1-1101 | H | Cl | H | CF₃ | H | OCH₂ CON(CH₃) ₂ |
| 1-1102 | H | Cl | H | CF₃ | H | OCH₂ CON(C ₂H ₅) ₂ |
| 1-1103 | H | Cl | H | CF₃ | H | OCH₂ CON(CH₃) C₂ H₅ |
| 1-1104 | H | Cl | H | CF₃ | H | OCH(CH ₃ )CON(CH ₃ ) ₂ |
| 1-1105 | H | Cl | H | CF₃ | H | OCH(CH₃ )CON(C ₂ H ₅)₂ |
| 1-1106 | H | Cl | H | CF₃ | H | OCH(CH₃ )CON(CH ₃ )C₂ H ₅ |
| 1-1107 | H | Cl | H | CF₃ | H | OCH₂ COON(CH₃)₂ |
| 1-1108 | H | Cl | H | CF₃ | H | OCH₂ COON(C₂ H ₅ ) ₂ |
| 1-1109 | H | Cl | H | CF₃ | H | OCH(CH₃ )COON(CH ₃) ₂ |
| 1-1110 | H | Cl | H | CF₃ | H | OCH(CH₃ )COON(C ₂ H ₅ ) ₂ |
| 1-1111 | F | Cl | H | CF₂ | H | OCH₃ |
| 1-1112 | F | Cl | H | CF₃ | H | OC ₂ H ₅ |
| 1-1113 | F | Cl | H | CF₃ | H | Oⁱ C ₃ H ₇ |
| 1-1114 | F | Cl | H | CF₃ | H | O ⁿ C ₃ H ₇ |
| 1-1115 | F | Cl | H | CF₃ | H | OCH₂ CH₂ Cl |
| 1-1116 | F | Cl | H | CF₃ | H | OCF₂ CF₂ H |
| 1-1117 | F | Cl | H | CF₃ | H | O^{c} C s H ₉ |
| 1-1118 | F | Cl | H | CF₃ | H | O^{c} C ₆ H ₁₁ |
| 1-1119 | F | Cl | H | CF₃ | H | OCH₂ CH = CH ₂ |
| 1-1120 | F | Cl | H | CF₃ | H | OCH₂ CCl = CH₂ |
| 1-1121 | F | Cl | H | CF₃ | H | OCH₂ CCl = CHCl |
| 1-1122 | F | Cl | H | CF₃ | H | OCH(CH ₃)CH =CH₂ |
| 1-1123 | F | Cl | H | CF₃ | H | OCH₂ C ≡CH |
| 1-1124 | F | Cl | H | CF₃ | H | OCH(CH₃ )C≡CH |
| 1-1125 | F | Cl | H | CF₃ | H | OCH₂ C = CBr |
| 1-1126 | F | Cl | H | CF₃ | H | OCH₂ C ≡CCl |
| 1-1127 | F | Cl | H | CF₃ | H | OCH₂ C ≡CCH₂ Cl |
| 1-1128 | F | Cl | H | CF₃ | H | OCH₂ CN |
| 1-1129 | F | Cl | H | CF₃ | H | OCH₂ OCH₃ |
| 1-1130 | F | Cl | H | CF₃ | H | OCH₂ OC₂ H ₅ |
| 1-1131 | F | Cl | H | CF₃ | H | OCH₂ SCH₃ |
| 1-1132 | F | Cl | H | CF₃ | H | OCH₂ COOCH ₃ |
| 1-1133 | F | Cl | H | CF₃ | H | OCH₂ COOC₂ H ₅ |
| 1-1134 | F | Cl | H | CF₃ | H | OCH₂ COO ⁿ C ₃ H ₇ |
| 1-1135 | F | Cl | H | CF₃ | H | OCH₂ COO ⁿ C ₄ H₉ |
| 1-1136 | F | Cl | H | CF₃ | H | OCH₂ COO ⁿ C₅ H₁₁ |
| 1-1137 | F | Cl | H | CF₃ | H | OCH₂ COOⁱ C₉ H₇ |
| 1-1138 | F | Cl | H | CF₃ | H | OCH₂ COO ^{c} C ₅ H₉ |
| 1-1139 | F | Cl | H | CF₃ | H | OCH₂ COO ^{c} C₆ H₁₁ |
| 1-1140 | F | Cl | H | CF₃ | H | OCH(CH₃)COOCH₃ |
| 1-1141 | F | Cl | H | CF₃ | H | OCH(CH₃)COOC₂ H₅ |
| 1-1142 | F | Cl | H | CF₃ | H | OCH(CH₃)COOⁿ C₃ H₇ |
| 1-1143 | F | Cl | H | CF₃ | H | OCH(CH₃)COOⁿ C₄ H₉ |
| 1-1144 | F | Cl | H | CF₃ | H | OCH(CH₃)COO ⁿ C₅ H₁₁ |
| 1-1145 | F | Cl | H | CF₃ | H | OCH(CH₃)COO ⁱ C₃ H ₇ |
| 1-1146 | F | Cl | H | CF₃ | H | OCH(CH₃)COO ^{c} C₅ H₉ |
| 1-1147 | F | Cl | H | CF₃ | H | OCH(CH ₃)COO^{c} C₆ H₁₁ |
| 1-1148 | F | Cl | H | CF₃ | H | OCH₂ CON(CH₃) ₂ |
| 1-1149 | F | Cl | H | CF₃ | H | OCH₂ CON(C ₂H₅) ₂ |
| 1-1150 | F | Cl | H | CF₃ | H | OCH₂ CON(CH₃) C ₂H₅ |
| 1-1151 | F | Cl | H | CF₃ | H | OCH(CH₃)CON(CH₃) ₂ |
| 1-1152 | F | Cl | H | CF₃ | H | OCH(CH₃ )CON(C ₂H₅ )₂ |
| 1-1153 | F | Cl | H | CF₃ | H | OCH(CH₃ )CON(CH ₃ )C₂ H₅ |
| 1-1154 | F | Cl | H | CF₃ | H | OCH ₂ COON(CH₃) ₂ |
| 1-1155 | F | Cl | H | CF₃ | H | OCH ₂ COON(C ₂ H₅) ₂ |
| 1-1156 | F | Cl | H | CF₃ | H | OCH(CH₃ )COON(CH₃ ) ₂ |
| 1-1157 | F | Cl | H | CF₃ | H | OCH(CH₃ )COON(C ₂ H ₅) ₂ |
| 1-1158 | H | F | H | CF₃ | H | SH |
| 1-1159 | H | Cl | H | CF₃ | H | SH |
| 1-1160 | H | Br | H | CF₃ | H | SH |
| 1-1161 | F | F | H | CF₃ | H | SH |
| 1-1162 | F | Cl | H | CF₃ | H | SH |
| 1-1163 | F | Br | H | CF₃ | H | SH |
| 1-1164 | H | Cl | H | CF₃ | H | SCH ₃ |
| 1-1165 | H | Cl | H | CF₃ | H | SC₂ H ₅ |
| 1-1166 | H | Cl | H | CF₃ | H | Sⁱ C ₃ H ₇ |
| 1-1167 | H | Cl | H | CF₃ | H | SCH₂ CH₂ Cl |
| 1-1168 | H | Cl | H | CF₃ | H | S^{c} C ₅ H ₉ |
| 1-1169 | H | Cl | H | CF₃ | H | S^{c} C ₆ H ₁₁ |
| 1-1170 | H | Cl | H | CF₃ | H | SCH₂ CH=CH₂ |
| 1-1171 | H | Cl | H | CF₃ | H | SCH₂ CCl = CH₂ |
| 1-1172 | H | Cl | H | CF₃ | H | SCH ₂ CCl = CHCl |
| 1-1173 | H | Cl | H | CF₃ | H | SCH(CH ₃ )CH = CH₂ |
| 1-1174 | H | Cl | H | CF₃ | H | SCH₂ C ≡ CH |
| 1-1175 | H | Cl | H | CF₃ | H | SCH(CH ₃ )C≡CH |
| 1-1176 | H | Cl | H | CF₃ | H | SCH₂ COOCH ₃ |
| 1-1177 | H | Cl | H | CF₃ | H | SCH₂ COOC₂ H ₅ |
| 1-1178 | H | Cl | H | CF₃ | H | SCH₂ COO ⁿ C ₃ H ₇ |
| 1-1179 | H | Cl | H | CF₃ | H | SCH₂ COO ⁿ C ₄ H ₉ |
| 1-1180 | H | Cl | H | CF₃ | H | SCH₂ COO ⁿ C ₅ H ₁₁ |
| 1-1181 | H | Cl | H | CF₃ | H | SCH₂ COO ⁱ C₃ H₇ |
| 1-1182 | H | Cl | H | CF₃ | H | SCH₂ COO^{c} C₅ H ₉ |
| 1-1183 | H | Cl | H | CF₃ | H | SCH₂ COO ^{c} C ₆ H ₁₁ |
| 1-1184 | H | Cl | H | CF₃ | H | SCH(CH ₃ )COOCH₃ |
| 1-1185 | H | Cl | H | CF₂ | H | SCH(CH₃ )COOC ₂ H ₅ |
| 1-1186 | H | Cl | H | CF₃ | H | SCH(CH₃ )COO ⁿ C₃ H ₇ |
| 1-1187 | H | Cl | H | CF₃ | H | SCH(CH₃ )COO ⁿ C₄ H ₉ |
| 1-1188 | H | Cl | H | CF₃ | H | SCH(CH₃ )COOⁿ C₅ H₁₁ |
| 1-1189 | H | Cl | H | CF₃ | H | SCH(CH ₃)COO ⁱ C₃ H₇ |
| 1-1190 | H | Cl | H | CF₃ | H | SCH(CH₃)COO^{c} C₅ H₉ |
| 1-1191 | H | Cl | H | CF₃ | H | SCH(CH ₃)COO C₆ H₁₁ |
| 1-1192 | H | Cl | H | CF₃ | H | SCH₂ CON(CH₃) ₂ |
| 1-1193 | H | Cl | H | CF₃ | H | SCH₂ CON(C ₂ H₅ ) ₂ |
| 1-1194 | H | Cl | H | CF₃ | H | SCH₂CON(tetramethylene) |
| 1-1195 | H | Cl | H | CF₃ | H | SCH₂CON (pentamethylene) |
| 1-1196 | H | Cl | H | CF₃ | H | SCH₂CON (ethyleneoxyethylene) |
| 1-1197 | H | Cl | H | CF₃ | H | SCH(CH₃ )CON(CH ₃ ) ₂ |
| 1-1198 | H | Cl | H | CF₃ | H | SCH(CH₃ )CON(C₂ H ₅ ) ₂ |
| 1-1199 | H | Cl | H | CF₃ | H | SCH(CH₃)CON (tetramethylene) |
| 1-1200 | H | Cl | H | CF₃ | H | SCH(CH₃)CON (pentamethylene) |
| 1-1201 | F | Cl | H | CF₃ | H | SCH ₃ |
| 1-1202 | F | Cl | H | CF₃ | H | SC₂ H ₅ |
| 1-1203 | F | Cl | H | CF₃ | H | S ⁱ C ₃ H ₇ |
| 1-1204 | F | Cl | H | CF₃ | H | SCH₂ CH₂ Cl |
| 1-1205 | F | Cl | H | CF₃ | H | S ^{c} C ₅ H ₉ |
| 1-1206 | F | Cl | H | CF₃ | H | S^{c} C ₆ H ₁₁ |
| 1-1207 | F | Cl | H | CF₃ | H | SCH₂ CH=CH₂ |
| 1-1208 | F | Cl | H | CF₃ | H | SCH₂ CCl = CH₂ |
| 1-1209 | F | Cl | H | CF₃ | H | SCH₂ CCl = CHCl |
| 1-1210 | F | Cl | H | CF₃ | H | SCH(CH ₃ )CH = CH₂ |
| 1-1211 | F | Cl | H | CF₃ | H | SCH₂ C ≡CH |
| 1-1212 | F | Cl | H | CF₃ | H | SCH(CH₃)C≡CH |
| 1-1213 | F | Cl | H | CF₃ | H | SCH₂ COOCH ₃ |
| 1-1214 | F | Cl | H | CF₃ | H | SCH₂ COOC₂ H ₅ |
| 1-1215 | F | Cl | H | CF₃ | H | SCH₂ COO ⁿC ₃ H ₇ |
| 1-1216 | F | Cl | H | CF₃ | H | SCH₂ COO ⁿ C ₄ H ₉ |
| 1-1217 | F | Cl | H | CF₃ | H | SCH₂ COO ⁿ C ₅ H ₁₁ |
| 1-1218 | F | Cl | H | CF₃ | H | SCH₂ COO ⁱ C ₃ H ₇ |
| 1-1219 | F | Cl | H | CF₃ | H | SCH₂ COO ^{c} C ₅ H ₉ |
| 1-1220 | F | Cl | H | CF₃ | H | SCH₂ COO ^{c} C ₆ H ₁₁ |
| 1-1221 | F | Cl | H | CF₃ | H | SCH(CH ₃ )COOCH ₃ |
| 1-1222 | F | Cl | H | CF₃ | H | SCH(CH ₃ )COOC ₂ H ₅ |
| 1-1223 | F | Cl | H | CF₃ | H | SCH(CH ₃ )COO ⁿ C₃ H ₇ |
| 1-1224 | F | Cl | H | CF₃ | H | SCH(CH ₃ )COO ⁿ C₄ H ₉ |
| 1-1225 | F | Cl | H | CF₃ | H | SCH(CH ₃ )COO ⁿ C₅ H ₁₁ |
| 1-1226 | F | Cl | H | CF₃ | H | SCH(CH ₃ )COO ⁱ C₃ H ₇ |
| 1-1227 | F | Cl | H | CF₃ | H | SCH(CH ₃ )COO ^{c} C₅ H ₉ |
| 1-1228 | F | Cl | H | CF₃ | H | SCH(CH ₃ )COO ^{c} C₆ H ₁₁ |
| 1-1229 | F | Cl | H | CF₃ | H | SCH₂ CON(CH₃ ) ₂ |
| 1-1230 | F | Cl | H | CF₃ | H | SCH₂ CON(C ₂ H ₅ ) ₂ |
| 1-1231 | F | Cl | H | CF₃ | H | SCH₂CON (tetramethylene) |
| 1-1232 | F | Cl | H | CF₃ | H | SCH₂CON (pentamethylene) |
| 1-1233 | F | Cl | H | CF₃ | H | SCH₂CON (ethyleneoxyethylene) |
| 1-1234 | F | Cl | H | CF₃ | H | SCH(CH₃ )CON(CH ₃ ) ₂ |
| 1-1235 | F | Cl | H | CF₃ | H | SCH(CH₃ )CON(C₂ H ₅ ) ₂ |
| 1-1236 | F | Cl | H | CF₃ | H | SCH(CH₃)CON (tetramethylene) |
| 1-1237 | F | Cl | H | CF₃ | H | SCH (CH₃) CON (pentamethylene) |
| 1-1238 | H | F | H | CF₃ | H | SO₂ Cl |
| 1-1239 | H | Cl | H | CF₃ | H | SO₂ Cl |
| 1-1240 | H | Br | H | CF₃ | H | SO₂ Cl |
| 1-1241 | F | F | H | CF₃ | H | SO₂ Cl |
| 1-1242 | F | Cl | H | CF₃ | H | SO₂ Cl |
| 1-1243 | F | Br | H | CF₃ | H | S0₂ Cl |
| 1-1244 | H | Cl | H | CF₃ | H | SO₂ OCH ₃ |
| 1-1245 | H | Cl | H | CF₃ | H | SO₂ OC₂ H ₅ |
| 1-1246 | H | Cl | H | CF₃ | H | SO₂ O ⁱ C ₃ H ₇ |
| 1-1247 | H | Cl | H | CF₃ | H | SO₂ OCH ₂ CH=CH₂ |
| 1-1248 | F | Cl | H | CF₃ | H | SO₂ OCN ₃ |
| 1-1249 | F | Cl | H | CF₃ | H | SO₂ OC₂ H ₅ |
| 1-1250 | F | Cl | H | CF₃ | H | SO₂ O ⁱ C ₃ H ₇ |
| 1-1251 | F | Cl | H | CF₃ | H | SO₂ OCH ₂ CH = CH₂ |
| 1-1252 | H | Cl | H | CF₃ | H | SO₂ N(CH₃ ) ₂ |
| 1-1253 | H | Cl | H | CF₃ | H | SO₂ N (C₂ H₅ )₂ |
| 1-1254 | F | Cl | H | CF₃ | H | SO₂N(CH₃)₂ |
| 1-1255 | F | Cl | H | CF₃ | H | SO₂ N (C₂ H ₅ ) ₂ |
| 1-1256 | H | Cl | H | CF₃ | H | COOH |
| 1-1257 | H | Cl | H | CF₃ | H | COOCH₃ |
| 1-1258 | H | Cl | H | CF₃ | H | COOC ₂ H₅ |
| 1-1259 | H | Cl | H | CF₃ | H | COOⁿ C₃ H₇ |
| 1-1260 | H | Cl | H | CF₃ | H | COOⁿ C₄ H ₉ |
| 1-1261 | H | Cl | H | CF₃ | H | COOⁿ C ₅ H₁₁ |
| 1-1262 | H | Cl | H | CF₃ | H | COOⁱ C₃ H₇ |
| 1-1263 | H | Cl | H | CF₃ | H | COOCH₂ CH₂ Cl |
| 1-1264 | H | Cl | H | CF₃ | H | COOCH₂ CH₂ Br |
| 1-1265 | H | Cl | H | CF₃ | H | CON(CH₃) ₂ |
| 1-1266 | H | Cl | H | CF₃ | H | CONHCH₃ |
| 1-1267 | H | Cl | H | CF₃ | H | CON(C₂ H₅)₂ |
| 1-1268 | H | Cl | H | CF₃ | H | CONHC₂ H₅ |
| 1-1269 | H | Cl | H | CF₃ | H | COCH₃ |
| 1-1270 | H | Cl | H | CF₃ | H | COC₂ H₅ |
| 1-1271 | H | Cl | H | CF₃ | H | COCH₂ Cl |
| 1-1272 | H | Cl | H | CF₃ | H | CHO |
| 1-1273 | H | Cl | H | CF₃ | H | CH=CHCOOCH₃ |
| 1-1274 | H | Cl | H | CF₃ | H | CH=CHCOOC₂ H ₅ |
| 1-1275 | H | Cl | H | CF₃ | H | CH₂ CH₂ COOCH₃ |
| 1-1276 | H | Cl | H | CF₃ | H | CH₂ CH₂ COOC₂ H ₅ |
| 1-1277 | F | Cl | H | CF₃ | H | COOH |
| 1-1278 | F | Cl | H | CF₃ | H | COOCH₃ |
| 1-1279 | F | Cl | H | CF₃ | H | COOC₂ H ₅ |
| 1-1280 | F | Cl | H | CF₃ | H | COOⁿ C₃H₇ |
| 1-1281 | F | Cl | H | CF₃ | H | COOⁿ C ₄ H₉ |
| 1-1282 | F | Cl | H | CF₃ | H | COOⁿ C₅H₁₁ |
| 1-1283 | F | Cl | H | CF₃ | H | COOⁱ C₃ H₇ |
| 1-1284 | F | Cl | H | CF₃ | H | COOCH₂ CH₂ Cl |
| 1-1285 | F | Cl | H | CF₃ | H | COOCH₂ CH₂ Br |
| 1-1286 | F | Cl | H | CF₃ | H | CON(CH₃) ₂ |
| 1-1287 | F | Cl | H | CF₃ | H | CONHCH ₃ |
| 1-1288 | F | Cl | H | CF₃ | H | CON(C₂ H₆) ₂ |
| 1-1289 | F | Cl | H | CF₃ | H | CONHC₂ H ₅ |
| 1-1290 | F | Cl | H | CF₃ | H | COCH ₃ |
| 1-1291 | F | Cl | H | CF₃ | H | COC₂ H ₅ |
| 1-1292 | F | Cl | H | CF₃ | H | COCH₂ Cl |
| 1-1293 | F | Cl | H | CF₃ | H | CHO |
| 1-1294 | F | Cl | H | CF₃ | H | CH = CHCOOCH ₃ |
| 1-1295 | F | Cl | H | CF₃ | H | CH=CHCOOC₂ H ₅ |
| 1-1296 | F | Cl | H | CF₃ | H | CH₂ CH₂ COOCH ₃ |
| 1-1297 | F | Cl | H | CF₃ | H | CH₂ CH₂ COOC₂ H ₅ |
| 1-1298 | H | F | CH₃ | CF₃ | H | NO₂ |
| 1-1299 | H | Cl | CH₃ | CF₃ | H | NO₂ |
| 1-1300 | H | Br | CH₃ | CF₃ | H | NO₂ |
| 1-1301 | F | F | CH₃ | CF₃ | H | NO₂ |
| 1-1302 | F | Cl | CH₃ | CF₃ | H | NO₂ |
| 1-1303 | F | Br | CH₃ | CF₃ | H | NO₂ |
| 1-1304 | H | F | CH₃ | CF₃ | H | NH₂ |
| 1-1305 | H | Cl | CH₃ | CF₃ | H | NH₂ |
| 1-1306 | H | Br | CH₃ | CF₃ | H | NH₂ |
| 1-1307 | F | F | CH₃ | CF₃ | H | NH₂ |
| 1-1308 | F | Cl | CH₃ | CF₃ | H | NH₂ |
| 1-1309 | H | Cl | CH₃ | CF₃ | H | NHCH(CH₃ )COOCH ₃ |
| 1-1310 | H | Cl | CH₃ | CF₃ | H | NHCH(CH₃ )COOC ₂ H ₅ |
| 1-1311 | H | Cl | CH₃ | CF₃ | H | NHCH(CH₃ )COO ⁿ C₃ H ₇ |
| 1-1312 | H | Cl | CH₃ | CF₃ | H | NHCH(CH₃ )COO ⁿ C₄ H₉ |
| 1-1313 | H | Cl | CH₃ | CF₃ | H | NHCH(CH₃ )COO ⁿ C₅ H₁₁ |
| 1-1314 | H | Cl | CH₃ | CF₃ | H | NHCH(CH₃ )COO ⁱ C₃ H₇ |
| 1-1315 | H | Cl | CH₃ | CF₃ | H | NHCH(CH₃ )COO^{c} C₅ H₉ |
| 1-1316 | H | Cl | CH₃ | CF₃ | H | NHCH(CH₃ )COO^{c} C₆ H₁₁ |
| 1-1317 | F | Cl | CH₃ | CF₃ | H | NHCH₃ |
| 1-1318 | F | Cl | CH₃ | CF₃ | H | NHC ₂ H₅ |
| 1-1319 | F | Cl | CH₃ | CF₃ | H | NHCH₂ CH=CH₂ |
| 1-1320 | F | Cl | CH₃ | CF₃ | H | NHCH₂ C ≡ CH |
| 1-1321 | F | Cl | CH₃ | CF₃ | H | NHCH(CH₃ )C ≡ CH |
| 1-1322 | F | Cl | CH₃ | CF₃ | H | NHSO₂ CH₃ |
| 1-1323 | F | Cl | CH₃ | CF₃ | H | NHSO₂ C ₂ H ₅ |
| 1-1324 | F | Cl | CH₃ | CF₃ | H | NHSO₂ CH₂ Cl |
| 1-1325 | F | Cl | CH₃ | CF₃ | H | NHSO₂ CF₃ |
| 1-1326 | F | Cl | CH₃ | CF₃ | H | N(CH₃ )SO ₂ CH₃ |
| 1-1327 | F | Cl | CH₃ | CF₃ | H | N(CH ₂ C ≡ CH)SO ₂ CH₃ |
| 1-1328 | F | Cl | CH₃ | CF₃ | H | NHCOOCH ₃ |
| 1-1329 | F | Cl | CH₃ | CF₃ | H | NHCOOC₂ H ₅ |
| 1-1330 | F | Cl | CH₃ | CF₃ | H | NHCOO ⁿ C ₃ H ₇ |
| 1-1331 | F | Cl | CH₃ | CF₃ | H | NHCOO ⁱ C ₃ H ₇ |
| 1-1332 | F | Cl | CH₃ | CF₃ | H | NHCOO ⁿ C ₄ H ₉ |
| 1-1333 | F | Cl | CH₃ | CF₃ | H | NHCOO ⁿ C ₅ H ₁₁ |
| 1-1334 | F | Cl | CH₃ | CF₃ | H | NHCH₂ COOCH₃ |
| 1-1335 | F | Cl | CH₃ | CF₃ | H | NHCH₂ COOC₂ H₅ |
| 1-1336 | F | Cl | CH₃ | CF₃ | H | NHCH₂ COO ⁿ C₃ H₇ |
| 1-1337 | F | Cl | CH₃ | CF₃ | H | NHCH₂ COO ⁿ C₄ H₉ |
| 1-1338 | F | Cl | CH₃ | CF₃ | H | NHCH₂ COO ⁿ C ₅ H₁₁ |
| 1-1339 | F | Cl | CH₃ | CF₂ | H | NHCH₂ COO ⁱ C ₃ H ₇ |
| 1-1340 | F | Cl | CH₃ | CF₃ | H | NHCH₂ COO ^{c} C ₅ H ₉ |
| 1-1341 | F | Br | CH₃ | CF₃ | H | NH₂ |
| 1-1342 | H | F | CH₃ | CF₃ | H | OH |
| 1-1343 | H | Cl | CH₃ | CF₃ | H | OH |
| 1-1344 | H | Br | CH₃ | CF₃ | H | OH |
| 1-1345 | F | F | CH₃ | CF₃ | H | OH |
| 1-1346 | F | Cl | CH₃ | CF₃ | H | OH |
| 1-1347 | F | Br | CH₃ | CF₃ | H | OH |
| 1-1348 | H | Cl | CH₃ | CF₃ | H | NHCH₃ |
| 1-1349 | H | Cl | CH₃ | CF₃ | H | NHC ₂ H ₅ |
| 1-1350 | H | Cl | CH₃ | CF₃ | H | NHCH₂ CH = CH₂ |
| 1-1351 | H | Cl | CH₃ | CF₃ | H | NHCH₂ C ≡ CH |
| 1-1352 | H | Cl | CH₃ | CF₃ | H | NHCH(CH ₃ ) C≡CH |
| 1-1353 | H | Cl | CH₃ | CF₃ | H | NHSO₂ CH₃ |
| 1-1354 | H | Cl | CH₃ | CF₃ | H | NHSO₂ C ₂ H ₅ |
| 1-1355 | H | Cl | CH₃ | CF₃ | H | NHSO₂ CH₂ Cl |
| 1-1356 | H | Cl | CH₃ | CF₃ | H | NHSO₂ CF₃ |
| 1-1357 | H | Cl | CH₃ | CF₃ | H | N(CH₃ )SO ₂ CH₃ |
| 1-1358 | H | Cl | CH₃ | CF₃ | H | N(CH₂ C ≡ CH)SO ₂ CH₃ |
| 1-1359 | H | Cl | CH₃ | CF₃ | H | NHCOOCH ₃ |
| 1-1360 | H | Cl | CH₃ | CF₃ | H | NHCOOC₂ H ₅ |
| 1-1361 | H | Cl | CH₃ | CF₃ | H | NHCOO ⁿ C ₃ H ₇ |
| 1-1362 | H | Cl | CH₃ | CF₃ | H | NHCOO ⁱ C ₃ H ₇ |
| 1-1363 | H | Cl | CH₃ | CF₃ | H | NHCOO ⁿ C ₄ H ₉ |
| 1-1364 | H | Cl | CH₃ | CF₃ | H | NHCOO ⁿ C ₅ H ₁₁ |
| 1-1365 | H | Cl | CH₃ | CF₃ | H | NHCH₂ COOCH ₃ |
| 1-1366 | H | Cl | CH₃ | CF₃ | H | NHCH₂ COOC₂ H ₅ |
| 1-1367 | H | Cl | CH₃ | CF₃ | H | NHCH₂ COO ⁿ C₃ H ₇ |
| 1-1368 | H | Cl | CH₃ | CF₃ | H | NHCH₂ COO ⁿ C₄ H ₉ |
| 1-1369 | H | Cl | CH₃ | CF₃ | H | NHCH₂ COO ⁿ C ₅ H₁₁ |
| 1-1370 | H | Cl | CH₃ | CF₃ | H | NHCH₂ COO ⁱ C₃ H ₇ |
| 1-1371 | H | Cl | CH₃ | CF₃ | H | NHCH₂ COO ^{c} C ₅ H ₉ |
| 1-1372 | H | Cl | CH₃ | CF₃ | H | NHCH₂ COO ^{c} C ₆ H ₁₁ |
| 1-1373 | F | Cl | CH₃ | CF₃ | H | NH CH₂ COO ^{c} C ₆ H ₁₁ |
| 1-1374 | F | Cl | CH₃ | CF₃ | H | NHCH(CH₃ )COOCH₃ |
| 1-1375 | F | Cl | CH₃ | CF₃ | H | NHCH(CH₃ )COOC ₂ H ₅ |
| 1-1376 | F | Cl | CH₃ | CF₃ | H | NHCH(CH₃ )COO ⁿ C₃ H ₇ |
| 1-1377 | F | Cl | CH₃ | CF₃ | H | NHCH(CH₃ )COO ⁿ C₄ H ₉ |
| 1-1378 | F | Cl | CH₃ | CF₃ | H | NHCH(CH₃ )COO ⁿ C₅ H ₁₁ |
| 1-1379 | F | Cl | CH₃ | CF₃ | H | NHCH(CH₃ )COO ⁱ C₃ H ₇ |
| 1-1380 | F | Cl | CH₃ | CF₃ | H | NHCH(CH₃ )COO ^{c} C₅ H ₉ |
| 1-1381 | F | Cl | CH₃ | CF₃ | H | NHCH(CH₃ )COO ^{c} C₆ H ₁₁ |
| 1-1382 | H | Cl | CH₃ | CF₃ | H | OCH₃ |
| 1-1383 | H | Cl | CH₃ | CF₃ | H | OC ₂ H ₅ |
| 1-1389 | H | Cl | CH₃ | CF₃ | H | Oⁱ C ₃ H ₇ |
| 1-1385 | H | Cl | CH₃ | CF₃ | H | Oⁿ C ₃ H ₇ |
| 1-1386 | H | Cl | CH₃ | CF₃ | H | OCH₂ CH₂ Cl |
| 1-1387 | H | Cl | CH₃ | CF₃ | H | OCF₂ CF₂ H |
| 1-1388 | H | Cl | CH₃ | CF₃ | H | O^{c} C ₅ H ₉ |
| 1-1389 | H | Cl | CH₃ | CF₃ | H | O^{c} C₆ H ₁₁ |
| 1-1390 | H | Cl | CH₃ | CF₃ | H | OCH₂ CH = CH₂ |
| 1-1391 | H | Cl | CH₃ | CF₃ | H | OCH₂ CCl = CH₂ |
| 1-1392 | H | Cl | CH₃ | CF₃ | H | OCH₂ CCl = CHCl |
| 1-1393 | H | Cl | CH₃ | CF₃ | H | OCH(CH ₃ )CH = CH₂ |
| 1-1394 | H | Cl | CH₃ | CF₃ | H | OCH₂ C ≡ CH |
| 1-1395 | H | Cl | CH₃ | CF₃ | H | OCH (CH₃ )C≡CH |
| 1-1396 | H | Cl | CH₃ | CF₃ | H | OCH₂ C ≡ CBr |
| 1-1397 | H | Cl | CH₃ | CF₃ | H | OCH₂ C ≡ CCl |
| 1-1398 | H | Cl | CH₃ | CF₃ | H | OCH₂ C ≡ CCH₂ Cl |
| 1-1399 | H | Cl | CH₃ | CF₃ | H | OCH₂ CN |
| 1-1400 | H | Cl | CH₃ | CF₃ | H | OCH₂ OCH ₃ |
| 1-1401 | H | Cl | CH₃ | CF₃ | H | OCH₂ OC₂ H ₅ |
| 1-1402 | H | Cl | CH₃ | CF₂ | H | OCH₂ SCH ₃ |
| 1-1403 | H | Cl | CH₃ | CF₃ | H | OCH₂ COOCH ₃ |
| 1-1404 | H | Cl | CH₃ | CF₃ | H | OCH₂ COOC₂ H ₅ |
| 1-1405 | H | Cl | CH₃ | CF₃ | H | OCH₂ COO ⁿ C ₃ H ₇ |
| 1-1406 | H | Cl | CH₃ | CF₃ | H | OCH₂ COO ⁿ C ₄ H ₉ |
| 1-1407 | H | Cl | CH₃ | CF₃ | H | OCH₂ COO C₅ H ₁₁ |
| 1-1408 | H | Cl | CH₃ | CF₃ | H | OCH₂ COO ⁱ C ₃ H ₇ |
| 1-1909 | H | Cl | CH₃ | CF₃ | H | OCH₂ COO ^{c} C ₅ H ₉ |
| 1-1410 | H | Cl | CH₃ | CF₃ | H | OCH₂ COO ^{c} C ₆ H ₁₁ |
| 1-1411 | H | Cl | CH₃ | CF₃ | H | OCH(CH ₃ )COOCH ₃ |
| 1-1412 | H | Cl | CH₃ | CF₃ | H | OCH(CH ₃ )COOC ₂ H ₅ |
| 1-1413 | H | Cl | CH₃ | CF₃ | H | OCH(CH ₃ )COO ⁿ C₃ H ₇ |
| 1-1414 | H | Cl | CH₃ | CF₃ | H | OCH(CH ₃ )COO ⁿ C₄ H ₉ |
| 1-1415 | H | Cl | CH₃ | CF₃ | H | OCH(CH ₃ )COO ⁿ C₅ H ₁₁ |
| 1-1416 | H | Cl | CH₃ | CF₃ | H | OCH(CH ₃ )COO ⁱ C₃ H ₇ |
| 1-1417 | H | Cl | CH₃ | CF₃ | H | OCH(CH ₃ )COO ^{c} C₅ H ₉ |
| 1-1418 | H | Cl | CH₃ | CF₃ | H | OCH(CH ₃ )COO ^{c}C₅ H ₁₁ |
| 1-1419 | H | Cl | CH₃ | CF₃ | H | OCH₂ CON(CH₃ ) ₂ |
| 1-1420 | H | Cl | CH₃ | CF₃ | H | OCH₂ CON(C ₂ H ₅ ) ₂ |
| 1-1421 | H | Cl | CH₃ | CF₃ | H | OCH₂ CON(CH₃ ) C ₂ H ₅ |
| 1-1422 | H | Cl | CH₃ | CF₃ | H | OCH(CH ₃ )CON(CH ₃ ) ₂ |
| 1-1423 | H | Cl | CH₃ | CF₃ | H | OCH(CH₂ )CON(C ₂ H ₅ )₂ |
| 1-1424 | H | Cl | CH₃ | CF₃ | H | OCH(CH₃ )CON(CH₃ )C₂ H ₅ |
| 1-1425 | H | Cl | CH₃ | CF₃ | H | OCH 2 COON(CH₃) ₂ |
| 1-1426 | H | Cl | CH₃ | CF₃ | H | OCH ₂ COON(C ₂ H ₅ )₂ |
| 1-1427 | H | Cl | CH₃ | CF₃ | H | OCH(CH₃ )COON(CH₃)₂ |
| 1-1428 | H | Cl | CH₃ | CF₃ | H | OCH(CH₃ )COON(C ₂ H ₅ ) ₂ |
| 1-1429 | F | Cl | CH₃ | CF₃ | H | OCH₃ |
| 1-1430 | F | Cl | CH₃ | CF₃ | H | OC ₂ H ₅ |
| 1-1431 | F | Cl | CH₃ | CF₃ | H | O' C ₃ H ₇ |
| 1-1432 | F | Cl | CH₃ | CF₃ | H | Oⁿ C ₃ H ₇ |
| 1-1433 | F | Cl | CH₃ | CF₃ | H | OCH₂ CH₂ Cl |
| 1-1434 | F | Cl | CH₃ | CF₃ | H | OCF₂ CF₂ H |
| 1-1435 | F | Cl | CH₃ | CF₃ | H | O^{c} C ₅ H ₉ |
| 1-1436 | F | Cl | CH₃ | CF₃ | H | O^{c} C₆H₁₁ |
| 1-1437 | F | Cl | CH₃ | CF₃ | H | OCH₂ CH =CH₂ |
| 1-1438 | F | Cl | CH₃ | CF₃ | H | OCH₂ CCl = CH₂ |
| 1-1439 | F | Cl | CH₃ | CF₃ | H | OCH₂ CCl = CHCl |
| 1-1440 | F | Cl | CH₃ | CF₃ | H | OCH(CH₃)CH = CH₂ |
| 1-1441 | F | Cl | CH₃ | CF₃ | H | OCH₂ C ≡ CH |
| 1-1442 | F | Cl | CH₃ | CF₃ | H | OCH(CH₃)C≡CH |
| 1-1443 | F | Cl | CH₃ | CF₃ | H | OCH₂ C ≡ CBr |
| 1-1444 | F | Cl | CH₃ | CF₃ | H | OCH₂ C ≡ CCl |
| 1-1445 | F | Cl | CH₃ | CF₃ | H | OCH₂ C ≡ CCH₂ Cl |
| 1-1446 | F | Cl | CH₃ | CF₃ | H | OCH₂ CN |
| 1-1447 | F | Cl | CH₃ | CF₃ | H | OCH₂ OCH₃ |
| 1-1448 | F | Cl | CH₃ | CF₃ | H | OCH₂ OC₂ H ₅ |
| 1-1449 | F | Cl | CH₃ | CF₃ | H | OCH₂ SCH ₃ |
| 1-1450 | F | Cl | CH₃ | CF₃ | H | OCH₂ COOCH ₃ |
| 1-1451 | F | Cl | CH₃ | CF₃ | H | OCH₂ COOC₂ H ₅ |
| 1-1452 | F | Cl | CH₃ | CF₃ | H | OCH₂ COO ⁿ C₃ H ₇ |
| 1-1453 | F | Cl | CH₃ | CF₃ | H | OCH₂ COO ⁿ C₄ H ₉ |
| 1-1454 | F | Cl | CH₃ | CF₃ | H | OCH₂ COO ⁿ C ₅H₁₁ |
| 1-1455 | F | Cl | CH₃ | CF₃ | H | OCH₂ COO ⁱ C₃ H ₇ |
| 1-1456 | F | Cl | CH₃ | CF₃ | H | OCH₂ COO ^{c} C₅ H ₉ |
| 1-1457 | F | Cl | CH₃ | CF₃ | H | OCH₂ COO ^{c} C ₆H₁₁ |
| 1-1458 | F | Cl | CH₃ | CF₃ | H | OCH(CH ₃)COOCH₃ |
| 1-1459 | F | Cl | CH₃ | CF₃ | H | OCH(CH ₃)COOC₂ H ₅ |
| 1-1460 | F | Cl | CH₃ | CF₃ | H | OCH(CH ₃ )COO ⁿ C₃ H ₇ |
| 1-1461 | F | Cl | CH₃ | CF₃ | H | OCH(CH ₃ )COO ⁿ C₄ H ₉ |
| 1-1462 | F | Cl | CH₃ | CF₃ | H | OCH(CH₃ )COO ⁿ C₅ H₁₁ |
| 1-1463 | F | Cl | CH₃ | CF₃ | H | OCH(CH₃ )COO ⁱ C₃ H ₇ |
| 1-1464 | F | Cl | CH₃ | CF₃ | H | OCH(CH ₃ )COO ^{c}C₅ H ₉ |
| 1-1465 | F | Cl | CH₃ | CF₃ | H | OCH(CH₃) COO ^{c} C₆ H₁₁ |
| 1-1466 | F | Cl | CH₃ | CF₃ | H | OCH₂ CON(CH₃ ) ₂ |
| 1-1467 | F | Cl | CH₃ | CF₃ | H | OCH₂ CON(C ₂H₅ ) ₂ |
| 1-1468 | F | Cl | CH₃ | CF₃ | H | OCH₂ CON(CH₃ ) C ₂ H ₅ |
| 1-1469 | F | Cl | CH₃ | CF₃ | H | OCH(CH ₃)CON(CH₃ ) ₂ |
| 1-1470 | F | Cl | CH₃ | CF₃ | H | OCH(CH₃ )CON(C ₂ H ₅ )₂ |
| 1-1471 | F | Cl | CH₃ | CF₃ | H | OCH(CH₃ )CON(CH ₃ )C₂ H ₅ |
| 1-1472 | F | Cl | CH₃ | CF₃ | H | OCH ₂ COON(CH ₃ ) ₂ |
| 1-1473 | F | Cl | CH₃ | CF₃ | H | OCH ₂ COON(C ₂ H ₅)₂ |
| 1-1474 | F | Cl | CH₃ | CF₃ | H | OCH(CH₃ )COON(CH₃ ) ₂ |
| 1-1475 | F | Cl | CH₃ | CF₃ | H | OCH(CH₃ )COON(C ₂ H ₅ ) ₂ |
| 1-1476 | H | F | CH₃ | CF₃ | H | SH |
| 1-1477 | H | Cl | CH₃ | CF₃ | H | SH |
| 1-1478 | H | Br | CH₃ | CF₃ | H | SH |
| 1-1479 | F | F | CH₃ | CF₃ | H | SH |
| 1-1480 | F | Cl | CH₃ | CF₃ | H | SH |
| 1-1481 | F | Br | CH₃ | CF₃ | H | SH |
| 1-1482 | H | Cl | CH₃ | CF₃ | H | SCH ₃ |
| 1-1483 | H | Cl | CH₃ | CF₃ | H | SC₂ H₅ |
| 1-1484 | H | Cl | CH₃ | CF₃ | H | Sⁱ C₃ H₇ |
| 1-1485 | H | Cl | CH₃ | CF₃ | H | SCH₂ CH₂ Cl |
| 1-1486 | H | Cl | CH₃ | CF₃ | H | S^{c} C ₅ H₉ |
| 1-1487 | H | Cl | CH₃ | CF₃ | H | S^{c} C₆ H₁₁ |
| 1-1488 | H | Cl | CH₃ | CF₃ | H | SCH₂ CH=CH₂ |
| 1-1489 | H | Cl | CH₃ | CF₃ | H | SCH₂ CCl =CH₂ |
| 1-1490 | H | Cl | CH₃ | CF₃ | H | SCH₂ CCl = CHCl |
| 1-1491 | H | Cl | CH₃ | CF₃ | H | SCH(CH ₃ )CH = CH₂ |
| 1-1492 | H | Cl | CH₃ | CF₃ | H | SCH₂ C ≡ CH |
| 1-1493 | H | Cl | CH₃ | CF₃ | H | SCH (CH ₃ )C ≡ CH |
| 1-1494 | H | Cl | CH₃ | CF₃ | H | SCH₂ COOCH ₃ |
| 1-1495 | H | Cl | CH₃ | CF₃ | H | SCH₂ COOC₂ H ₅ |
| 1-1496 | H | Cl | CH₃ | CF₃ | H | SCH₂ COO ⁿ C ₃ H ₇ |
| 1-1497 | H | Cl | CH₃ | CF₃ | H | SCH₂ COO ⁿ C₄ H ₉ |
| 1-1498 | H | Cl | CH₃ | CF₃ | H | SCH₂ COO ⁿ C ₅ H ₁₁ |
| 1-1499 | H | Cl | CH₃ | CF₃ | H | SCH₂ COO ⁱ C ₃ H ₇ |
| 1-1500 | H | Cl | CH₃ | CF₃ | H | SCH₂ COO ^{c} C ₅ H ₉ |
| 1-1501 | H | Cl | CH₃ | CF₃ | H | SCH₂ COO ^{c} C ₆ H ₁₁ |
| 1-1502 | H | Cl | CH₃ | CF₃ | H | SCH(CH ₃)COOCH ₃ |
| 1-1503 | H | Cl | CH₃ | CF₃ | H | SCH(CH ₃ )COOC ₂ H ₅ |
| 1-1504 | H | Cl | CH₃ | CF₃ | H | SCH(CH₃)COO ⁿ C₃ H₇ |
| 1-1505 | H | Cl | CH₃ | CF₃ | H | SCH(CH₃)COO ⁿ C₄ H ₉ |
| 1-1506 | H | Cl | CH₃ | CF₃ | H | SCH(CH₃)COO ⁿ C₅ H₁₁ |
| 1-1507 | H | Cl | CH₃ | CF₃ | H | SCH(CH ₃)COO ⁱ C₃H ₇ |
| 1-1508 | H | Cl | CH₃ | CF₃ | H | SCH(CH₃)COO ^{c} C₅ H ₉ |
| 1-1509 | H | Cl | CH₃ | CF₃ | H | SCH(CH₃ )COO C₆ H₁₁ |
| 1-1510 | H | Cl | CH₃ | CF₃ | H | SCH₂ CON(CH₃) ₂ |
| 1-1511 | H | Cl | CH₃ | CF₃ | H | SCH₂ CON(C ₂ H ₅)₂ |
| 1-1512 | H | Cl | CH₃ | CF₃ | H | SCH₂CON (tetramethylene) |
| 1-1513 | H | Cl | CH₃ | CF₃ | H | SCH₂CON (pentamethylene) |
| 1-1514 | H | Cl | CH₃ | CF₃ | H | SCH₂CON (ethyleneoxyethylene) |
| 1-1515 | H | Cl | CH₃ | CF₃ | H | SCH(CH₃ )CON(CH ₃ ) ₂ |
| 1-1516 | H | Cl | CH₃ | CF₃ | H | SCH(CH₃ )CON(C₂ H₅ ) ₂ |
| 1-1517 | H | Cl | CH₃ | CF₃ | H | SCH(CH₃)CON (tetramethylene) |
| 1-1518 | H | Cl | CH₃ | CF₃ | H | SCH(CH₃)CON (pentamethylene) |
| 1-1519 | F | Cl | CH₃ | CF₃ | H | SCH₃ |
| 1-1520 | F | Cl | CH₃ | CF₃ | H | SC₂ H ₅ |
| 1-1521 | F | Cl | CH₃ | CF₃ | H | Sⁱ C₃ H₇ |
| 1-1522 | F | Cl | CH₃ | CF₃ | H | SCH₂ CH₂ Cl |
| 1-1523 | F | Cl | CH₃ | CF₃ | H | S^{c} C H ₉ |
| 1-1524 | F | Cl | CH₃ | CF₃ | H | S^{c} C ₆H₁₁ |
| 1-1525 | F | Cl | CH₃ | CF₃ | H | SCH₂ CH=CH₂ |
| 1-1526 | F | Cl | CH₃ | CF₃ | H | SCH₂ CCl = CH₂ |
| 1-1527 | F | Cl | CH₃ | CF₃ | H | SCH₂ CCI = CHCl |
| 1-1528 | F | Cl | CH₃ | CF₃ | H | SCH(CH₃)CH = CH₂ |
| 1-1529 | F | Cl | CH₃ | CF₃ | H | SCH₂ C ≡CH |
| 1-1530 | F | Cl | CH₃ | CF₃ | H | SCH(CH₃)C≡CH |
| 1-1531 | F | Cl | CH₃ | CF₃ | H | SCH₂ COOCH ₃ |
| 1-1532 | F | Cl | CH₃ | CF₃ | H | SCH₂ COOC₂ H₅ |
| 1-1533 | F | Cl | CH₃ | CF₃ | H | SCH₂ COO ⁿ C₃ H ₇ |
| 1-1534 | F | Cl | CH₃ | CF₃ | H | SCH₂ COO ⁿ C₄ H₉ |
| 1-1535 | F | Cl | CH₃ | CF₃ | H | SCH₂ COO ⁿ C ₅ H ₁₁ |
| 1-1536 | F | Cl | CH₃ | CF₃ | H | SCH₂ COO ⁱ C ₃ H ₇ |
| 1-1537 | F | Cl | CH₃ | CF₃ | H | SCH₂ COO ^{c} C ₅ H ₉ |
| 1-1538 | F | Cl | CH₃ | CF₃ | H | SCH₂ COO ^{c} C ₆ H ₁₁ |
| 1-1539 | F | Cl | CH₃ | CF₃ | H | SCH(CH ₃)COOCH₃ |
| 1-1540 | F | Cl | CH₃ | CF₃ | H | SCH(CH ₃)COOC ₂ H ₅ |
| 1-1541 | F | Cl | CH₃ | CF₃ | H | SCH(CH₃ )COO ⁿ C₃ H ₇ |
| 1-1542 | F | Cl | CH₃ | CF₃ | H | SCH(CH ₃ )COO ⁿ C₄ H ₉ |
| 1-1543 | F | Cl | CH₃ | CF₃ | H | SCH(CH ₃ )COO ⁿ C₅ H₁₁ |
| 1-1544 | F | Cl | CH₃ | CF₃ | H | SCH(CH ₃)COOⁱ C₃ H ₇ |
| 1-1545 | F | Cl | CH₃ | CF₃ | H | SCH(CH₃ )COO ^{c} C₅ H ₉ |
| 1-1546 | F | Cl | CH₃ | CF₃ | H | SCH(CH ₃)COO^{c} C₆ H ₁₁ |
| 1-1547 | F | Cl | CH₃ | CF₃ | H | SCH₂ CON(CH₃)₂ |
| 1-1548 | F | Cl | CH₃ | CF₃ | H | SCH₂ CON(C₂ H₅)₂ |
| 1-1549 | F | Cl | CH₃ | CF₃ | H | SCH₂CON(tetramethylene) |
| 1-1550 | F | Cl | CH₃ | CF₃ | H | SCH₂CON(pentamethylene) |
| 1-1551 | F | Cl | CH₃ | CF₃ | H | SCH₂CON (ethyleneoxyethylene) |
| 1-1552 | F | Cl | CH₃ | CF₃ | H | SCH(CH₃ )CON(CH₃)₂ |
| 1-1553 | F | Cl | CH₃ | CF₃ | H | SCH(CH₃ )CON(C₂ H ₅)₂ |
| 1-1554 | F | Cl | CH₃ | CF₃ | H | SCH(CH₃) CON (tetramethylene) |
| 1-1555 | F | Cl | CH₃ | CF₃ | H | SCH(CH₃)CON (pentamethylene) |
| 1-1556 | H | F | CH₃ | CF₃ | H | SO₂ Cl |
| 1-1557 | H | Cl | CH₃ | CF₃ | H | SO₂ Cl |
| 1-1558 | H | Br | CH₃ | CF₃ | H | SO₂ Cl |
| 1-1559 | F | F | CH₃ | CF₃ | H | SO₂ Cl |
| 1-1560 | F | Cl | CH₃ | CF₃ | H | SO₂ Cl |
| 1-1561 | F | Br | CH₃ | CF₃ | H | SO₂ Cl |
| 1-1562 | H | Cl | CH₃ | CF₃ | H | SO₂ OCH ₃ |
| 1-1563 | H | Cl | CH₃ | CF₃ | H | SO₂ OC₂ H ₅ |
| 1-1564 | H | Cl | CH₃ | CF₃ | H | SO₂ O ⁱ C ₃ H₇ |
| 1-1565 | H | Cl | CH₃ | CF₃ | H | SO₂ OCH ₂ CH = CH₂ |
| 1-1566 | F | Cl | CH₃ | CF₃ | H | SO₂ OCH₃ |
| 1-1567 | F | Cl | CH₃ | CF₃ | H | SO₂ OC₂ H ₅ |
| 1-1568 | F | Cl | CH₃ | CF₃ | H | SO₂ Oⁱ C₃ H ₇ |
| 1-1569 | F | Cl | CH₃ | CF₃ | H | SO₂ OCH ₂ CH = CH₂ |
| 1-1570 | H | Cl | CH₃ | CF₃ | H | SO₂ N(CH₃ ) ₂ |
| 1-1571 | H | Cl | CH₃ | CF₃ | H | SO₂ N (C₂ H ₅ ) ₂ |
| 1-1572 | F | Cl | CH₃ | CF₃ | H | SO₂ N (CH₃) ₂ |
| 1-1573 | F | Cl | CH₃ | CF₃ | H | SO₂ N (C₂ H ₅) ₂ |
| 1-1574 | H | Cl | CH₃ | CF₃ | H | COOH |
| 1-1575 | H | Cl | CH₃ | CF₃ | H | COOCH ₃ |
| 1-1576 | H | Cl | CH₃ | CF₃ | H | COOC ₂ H ₅ |
| 1-1577 | H | Cl | CH₃ | CF₃ | H | COOⁿ C ₃ H ₇ |
| 1-1578 | H | Cl | CH₃ | CF₃ | H | COOⁿ C ₄ H ₉ |
| 1-1579 | H | Cl | CH₃ | CF₃ | H | COOⁿ C ₅ H₁₁ |
| 1-1580 | H | Cl | CH₃ | CF₃ | H | COOⁱ C₃ H ₇ |
| 1-1581 | H | Cl | CH₃ | CF₃ | H | COOCH₂ CH₂ Cl |
| 1-1582 | H | Cl | CH₃ | CF₃ | H | COOCH₂ CH₂ Br |
| 1-1583 | H | Cl | CH₃ | CF₃ | H | CON(CH₃)₂ |
| 1-1584 | H | Cl | CH₃ | CF₃ | H | CONHCH₃ |
| 1-1585 | H | Cl | CH₃ | CF₃ | H | CON(C₂H ₅)₂ |
| 1-1586 | H | Cl | CH₃ | CF₃ | H | CONHC₂ H ₅ |
| 1-1587 | H | Cl | CH₃ | CF₃ | H | COCH₃ |
| 1-1588 | H | Cl | CH₃ | CF₃ | H | COC₂ H ₅ |
| 1-1589 | H | Cl | CH₃ | CF₃ | H | COCH₂ Cl |
| 1-1590 | H | Cl | CH₃ | CF₃ | H | CHO |
| 1-1591 | H | Cl | CH₃ | CF₃ | H | CH=CHCOOCH ₃ |
| 1-1592 | H | Cl | CH₃ | CF₃ | H | CH=CHCOOC₂ H ₅ |
| 1-1593 | H | Cl | CH₃ | CF₃ | H | CH₂ CH₂ COOCH ₃ |
| 1-1594 | H | Cl | CH₃ | CF₃ | H | CH₂ CH₂ COOC₂ H ₅ |
| 1-1595 | F | Cl | CH₃ | CF₃ | H | COOH |
| 1-1596 | F | Cl | CH₃ | CF₃ | H | COOCH ₃ |
| 1-1597 | F | Cl | CH₃ | CF₃ | H | COOC₂ H ₅ |
| 1-1598 | F | Cl | CH₃ | CF₃ | H | COOⁿ C₃ H₇ |
| 1-1599 | F | Cl | CH₃ | CF₃ | H | COOⁿ C ₄ H₉ |
| 1-1600 | F | Cl | CH₃ | CF₃ | H | COOⁿ C ₅ H₁₁ |
| 1-1601 | F | Cl | CH₃ | CF₃ | H | COOⁱ C₃ H ₇ |
| 1-1602 | F | Cl | CH₃ | CF₃ | H | COOCH₂ CH₂ Cl |
| 1-1603 | F | Cl | CH₃ | CF₃ | H | COOCH₂ CH₂ Br |
| 1-1604 | F | Cl | CH₃ | CF₃ | H | CON(CH ₃) ₂ |
| 1-1605 | F | Cl | CH₃ | CF₃ | H | CONHCH ₃ |
| 1-1606 | F | Cl | CH₃ | CF₃ | H | CON(C₂ H ₅ ) ₂ |
| 1-1607 | F | Cl | CH₃ | CF₃ | H | CONHC₂ H ₅ |
| 1-1608 | F | Cl | CH₃ | CF₃ | H | COCH ₃ |
| 1-1609 | F | Cl | CH₃ | CF₃ | H | COC₂ H ₅ |
| 1-1610 | F | Cl | CH₃ | CF₃ | H | COCH₂ Cl |
| 1-1611 | F | Cl | CH₃ | CF₃ | H | CHO |
| 1-1612 | F | Cl | CH₃ | CF₃ | H | CH= CHCOOCH ₃ |
| 1-1613 | F | Cl | CH₃ | CF₃ | H | CH=CHCOOC₂ H ₅ |
| 1-1614 | F | Cl | CH₃ | CF₃ | H | CH₂ CH₂ COOCH ₃ |
| 1-1615 | F | Cl | CH₃ | CF₃ | H | CH₂ CH₂ COOC₂ H ₅ |
| 1-1616 | H | F | CH₃ | CF₃ | H | H |
| 1-1617 | H | Cl | CH₃ | CF₃ | H | H |
| 1-1618 | H | Br | CH₃ | CF₃ | H | H |
| 1-1619 | F | F | CH₃ | CF₃ | H | H |
| 1-1620 | F | Cl | CH₃ | CF₃ | H | H |
| 1-1621 | F | Br | CH₃ | CF₃ | H | H |
| 1-1622 | F | Cl | H | CF₃ | CH₃ | CH₂ CHClCO₂ C₂H₅ |
| 1-1623 | F | Cl | H | CF₃ | H | CH₂ CHClCO₂ C₂H₅ |
| 1-1624 | F | Cl | H | CF₃ | CH₃ | CH₂ CHClCO₂ CH₃ |
| 1-1625 | F | Cl | H | CF₃ | H | CH₂ CHClCO₂ CH₃ |
| 1-1626 | F | Cl | H | CF₃ | H | OCH₂ CO₂ H |
| 1-1627 | F | Cl | H | CF₃ | CH₃ | OCH₂ CO₂ H |
| 1-1628 | F | Cl | CH₃ | CF₃ | H | OCH₂ CO₂ H |
| 1-1629 | F | Cl | H | CF₃ | H | OCH(CH₃ ) CO₂ H |
| 1-1630 | F | Cl | H | CF₃ | CH₃ | OCH(CH₃) CO₂ H |
| 1-1631 | F | Cl | CH₃ | CF₃ | H | OCH(CH₃) CO₂ H |
| 1-1632 | H | Cl | H | CF₃ | H | OCH₂ CO₂ H |
| 1-1633 | H | Cl | H | CF₃ | CH₃ | OCH₂ CO₂ H |
| 1-1634 | H | Cl | CH₃ | CF₃ | H | OCH₂ CO₂ H |
| 1-1635 | H | Cl | H | CF₃ | H | OCH(CH₃) CO₂ H |
| 1-1636 | H | Cl | H | CF₃ | CH₃ | OCH(CH₃) CO₂ H |
| 1-1637 | H | Cl | CH₃ | CF₃ | H | OCH(CH₃) CO₂ H |
| 1-1638 | F | Cl | H | CF₃ | H | SCH₂ CO₂ H |
| 1-1639 | F | Cl | H | CF₃ | CH₃ | SCH₂ CO₂ H |
| 1-1640 | F | Cl | CH₃ | CF₃ | H | SCH₂ CO₂ H |
| 1-1641 | F | Cl | H | CF₃ | H | SCH(CH₃ ) CO₂ H |
| 1-1642 | F | Cl | H | CF₃ | CH₃ | SCH(CH₃ ) CO₂ H |
| 1-1643 | F | Cl | CH₃ | CF₃ | H | SCH(CH₃ ) CO₂ H |
| 1-1644 | H | Cl | H | CF₃ | H | SCH₂ CO₂ H |
| 1-1645 | H | Cl | H | CF₃ | CH₃ | SCH₂ CO₂ H |
| 1-1646 | H | Cl | CH₃ | CF₃ | H | SCH₂ CO₂ H |
| 1-1647 | H | Cl | H | CF₃ | H | SCH(CH₃ ) CO₂ H |
| 1-1648 | H | Cl | H | CF₃ | CH₃ | SCH(CH₃) CO₂ H |
| 1-1649 | H | Cl | CH₃ | CF₃ | H | SCH(CH₃) CO₂ H |
| 1-1650 | F | Cl | H | CF₃ | CH₃ | OCH (C₂ H ₅ )CO ₂ CH₃ |
| 1-1651 | Cl | Cl | H | CF₃ | H | OCH₂ CO₂ CH₃ |
| 1-1652 | Cl | | H | CF₃ | H | OCH₂CO₂C₂H₅ |
| 1-1653 | Cl | Cl | H | CF₃ | H | OCH₂CO₂ ⁱC₃H₇ |
| 1-1654 | Cl | Cl | H | CF₃ | CH₃ | OCH₂CO₂CH₃ |
| 1-1655 | Cl | Cl | H | CF₃ | CH₃ | OCH₂CO₂C₂H₅ |
| 1-1656 | Cl | Cl | H | CF₃ | CH₃ | OCH₂CO₂ ⁱC₃H₇ |
| 1-1657 | Cl | Cl | CH₃ | CF₃ | H | OCH₂CO₂CH₃ |
| 1-1658 | Cl | Cl | CH₃ | CF₃ | H | OCH₂CO₂C₂H₅ |
| 1-1659 | Cl | Cl | CH₃ | CF₃ | H | OCH₂CO₂ C₃H₇ |
| 1-1660 | Cl | Cl | H | CF₃ | H | OCH₂C ≡ CH |
| 1-1661 | Cl | Cl | H | CF₃ | H | OCH(CH₃)C ≡CH |
| 1-1662 | Cl | Cl | H | CF₃ | H | OCH(CH₃)CO₂C₂H₅ |
| 1-1663 | Cl | Cl | H | CF₃ | CH₃ | OCH₂C ≡CH |
| 1-1664 | Cl | Cl | H | CF₃ | CH₃ | OCH(CH ₃)C ≡CH |
| 1-1665 | Cl | Cl | H | CF₃ | CH₃ | OCH(CH ₃ )CO₂C₂H₅ |
| 1-1666 | Cl | Cl | CH₃ | CF₃ | H | OCH₂C ≡CH |
| 1-1667 | Cl | Cl | CH₃ | CF₃ | H | OCH(CH ₃)C ≡ CH |
| 1-1668 | Cl | Cl | CH₃ | CF₃ | H | OCH(CH₃ )CO₂C₂H₅ |
| 1-1669 | F | Br | H | CF₃ | H | OCH₂CO₂CH₃ |
| 1-1670 | F | Br | H | CF₃ | H | OCH₂CO₂C₂H₅ |
| 1-1671 | F | Br | H | CF₃ | H | OCH₂CO₂ C₃H₇ |
| 1-1672 | F | Br | H | CF₃ | CH₃ | OCH₂ CO₂CH₃ |
| 1-1673 | F | Br | H | CF₃ | CH₃ | OCH₂CO₂C₂H₅ |
| 1-1674 | F | Br | H | CF₃ | CH₃ | OCH₂CO₂ ⁱ C₃H₇ |
| 1-1675 | F | Br | CH₃ | CF₃ | H | OCH₂CO₂CH₃ |
| 1-1676 | F | Br | CH₃ | CF₃ | H | OCH₂CO₂C₂H₅ |
| 1-1677 | F | Br | CH₃ | CF₃ | H | OCH₂CO₂ C₃H₇ |
| 1-1678 | F | Br | H | CF₃ | H | OCH₂C ≡CH |
| 1-1679 | F | Br | H | CF₃ | H | OCH(CH ₃ )C ≡ CH |
| 1-1680 | F | Br | H | CF₃ | H | OCH(CH ₃)CO₂C₂H₅ |
| 1-1681 | F | Br | H | CF₃ | CH₃ | OCH₂C ≡ CH |
| 1-1682 | F | Br | H | CF₃ | CH₃ | OCH(CH ₃)C ≡CH |
| 1-1683 | F | Br | H | CF₃ | CH₃ | OCH(CH ₃)CO₂C₂H₅ |
| 1-1684 | F | Br | CH₃ | CF₃ | H | OCH₂C ≡ CH |
| 1-1685 | F | Br | CH₃ | CF₃ | H | OCH(CH₃ )C ≡CH |
| 1-1686 | F | Br | CH₃ | CF₃ | H | OCH(CH ₃ )CO₂C₂H₅ |
| 1-1687 | Cl | Cl | H | CF₃ | H | H |
| 1-1688 | Cl | Cl | H | CF₃ | H | OH |
| 1-1689 | Cl | Cl | H | CF₃ | H | NO₂ |
| 1-1690 | Cl | Cl | H | CF₃ | H | NH₂ |
| 1-1691 | Cl | Cl | H | CF₃ | H | NHSO₂CH₃ |
| 1-1692 | Cl | Cl | H | CF₃ | H | NHSO₂CH₂Cl |
| 1-1693 | Cl | Cl | H | CF₃ | H | NHCH₂CO₂CH₃ |
| 1-1694 | Cl | Cl | H | CF₃ | H | NHCH₂CO₂C₂H₅ |
| | | | | | | |
| 1-1695 | Cl | Cl | H | CF₃ | H | NHCH₂CO₂ⁱC₃H₇ |
| 1-1696 | Cl | Cl | H | CF₃ | H | NHCH(CH₃)CO₂CH₃ |
| 1-1697 | Cl | Cl | H | CF₃ | H | NHCH(CH₃)CO₂C₂H₅ |
| | | | | | | |
| 1-1698 | Cl | Cl | H | CF₃ | H | NHCH(CH₃)CO₂ⁱC₃H₇ |
| 1-1699 | Cl | Cl | H | CF₃ | H | CO₂H |
| 1-1700 | Cl | Cl | H | CF₃ | H | CO₂CH₃ |
| 1-1701 | Cl | Cl | H | CF₃ | H | CO₂C₂H₅ |
| | | | | | | |
| 1-1702 | Cl | Cl | H | CF₃ | H | CO₂ⁿC₃H₇ |
| | | | | | | |
| 1-1703 | Cl | Cl | H | CF₃ | H | CO₂ⁿC₄H₉ |
| | | | | | | |
| 1-1704 | Cl | Cl | H | CF₃ | H | CO₂ⁿC₅H₁₁ |
| 1-1705 | Cl | Cl | H | CF₃ | H | CO₂ⁱC₃H₇ |
| | | | | | | |
| 1-1706 | Cl | Cl | H | CF₃ | H | CO₂CH₂CH₂Cl |
| 1-1707 | Cl | Cl | H | CF₃ | H | CO₂CH₂CH₂Br |
| 1-1708 | Cl | Cl | H | CF₃ | H | CON(CH₃)₂ |
| 1-1709 | Cl | Cl | H | CF₃ | H | CONHCH₃ |
| 1-1710 | Cl | Cl | H | CF₃ | H | CON(C₂H₅)₂ |
| 1-1711 | Cl | Cl | H | CF₃ | H | CONHC₂H₅ |
| 1-1712 | Cl | Cl | H | CF₃ | H | COCH₃ |
| 1-1713 | Cl | Cl | H | CF₃ | H | COC₂H₅ |
| 1-1714 | Cl | Cl | H | CF₃ | H | COCH₂Cl |
| 1-1715 | Cl | Cl | H | CF₃ | H | CHO |
| 1-1716 | Cl | Cl | H | CF₃ | H | CH=CHCO₂CH₃ |
| | | | | | | |
| 1-1717 | Cl | Cl | H | CF₃ | H | OⁱC₃H₇ |
| 1-1718 | Cl | Cl | H | CF₃ | CH₃ | H |
| 1-1719 | Cl | Cl | H | CF₃ | CH₃ | OH |
| | | | | | | |
| 1-1720 | Cl | Cl | H | CF₃ | CH₃ | NO₂ |
| 1-1721 | Cl | Cl | H | CF₃ | CH₃ | NH₂ |
| 1-1722 | Cl | Cl | H | CF₃ | CH₃ | NHSO₂CH₃ |
| 1-1723 | Cl | Cl | H | CF₃ | CH₃ | NHSO₂CH₂Cl |
| 1-1724 | Cl | Cl | H | CF₃ | CH₃ | NHCH₂CO₂CH₃ |
| 1-1725 | Cl | Cl | H | CF₃ | CH₃ | NHCH₂CO₂C₂H₅ |
| | | | | | | |
| 1-1726 | Cl | Cl | H | CF₃ | CH₃ | NHCH₂CO₂ⁱC₃H₇ |
| 1-1727 | Cl | Cl | H | CF₃ | CH₃ | NHCH(CH₃)CO₂CH₃ |
| 1-1728 | Cl | Cl | H | CF₃ | CH₃ | NHCH(CH₃)CO₂C₂H₅ |
| | | | | | | |
| 1-1729 | Cl | Cl | H | CF₃ | CH₃ | NHCH(CH₃)CO₂ⁱC₃H₇ |
| 1-1730 | Cl | Cl | H | CF₃ | CH₃ | CO₂H |
| 1-1731 | Cl | Cl | H | CF₃ | CH₃ | CO₂CH₃ |
| 1-1732 | Cl | Cl | H | CF₃ | CH₃ | CO₂C₂H₅ |
| | | | | | | |
| 1-1733 | Cl | Cl | H | CF₃ | CH₃ | CO₂ⁿC₃H₇ |
| | | | | | | |
| 1-1734 | Cl | Cl | H | CF₃ | CH₃ | CO₂ⁿC₄H₉ |
| 1-1735 | Cl | Cl | H | CF₃ | CH₃ | CO₂ⁿC₅H₁₁ |
| | | | | | | |
| 1-1736 | Cl | Cl | H | CF₃ | CH₃ | CO₂ⁱC₃H₇ |
| 1-1737 | Cl | Cl | H | CF₃ | CH₃ | CO₂CH₂CH₂Cl |
| 1-1738 | Cl | Cl | H | CF₃ | CH₃ | CO₂CH₂CH₂Br |
| 1-1739 | Cl | Cl | H | CF₃ | CH₃ | CON(CH₃)₂ |
| 1-1740 | Cl | Cl | H | CF₃ | CH₃ | CONHCH₃ |
| 1-1741 | Cl | Cl | H | CF₃ | CH₃ | CON(C₂H₅) ₂ |
| 1-1742 | Cl | Cl | H | CF₃ | CH₃ | CONHC₂H₅ |
| 1-1743 | Cl | Cl | H | CF₃ | CH₃ | COCH₃ |
| 1-1744 | Cl | Cl | H | CF₃ | CH₃ | COC₂H₅ |
| 1-1745 | Cl | Cl | H | CF₃ | CH₃ | COCH₂Cl |
| 1-1746 | Cl | Cl | H | CF₃ | CH₃ | CHO |
| 1-1747 | Cl | Cl | H | CF₃ | CH₃ | CH=CHCO₂CH₃ |
| | | | | | | |
| 1-1748 | Cl | Cl | H | CF₃ | CH₃ | OⁱC₃H₇ |
| 1-1749 | Cl | Cl | CH₃ | CF₃ | H | H |
| 1-1750 | Cl | Cl | CH₃ | CF₃ | H | OH |
| 1-1751 | Cl | Cl | CH₃ | CF₃ | H | NO₂ |
| 1-1752 | Cl | Cl | CH₃ | CF₃ | H | NH₂ |
| | | | | | | |
| 1-1753 | Cl | Cl | CH₃ | CF₃ | H | NHSO₂CH₃ |
| 1-1754 | Cl | Cl | CH₃ | CF₃ | H | NHSO₂CH₂Cl |
| 1-1755 | Cl | Cl | CH₃ | CF₃ | H | NHCH₂CO₂CH₃ |
| 1-1756 | Cl | Cl | CH₃ | CF₃ | H | NHCH₂CO₂C₂H₅ |
| | | | | | | |
| 1-1757 | Cl | Cl | CH₃ | CF₃ | H | NHCH₂CO₂ⁱC₃H₇ |
| 1-1758 | Cl | Cl | CH₃ | CF₃ | H | NHCH(CH₃)CO₂CH₃ |
| 1-1759 | Cl | Cl | CH₃ | CF₃ | H | NHCH(CH₃)CO₂C₂H₅ |
| | | | | | | |
| 1-1760 | Cl | Cl | CH₃ | CF₃ | H | NHCH(CH₃)COⁱ₂C₃H₇ |
| 1-1761 | Cl | Cl | CH₃ | CF₃ | H | CO₂H |
| 1-1762 | Cl | Cl | CH₃ | CF₃ | H | CO₂CH₃ |
| 1-1763 | Cl | Cl | CH₃ | CF₃ | H | CO₂C₂H₅ |
| 1-1764 | Cl | Cl | CH₃ | CF₃ | H | CO₂ⁿC₃H₇ |
| | | | | | | |
| 1-1765 | Cl | Cl | CH₃ | CF₃ | H | CO₂ⁿC₄H₉ |
| | | | | | | |
| 1-1766 | Cl | Cl | CH₃ | CF₃ | H | CO₂ⁿC₅H₁₁ |
| 1-1767 | Cl | Cl | CH₃ | CF₃ | H | CO₂ⁱC₃H₇ |
| 1-1768 | Cl | Cl | CH₃ | CF₃ | H | CO₂CH₂CH₂Cl |
| 1-1769 | Cl | Cl | CH₃ | CF₃ | H | CO₂CH₂CH₂Br |
| 1-1770 | Cl | Cl | CH₃ | CF₃ | H | CON(CH₃)₂ |
| 1-1771 | Cl | Cl | CH₃ | CF₃ | H | CONHCH₃ |
| 1-1772 | Cl | Cl | CH₃ | CF₃ | H | CON(C₂H₅)₂ |
| 1-1773 | Cl | Cl | CH₃ | CF₃ | H | CONHC₂H₅ |
| 1-1774 | Cl | Cl | CH₃ | CF₃ | H | COCH₃ |
| 1-1775 | Cl | Cl | CH₃ | CF₃ | H | COC₂H₅ |
| 1-1776 | Cl | Cl | CH₃ | CF₃ | H | COCH₂Cl |
| 1-1777 | Cl | Cl | CH₃ | CF₃ | H | CHO |
| 1-1778 | Cl | Cl | CH₃ | CF₃ | H | CH=CHCO₂CH₃ |
| | | | | | | |
| 1-1779 | Cl | Cl | CH₃ | CF₃ | H | OⁱC₃H₇ |
| | | | | | | |
| 1-1780 | F | Br | H | CF₃ | H | OⁱC₃H₇ |
| 1-1781 | F | Br | H | CF₃ | H | N(SO₂CH₃)₂ |
| 1-1782 | F | Br | H | CF₃ | H | NHSO₂CH₃ |
| | | | | | | |
| 1-1783 | F | Br | H | CF₃ | CH₃ | OⁱC₃H₇ |
| 1-1784 | F | Br | H | CF₃ | CH₃ | N(SO₂CH₃)₂ |
| 1-1785 | F | Br | H | CF₃ | CH₃ | NHSO₂CH₃ |
| 1-1786 | F | Br | CH₃ | CF₃ | H | OⁱC₃H₇ |
| 1-1787 | F | Br | CH₃ | CF₃ | H | N(SO₂CH₃)₂ |
| 1-1788 | F | Br | CH₃ | CF₃ | H | NHSO₂CH₃ |
| 1-1789 | F | Cl | H | CF₃ | CH₃ | OCH₂C(CH₃)=CH₂ |
| 1-1790 | F | Cl | H | CF₃ | CH₃ | OCH₂CO₂C₇H₁₅ |
| 1-1791 | F | Cl | H | CF₃ | CH₃ | OCH₂CO₂C₈H₁₇ |
| 1-1792 | F | Cl | H | CF₃ | CH₃ | COOCH₂C₆H₅ |
| 1-1793 | F | Cl | H | CF₃ | CH₃ | C(CH₃)=NOH |
| 1-1794 | F | Cl | H | CF₃ | CH₃ | C(CH₃)=NOCH₃ |
| 1-1795 | F | Cl | H | CF₃ | CH₃ | C(CH₃)=NOC₂H₅ |
| | | | | | | |
| 1-1796 | F | Cl | H | CF₃ | CH₃ | C(CH₃)=NOⁱC₃H₇ |
| 1-1797 | F | Cl | H | CF₃ | CH₃ | C(C₂H₅)=NOH |
| 1-1798 | F | Cl | H | CF₃ | CH₃ | C(C₂H₅)=NOCH₃ |
| 1-1799 | F | Cl | H | CF₃ | CH₃ | C(C₂H₅)=NOC₂H₅ |
| | | | | | | |
| 1-1800 | F | Cl | H | CF₃ | CH₃ | C(C₂H₅)=NOⁱC₃H₇ |
| 1-1801 | F | Cl | H | CF₃ | CH₃ | C(CH₃)=NNH₂ |
| 1-1802 | F | Cl | H | CF₃ | CH₃ | C(CH₃)=NNHCH₃ |
| 1-1803 | F | Cl | H | CF₃ | CH₃ | C(CH₃)=NN(CH₃)₂ |
| 1-1804 | F | Cl | H | CF₃ | CH₃ | C(CH₃)=NNHC₂H₅ |
| 1-1805 | F | Cl | H | CF₃ | CH₃ | C(CH₃)=NN(C₂H₅)₂ |
| 1-1806 | F | Cl | H | CF₃ | CH₃ | C(C₂H₅)=NNH₂ |
| 1-1807 | F | Cl | H | CF₃ | CH₃ | C(C₂H₅)=NNHCH₃ |
| 1-1808 | F | Cl | H | CF₃ | CH₃ | C(C₂H₅)=NN(CH₃)₂ |
| 1-1809 | F | Cl | H | CF₃ | CH₃ | C(C₂H₅)=NNHC₂H₅ |
| 1-1810 | F | Cl | H | CF₃ | CH₃ | C(C₂H₅)=NN(C₂H₅)₂ |
| 1-1811 | F | Cl | H | CF₃ | CH₃ | C(CH₃)(OCH₃)₂ |
| 1-1812 | F | Cl | H | CF₃ | CH₃ | C(CH₃)(OC₂H₅)₂ |
| | | | | | | |
| 1-1813 | F | Cl | H | CF₃ | CH₃ | C(CH₃)(CⁱC)H₇)₂ |
| 1-1814 | F | Cl | H | CF₃ | CH₃ | 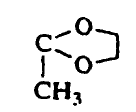 |
| 1-1815 | F | Cl | H | CF₃ | CH₃ | 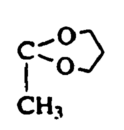 |
| 1-1816 | F | Cl | H | CF₃ | CH₃ | 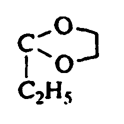 |
| 1-1817 | F | Cl | H | CF₃ | CH₃ | 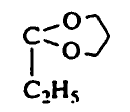 |
| 1-1818 | F | Cl | H | CF₃ | H | OCH₂C(CH₃)=CH₂ |
| 1-1819 | F | Cl | H | CF₃ | H | OCH₂CO₂C₇H₁₅ |
| 1-1820 | F | Cl | H | CF₃ | H | OCH₂CO₂C₈H₁₇ |
| 1-1821 | F | Cl | H | CF₃ | H | COOCH₂C₆H₅ |
| 1-1822 | F | Cl | H | CF₃ | H | C(CH₃)=NOH |
| 1-1823 | F | Cl | H | CF₃ | H | C(CH₃)=NOCH₃ |
| 1-1824 | F | Cl | H | CF₃ | H | C(CH₃)=NOC₂H₅ |
| | | | | | | |
| 1-1825 | F | Cl | H | CF₃ | H | C(CH₃)=NOⁱC₃H₇ |
| 1-1826 | F | Cl | H | CF₃ | H | C(C₂H₅)=NOH |
| 1-1827 | F | Cl | H | CF₃ | H | C(C₂H₅)=NOCH₃ |
| 1-1828 | F | Cl | H | CF₃ | H | C(C₂H₅)=NOC₂H₅ |
| | | | | | | |
| 1-1829 | F | Cl | H | CF₃ | H | C(C₂H₅)=NOⁱC₃H₇ |
| 1-1830 | F | Cl | H | CF₃ | H | C(CH₃)=NNH₂ |
| 1-1831 | F | Cl | H | CF₃ | H | C(CH₃)=NNHCH₃ |
| 1-1832 | F | Cl | H | CF₃ | H | C(CH₃)=NN(CH₃)₂ |
| 1-1833 | F | Cl | H | CF₃ | H | C(CH₃)=NNHC₂H₅ |
| 1-1834 | F | Cl | H | CF₃ | H | C(CH₃)=NN(C₂H₅)₂ |
| 1-1835 | F | Cl | H | CF₃ | H | C(C₂H₅)=NNH₂ |
| 1-1836 | F | Cl | H | CF₃ | H | C(C₂H₅)=NNHCH₃ |
| 1-1837 | F | Cl | H | CF₃ | H | C(C₂H₅)=NN(CH₃)₂ |
| 1-1838 | F | Cl | H | CF₃ | H | C(C₂H₅)=NNHC₂H₅ |
| 1-1839 | F | Cl | H | CF₃ | H | C(C₂H₅)=NN(C₂H₅)₂ |
| 1-1840 | F | Cl | H | CF₃ | H | C(CH₃)(OCH₃)₂ |
| 1-1841 | F | Cl | H | CF₃ | H | C(CH₃)(OC₂H₅)₂ |
| 1-1842 | F | Cl | H | CF₃ | H | C(CH₃)(CⁱC₃H₇)₂ |
| 1-1843 | F | Cl | H | CF₃ | H | 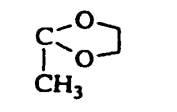 |
| 1-1844 | F | Cl | H | CF₃ | H | 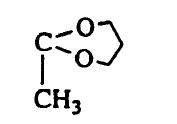 |
| 1-1845 | F | Cl | H | CF₃ | H | 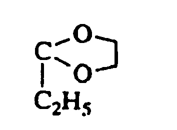 |
| 1-1846 | F | Cl | H | CF₃ | H | 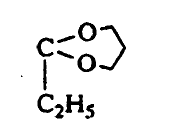 |
| 1-1847 | F | Cl | CH₃ | CF₃ | H | OCH₂C(CH₃)=CH₂ |
| 1-1848 | F | Cl | CH₃ | CF₃ | H | OCH₂CO₂C₇H₁₅ |
| 1-1849 | F | Cl | CH₃ | CF₃ | H | OCH₂CO₂C₈H₁₇ |
| 1-1850 | F | Cl | CH₃ | CF₃ | H | COOCH₂C₆H₅ |
| 1-1851 | F | Cl | CH₃ | CF₃ | H | C(CH₃)=NOH |
| 1-1852 | F | Cl | CH₃ | CF₃ | H | C(CH₃)=NOCH₃ |
| 1-1853 | F | Cl | CH₃ | CF₃ | H | C(CH₃)=NOC₂H₅ |
| | | | | | | |
| 1-1854 | F | Cl | CH₃ | CF₃ | H | C(CH₃)=NOⁱC₃H₇ |
| 1-1855 | F | Cl | CH₃ | CF₃ | H | C(C₂H₅)=NOH |
| 1-1856 | F | Cl | CH₃ | CF₃ | H | C(C₂H₅)=NOCH₃ |
| 1-1857 | F | Cl | CH₃ | CF₃ | H | C(C₂H₅)=NOC₂H₅ |
| | | | | | | |
| 1-1858 | F | Cl | CH₃ | CF₃ | H | C(C₂H₅)=NOⁱC₃H₇ |
| 1-1859 | F | Cl | CH₃ | CF₃ | H | C(CH₃)=NNH₂ |
| 1-1860 | F | Cl | CH₃ | CF₃ | H | C(CH₃)=NNHCH₃ |
| 1-1861 | F | Cl | CH₃ | CF₃ | H | C(CH₃)=NN(CH₃)₂ |
| 1-1862 | F | Cl | CH₃ | CF₃ | H | C(CH₃)=NNHC₂H₅ |
| 1-1863 | F | Cl | CH₃ | CF₃ | H | C(CH₃)=NN(C₂H₅)₂ |
| 1-1864 | F | Cl | CH₃ | CF₃ | H | C(C₂H₅)=NNH₂ |
| 1-1865 | F | Cl | CH₃ | CF₃ | H | C(C₂H₅)=NNHCH₃ |
| 1-1866 | F | Cl | CH₃ | CF₃ | H | C(C₂H₅)=NN(CH₃)₂ |
| 1-1867 | F | Cl | CH₃ | CF₃ | H | C(C₂H₅)=NNHC₂H₅ |
| 1-1868 | F | Cl | CH₃ | CF₃ | H | C(C₂H₅)=NN(C₂H₅)₂ |
| 1-1869 | F | Cl | CH₃ | CF₃ | H | C(CH₃)(OCH₃)₂ |
| 1-1870 | F | Cl | CH₃ | CF₃ | H | C(CH₃)(OC₂H₅)₂ |
| | | | | | | |
| 1-1871 | F | Cl | CH₃ | CF₃ | H | C(CH₃)(CⁱC₃H₇)₂ |
| 1-1872 | F | Cl | CH₃ | CF₃ | H | 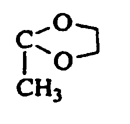 |
| 1-1873 | F | Cl | CH₃ | CF₃ | H | 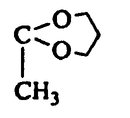 |
| 1-1874 | F | Cl | CH₃ | CF₃ | H | 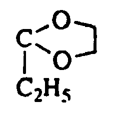 |
| 1-1875 | F | Cl | CH₃ | CF₃ | H | 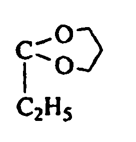 |
| 1-1876 | H | Cl | H | CF₃ | CH₃ | OCH₂C(CH₃)=CH₂ |
| 1-1877 | H | Cl | H | CF₃ | CH₃ | OCH₂CO₂C₇H₁₅ |
| 1-1878 | H | Cl | H | CF₃ | CH₃ | OCH₂CO₂C₈H₁₇ |
| 1-1879 | H | Cl | H | CF₃ | CH₃ | COOCH₂C₆H₅ |
| 1-1880 | H | Cl | H | CF₃ | CH₃ | C(CN₃)=NOH |
| 1-1881 | H | Cl | H | CF₃ | CH₃ | C(CH₃)=NOCH₃ |
| 1-1882 | H | Cl | H | CF₃ | CH₃ | C(CH₃)=NOC₂H₅ |
| | | | | | | |
| 1-1883 | H | Cl | H | CF₃ | CH₃ | C(CH₃)=NOⁱ C₃H₇ |
| 1-1884 | H | Cl | H | CF₃ | CH₃ | C(C₂H₅)=NOH |
| 1-1885 | H | Cl | H | CF₃ | CH₃ | C(C₂H₅)=NOCH₃ |
| 1-1886 | H | Cl | H | CF₃ | CH₃ | C(C₂H₅)=NOC₂H₅ |
| | | | | | | |
| 1-1887 | H | Cl | H | CF₃ | CH₃ | C(C₂H₅)=NOⁱC₃H₇ |
| 1-1888 | H | Cl | H | CF₃ | CH₃ | C(CH₃)=NNH₂ |
| 1-1889 | H | Cl | H | CF₃ | CH₃ | C(CH₃)=NNHCH₃ |
| 1-1890 | H | Cl | H | CF₃ | CH₃ | C(CH₃)=NN(CH₃)₂ |
| 1-1891 | H | Cl | H | CF₃ | CH₃ | C(CH₃)=NNHC₂H₅ |
| 1-1892 | H | Cl | H | CF₃ | CH₃ | C(CH₃)=NN(C₂H₅)₂ |
| 1-1893 | H | Cl | H | CF₃ | CH₃ | C(C₂H₅)=NNH₂ |
| 1-1894 | H | Cl | H | CF₃ | CH₃ | C(C₂H₅)=NNHCH₃ |
| 1-1895 | H | Cl | H | CF₃ | CH₃ | C(C₂H₅)=NN(CH₃)₂ |
| 1-1896 | H | Cl | H | CF₃ | CH₃ | C(C₂H₅)-ONNHC₂H₅ |
| 1-1897 | H | Cl | H | CF₃ | CH₃ | C(C₂H₅)=NN(C₂H₅)₂ |
| 1-1898 | H | Cl | H | CF₃ | CH₃ | C(CH₃)(OCH₃)₂ |
| 1-1899 | H | Cl | H | CF₃ | CH₃ | C(CH₃)(OC₂H₅)₂ |
| | | | | | | |
| 1-1900 | H | Cl | H | CF₃ | CH₃ | C(CH₃)(CⁱC₃H₇)₂ |
| 1-1901 | H | Cl | H | CF₃ | CH₃ | 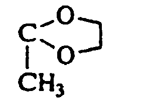 |
| 1-1902 | H | Cl | H | CF₃ | CH₃ | 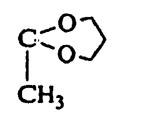 |
| 1-1903 | H | Cl | H | CF₃ | CH₃ | 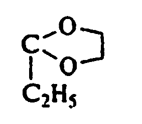 |
| 1-1904 | H | Cl | H | CF₃ | CH₃ | 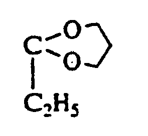 |
| 1-1905 | H | Cl | H | CF₃ | H | OCH₂C(CH₃)=CH₂ |
| 1-1906 | H | Cl | H | CF₃ | H | OCH₂CO₂C₇H₁₅ |
| 1-1907 | H | Cl | H | CF₃ | H | OCH₂CO₂C₈H₁₇ |
| 1-1908 | H | Cl | H | CF₃ | H | COOCH₂C₆H₅ |
| 1-1909 | H | Cl | H | CF₃ | H | C(CH₃)=NOH |
| 1-1910 | H | Cl | H | CF₃ | H | C(CH₃)=NOCH₃ |
| 1-1911 | H | Cl | H | CF₃ | H | C(CH₃)=NOC₂H₅ |
| | | | | | | |
| 1-1912 | H | Cl | H | CF₃ | H | C(CH₃)=NOⁱC₃H₇ |
| 1-1913 | H | Cl | H | CF₃ | H | C(C₂H₅)=NOH |
| 1-1914 | H | Cl | H | CF₃ | H | C(C₂H₅)=NOCH₃ |
| 1-1915 | H | Cl | H | CF₃ | H | C(C₂H₅)=NOC₂H₅ |
| | | | | | | |
| 1-1916 | H | Cl | H | CF₃ | H | C(C₂H₅)=NOⁱC₃H₇ |
| 1-1917 | H | Cl | H | CF₃ | H | C(CH₃)=NNH₂ |
| 1-1918 | H | Cl | H | CF₃ | H | C(CH₃)=NNHCH₃ |
| 1-1919 | H | Cl | H | CF₃ | H | C(CH₃)=NN(CH₃)₂ |
| 1-1920 | H | Cl | H | CF₃ | H | C(CH₃)=NNHC₂H₅ |
| 1-1921 | H | Cl | H | CF₃ | H | C(CH₃)=NN(C₂H₅)₂ |
| 1-1922 | H | Cl | H | CF₃ | H | C(C₂H₅)=NNH₂ |
| 1-1923 | H | Cl | H | CF₃ | H | C(C₂H₅)=NNHCH₃ |
| 1-1924 | H | Cl | H | CF₃ | H | C (C₂H₅) =NN (CH₃) ₂ |
| 1-1925 | H | Cl | H | CF₃ | H | C(C₂H₅)=NNHC₂H₅ |
| 1-1926 | H | Cl | H | CF₃ | H | C(C₂H₅)=NN(C₂H₅)₂ |
| 1-1927 | H | Cl | H | CF₃ | H | C(CH₃)(OCH₃)₂ |
| 1-1928 | H | Cl | H | CF₃ | H | C(CH₃)(OC₂H₅)₂ |
| | | | | | | |
| 1-1929 | H | Cl | H | CF₃ | H | C(CH₃)(CⁱC₃H₇)₂ |
| 1-1930 | H | Cl | H | CF₃ | H | 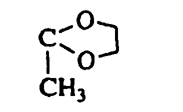 |
| 1-1931 | H | Cl | H | CF₃ | H | 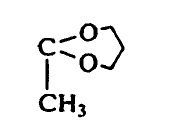 |
| 1-1932 | H | Cl | H | CF₃ | H | 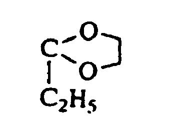 |
| 1-1933 | H | Cl | H | CF₃ | H | 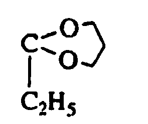 |
| 1-1934 | H | Cl | CH₃ | CF₃ | H | OCH₂C(CH₃)=CH₂ |
| 1-1935 | H | Cl | CH₃ | CF₃ | H | OCH₂CO₂C₇15 |
| 1-1936 | H | Cl | CH₃ | CF₃ | H | OCH₂CO₂C₈H₁₇ |
| 1-1937 | H | Cl | CH₃ | CF₃ | H | COOCH₂C₆H₅ |
| 1-1938 | H | Cl | CH₃ | CF₃ | H | C(CH₃)=NOH |
| 1-1939 | H | Cl | CH₃ | CF₃ | H | C(CH₃)=NOCH₃ |
| 1-1940 | H | Cl | CH₃ | CF₃ | H | C(CH₃)=NOC₂H₅ |
| | | | | | | |
| 1-1941 | H | Cl | CH₃ | CF₃ | H | C(CH₃) =NOⁱC₃H₇ |
| 1-1942 | H | Cl | CH₃ | CF₃ | H | C(C₂H₅)=NOH |
| 1-1943 | H | Cl | CH₃ | CF₃ | H | C(C₂H₅)=NOCH₃ |
| 1-1944 | H | Cl | CH₃ | CF₃ | H | C(C₂H₅)=NOC₂H₅ |
| | | | | | | |
| 1-1945 | H | Cl | CH₃ | CF₃ | H | C(C₂H₅)=NOⁱC₃H₇ |
| 1-1946 | H | Cl | CH₃ | CF₃ | H | C(CH₃)=NNH₂ |
| 1-1947 | H | Cl | CH₃ | CF₃ | H | C(CH₃)=NNHCH₃ |
| 1-1948 | H | Cl | CH₃ | CF₃ | H | C(CH₃)=NN(CH₃)₂ |
| 1-1949 | H | Cl | CH₃ | CF₃ | H | C(CH₃)=NNHC₂H₅ |
| 1-1950 | H | Cl | CH₃ | CF₃ | H | C(CH₃)=NN(C₂H₅)₂ |
| 1-1951 | H | Cl | CH₃ | CF₃ | H | C(C₂H₅)=NNH₂ |
| 1-1952 | H | Cl | CH₃ | CF₃ | H | C(C₂H₅)=NNHCH₃ |
| 1-1953 | H | Cl | CH₃ | CF₃ | H | C(C₂H₅)=NN(CH₃)₂ |
| 1-1954 | H | Cl | CH₃ | CF₃ | H | C(C₂H₅)=NNHC₂H₅ |
| 1-1955 | H | Cl | CH₃ | CF₃ | H | C(C₂H₅)=NN(C₂H₅)₂ |
| 1-1956 | H | Cl | CH₃ | CF₃ | H | C(CH₃) (OCH₃)₂ |
| 1-1957 | H | Cl | CH₃ | CF₃ | H | C(CH₃) (OC₂H₅)₂ |
| | | | | | | |
| 1-1958 | H | Cl | CH₃ | CF₃ | H | C(CH₃)(CⁱC₃H₇)₂ |
| 1-1959 | H | Cl | CH₃ | CF₃ | H | 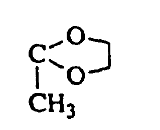 |
| 1-1960 | H | Cl | CH₃ | CF₃ | H | 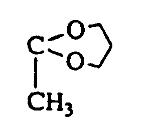 |
| 1-1961 | H | Cl | CH₃ | CF₃ | H | 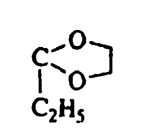 |
| 1-1962 | H | Cl | CH₃ | CF₃ | H | 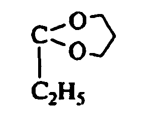 |
| 1-1963 | Cl | Cl | H | CF₃ | CH₃ | OCH₂C(CH₃)=CH₂ |
| 1-1964 | Cl | Cl | H | CF₃ | CH₃ | OCH₂CO₂C₇H₁₅ |
| 1-1965 | Cl | Cl | H | CF₃ | CH₃ | OCH₂CO₂C₈H₁₇ |
| 1-1966 | Cl | Cl | H | CF₃ | CH₃ | COOCH₂C₆H₅ |
| 1-1967 | Cl | Cl | H | CF₃ | CH₃ | C(CH₃)=NOH |
| 1-1968 | Cl | Cl | H | CF₃ | CH₃ | C(CH₃)=NOCH₃ |
| 1-1969 | Cl | Cl | H | CF₃ | CH₃ | C(CH₃)=NOC₂H₅ |
| | | | | | | |
| 1-1970 | Cl | Cl | H | CF₃ | CH₃ | C(CH₃)=NOⁱC₃H₇ |
| 1-1971 | Cl | Cl | H | CF₃ | CH₃ | C(C₂H₅)=NOH |
| 1-1972 | Cl | Cl | H | CF₃ | CH₃ | C(C₂H₅)=NOCH₃ |
| 1-1973 | Cl | Cl | H | CF₃ | CH₃ | C (C₂H₅) =NOC₂H₅ |
| | | | | | | |
| 1-1974 | Cl | Cl | H | CF₃ | CH₃ | C(C₂H₅)=NOⁱC₃H₇ |
| 1-1975 | Cl | Cl | H | CF₃ | CH₃ | C(CH₃)=NNH₂ |
| 1-1976 | Cl | Cl | H | CF₃ | CH₃ | C(CH₃)=NNHCH₃ |
| 1-1977 | Cl | Cl | H | CF₃ | CH₃ | C(CH₃)=NN(CH₃)₂ |
| 1-1978 | Cl | Cl | H | CF₃ | CH₃ | C(CH₃)=NNHC₂H₅ |
| 1-1979 | Cl | Cl | H | CF₃ | CH₃ | C(CH₃)=NN(C₂H₅)₂ |
| 1-1980 | Cl | Cl | H | CF₃ | CH₃ | C(C₂H₅)=NNH₂ |
| 1-1981 | Cl | Cl | H | CF₃ | CH₃ | C(C₂H₅)=NNHCH₃ |
| 1-1982 | Cl | Cl | H | CF₃ | CH₃ | C(C₂H₅)=NN(CH₃)₂ |
| 1-1983 | Cl | Cl | H | CF₃ | CH₃ | C(C₂H₅)=NNHC₂H₅ |
| 1-1984 | Cl | Cl | H | CF₃ | CH₃ | C(C₂H₅)=NN(C₂H₅)₂ |
| 1-1985 | Cl | Cl | H | CF₃ | CH₃ | C(CH₃)(OCH₃)₂ |
| 1-1986 | Cl | Cl | H | CF₃ | CH₃ | C(CH₃)(OC₂H₅)₂ |
| | | | | | | |
| 1-1987 | Cl | Cl | H | CF₃ | CH₃ | C(CH₃)(CⁱC₃H₇)₂ |
| 1-1988 | Cl | Cl | H | CF₃ | CH₃ | 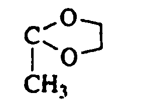 |
| 1-1989 | Cl | Cl | H | CF₃ | CH₃ | 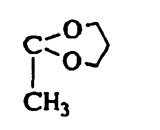 |
| 1-1990 | Cl | Cl | H | CF₃ | CH₃ | 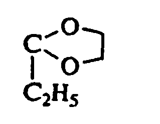 |
| 1-1991 | Cl | Cl | H | CF₃ | CH₃ | 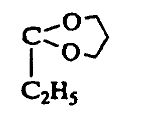 |
| 1-1992 | Cl | Cl | H | CF₃ | H | OCH₂C(CH₃)=CH₂ |
| 1-1993 | Cl | Cl | H | CF₃ | H | OCH₂CO₂C₇H₁₅ |
| 1-1994 | Cl | Cl | H | CF₃ | H | OCH₂CO₂C₈H₁₇ |
| 1-1995 | Cl | Cl | H | CF₃ | H | COOCH₂C₆H₅ |
| 1-1996 | Cl | Cl | H | CF₃ | H | C(CH₃)=NOH |
| 1-1997 | Cl | Cl | H | CF₃ | H | C(CH₃)=NOCH₃ |
| 1-1998 | Cl | Cl | H | CF₃ | H | C(CH₃)=NOC₂H₅ |
| | | | | | | |
| 1-1999 | Cl | Cl | H | CF₃ | H | C(CH₃)=NOⁱC₃H₇ |
| 1-2000 | Cl | Cl | H | CF₃ | H | C(C₂H₅)=NOH |
| 1-2001 | Cl | Cl | H | CF₃ | H | C(C₂H₅)=NOCH₃ |
| 1-2002 | Cl | Cl | H | CF₃ | H | C(C₂H₅)=NOC₂H₅ |
| | | | | | | |
| 1-2003 | Cl | Cl | H | CF₃ | H | C(C₂H₅)=NOⁱC₃H₇ |
| 1-2004 | Cl | Cl | H | CF₃ | H | C(CH₃)=NNH₂ |
| 1-2005 | Cl | Cl | H | CF₃ | H | C(CH₃)=NNHCH₃ |
| 1-2006 | Cl | Cl | H | CF₃ | H | C(CH₃)=NN(CH₃)₂ |
| 1-2007 | Cl | Cl | H | CF₃ | H | C(CH₃)=NNHC₂H₅ |
| 1-2008 | Cl | Cl | H | CF₃ | H | C(CH₃)=NN(C₂H₅)₂ |
| 1-2009 | Cl | Cl | H | CF₃ | H | C(C₂H₅)=NNH₂ |
| 1-2010 | Cl | Cl | H | CF₃ | H | C(C₂H₅)=NNHCH₃ |
| 1-2011 | Cl | Cl | H | CF₃ | H | C(C₂H₅)=NN(CH₃)₂ |
| 1-2012 | Cl | Cl | H | CF₃ | H | C(C₂H₅)=NNHC₂H₅ |
| 1-2013 | Cl | Cl | H | CF₃ | H | C(C₂H₅)-NN(C₂H₅)₂ |
| 1-2014 | Cl | | H | CF₃ | H | C(CH₃)(OCH₃)₂ |
| 1-2015 | Cl | Cl | H | CF₃ | H | C(CH₃)(OC₂H₅)2 |
| 1-2016 | Cl | Cl | H | CF₃ | H | C(CH₃)(CⁱC₃H₇)₂ |
| 1-2017 | Cl | Cl | H | CF₃ | H | 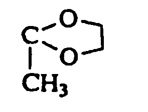 |
| 1-2018 | Cl | Cl | H | CF₃ | H | 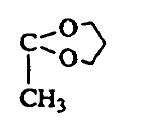 |
| 1-2019 | Cl | Cl | H | CF₃ | H | 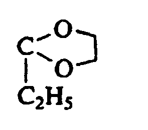 |
| 1-2020 | Cl | Cl | H | CF₃ | H | 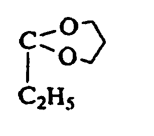 |
| 1-2021 | Cl | Cl | CH₃ | CF₃ | H | OCH₂C(CH₃)=CH₂ |
| 1-2022 | Cl | Cl | CH₃ | CF₃ | H | OCH₂CO₂C₇H₁₅ |
| 1-2023 | Cl | Cl | CH₃ | CF₃ | H | OCH₂CO₂C₈H₁₇ |
| 1-2024 | Cl | Cl | CH₃ | CF₃ | H | COOCH₂C₆H₅ |
| 1-2025 | Cl | Cl | CH₃ | CF₃ | H | C(CH₃)=NOH |
| 1-2026 | Cl | Cl | CH₃ | CF₃ | H | C(CH₃)=NOCH₃ |
| 1-2027 | Cl | Cl | CH₃ | CF₃ | H | C(CH₃)=NOC₂H₅ |
| | | | | | | |
| 1-2028 | Cl | Cl | CH₃ | CF₃ | H | C(CH₃)=NOⁱC₃H₇ |
| 1-2029 | Cl | Cl | CH₃ | CF₃ | H | C(C₂H₅)=NOH |
| 1-2030 | Cl | Cl | CH₃ | CF₃ | H | C(C₂H₅)=NOCH₃ |
| 1-2031 | Cl | Cl | CH₃ | CF₃ | H | C(C₂H₅)=NOC₂H₅ |
| | | | | | | |
| 1-2032 | Cl | Cl | CH₃ | CF₃ | H | C(C₂H₅)=NOⁱC₃H₇ |
| 1-2033 | Cl | Cl | CH₃ | CF₃ | H | C(CH₃)=NNH₂ |
| 1-2034 | Cl | Cl | CH₃ | CF₃ | H | C(CH₃)=NNHCH₃ |
| 1-2035 | Cl | Cl | CH₃ | CF₃ | H | C(CH₃)=NN(CH₃)₂ |
| 1-2036 | Cl | Cl | CH₃ | CF₃ | H | C(CH₃)=NNHC₂H₅ |
| 1-2037 | Cl | Cl | CH₃ | CF₃ | H | C(CH₃)=NN(C₂H₅)₂ |
| 1-2038 | Cl | Cl | CH₃ | CF₃ | H | C(C₂H₅)=NNH₂ |
| 1-2039 | Cl | Cl | CH₃ | CF₃ | H | C(C₂H₅)=NNHCH₃ |
| 1-2040 | Cl | Cl | CH₃ | CF₃ | H | C(C₂H₅)=NN(CH₃)₂ |
| 1-2041 | Cl | Cl | CH₃ | CF₃ | H | C(C₂H₅)=NNHC₂H₅ |
| 1-2042 | Cl | Cl | CH₃ | CF₃ | H | C(C₂H₅)=NN(C₂H₅)₂ |
| 1-2043 | Cl | Cl | CH₃ | CF₃ | H | C(CH₃)(OCH₃)₂ |
| 1-2044 | Cl | Cl | CH₃ | CF₃ | H | C(CH₃) (OC₂H₅)₂ |
| | | | | | | |
| 1-2045 | Cl | Cl | CH₃ | CF₃ | H | C(CH₃) (CⁱC₃H₇)₂ |
| 1-2046 | Cl | Cl | CH₃ | CF₃ | H | 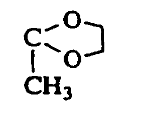 |
| 1-2047 | Cl | Cl | CH₃ | CF₃ | H | 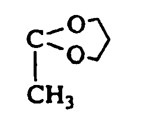 |
| 1-2048 | Cl | Cl | CH₃ | CF₃ | H | 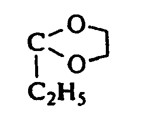 |
| 1-2049 | Cl | Cl | CH₃ | CF₃ | H | 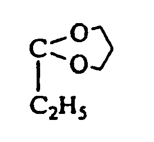 |
| 1-2050 | F | Cl | H | CF₃ | CH₃ | OCH₂COOCH₂COOCH₃ |
| 1-2051 | F | Cl | H | CF₃ | CH₃ | OCH₂COOCH₂COOC₂H₅ |
| | | | | | | |
| 1-2052 | F | Cl | H | CF₃ | CH₃ | OCH₂COOCH_{Z}COOⁱC₃H₇ |
| 1-2053 | F | Cl | H | CF₃ | CH₃ | OCH₂COOCH(CH₃)COOCH₃ |
| 1-2054 | F | Cl | H | CF₃ | CH₃ | OCH₂COOCH(CH₃)COOC₂H₅ |
| | | | | | | |
| 1-2055 | F | Cl | H | CF₃ | CH₃ | OCH₂COOCH(CH₃)COOⁱC₃H₇ |
| 1-2056 | F | Cl | H | CF₃ | CH₃ | OCH(CH₃)COOCH₂COOCH₃ |
| 1-2057 | F | Cl | H | CF₃ | CH₃ | OCH(CH₃)COOCH₂COOC₂H₅ |
| | | | | | | |
| 1-2058 | F | Cl | H | CF₃ | CH₃ | OCH(CH₃)COOCH₂COOⁱC₃H₇ |
| 1-2059 | F | Cl | H | CF₃ | CH₃ | OCH(CH₃)COOCH(CH₃)COOCH₃ |
| 1-2060 | F | Cl | H | CF₃ | CH₃ | OCH(CH₃)COOCH(CH₃)COOC₂H₅ |
| | | | | | | |
| 1-2061 | F | Cl | H | CF₃ | CH₃ | OCH(CH₃)COOCH(CH₃)COOⁱC₃H₇ |
| 1-2062 | F | Cl | H | CF₃ | H | OCH₂COOCH₂COOCH₃ |
| 1-2063 | F | Cl | H | CF₃ | H | OCH₂COOCH₂COOC₂H₅ |
| 1-2064 | F | Cl | H | CF₃ | H | OCH₂COOCH₂COOⁱC₃H₇ |
| 1-2065 | F | Cl | H | CF₃ | H | OCH₂COOCH(CH₃)COOCH₃ |
| 1-2066 | F | Cl | H | CF₃ | H | OCH₂COOCH(CH₃)COOC₂H₅ |
| | | | | | | |
| 1-2067 | F | Cl | H | CF₃ | H | OCH₂COOCH(CH₃)COOⁱC₃H₇ |
| 1-2068 | F | Cl | H | CF₃ | H | OCH(CH₃)COOCH₂COOCH₃ |
| 1-2069 | F | Cl | H | CF₃ | H | OCH(CH₃)COOCH₂COOC₂H₅ |
| 1-2070 | F | Cl | H | CF₃ | H | OCH(CH₃)COOCH₂COOⁱC₃H₇ |
| 1-2071 | F | Cl | H | CF₃ | H | OCH(CH₃)COOCH(CH₃)COOCH₃ |
| 1-2072 | F | Cl | H | CF₃ | H | OCH(CH₃)COOCH(CH₃)COOC₂H₅ |
| | | | | | | |
| 1-2073 | F | Cl | H | CF₃ | H | OCH(CH₃)COOCH(CH₃)COOⁱC₃H₇ |
| 1-2074 | F | Cl | CH₃ | CF₃ | H | OCH₂COOCH₂COOCH₃ |
| 1-2075 | F | Cl | CH₃ | CF₃ | H | OCH₂COOCH₂COOC₂H₅ |
| | | | | | | |
| 1-2076 | F | Cl | CH₃ | CF₃ | H | OCH₂COOCH₂COOⁱC₃H₇ |
| 1-2077 | F | Cl | CH₃ | CF₃ | H | OCH₂COOCH(CH₃)COOCH₃ |
| 1-2078 | F | Cl | CH₃ | CF₃ | H | OCH₂COOCH(CH₃)COOC₂H₅ |
| | | | | | | |
| 1-2079 | F | Cl | CH₃ | CF₃ | H | OCH₂COOCH(CH₃)COOⁱC₃H₇ |
| 1-2080 | F | Cl | CH₃ | CF₃ | H | OCH(CH₃)COOCH₂COOCH₃ |
| 1-2081 | F | Cl | CH₃ | CF₃ | H | OCH(CH₃)COOCH₂COOC₂H₅ |
| | | | | | | |
| 1-2082 | F | Cl | CH₃ | CF₃ | H | OCH(CH₃)COOCH₂COOⁱC₃H₇ |
| 1-2083 | F | Cl | CH₃ | CF₃ | H | OCH(CH₃)COOCH(CH₃)COOCH₃ |
| 1-2084 | F | Cl | CH₃ | CF₃ | H | OCH(CH₃)COOCH(CH₃)COOC₂H₅ |
| | | | | | | |
| 1-2085 | F | Cl | CH₃ | CF₃ | H | OCH(CH₃)COOCH(CH₃)COOⁱC₃H₇ |
| 1-2086 | H | Cl | H | CF₃ | CH₃ | OCH₂COOCH₂COOCH₃ |
| 1-2087 | H | Cl | H | CF₃ | CH₃ | OCH₂COOCH₂COOC₂H₅ |
| | | | | | | |
| 1-2088 | H | Cl | H | CF₃ | CH₃ | OCH₂COOCH₂COOⁱC₃H₇ |
| 1-2089 | H | Cl | H | CF₃ | CH₃ | OCH₂COOCH(CH₃)COOCH₃ |
| 1-2090 | H | Cl | H | CF₃ | CH₃ | OCH₂COOCH(CH₃)COOC₂H₅ |
| | | | | | | |
| 1-2091 | H | Cl | H | CF₃ | CH₃ | OCH₂COOCH(CH₃)COOⁱC₃H₇ |
| 1-2092 | H | Cl | H | CF₃ | CH₃ | OCH(CH₃)COOCH₂COOCH₃ |
| 1-2093 | H | Cl | H | CF₃ | CH₃ | OCH(CH₃)COOCH₂COOC₂H₅ |
| | | | | | | |
| 1-2094 | H | Cl | H | CF₃ | CH₃ | OCH(CH₃)COOCH₂COOⁱC₃H₇ |
| 1-2095 | H | Cl | H | CF₃ | CH₃ | OCH(CH₃)COOCH(CH₃)COOCH₃ |
| 1-2096 | H | Cl | H | CF₃ | CH₃ | OCH(CH₃)COOCH(CH₃)COOC₂H₅ |
| | | | | | | |
| 1-2097 | H | Cl | H | CF₃ | CH₃ | OCH(CH₃)COOCH(CH₃)COOⁱC₃H₇ |
| 1-2098 | H | Cl | H | CF₃ | H | OCH₂COOCH₂COOCH₃ |
| 1-2099 | H | Cl | H | CF₃ | H | OCH₂COOCH₂COOC₂H₅ |
| | | | | | | |
| 1-2100 | H | Cl | H | CF₃ | H | OCH₂COOCH₂COOⁱC₃H₇ |
| 1-2101 | H | Cl | H | CF₃ | H | OCH₂COOCH(CH₃)COOCH₃ |
| 1-2102 | H | Cl | H | CF₃ | H | OCH₂COOCH(CH₃)COOC₂H₅ |
| | | | | | | |
| 1-2103 | H | Cl | H | CF₃ | H | OCH₂COOCH(CH₃)COOⁱC₃H₇ |
| 1-2104 | H | Cl | H | CF₃ | H | OCH(CH₃)COOCH₂COOCH₃ |
| 1-2105 | H | Cl | H | CF₃ | H | OCH(CH₃)COOCH₂COOC₂H₅ |
| | | | | | | |
| 1-2106 | H | Cl | H | CF₃ | H | OCH(CH₃)COOCH₂COOⁱC₃H₇ |
| 1-2107 | H | Cl | H | CF₃ | H | OCH(CH₃)COOCH(CH₃)COOCH₃ |
| 1-2108 | H | Cl | H | CF₃ | H | OCH(CH₃)COOCH(CH₃)COOC₂H₅ |
| | | | | | | |
| 1-2109 | H | Cl | H | CF₃ | H | OCH(CH₃)COOCH(CH₃)COOⁱC₃H₇ |
| 1-2110 | H | Cl | CH₃ | CF₃ | H | OCH₂COOCH₂COOCH₃ |
| 1-2111 | H | Cl | CH₃ | CF₃ | H | OCH₂COOCH₂COOC₂H₅ |
| | | | | | | |
| 1-2112 | H | Cl | CH₃ | CF₃ | H | OCH₂COOCH₂COOⁱC₃H₇ |
| 1-2113 | H | Cl | CH₃ | CF₃ | H | OCH₂COOCH(CH₃)COOCH₃ |
| 1-2114 | H | Cl | CH₃ | CF₃ | H | OCH₂COOCH(CH₃)COOC₂H₅ |
| | | | | | | |
| 1-2115 | H | Cl | CH₃ | CF₃ | H | OCH₂COOCH(CH₃)COOⁱC₃H₇ |
| 1-2116 | H | Cl | CH₃ | CF₃ | H | OCH(CH₃)COOCH₂COOCH₃ |
| 1-2117 | H | Cl | CH₃ | CF₃ | H | OCH(CH₃)COOCH₂COOC₂H₅ |
| | | | | | | |
| 1-2118 | H | Cl | CH₃ | CF₃ | H | OCH(CH₃)COOCH₂COOⁱC₃H₇ |
| 1-2119 | H | Cl | CH₃ | CF₃ | H | OCH(CH₃)COOCH(CH₃)COOCH₃ |
| 1-2120 | H | Cl | CH₃ | CF₃ | H | OCH(CH₃)COOCH(CH₃)COOC₂H₅ |
| | | | | | | |
| 1-2121 | H | Cl | CH₃ | CF₃ | H | OCH(CH₃)COOCH(CH)COOⁱC₃H₇ |
| 1-2122 | Cl | Cl | H | CF₃ | CH₃ | OCH₂COOCH₂COOCH₃ |
| 1-2123 | Cl | Cl | H | CF₃ | CH₃ | OCH₂COOCH₂COOC₂H₅ |
| | | | | | | |
| 1-2124 | Cl | Cl | H | CF₃ | CH₃ | OCH₂COOCH₂COOⁱC₃H₇ |
| 1-2125 | Cl | Cl | H | CF₃ | CH₃ | OCH₂COOCH(CH₃)COOCH₃ |
| 1-2126 | Cl | Cl | H | CF₃ | CH₃ | OCH₂COOCH(CH₃)COOC₂H₅ |
| | | | | | | |
| 1-2127 | Cl | Cl | H | CF₃ | CH₃ | OCH₂COOCH(CH₃)COOⁱC₃H₇ |
| 1-2128 | Cl | Cl | H | CF₃ | CH₃ | OCH(CH₃)COOCH₂COOCH₃ |
| 1-2129 | Cl | Cl | H | CF₃ | CH₃ | OCH(CH₃)COOCH₂COOC₂H₅ |
| | | | | | | |
| 1-2130 | Cl | Cl | H | CF₃ | CH₃ | OCH(CH₃)COOCH₂COOⁱC₃H₇ |
| 1-2131 | Cl | Cl | H | CF₃ | CH₃ | OCH(CH₃)COOCH(CH₃)COOCH₃ |
| | | | | | | |
| 1-2132 | Cl | Cl | H | CF₃ | CH₃ | OCH(CH₃)COOCH(CH₃)COOC₂H₅ |
| | | | | | | |
| 1-2133 | Cl | Cl | H | CF₃ | CH₃ | OCH(CH₃)COOCH(CH₃)COOⁱC₃H₇ |
| 1-2134 | Cl | Cl | H | CF₃ | H | OCH₂COOCH₂COOCH₃ |
| 1-2135 | Cl | Cl | H | CF₃ | H | OCH₂COOCH₂COOC₂H₅ |
| 1-2136 | Cl | Cl | H | CF₃ | H | OCH₂COOCH₂COO¹C₃H₇ |
| 1-2137 | Cl | Cl | H | CF₃ | H | OCH₂COOCH(CH₃)COOCH₃ |
| 1-2138 | Cl | Cl | H | CF₃ | H | OCH₂COOCH(CH₃)COOC₂H₅ |
| | | | | | | |
| 1-2139 | Cl | Cl | H | CF₃ | H | OCH₂COOCH(CH₃)COOⁱC₃H₇ |
| 1-2140 | Cl | Cl | H | CF₃ | H | OCH(CH₃)COOCH₂COOCH₃ |
| 1-2141 | Cl | Cl | H | CF₃ | H | OCH(CH₃)COOCH₂COOC₂H₅ |
| | | | | | | |
| 1-2142 | Cl | Cl | H | CF₃ | H | OCH(CH₃)COOCH₂COOⁱC₃H₇ |
| 1-2143 | Cl | Cl | H | CF₃ | H | OCH(CH₃)COOCH(CH₃)COOCH₃ |
| 1-2144 | Cl | Cl | H | CF₃ | H | OCH(CH₃)COOCH(CH₃)COOC₂H₅ |
| | | | | | | |
| 1-2145 | Cl | Cl | H | CF₃ | H | OCH(CH₃)COOCH(CH₃)COOⁱC₃H₇ |
| 1-2146 | Cl | Cl | CH₃ | CF₃ | H | OCH₂COOCH₂COOCH₃ |
| 1-2147 | Cl | Cl | CH₃ | CF₃ | H | OCH₂COOCH₂COOC₂H₅ |
| | | | | | | |
| 1-2148 | Cl | Cl | CH₃ | CF₃ | H | OCH₂COOCH₂COOⁱC₃H₇ |
| 1-2149 | Cl | Cl | CH₃ | CF₃ | H | OCH₂COOCH(CH₃)COOCH₃ |
| 1-2150 | Cl | Cl | CH₃ | CF₃ | H | OCH₂COOCH(CH₃)COOC₂H₅ |
| | | | | | | |
| 1-2151 | Cl | Cl | CH₃ | CF₃ | H | OCH₂COOCH(CH₃)COOⁱC₃H₇ |
| 1-2152 | Cl | Cl | CH₃ | CF₃ | H | OCH(CH₃)COOCH₂COOCH₃ |
| 1-2153 | Cl | Cl | CH₃ | CF₃ | H | OCH(CH₃)COOCH₂COOC₂H₅ |
| | | | | | | |
| 1-2154 | Cl | Cl | CH₃ | CF₃ | H | OCH(CH₃)COOCH₂COOⁱC₃H₇ |
| 1-2155 | Cl | Cl | CH₃ | CF₃ | H | OCH(CH₃)COOCH(CH₃)COOCH₃ |
| 1-2156 | Cl | Cl | CH₃ | CF₃ | H | OCH(CH₃)COOCH(CH₃)COOC₂H₅ |
| | | | | | | |
| 1-2157 | Cl | Cl | CH₃ | CF₃ | H | OCH(CH₃)COOCH(CH₃)COOⁱC₃H₇ |

**TABLE 2**

| | | | | | | | | |
|---|---|---|---|---|---|---|---|---|
| Compounds of the formula: | | | | | | | | |
| 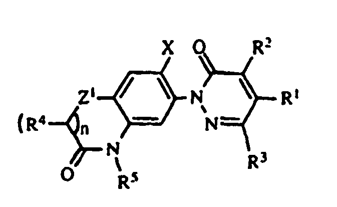 | | | | | | | | |

| Compound No. | X | Z¹ n R³ | | | R¹ | R² | R⁴ | R⁵ |
|---|---|---|---|---|---|---|---|---|
| 2-1 | H | O | 1 | H | CF₂ Cl | CH₃ | H | H |
| 2-2 | H | O | 1 | H | CF₂ Cl | CH₃ | H | CH₃ |
| 2-3 | H | O | 1 | H | CF₂ Cl | CH₃ | H | C₂ H₅ |
| 2-4 | H | O | 1 | H | CF₂ Cl | CH₃ | H | ⁿC₃ H ₇ |
| 2-5 | H | O | 1 | H | CF₂ Cl | CH₃ | H | ⁱC₃ H₇ |
| 2-6 | H | O | 1 | H | CF₂ Cl | CH₃ | H | ⁱC₄H₉ |
| 2-7 | H | O | 1 | H | CF₂ Cl | CH₃ | H | ⁿC₄H₉ |
| 2-8 | H | O | 1 | H | CF₂ Cl | CH₃ | H | CH₂ CH₂ Cl |
| 2-9 | H | O | 1 | H | CF₂ Cl | CH₃ | H | CH₂ CH₂ Br |
| 2-10 | H | O | 1 | H | CF₂ Cl | CH₃ | H | CH₂ CH=CH₂ |
| 2-11 | H | O | 1 | H | CF₂ Cl | CH₃ | H | CH(CH₃ )CH =CH₂ |
| 2-12 | H | O | 1 | H | CF₂ Cl | CH₃ | H | CH₂ CCl = CH₂ |
| 2-13 | H | O | 1 | H | CF₂ | CH₃ | H | CH₂ C ≡ CH |
| 2-14 | H | O | 1 | H | CF₂ Cl | CH₃ | H | CH(CH ₂) C≡CH |
| 2-15 | H | O | 1 | H | CF₂ Cl | CH₃ | H | CH₂ CN |
| 2-16 | H | O | 1 | H | CF₂ Cl | CH₃ | H | CH₂ OCH₃ |
| 2-17 | H | O | 1 | 1 H | CF₂ Cl | CH₃ | H | CH₂ OC₂ H ₅ |
| 2-18 | H | O | 1 | H | CF₂ Cl | CH₃ | H | CH₂ COOH |
| 2-19 | H | O | 1 | H | CF₂ Cl | CH₃ | H | CH₂ COOCH ₃ |
| 2-20 | H | O | 1 | H | CF₂ Cl | CH₃ | H | CH₂ COOC₂ H s |
| 2-21 | H | O | 1 | H | CF₂ Cl | CH₃ | H | CH₂ COO ⁿ C ₃ H ₇ |
| 2-22 | H | O | 1 | H | CF₂ Cl | CH₃ | H | CH₂ COO ⁿ C ₄ H ₉ |
| 2-23 | H | O | 1 | H | CF₂ Cl | CH₃ | H | CH₂ COO ⁿ C ₅ H ₁₁ |
| 2-24 | H | O | 1 | H | CF₂ Cl | CH₃ | H | CH₂ COO ⁱ C ₃ H ₇ |
| 2-25 | H | O | 1 | H | CF₂ Cl | CH₃ | H | CH₂ COO ^{c} C ₅ H ₉ |
| 2-26 | H | O | 1 | H | CF₂ Cl | CH₃ | H | CH₂ COO ^{c} C ₆ H ₁₁ |
| 2-27 | H | O | 1 | H | CF₂ | CH₃ | H | CH(CH ₃)COOH |
| 2-28 | H | O | 1 | H | CF₂ Cl | CH₃ | H | CH (CH ₃ )COOCH ₃ |
| 2-29 | H | O | 1 | H | CF₂ | CH₃ | H | CH(CH ₃)COOC ₂ H ₅ |
| 2-30 | H | O | 1 | H | CF₂ | CH₃ | H | CH(CH₃ ) COOⁿ C ₃ H ₇ |
| 2-31 | H | O | 1 | H | CF₂ Cl | CH₃ | H | CH(CH₃ ) COOⁿ C ₄ H ₉ |
| 2-32 | H | O | 1 | H | CF₂ Cl | CH₃ | H | CH(CH₃ ) COOⁿ C ₅ H ₁₁ |
| 2-33 | H | O | 1 | H | CF₂ Cl | CH₃ | H | CH(CH₃ )COOⁱ C ₃ H ₇ |
| 2-34 | H | O | 1 | H | CF₂ | CH₃ | H | CH (CH₃)COO^{c}C₅H₉ |
| 2-35 | H | O | 1 | H | CF₂ Cl | CH₃ | H | CH(CH₃ )COO^{c} C ₆H₁₁ |
| 2-36 | H | O | 1 | H | CF₂ Cl | CH₃ | CH₃ | H |
| 2-37 | H | O | 1 | H | CF₂ Cl | CH₃ | CH₃ | CH₃ |
| 2-38 | H | O | 1 | H | CF₂ Cl | CH₃ | CH₃ | C₂ H ₅ |
| 2-39 | H | O | 1 | H | CF₂ Cl | CH₃ | CH₃ | ⁿC ₃ H ₇ |
| 2-40 | H | O | 1 | H | CF₂ Cl | CH₃ | CH₃ | ⁱC ₃ H ₇ |
| 2-41 | H | O | 1 | H | CF₂ Cl | CH₃ | CH₃ | ⁱC ₄ H ₉ |
| 2-42 | H | O | 1 | H | CF₂ Cl | CH₃ | CH₃ | ⁿC H ₉ |
| 2-43 | H | O | 1 | H | CF₂ Cl | CH₂ | CH₃ | CH₂ CH=CH₂ |
| 2-44 | H | O | 1 | H | CF₂ Cl | CH₃ | CH₃ | CH(CH ₃)CH =CH₂ |
| 2-45 | H | O | 1 | H | CF₂ Cl | CH₃ | CH₃ | CH₂ C ≡ CH |
| 2-46 | H | O | 1 | H | CF₂ Cl | CH₃ | CH₃ | CH(CH ₃ )C≡CH |
| 2-47 | H | O | 1 | H | CF₂ Cl | CH₃ | CH₃ | CH₂ OCH ₃ |
| 2-48 | H | O | 1 | H | CF₂ Cl | CH₃ | CH₃ | CH₂ OC₂ H ₅ |
| 2-49 | F | O | 1 | H | CF₂ Cl | CH₃ | H | H |
| 2-50 | F | O | 1 | H | CF₂ Cl | CH₃ | H | CH₃ |
| 2-51 | F | O | 1 | H | CF₂ Cl | CH₃ | H | C₂ H s |
| 2-52 | F | O | 1 | H | CF₂ | CH₃ | H | ⁿC ₃ H ₇ |
| 2-53 | F | O | 1 | H | CF₂ Cl | CH₃ | H | ⁱC ₃ H ₇ |
| 2-54 | F | O | 1 | H | CF₂ Cl | CH₃ | H | ⁱC ₄ H ₉ |
| 2-55 | F | O | 1 | H | CF₂ Cl | CH₃ | H | ⁿC ₄ H ₉ |
| 2-56 | F | O | 1 | H | CF₂ Cl | CH₃ | H | CH₂ CH₂ Cl |
| 2-57 | F | O | 1 | H | CF₂ Cl | CH₃ | H | CH₂ CH₂ Br |
| 2-58 | F | O | 1 | H | CF₂ Cl | CH₃ | H | CH₂ CH=CH₂ |
| 2-59 | F | O | 1 | H | CF₂ Cl | CH₃ | H | CH(CH ₃)CH =CH₂ |
| 2-60 | F | O | 1 | H | CF₂ Cl | CH₃ | H | CH₂ CCl = CH₂ |
| 2-61 | F | O | 1 | H | CF₂ Cl | CH₃ | H | CH₂ C ≡CH |
| 2-62 | F | O | 1 | H | CF₂ Cl | -CH₃ | H | CH (CH ₃ )C ≡CH |
| 2-63 | F | O | 1 | H | CF₂ Cl | CH₃ | H | CH₂ CN |
| 2-64 | F | O | 1 | H | CF₂ Cl | CH₃ | H | CH₂ OCH ₃ |
| 2-65 | F | O | 1 | H | CF₂ Cl | CH₃ | H | CH₂ OC₂ H ₅ |
| 2-66 | F | O | 1 | H | CF₂ Cl | CH₃ | H | CH₂ COOH |
| 2-67 | F | O | 1 | H | CF₂ Cl | CH₃ | H | CH₂ COOCH ₃ |
| 2-68 | F | O | 1 | H | CF₂ Cl | CH₃ | H | CH₂ COOC₂ H ₅ |
| 2-69 | F | O | 1 | H | CF₂ Cl | CH₃ | H | CH₂ COO ⁿ C ₃ H ₇ |
| 2-70 | F | O | 1 | H | CF₂ Cl | CH₃ | H | CH₂ COO ⁿ C ₄ H ₉ |
| 2-71 | F | O | 1 | H | CF₂ Cl | CH₃ | H | CH₂ COO ⁿ C ₅ H₁₁ |
| 2-72 | F | O | 1 | H | CF₂ Cl | CH₃ | H | CH₂ COO C ₃ H ₇ |
| 2-73 | F | O | 1 | H | CF₂ Cl | CH₃ | H | CH₂ COO C ₅ H ₉ |
| 2-74 | F | O | 1 | H | CF₂ Cl | CH₃ | H | CH₂ COO ^{c} C ₆ H ₁₁ |
| 2-75 | F | O | 1 | H | CF₂ Cl | CH₃ | H | CH(CH ₃ )COOH |
| 2-76 | F | O | 1 | H | CF₂ Cl | CH₃ | H | CH(CH ₃)COOCH₃ |
| 2-77 | F | O | 1 | H | CF₂ Cl | CH₃ | H | CH(CH₃ )COOC ₂ H ₅ |
| 2-78 | F | O | 1 | H | CF₂ Cl | CH₃ | H | CH(CH₃ )COOⁿ C ₃ H ₇ |
| 2-79 | F | O | 1 | H | CF₂ Cl | CH₃ | H | CH(CH₃ )COOⁿ C ₄H₉ |
| 2-80 | F | O | 1 | H | CF₂ Cl | CH₃ | H | CH(CH₃ )COOⁿ C ₃H₁₁ |
| 2-81 | F | O | 1 | H | CF₂ Cl | CH₃ | H | CH(CH₃ )COOⁱ C₃ H₇ |
| 2-82 | F | O | 1 | H | CF₂ Cl | CH₃ | H | CH(CH₃ )COO^{c} C ₅ H₉ |
| 2-83 | F | O | 1 | H | CF₂ Cl | CH₃ | H | CH(CH₃ )COO^{c} C ₆H₁₁ |
| 2-84 | F | O | 1 | H | CF₂ Cl | CH₃ | CH₃ | H |
| 2-85 | F | O | 1 | H | CF₂ Cl | CH₃ | CH₃ | CH₃ |
| 2-86 | F | O | 1 | H | CF₂ Cl | CH₃ | CH₃ | C₂ H ₅ |
| 2-87 | F | O | 1 | H | CF₂ Cl | CH₃ | CH₃ | ⁿC ₃ H ₇ |
| 2-88 | F | O | 1 | H | CF₂ Cl | CH₃ | CH₃ | ⁱC ₃ H ₇ |
| 2-89 | F | O | 1 | H | CF₂ Cl | CH₃ | CH₃ | ⁱC ₄ H ₉ |
| 2-90 | F | O | 1 | H | CF₂ Cl | CH₃ | CH₃ | ⁿ C ₄ H ₉ |
| 2-91 | F | O | 1 | H | CF₂ Cl | CH₃ | CH₃ | CH₂ CH=CH₂ |
| 2-92 | F | O | 1 | H | CF₂ Cl | CH₃ | CH₃ | CH(CH ₃ )CH =CH₂ |
| 2-93 | F | O | 1 | H | CF₂ Cl | CH₃ | CH₃ | CH₂ C ≡CH |
| 2-94 | F | O | 1 | H | CF₂ Cl | CH₃ | CH₃ | CH(CH ₃ )C ≡ CH |
| 2-95 | F | O | 1 | H | CF₂ Cl | CH₃ | CH₃ | CH₂ OCH ₃ |
| 2-96 | F | O | 1 | H | CF₂ Cl | CH₃ | - | CH₂ OC₂ H ₅ |
| 2-97 | H | S | 0 | H | CF₂ Cl | CH₃ | - | H |
| 2-98 | H | S | 0 | H | CF₂ Cl | CH₃ | - | CH₃ |
| 2-99 | H | S | 0 | H | CF₂ Cl | CH₃ | - | C₂ H ₅ |
| 2-100 | H | S | 0 | H | CF₂ Cl | CH₃ | - | ⁿ C ₃ H ₇ |
| 2-101 | H | S | 0 | H | CF₂ Cl | CH₃ | - | ⁿC₄ H ₉ |
| 2-102 | H | S | 0 | H | CF₂ Cl | CH₃ | - | ⁱC ₃ H ₇ |
| 2-103 | H | S | 0 | H | CF₂ Cl | CH₃ | - | ⁱC ₄ H ₉ |
| 2-104 | H | S | 0 | H | CF₂ Cl | CH₃ | - | CH₂ CH₂ Cl |
| 2-105 | H | S | 0 | H | CF₂ Cl | CH₃ | - | CH₂ CH₂ Br |
| 2-106 | H | S | 0 | H | CF₂ Cl | CH₃ | - | CH₂ CH=CH₂ |
| 2-107 | H | S | 0 | H | CF₂ Cl | CH₃ | - | CH(CH₃) CH = CH₂ |
| 2-108 | H | S | 0 | H | CF₂ Cl | CH₃ | - | CH₂ CCl = CH₂ |
| 2-109 | H | S | 0 | H | CF₂ Cl | CH₃ | - | CH₂ C = CH |
| 2-110 | H | S | 0 | H | CF₂ Cl | CH₃ | - | CH(CH₃) C≡CH |
| 2-111 | H | S | 0 | H | CF₂ Cl | CH₃ | - | CH₂ CN |
| 2-112 | H | S | 0 | H | CF₂ Cl | CH₃ | - | CH₂ OCH₃ |
| 2-113 | H | S | 0 | H | CF₂ Cl | CH₃ | - | CH₂ OC₂ H ₅ |
| 2-114 | H | S | 0 | H | CF₂ Cl | CH₃ | - | CH₂ COOH |
| 2-115 | H | S | 0 | H | CF₂ Cl | CH₃ | - | CH₂ COOCH₃ |
| 2-116 | H | S | 0 | H | CF₂ Cl | CH₃ | - | CH₂ COOC₂ H ₅ |
| 2-117 | H | S | 0 | H | CF₂ Cl | CH₃ | - | CH₂ COO ⁿ C₃H₇ |
| 2-118 | H | S | 0 | H | CF₂ Cl | CH₃ | - | CH₂ COO ⁿ C₄H₉ |
| 2-119 | H | S | 0 | H | CF₂ Cl | CH₃ | - | CH₂ COO ⁿ C₅H₁₁ |
| 2-120 | H | S | 0 | H | CF₂ Cl | CH₃ | - | CH₂ COO ⁱ C₃H₇ |
| 2-121 | H | S | 0 | H | CF₂ Cl | CH₃ | - | CH₂ COO ^{c} C₅H₉ |
| 2-122 | H | S | 0 | H | CF₂ Cl | CH₃ | - | CH₂ COO ^{c} C₆H₁₁ |
| 2-123 | H | S | 0 | H | CF₂ Cl | CH₃ | - | CH(CH₃)COOH |
| 2-124 | H | S | 0 | H | CF₂ Cl | CH₃ | - | CH(CH)COOCH₃ |
| 2-125 | H | S | 0 | H | CF₂ Cl | CH₃ | - | CH(CH₃)COOC₂H₅ |
| 2-126 | H | S | 0 | H | CF₂ Cl | CH₃ | - | CH(CH₃ )COOⁿ C ₃ H ₇ |
| 2-127 | H | S | 0 | H | CF₂ Cl | CH₃ | - | CH(CH₃ )COOⁿ C ₄ H ₉ |
| 2-128 | H | S | 0 | H | CF₂ Cl | CH₃ | - | CH(CH₃ )COOⁿ C ₆ H ₁₁ |
| 2-129 | H | S | 0 | H | CF₂ Cl | CH₃ | - | CH(CH₃)COOⁱ C ₃ H ₇ |
| 2-130 | H | S | 0 | H | CF₂ Cl | CH₃ | - | CH(CH₃ )COO^{c} C ₅ H₉ |
| 2-131 | H | S | 0 | H | CF₂ Cl | CH₃ | - | CH(CH₃ )COO^{c} C ₆H₁₁ |
| 2-132 | F | S | 0 | H | CF₂ Cl | CH₃ | - | H |
| 2-133 | F | S | 0 | H | CF₂ Cl | CH₃ | - | CH₃ |
| 2-134 | F | S | 0 | H | CF₂ Cl | CH₃ | - | C₂ H ₅ |
| 2-135 | F | S | 0 | H | CF₂ Cl | CH₃ | - | ⁿ C₃ H ₇ |
| 2-136 | F | S | 0 | H | CF₂ Cl | CH₃ | - | ⁿ C ₄ H₉ |
| 2-137 | F | S | 0 | H | CF₂ Cl | CH₃ | - | ⁱC ₃ H ₇ |
| 2-138 | F | S | 0 | H | CF₂ Cl | CH₃ | - | ⁱC ₄ H ₉ |
| 2-139 | F | S | 0 | H | CF₂ Cl | CH₃ | - | CH₂ CH₂ Cl |
| 2-140 | F | S | 0 | H | CF₂ Cl | CH₃ | - | CH₂ CH₂ Br |
| 2-141 | F | S | 0 | H | CF₂ Cl | CH₃ | - | CH₂ CH=CH₂ |
| 2-142 | F | S | 0 | H | CF₂ Cl | CH₃ | - | CH(CH ₃ )CH =CH₂ |
| 2-143 | F | S | 0 | H | CF₂ Cl | CH₃ | - | CH₂ CCl =CH₂ |
| 2-144 | F | S | 0 | H | CF₂ Cl | CH₃ | - | CH₂ C ≡CH |
| 2-145 | F | S | 0 | H | CF₂ Cl | CH₃ | - | CH(CH ₃ )C≡CH |
| 2-146 | F | S | 0 | H | CF₂ Cl | CH₃ | - | CH₂ CN |
| 2-147 | F | S | 0 | H | CF₂ Cl | CH₃ | - | CH₂ OCH ₃ |
| 2-148 | F | S | 0 | H | CF₂ Cl | CH₃ | - | CH₂ OC₂ H₅ |
| 2-149 | F | S | 0 | H | CF₂ Cl | CH₃ | - | CH₂ COOH |
| 2-150 | F | S | 0 | H | CF₂ Cl | CH₃ | - | CH₂ COOCH ₃ |
| 2-151 | F | S | 0 | H | CF₂ Cl | CH₃ | - | CH₂ COOC₂ H ₅ |
| 2-152 | F | S | 0 | H | CF₂ Cl | CH₃ | - | CH₂ COO ⁿ C₃ H₇ |
| 2-153 | F | S | 0 | H | CF₂ Cl | CH₃ | - | CH₂ COO ⁿ C ₄ H ₉ |
| 2-154 | F | S | 0 | H | CF₂ Cl | CH₃ | - | CH₂ COO ⁿ C ₅ H₁₁ |
| 2-155 | F | S | 0 | H | CF₂ Cl | CH₃ | - | CH₂ COO C ₃ H ₇ |
| 2-156 | F | S | 0 | H | CF₂ Cl | CH₃ | - | CH₂ COO ^{c} C ₅ H₉ |
| 2-157 | F | S | 0 | H | CF₂ Cl | CH₃ | - | CH₂ COO ^{c} C ₆ H₁₁ |
| 2-158 | F | S | 0 | H | CF₂ Cl | CH₃ | - | CH(CH₃ )COOH |
| 2-159 | F | S | 0 | H | CF₂ Cl | CH₃ | - | CH(CH ₃)COOCH ₃ |
| 2-160 | F | S | 0 | H | CF₂ Cl | CH₃ | - | CH(CH ₃ )COOC ₂ H ₅ |
| 2-161 | F | S | 0 | H | CF₂ Cl | CH₃ | - | CH(CH₃ )COOⁿ C ₃ H ₇ |
| 2-162 | F | S | 0 | H | CF₂ Cl | CH₃ | - | CH(CH₃ )COOⁿ C H ₉ |
| 2-163 | F | S | 0 | H | CF₂ Cl | CH₃ | - | CH(CH₃ )COOⁿ C ₅ H₁₁ |
| 2-164 | F | S | 0 | H | CF₂ Cl | CH₃ | - | CH(CH₃ )COOⁱ C ₃ H ₇ |
| 2-165 | F | S | 0 | H | CF₂ Cl | CH₃ | - | CH(CH₃ )COO^{c} C ₅ H ₉ |
| 2-166 | F | S | 0 | H | CF₂ Cl | CH₃ | - | CH(CH₃ )COO^{c} C ₆ H ₁₁ |
| 2-167 | H | O | 0 | H | CF₂ Cl | CH₃ | - | H |
| 2-168 | H | O | 0 | H | CF₂ Cl | CH₃ | - | CH₃ |
| 2-169 | H | O | 0 | H | CF₂ Cl | CH₃ | - | C₂ H ₅ |
| 2-170 | H | O | 0 | H | CF₂ Cl | CH₃ | - | ⁿC H ₇ |
| 2-171 | H | O | 0 | H | CF₂ Cl | CH₃ | - | ⁿC₄ H₉ |
| 2-172 | H | O | 0 | H | CF₂ Cl | CH₃ | - | ⁱC₃ H₇ |
| 2-173 | H | O | 0 | H | CF₂ Cl | CH₃ | - | ⁱC₄ H₉ |
| 2-174 | H | O | 0 | H | CF₂ Cl | CH₃ | - | CH₂ CH=CH₂ |
| 2-175 | H | O | 0 | H | CF₂ Cl | CH₃ | - | CH(CH ₃ )CH =CH₂ |
| 2-176 | H | O | 0 | H | CF₂ Cl | CH₃ | - | CH₂ C ≡CH |
| 2-177 | H | O | 0 | H | CF₂ Cl | CH₃ | - | CH(CH₃ )C≡CH |
| 2-178 | H | O | 0 | H | CF₂ Cl | CH₃ | - | CH₂ OCH₃ |
| 2-179 | H | O | 0 | H | CF₂ Cl | CH₃ | - | CH₂ OC₂ H₅ |
| 2-180 | F | O | 0 | H | CF₂ Cl | CH₃ | - | H |
| 2-181 | F | O | 0 | H | CF₂ Cl | CH₃ | - | CH₃ |
| 2-182 | F | O | 0 | H | CF₂ Cl | CH₃ | - | C₂ H ₅ |
| 2-183 | F | O | 0 | H | CF₂ Cl | CH₃ | - | ⁿC₃ H₇ |
| 2-184 | F | O | 0 | H | CF₂ Cl | CH₃ | - | ⁿC₄ H ₉ |
| 2-185 | F | O | 0 | H | CF₂ Cl | CH₃ | - | CH₂ CH=CH₂ |
| 2-186 | F | O | 0 | H | CF₂ Cl | CH₃ | - | CH(CH₃ )CH =CH₂ |
| 2-187 | F | O | 0 | H | CF₂ Cl | CH₃ | - | CH₂ C ≡ CH |
| 2-188 | F | O | 0 | H | CF₂ Cl | CH₃ | - | CH(CH₃ )C≡CH |
| 2-189 | F | O | 0 | H | CF₂ Cl | CH₃ | - | CH₂ OCH ₃ |
| 2-190 | F | O | 0 | H | CF₂ Cl | CH₃ | - | CH₂ OC₂ H ₅ |
| 2-191 | H | O | 1 | H | CF₃ | CH₃ | - | H |
| 2-192 | H | O | 1 | H | CF₃ | CH₃ | H | CH₃ |
| 2-193 | H | O | 1 | H | CF₃ | CH₃ | H | C₂ H ₅ |
| 2-194 | H | O | 1 | H | CF₃ | CH₃ | H | ⁿC₃ H₇ |
| 2-195 | H | O | 1 | H | CF₃ | CH₃ | H | ⁱC₃ H₇ |
| 2-196 | H | O | 1 | H | CF₃ | CH₃ | H | ⁱC₄ H ₉ |
| 2-197 | H | O | 1 | H | CF₃ | CH₃ | H | ⁿC₄ H₉ |
| 2-198 | H | O | 1 | H | CF₃ | CH₃ | H | CH₂ CH₂ Cl |
| 2-199 | H | O | 1 | H | CF₃ | CH₂ | H | CH₂ CH₂ Br |
| 2-200 | H | O | 1 | H | CF₃ | CH₃ | H | CH₂ CH=CH₂ |
| 2-201 | H | O | 1 | H | CF₃ | CH₃ | H | CH(CH ₃)CH =CH₂ |
| 2-202 | H | O | 1 | H | CF₃ | CH₃ | H | CH₂ CCl = CH₂ |
| 2-203 | H | O | 1 | H | CF₃ | CH₃ | H | CH₂ C = CH |
| 2-204 | H | O | 1 | H | CF₃ | CH₃ | H | CH(CH ₃ )C=CH |
| 2-205 | H | O | 1 | H | CF₃ | CH₃ | H | CH₂ CN |
| 2-206 | H | O | 1 | H | CF₃ | CH₃ | H | CH₂ OCH₃ |
| 2-207 | H | O | 1 | H | CF₃ | CH₃ | H | CH₂ OC₂ H ₅ |
| 2-208 | H | O | 1 | H | CF₃ | CH₃ | H | CH₂ COOH |
| 2-209 | H | O | 1 | H | CF₃ | CH₃ | H | CH₂ COOCH ₃ |
| 2-210 | H | O | 1 | H | CF₃ | CH₃ | H | CH₂ COOC₂ H ₅ |
| 2-211 | H | O | 1 | H | CF ₃ | CH₃ | H | CH₂ COO ⁿ C ₃ H ₇ |
| 2-212 | H | O | 1 | H | CF₃ | CH₃ | H | CH₂ COO ⁿ C ₄ H ₉ |
| 2-213 | H | O | 1 | H | CF₃ | CH₃ | H | CH₂ COO ⁿ C ₅ H ₁₁ |
| 2-214 | H | O | 1 | H | CF₃ | CH₃ | H | CH₂ COO ⁱ C ₃ H ₇ |
| 2-215 | H | O | 1 | H | CF₃ | CH₃ | H | CH₂ COO ^{c} C ₅ H ₉ |
| 2-216 | H | O | 1 | H | CF₃ | CH₃ | H | CH₂ COO ^{c} C ₆ H ₁₁ |
| 2-217 | H | O | 1 | H | CF₃ | CH₃ | H | CH(CH ₃ )COOH |
| 2-218 | H | O | | H | CF₃ | CH₃ | H | CN(CH ₃ )COOCH ₃ |
| 2-219 | H | O | 1 | H | CF₃ | CH₃ | H | CH(CH ₃ )COOC ₂ H ₅ |
| 2-220 | H | O | 1 | H | CF₃ | CH₃ | H | CH(CH₃ )COOⁿ C₃H₇ |
| 2-221 | H | O | 1 | H | CF₃ | CH₂ | H | CH(CH₃ )COOⁿ C₄H₉ |
| 2-222 | H | O | 1 | H | CF₃ | CH₃ | H | CH(CH₃ )COOⁿ C₅H₁₁ |
| 2-223 | H | O | 1 | H | CF₃ | CH₃ | H | CH(CH₃ )COOⁱ C₃H₇ |
| 2-224 | H | O | 1 | H | CF₃ | CH₃ | H | CH(CH₃ )COO^{c} C₅H₉ |
| 2-225 | H | O | 1 | H | CF₃ | CH₃ | H | CH(CH₃ )COO^{c} C₆H₁₁ |
| 2-226 | H | O | 1 | H | CF₃ | CH₃ | CH₃ | H |
| 2-227 | H | O | 1 | H | CF₃ | CH₃ | CH₃ | CH₃ |
| 2-228 | H | O | 1 | H | CF₃ | CH₃ | CH₃ | C₂ H ₅ |
| 2-229 | H | O | 1 | H | CF₃ | CH₃ | CH₃ | ⁿC₃ H₇ |
| 2-230 | H | O | 1 | H | CF₃ | CH₃ | CH₃ | ⁱC₃ H₇ |
| 2-231 | H | O | 1 | H | CF₃ | CH₃ | CH₃ | ⁱC₄ H₉ |
| 2-232 | H | O | 1 | H | CF₃ | CH₃ | CH₃ | ⁿC₄ H₉ |
| 2-233 | H | O | 1 | H | CF₃ | CH₃ | CH₃ | CH₂ CH = CH₂ |
| 2-234 | H | O | 1 | H | CF₃ | CH₃ | CH₃ | CH(CH₃) CH = CH₂ |
| 2-235 | H | O | 1 | H | CF₃ | CH₃ | CH₃ | CH₂ C ≡ CH |
| 2-236 | H | O | 1 | H | CF₃ | CH₃ | CH₃ | CH(CH₃) C≡CH |
| 2-237 | H | O | 1 | H | CF₃ | CH₃ | CH₃ | CH₂ OCH₃ |
| 2-238 | H | O | 1 | H | CF₃ | CH₃ | CH₃ | CH₂ OC₂ H₅ |
| 2-239 | F | O | 1 | H | CF₃ | CH₃ | H | H |
| 2-240 | F | O | 1 | H | CF₃ | CH₃ | H | CH₃ |
| 2-241 | F | O | 1 | H | CF₃ | CH₃ | H | C₂ H₅ |
| 2-242 | F | O | 1 | H | CF₃ | CH₃ | H | ⁿC₃ H₇ |
| 2-243 | F | O | 1 | H | CF₃ | CH₃ | H | ⁱC ₃ H ₇ |
| 2-244 | F | O | 1 | H | CF₃ | CH₃ | H | ⁱC₄ H ₉ |
| 2-245 | F | O | 1 | H | CF₃ | CH₃ | H | ⁿC ₄ H ₉ |
| 2-246 | F | O | 1 | H | CF₃ | CH₃ | H | CH₂ CH₂ Cl |
| 2-247 | F | O | 1 | H | CF₃ | CH₃ | H | CH₂ CH₂ Br |
| 2-248 | F | O | 1 | H | CF₃ | CH₃ | H | CH₂ CH=CH₂ |
| 2-249 | F | O | 1 | H | CF₃ | CH₃ | H | CH(CH ₃ )CH = CH₂ |
| 2-250 | F | O | 1 | H | CF₃ | CH₃ | H | CH₂ CCl =CH₂ |
| 2-251 | F | O | 1 | H | CF₃ | CH₃ | H | CH₂ C ≡CH |
| 2-252 | F | O | 1 | H | CF₃ | CH₃ | H | CH(CH ₃ )C≡CH |
| 2-253 | F | O | 1 | H | CF₃ | CH₃ | H | CH₂ CN |
| 2-254 | F | O | 1 | H | CF₃ | CH₃ | H | CH₂ OCH ₃ |
| 2-255 | F | O | 1 | H | CF₃ | CH₃ | H | CH₂ OC₂ H ₅ |
| 2-256 | F | O | 1 | H | CF, | CH₃ | H | CH₂ COOH |
| 2-257 | F | O | 1 | H | CF₃ | CH₃ | H | CH₂ COOCH ₃ |
| 2-258 | F | O | 1 | H | CF₃ | CH₃ | H | CH₂ COOC₂ H ₅ |
| 2-259 | F | O | 1 | H | CF₃ | CH₃ | H | CH₂ COO ⁿ C ₃H ₇ |
| 2-260 | F | O | 1 | H | CF₃ | CH₃ | H | CH₂ COO ⁿ C ₄ H ₉ |
| 2-261 | F | O | 1 | H | CF₃ | CH₃ | H | CH₂ COO ⁿ C ₅ H₁₁ |
| 2-262 | F | O | 1 | H | CF₃ | CH₃ | H | CH₂ COO ⁱ C ₃ H₇ |
| 2-263 | F | O | 1 | H | CF₃ | CH₃ | H | CH₂ COO^{c} C₆ H₉ |
| 2-264 | F | O | | H | CF₃ | CH₃ | H | CH₂ COO ^{c} C₆ H₁₁ |
| 2-265 | F | O | 1 | H | CF₃ | CH₃ | H | CH(CH₃ )COOH |
| 2-266 | F | O | 1 | H | CF₃ | CH₃ | H | CH(CH ₃ )COOCH ₃ |
| 2-267 | F | O | 1 | H | CF₃ | CH₃ | H | CH (CH₃ ) COOC ₂ H ₅ |
| 2-268 | F | O | 1 | H | CF₃ | CH₃ | H | CH(CH₃ )COOⁿ C ₃ H ₇ |
| 2-269 | F | O | 1 | H | CF₃ | CH₃ | H | CH(CH₃ )COOⁿ C ₄ H ₉ |
| 2-270 | F | O | 1 | H | CF₃ | CH₃ | H | CH(CH₃ )COOⁿ C ₅ H ₁₁ |
| 2-271 | F | O | 1 | H | CF₃ | CH₃ | H | CH(CH₃ )COOⁿ C₃ H ₇ |
| 2-272 | F | O | 1 | H | CF₃ | CH₃ | H | CH(CH₃ )COO^{c} C ₅ H ₉ |
| 2-273 | F | O | 1 | H | CF₃ | CH₃ | H | CH(CH₃ )COO^{c} C ₆ H ₁₁ |
| 2-274 | F | O | 1 | H | CF₃ | CH₃ | CH₃ | H |
| 2-275 | F | O | 1 | H | CF₃ | CH₃ | CH₃ | CH₃ |
| 2-276 | F | O | 1 | H | CF₃ | CH₃ | CH₃ | C₂ H₅ |
| 2-277 | F | O | 1 | H | CF₃ | CH₃ | CH₃ | ⁿC₃ H₇ |
| 2-278 | F | O | 1 | H | CF₃ | CH₃ | CH₃ | ⁱC₃ H₇ |
| 2-279 | F | O | | H | CF₃ | CH₃ | CH₃ | ⁱC₄ H₉ |
| 2-280 | F | O | 1 | H | CF₃ | CH₃ | CH₃ | ⁿC₄ H₉ |
| 2-281 | F | O | 1 | H | CF₃ | CH₃ | CH₃ | CH₂ CH=CH₂ |
| 2-282 | F | O | 1 | H | CF₃ | CH₃ | CH₃ | CH(CH₃)CH =CH₂ |
| 2-283 | F | O | 1 | H | CF₃ | CH₃ | CH₃ | CH₂ C ≡CH |
| 2-284 | F | O | 1 | H | CF₃ | CH₃ | CH₃ | CH(CH₃)C≡CH |
| 2-285 | F | O | 1 | H | CF₃ | CH₃ | CH₃ | CH₂ OCH ₃ |
| 2-286 | F | O | 1 | H | CF₃ | CH₃ | CH₃ | CH₂ OC₂ H ₅ |
| 2-287 | H | S | 0 | H | CF₃ | CH₃ | - | H |
| 2-288 | H | S | 0 | H | CF₃ | CH₃ | - | CH₃ |
| 2-289 | H | S | 0 | H | CF₃ | CH₃ | - | C ₂ H s |
| 2-290 | H | S | 0 | H | CF₃ | CH₃ | - | ⁿC ₃ H ₇ |
| 2-291 | H | S | 0 | H | CF₃ | CH₃ | - | ⁿC ₄ H ₉ |
| 2-292 | H | S | 0 | H | CF₃ | CH₃ | - | ⁱC ₃ H ₇ |
| 2-293 | H | S | 0 | H | CF₃ | CH₃ | - | ⁱC ₄ H ₉ |
| 2-294 | H | S | 0 | H | CF₃ | CH₃ | - | CH₂ CH₂ Cl |
| 2-295 | H | S | 0 | H | CF₃ | CH₃ | - | CH₂ CH₂ Br |
| 2-296 | H | S | 0 | H | CF₃ | CH₃ | - | CH₂ CH = CH₂ |
| 2-297 | H | S | 0 | H | CF₃ | CH₃ | - | CH(CH ₃ )CH =CH₂ |
| 2-298 | H | S | 0 | H | CF₃ | CH₃ | - | CH₂ CCI =CH₂ |
| 2-299 | H | S | 0 | H | CF₃ | CH₃ | - | CH₂ C ≡CH |
| 2-300 | H | S | 0 | H | CF₃ | CH₃ | - | CH(CH ₃ )C ≡ CH |
| 2-301 | H | S | 0 | H | CF₂ | CH₃ | - | CH₂ CN |
| 2-302 | H | S | 0 | H | CF₃ | CH₃ | - | CH₂ OCH₃ |
| 2-303 | H | S | 0 | H | CF₃ | CH₃ | - | CH₂ OC₂ H₅ |
| 2-304 | H | S | 0 | H | CF₃ | CH₃ | - | CH₂ COOH |
| 2-305 | H | S | 0 | H | CF₃ | CH₃ | - | CH₂ COOCH₃ |
| 2-306 | H | S | 0 | H | CF₃ | CH₃ | - | CH₂ COOC₂ H |
| 2-307 | H | S | 0 | H | CF₃ | CH₃ | - | CH₂ COO ⁿ C₃ H₇ |
| 2-308 | H | S | 0 | H | CF₃ | CH₃ | - | CH₂ COO ⁿ C₄ H₉ |
| 2-309 | H | S | 0 | H | CF₃ | CH₃ | - | CH₂ COO ⁿ C ₅ H ₁₁ |
| 2-310 | H | S | 0 | H | CF₃ | CH₃ | - | CH₂ 2 COO ⁱ C ₃ H ₇ |
| 2-311 | H | S | 0 | H | CF₃ | CH₃ | - | CH₂ COO ^{c} C ₅ H ₉ |
| 2-312 | H | S | 0 | H | CF₃ | CH₃ | - | CH₂ COO ^{c} C ₆ H₁₁ |
| 2-313 | H | S | 0 | H | CF₃ | CH₃ | - | CH(CH₃)COOH |
| 2-314 | H | S | 0 | H | CF₃ | CH₃ | - | CH(CH₃)COOCH₃ |
| 2-315 | H | S | 0 | H | CF₃ | CH₃ | - | CH(CH₃ )COOC ₂ H ₅ |
| 2-316 | H | S | 0 | H | CF₃ | CH₃ | - | CH(CH₃ )COOⁿ C ₃ H ₇ |
| 2-317 | H | S | 0 | H | CF₃ | CH₃ | - | CH(CH₃ )COOⁿ C ₄ H ₉ |
| 2-318 | H | S | 0 | H | CF₃ | CH₃ | - | CH(CH₃ )COOⁿ C₅H₁₁ |
| 2-319 | H | S | 0 | H | CF₃ | CH₃ | - | CH(CH₃ )COOⁱ C ₃ H₇ |
| 2-320 | H | S | 0 | H | CF₃ | CH₃ | - | CH(CH₃ )COO^{c} C ₅ H ₉ |
| 2-321 | H | S | 0 | H | CF₃ | CH₃ | - | CH(CH₃ )COO^{c} C₆H₁₁ |
| 2-322 | F | S | 0 | H | CF₃ | CH₃ | - | H |
| 2-323 | F | S | 0 | H | CF₃ | CH₃ | - | CH₃ |
| 2-324 | F | S | 0 | H | CF₃ | CH₃ | - | C₂ H ₅ |
| 2-325 | F | S | 0 | H | CF₃ | CH₃ | - | ⁿC ₃ H ₇ |
| 2-326 | F | S | 0 | H | CF₃ | CH₃ | - | ⁿC ₄ H ₉ |
| 2-327 | F | S | 0 | H | CF₃ | CH₃ | - | ⁱC ₃ H ₇ |
| 2-328 | F | S | 0 | H | CF₃ | CH₃ | - | ^{s}C ₄ H ₉ |
| 2-329 | F | S | 0 | H | CF₃ | CH₃ | - | CH₂ CH₂ Cl |
| 2-330 | F | S | 0 | H | CF₃ | CH₃ | - | CH₂ CH₂ Br |
| 2-331 | F | S | 0 | H | CF₃ | CH₃ | - | CH₂ CH=CH₂ |
| 2-332 | F | S | 0 | H | CF₃ | CH₃ | - | CH(CH ₃ )CH = CH₂ |
| 2-333 | F | S | 0 | H | CF₃ | CH₃ | - | CH₂ CCl = CH₂ |
| 2-334 | F | S | 0 | H | CF₃ | CH₃ | - | CH₂ C = CH |
| 2-335 | F | S | 0 | H | CF₃ | CH₃ | - | CH(CH₃ )C ≡ CH |
| 2-336 | F | S | 0 | H | CF₃ | CH₃ | - | CH₂ CN |
| 2-337 | F | S | 0 | H | CF₃ | CH₃ | - | CH₂ OCH₃ |
| 2-338 | F | S | 0 | H | CF₃ | CH₃ | - | CH₂ OC₂ H ₅ |
| 2-339 | F | S | 0 | H | CF₃ | CH₃ | - | CH₂ COOH |
| 2-340 | F | S | 0 | H | CF₃ | CH₃ | - | CH₂ COOCH ₃ |
| 2-341 | F | S | 0 | H | CF₃ | CH₃ | - | CH₂ COOC₂ H ₅ |
| 2-342 | F | S | 0 | H | CF₃ | CH₃ | - | CH₂ COO ⁿ C ₃ H ₇ |
| 2-343 | F | S | 0 | H | CF₃ | CH₃ | - | CH₂ COO ⁿ C ₄H ₉ |
| 2-344 | F | S | 0 | H | CF₃ | CH₃ | - | CH₂ COO ⁿ C₅ H₁₁ |
| 2-345 | F | S | 0 | H | CF₃ | CH₃ | - | CH₂ COO ⁱ C₃ H₇ |
| 2-346 | F | S | 0 | H | CF₃ | CH₃ | - | CH₂ COO ^{c} C ₅ H ₉ |
| 2-347 | F | S | 0 | H | CF₃ | CH₃ | - | CH₂ COO ^{c} C ₆ H₁₁ |
| 2-348 | F | S | 0 | H | CF₃ | CH₃ | - | CH(CH ₃)COOH |
| 2-349 | F | S | 0 | H | CF₃ | CH₃ | - | CH(CH ₃)COOCH₃ |
| 2-350 | F | S | 0 | H | CF₃ | CH₃ | - | CH(CH₃ )COOC ₂ H ₅ |
| 2-351 | F | S | 0 | H | CF₃ | CH₃ | - | CH(CH₃ )COOⁿ C ₃ H ₇ |
| 2-352 | F | S | 0 | H | CF₃ | CH₃ | - | CH(CH₃ )COOⁿ C₄ H ₉ |
| 2-353 | F | S | 0 | H | CF₃ | CH₃ | - | CH(CH₃ )COOⁿ C ₅ H₁₁ |
| 2-354 | F | S | 0 | H | CF₃ | CH₃ | - | CH(CH₃ )COOⁱ C ₃ H ₇ |
| 2-355 | F | S | 0 | H | CF₃ | CH₃ | - | CH(CH₃ )COO^{c} C ₅ H ₉ |
| 2-356 | F | S | 0 | H | CF₃ | CH₃ | - | CH(CH₃ )COO^{c} C ₆ H ₁₁ |
| 2-357 | H | O | 0 | H | CF₃ | CH₃ | - | H |
| 2-358 | H | O | 0 | H | CF₃ | CH₃ | - | CH₃ |
| 2-359 | H | O | 0 | H | CF₃ | CH₃ | - | C₂ H ₅ |
| 2-360 | H | O | 0 | H | CF₃ | CH₃ | - | ⁿC₃ H₇ |
| 2-361 | H | O | 0 | H | CF₃ | CH₃ | - | ⁿC₄ H₉ |
| 2-362 | H | O | 0 | H | CF₃ | CH₃ | - | ⁱC₃ H ₇ |
| 2-363 | H | O | 0 | H | CF₃ | CH₃ | - | ⁱC ₄ H ₉ |
| 2-364 | H | O | 0 | H | CF₃ | CH₃ | - | CH₂ CH=CH₂ |
| 2-365 | H | O | 0 | H | CF₃ | CH₃ | - | CH(CH₃ )CH = CH₂ |
| 2-366 | H | O | 0 | H | CF₃ | CH₃ | - | CH₂ C ≡ CH |
| 2-367 | H | O | 0 | H | CF₃ | CH₃ | - | CH(CH₃ )C≡CH |
| 2-368 | H | O | 0 | H | CF₃ | CH₃ | - | CH₂ OCH₃ |
| 2-369 | H | O | 0 | H | CF₃ | CH₃ | - | CH₂ OC₂ H ₅ |
| 2-370 | F | O | 0 | H | CF₃ | CH₃ | - | H |
| 2-371 | F | O | 0 | H | CF₃ | CH₃ | - | CH₃ |
| 2-372 | F | O | 0 | H | CF₃ | CH₃ | - | C₂ H |
| 2-373 | F | O | 0 | H | CF₃ | CH₃ | - | ⁿC₃ H₇ |
| 2-374 | F | O | 0 | H | CF₃ | CH₃ | - | ⁿC₄ H₉ |
| 2-375 | F | O | 0 | H | CF₃ | CH₃ | - | CH₂ CH=CH₂ |
| 2-376 | F | O | 0 | H | CF₃ | CH₃ | - | CH(CH₃ )CH = CH₂ |
| 2-377 | F | O | 0 | H | CF₃ | CH₃ | - | CH₂ C ≡ CH |
| 2-378 | F | O | 0 | H | CF₃ | CH₃ | - | CH(CH₃ )C≡CH |
| 2-379 | F | O | 0 | H | CF₃ | CH₃ | - | CH₂ OCH₃ |
| 2-380 | F | O | 0 | H | CF₃ | CH₃ | - | CH₂ OC₂ H ₅ |
| 2-381 | H | O | l | H | CF₂ Cl | H | H | H |
| 2-382 | H | O | l | H | CF₂ Cl | H | H | CH₃ |
| 2-383 | H | O | l | H | CF₂ Cl | H | H | C₂ H ₅ |
| 2-384 | H | O | l | H | CF₂ Cl | H | H | ⁿC ₃ H ₇ |
| 2-385 | H | O | l | H | CF₂ Cl | H | H | ⁱC ₃ H ₇ |
| 2-386 | H | O | l | H | CF₂ Cl | H | H | ⁱC ₄ H ₉ |
| 2-387 | H | O | l | H | CF₂ Cl | H | H | ⁿC ₄ H ₉ |
| 2-388 | H | O | l | H | CF₂ Cl | H | H | CH₂ CH₂ Cl |
| 2-389 | H | O | l | H | CF₂ Cl | H | H | CH₂ CH₂ Br |
| 2-390 | H | O | l | H | CF₂ Cl | H | H | CH₂ CH=CH₂ |
| 2-391 | H | O | l | H | CF₂ Cl | H | H | CH(CH ₃ )CH = CH₂ |
| 2-392 | H | O | l | H | CF₂ Cl | H | H | CH₂ CCl = CH₂ |
| 2-393 | H | O | l | H | CF₂ Cl | H | H | CH₂ C ≡ CH |
| 2-394 | H | O | l | H | CF₂ Cl | H | H | CH(CH ₃)C ≡ CH |
| 2-395 | H | O | l | H | CF₂ Cl | H | H | CH₂ CN |
| 2-396 | H | O | l | H | CF₂ Cl | H | H | CH₂ OCH ₃ |
| 2-397 | H | O | l | H | CF₂ Cl | H | H | CH₂ OC₂ H ₅ |
| 2-398 | H | O | l | H | CF₂ Cl | H | H | CH₂ COOH |
| 2-399 | H | O | l | H | CF₂ Cl | H | H | CH₂ COOCH ₃ |
| 2-400 | H | O | l | H | CF₂ Cl | H | H | CH₂ COOC₂ H ₅ |
| 2-401 | H | O | l | H | CF₂ Cl | H | H | CH₂ COO ⁿ C ₂ H ₇ |
| 2-402 | H | O | l | H | CF₂ Cl | H | H | CH₂ COO ⁿ C ₄ H ₉ |
| 2-403 | H | O | l | H | CF₂ Cl | H | H | CH₂ COO ⁿ C ₅ H ₁₁ |
| 2-404 | H | O | l | H | CF₂ Cl | H | H | CH₂ COO ⁱ C ₃ H ₇ |
| 2-405 | H | O | l | H | CF₂ Cl | H | H | CH₂ COO ^{c} C ₅ H ₉ |
| 2-406 | H | O | l | H | CF₂ Cl | H | H | CH₂ COO ^{c} C ₆ H ₁₁ |
| 2-407 | H | O | l | H | CF₂ Cl | H | H | CH(CH ₃ )COOH |
| 2-408 | H | O | l | H | CF₂ Cl | H | H | CH(CH ₃ )COOCH ₃ |
| 2-409 | H | O | l | H | CF₂ Cl | H | H | CH(CH ₃ )COOC ₂ H ₅ |
| 2-410 | H | O | L | H | CF₂ Cl | H | H | CH(CH₃ )COOⁿ C ₃ H ₇ |
| 2-411 | H | O | l | H | CF₂ Cl | H | H | CH(CH₃ )COOⁿ C ₄ H ₉ |
| 2-412 | H | O | l | H | CF₂ Cl | H | H | CH(CH₃ )COOⁿ C ₅ H ₁₁ |
| 2-413 | H | O | l | H | CF₂ Cl | H | H | CH(CH₃ )COOⁱ C₃ H₇ |
| 2-414 | H | O | l | H | CF₂ Cl | H | H | CH(CH₃)COO^{c}C₅H₉ |
| 2-415 | H | O | l | H | CF₂ Cl | H | H | CH(CH₃ )COO^{c} C ₆H₁₁ |
| 2-416 | H | O | l | H | CF₂ Cl | H | CH₃ | H |
| 2-417 | H | O | l | H | CF₂ Cl | H | CH₃ | CH₃ |
| 2-418 | H | O | l | H | CF₂ Cl | H | CH₃ | C₂ H ₅ |
| 2-419 | H | O | l | H | CF₂ Cl | H | CH₃ | C ₃H ₇ |
| 2-420 | H | O | l | H | CF₂ Cl | H | CH₃ | ⁱC₃ H₇ |
| 2-421 | H | O | l | H | CF₂ Cl | H | CH₃ | ⁱC₄ H ₉ |
| 2-422 | H | O | l | H | CF₂ Cl | H | CH₃ | ⁿC₄ H₉ |
| 2-423 | H | O | l | H | CF₂ Cl | H | CH₃ | CH₂ CN≡CH₂ |
| 2-424 | H | O | l | H | CF₂ Cl | H | CH₃ | CH(CH ₃ )CH ≡CH₂ |
| 2-425 | H | O | l | H | CF₂ Cl | H | CH₃ | CH₂ C ≡CH |
| 2-426 | H | O | l | H | CF₂ Cl | H | CH ₃ | CH(CH ₃)C≡CH |
| 2-427 | H | O | l | H | CF₂ Cl | H | CH₃ | CH₂ OCH ₃ |
| 2-428 | H | O | l | H | CF₂ Cl | H | CH₃ | CH₂ OC₂ H ₅ |
| 2-429 | F | O | l | H | CF₂ Cl | H | H | H |
| 2-430 | F | O | l | H | CF₂ Cl | H | H | CH₃ |
| 2-431 | F | O | l | H | CF₂ Cl | H | H | C₂ H ₅ |
| 2-432 | F | O | l | H | CF₂ Cl | H | H | ⁿC ₃ H ₇ |
| 2-433 | F | O | l | H | CF₂ Cl | H | H | ⁱ C ₃ H ₇ |
| 2-434 | F | O | l | H | CF₂ Cl | H | H | ⁱC ₄ H ₉ |
| 2-435 | F | O | l | H | CF₂ Cl | H | H | ⁿC ₄ H ₉ |
| 2-436 | F | O | l | H | CF₂ Cl | H | H | CH₂ CH₂ Cl |
| 2-437 | F | O | l | H | CF₂ Cl | H | H | CH₂ CH₂ Br |
| 2-438 | F | O | l | H | CF₂ Cl | H | H | CH₂ CH=CH₂ |
| 2-439 | F | O | l | H | CF₂ Cl | H | H | CH(CH₃) CH =CH₂ |
| 2-440 | F | O | l | H | CFz Cl | H | H | CH₂ CCl = CH₂ |
| 2-441 | F | O | l | H | CF₂ Cl | H | H | CH₂ C ≡CH |
| 2-442 | F | O | l | H | CF₂ Cl | H | H | CH(CH ₃ )C≡CH |
| 2-443 | F | O | l | H | CF₂ Cl | H | H | CH₂ CN |
| 2-444 | F | O | l | H | CF₂ Cl | H | H | CH₂ OCH ₃ |
| 2-445 | F | O | l | H | CF₂ Cl | H | H | CH₂ OC₂ H ₅ |
| 2-446 | F | O | l | H | CF₂ Cl | H | H | CH₂ COOH |
| 2-447 | F | O | l | H | CF₂ Cl | H | H | CH₂ COOCH₃ |
| 2-448 | F | O | l | H | CF₂ Cl | H | H | CH₂ COOC₂ H s |
| 2-449 | F | O | l | H | CF₂ Cl | H | H | CH₂ COO ⁿ C ₃ H ₇ |
| 2-450 | F | O | l | H | CF₂ Cl | H | H | CH₂ COO ⁿ C₄ H ₉ |
| 2-451 | F | O | l | H | CF₂ Cl | H | H | CH₂ COO ⁿ C ₅ H ₁₁ |
| 2-452 | F | O | l | H | CF₂ Cl | H | H | CH₂ COO ⁱ C ₃ H ₇ |
| 2-453 | F | O | l | H | CF₂ Cl | H | H | CH₂ COO ^{c} C ₅ H ₉ |
| 2-454 | F | O | l | H | CF₂ Cl | H | H | CH₂ COO ^{c} C₆ H ₁₁ |
| 2-455 | F | O | l | H | CF₂ Cl | H | H | CH(CH ₃ )COOH |
| 2-456 | F | O | l | H | CF₂ Cl | H | H | CH(CH ₃ )COOCH ₃ |
| 2-457 | F | O | l | H | CF₂ Cl | H | H | CH(CH ₃ )COOC ₂ H ₅ |
| 2-458 | F | O | l | H | CF₂ Cl | H | H | CH(CH₃ )COOⁿ C₃ H₇ |
| 2-459 | F | O | l | H | CF₂ Cl | H | H | CH(CH₃ )COOⁿ C₄ H₉ |
| 2-460 | F | O | l | H | CF₂ Cl | H | H | CH(CH₃ )COOⁿ C ₅ H₁₁ |
| 2-461 | F | O | l | H | CF₂ Cl | H | H | CH(CH₃)COOⁱ C ₃ H₇ |
| 2-462 | F | O | l | H | CF₂ Cl | H | H | CH(CH₃ )COO^{c} C ₅ H₉ |
| 2-463 | F | O | l | H | CF₂ Cl | H | H | CH(CH₃ )COO^{c} C ₆ H₁₁ |
| 2-464 | F | O | l | H | CF₂ Cl | H | CH₃ | H |
| 2-465 | F | O | l | H | CF₂ Cl | H | CH₃ | CH₃ |
| 2-466 | F | O | l | H | CF₂ Cl | H | CH₃ | C₂ H ₅ |
| 2-467 | F | O | l | H | CF₂ Cl | H | CH₃ | ⁿC₃ H₇ |
| 2-468 | F | O | l | H | CF₂ Cl | H | CH₃ | ⁱC ₃ H ₇ |
| 2-469 | F | O | l | H | CF₂ Cl | H | CH₃ | ⁱC ₄ H ₉ |
| 2-470 | F | O | l | H | CF₂ Cl | H | CH₃ | ⁿC₄ H ₉ |
| 2-471 | F | O | l | H | CF₂ Cl | H | CH₃ | CH₂ CH=CH₂ |
| 2-472 | F | O | l | H | CF₂ Cl | H | CH₃ | CH(CH ₃ )CH = CH₂ |
| 2-473 | F | O | l | H | CF₂ Cl | H | CH₃ | CH₂ C ≡ CH |
| 2-474 | F | O | l | H | CF₂ Cl | H | CH₃ | CH(CH ₂ )C ≡ CH |
| 2-475 | F | O | l | H | CF₂ Cl | H | CH₃ | CH₂ OCH ₃ |
| 2-476 | F | O | l | H | CF₂ Cl | H | CH₃ | CH₂ OC₂ H ₅ |
| 2-477 | H | S | 0 | H | CF₂ Cl | H | - | H |
| 2-478 | H | S | 0 | H | CF₂ Cl | H | - | CH₃ |
| 2-479 | H | S | 0 | H | CF₂ Cl | H | - | C₂ H ₅ |
| 2-480 | H | S | 0 | H | CF₂ Cl | H | - | ⁿC ₃ H ₇ |
| 2-481 | H | S | 0 | H | CF₂ Cl | H | - | ⁿ C ₄ H ₉ |
| 2-482 | H | S | 0 | H | CF: Cl | H | - | ⁱC ₃ H ₇ |
| 2-483 | H | S | 0 | H | CF₂ Cl | H | - | ⁱ C ₄ H ₉ |
| 2-484 | H | S | 0 | H | CF₂ Cl | H | - | CH₂ CH₂ Cl |
| 2-485 | H | S | 0 | H | CF₂ Cl | H | - | CH₂ CH₂ Br |
| 2-486 | H | S | 0 | H | CF₂ Cl | H | - | CH₂ CH= CH₂ |
| 2-487 | H | S | 0 | H | CF₂ Cl | H | - | CH(CH₃ )CH = CH₂ |
| 2-488 | H | S | 0 | H | CF₂ Cl | H | - | CH₂ CCl = CH₂ |
| 2-489 | H | S | 0 | H | CF₂ Cl | H | - | CH₂ C ≡ CH |
| 2-490 | H | S | 0 | H | CF₂ Cl | H | - | CH(CH₃ )C≡CH |
| 2-491 | H | S | 0 | H | CF₂ Cl | H | - | CH₂ CN |
| 2-492 | H | S | 0 | H | CF₂ Cl | H | - | CH₂ OCH ₃ |
| 2-493 | H | S | 0 | H | CFx Cl | H | - | CH₂ OC₂ H ₅ |
| 2-494 | H | S | 0 | H | CF₂ Cl | H | - | CH₂ COOH |
| 2-495 | H | S | 0 | H | CF₂ Cl | H | - | CH₂ COOCH₃ |
| 2-496 | H | S | 0 | H | CF₂ Cl | H | - | CH₂ COOC₂ H ₅ |
| 2-497 | H | S | 0 | H | CF₂ Cl | H | - | CH₂ COOⁿ C₃H₇ |
| 2-498 | H | S | 0 | H | CF₂ Cl | H | - | CH₂ COO ⁿ C₄H₉ |
| 2-499 | H | S | 0 | H | CF₂ Cl | H | - | CH₂ COO ⁿ C₅ H₁₁ |
| 2-500 | H | S | 0 | H | CF₂ Cl | H | - | CH₂ COO ⁱ C₃H₇ |
| 2-501 | H | S | 0 | H | CF₂ Cl | H | - | CH₂ COO^{c} C₃H₉ |
| 2-502 | H | S | 0 | H | CF₂ Cl | H | - | CH₂COO^{c}C₆H₁₁ |
| 2-503 | H | S | 0 | H | CF₂ Cl | H | - | CH(CH₃ )COOH |
| 2-504 | H | S | 0 | H | CF₂ Cl | H | - | CH(CH₃)COOCH ₃ |
| 2-505 | H | S | 0 | H | CF₂ Cl | H | - | CH(CH₃)COOC ₂ H ₅ |
| 2-506 | H | S | 0 | H | CF₂ Cl | H | - | CH(CH₃ )COOⁿ C₃ H₇ |
| 2-507 | H | S | 0 | H | CF₂ Cl | H | - | CH(CH₃ )COOⁿ C₄ H₉ |
| 2-508 | H | S | 0 | H | CF₂ Cl | H | - | CH(CH₃ )COOⁿ C ₅ H₁₁ |
| 2-509 | H | S | 0 | H | CF₂ Cl | H | - | CH(CH₃ )COOⁱ C₃ H ₇ |
| 2-510 | H | S | 0 | H | CF₂ Cl | H | - | CH(CH₃ )COO^{c} C₅ H ₉ |
| 2-511 | H | S | 0 | H | CF₂ Cl | H | - | CH(CH₃ )COO^{c} C ₆ H ₁₁ |
| 2-512 | F | S | 0 | H | CF₂ Cl | H | - | H |
| 2-513 | F | S | 0 | H | CF₂ Cl | H | - | CH₃ |
| 2-514 | F | S | 0 | H | CF₂ Cl | H | - | C₂ H ₆ |
| 2-515 | F | S | 0 | H | CF₂ Cl | H | - | ⁿC H ₇ |
| 2-516 | F | S | 0 | H | CF₂ Cl | H | - | ⁿC ₄ H ₉ |
| 2-517 | F | S | 0 | H | CF₂ Cl | H | - | C₃ H₇ |
| 2-518 | F | S | 0 | H | CF₂ Cl | H | - | ⁱ C₄ H ₉ |
| 2-519 | F | S | 0 | H | CF₂ Cl | H | - | CH₂ CH₂ Cl |
| 2-520 | F | S | 0 | H | CF₂ Cl | H | - | CH₂ CH₂ Br |
| 2-521 | F | S | 0 | H | CF₂ Cl | H | - | CH₂ CH=CH₂ |
| 2-522 | F | S | 0 | H | CF₂ Cl | H | - | CH(CH ₃)CH = CH₂ |
| 2-523 | F | S | 0 | H | CF₂ Cl | H | - | CH₂ CCl =CH₂ |
| 2-524 | F | S | 0 | H | CF₂ Cl | H | - | CH₂ C ≡ CH |
| 2-525 | F | S | 0 | H | CF₂ Cl | H | - | CH(CH₃ ) C≡CH |
| 2-526 | F | S | 0 | H | CF₂ Cl | H | - | CH₂ CN |
| 2-527 | F | S | 0 | H | CF₂ Cl | H | - | CH₂ OCH₃ |
| 2-528 | F | S | 0 | H | CF₂ Cl | H | - | CH₂ OC₂ H ₅ |
| 2-529 | F | S | 0 | H | CF₂ Cl | H | - | CH₂ COOH |
| 2-530 | F | S | 0 | H | CF₂ Cl | H | - | CH₂ COOCH ₃ |
| 2-531 | F | S | 0 | H | CF₂ Cl | H | - | CH₂ COOC₂ H ₅ |
| 2-532 | F | S | 0 | H | CF₂ Cl | H | - | CH₂ COO ⁿ C₃ H₇ |
| 2-533 | F | S | 0 | H | CF₂ Cl | H | - | CH₂ COO ⁿ C ₄ H₉ |
| 2-534 | F | S | 0 | H | CF₂ Cl | H | - | CH₂ COO ⁿ C ₅ H₁₁ |
| 2-535 | F | S | 0 | H | CF₂ Cl | H | - | CH₂ COO ⁱ C ₃ H₇ |
| 2-536 | F | S | 0 | H | CF₂ Cl | H | - | CH₂ COO ^{c} C₅ H₉ |
| 2-537 | F | S | 0 | H | CF₂ Cl | H | - | CH₂ COO ^{c} C₆ H₁₁ |
| 2-538 | F | S | 0 | H | CF₂ Cl | H | - | CH(CH₃)COOH |
| 2-539 | F | S | 0 | H | CF₂ Cl | H | - | CH(CH₃ )COOCH₃ |
| 2-540 | F | S | 0 | H | CF₂ Cl | H | - | CH(CH₃ )COOC₂H₅ |
| 2-541 | F | S | 0 | H | CF₂ Cl | H | - | CH(CH₃ )COOⁿ C₃ H₇ |
| 2-542 | F | S | 0 | H | CF₂ Cl | H | - | CH(CH₃ )COOⁿ C₄ H₉ |
| 2-543 | F | S | 0 | H | CF₂ Cl | H | - | CH(CH₃ )COOⁿC₅H₁₁ |
| 2-544 | F | S | 0 | H | CF₂ Cl | H | - | CH(CH₃ )COOⁱC₃H₇ |
| 2-545 | F | S | 0 | H | CF₂ Cl | H | - | CH(CH₃ )COO^{c}C₅ H ₉ |
| 2-546 | F | S | 0 | H | CF₂ Cl | H | - | CH(CH₃ )COO^{c} C ₆H₁₁ |
| 2-547 | H | O | 0 | H | CF₂ Cl | H | - | H |
| 2-548 | H | O | 0 | H | CF₂ Cl | H | - | CH₃ |
| 2-549 | H | O | 0 | H | CF₂ Cl | H | - | C₂ H ₅ |
| 2-550 | H | O | 0 | H | CF₂ Cl | H | - | ⁿC₃H₇ |
| 2-551 | H | O | 0 | H | CF₂ Cl | H | - | ⁿC₄H₉ |
| 2-552 | H | O | 0 | H | CF₂ Cl | H | - | ⁱC₃H₇ |
| 2-553 | H | O | 0 | H | CF₂ Cl | H | - | ⁱC₄H₉ |
| 2-554 | H | O | 0 | H | CF₂ Cl | H | - | CH₂ CH=CH₂ |
| 2-555 | H | O | 0 | H | CF₂ Cl | H | - | CH(CH₃ )CH =CH₂ |
| 2-556 | H | O | 0 | H | CF₂ Cl | H | - | CH₂ C ≡CH |
| 2-557 | H | O | 0 | H | CF₂ Cl | H | - | CH(CH₃)C≡CH |
| 2-558 | H | O | 0 | H | CF₂ Cl | H | - | CH₂ OCH₃ |
| 2-559 | H | O | 0 | H | CF₂ Cl | H | - | CH₂ OC₂ H₅ |
| 2-560 | F | O | 0 | H | CF₂ Cl | H | - | H |
| 2-561 | F | O | 0 | H | CF₂ Cl | H | - | CH₃ |
| 2-562 | F | O | 0 | H | CF₂ Cl | H | - | C₂ H ₅ |
| 2-563 | F | O | 0 | H | CF₂ Cl | H | - | ⁿC₃ H₇ |
| 2-564 | F | O | 0 | H | CF₂ Cl | H | - | ⁿC ₄ H ₉ |
| 2-565 | F | O | 0 | H | CF₂ Cl | H | - | CH₂ CH=CH₂ |
| 2-566 | F | O | 0 | H | CF₂ Cl | H | - | CH(CH₃)CH = CH₂ |
| 2-567 | F | O | 0 | H | CF₂ Cl | H | - | CH₂ C ≡ CH |
| 2-568 | F | O | 0 | H | CF₂ Cl | H | - | CH(CH ₃ )C≡CH |
| 2-569 | F | O | 0 | H | CF₂ Cl | H | - | CH₂ OCH₃ |
| 2-570 | F | O | 0 | H | CF₂ Cl | H | - | CH₂ OC₂ H ₅ |
| 2-571 | H | O | 1 | H | CF₃ | H | H | H |
| 2-572 | H | O | 1 | H | CF₃ | H | H | CH₃ |
| 2-573 | H | O | 1 | H | CF₃ | H | H | C₂ H ₅ |
| 2-574 | H | O | 1 | H | CF₃ | H | H | ⁿC ₃ H ₇ |
| 2-575 | H | O | 1 | H | CF₃ | H | H | ⁱC ₃ H ₇ |
| 2-576 | H | O | 1 | H | CF₃ | H | H | ⁱC ₄ H ₉ |
| 2-577 | H | O | 1 | H | CF₃ | H | H | ⁿC ₄ H ₉ |
| 2-578 | H | O | 1 | H | CF₃ | H | H | CH₂ CH₂ Cl |
| 2-579 | H | O | 1 | H | CF₃ | H | H | CH₂ CH₂ Br |
| 2-580 | H | O | 1 | H | CF₃ | H | H | CH₂ CH=CH₂ |
| 2-581 | H | O | 1 | H | CF₃ | H | H | CH(CH ₃ )CH =CH₂ |
| 2-582 | H | O | 1 | H | CF₃ | H | H | CH₂ CCl =CH₂ |
| 2-583 | H | O | 1 | H | CF₃ | H | H | CH₂ C ≡ CH |
| 2-584 | H | O | 1 | H | CF₃ | H | H | CH(CH₃ )C≡CH |
| 2-585 | H | O | 1 | H | CF₃ | H | H | CH₂ CN |
| 2-586 | H | O | 1 | H | CF₃ | H | H | CH₂ OCH ₃ |
| 2-587 | H | O | 1 | H | CF₃ | H | H | CH₂ OC ₂ H ₅ |
| 2-588 | H | O | 1 | H | CF₃ | H | H | CH₂ COOH |
| 2-589 | H | O | 1 | H | CF₃ | H | H | CH₂ COOCH ₃ |
| 2-590 | H | O | 1 | H | CF₃ | H | H | CH₂ COOC₂ H ₅ |
| 2-591 | H | O | 1 | H | CF₃ | H | H | CH₂ COO ⁿ C₃ H ₇ |
| 2-592 | H | O | 1 | H | CF₃ | H | H | CH₂ COO ⁿ C₄ H ₉ |
| 2-593 | H | O | 1 | H | CF₃ | H | H | CH₂ COOⁿ C ₅ H₁₁ |
| 2-594 | H | O | 1 | H | CF₃ | H | H | CH₂ COOⁱ C₃ H ₇ |
| 2-595 | H | O | 1 | H | CF₃ | H | H | CH₂ COO ^{c} C ₅ H ₉ |
| 2-596 | H | O | 1 | H | CF₃ | H | H | CH₂ COO ^{c} C ₆ H ₁₁ |
| 2-597 | H | O | 1 | H | CF₃ | H | H | CH (CH ₃ ) COOH |
| 2-598 | H | O | 1 | H | CF₃ | H | H | CH (CH ₃ ) COOCH ₃ |
| 2-599 | H | O | 1 | H | CF₃ | H | H | CH(CH ₃ )COOC ₂ H ₅ |
| 2-600 | H | O | 1 | H | CF₃ | H | H | CH(CH₃ )COOⁿ C ₃ H ₇ |
| 2-601 | H | O | 1 | H | CF₃ | H | H | CH(CH₃ )COOⁿ C ₄ H ₉ |
| 2-602 | H | O | 1 | H | CF₃ | H | H | CH(CH₃ )COOⁿ C ₅ H ₁₁ |
| 2-603 | H | O | 1 | H | CF₃ | H | H | CH(CH₃ )COOⁱ C ₃ H ₇ |
| 2-604 | H | O | 1 | H | CF₃ | H | H | CH(CH₃ )COO^{c} C ₅ H ₉ |
| 2-605 | H | O | 1 | H | CF₃ | H | H | CH(CH₃ )COO^{c} C ₆H₁₁ |
| 2-606 | H | O | 1 | H | CF₃ | H | CH₃ | H |
| 2-607 | H | O | 1 | H | CF₃ | H | CH₃ | CH₃ |
| 2-608 | H | O | 1 | H | CF₃ | H | CH₃ | C₂ H₅ |
| 2-609 | H | O | 1 | H | CF₃ | H | CH₃ | ⁿC ₃ H₇ |
| 2-610 | H | O | 1 | H | CF₃ | H | CH₃ | ⁱC ₃ H₇ |
| 2-611 | H | O | 1 | H | CF₃ | H | CH₃ | ⁱC ₄ H₉ |
| 2-612 | H | O | 1 | H | CF₃ | H | CH₃ | ⁿC₄ H ₉ |
| 2-613 | H | O | 1 | H | CF₃ | H | CH₃ | CH₂ CH=CH₂ |
| 2-614 | H | O | 1 | H | CF₃ | H | CH₃ | CH(CH ₃)CH = CH₂ |
| 2-615 | H | O | 1 | H | CF₃ | H | CH₃ | CH₂ C ≡CH |
| 2-616 | H | O | 1 | H | CF₃ | H | CH₃ | CH(CH₃ )C≡CH |
| 2-617 | H | O | 1 | H | CF₃ | H | CH₃ | CH₂ OCH ₃ |
| 2-618 | H | O | 1 | H | CF₃ | H | CH₃ | CH₂ OC₂ H ₅ |
| 2-619 | F | O | 1 | H | CF₃ | H | H | H |
| 2-620 | F | O | 1 | H | CF₃ | H | H | CH₃ |
| 2-621 | F | O | 1 | H | CF₃ | H | H | C₂ H ₅ |
| 2-622 | F | O | 1 | H | CF₃ | H | H | ⁿC ₃ H ₇ |
| 2-623 | F | O | 1 | H | CF₃ | H | H | ⁱC ₃ H ₇ |
| 2-624 | F | O | 1 | H | CF₃ | H | H | ⁱC ₄ H ₉ |
| 2-625 | F | O | 1 | H | CF₃ | H | H | ⁿC ₄ H ₉ |
| 2-626 | F | O | 1 | H | CF₃ | H | H | CH₂ CH₂ Cl |
| 2-627 | F | O | 1 | H | CF₃ | H | H | CH₂ CH₂ Br |
| 2-628 | F | O | 1 | H | CF₃ | H | H | CH₂ CH=CH₂ |
| 2-629 | F | O | 1 | H | CF₃ | H | H | CH(CH₃)CH = CH₂ |
| 2-630 | F | O | 1 | H | CF₃ | H | H | CH₂ CCl = CH₂ |
| 2-631 | F | O | 1 | H | CF₃ | H | H | CHz C ≡CH |
| 2-632 | F | O | 1 | H | CF₃ | H | H | CH(CH ₃ )C≡CH |
| 2-633 | F | O | 1 | H | CF₃ | H | H | CH₂ CN |
| 2-634 | F | O | 1 | H | CF₃ | H | H | CH₂ OCH ₃ |
| 2-635 | F | O | 1 | H | CF₃ | H | H | CH₂ OC₂ H ₅ |
| 2-636 | F | O | 1 | H | CF₃ | H | H | CH₂ COOH |
| 2-637 | F | O | 1 | H | CF₃ | H | H | CH₂ COOCH ₃ |
| 2-638 | F | O | 1 | H | CF₃ | H | H | CH₂ COOC₂ H ₅ |
| 2-639 | F | O | 1 | H | CF₃ | H | H | CH₂ COO ⁿ C ₃ H ₇ |
| 2-640 | F | O | 1 | H | CF₃ | H | H | CH₂ COO ⁿ C ₄ H ₉ |
| 2-641 | F | O | 1 | H | CF₃ | H | H | CH₂ COO ⁿ C ₅ H₁₁ |
| 2-642 | F | O | 1 | H | CF₃ | H | H | CH₂ COO ⁱ C ₃ H₇ |
| 2-643 | F | O | 1 | H | CF₃ | H | H | CH₂ COO ^{c} C₅ H₉ |
| 2-644 | F | O | 1 | H | CF₃ | H | H | CH₂ COO ^{c} C₅ H₁₁ |
| 2-645 | F | O | 1 | H | CF₃ | H | H | CH(CH₃ )COOH |
| 2-646 | F | O | 1 | H | CF₃ | H | H | CH(CH₃) COOCH₃ |
| 2-647 | F | O | 1 | H | CF₃ | H | H | CH(CH₃) COOC₂ H₅ |
| 2-648 | F | O | 1 | H | CF₃ | H | H | CH(CH₃ )COOⁿ C ₃ H ₇ |
| 2-648 | F | O | 1 | H | CF₃ | H | H | CH(CH₃ )COOⁿ C ₄ H ₉ |
| 2-650 | F | O | 1 | H | CF₃ | H | H | CH(CH₃ )COOⁿ C ₅ H ₁₁ |
| 2-651 | F | O | 1 | H | CF₃ | H | H | CH(CH₃ )COOⁱ C ₃ H ₇ |
| 2-652 | F | O | 1 | H | CF₃ | H | H | CH(CH₃ )COO^{c} C ₅ H ₉ |
| 2-653 | F | O | 1 | H | CF₃ | H | H | CH(CH₃ )COO^{c} C ₆ H ₁₁ |
| 2-654 | F | O | 1 | H | CF₃ | H | CH₃ | H |
| 2-655 | F | O | 1 | H | CF₃ | H | CH₃ | CH₃ |
| 2-656 | F | O | 1 | H | CF₃ | H | CH₃ | C₂ H₅ |
| 2-657 | F | O | 1 | H | CF₃ | H | CH₃ | ⁿC ₃ H₇ |
| 2-658 | F | O | 1 | H | CF₃ | H | CH₃ | ⁱC ₃ H ₇ |
| 2-659 | F | O | 1 | H | CF₃ | H | CH₃ | ⁱC ₄ H ₉ |
| 2-660 | F | O | 1 | H | CF₃ | H | CH₃ | ⁿC ₄ H ₉ |
| 2-661 | F | O | 1 | H | CF₃ | H | CH₃ | CH₂ CH = CH₂ |
| 2-662 | F | O | 1 | H | CF₃ | H | CH₃ | CH(CH₃)CH = CH₂ |
| 2-663 | F | O | 1 | H | CF₃ | H | CH₃ | CH₂ C ≡CH |
| 2-664 | F | O | 1 | H | CF₃ | H | CH₃ | CH(CH ₃)C≡CH |
| 2-665 | F | O | 1 | H | CF₃ | H | CH₃ | CH₂ OCH₃ |
| 2-666 | F | O | 1 | H | CF₃ | H | CH₃ | CH₂ OC₂ H ₅ |
| 2-667 | H | S | 0 | H | CF₃ | H | - | H |
| 2-668 | H | S | 0 | H | CF₃ | H | - | CH₃ |
| 2-669 | H | S | 0 | H | CF₃ | H | - | C₂ H ₅ |
| 2-670 | H | S | 0 | H | CF₃ | H | - | ⁿC₃ H ₇ |
| 2-671 | H | S | 0 | H | CF₃ | H | - | ⁿC ₄ H ₉ |
| 2-672 | H | S | 0 | H | CF₃ | H | - | ⁱC ₃ H ₇ |
| 2-673 | H | S | 0 | H | CF₃ | H | - | ⁱC₄ H ₉ |
| 2-674 | H | S | 0 | H | CF₃ | H | - | CH₂ CH₂ Cl |
| 2-675 | H | S | 0 | H | CF₃ | H | - | CH₂ CH₂ Br |
| 2-676 | H | S | 0 | H | CF₃ | H | - | CH₂ CH=CH₂ |
| 2-677 | H | S | 0 | H | CF₃ | H | - | CH(CH ₃ )CH = CH₂ |
| 2-678 | H | S | 0 | H | CF₃ | H | - | CHz CCl = CH₂ |
| 2-679 | H | S | 0 | H | CF₃ | H | - | CH₂ C ≡ CH |
| 2-680 | H | S | 0 | H | CF₃ | H | - | CH(CH ₃ )C=CH |
| 2-681 | H | S | 0 | H | CF₃ | H | - | CHz CN |
| 2-682 | H | S | 0 | H | CF ₃ | H | - | CH₂ OCH ₃ |
| 2-683 | H | S | 0 | H | CF₃ | H | - | CH₂ OC₂ H ₅ |
| 2-684 | H | S | 0 | H | CF₃ | H | - | CH₂ COOH |
| 2-685 | H | S | 0 | H | CF₃ | H | - | CH₂ COOCH ₃ |
| 2-686 | H | S | 0 | H | CF₃ | H | - | CH₂ COOC₂ H ₅ |
| 2-687 | H | S | 0 | H | CF₃ | H | - | CH₂ COO ⁿ C ₃ H ₇ |
| 2-688 | H | S | 0 | H | CF₃ | H | - | CH₂ COO ⁿC ₄ H ₉ |
| 2-689 | H | S | 0 | H | CF₃ | H | - | CH₂ COO ⁿ C ₅ H₁₁ |
| 2-690 | H | S | 0 | H | CF₃ | H | - | CH₂ COO ⁱ C ₃ H ₇ |
| 2-691 | H | S | 0 | H | CF₃ | H | - | CH₂ COO ^{c} C ₅ H ₉ |
| 2-692 | H | S | 0 | H | CF₃ | H | - | CH₂ COO ^{c}C ₆H₁₁ |
| 2-693 | H | S | 0 | H | CF₃ | H | - | CH(CH ₃ )COOH |
| 2-694 | H | S | 0 | H | CF₃ | H | - | CH(CH₃)COOCH₃ |
| 2-695 | H | S | 0 | H | CF₃ | H | - | CH(CH₃ )COOC ₂ H ₅ |
| 2-696 | H | S | 0 | H | CF₃ | H | - | CH(CH₃ )COOⁿ C₃ H₇ |
| 2-697 | H | S | 0 | H | CF₃ | H | - | CH(CH₃ )COOⁿ C ₄ H₉ |
| 2-698 | H | S | 0 | H | CF₃ | H | - | CH(CH₃ )COOⁿ C ₅ H ₁₁ |
| 2-699 | H | S | 0 | H | CF₃ | H | - | CH(CH₃ )COOⁱ C ₃ H ₇ |
| 2-700 | H | S | 0 | H | CF₃ | H | - | CH(CH₃ )COO^{c} C ₅ H ₉ |
| 2-701 | H | S | 0 | H | CF₃ | H | - | CH (CH₃) COO^{c} C ₆ H ₁₁ |
| 2-702 | F | S | 0 | H | CF₃ | H | - | H |
| 2-703 | F | S | 0 | H | CF₃ | H | - | CH₃ |
| 2-704 | F | S | 0 | H | CF₃ | H | - | C₂ H ₅ |
| 2-705 | F | S | 0 | H | CF₃ | H | - | ⁿC ₃ H ₇ |
| 2-706 | F | S | 0 | H | CF₃ | H | - | ⁿ C ₄ H ₉ |
| 2-707 | F | S | 0 | H | CF₃ | H | - | ⁱC ₃ H ₇ |
| 2-708 | F | S | 0 | H | CF₃ | H | - | ^{c}C ₄ H ₉ |
| 2-709 | F | S | 0 | H | CF₃ | H | - | CH₂ CH₂ Cl |
| 2-710 | F | S | 0 | H | CF₃ | H | - | CH₂ CH₂ Br |
| 2-711 | F | S | 0 | H | CF₃ | H | - | CHz CH=CH₂ |
| 2-712 | F | S | 0 | H | CF₃ | H | - | CH(CH ₃ )CH = CH₂ |
| 2-713 | F | S | 0 | H | CF₃ | H | - | CH₂ CCl = CH₂ |
| 2-714 | F | S | 0 | H | CF₃ | H | - | CH₂ C ≡ CH |
| 2-715 | F | S | 0 | H | CF₃ | H | - | CH(CH₃)C≡ CH |
| 2-716 | F | S | 0 | H | CF₃ | H | - | CH₂ CN |
| 2-717 | F | S | 0 | H | CF₃ | H | - | CH₂ OCH ₃ |
| 2-718 | F | S | 0 | H | CF₃ | H | - | CH₂ OC₂ H ₅ |
| 2-719 | F | S | 0 | H | CF₃ | H | - | CH₂ COOH |
| 2-720 | F | S | 0 | H | CF₃ | H | - | CH₂COOCH₃ |
| 2-721 | F | S | 0 | H | CF₃ | H | - | CH₂COOC₂H ₅ |
| 2-722 | F | S | 0 | H | CF₃ | H | - | CH₂ COO ⁿC₃ H ₇ |
| 2-723 | F | S | 0 | H | CF₃ | H | - | CH₂ COO ⁿ C ₄H₉ |
| 2-724 | F | S | 0 | H | CF₃ | H | - | CH₂ COO ⁿ C ₅ H₁₁ |
| 2-725 | F | S | 0 | H | CF₃ | H | - | CH₂ COO ⁱ C₃ H₇ |
| 2-726 | F | S | 0 | H | CF₃ | H | - | CH₂ COO ^{c} C ₅ H₉ |
| 2-727 | F | S | 0 | H | CF₃ | H | - | CH₂ COO ^{c} C ₆ H ₁₁ |
| 2-728 | F | S | 0 | H | CF₃ | H | - | CH(CH₃ )COOH |
| 2-729 | F | S | 0 | H | CF₃ | H | - | CH(CH₂ )COOCH₃ |
| 2-730 | F | S | 0 | H | CF₃ | H | - | CH(CH₃ )COOC₂ H ₅ |
| 2-731 | F | S | 0 | H | CF₃ | H | - | CH(CH₃ )COOⁿ C₃ H₇ |
| 2-732 | F | S | 0 | H | CF₃ | H | - | CH(CH₃ )COOⁿ C ₄ H ₉ |
| 2-733 | F | S | 0 | H | CF₃ | H | - | CH(CH₃ )COOⁿ C ₅ H ₁₁ |
| 2-734 | F | S | 0 | H | CF₃ | H | - | CH(CH₃ )COOⁱ C ₃ H ₇ |
| 2-735 | F | S | 0 | H | CF₃ | H | - | CH(CH₃ )COO^{c} C ₅ H ₉ |
| 2-736 | F | S | 0 | H | CF₃ | H | - | CH(CH₃ )COO^{c} C ₆ H ₁₁ |
| 2-737 | H | O | 0 | H | CF₃ | H | - | H |
| 2-738 | H | O | 0 | H | CF₃ | H | - | CH₃ |
| 2-739 | H | O | 0 | H | CF₃ | H | - | C₂ H ₅ |
| 2-740 | H | O | 0 | H | CF₃ | H | - | ⁿC ₃ H ₇ |
| 2-741 | H | O | 0 | H | CF₃ | H | - | ⁿC ₄ H ₉ |
| 2-742 | H | O | 0 | H | CF₃ | H | - | ⁱC₃ H₇ |
| 2-743 | H | O | 0 | H | CF₃ | H | - | ⁱC₄ H₉ |
| 2-744 | H | O | 0 | H | CF₃ | H | - | CH₂ CH=CH₂ |
| 2-745 | H | O | 0 | H | CF₃ | H | - | CH(CH ₃ ) CH = CH₂ |
| 2-746 | H | O | 0 | H | CF₃ | H | - | CH₂ C ≡ CH |
| 2-747 | H | O | 0 | H | CF₃ | H | - | CH(CH ₃ ) C≡CH |
| 2-748 | H | O | 0 | H | CF₃ | H | - | CH₂ OCH ₃ |
| 2-749 | H | O | 0 | H | CF₃ | H | - | CH₂ OC₂ H ₅ |
| 2-750 | F | O | 0 | H | CF₃ | H | - | H |
| 2-751 | F | O | 0 | H | CF₃ | H | - | CH₃ |
| 2-752 | F | O | 0 | H | CF₃ | H | - | C₂ H ₅ |
| 2-753 | F | O | 0 | H | CF₃ | H | - | ⁿC₃ H ₇ |
| 2-754 | F | O | 0 | H | CF₃ | H | - | ⁿC₄ H ₉ |
| 2-755 | F | O | 0 | H | CF₃ | H | - | CHz CH=CH₂ |
| 2-756 | F | O | 0 | H | CF₃ | H | - | CH(CH ₃)CH = CH₂ |
| 2-757 | F | O | 0 | H | CF₃ | H | - | CH₂ C ≡ CH |
| 2-758 | F | O | 0 | H | CF₃ | H | - | CH(CH₃) C≡CH |
| 2-759 | F | O | 0 | H | CF₃ | H | - | CH₂ OCH₃ |
| 2-760 | F | O | 0 | H | CF₃ | H | - | CH₂ OC₂ H ₅ |
| 2-761 | H | O | 1 | CH₃ | CF₃ | H | H | H |
| 2-762 | H | O | 1 | CH₃ | CF₃ | H | H | CH₃ |
| 2-763 | H | O | 1 | CH₃ | CF₃ | H | H | C₂ H ₅ |
| 2-764 | H | O | 1 | CH₃ | CF₃ | H | H | ⁿC₃ H₇ |
| 2-765 | H | O | 1 | CH₃ | CF₃ | H | H | ⁱC ₃ H ₇ |
| 2-766 | H | O | 1 | CH₃ | CF₃ | H | H | ⁱC ₄ H ₉ |
| 2-767 | H | O | 1 | CH₃ | CF₃ | H | H | ⁿC ₄ H ₉ |
| 2-768 | H | O | 1 | CH₃ | CF₃ | H | H | CH₂ CH₂ Cl |
| 2-769 | H | O | 1 | CH₃ | CF₃ | H | H | CH₂ CH₂ Br |
| 2-770 | H | O | 1 | CH₃ | CF₃ | H | H | CH₂ CH= CH₂ |
| 2-771 | H | O | 1 | CH₃ | CF₃ | H | H | CH(CH ₃ )CH = CH₂ |
| 2-772 | H | O | 1 | CH₃ | CF₃ | H | H | CH₂ CCl = CH₂ |
| 2-773 | H | O | 1 | CH₃ | CF₃ | H | H | CH₂ C ≡ CH |
| 2-774 | H | O | 1 | CH₃ | CF₃ | H | H | CH(CH₃ )C≡CH |
| 2-775 | H | O | 1 | CH₃ | CF₃ | H | H | CH₂ CN |
| 2-776 | H | O | 1 | CH₃ | CF₃ | H | H | CH₂OCH₃ |
| 2-777 | H | O | 1 | CH₃ | CF₃ | H | H | CH₂ OC₂ H₅ |
| 2-778 | H | O | 1 | CH₃ | CF₃ | H | H | CH₂ COOH |
| 2-779 | H | O | 1 | CH₃ | CF₃ | H | H | CH₂ COOCH ₃ |
| 2-780 | H | O | 1 | CH₃ | CF₃ | H | H | CH₂ COOC₂ H ₅ |
| 2-781 | H | O | 1 | CH₃ | CF₃ | H | H | CH₂ COO ⁿ C ₃ H ₇ |
| 2-782 | H | O | 1 | CH₃ | CF₃ | H | H | CH₂ COOⁿ C₄H₉ |
| 2-783 | H | O | 1 | CH₃ | CF₃ | H | H | CH₂ COOⁿ C₅ H₁₁ |
| 2-784 | H | O | 1 | CH₃ | CF₃ | H | H | CH₂ COO ⁱ C ₃ H ₇ |
| 2-785 | H | O | 1 | CH₃ | CF₃ | H | H | CH₂ COO ^{c} C ₆ H ₉ |
| 2-786 | H | O | 1 | CH₃ | CF₃ | H | H | CH₂ COO ^{c} C ₆H₁₁ |
| 2-787 | H | O | 1 | 1 CH₃ | CF₃ | H | H | CH(CH₃ )COOH |
| 2-788 | H | O | 1 | CH₃ | CF₃ | H | H | CH(CH ₃ )COOCH ₃ |
| 2-789 | H | O | 1 | CH₃ | CF₃ | H | H | CH(CH ₃ )COOC ₂H ₅ |
| 2-790 | H | O | 1 | CH₃ | CF₃ | H | H | CH(CH₃ )COOⁿ C₃ H₇ |
| 2-791 | H | O | 1 | CH₃ | CF₃ | H | H | CH(CH₃ )COOⁿ C ₄ H₉ |
| 2-792 | H | O | 1 | CH₃ | CF₃ | H | H | CH(CH₃)COOⁿ C ₅ H₁₁ |
| 2-793 | H | O | 1 | CH₃ | CF₃ | H | H | CH(CH₃ )COOⁱ C₃ H₇ |
| 2-794 | H | O | 1 | CH₃ | CF₃ | H | H | CH(CH₃ )COO^{c} C ₅ H ₉ |
| 2-795 | H | O | 1 | CH₃ | CF₃ | H | H | CH(CH₃ )COO^{c} C ₆H₁₁ |
| 2-796 | H | O | 1 | CH₃ | CF₃ | H | CH₃ | H |
| 2-797 | H | O | 1 | CH₃ | CF₃ | H | CH₃ | CH₃ |
| 2-798 | H | O | 1 | CH₃ | CF₃ | H | CH₃ | C₂ H₅ |
| 2-799 | H | O | 1 | CH₃ | CF₃ | H | CH₃ | ⁿC₃ H₇ |
| 2-800 | H | O | 1 | CH₃ | CF₃ | H | CH₃ | ⁱC ₃ H ₇ |
| 2-801 | H | O | 1 | CH₃ | CF₃ | H | CH₃ | ⁱC ₄ H ₉ |
| 2-802 | H | O | 1 | CH₃ | CF₃ | H | CH₃ | ⁿC ₄ H ₉ |
| 2-803 | H | O | 1 | CH₃ | CF₃ | H | CH₃ | CH₂ CH=CH₂ |
| 2-804 | H | O | 1 | CH₃ | CF₃ | H | CH₃ | CH(CH ₃ )CH = CH₂ |
| 2-805 | H | O | 1 | CH₃ | CF₃ | H | CH₃ | CH₂ C ≡ CH |
| 2-806 | H | O | 1 | CH₃ | CF₃ | H | CH₃ | CH(CH ₃ )C≡CH |
| 2-807 | H | O | 1 | CH₃ | CF₃ | H | CH₃ | CH₂ OCH ₃ |
| 2-808 | H | O | 1 | CH₃ | CF₃ | H | CH₃ | CH₂ OC₂ H ₅ |
| 2-809 | F | O | 1 | CH₃ | CF₃ | H | H | H |
| 2-810 | F | | 1 | CH₃ | CF₃ | H | H | CH₃ |
| 2-811 | F | O | 1 | CH₃ | CF₃ | H | H | C₂ H ₅ |
| 2-812 | F | O | 1 | CH₃ | CF₃ | H | H | ⁿ C₃ H₇ |
| 2-813 | F | O | 1 | CH₃ | CF₃ | H | H | ⁱC₃ H ₇ |
| 2-814 | F | O | 1 | CH₃ | CF₃ | H | H | ⁱ C₄ H ₉ |
| 2-815 | F | O | 1 | CH₃ | CF₃ | H | H | ⁿC₄ H ₉ |
| 2-816 | F | O | 1 | CH₃ | CF₃ | H | H | CH₂ CH₂ Cl |
| 2-817 | F | O | 1 | CH₃ | CF₃ | H | H | CH₂ CH₂ Br |
| 2-818 | F | O | 1 | CH₃ | CF₃ | H | H | CH₂ CH= CH₂ |
| 2-819 | F | O | 1 | CH₃ | CF₃ | H | H | CH(CH ₃ )CH = CH₂ |
| 2-820 | F | O | 1 | CH₃ | CF₃ | H | H | CH₂ CCl = CH₂ |
| 2-821 | F | O | 1 | CH₃ | CF₃ | H | H | CH₂ C ≡ CH |
| 2-822 | F | O | 1 | CH₃ | CF₃ | H | H | CH (CH ₃ )C ≡ CH |
| 2-823 | F | O | 1 | CH₃ | CF₃ | H | H | CH₂ CN |
| 2-824 | F | O | 1 | CH₃ | CF₃ | H | H | CH₂ OCH₃ |
| 2-825 | F | O | 1 | CH₃ | CF₃ | H | H | CH₂ OC₂ H ₅ |
| 2-826 | F | O | 1 | CH₃ | CF₃ | H | H | CH₂ COOH |
| 2-827 | F | O | 1 | CH₃ | CF₃ | H | H | CH₂ COOCH ₃ |
| 2-828 | F | O | 1 | CH₃ | CF₃ | H | H | CH₂ COOC₂ H ₅ |
| 2-829 | F | O | 1 | CH₃ | CF₃ | H | H | CH₂ COO ⁿ C₃ H ₇ |
| 2-830 | F | O | 1 | CH₃ | CF₃ | H | H | CH₂ COO ⁿ C₄ H ₉ |
| 2-831 | F | O | 1 | CH₃ | CF₃ | H | H | CH₂ COO ⁿ C₅ H₁₁ |
| 2-832 | F | O | 1 | CH₃ | CF₃ | H | H | CH₂ COOⁱ C₃ H₇ |
| 2-833 | F | O | 1 | CH₃ | CF₃ | H | H | CH₂ COO ^{c} C₅ H ₉ |
| 2-834 | F | O | 1 | CH₃ | CF₃ | H | H | CH₂ COO ^{c} C₆ H₁₁ |
| 2-835 | F | O | 1 | CH₃ | CF₃ | H | H | CH(CH₃)COOH |
| 2-836 | F | O | 1 | CH₃ | CF₃ | H | H | CH(CH₃)COOCH₃ |
| 2-837 | F | O | 1 | CH₃ | CF₃ | H | H | CH(CH₃)COOC ₂ H₅ |
| 2-838 | F | O | 1 | CH₃ | CF₃ | H | H | CH(CH₃ )COOⁿ C₃ H₇ |
| 2-839 | F | 0 | 1 | CH₃ | CF₃ | H | H | CH(CH₃ )COOⁿ C H₉ |
| 2-840 | F | O | 1 | CH₃ | CF₃ | H | H | CH(CH₃ )COOⁿ C ₅ H₁₁ |
| 2-841 | F | O | 1 | CH₃ | CF₃ | H | H | CH (CH₃ )COOⁱ C₃ H ₇ |
| 2-842 | F | O | 1 | CH₃ | CF₃ | H | H | CH(CH₃ )COO^{c} C ₅ H ₉ |
| 2-843 | F | O | 1 | CH₃ | CF₃ | H | H | CH(CH₃ )COO^{c} C₆ H₁₁ |
| 2-843 | F | O | 1 | CH₃ | CF₃ | H | CH₃ | H |
| 2-845 | F | O | 1 | CH₃ | CF₃ | H | CH₃ | CH₃ |
| 2-846 | F | O | 1 | CH₃ | CF₃ | H | CH₃ | C₂ H s |
| 2-847 | F | O | 1 | CH₃ | CF₃ | H | CH₃ | ⁿC ₃ H ₇ |
| 2-848 | F | O | 1 | CH₃ | CF₃ | H | CH₃ | ⁱC ₃ H ₇ |
| 2-849 | F | O | 1 | CH₃ | CF₃ | H | CH₃ | ⁱC ₄ H ₉ |
| 2-850 | F | 0 | 1 | CH₃ | CF₃ | H | CH₃ | ⁿC ₄ H ₉ |
| 2-851 | F | 0 | 1 | CH₃ | CF₃ | H | CH₃ | CH₂ CH=CH₂ |
| 2-852 | F | 0 | 1 | CH₃ | CF₃ | H | CH₃ | CH(CH ₂ )CH = CH₂ |
| 2-853 | F | 0 | 1 | CH₃ | CF₃ | H | CH₃ | CH₂ C ≡ CH |
| 2-854 | F | O | 1 | CH₃ | CF₃ | H | CH₃ | CH(CH ₃ )C≡CH |
| 2-855 | F | O | 1 | CH₃ | CF₃ | H | CH₃ | CH₂ OCH ₃ |
| 2-856 | F | O | 1 | CH₃ | CF₃ | H | CH₃ | CH₂ OC₂ H ₅ |
| 2-857 | H | S | 0 | CH₃ | CF₃ | H | - | H |
| 2-858 | H | S | 0 | CH₃ | CF₃ | H | - | CH₃ |
| 2-859 | H | S | 0 | CH₃ | CF₃ | H | - | C₂ H s |
| 2-860 | H | S | 0 | CH₃ | CF₃ | H | - | ⁿC₃ H₇ |
| 2-861 | H | S | 0 | CH ₃ | CF₃ | H | - | ⁿC₄ H ₉ |
| 2-862 | H | S | 0 | CH₃ | CF₃ | H | - | ⁱC₃ H ₇ |
| 2-863 | H | S | 0 | CH₃ | CF₃ | H | - | ⁱC₄ H ₉ |
| 2-864 | H | S | 0 | CH₃ | CF₃ | H | - | CH₂ CH₂ Cl |
| 2-865 | H | S | 0 | CH₃ | CF₃ | H | - | CH₂ CH₂ Br |
| 2-866 | H | S | 0 | CH₃ | CF₃ | H | - | CH₂ CH= CH₂ |
| 2-867 | H | S | 0 | CH₃ | CF₃ | H | - | CH (CH ₃)CH = CH₂ |
| 2-868 | H | S | 0 | CH₃ | CF₃ | H | - | CH₂ CCl =CH₂ |
| 2-869 | H | S | 0 | CH₃ | CF₃ | H | - | CH₂ C ≡CH |
| 2-870 | H | S | 0 | CH₃ | CF₃ | H | - | CH(CH₃)C≡CH |
| 2-871 | H | S | 0 | CH₃ | CF₃ | H | - | CH₂CN |
| 2-872 | H | S | 0 | CH₃ | CF₃ | H | - | CH₂OCH₃ |
| 2-873 | H | S | 0 | CH₃ | CF₃ | H | - | CH₂ OC₂ H ₅ |
| 2-874 | H | S | 0 | CH₃ | CF₃ | H | - | CH₂ COOH |
| 2-875 | H | S | 0 | CH₃ | CF₃ | H | - | CH₂ COOCH ₃ |
| 2-876 | H | S | 0 | CH₃ | CF₃ | H | - | CH₂ COOC₂ H ₅ |
| 2-877 | H | S | 0 | CH₃ | CF₃ | H | - | CH₂ COO ⁿ C₃H₇ |
| 2-878 | H | S | 0 | CH₃ | CF₃ | H | - | CH₂ COOⁿC₄H₉ |
| 2-879 | H | S | 0 | CH₃ | CF₃ | H | - | CH₂ COO ⁿ C ₅ H ₁₁ |
| 2-880 | H | S | 0 | CH₃ | CF₃ | H | - | CH₂ COO ⁱ C ₃ H ₇ |
| 2-881 | H | S | 0 | CH₃ | CF₃ | H | - | CH₂ COO ^{c} C ₅ H ₉ |
| 2-882 | H | S | 0 | CH₃ | CF₃ | H | - | CH₂ COO ^{c} C ₆ H₁₁ |
| 2-883 | H | S | 0 | CH₃ | CF₃ | H | - | CH(CH₃ )COOH |
| 2-884 | H | S | 0 | CH₃ | CF₃ | H | - | CH(CH ₃ )COOCH ₃ |
| 2-885 | H | S | 0 | CH₃ | CF₃ | H | - | CH(CH₃ )COOC ₂ H ₅ |
| 2-886 | H | S | 0 | CH₃ | CF₃ | H | - | CH(CH₃ )COOⁿ C ₃ H ₇ |
| 2-887 | H | S | 0 | CH₃ | CF₃ | H | - | CH(CH₃ )COOⁿ C ₄ H ₉ |
| 2-888 | H | S | 0 | CH₃ | CF₃ | H | - | CH(CH₃ )COOⁿ C ₅ H ₁₁ |
| 2-889 | H | S | 0 | CH₃ | CF₃ | H | - | CH(CH₃ )COO C₃ H ₇ |
| 2-890 | H | S | 0 | CH₃ | CF₃ | H | - | CH(CH₃ )COO^{c} C s H ₉ |
| 2-891 | H | S | 0 | CH₃ | CF₃ | H | - | CH(CH₃ )COO^{c} C ₆ H₁₁ |
| 2-892 | F | S | 0 | CH₃ | CF₃ | H | - | H |
| 2-893 | F | S | 0 | CH₃ | CF₃ | H | - | CH₃ |
| 2-894 | F | S | 0 | CH₃ | CF₃ | H | - | C₂ H ₅ |
| 2-895 | F | S | 0 | CH₃ | CF₃ | H | - | ⁿC₃ H₇ |
| 2-896 | F | S | 0 | CH₃ | CF₃ | H | - | ⁿC₄ H₉ |
| 2-897 | F | S | 0 | CH₃ | CF₃ | H | - | ⁱC₃ H₇ |
| 2-898 | F | S | 0 | CH₃ | CF₃ | H | - | ^{s}C₄ H₉ |
| 2-899 | F | S | 0 | CH₃ | CF₃ | H | - | CH₂ CH₂ Cl |
| 2-900 | F | S | 0 | CH₃ | CF₃ | H | - | CH₂ CH₂ Br |
| 2-901 | F | S | 0 | CH₃ | CF₃ | H | - | CH₂ CH=CH₂ |
| 2-902 | F | S | 0 | CH₃ | CF₃ | H | - | CH(CH ₃ )CH =CH₂ |
| 2-903 | F | S | 0 | CH₃ | CF₃ | H | - | CH₂ CCl =CH₂ |
| 2-904 | F | S | 0 | CH₃ | CF₃ | H | - | CH₂ C ≡ CH |
| 2-905 | F | S | 0 | CH₃ | CF₃ | H | - | CH(CH ₃ )C≡CH |
| 2-906 | F | S | 0 | CH₃ | CF₃ | H | - | CH₂ CN |
| 2-907 | F | S | 0 | CH₃ | CF₃ | H | - | CH₂ OCH ₃ |
| 2-908 | F | S | 0 | CH₃ | CF₃ | H | - | CH₂ OC₂ H ₅ |
| 2-909 | F | S | 0 | CH₃ | CF₃ | H | - | CH₂ COOH |
| 2-910 | F | S | 0 | CH₃ | CF₃ | H | - | CH₂ COOCH ₃ |
| 2-911 | F | S | 0 | CH₃ | CF₃ | H | - | CH₂ COOC₂ H ₅ |
| 2-912 | F | S | 0 | CH₃ | CF₃ | H | - | CH₂ COO ⁿ C₃ H ₇ |
| 2-913 | F | S | 0 | CH₃ | CF₃ | H | - | CH₂ COO ⁿ C ₄ H ₉ |
| 2-914 | F | S | 0 | CH₃ | CF₃ | H | - | CH₂ COO ⁿ C ₅ H₁₁ |
| 2-915 | F | S | 0 | CH₃ | CF₃ | H | - | CH₂ COO ⁱ C₃ H₇ |
| 2-916 | F | S | 0 | CH₃ | CF₃ | H | - | CH₂ COO ^{c} C ₅ H ₉ |
| 2-917 | F | S | 0 | CH₃ | CF₃ | H | - | CH₂ COO ^{c} C₆ H₁₁ |
| 2-918 | F | S | 0 | CH₃ | CF₃ | H | - | CH(CH ₃ )COOH |
| 2-919 | F | S | 0 | CH₃ | CF₃ | H | - | CH(CH₃)COOCH ₃ |
| 2-920 | F | S | 0 | CH₃ | CF₃ | H | - | CH(CH₃)COOC ₂ H₅ |
| 2-921 | F | S | | 0 CH₃ | CF₃ | H | - | CH(CH₃ )COOⁿ C₃ H₇ |
| 2-922 | F | S | 0 | CH₃ | CF₃ | H | - | CH(CH₃ )COOⁿ C₄ H ₉ |
| 2-923 | F | S | 0 | CH₃ | CF₃ | H | - | CH(CH₃ )COOⁿ C ₅H₁₁ |
| 2-924 | F | S | 0 | CH₃ | CF₃ | H | - | CH(CH₃ )COOⁱ C₃ H₇ |
| 2-925 | F | S | 0 | CH₃ | CF₃ | H | - | CH(CH₃ )COO^{c} C₅ H₉ |
| 2-926 | F | S | 0 | CH₃ | CF₃ | H | - | CH(CH₃ )COO^{c} C₆ H₁₁ |
| 2-927 | H | O | 0 | CH₃ | CF₃ | H | - | H |
| 2-928 | H | O | 0 | CH₃ | CF₃ | H | - | CH₃ |
| 2-929 | H | O | 0 | CH₃ | CF₃ | H | - | C₂ H ₅ |
| 2-930 | H | O | 0 | CH₃ | CF₃ | H | - | ⁿC ₃ H ₇ |
| 2-931 | H | O | 0 | CH₃ | CF₃ | H | - | ⁿC ₄ H ₉ |
| 2-932 | H | O | 0 | CH₃ | CF₃ | H | - | ⁱC ₃ H ₇ |
| 2-933 | H | O | 0 | CH₃ | CF₃ | H | - | ⁱC ₄ H ₉ |
| 2-934 | H | O | 0 | CH₃ | CF₃ | H | - | CH₂ CH=CH₂ |
| 2-935 | H | O | 0 | CH₃ | CF₃ | H | - | CH (CH ₃ )CH = CH₂ |
| 2-936 | H | O | 0 | CH₃ | CF₃ | H | - | CH₂ C ≡ CH |
| 2-937 | H | O | 0 | CH₃ | CF₃ | H | - | CH(CH₃)C≡CH |
| 2-938 | H | O | 0 | CH₃ | CF₃ | H | - | CH₂ OCH₃ |
| 2-939 | H | O | 0 | CH₃ | CF₃ | H | - | CH₂ OC₂ H |
| 2-940 | F | O | 0 | CH₃ | CF₃ | H | - | H |
| 2-941 | F | O | 0 | CH₃ | CF₃ | H | - | CH₃ |
| 2-942 | F | O | 0 | CH₃ | CF₃ | H | - | C₂ H ₅ |
| 2-943 | F | O | 0 | CH₃ | CF₃ | H | - | ⁿC₃ H₇ |
| 2-944 | F | O | 0 | CH₃ | CF₃ | H | - | ⁿC₄ H₉ |
| 2-945 | F | O | 0 | CH₃ | CF₃ | H | - | CH₂ CH=CH₂ |
| 2-946 | F | O | 0 | CH₃ | CF₃ | H | - | CH(CH₃ )CH = CH₂ |
| 2-947 | F | O | 0 | CH₃ | CF₃ | H | - | CH₂ C ≡ CH |
| 2-948 | F | O | 0 | CH₃ | CF₃ | H | - | CH(CH ₃)C≡CH |
| 2-949 | F | O | 0 | CH₃ | CF₃ | H | - | CH₂ OCH ₃ |
| 2-950 | F | O | 0 | CH₃ | CF₃ | H | - | CH₂ OC₂ H₅ |

**TABLE 3**

| | | | | | | | |
|---|---|---|---|---|---|---|---|
| Compounds of the formula: | | | | | | | |
| 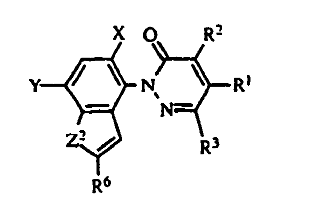 | | | | | | | |

| Compound No. | X | Y | Z ² | R³ | R¹ | R ² | R ⁶ |
|---|---|---|---|---|---|---|---|
| 3-1 | H | F | O | H | CF₂ Cl | H | CH₃ |
| 3-2 | H | Cl | O | H | CF₂ Cl | H | CH₃ |
| 3-3 | H | Br | O | H | CF₂ Cl | H | CH₃ |
| 3-4 | F | F | O | H | CF₂ Cl | H | CH₃ |
| 3-5 | F | Cl | O | H | CF₂ Cl | H | CH₃ |
| 3-6 | F | Br | O | H | CF₂ Cl | H | CH₃ |
| 3-7 | H | F | O | H | CF₂ Cl | CH ₃ | CH₃ |
| 3-8 | H | Cl | O | H | CF₂ Cl | CH₃ | CH₃ |
| 3- 9 | H | Br | O | H | CF₂ Cl | CH₃ | CH₃ |
| 3-10 | F | F | O | H | CF₂ Cl | CH₃ | CH₃ |
| 3-11 | F | Cl | O | H | CF₂ Cl | CH₃ | CH₃ |
| 3-12 | F | Br | O | H | CF₂ Cl | CH₃ | CH₃ |
| 3-13 | H | F | O | H | CF₂ Cl | H | C₂ H ₆ |
| 3-14 | H | Cl | O | H | CF₂ Cl | H | C₂ H ₅ |
| 3-15 | H | Br | O | H | CF₂ Cl | H | C₂ H₅ |
| 3-16 | F | F | O | H | CF₂ Cl | H | C₂ H ₅ |
| 3-17 | F | Cl | O | H | CF₂ Cl | H | C₂ H ₅ |
| 3-18 | F | Br | O | H | CF₂ Cl | H | C₂ H₅ |
| 3-19 | H | F | O | H | CF₂ Cl | CH₃ | C₂ H ₅ |
| 3-20 | H | Cl | O | H | CF₂ Cl | CH₃ | C₂ H ₅ |
| 3-21 | H | Br | O | H | CF₂ Cl | CH₃ | C₂ H ₅ |
| 3-22 | F | F | O | H | CF₂ Cl | CH₃ | C₂ H ₅ |
| 3-23 | F | Cl | O | H | CF₂ Cl | CH₃ | C₂ H ₅ |
| 3-24 | F | Br | O | H | CF₂ Cl | CH₃ | C₂ H ₅ |
| 3-25 | H | F | O | H | CF₂ Cl | CH₃ | CH₂ Br |
| 3-26 | H | F | O | H | CF₂ Cl | CH₃ | CH Br₂ |
| 3-27 | H | F | O | H | CF₂ Cl | CH₃ | CBr ₃ |
| 3-28 | H | F | O | H | CF₂ Cl | CH₃ | CHO |
| 3-29 | H | F | O | H | CF₂ Cl | CH₃ | CN |
| 3-30 | H | F | O | H | CF₂ Cl | CH₃ | COOH |
| 3-31 | H | F | O | H | CF₂ Cl | CH₃ | CH₂ OH |
| 3-32 | H | F | O | H | CF₂ Cl | CH₃ | CH₂ OCH ₃ |
| 3-33 | H | F | O | H | CF₂ Cl | CH₃ | CH₂ OC₂ H ₅ |
| 3-39 | H | F | O | H | CF₂ Cl | CH₃ | CH₂ Oⁱ C₃ H ₇ |
| 3-35 | H | F | O | H | CF₂ Cl | CH₃ | CH₂ OCH₂ OCH ₃ |
| 3-36 | H | F | O | H | CF₂ Cl | CH₃ | CH₂ OCH ₂ OC₂ H₅ |
| 3-37 | H | F | O | H | CF₂ Cl | CH₂ | CH₂ OCOCH₃ |
| 3-38 | H | F | O | H | CF₂ Cl | CH₃ | CH₂ OCOC₂ H ₅ |
| 3-39 | H | F | O | H | CF₂ Cl | CH₃ | CH₂ OCO ⁱ C ₃ H ₇ |
| 3-40 | H | F | O | H | CF₂ Cl | CH₃ | CH₂ OCOCH ₂ Cl |
| 3-41 | H | F | O | H | CF₂ Cl | CH₃ | CH₂ OCOCCl ₃ |
| 3-42 | H | F | O | H | CF₂ Cl | CH₃ | CH₂ OCOCF ₃ |
| 3-43 | H | F | O | H | CF₂ Cl | CH₃ | COOCH₃ |
| 3-44 | H | F | O | H | CF₂ Cl | CH₃ | COOC₂ H ₅ |
| 3-45 | H | F | O | H | CF₂ Cl | CH₃ | COO ⁿ C ₃ H ₇ |
| 3-46 | H | F | O | H | CF₂ Cl | CH₃ | COO ⁿ C H ₉ |
| 3-47 | H | F | O | H | CF₂ Cl | CH₃ | COO ⁿ C ₅ H ₁₁ |
| 3-48 | H | F | O | H | CF₂ Cl | CH₃ | COO ⁱ C₃ H₇ |
| 3-49 | H | F | O | H | CF₂ Cl | CH₃ | COCH₃ |
| 3-50 | H | F | O | H | CF₂ Cl | CH₃ | COC₂ H₅ |
| 3-51 | H | Cl | O | H | CF₂ Cl | CH₃ | CH₂ Br |
| 3-52 | H | Cl | O | H | CFz Cl | CH₃ | CH Br₂ |
| 3-53 | H | Cl | O | H | CF₂ Cl | CH₃ | CBr₃ |
| 3-54 | H | Cl | O | H | CF₂ Cl | CH₃ | CHO |
| 3-55 | H | Cl | O | H | CF₂ Cl | CH₃ | CN |
| 3-56 | H | Cl | O | H | CF₂ Cl | CH₃ | COOH |
| 3-57 | H | Cl | O | H | CF₂ Cl | CH₃ | CH₂ OH |
| 3-58 | H | Cl | O | H | CF₂ Cl | CH₃ | CH₂ OCH ₃ |
| 3-59 | H | Cl | O | H | CF₂ Cl | CH₃ | CH₂ OC₂ H ₅ |
| 3-60 | H | Cl | O | H | CF₂ Cl | CH₃ | CH₂ O ⁱ C ₃ H₇ |
| 3-61 | H | Cl | O | H | CF₂ Cl | CH₃ | CH₂ OCH ₂ OCH ₃ |
| 3-62 | H | Cl | O | H | CF₂ Cl | CH₃ | CH₂ OCH ₂ OC₂ H ₅ |
| 3-63 | H | Cl | O | H | CF₂ Cl | CH₃ | CH₂ OCOCH ₃ |
| 3-64 | H | Cl | O | H | CF₂ Cl | CH₃ | CH₂ OCOC₂ H ₅ |
| 3-65 | H | Cl | O | H | CF₂ Cl | CH₃ | CH₂ OCO C ₃ H ₇ |
| 3-66 | H | Cl | O | H | CF₂ Cl | CH₃ | CH₂ OCOCH ₂ Cl |
| 3-67 | H | Cl | O | H | CF₂ Cl | CH₃ | CH₂ OCOCCl₃ |
| 3-67 | H | Cl | O | H | CF₂ Cl | CH₃ | CH₂ OCOCF ₃ |
| 3-69 | H | Cl | O | H | CF₂ Cl | CH₃ | COOCH₃ |
| 3-70 | H | Cl | O | H | CF₂ Cl | CH₃ | COOC₂ H ₅ |
| 3-71 | H | Cl | O | H | CF₂ Cl | CH₃ | COO ⁿ C ₃ H ₇ |
| 3-72 | H | Cl | O | H | CF₂ Cl | CH₃ | COOⁿC ₄ H ₉ |
| 3-73 | H | Cl | O | H | CF₂ Cl | CH₃ | COO ⁿ C ₅ H ₁₁ |
| 3-74 | H | Cl | O | H | CF₂ Cl | CH₃ | COO ⁱ C ₃ H ₇ |
| 3-75 | H | Cl | O | H | CF₂ Cl | CH₃ | COCH₃ |
| 3-76 | H | Cl | O | H | CF₂ Cl | CH₃ | COC₂ H ₅ |
| 3-77 | F | F | O | H | CF₂ Cl | CH₃ | CH₂ Br |
| 3-78 | F | F | O | H | CF₂ Cl | CH₃ | CH Br₂ |
| 3-79 | F | F | O | H | CF₂ Cl | CH₃ | CBr₃ |
| 3-80 | F | F | O | H | CF₂ Cl | CH₃ | CHO |
| 3-81 | F | F | O | H | CF₂ Cl | CH₃ | CN |
| 3-82 | F | F | O | H | CF₂ Cl | CH₃ | COOH |
| 3-83 | F | F | O | H | CF₂ Cl | CH₃ | CH₂ OH |
| 3-84 | F | F | O | H | CF₂ Cl | CH₃ | CH₂ OCH₃ |
| 3-85 | F | F | O | H | CF₂ Cl | CH₃ | CH₂ OC₂ H₅ |
| 3-86 | F | F | O | H | CF₂ Cl | CH₃ | CH₂ Oⁱ C₃ H₇ |
| 3-87 | F | F | O | H | CF₂ Cl | CH₃ | CH₂ OCH₂ OCH₃ |
| 3-88 | F | F | O | H | CF₂ Cl | CH₃ | CH₂ OCH ₂ OC₂ H ₅ |
| 3-89 | F | F | O | H | CF₂ Cl | CH₃ | CH₂ OCOCH ₃ |
| 3-90 | F | F | O | H | CF₂ Cl | CH₃ | CH₂ OCOC₂ H ₅ |
| 3-91 | F | F | O | H | CF₂ Cl | CH₃ | CH₂ OCO ⁱ C ₃ H₇ |
| 3-92 | F | F | O | H | CF₂ Cl | CH₃ | CH₂ OCOCH ₂ Cl |
| 3-93 | F | F | O | H | CF₂ Cl | CH₃ | CH₂ OCOCCl₃ |
| 3-94 | F | F | O | H | CF₂ Cl | CH₃ | CH₂ OCOCF₃ |
| 3-95 | F | F | O | H | CF₂ Cl | CH₃ | COOCH₃ |
| 3-96 | F | F | O | H | CF₂ Cl | CH₃ | COOC₂ H₅ |
| 3-97 | F | F | O | H | CF₂ Cl | CH₃ | COO ⁿC₃ H₇ |
| 3-98 | F | F | O | H | CF₂ Cl | CH₃ | COO ⁿC₄ H₉ |
| 3-99 | F | F | O | H | CF₂ Cl | CH₃ | COO ⁿC ₅ H₁₁ |
| 3-100 | F | F | O | H | CF₂ Cl | CH₃ | COO ⁿ C₃H₇ |
| 3-101 | F | F | O | H | CF₂ Cl | CH₃ | COCH₃ |
| 3-102 | F | F | O | H | CF₂ Cl | CH₃ | COC₂H₅ |
| 3-103 | F | Cl | O | H | CF₂ Cl | CH₃ | CH₂Br |
| 3-104 | F | Cl | O | H | CF₂ Cl | CH₃ | CH Br₂ |
| 3-105 | F | Cl | O | H | CF₂ Cl | CH₃ | CBr₃ |
| 3-106 | F | C 1 | O | H | CF₂ Cl | CH₃ | CHO |
| 3-107 | F | C1 | O | H | CF₂ C1 | CH₃ | CN |
| 3-108 | F | C1 | O | H | CF₂ Cl | CH₃ | COOH |
| 3-109 | F | C1 | O | H | CF₂ C1 | CH₃ | CH₂ OH |
| 3-110 | F | Cl | O | H | CF₂ C1 | CH₃ | CH₂ OCH₃ |
| 3-111 | F | C1 | O | H | CF₂ Cl | CH₃ | CH₂ OC₂ H ₅ |
| 3-112 | F | C 1 | O | H | CF₂ Cl | CH₃ | CH₂ Oⁱ C ₃ H₇ |
| 3-113 | F | Cl | O | H | CF₂ Cl | CH₃ | CH₂ OCH ₂ OCH ₃ |
| 3-114 | F | Cl | O | H | CF₂ Cl | CH₃ | CH₂ OCH ₂ OC₂ H ₅ |
| 3-115 | F | Cl | O | H | CF₂ Cl | CH₃ | CH₂ OCOCH ₃ |
| 3-116 | F | Cl | O | H | CF₂ Cl | CH₃ | CH₂ OCOC₂ H₅ |
| 3-117 | F | Cl | O | H | CF₂ Cl | CH₃ | CH₂ OCO ⁱ C₃H₇ |
| 3-118 | F | Cl | O | H | CF₂ Cl | CH₃ | CH₂ OCOCH₂ Cl |
| 3-119 | F | Cl | O | H | CF₂ Cl | CH₃ | CH₂ OCOCCl₃ |
| 3-120 | F | Cl | O | H | CF₂ Cl | CH₃ | CH₂ OCOCF₃ |
| 3-121 | F | Cl | O | H | CF₂ Cl | CH₃ | COOCH₃ |
| 3-122 | F | Cl | O | H | CF₂ Cl | CH₃ | COOC₂ H₅ |
| 3-123 | F | Cl | O | H | CF₂ Cl | CH₃ | COO ⁿ C₃H₇ |
| 3-124 | F | Cl | O | H | CF₂ Cl | CH₃ | COO ⁿC₄H₉ |
| 3-125 | F | Cl | O | H | CF₂ Cl | CH₃ | COO ⁿC₅H₁₁ |
| 3-126 | F | Cl | O | H | CF₂ Cl | CH₃ | COO ^{s}C₃ H₇ |
| 3-127 | F | Cl | O | H | CF₂ Cl | CH₃ | COCH₃ |
| 3-128 | F | Cl | O | H | CF₂ Cl | CH₃ | COC₂ H₅ |
| 3-129 | H | F | O | H | CF₃ | H | CH₃ |
| 3-130 | H | Cl | O | H | CF₃ | H | CH₃ |
| 3-131 | H | Br | O | H | CF₃ | H | CH₃ |
| 3-132 | F | F | O | H | CF₃ | H | CH₃ |
| 3-133 | F | Cl | O | H | CF₃ | H | CH₃ |
| 3-134 | F | Br | O | H | CF₃ | H | CH₃ |
| 3-135 | H | F | O | H | CF₃ | CH₃ | CH₃ |
| 3-136 | H | Cl | O | H | CF₃ | CH₃ | CH₃ |
| 3-137 | H | Br | O | H | CF₃ | CH₃ | CH₃ |
| 3-138 | F | F | O | H | CF₃ | CH₃ | CH₃ |
| 3-139 | F | Cl | O | H | CF₃ | CH₃ | CH₃ |
| 3-140 | F | Br | O | H | CF₃ | CH₃ | CH₃ |
| 3-141 | H | F | O | H | CF₃ | H | C₂ H₅ |
| 3-142 | H | Cl | O | H | CF₃ | H | C₂ H₅ |
| 3-143 | H | Br | O | H | CF₃ | H | C₂ H₅ |
| 3-144 | F | F | O | H | CF₃ | H | C₂ H₅ |
| 3-145 | F | Cl | O | H | CF₃ | H | C₂ H₅ |
| 3-146 | F | Br | O | H | CF₃ | H | C₂ H₅ |
| 3-147 | H | F | O | H | CF₃ | CH₃ | C₂H₅ |
| 3-148 | H | Cl | O | H | CF₃ | CH₃ | C₂H₅ |
| 3-149 | H | Br | O | H | CF₃ | CH₃ | C₂H₅ |
| 3-150 | F | F | O | H | CF₃ | CH₃ | C₂ H₅ |
| 3-151 | F | Cl | O | H | CF₃ | CH₃ | C₂ H₅ |
| 3-152 | F | Br | O | H | CF₃ | CH₃ | C₂ H ₅ |
| 3-153 | H | F | O | H | CF₃ | CH₃ | CH₂ Br |
| 3-154 | H | F | O | H | CF₃ | CH₃ | CH Br₂ |
| 3-155 | H | F | O | H | CF₃ | CH₃ | CBr ₃ |
| 3-156 | H | F | O | H | CF₃ | CH₃ | CHO |
| 3-157 | H | F | O | H | CF₃ | CH₃ | CN |
| 3-158 | H | F | O | H | CF₃ | CH₃ | COOH |
| 3-159 | H | F | O | H | CF₃ | CH₃ | CH₂ OH |
| 3-160 | H | F | O | H | CF₃ | CH₃ | CH₂ OCH₃ |
| 3-161 | H | F | O | H | CF₃ | CH₃ | CH₂ OC₂ H ₅ |
| 3-162 | H | F | O | H | CF₃ | CH₃ | CH₂ O ⁱ C₃ H ₇ |
| 3-163 | H | F | O | H | CF₃ | CH₃ | CH₂ OCH₂ OCH₃ |
| 3-164 | H | F | O | H | CF₃ | CH₃ | CH₂ OCH ₂ OC₂ H ₅ |
| 3-165 | H | F | O | H | CF₃ | CH₃ | CH₂ OCOCH₃ |
| 3-166 | H | F | O | | CF₃ | CH₃ | CH₂ OCOC₂ H ₅ |
| 3-167 | H | F | O | H | CF₃ | CH₃ | CH₂ OCO ⁱ C₃ H₇ |
| 3-168 | H | F | O | H | CF₃ | CH₃ | CH₂ OCOCH ₂ Cl |
| 3-169 | H | F | O | H | CF₃ | CH₃ | CH₂ OCOCCl₃ |
| 3-170 | H | F | O | H | CF₃ | CH₃ | CH₂ OCOCF ₃ |
| 3-171 | H | F | O | H | CF₃ | CH₃ | COOCH₃ |
| 3-172 | H | F | O | H | CF₃ | CH₃ | COOC₂ H₅ |
| 3-173 | H | F | O | H | CF₃ | CH₃ | COO ⁿ C ₃ H₇ |
| 3-174 | H | F | O | H | CF₃ | CH₃ | COO ⁿ C₄ H ₉ |
| 3-175 | H | F | O | H | CF₃ | CH₃ | COO ⁿ C ₅ H ₁₁ |
| 3-176 | H | F | O | H | CF₃ | CH₃ | COO C ₃ H ₇ |
| 3-177 | H | F | O | H | CF₃ | CH₃ | COCH₃ |
| 3-178 | H | F | O | H | CF₃ | CH₃ | COC₂ H ₅ |
| 3-179 | H | Cl | O | H | CF₃ | CH₃ | CH₂ Br |
| 3-180 | H | Cl | O | H | CF₃ | CH₃ | CH Br₂ |
| 3-181 | H | Cl | O | H | CF₃ | CH₃ | CBr₃ |
| 3-182 | H | Cl | O | H | CF₃ | CH₃ | CHO |
| 3-183 | H | Cl | O | H | CF₃ | CH₃ | CN |
| 3-184 | H | Cl | O | H | CF₃ | CH₃ | COOH |
| 3-185 | H | Cl | O | H | CF₃ | CH₃ | CH₂ OH |
| 3-186 | H | Cl | O | H | CF₃ | CH₃ | CH₂ OCH ₃ |
| 3-187 | H | Cl | O | H | CF₃ | CH₃ | CH₂ OC₂ H ₅ |
| 3-188 | H | Cl | O | H | CF₃ | CH₃ | CH₂ O ⁱ C ₃ H₇ |
| 3-189 | H | Cl | O | H | CF₃ | CH₃ | CH₂ OCH ₂ OCH ₃ |
| 3-190 | H | Cl | O | H | CF₃ | CH₃ | CH₂ OCH ₂ OC₂ H ₅ |
| 3-191 | H | Cl | O | H | CF₃ | CH₃ | CH₂ OCOCH ₃ |
| 3-192 | H | Cl | O | H | CF₃ | CH₃ | CH₂ OCOC₂ H ₅ |
| 3-193 | H | Cl | O | H | CF₃ | CH₃ | CH₂ OCO ⁱ C ₃ H ₇ |
| 3-194 | H | Cl | O | H | CF₃ | CH₃ | CH₂ OCOCH ₂ Cl |
| 3-195 | H | Cl | O | H | CF₃ | CH₃ | CH₂ OCOCCl₃ |
| 3-196 | H | Cl | O | H | CF₃ | CH₃ | CH₂ OCOCF ₃ |
| 3-197 | H | Cl | O | H | CF₃ | CH₃ | COOCH₃ |
| 3-198 | H | Cl | O | H | CF₃ | CH₃ | COOC₂ H₅ |
| 3-199 | H | Cl | O | H | CF₃ | CH₃ | COO ⁿ C₃ H₇ |
| 3-200 | H | Cl | O | H | CF₃ | CH₃ | COO ⁿ C₄ H₉ |
| 3-201 | H | Cl | O | H | CF₃ | CH₃ | COO ⁿ C₅ H₁₁ |
| 3-202 | H | Cl | O | H | CF₃ | CH₃ | COOⁱ C₃ H₇ |
| 3-203 | H | Cl | O | H | CF₃ | CH₃ | COCH₃ |
| 3-204 | H | Cl | O | H | CF₃ | CH₃ | COC₂ H₅ |
| 3-205 | F | F | O | H | CF₃ | CH₃ | CH₂ Br |
| 3-206 | F | F | O | H | CF₃ | CH₃ | CH Br₂ |
| 3-207 | F | F | O | H | CF₃ | CH₃ | CBr₃ |
| 3-208 | F | F | O | H | CF₃ | CH₃ | CHO |
| 3-209 | F | F | O | H | CF₃ | CH₃ | CN |
| 3-210 | F | F | O | H | CF₃ | CH₃ | COOH |
| 3-211 | F | F | O | H | CF₃ | CH₃ | CH₂ OH |
| 3-212 | F | F | O | H | CF₃ | CH₃ | CH₂ OCH ₃ |
| 3-213 | F | F | O | H | CF₃ | CH₃ | CH₂ OC₂ H ₆ |
| 3-214 | F | F | O | H | CF₃ | CH₃ | CH₂ Oⁱ C₃ H₇ |
| 3-215 | F | F | O | H | CF₃ | CH₃ | CH₂ OCH ₂ OCH ₃ |
| 3-216 | F | F | O | H | CF₃ | CH₃ | CH₂ OCH ₂ OC₂ H ₅ |
| 3-217 | F | F | O | H | CF₃ | CH₃ | CH₂ OCOCH ₃ |
| 3-218 | F | F | O | H | CF₃ | CH₃ | CH₂ OCOC₂ H₅ |
| 3-219 | F | F | O | H | CF₃ | CH₃ | CH₂ OCO ⁱ C₃ H₇ |
| 3-220 | F | F | O | H | CF₃ | CH₃ | CH₂ OCOCH₂Cl |
| 3-221 | F | F | O | H | CF₃ | CH₃ | CH₂ OCOCCl₃ |
| 3-222 | F | F | O | H | CF₃ | CH₃ | CH₂ OCOCF₃ |
| 3-223 | F | F | O | H | CF₃ | CH₃ | COOCH₃ |
| 3-224 | F | F | O | H | CF₃ | CH₃ | COOC₂ H₅ |
| 3-225 | F | F | O | H | CF₃ | CH₃ | COO ⁿ C₃ H₇ |
| 3-226 | F | F | O | H | CF₃ | CH₃ | COO ⁿ C₄ H₉ |
| 3-227 | F | F | O | H | CF₃ | CH₃ | COO ⁿC₅H₁₁ |
| 3-228 | F | F | O | H | CF₃ | CH₃ | COO ⁱ C₃ H₇ |
| 3-229 | F | F | O | H | CF₃ | CH₃ | COCH₃ |
| 3-230 | F | F | O | H | CF₃ | CH₃ | COC₂ H ₅ |
| 3-231 | F | Cl | O | H | CF₃ | CH₃ | CH₂ Br |
| 3-232 | F | Cl | O | H | CF₃ | CH₃ | CH Br₂ |
| 3-233 | F | Cl | O | H | CF₃ | CH₃ | CBr₃ |
| 3-234 | F | Cl | O | H | CF₃ | CH₃ | CHO |
| 3-235 | F | Cl | O | H | CF₃ | CH₃ | CN |
| 3-236 | F | Cl | O | H | CF₃ | CH₃ | COOH |
| 3-237 | F | Cl | O | H | CF₃ | CH₃ | CH₂ OH |
| 3-238 | F | Cl | O | H | CF₃ | CH₃ | CH₂ OCH ₃ |
| 3-239 | F | Cl | O | H | CF₃ | CH₃ | CH₂ OC₂ H₅ |
| 3-240 | F | Cl | O | H | CF₃ | CH₃ | CH₂ O ⁱ C ₃ H ₇ |
| 3-241 | F | Cl | O | H | CF₃ | CH₃ | CH₂ OCH ₂ OCH ₃ |
| 3-242 | F | Cl | O | H | CF₃ | CH₃ | CH₂ OCH ₂ OC₂ H ₅ |
| 3-243 | F | Cl | O | H | CF₃ | CH₃ | CH₂ OCOCH ₃ |
| 3-244 | F | Cl | O | H | CF₃ | CH₃ | CH₂ OCOC₂ H s |
| 3-245 | F | Cl | O | H | CF₃ | CH₃ | CH₂ OCO ⁱ C₃ H ₇ |
| 3-246 | F | Cl | O | H | CF₃ | CH₃ | CH₂ OCOCH₂ Cl |
| 3-247 | F | Cl | O | H | CF₃ | CH₃ | CH₂ OCOCCl₃ |
| 3-248 | F | Cl | O | H | CF₃ | CH₃ | CH₂ OCOCF ₃ |
| 3-249 | F | Cl | O | H | CF ₃ | CH₃ | COOCH₃ |
| 3-250 | F | Cl | O | H | CF₃ | CH₃ | COOC₂ H s |
| 3-251 | F | Cl | O | H | CF₃ | CH₃ | COO ⁿ C ₃ H ₇ |
| 3-252 | F | Cl | O | H | CF₃ | CH₃ | COO ⁿ C ₄ H ₉ |
| 3-253 | F | Cl | O | H | CF₃ | CH₃ | COO ⁿ C ₅ H₁₁ |
| 3-254 | F | Cl | O | H | CF₃ | CH₃ | COO ⁱ C₃ H ₇ |
| 3-255 | F | Cl | O | H | CF₃ | CH₃ | COCH₃ |
| 3-256 | F | Cl | O | H | CF₃ | CH₃ | COC₂ H ₅ |
| 3-257 | H | F | O | H | CF₂ Cl | H | CH₂ Br |
| 3-258 | H | F | O | H | CF₂ Cl | H | CH Br₂ |
| 3-259 | H | F | O | H | CF₂ Cl | H | CBr ₃ |
| 3-260 | H | F | O | H | CF₂ Cl | H | CHO |
| 3-261 | H | F | O | H | CF₂ Cl | H | CN |
| 3-262 | H | F | O | H | CF₂ Cl | H | COOH |
| 3-263 | H | F | O | H | CF₂ Cl | H | CH₂ OH |
| 3-264 | H | F | O | H | CF₂ Cl | H | CH₂ OCH₃ |
| 3-265 | H | F | O | H | CF₂ Cl | H | CH₂ OC₂ H₅ |
| 3-266 | H | F | O | H | CF₂ Cl | H | CH₂ O ⁱ C ₃ H₇ |
| 3-267 | H | F | O | H | CF₂ Cl | H | CH₂ OCH₂ OCH₃ |
| 3-268 | H | F | O | H | CF₂ Cl | H | CH₂ OCH ₂ OC₂ H ₅ |
| 3-269 | H | F | O | H | CF₂ Cl | H | CH₂ OCOCH₃ |
| 3-270 | H | F | O | H | CF₂ Cl | H | CH₂ OCOC₂ H ₅ |
| 3-271 | H | F | O | H | CF₂ Cl | H | CH₂ OCO ⁱ C ₃ H ₇ |
| 3-272 | H | F | O | H | CF₂ Cl | H | CH₂ OCOCH ₂ Cl |
| 3-273 | H | F | O | H | CF₂ Cl | H | CH₂ OCOCCl₃ |
| 3-274 | H | F | O | H | CF₂ Cl | H | CH₂ OCOCF₃ |
| 3-275 | H | F | O | H | CF₂ Cl | H | COOCH₃ |
| 3-276 | H | F | O | H | CF₂ Cl | H | COOC₂ H ₅ |
| 3-277 | H | F | O | H | CF₂ Cl | H | COO ⁿ C ₃ H ₇ |
| 3-278 | H | F | O | H | CF₂ Cl | H | COO ⁿ C ₄ H ₉ |
| 3-279 | H | F | O | H | CF₂ Cl | H | COO ⁿ C ₅ H ₁₁ |
| 3-280 | H | F | O | H | CF₂ Cl | H | COOⁱ C ₃ H ₇ |
| 3-281 | H | F | O | H | CF₂ Cl | H | COCH₃ |
| 3-282 | H | F | O | H | CF₂ Cl | H | COC₂ H |
| 3-283 | H | Cl | O | H | CF₂ Cl | H | CH₂ Br |
| 3-284 | H | Cl | O | H | CF₂ Cl | H | CH Br₂ |
| 3-285 | H | Cl | O | H | CF₂ Cl | H | CBr₃ |
| 3-286 | H | Cl | O | H | CF₂ Cl | H | CHO |
| 3-287 | H | Cl | O | H | CF₂ Cl | H | CN |
| 3-288 | H | Cl | O | H | CF₂ Cl | H | COOH |
| 3-289 | H | Cl | O | H | CF₂ Cl | H | CH₂ OH |
| 3-290 | H | Cl | O | H | CF₂ Cl | H | CH₂ OCH₃ |
| 3-291 | H | Cl | O | H | CF₂ Cl | H | CH₂ OC₂ H ₅ |
| 3-292 | H | Cl | O | H | CF₂ Cl | H | CH₂ 0 C₃H₇ |
| 3-293 | H | Cl | O | H | CF₂ Cl | H | CH₂ OCH₂ OCH₃ |
| 3-294 | H | Cl | O | H | CF₂ Cl | H | CH₂ OCH ₂ OC₂ H |
| 3-295 | H | Cl | O | H | CF₂ Cl | H | CH₂ OCOCH₃ |
| 3-296 | H | Cl | O | H | CF₂ Cl | H | CH₂ OCOC₂ H |
| 3-297 | H | Cl | O | H | CF₂ Cl | H | CH₂ OCO ⁱ C₃H₇ |
| 3-298 | H | Cl | O | H | CF₂ Cl | H | CH₂ OCOCH₂Cl |
| 3-299 | H | Cl | O | H | CF₂ Cl | H | CH₂ OCOCCl₃ |
| 3-300 | H | Cl | O | H | CF₂ Cl | H | CH₂ OCOCF₃ |
| 3-301 | H | Cl | O | H | CF₂ Cl | H | COOCH₃ |
| 3-302 | H | Cl | O | H | CF₂ Cl | H | COOC₂ H ₅ |
| 3-303 | H | Cl | O | H | CF₂ Cl | H | COOⁿ C₃ H₇ |
| 3-304 | H | Cl | O | H | CF₂ Cl | H | COO ⁿ C₄ H₉ |
| 3-305 | H | Cl | O | H | CF₂ Cl | H | COO ⁿ C ₅ H₁₁ |
| 3-306 | H | Cl | O | H | CF₂ Cl | H | COOⁱ C₃ H₇ |
| 3-307 | H | Cl | O | H | CF₂ Cl | H | COCH₃ |
| 3-308 | H | Cl | O | H | CF₂ Cl | H | COC₂ H ₅ |
| 3-309 | F | F | O | H | CF₂ Cl | H | CH₂ Br |
| 3-310 | F | F | O | H | CF₂ Cl | H | CH Br₂ |
| 3-311 | F | F | O | H | CF₂ Cl | H | CBr₃ |
| 3-312 | F | F | O | H | CF₂ Cl | H | CHO |
| 3-313 | F | F | O | H | CF₂ Cl | H | CN |
| 3-314 | F | F | O | H | CF₂ Cl | H | COOH |
| 3-315 | F | F | O | H | CF₂ Cl | H | CH₂ OH |
| 3-316 | F | F | O | H | CF₂ Cl | H | CH₂ OCH ₃ |
| 3-317 | F | F | O | H | CF₂ Cl | H | CH₂ OC₂ H ₅ |
| 3-318 | F | F | O | H | CF₂ Cl | H | CH₂ O ⁱ C ₃ H ₇ |
| 3-319 | F | F | O | H | CF₂ Cl | H | CH₂ OCH ₂ OCH ₃ |
| 3-320 | F | F | O | H | CF₂ Cl | H | CH₂ OCH ₂ OC ₂ H ₅ |
| 3-321 | F | F | O | H | CF₂ Cl | H | CH₂ OCOCH ₃ |
| 3-322 | F | F | O | H | CF₂ Cl | H | CH₂ OCOC₂ H ₅ |
| 3-323 | F | F | O | H | CF₂ Cl | H | CH₂ OCO ⁱ C₃ H ₇ |
| 3-324 | F | F | O | H | CF₂ Cl | H | CH₂ OCOCH ₂ Cl |
| 3-325 | F | F | O | H | CF₂ Cl | H | CH₂ OCOCCl₃ |
| 3-326 | F | F | O | H | CF₂ Cl | H | CH₂ OCOCF ₃ |
| 3-327 | F | F | O | H | CF₂ Cl | H | COOCH₃ |
| 3-328 | F | F | O | H | CF₂ Cl | H | COOC₂ H ₅ |
| 3-329 | F | F | O | H | CF₂ Cl | H | COO ⁿ C₃ H ₇ |
| 3-330 | F | F | O | H | CF₂ Cl | H | COO ⁿ C ₄ H ₉ |
| 3-331 | F | F | O | H | CF₂ Cl | H | COO ⁿ C ₅ H₁₁ |
| 3-332 | F | F | O | H | CF₂ Cl | H | COO ⁱ C₃ H₇ |
| 3-333 | F | F | O | H | CF₂ Cl | H | COCH₃ |
| 3-334 | F | F | 0 | H | CF₂ Cl | H | COC₂ H ₅ |
| 3-335 | F | Cl | O . | H | CF₂ Cl | H | CH₂ Br |
| 3-336 | F | Cl | O | H | CF₂ Cl | H | CH Br₂ |
| 3-337 | F | Cl | O | H | CF₂ Cl | H | CBr₃ |
| 3-338 | F | Cl | O | H | CF₂ Cl | H | CHO |
| 3-339 | F | Cl | O | H | CF₂ Cl | H | CN |
| 3-340 | F | Cl | O | H | CF₂ Cl | H | COOH |
| 3-341 | F | Cl | O | H | CF₂ Cl 1 | H | CH₂ OH |
| 3-342 | F | Cl | O | H | CF₂ Cl | H | CH₂ OCH ₃ |
| 3-343 | F | Cl | O | H | CF₂ Cl | H | CH₂ OC₂ H ₅ |
| 3-344 | F | Cl | O | H | CF₂ Cl | H | CH₂ O ⁱ C₃ H₇ |
| 3-345 | F | Cl | O | H | CF₂ Cl | H | CH₂ OCH ₂ OCH ₃ |
| 3-346 | F | Cl | O | H | CF₂ Cl | H | CH₂ OCH ₂ OC₂ H ₅ |
| 3-347 | F | Cl | O | H | CF₂ Cl | H | CH₂ OCOCH₃ |
| 3-348 | F | Cl | O | H | CF₂ Cl | H | CH₂ OCOC₂ H ₅ |
| 3-349 | F | Cl | O | H | CF₂ Cl | H | CH₂ OCO ⁱ C₃ H ₇ |
| 3-350 | F | Cl | O | H | CF₂ Cl | H | CH₂ OCOCH ₂ Cl |
| 3-351 | F | Cl | O | H | CF₂ Cl | H | CH₂ OCOCCl₃ |
| 3-352 | F | Cl | O | H | CF₂ Cl | H | CH₂ OCOCF₃ |
| 3-353 | F | Cl | O | H | CF₂ Cl | H | COOCH ₃ |
| 3-354 | F | Cl | O | H | CF₂ Cl | H | COOC₂ H ₅ |
| 3-355 | F | Cl | O | H | CF₂ Cl | H | COO ⁿ C ₃ H ₇ |
| 3-356 | F | Cl | O | H | CF₂ Cl | H | COOⁿ C₄ H₉ |
| 3-357 | F | Cl | O | H | CF₂ Cl | H | COO ⁿ C ₅ H₁₁ |
| 3-358 | F | Cl | O | H | CF₂ Cl | H | COO ⁱ C₃ H₇ |
| 3-359 | F | Cl | O | H | CF₂ Cl | H | COCH₃ |
| 3-360 | F | Cl | O | H | CF₂ Cl | H | COC₂ H₅ |
| 3-361 | F | Br | O | H | CF₃ | H | C₂ H ₅ |
| 3-362 | H | F | O | H | CF₃ | H | CH₂ Br |
| 3-363 | H | F | O | H | CF₃ | H | CH Br₂ |
| 3-364 | H | F | O | H | CF₃ | H | CBr₃ |
| 3-365 | H | F | O | H | CF₃ | H | CHO |
| 3-366 | H | F | O | H | CF₃ | H | CN |
| 3-367 | H | F | O | H | CF₃ | H | COOH |
| 3-368 | H | F | O | H | CF₃ | H | CH₂ OH |
| 3-369 | H | F | O | H | CF₃ | H | CH₂ OCH₃ |
| 3-370 | H | F | O | H | CF₃ | H | CH₂ OC₂ H₅ |
| 3-371 | H | F | O | H | CF₃ | H | CH₂ O ⁱ C₃ H₇ |
| 3-372 | H | F | O | H | CF₃ | H | CH₂ OCH ₂ OCH₃ |
| 3-373 | H | F | O | H | CF₃ | H | CH₂ OCH ₂ OC₂ H₅ |
| 3-374 | H | F | O | H | CF₃ | H | CH₂ OCOCH₃ |
| 3-375 | H | F | O | H | CF₃ | H | CH₂ OCOC₂ H₅ |
| 3-376 | H | F | O | H | CF₃ | H | CH₂ OCO ⁱ C₃ H₇ |
| 3-377 | H | F | O | H | CF₃ | H | CH₂ OCOCH₂Cl |
| 3-378 | H | F | O | H | CF₃ | H | CH₂ OCOCCl₃ |
| 3-379 | H | F | O | H | CF₃ | H | CH₂ OCOCF ₃ |
| 3-380 | H | F | O | H | CF₃ | H | COOCH₃ |
| 3-381 | H | F | O | H | CF₃ | H | COOC₂ H₅ |
| 3-382 | H | F | O | H | CF₃ | H | COO ⁿ C ₃ H ₇ |
| 3-383 | H | F | O | H | CF₃ | H | COO ⁿ C ₄ H ₉ |
| 3-384 | H | F | O | H | CF₃ | H | COO ⁿ C ₅ H ₁₁ |
| 3-385 | H | F | O | H | CF₃ | H | COO ⁱ C ₃ H ₇ |
| 3-386 | H | F | O | H | CF₃ | H | COCH₃ |
| 3-387 | H | F | O | H | CF₃ | H | COC₂ H ₅ |
| 3-388 | H | Cl | O | H | CF₃ | H | CH₂ Br |
| 3-389 | H | Cl | O | H | CF₃ | H | CH Br₂ |
| 3-390 | H | Cl | O | H | CF₃ | H | CBr₃ |
| 3-391 | H | Cl | O | H | CF₃ | H | CHO |
| 3-392 | H | Cl | O | H | CF₃ | H | CN |
| 3-393 | H | Cl | O | H | CF₃ | H | COOH |
| 3-394 | H | Cl | O | H | CF₃ | H | CH₂ OH |
| 3-395 | H | Cl | O | H | CF₃ | H | CH₂ OCH₃ |
| 3-396 | H | Cl | O | H | CF₃ | H | CH₂ OC₂ H₅ |
| 3-397 | H | Cl | O | H | CF₃ | H | CH₂ O ⁱ C₃ H₇ |
| 3-398 | H | Cl | O | H | CF₃ | H | CH₂ OCH₂ OCH₃ |
| 3-399 | H | Cl | O | H | CF₃ | H | CH₂ OCH₂ OC₂ H₅ |
| 3-400 | H | Cl | O | H | CF₃ | H | CH₂ OCOCH ₃ |
| 3-401 | H | Cl | O | H | CF₃ | H | CH₂ OCOC₂ H ₅ |
| 3-402 | H | Cl | O | H | CF₃ | H | CH₂ OCO ⁱ C₃ H ₇ |
| 3-403 | H | Cl | O | H | CF₃ | H | CH₂ OCOCH ₂ Cl |
| 3-404 | H | Cl | O | H | CF₃ | H | CH₂ OCOCCl₃ |
| 3-405 | H | Cl | O | H | CF₃ | H | CH₂ OCOCF ₃ |
| 3-406 | H | Cl | O | H | CF₃ | H | COOCH₃ |
| 3-407 | H | Cl | O | H | CF₃ | H | COOC₂ H ₅ |
| 3-408 | H | Cl | O | H | CF₃ | H | COO ⁿ C ₃ H ₇ |
| 3-409 | H | Cl | O | H | CF₃ | H | COO ⁿ C ₄ H ₉ |
| 3-410 | H | Cl | O | H | CF₃ | H | COO ⁿ C ₅ H ₁₁ |
| 3-411 | H | Cl | O | H | CF₃ | H | COO ⁱ C ₃ H ₇ |
| 3-412 | H | Cl | O | H | CF₃ | H | COCH₃ |
| 3-413 | H | Cl | O | H | CF₃ | H | COC₂ H ₅ |
| 3-414 | F | F | O | H | CF₃ | H | CH₂ Br |
| 3-415 | F | F | O | H | CF₃ | H | CH Br₂ |
| 3-416 | F | F | O | H | CF₃ | H | CBr₃ |
| 3-417 | F | F | O | H | CF₃ | H | CHO |
| 3-418 | F | F | O | H | CF₃ | H | CN |
| 3-419 | F | F | O | H | CF₃ | H | COOH |
| 3-420 | F | F | O | H | CF₃ | H | CH₂ OH |
| 3-421 | F | F | O | H | CF₃ | H | CH₂ OCH ₃ |
| 3-422 | F | F | O | H | CF₃ | H | CH₂ OC₂ H ₅ |
| 3-423 | F | F | O | H | CF₃ | H | CH₂ O ⁱ C ₃ H ₇ |
| 3-424 | F | F | O | H | CF₃ | H | CH₂ OCH ₂ OCH ₃ |
| 3-425 | F | F | O | H | CF₃ | H | CH₂ OCH ₂ OC₂ H ₅ |
| 3-426 | F | F | O | H | CF₃ | H | CH₂ OCOCH ₃ |
| 3-427 | F | F | O | H | CF₃ | H | CH₂ OCOC₂ H₅ |
| 3-428 | F | F | O | H | CF₃ | H | CH₂ OCO ⁱ C₃ H ₇ |
| 3-429 | F | F | O | H | CF₃ | H | CH₂ OCOCH₂ Cl |
| 3-430 | F | F | O | H | CF₃ | H | CH₂ OCOCCl₃ |
| 3-431 | F | F | O | H | CF₃ | H | CH₂ OCOCF₃ |
| 3-432 | F | F | O | H | CF₃ | H | COOCH₃ |
| 3-433 | F | F | O | H | CF₃ | H | COOC₂ H₅ |
| 3-434 | F | F | O | H | CF₃ | H | COO ⁿ C ₃ H ₇ |
| 3-435 | F | F | O | H | CF₃ | H | COO ⁿ C₄ H ₉ |
| 3-436 | F | F | O | H | CF₃ | H | COO ⁿ C ₅ H₁₁ |
| 3-437 | F | F | O | H | CF₃ | H | COOⁱ C₃ H₇ |
| 3-438 | F | F | O | H | CF₃ | H | COCH₃ |
| 3-439 | F | F | O | H | CF₃ | H | COC₂ H ₅ |
| 3-440 | F | Cl | O | H | CF₃ | H | CH₂ Br |
| 3-441 | F | Cl | O | H | CF₃ | H | CH Br₂ |
| 3-442 | F | Cl | O | H | CF₃ | H | CBr₃ |
| 3-443 | F | Cl | O | H | CF₃ | H | CHO |
| 3-444 | F | Cl | O | H | CF₃ | H | CN |
| 3-445 | F | Cl | O | H | CF₃ | H | COOH |
| 3-446 | F | Cl | O | H | CF₃ | H | CH₂ OH |
| 3-447 | F | Cl | O | H | CF₃ | H | CH₂ OCH₃ |
| 3-448 | F | Cl | O | H | CF₃ | H | CH₂ OC₂ H ₅ |
| 3-449 | F | Cl | O | H | CF₃ | H | CH₂ O ⁱ C₃ H₇ |
| 3-450 | F | Cl | O | H | CF₃ | H | CH₂ OCH₂ OCH₃ |
| 3-451 | F | Cl | O | H | CF₃ | H | CH₂ OCH₂ OC₂ H₅ |
| 3-452 | F | Cl | O | H | CF₃ | H | CH₂ OCOCH ₃ |
| 3-453 | F | Cl | O | H | CF₃ | H | CH₂ OCOC₂ H ₅ |
| 3-454 | F | Cl | O | H | CF₃ | H | CH₂ OCO ⁱ C₃ H ₇ |
| 3-455 | F | Cl | O | H | CF₃ | H | CH₂ OCOCH ₂ Cl |
| 3-456 | F | Cl | O | H | CF₃ | H | CH₂ OCOCCl₃ |
| 3-457 | F | Cl | O | H | CF₃ | H | CH₂ OCOCF ₃ |
| 3-458 | F | Cl | O | H | CF₃ | H | COOCH₃ |
| 3-459 | F | Cl | O | H | CF₃ | H | COOC₂ H |
| 3- 460 | F | Cl | O | H | CF₃ | H | COO ⁿ C₃ H ₇ |
| 3-461 | F | Cl | O | H | CF₃ | H | COO ⁿ C ₄ H ₉ |
| 3-462 | F | Cl | O | H | CF₃ | H | COOⁿ C ₅ H₁₁ |
| 3-463 | F | Cl | O | H | CF₃ | H | COO ⁱC ₃ H ₇ |
| 3-464 | F | Cl | O | H | CF₃ | H | COCH₃ |
| 3-465 | F | Cl | O | H | CF₃ | H | COC₂ H ₅ |
| 3-466 | H | F | O | CH₃ | CF₃ | H | CH₃ |
| 3-467 | H | Cl | O | CH₃ | CF₃ | H | CH₃ |
| 3-468 | H | Br | O | CH₃ | CF₃ | H | CH₃ |
| 3-469 | F | F | O | CH₃ | CF₃ | H | CH₃ |
| 3-470 | F | Cl | O | CH₃ | CF₃ | H | CH₃ |
| 3-471 | F | Br | O | CH₃ | CF₃ | H | CH₃ |
| 3-472 | H | F | O | CH₃ | CF₃ | H | C₂ H₅ |
| 3-473 | H | Cl | O | CH₃ | CF₃ | H | C₂ H |
| 3-474 | H | Br | O | CH₃ | CF₃ | H | C₂ H₅ |
| 3-475 | F | F | O | CH₃ | CF₃ | H | C₂ H ₅ |
| 3-476 | F | Cl | O | CH₃ | CF₃ | H | C₂ H₅ |
| 3-477 | F | Br | O | CH₃ | CF₃ | H | C₂ H ₅ |
| 3-478 | F | Br | O | CH₃ | CF₃ | H | C₂ H₅ |
| 3-479 | H | F | O | CH₃ | CF₃ | H | CH₂ Br |
| 3-480 | H | F | O | CH₃ | CF₃ | H | CH Br₂ |
| 3-481 | H | F | O | CH₃ | CF₃ | H | CBr₃ |
| 3-482 | H | F | O | CH₃ | CF₃ | H | CHO |
| 3-483 | H | F | O | CH₃ | CF₃ | H | CN |
| 3-484 | H | F | O | CH₃ | CF₃ | H | COOH |
| 3-485 | H | F | O | CH₃ | CF₃ | H | CH₂ OH |
| 3-486 | H | F | O | CH₃ | CF₃ | H | CH₂ OCH ₃ |
| 3-487 | H | F | O | CH₃ | CF₃ | H | CH₂ OC₂ H ₅ |
| 3-488 | H | F | O | CH₃ | CF₃ | H | CH₂ O ⁱ C ₃ H ₇ |
| 3-489 | H | F | O | CH₃ | CF₃ | H | CH₂ OCH ₂ OCH ₃ |
| 3-490 | H | F | O | CH₃ | CF₃ | H | CH₂ OCH ₂ OC₂ H ₆ |
| 3-491 | H | F | O | CH₃ | CF₃ | H | CH₂ OCOCH ₃ |
| 3-492 | H | F | O | CH₃ | CF₃ | H | CH₂ OCOC₂ H ₆ |
| 3-493 | H | F | O | CH₃ | CF₃ | H | CH₂ OCO ⁱ C ₃ H₇ |
| 3-494 | H | F | O | CH₃ | CF₃ | H | CH₂ OCOCH ₂ Cl |
| 3-495 | H | F | O | CH₃ | CF₃ | H | CH₂ OCOCCl₃ |
| 3-496 | H | F | O | CH₃ | CF₃ | H | CH₂ OCOCF ₃ |
| 3-497 | H | F | O | CH₃ | CF₃ | H | COOCH₃ |
| 3-498 | H | F | O | CH₃ | CF₃ | H | COOC₂ H ₅ |
| 3-499 | H | F | O | CH₃ | CF₃ | H | COO ⁿ C₃ H₇ |
| 3-500 | H | F | O | CH₃ | CF₃ | H | COO ⁿ C₄ H ₉ |
| 3-501 | H | F | O | CH₃ | CF₃ | H | COO ⁿ C ₅ H ₁₁ |
| 3-502 | H | F | O | CH₃ | CF₃ | H | COO ⁱ C₃ H ₇ |
| 3-503 | H | F | O | CH₃ | CF₃ | H | COCH₃ |
| 3-504 | H | F | O | CH₃ | CF₃ | H | COC₂ H₅ |
| 3-505 | H | Cl | O | CH₃ | CF₃ | H | CH₂ Br |
| 3-506 | H | Cl | O | CH₃ | CF₃ | H | CH Br₂ |
| 3-507 | H | Cl | O | CH₃ | CF₃ | H | CBr₃ |
| 3-508 | H | Cl | O | CH₃ | CF₃ | H | CHO |
| 3-509 | H | Cl | O | CH₃ | CF₃ | H | CN |
| 3-510 | H | Cl | O | CH₃ | CF₃ | H | COOH |
| 3-511 | H | Cl | O | CH₃ | CF₃ | H | CH₂ OH |
| 3-512 | H | Cl | O | CH₃ | CF₃ | H | CH₂ OCH₃ |
| 3-513 | H | Cl | O | CH₃ | CF₃ | H | CH₂ OC₂ H ₅ |
| 3-514 | H | Cl | O | CH₃ | CF₃ | H | CH₂ O ⁱ C₃ H ₇ |
| 3-515 | H | Cl | O | CH₃ | CF₃ | H | CH₂ OCH ₂ OCH ₃ |
| 3-516 | H | Cl | O | CH₃ | CF₃ | H | CH₂ OCH ₂ OC₂ H₅ |
| 3-517 | H | Cl | O | CH₃ | CF₃ | H | CH₂ OCOCH ₃ |
| 3-518 | H | Cl | O | CH₃ | CF₃ | H | CH₂ OCOC₂ H ₅ |
| 3-519 | H | Cl | O | CH₃ | CF₃ | H | CH₂ OCO C₃ H ₇ |
| 3-520 | H | Cl | O | CH₃ | CF₃ | H | CH₂ OCOCH ₂Cl |
| 3-521 | H | Cl | O | CH₃ | CF₃ | H | CH₂ OCOCCl₃ |
| 3-522 | H | Cl | O | CH₃ | CF₃ | H | CH₂ OCOCF₃ |
| 3-523 | H | Cl | O | CH₃ | CF₃ | H | COOCH₃ |
| 3-524 | H | Cl | O | CH₃ | CF₃ | H | COOC₂ H ₅ |
| 3-525 | H | Cl | O | CH₃ | CF₃ | H | COO ⁿ C₃ H₇ |
| 3-526 | H | Cl | O | CH₃ | CF₃ | H | COO ⁿC₄ H₉ |
| 3-527 | H | Cl | O | CH₃ | CF₃ | H | COO ⁿ C ₅ H ₁₁ |
| 3-528 | H | Cl | O | CH₃ | CF₃ | H | COO ⁱ C ₃H ₇ |
| 3-529 | H | Cl | O | CH₃ | CF₃ | H | COCH₃ |
| 3-530 | H | Cl | O | CH₃ | CF₃ | H | COC₂ H ₅ |
| 3-531 | F | F | O | CH₃ | CF₃ | H | CH₂ Br |
| 3-532 | F | F | O | CH₃ | CF₃ | H | CH Br₂ |
| 3-533 | F | F | O | CH₃ | CF₃ | H | CBr₃ |
| 3-534 | F | F | O | CH₃ | CF₃ | H | CHO |
| 3-535 | F | F | O | CH₃ | CF₃ | H | CN |
| 3-536 | F | F | O | CH₃ | CF₃ | H | COOH |
| 3-537 | F | F | O | CH₃ | CF₃ | H | CH₂ OH |
| 3-538 | F | F | O | CH₃ | CF₃ | H | CH₂ OCH ₃ |
| 3-539 | F | F | O | CH₃ | CF₃ | H | CH₂ OC₂ H ₅ |
| 3-540 | F | F | O | CH₃ | CF₃ | H | CH₂ O ⁱ C₃ H₇ |
| 3-541 | F | F | O | CH₃ | CF₃ | H | CH₂ OCH ₂OCH ₃ |
| 3-542 | F | F | O | CH₃ | CF₃ | H | CH₂ OCH ₂ OC₂ H ₅ |
| 3-543 | F | F | O | CH₃ | CF₃ | H | CH₂ OCOCH ₃ |
| 3-544 | F | F | O | CH₃ | CF₃ | H | CH₂ OCOC₂ H ₅ |
| 3-545 | F | F | O | CH₃ | CF₃ | H | CH₂ OCO C ₃ H ₇ |
| 3-546 | F | F | O | CH₃ | CF₃ | H | CH₂ OCOCH ₂ Cl |
| 3-547 | F | F | O | CH₃ | CF₃ | H | CH₂ OCOCCl₃ |
| 3-548 | F | F | O | CH₃ | CF₃ | H | CH₂ OCOCF ₃ |
| 3-549 | F | F | O | CH₃ | CF₃ | H | COOCH₃ |
| 3-550 | F | F | O | CH₃ | CF₃ | H | COOC₂ H ₅ |
| 3-551 | F | F | O | CH₃ | CF₃ | H | COO ⁿ C ₃ H ₇ |
| 3-552 | F | F | O | CH₃ | CF₃ | H | COO ⁿ C₄ H ₉ |
| 3-553 | F | F | O | CH₃ | CF₃ | H | COO ⁿ C ₅H₁₁ |
| 3-554 | F | F | O | CH₃ | CF₃ | H | COO ⁱ C ₃ H ₇ |
| 3-555 | F | F | O | CH₃ | CF₃ | H | COCH₃ |
| 3-556 | F | F | O | CH₃ | CF₃ | H | COC₂ H ₅ |
| 3-557 | F | Cl | O | H₃ | CF₃ | H | CH₂ Br |
| 3-558 | F | Cl | O | CH₃ | CF₃ | H | CH Br₂ |
| 3-559 | F | Cl | O | CH₃ | CF₃ | H | CBr₃ |
| 3-560 | F | Cl | O | CH₃ | CF₃ | H | CHO |
| 3-561 | F | Cl | O | CH₃ | CF₃ | H | CN |
| 3-562 | F | Cl | O | CH₃ | CF₃ | H | COOH |
| 3-563 | F | Cl | O | CH₃ | CF₃ | H | CH₂ OH |
| 3-564 | F | Cl | O | CH₃ | CF₃ | H | CH₂ OCH₃ |
| 3-565 | F | Cl | O | CH₃ | CF₃ | H | CH₂ OC₂ H₅ |
| 3-566 | F | Cl | O | CH₃ | CF₃ | H | CH₂ O ⁱ C₃ H₇ |
| 3-567 | F | Cl | O | CH₃ | CF₃ | H | CH₂ OCH₂ OCH₃ |
| 3-568 | F | Cl | O | CH₃ | CF₃ | H | CH₂ OCH₂ OC₂ H₅ |
| 3-569 | F | Cl | O | CH₃ | CF₃ | H | CH₂ OCOCH₃ |
| 3-570 | F | Cl | O | CH₃ | CF₃ | H | CH₂ OCOC₂ H₅ |
| 3-571 | F | Cl | O | CH₃ | CF₃ | H | CH₂ OCO ⁱ C₃ H₇ |
| 3-572 | F | Cl | O | CH₃ | CF₃ | H | CH₂ OCOCH ₂ Cl |
| 3-573 | F | Cl | O | CH₃ | CF₃ | H | CH₂ OCOCCl₃ |
| 3-574 | F | Cl | O | CH₃ | CF₃ | H | CH₂ OCOCF ₃ |
| 3-575 | F | Cl | O | CH₃ | CF₃ | H | COOCH₃ |
| 3-576 | F | Cl | O | CH₃ | CF₃ | H | COOC₂ H ₆ |
| 3-577 | F | Cl | O | CH₃ | CF₃ | H | COO ⁿ C ₃ H₇ |
| 3-578 | F | Cl | O | CH₃ | CF₃ | H | COO ⁿ C₄ H ₉ |
| 3-579 | F | Cl x | O | CH₃ | CF₃ | H | COO ⁿ C ₅ H₁₁ |
| 3-580 | F | Cl | O | CH₃ | CF₃ | H | COO ⁱ C₃ H₇ |
| 3-581 | F | Cl | O | CH₃ | CF₃ | H | COCH₃ |
| 3-582 | F | Cl | O | CH₃ | CF₃ | H | COC₂ H ₅ |

**TABLE 4**

| | | | | | | | |
|---|---|---|---|---|---|---|---|
| Compounds of the formula: | | | | | | | |
| 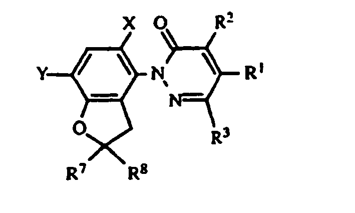 | | | | | | | |

| Compound X No. | | Y R³ R¹ | | | R² | R⁷ | R⁸ |
|---|---|---|---|---|---|---|---|
| 4-1 | H | Cl | H | CF₂ Cl | CH₃ | H | CH₂ Cl |
| 4-2 | H | Cl | H | CF₂ Cl | CH₃ | H | CH₂ Br |
| 4-3 | H | Cl | H | CF₂ Cl | CH₃ | H | CH₂ OCH ₃ |
| 4-4 | H | Cl | H | CF₂ Cl | CH₃ | H | CH₂ OC₂ H ₅ |
| 4-5 | H | Cl | H | CF₂ Cl | CH₃ | H | CH₂ OCH₂ OCH ₃ |
| 4-6 | H | Cl | H | CF₂ Cl | CH₃ | H | CH₂ OCH ₂ OC ₂ H₅ |
| 4-7 | H | Cl | H | CF₂ Cl | CH₃ | H | CH₂ OCOCH₃ |
| 4-8 | H | Cl | H | CF₂ Cl | CH₃ | H | CH₂ OCOC ₂ H₅ |
| 4-9 | H | Cl | H | CF₂ Cl | CH₃ | H | CH₂ OCO ⁱ C ₃ H₇ |
| 4-10 | H | Cl | H | CF₂ Cl | CH₃ | H | CH₂ OCOCH₂ Cl |
| 4-11 | H | Cl | H | CF₂ Cl | CH₃ | H | CH₂ OCOCCl₃ |
| 4-12 | H | Cl | H | CF₂ Cl | CH₃ | H | CH₂ OCOCF₃ |
| 4-13 | H | Cl | H | CF₂ Cl | CH₃ | H | COOH |
| 4-14 | H | Cl | H | CF₂ Cl | CH₃ | H | COOCH₃ |
| 4-15 | H | Cl | H | CF₂ Cl | CH₃ | H | COOC₂ H ₅ |
| 4-16 | H | Cl | H | CF₂ Cl | CH₃ | H | COO ⁿ C₃ H ₇ |
| 4-17 | H | Cl | H | CF₂ Cl | CH₃ | H | COO ⁿ C ₄H₉ |
| 4-18 | H | Cl | H | CF₂ Cl | CH₃ | H | COO ⁿ C ₅ H₁₁ |
| 4-19 | H | Cl | H | CF₂ Cl | CH₃ | H | COO ⁱ C₃ H₇ |
| 4-20 | H | Cl | H | CF₂ Cl | CH₃ | H | COO ^{c} C₅ H₉ |
| 4-21 | H | Cl | H | CF₂ Cl | CH₃ | H | COO ^{c} C ₆ H₁₁ |
| 4-22 | H | Cl | H | CF₂ Cl | CH₃ | H | COOCH₂ CH=CH₂ |
| 4-23 | H | Cl | H | CF₂ Cl | CH₃ | H | COOCH₂ C ≡ CH |
| 4-24 | H | Cl | H | CF₂ Cl | CH₃ | H | CONH₂ |
| 4-25 | H | Cl | H | CF₂ Cl | CH₃ | H | CONHCH₃ |
| 4-26 | H | Cl | H | CF₂ Cl | CH₃ | H | CONHC₂ H ₅ |
| 4-27 | H | Cl | H | CF₂ Cl | CH₃ | H | CON(CH₃)₂ |
| 4-28 | H | Cl | H | CF₂ Cl | CH₃ | H | CON(C₂ H₅)₂ |
| 4-29 | F | Cl | H | CF₂ Cl | CH₃ | H | CH₂ Cl |
| 4-30 | F | Cl | H | CF₂ Cl | CH₃ | H | CH₂ Br |
| 4-31 | F | Cl | H | CF₂ Cl | CH₃ | H | CH₂ OCH₃ |
| 4-32 | F | Cl | H | CF₂ Cl | CH₃ | H | CH₂ OC₂ H₅ |
| 4-33 | F | Cl | H | CF₂ Cl | CH₃ | H | CH₂ OCH ₂ OCH ₃ |
| 4-34 | F | Cl | H | CF₂ Cl | CH₃ | H | CH₂ OCH ₂ OC ₂ H₅ |
| 4-35 | F | Cl | H | CF₂ Cl | CH₃ | H | CH₂ OCOCH ₃ |
| 4-36 | F | Cl | H | CF₂ Cl | CH₃ | H | CH₂ OCOC ₂ H₅ |
| 4-37 | F | Cl | H | CF₂ Cl | CH₃ | H | CH₂ OCO ⁱ C₃ H₇ |
| 4-38 | F | Cl | H | CF₂ Cl | CH₃ | H | CH₂ OCOCH ₂ Cl |
| 4-39 | F | Cl | H | CF₂ Cl | CH₃ | H | CH₂ OCOCCl₃ |
| 4-40 | F | Cl | H | CF₂ Cl | CH₃ | H | CH₂ OCOCF ₃ |
| 4-41 | F | Cl | H | CF₂ Cl | CH₃ | H | COOH |
| 4-42 | F | Cl | H | CF₂ Cl | CH₃ | H | COOCH₃ |
| 4-43 | F | Cl | H | CF₂ Cl | CH₃ | H | COOC₂ H ₅ |
| 4-44 | F | Cl | H | CF₂ Cl | CH₃ | H | COO ⁿ C ₃ H ₇ |
| 4-45 | F | Cl | H | CF₂ Cl | CH₃ | H | COO ⁿ C ₄ H ₉ |
| 4-46 | F | Cl | H | CF₂ Cl | CH₃ | H | COO ⁿ C ₅ H ₁₁ |
| 4-47 | F | Cl | H | CF₂ Cl | CH₃ | H | COO ⁱ C ₃ H ₇ |
| 4-48 | F | Cl | H | CF₂ Cl | CH₃ | H | COO ^{c} C ₅ H ₉ |
| 4-49 | F | Cl | H | CF₂ Cl | CH₃ | H | COO ^{c} C ₆ H ₁₁ |
| 4-50 | F | Cl | H | CF₂ Cl | CH₃ | H | COOCH₂ CH=CH₂ |
| 4-51 | F | Cl | H | CF₂ Cl | CH₃ | H | COOCH₂ C ≡ CH |
| 4-52 | F | Cl | H | CF₂ Cl | CH₃ | H | CONH₂ |
| 4-53 | F | Cl | H | CF₂ Cl | CH₃ | H | CONHCH₃ |
| 4-54 | F | Cl | H | CF₂ Cl | CH₃ | H | CONHC₂ H ₅ |
| 4-55 | F | Cl | H | CF₂ Cl | CH₃ | H | CON(CH₃) ₂ |
| 4-56 | F | Cl | H | CF₂ Cl | CH₃ | H | CON(C₂ H₅) ₂ |
| 4-57 | H | Cl | H | CF₂ Cl | CH₃ | CH₃ | CH₂ Cl |
| 4-58 | H | Cl | H | CF₂ Cl | CH₃ | CH₃ | CH₂ Br |
| 4-59 | H | Cl | H | CF₂ Cl | CH₃ | CH₃ | CH₂ OCH ₃ |
| 4-60 | H | Cl | H | CF₂ Cl | CH₃ | CH₃ | CH₂ OC₂ H |
| 4-61 | H | Cl | H | CF₂ Cl | CH₃ | CH₃ | CH₂ OCH ₂ OCH ₃ |
| 4-62 | H | Cl | H | CF₂ Cl | CH₃ | CH₃ | CH₂ OCH₂ OC₂ H₅ |
| 4-63 | H | Cl | H | CF₂ Cl | CH₃ | CH₃ | CH₂ OCOCH₃ |
| 4-64 | H | Cl | H | CF₂ Cl | CH₃ | CH₃ | CH₂ OCOC₂ H₅ |
| 4-65 | H | Cl | H | CF₂ Cl | CH₃ | CH₃ | CH₂ OCO ⁱ C₃ H₇ |
| 4-66 | H | Cl | H | CF₂ Cl | CH₃ | CH₃ | CH₂ OCOCH₂ Cl |
| 4-67 | H | Cl | H | CF₂ Cl | CH₃ | CH₃ | CH₂ OCOCCl₃ |
| 4-68 | H | Cl | H | CF₂ Cl | CH₃ | CH₃ | CH₂ OCOCF₃ |
| 4-69 | H | Cl | H | CF₂ Cl | CH₃ | CH₃ | COOH |
| 4-70 | H | Cl | H | CF₂ Cl | CH₃ | CH₃ | COOCH₃ |
| 4-71 | H | Cl | H | CF₂ Cl | CH₃ | CH₃ | COOC₂ H₅ |
| 4-72 | H | Cl | H | CF₂ Cl | CH₃ | CH₃ | COO ⁿ C₃ H ₇ |
| 4-73 | H | Cl | H | CF₂ Cl | CH₃ | CH₃ | COO ⁿ C ₄ H₉ |
| 4-74 | H | Cl | H | CF₂ Cl | CH₃ | CH₃ | COO ⁿ C ₅ H₁₁ |
| 4-75 | H | Cl | H | CF₂ Cl | CH₃ | CH₃ | COO ⁱ C₃ H ₇ |
| 4-76 | H | Cl | H | CF₂ Cl | CH₃ | CH₃ | COO ^{c} C ₅ H ₉ |
| 4-77 | H | Cl | H | CF₂ Cl | CH₃ | CH₃ | COO ^{c} C ₆ H ₁₁ |
| 4-78 | H | Cl | H | CF₂ Cl | CH₃ | CH₃ | COOCH₂ CH = CH₂ |
| 4-79 | H | Cl | H | CF₂ Cl | CH₃ | CH₃ | COOCH₂ C ≡CH |
| 4-80 | H | Cl | H | CF₂ Cl | CH₃ | CH₃ | CONH₂ |
| 4-81 | H | Cl | H | CF₂ Cl | CH₃ | CH₃ | CONHCH₃ |
| 4-82 | H | Cl | H | CF₂ Cl | CH₃ | CH₃ | CONHC₂ H₅ |
| 4-83 | H | Cl | H | CF₂ Cl | CH₃ | CH₃ | CON(CH₃)₂ |
| 4-84 | H | Cl | H | CF₂ Cl | CH₃ | CH₃ | CON(C₂H₅)₂ |
| 4-85 | F | Cl | H | CF₂ Cl | CH₃ | CH₃ | CH₂ Cl |
| 4-86 | F | Cl | H | CF₂ Cl | CH₃ | CH₃ | CH₂ Br |
| 4-87 | F | Cl | H | CF₂ Cl | CH₃ | CH₃ | CH₂ OCH ₃ |
| 4-88 | F | Cl | H | CF₂ Cl | CH₃ | CH₃ | CH₂ OC₂ H ₅ |
| 4-89 | F | Cl | H | CF₂ Cl | CH₃ | CH₃ | CH₂ OCH ₂ OCH ₃ |
| 4-90 | F | Cl | H | CF₂ Cl | CH₃ | CH₃ | CH₂ OCH ₂ OC ₂ H₅ |
| 4-91 | F | Cl | H | CF₂ Cl | CH₃ | CH₃ | CH₂ OCOCH ₃ |
| 4-92 | F | Cl | H | CF₂ Cl | CH₃ | CH₃ | CH₂ OCOC ₂ H ₅ |
| 4-93 | F | Cl | H | CF₂ Cl | CH₃ | CH₃ | CH₂ OCO ⁱ C ₃ H ₇ |
| 4-94 | F | Cl | H | CF₂ Cl | CH₃ | CH₃ | CH₂ OCOCH ₂ Cl |
| 4-95 | F | Cl | H | CF₂ Cl | CH₃ | CH₃ | CH₂ OCOCCl₃ |
| 4-96 | F | Cl | H | CF₂ Cl | CH₃ | CH₃ | CH₂ OCOCF₃ |
| 4-97 | F | Cl | H | CF₂ Cl | CH₃ | CH₃ | COOH |
| 4-98 | F | Cl | H | CF₂ Cl | CH₃ | CH₃ | COOCH₃ |
| 4-99 | F | Cl | H | CF₂ Cl | CH₃ | CH₃ | COOC₂ H ₅ |
| 4-100 | F | Cl | H | CF₂ Cl | CH₃ | CH₃ | COO ⁿ C ₃ H ₇ |
| 4-101 | F | Cl | H | CF₂ Cl | CH₃ | CH₃ | COOⁿ C₄ H₉ |
| 4-102 | F | Cl | H | CF₂ Cl | CH₃ | CH₃ | COO ⁿ C ₅ H₁₁ |
| 4-103 | F | Cl | H | CF₂ Cl | CH₃ | CH₃ | COO ⁱ C₃ H₇ |
| 4-104 | F | Cl | H | CF₂ Cl | CH₃ | CH₃ | COO ^{c}C ₅H ₉ |
| 4-105 | F | Cl | H | CF₂ Cl | CH₃ | CH₃ | COO ^{c} C ₆ H ₁₁ |
| 4-106 | F | Cl | H | CF₂ Cl | CH₃ | CH₃ | COOCH₂ CH=CH₂ |
| 4-107 | F | Cl | H | CF₂ Cl | CH₃ | CH₃ | COOCH₂ C ≡ CH |
| 4-108 | F | Cl | H | CF₂ Cl | CH₃ | CH₃ | CONH₂ |
| 4-109 | F | Cl | H | CF₂ Cl | CH₃ | CH₃ | CONHCH₃ |
| 4-110 | F | Cl | H | CF₂ Cl | CH₃ | CH₃ | CONHC₂ H₅ |
| 4-111 | F | Cl | H | CF₂ Cl | CH₃ | CH₃ | CON(CH₃)₂ |
| 4-112 | F | Cl | H | CF₂ Cl | CH₃ | CH₃ | CON(C₂H₅)₂ |
| 4-113 | H | Cl | H | CF₃ | CH₃ | H | CH₂ Cl |
| 4-114 | H | Cl | H | CF₃ | CH₃ | H | CH₂ Br |
| 4-115 | H | Cl | H | CF₃ | CH₃ | H | CH₂ OCH₃ |
| 4-116 | H | Cl | H | CF₃ | CH₃ | H | CH₂ OC₂ H₅ |
| 4-117 | H | Cl | H | CF₃ | CH₃ | H | CH₂ OCH ₂ OCH₃ |
| 4-118 | H | Cl | H | CF₃ | CH₃ | H | CH₂ OCH ₂ OC ₂H₅ |
| 4-119 | H | Cl | H | CF₃ | CH₃ | H | CH₂ OCOCH₃ |
| 4-120 | H | Cl | H | CF₃ | CH₃ | H | CH₂ OCOC ₂H₅ |
| 4-121 | H | Cl | H | CF₃ | CH₃ | H | CH₂ OCO C ₃ H ₇ |
| 4-122 | H | Cl | H | CF₃ | CH₃ | H | CH₂ OCOCH ₂Cl |
| 4-123 | H | Cl | H | CF₃ | CH₃ | H | CH₂ OCOCCl₃ |
| 4-124 | H | Cl | H | CF₃ | CH₃ | H | CH₂ OCOCF ₃ |
| 4-125 | H | Cl | H | CF₃ | CH₃ | H | COOH |
| 4-126 | H | Cl | H | CF₃ | CH₃ | H | COOCH₃ |
| 4-127 | H | Cl | H | CF₃ | CH₃ | H | COOC₂ H ₅ |
| 4-128 | H | Cl | H | CF₃ | CH₃ | H | COO ⁿ C₃ H₇ |
| 4-129 | H | Cl | H | CF₃ | CH₃ | H | COOⁿ C₄ H₉ |
| 4-130 | H | Cl | H | CF₃ | CH₃ | H | COO ⁿ C ₅ H ₁₁ |
| 4-131 | H | Cl | H | CF₃ | CH₃ | H | COO ⁱ C ₃ H ₇ |
| 4-132 | H | Cl | H | CF₃ | CH₃ | H | COO ^{c} C ₅ H ₉ |
| 4-133 | H | Cl | H | CF₃ | CH₃ | H | COO ^{c} C ₆ H ₁₁ |
| 4-134 | H | Cl | H | CF₃ | CH₃ | H | COOCH₂ CH=CH₂ |
| 4-135 | H | Cl | H | CF₃ | CH₃ | H | COOCH₂ C ≡ CH |
| 4-136 | H | Cl | H | CF₃ | CH₃ | H | CONH₂ |
| 4-137 | H | Cl | H | CF₃ | CH₃ | H | CONHCH₃ |
| 4-138 | H | Cl | H | CF₃ | CH₃ | H | CONHC₂ H ₅ |
| 4-139 | H | Cl | H | CF₃ | CH₃ | H | CON(CH₃ ) ₂ |
| 4-140 | H | Cl | H | CF₃ | CH₃ | H | CON(C₂ H ₅ )₂ |
| 4-141 | F | Cl | H | CF₃ | CH₃ | H | CH₂ Cl |
| 4-142 | F | Cl | H | CF₃ | CH₃ | H | CH₂ Br |
| 4-143 | F | Cl | H | CF₃ | CH₃ | H | CH₂ OCH₃ |
| 4-144 | F | Cl | H | CF₃ | CH₃ | H | CH₂ OC₂ H ₅ |
| 4-145 | F | Cl | H | CF₃ | CH₃ | H | CH₂ OCH ₂ OCH₃ |
| 4-146 | F | Cl | H | CF₃ | CH₃ | H | CH₂ OCH ₂OC₂ H₅ |
| 4-147 | F | Cl | H | CF₃ | CH₃ | H | CH₂ OCOCH ₃ |
| 4-148 | F | Cl | H | CF₃ | CH₃ | H | CH₂ OCOC ₂ H₅ |
| 4-149 | F | Cl | H | CF₃ | CH₃ | H | CH₂ OCO ⁱ C₃ H₇ |
| 4-150 | F | Cl | H | CF₃ | CH₃ | H | CH₂ OCOCH₂ Cl |
| 4-151 | F | Cl | H | CF₃ | CH₃ | H | CH₂ OCOCCl₂ |
| 4-152 | F | Cl | H | CF₃ | CH₃ | H | CH₂ OCOCF₃ |
| 4-153 | F | Cl | H | CF₃ | CH₃ | H | COOH |
| 4-154 | F | Cl | H | CF₃ | CH₃ | H | COOCH₃ |
| 4-155 | F | Cl | H | CF₃ | CH₃ | H | COOC₂H₅ |
| 4-156 | F | Cl | H | CF₃ | CH₃ | H | COOⁿ C₃ H₇ |
| 4-157 | F | Cl | H | CF₃ | CH₃ | H | COO ⁿ C₄ H₉ |
| 4-158 | F | Cl | H | CF₃ | CH₃ | H | COO ⁿ C₅H₁₁ |
| 4-159 | F | Cl | H | CF₃ | CH₃ | H | COOⁱ C₃ H₇ |
| 4-160 | F | Cl | H | CF₃ | CH₃ | H | COO^{c} C₅ H₉ |
| 4-161 | F | Cl | H | CF₃ | CH₃ | H | COO ^{c}C₆ H₁₁ |
| 4-162 | F | Cl | H | CF₃ | CH₃ | H | COOCH₂ CH= CH₂ |
| 4-163 | F | Cl | H | CF₃ | CH₃ | H | COOCH₂ C ≡ CH |
| 4-164 | F | Cl | H | CF₃ | CH₃ | H | CONH₂ |
| 4-165 | F | Cl | H | CF₃ | CH₃ | H | CONHCH₃ |
| 4-166 | F | Cl | H | CF₃ | CH₃ | H | CONHC₂ H ₅ |
| 4-167 | F | Cl | H | CF₃ | CH₃ | H | CON(CH₃)₂ |
| 4-168 | F | Cl | H | CF₃ | CH₃ | H | CON(C₂ H₅)₂ |
| 4-169 | H | Cl | H | CF₃ | CH₃ | CH₃ | CH₂ Cl |
| 4-170 | H | Cl | H | CF₃ | CH₃ | CH₃ | CH₂ Br |
| 4-171 | H | Cl | H | CF₃ | CH₃ | CH₃ | CH₂ OCH₃ |
| 4-172 | H | Cl | H | CF₃ | CH₃ | CH₃ | CH₂ OC₂ H₆ |
| 4-173 | H | Cl | H | CF₃ | CH₃ | CH₃ | CH₂ OCH ₂ OCH ₃ |
| 4-174 | H | Cl | H | CF₃ | CH₃ | CH₃ | CH₂ OCH₂ OC ₂ H₅ |
| 4-175 | H | Cl | H | CF₃ | CH₃ | CH₃ | CH₂ OCOCH₃ |
| 4-176 | H | Cl | H | CF₃ | CH₃ | CM, | CH₂ OCOC ₂ H₅ |
| 4-177 | H | Cl | H | CF₃ | CH₃ | CH₃ | CH₂ OCOⁱ C₃ H₇ |
| 4-178 | H | Cl | H | CF₃ | CH₃ | CH₃ | CH₂ OCOCH ₂ Cl |
| 4-179 | H | Cl | H | CF₃ | CH₃ | CH₃ | CH₂ OCOCCl₃ |
| 4-180 | H | Cl | H | CF₃ | CH₃ | CH₃ | CH₂ OCOCF₃ |
| 4-181 | H | Cl | H | CF₃ | CH₃ | CH₃ | COOH |
| 4-182 | H | Cl | H | CF₃ | CH₃ | CH₃ | COOCH₃ |
| 4-183 | H | Cl | H | CF₃ | CH₃ | CH₃ | COOC₂ H₅ |
| 4-184 | H | Cl | H | CF₃ | CH₃ | CH₃ | COO ⁿ C₃ H ₇ |
| 4-185 | H | Cl | H | CF₃ | CH₃ | CH₃ | COO ⁿ C₄ H ₉ |
| 4-186 | H | Cl | H | CF₃ | CH₃ | CH₃ | COO ⁿ C ₅ H₁₁ |
| 4-187 | H | Cl | H | CF₃ | CH₃ | CH₃ | COO ⁱ C ₃ H₇ |
| 4-188 | H | Cl | H | CF₃ | CH₃ | CH₃ | COO ^{c} C ₅ H₉ |
| 4-189 | H | Cl | H | CF₃ | CH₃ | CH₃ | COO ^{c} C ₆ H₁₁ |
| 4-190 | H | Cl | H | CF₃ | CH₃ | CH₃ | COOCH₂ CH=CH₂ |
| 4-191 | H | Cl | H | CF₃ | CH₃ | CH₃ | COOCH₂ C ≡ CH |
| 4-192 | H | Cl | H | CF₃ | CH₃ | CH₃ | CONH₂ |
| 4-193 | H | Cl | H | CF₃ | CH₃ | CH₃ | CONHCH₃ |
| 4-194 | H | Cl | H | CF₃ | CH₃ | CH₃ | CONHC₂ H₅ |
| 4-195 | H | Cl | H | CF₃ | CH₃ | CH₃ | CON(CH₃) ₂ |
| 4-196 | H | Cl | H | CF₃ | CH₃ | CH₃ | CON(C₂ H ₅)₂ |
| 4-197 | F | Cl | H | CF₃ | CH₃ | CH₃ | CH₂ Cl |
| 4-198 | F | Cl | H | CF₃ | CH₃ | CH₃ | CH₂ Br |
| 4-199 | F | Cl | H | CF₃ | CH₃ | CH₃ | CH₂ OCH₃ |
| 4-200 | F | Cl | H | CF₃ | CH₃ | CH₃ | CH₂ OC₂ H₅ |
| 4-201 | F | Cl | H | CF₃ | CH₃ | CH₃ | CH₂ OCH ₂ OCH₃ |
| 4-202 | F | Cl | H | CF₃ | CH₃ | CH₃ | CH₂ OCH₂ OC ₂ H₅ |
| 4-203 | F | Cl | H | CF₃ | CH₃ | CH₃ | CH₂ OCOCH₃ |
| 4-204 | F | Cl | H | CF₃ | CH₃ | CH₃ | CH₂ OCOC ₂ H₅ |
| 4-205 | F | Cl | H | CF₃ | CH₃ | CH₃ | CH₂ OCO C₃ H₇ |
| 4-206 | F | Cl | H | CF₃ | CH₃ | CH₃ | CH₂ OCOCH ₂ Cl |
| 4-207 | F | Cl | H | CF₃ | CH₃ | CH₃ | CH₂ OCOCCl₃ |
| 4-208 | F | Cl | H | CF₃ | CH₃ | CH₃ | CH₂ OCOCF₃ |
| 4-209 | F | Cl | H | CF₃ | CH₃ | CH₃₃ | COOH |
| 4-210 | F | Cl | H | CF₃ | CH₃ | CH₃ | COOCH₃ |
| 4-211 | F | Cl | H | CF₃ | CH₃ | CH₃ | COOC₂ H ₅ |
| 4-212 | F | Cl | H | CF₃ | CH₃ | CH₃ | COOⁿ C ₃ H ₇ |
| 4-213 | F | Cl | H | CF₃ | CH₃ | CH₃ | COO ⁿ C₄ H ₉ |
| 4-214 | F | Cl | H | CF₃ | CH₃ | CH₃ | COO ⁿ C₅ H ₁₁ |
| 4-215 | F | Cl | H | CF₃ | CH₃ | CH₃ | COOⁿ C ₃ H ₇ |
| 4-216 | F | Cl | H | CF₃ | CH₃ | CH₃ | COO ^{c} C ₅ H ₉ |
| 4-217 | F | Cl | H | CF₃ | CH₃ | CH₃ | COO ^{c} C ₆ H ₁₁ |
| 4-218 | F | Cl | H | CF₃ | CH₃ | CH₃ | COOCH₂ CH=CH₂ |
| 4-219 | F | Cl | H | CF₃ | CH₃ | CH₃ | COOCH₂ C ≡ CH |
| 9 - 220 | F | Cl | H | CF₃ | CH₃ | CH₃ | CONH₂ |
| 4-221 | F | Cl | H | CF₃ | CH₃ | CH₃ | CONHCH₃ |
| 4-222 | F | Cl | H | CF₃ | CH₃ | CH₃ | CONHC₂ H ₅ |
| 4-223 | F | Cl | H | CF₃ | CH₃ | CH₃ | CON(CH₃ ) ₂ |
| 4-224 | F | Cl | H | CF₃ | CH₃ | CH₃ | CON(C₂ H ₅) ₂ |
| 4-225 | H | Cl | H | CF₂ Cl | CH₃ | H | CH₂ Cl |
| 4-226 | H | Cl | H | CF₂ Cl | CH₃ | H | CH₂ Br |
| 4-227 | H | Cl | H | CF₂ Cl | CH₃ | H | CH₂ OCH ₃ |
| 4-228 | H | Cl | H | CF₂ Cl | CH₃ | H | CH₂ OC₂ H ₅ |
| 4-229 | H | Cl | H | CF₂ Cl | CH₃ | H | CH₂ OCH ₂ OCH ₃ |
| 4-230 | H | Cl | H | CF₂ Cl | CH₃ | H | CH₂ OCH ₂ OC₂ H₅ |
| 4-231 | H | Cl | H | CF₂ Cl | CH₃ | H | CH₂ OCOCH ₃ |
| 4-232 | H | Cl | H | CF₂ Cl | CH₃ | H | CH₂ OCOC ₂ H ₅ |
| 4-233 | H | Cl | H | CF₂ Cl | CH₃ | H | CH₂ OCO ⁱ C ₃ H ₇ |
| 4-234 | H | Cl | H | CF₂ Cl | CH₃ | H | CH₂ OCOCH ₂ Cl |
| 4-235 | H | Cl | H | CF₂ Cl | CH₃ | H | CH₂ OCOCCl ₃ |
| 4-236 | H | Cl | H | CF₂ Cl | CH₃ | H | CH₂ OCOCF ₃ |
| 4-237 | H | Cl | H | CF₂ Cl | CH₃ | H | COOH |
| 4-238 | H | Cl | H | CF₂ Cl | CH₃ | H | COOCH₃ |
| 4-239 | H | Cl | H | CF₂ Cl | CH₃ | H | COOC₂ H ₅ |
| 4-240 | H | Cl | H | CF₂ Cl | CH₃ | H | COO ⁿ C ₃ H ₇ |
| 4-241 | H | Cl | H | CF₂ Cl | CH₃ | H | COO ⁿ C₄H₉ |
| 4-242 | H | Cl | H | CF₂Cl | CH₃ | H | COOⁿ C ₅H₁₁ |
| 4-243 | H | Cl | H | CF₂ Cl | CH₃ | H | COO ⁱ C₃H₇ |
| 4-244 | H | Cl | H | CF₂ Cl | CH₃ | H | COO ^{c} C ₅ H ₉ |
| 4-245 | H | Cl | H | CF₂ Cl | CH₃ | H | COO ^{c} C ₆ H ₁₁ |
| 4-246 | H | Cl | H | CF₂ Cl | CH₃ | H | COOCH₂ CH = CH₂ |
| 4-247 | H | Cl | H | CF₂ Cl | CH₃ | H | COOCH₂ C ≡ CH |
| 4-248 | H | Cl | H | CF₂ Cl | CH₃ | H | CONH₂ |
| 4-249 | H | Cl | H | CF₂ Cl | CH₃ | H | CONHCH₃ |
| 4-250 | H | Cl | H | CF₂ Cl | CH₃ | H | CONHC₂ H ₅ |
| 4-251 | H | Cl | H | CF₂ Cl | CH₃ | H | CON(CH₃ ) ₂ |
| 4-252 | H | Cl | H | CF₂ Cl | CH₃ | H | CON(C₂ H ₅ ) ₂ |
| 4-253 | F | Cl | H | CF₂ Cl | CH₃ | H | CH₂ Cl |
| 4-254 | F | Cl | H | CF₂ Cl | CH₃ | H | CH₂ Br |
| 4-255 | F | Cl | H | CF₂ Cl | CH₃ | H | CH₂ OCH ₃ |
| 4-256 | F | Cl | H | CF₂ Cl | CH₃ | H | CH₂ OC₂ H ₅ |
| 4-257 | F | Cl | H | CF₂ Cl | CH₃ | H | CH₂ OCH ₂ OCH ₃ |
| 4-258 | F | Cl | H | CF₂ Cl | CH₃ | H | CH₂ OCH ₂ OC₂ H₅ |
| 4-259 | F | Cl | H | CF₂ Cl | CH₃ | H | CH₂ OCOCH₃ |
| 4-260 | F | Cl | H | CF₂ Cl | CH₃ | H | CH₂ OCOC ₂ H₅ |
| 4-261 | F | Cl | H | CF₂ Cl | CH₃ | H | CH₂ OCO ⁱ C₃ H₇ |
| 4-262 | F | Cl | H | CF₂ Cl | CH₃ | H | CH₂ OCOCH Cl |
| 4-263 | F | Cl | H | CF₂ Cl | CH₃ | H | CH₂ OCOCCl₃ |
| 4-264 | F | Cl | H | CF₂ Cl | CH₃ | H | CH₂ OCOCF ₃ |
| 4-265 | F | Cl | H | CF₂ Cl | CH₃ | H | COOH |
| 4-266 | F | Cl | H | CF₂ Cl | CH₃ | H | COOCH₃ |
| 4-267 | F | Cl | H | CF₂ Cl | CH₃ | H | COOC₂ H ₅ |
| 4-268 | F | Cl | H | CF₂ Cl | CH₃ | H | COO ⁿ C₃ H₇ |
| 4-269 | F | Cl | H | CF₂ Cl | CH₃ | H | COO ⁿ C ₄ H ₉ |
| 4-270 | F | Cl | H | CF₂ Cl | CH₃ | H | COO ⁿ C₅ H ₁₁ |
| 4-271 | F | Cl | H | CF₂ Cl | CH₃ | H | COO ⁱ C₃ H ₇ |
| 4-272 | F | Cl | H | CF₂ Cl | CH₃ | H | COO^{c} C ₅ H ₉ |
| 4-273 | F | Cl | H | CF₂ Cl | CH₃ | H | COO^{c} C ₆ H ₁₁ |
| 4-274 | F | Cl | H | CF₂ Cl | CH₃ | H | COOCH₂ CH=CH₂ |
| 4-275 | F | Cl | H | CF₂ Cl | CH₃ | H | COOCH₂ C ≡CH |
| 4-276 | F | Cl | H | CF₂ Cl | CH₃ | H | CONH₂ |
| 4-277 | F | Cl | H | CF₂ Cl | CH₃ | H | CONHCH₃ |
| 4-278 | F | Cl | H | CF₂ Cl | CH₃ | H | CONHC₂ H ₅ |
| 4-279 | F | Cl | H | CF₂ Cl | CH₃ | H | CON(CH₃ ) ₂ |
| 4-280 | F | Cl | H | CF₂ Cl | CH₃ | H | CON(C₂ H ₅ ) ₂ |
| 4-281 | H | Cl | H | CF₂ Cl | CH₃ | CH₃ | CH₂ Cl |
| 4-282 | H | Cl | H | CF₂ Cl | CH₃ | CH₃ | CH₂ Br |
| 4-283 | H | Cl | H | CF₂ Cl | CH₃ | CH₃ | CH₂ OCH ₃ |
| 4-284 | H | Cl | H | CF₂ Cl | CH₃ | CH₃ | CH₂ OC₂ H ₅ |
| 4-285 | H | Cl | H | CF₂ Cl | CH₃ | CH₃ | CH₂ OCH ₂ OCH ₃ |
| 4-286 | H | Cl | H | CF₂ Cl | CH₃ | CH₃ | CH₂ OCH ₂ OC ₂ H₅ |
| 4-287 | H | Cl | H | CF₂ Cl | CH₃ | CH₃ | CH₂ OCOCH₃ |
| 4-288 | H | Cl | H | CF₂ Cl | CH₃ | CH₃ | CH₂ OCOC₂H₅ |
| 4-289 | H | Cl | H | CF₂ Cl | CH₃ | CH₃ | CH₂ OCO'C₃H₇ |
| 4-290 | H | Cl | H | CF₂ Cl | CH₃ | CH₃ | CH₂ OCOCH ₂ Cl |
| 4-291 | H | Cl | H | CF₂ Cl | CH₃ | CH₃ | CH₂ OCOCCl₃ |
| 4-292 | H | Cl | H | CF₂ Cl | CH₃ | CH₃ | CH₂ OCOCF ₃ |
| 4-293 | H | Cl | H | CF₂ Cl | CH₃ | CH₃ | COOH |
| 4-294 | H | Cl | H | CF₂ Cl | CH₃ | CH₃ | COOCH₃ |
| 4-295 | H | Cl | H | CF₂ Cl | CH₃ | CH₃ | COOC₂ H s |
| 4-296 | H | Cl | H | CF₂ Cl | CH₃ | CH₃ | COO ⁿ C ₃ H ₇ |
| 4-297 | H | Cl | H | CF₂ Cl | CH₃ | CH₃ | COO ⁿ C ₄ H ₉ |
| 4-298 | H | Cl | H | CF₂ Cl | CH₃ | CH₃ | COO ⁿ C ₅ H ₁₁ |
| 4-299 | H | Cl | H | CF₂ Cl | CH₃ | CH₃ | COO ⁱ C ₃ H ₇ |
| 4-300 | H | Cl | H | CF₂ Cl | CH₃ | CH₃ | COO ^{c} C ₅ H ₉ |
| 4-301 | H | Cl | H | CF₂ Cl | CH₃ | CH₃ | COO ^{c} C ₆ H₁₁ |
| 4-302 | H | Cl | H | CF₂ Cl | CH₃ | CH₃ | COOCH₂ CH=CH₂ |
| 4-303 | H | Cl | H | CF₂ Cl | CH₃ | CH₃ | COOCH₂ C ≡ CH |
| 4-304 | H | Cl | H | CF₂ Cl | CH₃ | CH₃ | CONH₂ |
| 4-305 | H | Cl | H | CF₂ Cl | CH₃ | CH₃ | CONHCH₃ |
| 4-306 | H | Cl | H | CF₂ Cl | CH₃ | CH₃ | CONHC₂ H ₅ |
| 4-307 | H | Cl | H | CF₂ Cl | CH₃ | CH₃ | CON(CH₃) ₂ |
| 4-308 | H | Cl | H | CF₂ Cl | CH₃ | CH₃ | CON(C₂ H₅) ₂ |
| 4-309 | F | Cl | H | CF₂ Cl | CH₃ | CH₃ | CH₂ Cl |
| 4-310 | F | Cl | H | CF₂ Cl | CH₃ | CH₃ | CH₂ Br |
| 4-311 | F | Cl | H | CF₂ Cl | CH₃ | CH₃ | CH₂ OCH ₃ |
| 4-312 | F | Cl | H | CF₂ Cl | CH₃ | CH₃ | CH₂ OC₂ H ₅ |
| 4-313 | F | Cl | H | CF₂ Cl | CH₃ | CH₃ | CH₂ OCH ₂ OCH₃ |
| 4-314 | F | Cl | H | CF₂ Cl | CH₃ | CH₃ | CH₂ OCH ₂ OC ₂ H₅ |
| 4-315 | F | Cl | H | CF₂ Cl | CH₃ | CH₃ | CH₂ OCOCH₃ |
| 4-316 | F | Cl | H | CF₂ Cl | CH₃ | CH₃ | CH₂ OCOC ₂ H₅ |
| 4-317 | F | Cl | H | CF₂ Cl | CH₃ | CH₃ | CH₂ OCO' C₃ H₇ |
| 4-318 | F | Cl | H | CF₂ Cl | CH₃ | CH₃ | CH₂ OCOCH Cl |
| 4-319 | F | Cl | H | CF₂ Cl | CH₃ | CH₃ | CH₂ OCOCCl₃ |
| 4-320 | F | Cl | H | CF₂ Cl | CH₃ | CH₃ | CH₂ OCOCF₃ |
| 4-321 | F | Cl | H | CF₂ Cl | CH₃ | CH₃ | COOH |
| 4-322 | F | Cl | H | CF₂ Cl | CH₃ | CH₃ | COOCH₃ |
| 4-323 | F | Cl | H | CF₂ Cl | CH₃ | CH₃ | COOC₂ H ₅ |
| 4-324 | F | Cl | H | CF₂ Cl | CH₃ | CH₃ | COO ⁿ C ₃ H ₇ |
| 4-325 | F | Cl | H | CF₂ Cl | CH₃ | CH₃ | COO ⁿ C ₄ H ₉ |
| 4-326 | F | Cl | H | CF₂ Cl | CH₃ | CH₃ | COO ⁿ C ₅ H ₁₁ |
| 4-327 | F | Cl | H | CF₂ Cl | CH₃ | CH₃ | COO ⁱ C ₃ H ₇ |
| 4-328 | F | Cl | H | CF₂ Cl | CH₃ | CH₃ | COO ^{c} C ₅ H ₉ |
| 4-329 | F | Cl | H | CF₂ Cl | CH₃ | CH₃ | COO ^{c} C ₆ H ₁₁ |
| 4-330 | F | Cl | H | CF₂ Cl | CH₃ | CH₃ | COOCH₂ CH = CH₂ |
| 4-331 | F | Cl | H | CF₂ Cl | CH₃ | CH₃ | COOCH₂ C ≡ CH |
| 4-332 | F | Cl | H | CF₂ Cl | CH₃ | CH₃ | CONH₂ |
| 4-333 | F | Cl | H | CF₂ Cl | CH₃ | CH₃ | CONHCH₃ |
| 4-334 | F | Cl | H | CF₂ Cl | CH₃ | CH₃ | CONHC₂ H |
| 4-335 | F | Cl | H | CF₂ Cl | CH₃ | CH₃ | CON(CH₃)₂ |
| 4-336 | F | Cl | H | CF₂ Cl | CH₃ | CH₃ | CON(C₂ H₃) ₂ |
| 4-337 | H | Cl | H | CF₃ | CH₃ | H | CH₂ Cl |
| 4-338 | H | Cl | H | CF₃ | CH ₃ | H | CH₂ Br |
| 4-339 | H | Cl | H | CF₃ | CH₃ | H | CH₂ OCH₃ |
| 4-340 | H | Cl | H | CF₃ | CH₃ | H | CH₂ OC₂ H ₅ |
| 4-341 | H | Cl | H | CF₃ | CH₃ | H | CH₂ OCH ₂ OCH ₃ |
| 4-342 | H | Cl | H | CF₃ | CH₃ | H | CH₂ OCH ₂ OC ₂ H₅ |
| 4-343 | H | Cl | H | CF₃ | CH₃ | H | CH₂ OCOCH ₃ |
| 4-344 | H | Cl | H | CF₃ | CH₃ | H | CH₂ OCOC ₂ H₅ |
| 4-345 | H | Cl | H | CF₃ | CH₃ | H | CH₂ OCO ⁱ C ₃ H ₇ |
| 4-346 | H | Cl | H | CF₃ | CH₃ | H | CH₂ OCOCH ₂ Cl |
| 4-347 | H | Cl | H | CF₃ | CH₃ | H | CH₂ OCOCCl₃ |
| 4-348 | H | Cl | H | CF₃ | CH₃ | H | CH₂ OCOCF ₃ |
| 4-349 | H | Cl | H | CF₃ | CH ₃ | H | COOH |
| 4-350 | H | Cl | H | CF₃ | CH₃ | H | COOCH₃ |
| 4-351 | H | Cl | H | CF₃ | CH₃ | H | COOC₂ H ₆ |
| 4-352 | H | Cl | H | CF₃ | CH₃ | H | COO ⁿ C₃ H₇ |
| 4-353 | H | Cl | H | CF₃ | CH₃ | H | COO ⁿ C ₄ H ₉ |
| 4-354 | H | Cl | H | CF₃ | CH₃ | H | COO ⁿ C₅ H₁₁ |
| 4-355 | H | Cl | H | CF₃ | CH₃ | H | COO ⁱ C ₃ H ₇ |
| 4-356 | H | Cl | H | CF₃ | CH₃ | H | COO ^{c} C ₅ H ₉ |
| 4-357 | H | Cl | H | CF₃ | CH₃ | H | COO ^{c} C ₆ H₁₁ |
| 4-358 | H | Cl | H | CF₃ | CH₃ | H | COOCH₂ CH=CH₂ |
| 4-359 | H | Cl | H | CF₃ | CH₃ | H | COOCH₂ C = CH |
| 4-360 | H | Cl | H | CF₃ | CH₃ | H | CONH₂ |
| 4-361 | H | Cl | H | CF₃ | CH₃ | H | CONHCH₃ |
| 4-362 | H | Cl | H | CF₃ | CH₃ | H | CONHC₂ H ₅ |
| 4-363 | H | Cl | H | CF₃ | CH₃ | H | CON(CH₃)₂ |
| 4-364 | H | Cl | H | CF₃ | CH₃ | H | CON(C₂ H ₅ ) ₂ |
| 4-365 | F | Cl | H | CF₃ | CH₃ | H | CH₂ Cl |
| 4-366 | F | Cl | H | CF₃ | CH₃ | H | CH₂ Br |
| 4-367 | F | Cl | H | CF₃ | CH₃ | H | CH₂ OCH₃ |
| 4-368 | F | Cl | H | CF₃ | CH₃ | H | CH₂ OC₂ H ₅ |
| 4-369 | F | Cl | H | CF₃ | CH₃ | H | CH₂ OCH ₂ OCH ₃ |
| 4-370 | F | Cl | H | CF₃ | CH₃ | H | CH₂ OCH ₂ OC ₂ H ₅ |
| 4-371 | F | Cl | H | CF₃ | CH₃ | H | CH₂ OCOCH ₃ |
| 4-372 | F | Cl | H | CF₃ | CH₃ | H | CH₂ OCOC ₂ H₅ |
| 4-373 | F | Cl | H | CF₃ | CH₃ | H | CH₂ OCO ⁱ C ₃ H ₇ |
| 4-374 | F | Cl | H | CF₃ | CH₃ | H | CH₂ OCOCH ₂ Cl |
| 4-375 | F | Cl | H | CF₃ | CH₃ | H | CH₂ OCOCCl₃ |
| 4-376 | F | Cl | H | CF₃ | CH₃ | H | CH₂ OCOCF ₃ |
| 4-377 | F | Cl | H | CF₃ | CH₃ | H | COOH |
| 4-378 | F | Cl | H | CF₃ | CH₃ | H | COOCH₃ |
| 4-379 | F | Cl | H | CF₃ | CH₃ | H | COOC₂ H ₅ |
| 4-380 | F | Cl | H | CF₃ | CH₃ | H | COO ⁿ C₃ H₇ |
| 4-381 | F | Cl | H | CF₃ | CH₃ | H | COO ⁿ C ₄ H ₉ |
| 4-382 | F | Cl | H | CF₃ | CH₃ | H | COO ⁿ C ₅ H ₁₁ |
| 4-383 | F | Cl | H | CF₃ | CH₃ | H | COO ⁱ C ₃ H ₇ |
| 4-384 | F | Cl | H | CF₃ | CH₃ | H | COO ^{c} C ₅ H ₉ |
| 4-385 | F | Cl | H | CF₃ | CH₃ | H | COO ^{c} C ₆ H ₁₁ |
| 4-386 | F | Cl | H | CF₃ | CH₃ | H | COOCH₂ CH = CH₂ |
| 4-387 | F | Cl | H | CF₃ | CH₃ | H | COOCH₂ C ≡ CH |
| 4-388 | F | Cl | H | CF₃ | CH₃ | H | CONH₂ |
| 4-389 | F | Cl | H | CF₃ | CH₃ | H | CONHCH₃ |
| 4-390 | F | Cl | H | CF₃ | CH₃ | H | CONHC₂ H₅ |
| 4-391 | F | Cl | H | CF₃ | CH₃ | H | CON(CH₃ ) ₂ |
| 4-392 | F | Cl | H | CF₃ | CH₃ | H | CON(C₂ H ₅ ) ₂ |
| 4-393 | H | Cl | H | CF₃ | CH₃ | CH₃ | CH₂ Cl |
| 4-394 | H | Cl | H | CF₃ | CH₃ | CH₃ | CH₂ Br |
| 4-395 | H | Cl | H | CF₃ | CH₃ | CH₃ | CH₂ OCH₃ |
| 4-396 | H | Cl | H | CF₃ | CH₃ | CH₃ | CH₂ OC₂ H ₅ |
| 4-397 | H | Cl | H | CF₃ | CH₃ | CH₃ | CH₂ OCH ₂ OCH ₃ |
| 4-398 | H | Cl | H | CF₃ | CH₃ | CH₃ | CH₂ OCH ₂ OC ₂ H₅ |
| 4-399 | H | Cl | H | CF₃ | CH₃ | CH₃ | CH₂ OCOCH ₃ |
| 4-400 | H | Cl | H | CF₃ | CH₃ | CH₃ | CH₂ OCOC ₂ H₅ |
| 4-401 | H | Cl | H | CF₃ | CH₃ | CH₃ | CH₂ OCO ⁱ C₃ H₇ |
| 4-402 | H | Cl | H | CF₃ | CH₃ | CH₃ | CH₂ OCOCH ₂ Cl |
| 4-403 | H | Cl | H | CF₃ | CH₃ | CH₃ | CH₂ OCOCCl₃ |
| 4-404 | H | Cl | H | CF₃ | CH₃ | CH₃ | CH₂ OCOCF ₃ |
| 4-405 | H | Cl | H | CF₃ | CH₃ | CH₃ | COOH |
| 4-406 | H | Cl | H | CF₃ | CH₃ | CH₃ | COOCH₃ |
| 4-407 | H | Cl | H | CF₃ | CH₃ | CH₃ | COOC₂ H ₅ |
| 4-408 | H | Cl | H | CF₃ | CH₃ | CH₃ | COO ⁿ C ₃ H ₇ |
| 4-409 | H | Cl | H | CF₃ | CH₃ | CH₃ | COO ⁿ C ₄ H ₉ |
| 4-410 | H | Cl | H | CF₃ | CH₃ | CH₃ | COO ⁿ C ₅ H ₁₁ |
| 4-411 | H | Cl | H | CF₃ | CH₃ | CH₃ | COO ⁱ C ₃ H ₇ |
| 4-412 | H | Cl | H | CF₃ | CH₃ | CH₃ | COO ^{c} C ₅ H ₉ |
| 4-413 | H | Cl | H | CF₃ | CH₃ | CH₃ | COO ^{c} C ₆ H ₁₁ |
| 4-414 | H | Cl | H | CF₃ | CH₃ | CH₃ | COOCH₂ CH=CH₂ |
| 4-415 | H | Cl | H | CF₃ | CH₃ | CH₃ | COOCH₂ C ≡CH |
| 4-416 | H | Cl | H | CF₃ | CH₃ | CH₃ | CONH₂ |
| 4-417 | H | Cl | H | CF₃ | CH₃ | CH₃ | CONHCH₃ |
| 4-418 | H | Cl | H | CF₃ | CH₃ | CH₃ | CONHC₂ H ₅ |
| 4-419 | H | Cl | H | CF₃ | CH₃ | CH₃ | CON(CH₃ ) ₂ |
| 4-420 | H | Cl | H | CF₃ | CH₃ | CH₃ | CON(C₂ H ₅) ₂ |
| 4-421 | F | Cl | H | CF₃ | CH₃ | CH₃ | CH₂ Cl |
| 4-422 | F | Cl | H | CF₃ | CH₃ | CH₃ | CH₂ Br |
| 4-423 | F | Cl | H | CF₃ | CH₃ | CH₃ | CH₂ OCH ₃ |
| 4-424 | F | Cl | H | CF₃ | CH₃ | CH₃ | CH₂ OC₂ H ₅ |
| 4-425 | F | Cl | H | CF₃ | CH₃ | CH₃ | CH₂ OCH ₂ OCH ₃ |
| 4-426 | F | Cl | H | CF₃ | CH₃ | CH₃ | CH₂ OCH ₂ OC ₂ H₅ |
| 4-427 | F | Cl | H | CF₃ | CH₃ | CH₃ | CH₂ OCOCH₃ |
| 4-428 | F | Cl | H | CF₃ | CH₃ | CH₃ | CH₂ OCOC₂ H₅ |
| 4-429 | F | Cl | H | CF₃ | CH₃ | CH₃ | CH₂ OCO C H ₇ |
| 4-430 | F | Cl | H | CF₃ | CH₃ | CH₃ | CH₂ OCOCH₂ Cl |
| 4-431 | F | Cl | H | CF₃ | CH₃ | CH₃ | CH₂ OCOCCl₃ |
| 4-432 | F | Cl | H | CF₃ | CH₃ | CH₃ | CH₂ OCOCF₃ |
| 4-433 | F | Cl | H | CF₃ | CH₃ | CH₃ | COOH |
| 4-434 | F | Cl | H | CF₃ | CH₃ | CH₃ | COOCH₃ |
| 4-435 | F | Cl | H | CF₃ | CH₃ | CH₃ | COOC₂ H₅ |
| 4-436 | F | Cl | H | CF₃ | CH₃ | CH₃ | COO ⁿ C₃ H ₇ |
| 4-437 | F | Cl | H | CF₃ | CH₃ | CH₃ | COO ⁿ C₄H ₉ |
| 4-438 | F | Cl | H | CF₃ | CH₃ | CH₃ | COO ⁿ C ₅H₁₁ |
| 4-439 | F | Cl | H | CF₃ | CH₃ | CH₃ | COO ⁱ C₃ H₇ |
| 4-440 | F | Cl | H | CF₃ | CH₃ | CH₃ | COO ^{c}C ₅ H ₉ |
| 4-441 | F | Cl | H | CF₃ | CH₃ | CH₃ | COO ^{c} C₆ H ₁₁ |
| 4-442 | F | Cl | H | CF₃ | CH₃ | CH₃ | COOCH₂ CH = CH₂ |
| 4-443 | F | Cl | H | CF₃ | CH₃ | CH₃ | COOCH₂ C ≡CH |
| 4-444 | F | Cl | H | CF₃ | CH₃ | CH₃ | CONH₂ |
| 4-445 | F | Cl | H | CF₃ | CH₃ | CH₃ | CONHCH₃ |
| 4-446 | F | Cl | H | CF₃ | CH₃ | CH₃ | CONHC₂ H ₅ |
| 4-447 | F | Cl | H | CF₃ | CH₃ | CH₃ | CON(CH₃ ) ₂ |
| 4-448 | F | Cl | H | CF₃ | CH₃ | CH₃ | CON(C₂ H ₅ ) ₂ |
| 4-449 | F | Cl | H | CF₃ | CH₃ | H | CH ₃ |
| 4-450 | F | Cl | H | CF₃ | CH₃ | H | CH ₂ OH |
| 4-451 | F | Cl | H | CF₃ | CH₃ | CH₃ | CH ₃ |
| 4-452 | F | Cl | H | CF₃ | CH₃ | CH₃ | CH₂ OH |
| 4-453 | F | Cl | H | CF₃ | H | H | CH ₃ |
| 4-454 | F | Cl | H | CF₃ | H | H | CH ₂ OH |
| 4-455 | F | Cl | H | CF₃ | H | CH₃ | CH ₃ |
| 4-456 | F | Cl | H | CF₃ | H | CH₃ | CH ₂ OH |
| 4-457 | H | Cl | H | CF₃ | CH₃ | H | CH ₃ |
| 4-458 | H | Cl | H | CF₃ | CH₃ | H | CH ₂ OH |
| 4-459 | H | Cl | H | CF₃ | CH₃ | CH₃ | CH₃ |
| 4-460 | H | Cl | H | CF₃ | CH₃ | CH₃ | CH ₂ OH |
| 4-461 | H | Cl | H | CF₃ | H | H | CH₃ |
| 4-462 | H | Cl | H | CF₃ | H | H | CH ₂ OH |
| 4-963 | H | Cl | H | CF₃ | H | CH₃ | CH₃ |
| 4-464 | H | Cl | H | CF₃ | H | CH₃ | CH ₂ OH |
| 4-465 | F | Cl | H | CF₂ Cl | CH₃ | H | CH₃ |
| 4-466 | F | Cl | H | CF₂ Cl | CH₃ | H | CH ₂ OH |
| 4-467 | F | Cl | H | CF₂ Cl | CH₃ | CH₃ | CH₃ |
| 4-468 | F | Cl | H | CF₂ Cl | CH₃ | CH₃ | CH ₂ OH |
| 4-469 | F | Cl | H | CF₂ Cl | H | H | CH ₃ |
| 4-470 | F | Cl | H | CF₂ Cl | H | H | CH ₂ OH |
| 4-471 | F | Cl | H | CF₂ Cl | H | CH₃ | CH ₃ |
| 4-472 | F | Cl | H | CF₂ Cl | H | CH₃ | CH ₂ OH |
| 4-973 | H | Cl | H | CF₂ Cl | CH₃ | H | CH ₃ |
| 4-474 | H | Cl | H | CF₂ Cl | CH₃ | H | CH ₂ OH |
| 4-475 | H | Cl | H | CF₂ Cl | CH₃ | CH₃ | CH ₃ |
| 4-476 | H | Cl | H | CF₂ Cl | CH₃ | CH₃ | CH ₂ OH |
| 4-477 | H | Cl | H | CF₂ Cl | H | H | CH ₃ |
| 4-478 | H | Cl | H | CF₂ Cl | H | H | CH ₂ OH |
| 4-479 | H | Cl | H | CF₂ Cl | H | CH₃ | CH ₃ |
| 4-480 | H | Cl | H | CF₂ Cl | H | CH₃ | CH ₂ OH |
| 4-481 | H | Cl | H | CF₃ | H | H | CH₂ Cl |
| 4-482 | H | Cl | H | CF₃ | H | H | CH₂ Br |
| 4-483 | H | Cl | H | CF₃ | H | H | CH₂ OCH ₃ |
| 4-484 | H | Cl | H | CF₃ | H | H | CH₂ OC₂ H ₅ |
| 4-485 | H | Cl | H | CF₃ | H | H | CH₂ OCH ₂OCH ₃ |
| 4-486 | H | Cl | H | CF₃ | H | H | CH₂ OCH₂ OC H₅ |
| 4-487 | H | Cl | H | CF₃ | H | H | CH₂ OCOCH ₃ |
| 4-488 | H | Cl | H | CF₃ | H | H | CH₂ OCOC ₂ H₅ |
| 4-489 | H | Cl | H | CF₃ | H | H | CH₂ OCO C ₃ H ₇ |
| 4-490 | H | Cl | H | CF₃ | H | H | CH₂ OCOCH ₂ Cl |
| 4-491 | H | Cl | H | CF₃ | H | H | CH₂ OCOCCl₃ |
| 4-492 | H | Cl | H | CF₃ | H | H | CH₂ OCOCF₃ |
| 4-493 | H | Cl | H | CF₃ | H | H | COOH |
| 4-494 | H | Cl | H | CF₃ | H | H | COOCH₃ |
| 4-495 | H | Cl | H | CF₃ | H | H | COOC₂ H ₅ |
| 4-496 | H | Cl | H | CF₃ | H | H | COO ⁿ C₃ H₇ |
| 4-497 | H | Cl | H | CF₃ | H | H | COO ⁿ C ₄ H ₉ |
| 4-498 | H | Cl | H | CF₃ | H | H | COO ⁿ C ₅ H ₁₁ |
| 4-499 | H | Cl | H | CF₃ | H | H | COO C ₃ H ₇ |
| 4-500 | H | Cl | H | CF₃ | H | H | COO ^{c} C₅ H ₉ |
| 4-501 | H | Cl | H | CF₃ | H | H | COO ^{c} C₆ H ₁₁ |
| 4-502 | H | Cl | H | CF₃ | H | H | COOCH₂ CH=CH₂ |
| 4-503 | H | Cl | H | CF₃ | H | H | COOCH₂ C ≡ CH |
| 4-504 | H | Cl | H | CF₃ | H | H | CONH₂ |
| 4-505 | H | Cl | H | CF₃ | H | H | CONHCH₃ |
| 4-506 | H | Cl | H | CF₃ | H | H | CONHC₂ H ₅ |
| 4-507 | H | Cl | H | CF₃ | H | H | CON(CH₃) ₂ |
| 4-508 | H | Cl | H | CF₃ | H | H | CON(C₂ H ₅) ₂ |
| 4-509 | F | Cl | H | CF₃ | H | H | CH₂ Cl |
| 4-510 | F | Cl | H | CF₃ | H | H | CH₂ Br |
| 4-511 | F | Cl | H | CF₃ | H | H | CH₂ OCH₃ |
| 4-512 | F | Cl | H | CF₃ | H | H | CH₂ OC₂ H ₅ |
| 4-513 | F | Cl | H | CF₃ | H | H | CH₂ OCH ₂ OCH ₃ |
| 4-514 | F | Cl | H | CF₃ | H | H | CH₂ OCH ₂ OC₂H₅ |
| 4-515 | F | Cl | H | CF₃ | H | H | CH₂ OCOCH ₃ |
| 4-516 | F | Cl | H | CF₃ | H | H | CH₂ OCOC ₂ H₅ |
| 4-517 | F | Cl | H | CF₃ | H | H | CH₂ OCO ⁱ C₃ H₇ |
| 4-518 | F | Cl | H | CF₃ | H | H | CH₂ OCOCH₂ Cl |
| 4-519 | F | Cl | H | CF₃ | H | H | CH₂ OCOCCl₃ |
| 4-520 | F | Cl | H | CF₃ | H | H | CH₂ OCOCF₃ |
| 4-521 | F | Cl | H | CF₃ | H | H | COOH |
| 4-522 | F | Cl | H | CF₃ | H | H | COOCH₃ |
| 4-523 | F | Cl | H | CF₃ | H | H | COOC₂ H ₅ |
| 4-524 | F | Cl | H | CF₃ | H | H | COO ⁿ C₃H₇ |
| 4-525 | F | Cl | H | CF₃ | H | H | COO ⁿ C₄H₉ |
| 4-526 | F | Cl | H | CF₃ | H | H | COO ⁿ C₅ H₁₁ |
| 4-527 | F | Cl | H | CF₃ | H | H | COO ⁱ C₃H₇ |
| 4-528 | F | Cl | H | CF₃ | H | H | COO ^{c} C ₅ H ₉ |
| 4-529 | F | Cl | H | CF₃ | H | H | COO ^{c} C ₆ H ₁₁ |
| 4-530 | F | Cl | H | CF₃ | H | H | COOCH₂ CH=CH ₂ |
| 4-531 | F | Cl | H | CF₃ | H | H | COOCH₂ C ≡CH |
| 4-532 | F | Cl | H | CF₃ | H | H | CONH₂ |
| 4-533 | F | Cl | H | CF₃ | H | H | CONHCH₃ |
| 4-534 | F | Cl | H | CF₃ | H | H | CONHC₂ H ₅ |
| 4-535 | F | Cl | H | CF₃ | H | H | CON(CH₃ ) ₂ |
| 4-536 | F | Cl | H | CF₃ | H | H | CON(C₂ H ₅ ) ₂ |
| 4-537 | H | Cl | H | CF₃ | H | CH₃ | CH₂ Cl |
| 4-538 | H | Cl | H | CF₃ | H | CH₃ | CH₂ Br |
| 4-539 | H | Cl | H | CF₃ | H | CH₃ | CH₂ OCH ₃ |
| 4-540 | H | Cl | H | CF₃ | H | CH₃ | CH₂ OC₂ H ₅ |
| 4-541 | H | Cl | H | CF₃ | H | CH₃ | CH₂OCH₂OCH₃ |
| 4-542 | H | Cl | H | CF₃ | H | CH₃ | CH₂ OCH₂OC₂H₅ |
| 4-543 | H | Cl | H | CF₃ | H | CH₃ | CH₂OCOCH₃ |
| 4-544 | H | Cl | H | CF₃ | H | CH₃ | CH₂OCOC₂H₅ |
| 4-545 | H | Cl | H | CF₃ | H | CH₃ | CH₂OCO ⁱ C₃H₇ |
| 4-546 | H | Cl | H | CF₃ | H | CH₃ | CH₂OCOCH₂Cl |
| 4-547 | H | Cl | H | CF₃ | H | CH₃ | CH₂OCOCCl₃ |
| 4-548 | H | Cl | H | CF₃ | H | CH₃ | CH₂OCOCF₃ |
| 4-549 | H | Cl | H | CF₃ | H | CH₃ | COOH |
| 4-550 | H | Cl | H | CF₃ | H | CH₃ | COOCH₃ |
| 4-551 | H | Cl | H | CF₃ | H | CH₃ | COOC₂ H s |
| 4-552 | H | Cl | H | CF₃ | H | CH₃ | COO ⁿ C₃ H ₇ |
| 4-553 | H | Cl | H | CF₃ | H | CH₃ | COO ⁿ C ₄ H ₉ |
| 4-554 | H | Cl | H | CF₃ | H | CH₃ | COO ⁿ C ₅ H ₁₁ |
| 4-555 | H | Cl | H | CF₃ | H | CH₃ | COO ⁱ C ₃ H ₇ |
| 4-556 | H | Cl | H | CF₃ | H | CH₃ | COO ^{c} C ₅ H₉ |
| 4-55T | H | Cl | H | CF₃ | H | CH₃ | COO ^{c} C ₆ H ₁₁ |
| 4-558 | H | Cl | H | CF₃ | H | CH₃ | COOCH₂ CH=CH₂ |
| 4-559 | H | Cl | H | CF₃ | H | CH₃ | COOCH₂ C ≡ CH |
| 4-560 | H | Cl | H | CF₃ | H | CH₃ | CONH₂ |
| 4-561 | H | Cl | H | CF₃ | H | CH₃ | CONHCH₃ |
| 4-562 | H | Cl | H | CF₃ | H | CH₃ | CONHC₂H₅ |
| 4-563 | H | Cl | H | CF₃ | H | CH₃ | CON(CH₃ ) ₂ |
| 4-564 | H | Cl | H | CF₃ | H | CH₃ | CON(C₂ H ₅ ) ₂ |
| 4-565 | F | Cl | H | CF₃ | H | CH₃ | CH₂ Cl |
| 4-566 | F | Cl | H | CF₃ | H | CH₃ | CH₂ Br |
| 4-567 | F | Cl | H | CF₃ | H | CH ₃ | CH₂ OCH ₃ |
| 4-568 | F | Cl | H | CF₃ | H | CH₃ | CH₂ OC₂ H ₅ |
| 4-569 | F | Cl | H | CF₃ | H | CH₃ | CH₂ OCH ₂ OCH ₃ |
| 4-570 | F | Cl | H | CF₃ | H | CH₃ | CH₂ OCH ₂ OC ₂ H₅ |
| 4-571 | F | Cl | H | CF₃ | H | CH₃ | CH₂ OCOCH ₃ |
| 4-572 | F | Cl | H | CF₃ | H | CH₃ | CH₂ OCOC ₂ H₅ |
| 4-573 | F | Cl | H | CF₃ | H | CH₃ | CH₂ OCO ⁱ C ₃ H ₇ |
| 4-574 | F | Cl | H | CF₃ | H | CH₃ | CH₂ OCOCH ₂ Cl |
| 4-575 | F | Cl | H | CF₃ | H | CH₃ | CH₂ OCOCCl₃ |
| 4-576 | F | Cl | H | CF₃ | H | CH₃ | CH₂ OCOCF₃ |
| 4-577 | F | Cl | H | CF₃ | H | CH₃ | COOH |
| 4-578 | F | Cl | H | CF₃ | H | CH₃ | COOCH₃ |
| 4-579 | F | Cl | H | CF₃ | H | CH₃ | COOC₂ H ₅ |
| 4-580 | F | Cl | H | CF₃ | H | CH₃ | COO ⁿ C ₃ H ₇ |
| 4-581 | F | Cl | H | CF₃ | H | CH₃ | COO ⁿ C ₄ H ₉ |
| 4-582 | F | Cl | H | CF₃ | H | CH₃ | COO ⁿ C ₅ H₁₁ |
| 4-583 | F | Cl | H | CF₃ | H | CH₃ | COO ⁱ C₃ H₇ |
| 4-584 | F | Cl | H | CF₃ | H | CH₃ | COO ^{c} C ₅ H ₉ |
| 4-585 | F | Cl | H | CF₃ | H | CH₃ | COO ^{c} C ₆ H ₁₁ |
| 4-586 | F | Cl | H | CF₃ | H | CH₃ | COOCH₂ CH=CH₂ |
| 4-587 | F | Cl | H | CF₃ | H | CH₃ | COOCH₂ C ≡CH |
| 4-588 | F | Cl | H | CF₃ | H | CH₃ | CONH₂ |
| 4-589 | F | Cl | H | CF₃ | H | CH₃ | CONHCH₃ |
| 4-590 | F | Cl | H | CF₃ | H | CH₃ | CONHC₂ H ₅ |
| 4-591 | F | Cl | H | CF₃ | H | CH₃ | CON(CH₃) ₂ |
| 4-592 | F | Cl | H | CF₃ | H | CH₃ | CON(C₂ H ₅ ) ₂ |
| 4-593 | H | Cl | H | CF₃ | H | H | CH₂ Cl |
| 4-594 | H | Cl | H | CF₃ | H | H | CH₂ Br |
| 4-595 | H | Cl | H | CF₃ | H | H | CH₂ OCH₃ |
| 4-596 | H | Cl | H | CF₃ | H | H | CH₂ OC₂ H ₅ |
| 4-597 | H | Cl | H | CF₃ | H | H | CH₂ OCH ₂ OCH ₃ |
| 4-598 | H | Cl | H | CF₃ | H | H | CH₂ OCH ₂ OC ₂ H₅ |
| 4-599 | H | Cl | H | CF₃ | H | H | CH₂ OCOCH ₃ |
| 4-600 | H | Cl | H | CF₃ | H | H | CH₂ OCOC ₂ H₅ |
| 4-601 | H | Cl | H | CF₃ | H | H | CH₂ OCO ⁱ C ₃ H ₇ |
| 4-602 | H | Cl | H | CF₃ | H | H | CH₂ OCOCH ₂ Cl |
| 4-603 | H | Cl | H | CF₃ | H | H | CH₂ OCOCCl₃ |
| 4-604 | H | Cl | H | CF₃ | H | H | CH₂ OCOCF ₃ |
| 4-605 | H | Cl | H | CF₃ | H | H | COOH |
| 4-606 | H | Cl | H | CF₃ | H | H | COOCH₃ |
| 4-607 | H | Cl | H | CF₃ | H | H | COOC₂ H ₅ |
| 4-608 | H | Cl | H | CF₃ | H | H | COO ⁿ C ₃ H ₇ |
| 4-609 | H | Cl | H | CF₃ | H | H | COO ⁿ C ₄ H ₉ |
| 4-610 | H | Cl | H | CF₃ | H | H | COO ⁿ C ₅ H ₁₁ |
| 4-611 | H | Cl | H | CF₃ | H | H | COO ⁱ C ₃ H ₇ |
| 4-612 | H | Cl | H | CF₃ | H | H | COO ^{c} C ₅ H ₉ |
| 4-613 | H | Cl | H | CF₃ | H | H | COO ^{c} C ₆ H ₁₁ |
| 4-614 | H | Cl | H | CF₃ | H | H | COOCH₂ CH=CH₂ |
| 4-615 | H | Cl | H | CF₃ | H | H | COOCH₂ C ≡ CH |
| 4-616 | H | Cl | H | CF₃ | H | H | CONH₂ |
| 4-617 | H | Cl | H | CF₃ | H | H | CONHCH₃ |
| 4-618 | H | Cl | H | CF₃ | H | H | CONHC₂ H ₅ |
| 4-619 | H | Cl | H | CF₃ | H | H | CON(CH₃) ₂ |
| 4-620 | H | Cl | H | CF₃ | H | H | CON(C₂ H ₅) ₂ |
| 4-621 | F | Cl | H | CF₃ | H | H | CH₂ Cl |
| 4-622 | F | Cl | H | CF₃ | H | H | CH₂ Br |
| 4-623 | F | Cl | H | CF₃ | H | H | CH₂ OCH ₃ |
| 4-624 | F | Cl | H | CF₃ | H | H | CH₂ OC₂ H ₅ |
| 4-625 | F | Cl | H | CF₃ | H | H | CH₂ OCH ₂ OCH ₃ |
| 4-626 | F | Cl | H | CF₃ | H | H | CH₂ OCH ₂ OC ₂ H ₅ |
| 4-627 | F | Cl | H | CF₃ | H | H | CH₂ OCOCH ₃ |
| 4-628 | F | Cl | H | CF₃ | H | H | CH₂ OCOC ₂ H ₅ |
| 4-629 | F | Cl | H | CF₃ | H | H | CH₂ OCO ⁱ C₃H₇ |
| 4-630 | F | Cl | H | CF₃ | H | H | CH₂ OCOCH ₂Cl |
| 9 - 631 | F | Cl | H | CF₃ | H | H | CH₂ OCOCCl₃ |
| 4-632 | F | Cl | H | CF₃ | H | H | CH₂ OCOCF ₃ |
| 4-633 | F | Cl | H | CF₃ | H | H | COOH |
| 4-634 | F | Cl | H | CF₃ | H | H | COOCH₃ |
| 4-635 | F | Cl | H | CF₃ | H | H | COOC₂ H s |
| 4-636 | F | Cl | H | CF₃ | H | H | COO ⁿ C ₃ H ₇ |
| 4-637 | F | Cl | H | CF₃ | H | H | COO ⁿ C₄ H ₉ |
| 4-638 | F | Cl | H | CF₃ | H | H | COO ⁿ C ₅ H₁₁ |
| 4-639 | F | Cl | H | CF₃ | H | H | COO ⁱ C₃ H ₇ |
| 4-640 | F | Cl | H | CF₃ | H | H | COO ^{c} C ₅ H ₉ |
| 4-641 | F | Cl | H | CF₃ | H | H | COO ^{c} C ₆ H₁₁ |
| 4-642 | F | Cl | H | CF₃ | H | H | COOCH₂ CH=CH₂ |
| 4-643 | F | Cl | H | CF₃ | H | H | COOCH₂ C ≡ CH |
| 4-644 | F | Cl | H | CF₃ | H | H | CONH₂ |
| 4-645 | F | Cl | H | CF₃ | H | H | CONHCH₃ |
| 4-646 | F | Cl | H | CF₃ | H | H | CONHC₂ H ₅ |
| 4-647 | F | Cl | H | CF₃ | H | H | CON(CH₃ ) ₂ |
| 4-648 | F | Cl | H | CF₃ | H | H | CON(C₂ H ₅ ) ₂ |
| 4-649 | H | Cl | H | CF₃ | H | CH₃ | CH₂ Cl |
| 4-650 | H | Cl | H | CF₃ | H | CH₃ | CH₂ Br |
| 4-651 | H | Cl | H | CF₃ | H | CH₃ | CH₂ OCH ₂ |
| 4-652 | H | Cl | H | CF₃ | H | CH₃ | CH₂ OC₂ H₅ |
| 4-653 | H | Cl | H | CF₃ | H | CH₃ | CH₂ OCH ₂ OCH₃ |
| 4-654 | H | Cl | H | CF₃ | H | CH₃ | CH₂ OCH ₂ OC ₂H₅ |
| 4-655 | H | Cl | H | CF₃ | H | CH₃ | CH₂ OCOCH ₃ |
| 4-656 | H | Cl | H | CF₃ | H | CH₃ | CH₂ OCOC ₂ H₅ |
| 4-657 | H | Cl | H | CF₃ | H | CH₃ | CH₂ OCO ⁱ C ₃ H ₇ |
| 4-658 | H | Cl | H | CF₃ | H | CH₃ | CH₂ OCOCH ₂ Cl |
| 4-659 | H | Cl | H | CF₃ | H | CH₃ | CH₂ OCOCCl₃ |
| 4-660 | H | Cl | H | CF₃ | H | CH₃ | CH₂ OCOCF ₃ |
| 4-661 | H | Cl | H | CF₃ | H | CH₃ | COOH |
| 4-662 | H | Cl | H | CF₃ | H | CH₃ | COOCH₃ |
| 4-663 | H | Cl | H | CF₃ | H | CH₃ | COOC₂ H ₅ |
| 4-664 | H | Cl | H | CF₃ | H | CH₃ | COO ⁿ C ₃ H ₇ |
| 4-665 | H | Cl | H | CF₃ | H | CH₃ | COO ⁿ C₄ H ₉ |
| 4-666 | H | Cl | H | CF₃ | H | CH₃ | COOⁿ C₅ H₁₁ |
| 4-667 | H | Cl | H | CF₃ | H | CH₃ | COOⁱ C₃ H₇ |
| 4-668 | H | Cl | H | CF₃ | H | CH₃ | COO^{c} C₅ H₉ |
| 4-669 | H | Cl | H | CF₃ | H | CH₃ | COO^{c} C₆ H₁₁ |
| 4-670 | H | Cl | H | CF₃ | H | CH₃ | COOCH₂ CH=CH₂ |
| 4-671 | H | Cl | H | CF₃ | H | CH₃ | COOCH₂ C ≡ CH |
| 4-672 | H | Cl | H | CF₃ | H | CH₃ | CONH₂ |
| 4-673 | H | Cl | H | CF₃ | H | CH₃ | CONHCH₃ |
| 4-674 | H | Cl | H | CF₃ | H | CH₃ | CONHC₂ H ₅ |
| 4-675 | H | Cl | H | CF₃ | H | CH₃ | CON(CH₃ ) ₂ |
| 4-676 | H | Cl | H | CF₃ | H | CH₃ | CON(C₂ H₅)₂ |
| 4-677 | F | Cl | H | CF₃ | H | CH₃ | CH₂ Cl |
| 4-678 | F | Cl | H | CF₃ | H | CH₃ | CH₂ Br |
| 4-679 | F | Cl | H | CF₃ | H | CH₃ | CH₂ OCH ₃ |
| 4-680 | F | Cl | H | CF₃ | H | CH₃ | CH₂ OC₂ H ₅ |
| 4-681 | F | Cl | H | CF₃ | H | CH₃ | CH₂ OCH ₂ OCH ₃ |
| 4-682 | F | Cl | H | CF₃ | H | CH₃ | CH₂ OCH ₂ OC ₂ H ₅ |
| 4-683 | F | Cl | H | CF₃ | H | CH₃ | CH₂ OCOCH ₃ |
| 4-684 | F | Cl | H | CF₃ | H | CH₃ | CH₂ OCOC ₂ H₅ |
| 4-685 | F | Cl | H | CF₃ | H | CH₃ | CH₂ OCO ^{l} C ₃ H ₇ |
| 4-686 | F | Cl | H | CF₃ | H | CH₃ | CH₂ OCOCH ₂ Cl |
| 4-687 | F | Cl | H | CF₃ | H | CH₃ | CH₂ OCOCCl₃ |
| 4-688 | F | Cl | H | CF₃ | H | CH₃ | CH₂ OCOCF ₃ |
| 4-689 | F | Cl | H | CF₃ | H | CH₃ | COOH |
| 4-690 | F | Cl | H | CF₃ | H | CH₃ | COOCH₃ |
| 4-691 | F | Cl | H | CF₃ | H | CH₃ | COOC₂. H ₅ |
| 4-692 | F | Cl | H | CF₃ | H | CH₃ | COO ⁿ C ₃ H ₇ |
| 4-693 | F | Cl | H | CF₃ | H | CH₃ | COO ⁿ C ₄ H ₉ |
| 4-694 | F | Cl | H | CF₃ | H | CH₃ | COO ⁿ C ₅ H ₁₁ |
| 4-695 | F | Cl | H | CF₃ | H | CH₃ | COO ⁱ C₃ H ₇ |
| 4-696 | F | Cl | H | CF₃ | H | CH₃ | COO ^{c}C₅ H ₉ |
| 4-697 | F | Cl | H | CF₃ | H | CH₃ | COO ^{c} C ₆ H ₁₁ |
| 4-698 | F | Cl | H | CF₃ | H | CH₃ | COOCH₂ CH=CH₂ |
| 4-699 | F | Cl | H | CF₃ | H | CH₃ | COOCH₂ C ≡ CH |
| 4-700 | F | Cl | H | CF₃ | H | CH₃ | CONH₂ |
| 4-701 | F | Cl | H | CF₃ | H | CH₃ | CONHCH₃ |
| 4-702 | F | Cl | H | CF₃ | H | CH₃ | CONHC₂ H ₅ |
| 4-703 | F | Cl | H | CF₃ | H | CH₃ | CON(CH₃ ) ₂ |
| 4-704 | F | Cl | H | CF₃ | H | CH₃ | CON (C₂ H ₅ ) ₂ |
| 4-705 | H | Cl | CH₃ | CF₃ | H | H | CH₂ Cl |
| 4-706 | H | Cl | CH₃ | CF₃ | H | H | CH₂ Br |
| 4-707 | H | Cl | CH₃ | CF₃ | H | H | CH₂ OCH ₃ |
| 4-708 | H | Cl | CH₃ | CF₃ | H | H | CH₂ OC₂ H ₅ |
| 4-709 | H | Cl | CH₃ | CF₃ | H | H | CH₂ OCH ₂ OCH ₃ |
| 4-710 | H | Cl | CH₃ | CF₃ | H | H | CH₂ OCH ₂ OC ₂ H₅ |
| 4-711 | H | Cl | CH₃ | CF₃ | H | H | CH₂ OCOCH ₃ |
| 4-712 | H | Cl | CH₃ | CF₃ | H | H | CH₂ OCOC ₂ H₅ |
| 4-713 | H | Cl | CH₃ | CF₃ | H | H | CH₂ OCO ⁱ C ₃ H ₇ |
| 4-714 | H | Cl | CH₃ | CF₃ | H | H | CH₂ OCOCH ₂ Cl |
| 4-715 | H | Cl | CH₃ | CF₃ | H | H | CH₂ OCOCCl₃ |
| 4-716 | H | Cl | CH₃ | CF₃ | H | H | CH₂ OCOCF ₃ |
| 4-717 | H | Cl | CH₃ | CF₃ | H | H | COOH |
| 4-718 | H | Cl | CH₃ | CF₃ | H | H | COOCH₃ |
| 4-719 | H | Cl | CH₃ | CF₃ | H | H | COOC₂ H ₅ |
| 4-720 | H | Cl | CH₃ | CF₃ | H | H | COO ⁿ C ₃ H ₇ |
| 4-721 | H | Cl | CH₃ | CF₃ | H | H | COO ⁿ C ₄ H ₉ |
| 4-722 | H | Cl | CH₃ | CF₃ | H | H | COO ⁿ C ₅ H ₁₁ |
| 4-723 | H | Cl | CH₃ | CF₃ | H | H | COO ⁱ C ₃ H ₇ |
| 4-724 | H | Cl | CH₃ | CF₃ | H | H | COO ^{c} C ₅ H ₉ |
| 4-725 | H | Cl | CH₃ | CF₃ | H | H | COO ^{c} C ₆ H ₁₁ |
| 4-726 | H | Cl | CH₃ | CF₃ | H | H | COOCH₂ CH=CH₂ |
| 4-727 | H | Cl | CH₃ | CF₃ | H | H | COOCH₂ C ≡ CH |
| 4-728 | H | Cl | CH₃ | CF₃ | H | H | CONH₂ |
| 4-729 | H | Cl | CH₃ | CF₃ | H | H | CONHCH₃ |
| 4-730 | H | Cl | CH₃ | CF₃ | H | H | CONHC₂ H ₅ |
| 4-731 | H | Cl | CH₃ | CF₃ | H | H | CON(CH₃) ₂ |
| 4-732 | H | Cl | CH₃ | CF₃ | H | H | CON(C₂ H ₅ ) ₂ |
| 4-733 | F | Cl | CH₃ | CF₃ | H | H | CH₂ Cl |
| 4-734 | F | Cl | CH₃ | CF₃ | H | H | CH₂ Br |
| 4-735 | F | Cl | CH₃ | CF₃ | H | H | CH₂ OCH ₃ |
| 4-736 | F | Cl | CH₃ | CF₃ | H | H | CH₂ OC₂ H ₅ |
| 4-737 | F | Cl | CH₃ | CF₃ | H | H | CH₂ OCH ₂ OCH ₃ |
| 4-738 | F | Cl | CH₃ | CF₃ | H | H | CH₂ OCH ₂ OC ₂ H₅ |
| 4-739 | F | Cl | CH₃ | CF₃ | H | H | CH₂ OCOCH ₃ |
| 4-740 | F | Cl | CH₃ | CF₃ | H | H | CH₂ OCOC ₂ H₅ |
| 4-741 | F | Cl | CH₃ | CF₃ | H | H | CH₂ OCO ⁱ C ₃ H ₇ |
| 4-742 | F | Cl | CH₃ | CF₃ | H | H | CH₂ OCOCH ₂ Cl |
| 4-743 | F | Cl | CH₃ | CF₃ | H | H | CH₂ OCOCCl₃ |
| 4-744 | F | Cl | CH₃ | CF₃ | H | H | CH₂ OCOCF ₃ |
| 4-745 | F | Cl | CH₃ | CF₃ | H | H | COOH |
| 4-746 | F | Cl | CH₃ | CF₃ | H | H | COOCH₃ |
| 4-747 | F | Cl | CH₃ | CF₃ | H | H | COOC₂ H₅ |
| 4-748 | F | Cl | CH₃ | CF₃ | H | H | COO ⁿ C₃ H₇ |
| 4-749 | F | Cl | CH₃ | CF₃ | H | H | COO ⁿ C₄ H₉ |
| 4-750 | F | Cl | CH₃ | CF₃ | H | H | COO ⁿ C₅ H₁₁ |
| 4-751 | F | Cl | CH₃ | CF₃ | H | H | COO ⁱ C₃ H₇ |
| 4-752 | F | Cl | CH₃ | CF₃ | H | H | COO ^{c} C₅ H₉ |
| 4-753 | F | Cl | CH₃ | CF₃ | H | H | COO ^{c} C₆ H₁₁ |
| 4-754 | F | Cl | CH₃ | CF₃ | H | H | COOCH₂ CH=CH₂ |
| 4-755 | F | Cl | CH₃ | CF₃ | H | H | COOCH₂ C ≡CH |
| 4-756 | F | Cl | CH₃ | CF₃ | H | H | CONH₂ |
| 4-757 | F | Cl | CH₃ | CF₃ | H | H | CONHCH₃ |
| 4-758 | F | Cl | CH₃ | CF₃ | H | H | CONHC₂ H ₅ |
| 4-759 | F | Cl | CH₃ | CF₃ | H | H | CON(CH₃)₂ |
| 4-760 | F | Cl | CH₃ | CF₃ | H | H | CON(C₂ H ₅ ) ₂ |
| 4-761 | H | Cl | CH₃ | CF₃ | H | CH₃ | CH₂ Cl |
| 4-762 | H | Cl | CH₃ | CF₃ | H | CH₃ | CH₂ Br |
| 4-763 | H | Cl | CH₃ | CF₃ | H | CH₃ | CH₂ OCH ₃ |
| 4-764 | H | Cl | CH₃ | CF₃ | H | CH₃ | CH₂ OC₂ H ₅ |
| 4-765 | H | Cl | CH₃ | CF₃ | H | CH₃ | CH₂ OCH ₂ OCH ₃ |
| 4-766 | H | Cl | CH₃ | CF₃ | H | CH₃ | CH₂ OCH₂ OC₂ H₅ |
| 4-767 | H | Cl | CH₃ | CF₃ | H | CH₃ | CH₂ OCOCH₃ |
| 4-768 | H | Cl | CH₃ | CF₃ | H | CH₃ | CH₂ OCOC₂ H₅ |
| 4-769 | H | Cl | CH₃ | CF₃ | H | CH₃ | CH₂ OCO ⁱ C₃ H₇ |
| 4-770 | H | Cl | CH₃ | CF₃ | H | CH₃ | CH₂OCOCH₂ Cl |
| 4-771 | H | Cl | CH₃ | CF₃ | H | CH₃ | CH₂ OCOCCl₃ |
| 4-772 | H | Cl | CH₃ | CF₃ | H | CH₃ | CH₂ OCOCF₃ |
| 4-773 | H | Cl | CH₃ | CF₃ | H | CH₃ | COOH |
| 4-774 | H | Cl | CH₃ | CF₃ | H | CH₃ | COOCH₃ |
| 4-775 | H | Cl | CH₃ | CF₃ | H | CH₃ | COOC₂ H₅ |
| 4-776 | H | Cl | CH₃ | CF₃ | H | CH₃ | COO ⁿ C₃ H₇ |
| 4-777 | H | Cl | CH₃ | CF₃ | H | CH₃ | COO ⁿ C₄ H₉ |
| 4-778 | H | Cl | CH₃ | CF₃ | H | CH₃ | COOⁿ C₅ H₁₁ |
| 4-779 | H | Cl | CH₃ | CF₃ | H | CH₃ | COO ⁱ C ₃ H₇ |
| 4-780 | H | Cl | CH₃ | CF₃ | H | CH₃ | COO ^{c} C₅ H ₉ |
| 4-781 | H | Cl | CH₃ | CF₃ | H | CH₃ | COO ^{c}C₆ H₁₁ |
| 4-782 | H | Cl | CH₃ | CF₃ | H | CH₃ | COOCH₂ CH= CH₂ |
| 4-783 | H | Cl | CH₃ | CF₃ | H | CH₃ | COOCH₂ C ≡ CH |
| 4-784 | H | Cl | CH₃ | CF₃ | H | CH₃ | CONH₂ |
| 4-785 | H | Cl | CH₃ | CF₃ | H | CH₃ | CONHCH₃ |
| 4-786 | H | Cl | CH₃ | CF₃ | H | CH₃ | CONHC₂ H ₅ |
| 4-787 | H | Cl | CH₃ | CF₃ | H | CH₃ | CON(CH₃)₂ |
| 4-788 | H | Cl | CH₃ | CF₃ | H | CH₃ | CON(C₂ H₆)₂ |
| 4-789 | F | Cl | CH₃ | CF₃ | H | CH₃ | CH₂ Cl |
| 4-790 | F | Cl | CH₃ | CF₃ | H | CH₃ | CH₂ Br |
| 4-791 | F | Cl | CH₃ | CF₃ | H | CH₃ | CH₂ OCH₃ |
| 4-792 | F | Cl | CH₃ | CF₃ | H | CH₃ | CH₂ OC₂ H ₅ |
| 4-793 | F | Cl | CH₃ | CF₃ | H | CH₃ | CH₂ OCH₂ OCH ₃ |
| 4-794 | F | Cl | CH₃ | CF₃ | H | CH₃ | CH₂ OCH ₂ OC H₅ |
| 4-795 | F | Cl | CH₃ | CF₃ | H | CH₃ | CH₂ OCOCH₃ |
| 4-796 | F | Cl | CH₃ | CF₃ | H | CH₃ | CH₂ OCOC ₂ H₅ |
| 4-797 | F | Cl | CH₃ | CF₃ | H | CH₃ | CH₂ OCO ⁱ C₃ H₇ |
| 4-798 | F | Cl | CH₃ | CF₃ | H | CH₃ | CH₂ OCOCH ₂ Cl |
| 4-799 | F | Cl | CH₃ | CF₃ | H | CH₃ | CH₂ OCOCCl₃ |
| 4-800 | F | Cl | CH₃ | CF₃ | H | CH₃ | CH₂ OCOCF₃ |
| 4-801 | F | Cl | CH₃ | CF₃ | H | CH₃ | COOH |
| 4-802 | F | Cl | CH₃ | CF₃ | H | CH₃ | COOCH₃ |
| 4-803 | F | Cl | CH₃ | CF₃ | H | CH₃ | COOC₂ H |
| 4-804 | F | Cl | CH₃ | CF₃ | H | CH₃ | COO ⁿ C ₃ H ₇ |
| 4-805 | F | Cl | CH₃ | CF₃ | H | CH₃ | COO ⁿ C ₄ H ₉ |
| 4-806 | F | Cl | CH₃ | CF₃ | H | CH₃ | COO ⁿ C ₅ H ₁₁ |
| 4-807 | F | Cl | CH₃ | CF₃ | H | CH₃ | COO ⁱ C ₃ H ₇ |
| 4-808 | F | Cl | CH₃ | CF₃ | H | CH₃ | COO ^{c} C ₅ H ₉ |
| 4-809 | F | Cl | CH₃ | CF₃ | H | CH₃ | COO ^{c} C ₆ H ₁₁ |
| 4-810 | F | Cl | CH₃ | CF₃ | H | CH₃ | COOCH₂ CH = CH₂ |
| 4-811 | F | Cl | CH₃ | CF₃ | H | CH₃ | COOCH₂ C = CH |
| 4-812 | F | Cl | CH₃ | CF₃ | H | CH₃ | CONH₂ |
| 4-813 | F | Cl | CH₃ | CF₃ | H | CH₃ | CONHCH₃ |
| 4-814 | F | Cl | CH₃ | CF₃ | H | CH₃ | CONHC₂ H ₅ |
| 4-815 | F | Cl | CH₃ | CF₃ | H | CH₃ | CON(CH₃ ) ₂ |
| 4-816 | F | Cl | CH₃ | CF₃ | H | CH₃ | CON(C₂ H ₅ ) ₂ |
| 4-817 | H | Cl | CH₃ | CF₃ | H | H | CH₂ Cl |
| 4-818 | H | Cl | CH₃ | CF₃ | H | H | CH₂ Br |
| 4-819 | H | Cl | CH₃ | CF₃ | H | H | CH₂ OCH ₃ |
| 4-820 | H | Cl | CH₃ | CF₃ | H | H | CH₂ OC₂ H ₅ |
| 4-821 | H | Cl | CH₃ | CF₃ | H | H | CH₂ OCH ₂ OCH ₃ |
| 4-822 | H | Cl | CH₃ | CF₃ | H | H | CH₂ OCH ₂ OC ₂ H₅ |
| 4-823 | H | Cl | CH₃ | CF₃ | H | H | CH₂ OCOCH ₃ |
| 4-824 | H | Cl | CH₃ | CF₃ | H | H | CH₂ OCOC ₂ H ₅ |
| 4-825 | H | Cl | CH₃ | CF₃ | H | H | CH₂ OCO ⁱ C ₃ H ₇ |
| 4-826 | H | Cl | CH₃ | CF₃ | H | H | CH₂ OCOCH ₂ Cl |
| 4-827 | H | Cl | CH₃ | CF₃ | H | H | CH₂ OCOCCl₃ |
| 4-828 | H | Cl | CH₃ | CF₃ | H | H | CH₂ OCOCF ₃ |
| 4-829 | H | Cl | CH₃ | CF₃ | H | H | COOH |
| 4-830 | H | Cl | CH₃ | CF₃ | H | H | COOCH₃ |
| 9 - 831 | H | Cl | CH₃ | CF₃ | H | H | COOC₂ H ₆ |
| 4-832 | H | Cl | CH₃ | CF₃ | H | H | COO ⁿ C ₃ H ₇ |
| 4-833 | H | Cl | CH₃ | CF₃ | H | H | COO ⁿ C ₄ H ₉ |
| 4-834 | H | Cl | CH₃ | CF₃ | H | H | COO ⁿ C ₅H₁₁ |
| 4-835 | H | Cl | CH₃ | CF₃ | H | H | COO ⁱ C₃ H₇ |
| 4-836 | H | Cl | CH₃ | CF₃ | H | H | COO ^{c} C ₅H₉ |
| 4-837 | H | Cl | CH₃ | CF₃ | H | H | COO ^{c} C ₆H₁₁ |
| 4-838 | H | Cl | CH₃ | CF₃ | H | H | COOCH₂ CH=CH₂ |
| 4-839 | H | Cl | CH₃ | CF₃ | H | H | COOCH₂ C ≡ CH |
| 4-840 | H | Cl | CH₃ | CF₃ | H | H | CONH₂ |
| 4-841 | H | Cl | CH₃ | CF₃ | H | H | CONHCH₃ |
| 4-842 | H | Cl | CH₃ | CF₃ | H | H | CONHC₂ H ₅ |
| 4-843 | H | Cl | CH₃ | CF₃ | H | H | CON(CH₃ ) ₂ |
| 4-844 | H | Cl | CH₃ | CF₃ | H | H | CON(C₂ H ₅ ) ₂ |
| 4-845 | F | Cl | CH₃ | CF₃ | H | H | CH₂ Cl |
| 4-846 | F | Cl | CH₃ | CF₃ | H | H | CH₂ Br |
| 4-847 | F | Cl | CH₃ | CF₃ | H | H | CH₂ OCH ₃ |
| 4-848 | F | Cl | CH₃ | CF₃ | H | H | CH₂ OC₂ H ₅ |
| 4-849 | F | Cl | CH₃ | CF₃ | H | H | CH₂ OCH ₂ OCH ₃ |
| 4-850 | F | Cl | CH₃ | CF₃ | H | H | CH₂ OCH ₂ OC ₂ H ₅ |
| 4-851 | F | Cl | CH₃ | CF₃ | H | H | CH₂ OCOCH ₃ |
| 4-852 | F | Cl | CH₃ | CF₃ | H | H | CH₂ OCOC ₂ H ₅ |
| 4-853 | F | Cl | CH₃ | CF₃ | H | H | CH₂ OCO ⁱ C ₃ H ₇ |
| 4-854 | F | Cl | CH₃ | CF₃ | H | H | CH ₂ OCOCH ₂ Cl |
| 4-855 | F | Cl | CH₃ | CF₃ | H | H | CH₂ OCOCCl ₃ |
| 4-856 | F | Cl | CH₃ | CF₃ | H | H | CH₂ OCOCF ₃ |
| 4-857 | F | Cl | CH₃ | CF₃ | H | H | COOH |
| 4-858 | F | Cl | CH₃ | CF₃ | H | H | COOCH₃ |
| 4-859 | F | Cl | CH₃ | CF₃ | H | H | COOC₂H₅ |
| 4-860 | F | Cl | CH₃ | CF₃ | H | H | COO ⁿ C ₃ H₇ |
| 4-861 | F | Cl | CH₃ | CF₃ | H | H | COO ⁿ C ₄ H ₉ |
| 4-862 | F | Cl | CH₃ | CF₃ | H | H | COO ⁿ C ₅ H ₁₁ |
| 4-863 | F | Cl | CH₃ | CF₃ | H | H | COO ⁱ C ₃ H₇ |
| 4-864 | F | Cl | CH₃ | CF₃ | H | H | COO ^{c} C ₅ H₉ |
| 4-865 | F | Cl | CH₃ | CF₃ | H | H | COO ^{c} C ₆ H₁₁ |
| 4-866 | F | Cl | CH₃ | CF₃ | H | H | COOCH₂ CH=CH₂ |
| 4-867 | F | Cl | CH₃ | CF₃ | H | H | COOCH₂ C ≡CH |
| 4-868 | F | Cl | CH₃ | CF₃ | H | H | CONH₂ |
| 4-869 | F | Cl | CH₃ | CF₃ | H | H | CONHCH₃ |
| 4-870 | F | Cl | CH₃ | CF₃ | H | H | CONHC₂ H₅ |
| 4-871 | F | Cl | CH₃ | CF₃ | H | H | CON(CH₃) ₂ |
| 4-872 | F | Cl | CH₃ | CF₃ | H | H | CON(C₂ H₅) ₂ |
| 4-873 | H | Cl | CH₃ | CF₃ | H | CH₃ | CH₂ Cl |
| 4-874 | H | Cl | CH₃ | CF₃ | H | CH₃ | CH₂ Br |
| 4-875 | H | Cl | CH₃ | CF₃ | H | CH₃ | CH₂ OCH₃ |
| 4-876 | H | Cl | CH₃ | CF₃ | H | CH₃ | CH₂ OC₂ H ₅ |
| 4-877 | H | Cl | CH₃ | CF₃ | H | CH₃ | CH₂ OCH ₂ OCH ₃ |
| 4-878 | H | Cl | CH₃ | CF₃ | H | CH₃ | CH₂ OCH ₂ OC ₂ H₅ |
| 4-879 | H | Cl | CH₃ | CF₃ | H | CH₃ | CH₂ OCOCH ₃ |
| 4-880 | H | Cl | CH₃ | CF₃ | H | CH₃ | CH₂ OCOC ₂ H₅ |
| 4-881 | H | Cl | CH₃ | CF₃ | H | CH₃ | CH₂ OCO' C ₃H₇ |
| 4-882 | H | Cl | CH₃ | CF₃ | H | CH₃ | CH₂ OCOCH ₂ Cl |
| 4-883 | H | Cl | CH₃ | CF₃ | H | CH₃ | CH₂ OCOCCl₃ |
| 4-884 | H | Cl | CH₃ | CF₃ | H | CH₃ | CH₂ OCOCF₃ |
| 4-885 | H | Cl | CH₃ | CF₃ | H | CH₃ | COOH |
| 4-886 | H | Cl | CH₃ | CF₃ | H | CH₃ | COOCH₃ |
| 4-887 | H | Cl | CH₃ | CF₃ | H | CH₃ | COOC₂ H s |
| 4-888 | H | Cl | CH₃ | CF₃ | H | CH₃ | COO ⁿ C₃ H ₇ |
| 4-889 | H | Cl | CH₃ | CF₃ | H | CH₃ | COO ⁿ C₄ H₉ |
| 4-890 | H | Cl | CH₃ | CF₃ | H | CH₃ | COO ⁿ C₅ H ₁₁ |
| 4-891 | H | Cl | CH₃ | CF₃ | H | CH₃ | COO ⁱ C ₃ H ₇ |
| 4-892 | H | Cl | CH₃ | CF₃ | H | CH₃ | COO ^{c} C ₅ H ₉ |
| 4-893 | H | Cl | CH₃ | CF₃ | H | CH₃ | COO ^{c}C₆ H₁₁ |
| 4-894 | H | Cl | CH₃ | CF₃ | H | CH₃ | COOCH₂ CH= CH₂ |
| 4-895 | H | Cl | CH₃ | CF₃ | H | CH₃ | COOCH₂ C ≡CH |
| 4-896 | H | Cl | CH₃ | CF₃ | H | CH₃ | CONH₂ |
| 4-897 | H | Cl | CH₃ | CF₃ | H | CH₃ | CONHCH₃ |
| 4-898 | H | Cl | CH₃ | CF₃ | H | CH₃ | CONHC₂ H ₅ |
| 4-899 | H | Cl | CH₃ | CF₃ | H | CH₃ | CON(CH₃ ) ₂ |
| 4-900 | H | Cl | CH₃ | CF₃ | H | CH₃ | CON(C₂ H₅) ₂ |
| 4-901 | F | Cl | CH₃ | CF₃ | H | CH₃ | CH₂ Cl |
| 4-902 | F | Cl | CH₃ | CF₃ | H | CH₃ | CH₂ Br |
| 4-903 | F | Cl | CH₃ | CF₃ | H | CH₃ | CH₂ OCH ₃ |
| 4-904 | F | Cl | CH₃ | CF₃ | H | CH₃ | CH₂ OC₂ H ₅ |
| 4-905 | F | Cl | CH₃ | CF₃ | H | CH₃ | CH₂ OCH ₂ OCH ₃ |
| 4-906 | F | Cl | CH₃ | CF₃ | H | CH₃ | CH₂ OCH ₂ OC ₂ H₅ |
| 4-907 | F | Cl | CH₃ | CF₃ | H | CH₃ | CH₂ OCOCH ₃ |
| 4-908 | F | Cl | CH₃ | CF₃ | H | CH₃ | CH₂ OCOC ₂H₅ |
| 4-909 | F | Cl | CH₃ | CF₃ | H | CH₃ | CH₂ OCO C H ₇ |
| 4-910 | F | Cl | CH₃ | CF₃ | H | CH₃ | CH₂ OCOCH ₂ Cl |
| 4-911 | F | Cl | CH₃ | CF₃ | H | CH₃ | CH₂ OCOCCl₃ |
| 4-912 | F | Cl | CH₃ | CF₃ | H | CH₃ | CH₂ OCOCF ₃ |
| 4-913 | F | Cl | CH₃ | CF₃ | H | CH₃ | COOH |
| 4-914 | F | Cl | CH₃ | CF₃ | H | CH₃ | COOCH₃ |
| 4-915 | F | Cl | CH₃ | CF₃ | H | CH₃ | COOC₂ H ₅ |
| 4-916 | F | Cl | CH₃ | CF₃ | H | CH₃ | COO ⁿ C₃ H₇ |
| 4-917 | F | Cl | CH₃ | CF₃ | H | CH₃ | COO ⁿ C ₄ H ₉ |
| 4-918 | F | Cl | CH₃ | CF₃ | H | CH₃ | COO ⁿ C ₅ H₁₁ |
| 4-919 | F | Cl | CH₃ | CF₃ | H | CH₃ | COO ⁱ C₃ H₇ |
| 4-920 | F | Cl | CH₃ | CF₃ | H | CH₃ | COO ^{c} C ₅ H₉ |
| 4-921 | F | Cl | CH₃ | CF₃ | H | CH₃ | COO ^{c} C ₆H₁₁ |
| 4-922 | F | Cl | CH₃ | CF₃ | H | CH₃ | COOCH₂ CH = CH₂ |
| 4-923 | F | Cl | CH₃ | CF₃ | H | CH₃ | COOCH₂ C ≡ CH |
| 4-924 | F | Cl | CH₃ | CF₃ | H | CH₃ | CONH₂ |
| 4-925 | F | Cl | CH₃ | CF₃ | H | CH₃ | CONHCH₃ |
| 4-926 | F | Cl | CH₃ | CF₃ | H | CH₃ | CONHC₂ H ₅ |
| 4-927 | F | Cl | CH₃ | CF₃ | H | CH₃ | CON(CH₃) ₂ |
| 4-928 | F | Cl | CH₃ | CF₃ | H | CH₃ | CON(C₂ H ₅ ) ₂ |
| 4-929 | F | Cl | CH₃ | CF₃ | H | H | CH₃ |
| 4-930 | F | Cl | CH₃ | CF₃ | H | H | CH ₂ OH |
| 4-931 | F | Cl | CH₃ | CF₃ | H | CH₃ | CH₃ |
| 4-932 | F | Cl | CH₃ | CF₃ | H | CH₃ | CH ₂ OH |
| 4-933 | H | Cl | CH₃ | CF₃ | H | H | CH₃ |
| 4-934 | H | Cl | CH₃ | CF₃ | H | H | CH ₂ OH |
| 4-935 | H | Cl | CH₃ | CF₃ | H | CH₃ | CH ₃ |
| 4-936 | H | Cl | CH₃ | CF₃ | H | CH₃ | CH ₂ OH |

The physical properties (melting point, m.p.) or ¹H-NMR (250 or 300 MHz, CDCl₃. TMS, δ(ppm)) data for some of the present compounds are shown below.
Compound 1-332, m.p. 97.0°C
Compound 1-335, m.p. 80.8°C
Compound 1-337, m.p. 91.5°C
Compound 1-338, m.p. 86.1°C
Compound 1-344, m.p. 94.2°C
Compound 1-347, m.p. 80.7°C
Compound 1-350, 2.41 (3H, q, J = 1.8 Hz), 4.05-4.35 (2H, b), 6.87-6.94 (1H, m), 7.03 (1H, d. J = 2.4 Hz), 7.33 (1H, d. J = 8.5 Hz), 7.98 (1H, s)
Compound 1-353, m.p. 124.0°C
Compound 1-367, 2.44 (3H, q, J = 1.9 Hz). 3.04 (3H, s), 6.88 (1H, s), 7.37 (1H, d, J = 9.0 Hz), 7.79 (1H, d, J = 7.0 Hz), 8.01 (1H, s)
Compound 1-369, 2.44 (3H, q, J = 2.0 Hz), 4.57 (2H, s), 7.06 (1H, s), 7.38 (1H, d, J = 9.0 Hz), 7.83 (1H, d, J = 6.9 Hz), 8.01 (1H, s)
Compound 1-391, m.p. 177.6°C
Compound 1-392, m.p. 172.5°C
Compound 1-398, m.p. 133.1 °C
Compound 1-420, 1.25 (3H, t, J = 7.5 Hz), 1.51 (3H, d, J = 7.0 Hz), 2.42 (3H, q, J = 1.8 Hz), 4.0-4.18 (3H, m), 4.82 (1H, d, J = 7.9 Hz), 6.59 (1H, d, J = 6.3 Hz), 7.23 (1H, d, J = 9.3 Hz), 7.97 (1H, s)
Compound 1-429, 1.40 (6H, d. J = 6.3 Hz), 2.43 (3H, q, J = 2.4 Hz), 4.52-4.63 (1H, m). 7.14 (0.5H, d. J = 2.4 Hz), 7.17 (0.5H, d, J = 2.4 Hz), 7.24 (1H, d. J = 3.6 Hz), 7.29 (1H, s), 7.46 (1H, d, J = 8.4 Hz)
Compound 1-439, m.p. 110.6°C
Compound 1-449, 1.29 (3H, t, J = 7.5 Hz), 2.42 (3H, q, J = 2.4 Hz), 4.28 (2H, q, J = 7.5 Hz). 4.73 (2H, s), 7.20-7.32 (2H, m), 7.49 (1H, d, J = 10.4 Hz), 8.00 (1H. s)
Compound 1-456, 1.70 (3H, d, J = 6.9 Hz), 2.41 (3H, q, J = 2.1 Hz), 3.77 (3H, s), 4.81 (1H, q, J = 6.9 Hz), 7.21-7.28 (2H, m), 7.47 (1H, d, J = 8.7 Hz), 7.99 (1H, s)
Compound 1-474, m.p. 110.6°C
Compound 1-475, 1.46 (3H, t, J = 5.8 Hz), 2.44 (3H, q, J = 1.5 Hz), 4.07 (2H, q, J = 5.8 Hz), 6.94 (1H, d, J = 5.0 Hz), 7.29 (1H, d, J = 7.5 Hz), 8.01 (1H, s)
Compound 1-476, 1.38 (6H, d, J = 6.3 Hz), 2.43 (3H, q, J = 2.0 Hz), 4.47 (1H, m), 6.99 (1H, d, J = 5.0 Hz), 7.29 (1H, d, J = 9.5 Hz), 8.00 (1H, s)
Compound 1-482, m.p. 79.8°C
Compound 1-483, m.p. 132.7°C
Compound 1-486, m.p. 140.7°C (decomp.)
Compound 1-487, m.p. 114.1°C
Compound 1-491, m.p. 82.9°C
Compound 1-495, m.p. 80.4°C
Compound 1-496, m.p. 102.0°C
Compound 1-497, m.p. 82.9°C
Compound 1-498, m.p. 75.6°C
Compound 1-499, 0.88 (3H, t, J = 7 Hz), 1.2-1.4 (4H, m), 1.55-1.70 (2H, m), 2.43 (3H, q, J = 2 Hz), 4.19 (2H, t, J = 7 Hz), 4.68 (2H, s), 6.98 (1H, d, J = 7 Hz), 7.33 (1H, d, J = 8 Hz), 7.99 (1H, s)
Compound 1-500, 1.26 (6H, d. J = 6.3 Hz), 2.43 (3H, q, J = 2 Hz), 4.65 (2H, s), S.OS-5.18 (1H, m), 6.98 (1H, d, J = 7 Hz), 7.33 (1H, d, J = 8 Hz), 7.98 (1H, s)
Compound 1-501, 1.5-1.9 (8H, m), 2.43 (3H, q. J = 2 Hz), 4.65 (2H, s), 5.2-5.4 (1H, m), 6.97 (1H, d, J = 7 Hz), 7.33 (1H. d, J = 8 Hz), 7.98 (1H, s)
Compound 1-503, 1.68 (3H, d, J = 7 Hz), 2.43 (3H, q, J = 2 Hz), 3.76 (3H, s), 4.73 (1H, q, J = 7 Hz), 6.98 (1H, d, J = 7 Hz), 7.32 (1H, d. J = 8 Hz), 7.99 (1H, s)
Compound 1-504. 1.25 (3H, t. J = 7.3 Hz), 1.68 (3H, d, J = 6.8 Hz), 2.42 (3H. q. J=2.0 Hz). 4.21 (2H, q, J = 7.3 Hz), 4.70 (1H, q, J = 6.8 Hz). 6.99 (1H, d, J = 6.8 Hz). 7.32 (1 H. d, J = 9.3 Hz), 7.98 (1H, s)
Compound 1-511, m.p. 110.7°C
Compound 1-518, m.p. 131.2°C (decomp.)
Compound 1-576, 2.43 (3H, q, J = 1.8 Hz). 3.67 (2H, s), 3.72 (3H, s), 7.32 (1H, d, J = 8.5 Hz), 7.59 (1H, d, J = 7.1 Hz), 8.2 (1H, s)
Compound 1-577, 1.15 (3H, t, J = 7.5 Hz), 2.36 (3H, q, J = 1.8 Hz), 3.58 (2H, s), 4.09 (2H, q, J = 7.5 Hz), 7.28 (1H, d, J = 8.6 Hz), 7.52 (1H, d. J = 7.1 Hz), 7.93 (1H, s)
Compound 1-579, m.p. 71.6°C
Compound 1-581, m.p. 97.5°C
Compound 1-584, 1.53 (3H, d, J = 7.2 Hz), 2.43 (3H, q, J = 1.8 Hz), 3.66 (3H, s), 3.88 (1H, q, J = 7.2 Hz), 7.38 (1H, d, J = 9.6 Hz), 7.66 (1H, d, J = 7.5 Hz), 7.99 (1H, s)
Compound 1-585, 1.17 (3H, t, J = 6.9 Hz), 1.53 (3H, d, J = 7.2 Hz), 2.43 (3H, q, J=1.8 Hz), 3.89 (1H, q, J = 7.2 Hz), 4.11 (2H, q, J = 6.9 Hz), 7.37 (1H, d, J = 9.6 Hz), 7.67 (1H, d, J = 9.0 Hz), 7.99 (1H, s)
Compound 1-586, 0.85 (3H, t, J = 6.8 Hz), 1.51-1.62 (5H, m), 2.43 (3H, q, J = 1.8 Hz), 3.89 (1H, q, J = 7.2 Hz), 4.02 (2H, t, J = 6.8 Hz), 7.33 (1H, d, J = 9.5 Hz). 7.66 (1H, d, J = 7.5 Hz), 7.98 (1H, s)
Compound 1-587. 0.88 (3H, t, J = 7.2 Hz), 1.30-1.40 (2H, m), 1.47-1.55 (5H, m), 2.43 (3H, q, J = 1.8 Hz), 3.89 (1H, q, J = 7.1 Hz), 4.02-4.08 (2H, m), 7.36 (1H, d, J = 9.4 Hz). 7.66 (1H, d. J = 7.5 Hz). 7.99 (1H, s)
Compound 1-619, 2.45 (3H, q, J = 1.6 Hz), 7.60 (1H, d, J = 8.6 Hz), 7.80-7.86 (1H, m), 8.04 (1H, s). 8.36 (1H, d. J = 2.5 Hz)
Compound 1-621, 1.40 (3H, t. J = 7.1 Hz), 2.43 (3H, q, J = 1.8 Hz), 4.41 (2H, q. J = 7.1 Hz), 7.56 (1H, d. J = 8.6 Hz), 7.72-7.78 (1H, m), 8.02 (1H, s), 8.16 (1H, d. J = 2.7 Hz)
Compound 1-625, 1.39 (6H, d, J = 6.2 Hz), 2.43 (3H, q, J = 1.6 Hz), 5.23-5.28 (1H, m), 7.54 (1H, d, J = 8.7 Hz), 7.70-7.76 (1H, m), 8.02 (1H, s), 8.10 (1H, d. J = 2.6 Hz)
Compound 1-632, m.p. 76.1 °C
Compound 1-637, m.p. 102.8°C
Compound 1-641, 2.43 (3H, q, J = 2.0 Hz), 3.92 (3H, s), 7.39 (1H, d, J = 9.5 Hz), 8.02 (1H, s), 8.07 (1H, d, J = 7.7 Hz)
Compound 1-642, 1.39 (3H, t, J = 7.2 Hz), 2.44 (3H, q, J = 1.9 Hz), 4.40 (2H, q. J = 7.2 Hz), 7.38 (1H, d, J = 9.5 Hz), 8.00-8.06 (2H, m)
Compound 1-981, m.p. 87.1°C
Compound 1-987, 4.0-4.4 (2H, b), 6.8-6.9 (1H, m), 7.04 (1H, d, J = 2.4 Hz), 7.28 (1H, q. J = 1.2 Hz), 7.35 (1H, d, J = 8.6 Hz), 8.02 (1H, d, J = 2.2 Hz)
Compound 1-1025, 5.92 (1H, s), 7.16 (0.5H. d, J = 2.4 Hz), 7.19 (0.5H, d, J = 2.4 Hz); 7.30 (1H, q, J = 1.1 Hz), 7.34 (1H, d, J = 5.7 Hz), 7.43 (1H, d, J = 9.0 Hz), 8.04 (1H, q, J = 3.0 Hz)
Compound 1-1028, m.p. 180.2°C, 5.65-5.9 (1H, br), 7.09 (1H, d, J = 7 Hz), 7.27-7.30 (2H, m), 8.10 (1H, q, J = 2.2 Hz)
Compound 1-1029, 7.09 (1H, d, J = 6.4 Hz), 7.31 (1H, q, J = 1.1 Hz), 7.42 (1H, d, J = 8.8 Hz), 8.04 (1H, q, J = 2.2 Hz)
Compound 1-1035, m.p. 61.1°C
Compound 1-1057, m.p. 158°C
Compound 1-1066, m.p. 89.1°C
Compound 1-1076, m.p. 113.5°C
Compound 1-1086, m.p. 83.9°C
Compound 1-1093, m.p. 83.1°C
Compound 1-1113. m.p. 68.6°C
Compound 1-1123. m.p. 147.4°C
Compound 1-1124, m.p. 117.2°C
Compound 1-1133, m.p. 149.2°C (decomp.)
Compound 1-1140, m.p. 99.1 °C
Compound 1-1141, m.p. 80.2°C
Compound 1-1213, m.p. 85.8°C
Compound 1-1214, m.p. 65.1 °C
Compound 1-1221, 1.54 (3H, d, J = 7.2 Hz), 3.66 (3H, s), 3.90 (1H, q, J = 7.2 Hz), 7.31 (1H, s), 7.39 (1H, d, J = 9.0 Hz), 7.67 (1H, d. J = 8.7 Hz), 8.04 (1H, d. J = 3.6 Hz)
Compound 1-1222, 1.16 (3H, t. J = 5.1 Hz), 1.53 (3H, d, J = 7.2 Hz), 3.89 (1H, q, J = 7.2 Hz), 4.10 (2H, q, J = 5.1 Hz), 7.30 (1H, q, J = 1. 1 Hz). 7.38 (1H. d. J = 9.0 Hz), 7.68 (1H, d, J = 7.5 Hz), 8.04 (1H, q, J = 2.2 Hz)
Compound 1-1226, 1.12 (3H, d, J = 6.0 Hz), 1.21 (3H, d, J = 6.0 Hz), 1.52 (3H. d, J = 3.0 Hz), 3.88 (1H, q, J = 3.0 Hz), 4.85-5.03 (1H, m), 7.30 (1H, q, J = 1.8 Hz). 7.37 (1H, d, J = 9.0 Hz), 7.67 (1H, d, J = 7.5 Hz), 8.02 (1H, q, J = 2.1 Hz)
Compound 1-1256. 7.35 (1H, q, J = 1.1 Hz), 7.62 (1H, d, J = 8.7 Hz), 7.82-7.88 (1H, m). 8.09 (1H, d, J = 2.2 Hz), 8.35 (1H, d, J = 2.6 Hz)
Compound 1-1258, 1.41 (3H, t, J = 7.1 Hz), 4.42 (2H, q, J = 7.1 Hz), 7.31 (1H, s), 7.57 (1H, d. J = 8.7 Hz), 7.74-7.79 (1H, m), 8.07 (1H, q, J = 2.1 Hz), 8.16 (1H, d, J = 2.6 Hz)
Compound 1-1269, m.p. 89.7°C
Compound 1-1274, m.p. 154.2°C
Compound 1-1278, m.p. 128.6°C
Compound 1-1279, 1.40 (3H, t, J = 7.1 Hz), 4.40 (2H, q, J = 7.1 Hz), 7.33 (1H, q, J = 1.1 Hz). 7.39 (1H, d, J = 9.4 Hz). 8.05 (1H, d, J = 8.3 Hz), 8.07 (1H, s)
Compound 1-1346, 2.48 (3H, s). 5.66 (1H, s), 7.08 (1H, d, J = 7.8 Hz), 7.28 (1H, d. J = 9.0 Hz). 7.32 (1H, s)
Compound 1-1431, m.p. 74.5°C
Compound 1-1441, m.p. 128.2°C
Compound 1-1442, 1.73 (3H, d, J = 6.6 Hz), 2.49 (3H, q, J = 1.3 Hz), 2.54 (1 H. d, J = 2.0 Hz), 4.84 (1H, m). 7.22 (1H, d, J = 6.5 Hz), 7.28-7.34 (2H, m)
Compound 1-1451, 1.29 (3H, t, J = 7.0 Hz), 2.47 (3H, q, J = 1.4 Hz), 4.27 (2H, q, J = 7.0 Hz), 4.68 (2H, s), 6.99 (1H, d, J = 7.1 Hz), 7.32 (1H, s), 7.34 (1H, d, J = 7.2 Hz)
Compound 1-1458, 1.69 (3H, d. J = 6.8 Hz), 2.48 (3H, q, J = 1.3 Hz), 3.76 (3H, s). 4.74 (1H, q, J = 6.8 Hz), 7.01 (1H, d, J = 6.5 Hz), 7.29-7.34 (2H, m)
Compound 1-1540, 1.17 (3H, t, J = 7.0 Hz), 1.54 (3H, d, J = 7.3 Hz), 2.48 (3H, q, J = 1.4 Hz), 3.89 (1H, q, J = 7.3 Hz), 4.11 (2H, q, J = 7.0 Hz), 7.31 (1H, s), 7.37 (1H, d, J = 9.5 Hz), 7.67 (1H, d, J =7.5 Hz)
Compound 1-1617, m.p. 105.7°C
Compound 1-1622, 1.27 (3H, t, J = 7.0 Hz), 2.42 (3H, q, J = 2.0 Hz), 3.26 (0.3H, d, J = 7.6 Hz), 3.32 (0.7H, d, J = 7.6 Hz), 3.49 (0.7H, d, J = 7.6 Hz), 3.54 (0.3H, d, J = 7.6 Hz), 4.23 (2H, q, J = 7.0 Hz), 4.54 (0.5H, d, J = 7.6 Hz), 4.57 (0.5 H, d, J = 7.6 Hz), 7.34 (1H, d, J = 9.3 Hz), 7.40 (1H, d, J = 7.5 Hz), 8.00 (1H, s)
Compound 1-1627, m.p. 182.2°C
Compound 1-1638, 3.66 (2H, s), 7.31 (1H, s), 7.34 (1H, d, J = 9.3 Hz), 7.62 (1H, d, J = 7.2 Hz), 8.08 (1H, s)
Compound 1-1639, m.p. 158.9°C (decomp.)
Compound 1-1641, 1.55 (3H, d, J = 7.2 Hz), 3.88 (1H. q, J = 7.2 Hz), 7.32 (1H, s), 7.37 (1H, d. J = 9.2 Hz), 7.69 (1H, d, J = 7.1 Hz), 8.03 (1H, s)
Compound 1-1650, 1.11 (3H, t, J = 7.5 Hz), 2.03-2.12 (2H, m), 2.43 (3H, q, J = 1.8 Hz), 3.75 (3H, s). 4.58 (1H, t, J = 7.5 Hz), 6.92 (1H, d, J = 8.2 Hz), 7.32 (1H, d, J = 9.3 Hz), 8.00 (1H, s)
Compound 1-1655, m.p. 119.7°C. 1.39 (3H, t, J = 7.1 Hz), 2.43 (3H, q. J = 1.8 Hz), 4.26 (2H, q, J = 7.1 Hz), 4.68 (2H, s). 6.91 (1H, s), 7.60 (1H, s), 7.99 (1H. s)
Compound 1-1663. m.p. 136.2°C, 2.44 (3H, q, J = 1.8 Hz), 2.58 (1H, t. J = 2.3 Hz), 4.78 (2H, d, J = 2.3 Hz), 7.12 (1H, s), 7.59 (1H, s), 8.01 (1H, s)
Compound 1-1665, 1.23 (3H, t. J = 6.9 Hz), 1.68 (3H, d, J = 6.8 Hz), 2.42 (3H, q, J = 1.8 Hz), 4.1-4.3 (2H, m), 4.72 (1H, q, J = 6.8 Hz), 6.90 (1H, s), 7.58 (1H, s), 7.97 (1H, s)
Compound 1-1670, m.p. 118.1 °C
Compound 1-1673, m.p. 107.2°C
Compound 1-1678, m.p. 164.7°C
Compound 1-1679, 1.73 (3H, d, J = 6.9 Hz), 2.54 (1H, d, J = 2.1 Hz), 4.73-4.90 (1H, m). 7.20 (1H, d. J = 6.3 Hz), 7.30 (1H, s), 7.49 (1H, d, J = 8.7 Hz), 8.00 (1H, s)
Compound 1-1680, m.p. 90.1°C
Compound 1-1681, m.p. 148.1°C
Compound 1-1682, m.p. 107.0°C
Compound 1-1683, 1.25 (3H, t, J = 7.2 Hz), 1.68 (3H, d, J = 6.8 Hz), 2.42 (3H, q, J = 1.5 Hz), 4.13-4.26 (2H, m), 4.70 (1H, q, J = 6.8 Hz), 6.96 (1H, d, J = 6.3 Hz). 7.48 (1H, d, J = 9.0 Hz), 7.97 (1H, s)
Compound 1- 1687, m.p. 200.1°C
Compound 1-1689, m.p. 76.3°C
Compound 1-1690, m.p. 196.1°C
Compound 1-1691, 3.06 (3H, s), 7.10-7.30 (1H, b), 7.30 (1H, s), 7.64 (1H, s), 7.74 (1H, s), 8.06 (1H, q. J = 2.1 Hz)
Compound 1 -1701, 1.39 (3H, t, J = 7.1 Hz), 4.40 (2H, q, J = 7.1 Hz), 7.33 (1H, q, J = 1, Hz), 7.69 (1H, s), 7.97 (1H. s), 8.06 (1H, q, J = 2.2 Hz)
Compound 1-1718, m.p. 63.9°C
Compound 1-1719, m.p. 189.5°C
Compound 1-1720, m.p. 117.3°C
Compound 1-1721, m.p. 156.1°C
Compound 1-1732, 2.47 (3H, q, J = 1.8 Hz), 3.05 (3H, s), 7.15-7.30 (1H, b), 7.66 (1H, s), 7.78 (1H, s), 8.03 (1H, s)
Compound 1-1732, 1.38 (3H, t, J = 7.1 Hz), 2.44 (3H, q, J = 1.8 Hz), 4.39 (2H, q, J = 7.1 Hz), 7.68 (1H, s), 7.96 (1H, s), 8.02 (1H, s)
Compound 1-1748, 1.38 (6H, d, J = 6.0 Hz), 2.44 (3H, q, J = 1.9 Hz), 4.40-4.59 (1H, m), 6.95 (1H, s), 7.55 (1H, s), 8.00 (1H, s)
Compound 1-1780, m.p. 76.4°C
Compound 1-1781, 3.51 (3H, s), 3.51 (3H, s), 7.30 (1H, q, J = 1.2 Hz), 7.59 (1H, d, J = 6.7 Hz). 7.65 (1H, d, J = 9.0 Hz), 8.06 (1H, d, J = 2.1 Hz)
Compound 1-1782, 3.03 (3H, s), 7.09 (1H, s), 7.32 (1H, q, J = 1.0 Hz), 7.53 (1H, d. J = 8.7 Hz), 7.77 (1H, d, J = 6.8 Hz), 8.07 (1H. q, J = 2.2 Hz)
Compound 1-1783, 1.38 (6H, d, J = 6.1 Hz), 2.43 (3H, q, J = 1.7 Hz), 4.4-4.6 (1H, m), 6.95 (1H, d, J = 6.4 Hz), 7.47 (1H, d, J = 8.9 Hz), 8.00 (1H, s)
Compound 1-1785, 2.43 (3H, q, J = 1.9 Hz), 3.03 (3H, s), 7.03 (1H, s), 7.52 (1H, d, J = 8.8 Hz), 7.76 (1H. d, J = 6.9 Hz), 8.02 (1H, s)
Compound 1-1789, m.p. 78.3°C
Compound 1-1790, m.p. 63.2°C
Compound 1-1879. 2.42 (3H, q, J = 1.8 Hz), 5.38 (2H, s), 7.28-7.47 (5H, m). 7.56 (1H, d, J = 8.7 Hz), 7.72-7.79 (1H, m), 8.00 (1H, s). 8.18 (1H, d, J = 2.5 Hz)
Compound 1-1881, 2.20 (0.75H, s). 2.24 (2.25H, s), 2.42 (3H, q, J = 1.9 Hz). 3.82 (0.75H. s), 3.98 (2.25H, s), 7.47-7.68 (3H, m), 8.00 (1H, s)
Compound 1-1901, m.p. 99.2°C
Compound 1-1908, m.p. 77.6°C
Compound 1-1910, m.p. 75.3°C
Compound 1-1930, m.p. 139.7°C
Compound 1-3051, 1.25 (3H, t. J = 7.2 Hz). 2.42 (3H, q, J = 1.9 Hz), 4.17 (2H. q, J = 7.2 Hz). 4.71 (2H, s), 4.82 (2H, s), 7.10 (1H, d, J = 6.3 Hz), 7.33 (1H, d, J = 9.1 Hz), 7.99 (1H, s)
Compound 1-2054, 1.24 (3H, t. J = 7.1 Hz), 1.51 (3H, d, J = 7.2 Hz), 2.42 (3H, q, J = 1.8 Hz), 4.15 (2H, q, J = 7.1 Hz), 4.78 (2H, s), 5.19 (1H, q, J = 7.2 Hz). 7.08 (1H. d, J = 6.3 Hz), 7.32 (1H, d, J = 9.1 Hz), 7.98 (1H, s)
Compound 2-203, 2.3-2.4 (1H, m), 2.35 (3H, q, J = 1.9 Hz), 4.5-4.7 (4H, m). 7.00 (1H, d. J = 6.5 Hz), 7.19 (1H, m), 7.39 (1H, d, J = 2.5 Hz), 7.95 (1H, s)
Compound 2-251, m.p. 168.3°C
Compound 2-328, 0.90 (3H, t, J = 7.3 Hz), 1.54 (3H, d, J = 7.0 Hz), 1.70-1.90 (2H, m), 2.46 (3H, m), 4.50 (1H, m), 7.18 (1H, d, J = 5.75 Hz), 7.35 (1H, d, J = 8.8 Hz), 8.04 (1H, s)
Compound 2-583, m.p. 149. 1°C
Compound 2-631, m.p. 168.3°C
Compound 2-708, 0.90 (3H, t, J = 7.3 Hz), 1.55 (3H, d, J = 7.0 Hz), 1.75-1.95 (2H. m), 4.50 (1H, m), 7.22 (1H, d, J = 5.8 Hz), 7.30-7.40 (2H, m), 8.08 (1H, q, J = 2.2 Hz)
Compound 2-821, m.p. 162.7°C
Compound 3-139, m.p. 88.2°C
Compound 4-434. 1.67 (3H, s), 2.35 (3H, q, J = 1.7 Hz), 3.0-3.2 (1H, m), 3.4-3.7 (1H, m). 3.71 (3H, s), 7.03 (1H, d, J = 5.0 Hz), 7.97 (1H, q, J = 3.3 Hz)
Compound 4-451, 1.53 (6H, s), 2.43 (3H, q, J = 1.9 Hz), 2.96 (1H, d, J = 16.2 Hz), 3.08 (1H, d, J = 16.2 Hz), 7.07 (1H, d, J = 9.9 Hz), 7.99 (1H, s)
Compound 4-452, 1.4-1.5 (3H, m), 2.43 (3H, q, J = 2.0 Hz), 2.7-3.0 (1H, m). 3.1-3.5 (1H, m), 3.5-3.8 (2H, m), 7.07 (1H. d. J = 10.0 Hz), 8.00 (1H, q, J = 2.5 Hz)

The following will describe formulation examples, in which the present compounds are designated by their compound numbers shown in Tables 1 to 4 and parts are by weight.

### Formulation Example 1

Fifty parts of each of compounds 1-1 to 1-2157, 2-1 to 2-950, 3-1 to 3-582, and 4-1 to 4-936, 3 parts of calcium ligninsulfonate, 2 parts of sodium laurylsulfate, and 45 parts of synthetic hydrated silicon oxide are well pulverized and mixed to give a wettable powder for each compound.

### Formulation Example 2

Ten parts of each of compounds 1-1 to 1-2157, 2-1 to 2-950, 3-1 to 3-582, and 4-1 to 4-936, 14 parts of polyoxyethylene styryl phenyl ether, 6 parts of calcium dodecylbenzenesulfonate, 35 parts of xylene, and 35 parts of cyclohexanone are well mixed to give an emulsifiable concentrate for each compound.

### Formulation Example 3

Two parts of each of compounds 1-1 to 1-2157, 2-1 to 2-950, 3-1 to 3-582, and 4-1 to 4-936, 2 parts of synthetic hydrated silicon oxide, 2 parts of calcium ligninsulfonate, 30 parts of bentonite, and 64 parts of kaoline clay are well pulverized and mixed, to which water is added, and the mixture is well kneaded, granulated, and dried to give a granule for each compound.

### Formulation Example 4

Twenty-five parts of each of compounds 1-1 to 1-2157, 2-1 to 2-950, 3-1 to 3-582, and 4-1 to 4-936, 50 parts of 10% aqueous polyvinyl alcohol solution, and 25 parts of water are mixed, and the mixture is pulverized until the average particle size becomes 5 µm or less to give a flowable for each compound.

### Formulation Example 5

Five parts of compound 1-1650 is added to 40 parts of 10% aqueous polyvinyl alcohol solution and dispersed by emulsion with a homogenizer until the mean particle size becomes 10 µm or less, to which 55 parts of water is added to give a concentrated emulsion.

The following test examples will demonstrate that the present compounds are useful as active ingredients of herbicides. The present compounds are designated by their compound numbers shown in Tables 1 to 4.

The herbicidal activity and phytotoxicity were evaluated at 6 levels with indices of 0 to 5, i.e., designated by the numeral "0", "1", "2", "3", "4" or "5", wherein "0" means that there was no or little difference in the degree of germination or growth between the treated and the untreated test plants at the time of examination, and "5" means that the test plants died complete or their germination or growth was completely inhibited. The herbicidal activity is excellent when rated at "4" or "5" but insufficient when rated at "3" or lower. The phytotoxicity is no problematic on practical use when rated at "0" or "1" but not allowed when rated at "2" or higher.

### Test Example 1: Foliar treatment on upland fields

Cylindrical plastic pots of 10 cm in diameter and 10 cm in depth were filled with soil, in which the seeds of entireleaf morningglory (*Ipomoea hederacea* var. *integriuscula)* and velvetleaf *(Abutilon theophrasti*) were sowed, and the test plants were grown in a greenhouse for 19 days. Each of the test compounds listed below was formulated into an emulsifiable concentrate according to Formulation Example 2, which was diluted with water containing a spreading agent to a prescribed concentration. The dilution was uniformly sprayed over the foliage of the test plants with a sprayer at a volume of 1000 liters per hectare. After the application, the test plants were grown in the greenhouse for 19 days, and the herbicidal activity was examined. The results are shown in Table 5.

**TABLE 5**

| Test compound | Application amount of active ingredient (g/ha) | Herbicidal activity | |
|---|---|---|---|
| | | Entireleaf morningglory | Velvetleaf |
| 1-332 | 500 | 5 | 5 |
| 1-335 | 500 | 5 | 5 |
| 1-338 | 500 | 5 | 5 |
| 1-347 | 500 | 5 | 5 |
| 1-350 | 500 | 5 | 5 |
| 1-353 | 500 | 5 | 5 |
| 1-367 | 500 | 5 | 5 |
| 1-369 | 500 | 5 | 5 |
| 1-391 | 500 | 5 | 5 |
| 1-392 | 500 | 5 | 5 |
| 1-398 | 500 | 5 | 5 |
| 1-420 | 500 | 5 | 5 |
| 1-429 | 500 | 5 | 5 |
| 1-439 | 500 | 5 | 5 |
| 1-449 | 500 | 5 | 5 |
| 1-456 | 500 | 5 | 5 |
| 1-474 | 500 | 5 | 5 |
| 1-475 | 500 | 5 | 5 |
| 1-476 | 500 | 5 | 5 |
| 1-482 | 500 | 5 | 5 |
| 1-483 | 500 | 5 | 5 |
| 1-486 | 500 | 5 | 5 |
| 1-487 | 500 | 5 | 5 |
| 1-491 | 500 | 5 | 5 |
| 1-495 | 500 | 5 | 5 |
| 1-496 | 500 | 5 | 5 |
| 1-497 | 500 | 5 | 5 |
| 1-498 | 500 | 5 | 5 |
| 1-499 | 500 | 5 | 5 |
| 1-500 | 500 | 5 | 5 |
| 1-501 | 500 | 5 | 5 |
| 1-503 | 500 | 5 | 5 |
| 1-504 | 500 | 5 | 5 |
| 1-511 | 500 | 5 | 5 |
| 1-576 | 500 | 5 | 5 |
| 1-577 | 500 | 5 | 5 |
| 1-579 | 500 | 5 | 5 |
| 1-581 | 500 | 5 | 5 |
| 1-584 | 500 | 5 | 5 |
| 1-585 | 500 | 5 | 5 |
| 1-586 | 500 | 5 | 5 |
| 1-587 | 500 | 5 | 5 |
| 1-619 | 500 | 5 | 5 |
| 1-621 | 500 | 5 | 5 |
| 1-625 | 500 | 5 | 5 |
| 1-637 | 500 | 5 | 5 |
| 1-641 | 500 | 5 | 5 |
| 1-642 | 500 | 5 | 5 |
| 1-987 | 500 | 5 | 5 |
| 1-1025 | 500 | 5 | 5 |
| 1-1028 | 500 | 5 | 5 |
| 1-1029 | 500 | 5 | 5 |
| 1-1035 | 500 | 5 | 5 |
| 1-1057 | 500 | 5 | 5 |
| 1-1066 | 500 | 5 | 5 |
| 1-1076 | 500 | 5 | 5 |
| 1-1086 | 500 | 5 | 5 |
| 1-1093 | 500 | 5 | 5 |
| 1-1113 | 500 | 5 | 5 |
| 1-1123 | 500 | 5 | 5 |
| 1-1124 | 500 | 5 | 5 |
| 1-1133 | 500 | 5 | 5 |
| 1-1140 | 500 | 5 | 5 |
| 1-1141 | 500 | 5 | 5 |
| 1-1213 | 500 | 5 | 5 |
| 1-1214 | 500 | 5 | 5 |
| 1-1221 . | 500 | 5 | 5 |
| 1-1222 | 500 | 5 | .5 |
| 1-1226 | 500 | 5 | 5 |
| 1-1274 | 500 | 5 | 5 |
| 1-1278 | 500 | 5 | 5 |
| 1-1279 | 500 | 5 | 5 |
| 1-1422 | 500 | 5 | 5 |
| 1-1431 | 500 | 5 | 5 |
| 1-1441 | 500 | 5 | 5 |
| 1-1451 | 500 | 5 | 5 |
| 1-1458 | 500 | 5 | 5 |
| 1-1540 | 500 | 5 | 5 |
| 1-1617 | 500 | 5 | 5 |
| 1-1622 | 500 | 5 | 5 |
| 1-1627 | 500 | 5 | 5 |
| 1-1638 | 500 | 5 | 5 |
| 1-1639 | 500 | 5 | 5 |
| 1-1641 | 500 | 5 | 5 |
| 1-1650 | 500 | 5 | 5 |
| 1-1655 | 500 | 5 | 5 |
| 1-1663 | 500 | 5 | 5 |
| 1-1665 | 500 | 5 | 5 |
| 1-1670 | 500 | 5 | 5 |
| 1-1673 | 500 | 5 | 5 |
| 1-1678 | 500 | 5 | 5 |
| 1-1679 | 500 | 5 | 5 |
| 1-1680 | 500 | 5 | 5 |
| 1-1681 | 500 | 5 | 5 |
| 1-1682 | 500 | 5 | 5 |
| 1-1683 | 500 | 5 | 5 |
| 1-1691 | 500 | 5 | 5 |
| 1-1701 | 500 | 5 | 5 |
| 1-1718 | 500 | 5 | 5 |
| 1-1719 | 500 | 5 | 5 |
| 1-1722 | 500 | 5 | 5 |
| 1-1732 | 500 | 5 | 5 |
| 1-1748 | 500 | 5 | 5 |
| 1-1780 | 500 | 5 | 5 |
| 1-1781 | 500 | 5 | 5 |
| 1-1782 | 500 | 5 | 5 |
| 1-1783 | 500 | 5 | 5 |
| 1-1785 | 500 | 5 | 5 |
| 2-203 | 500 | 5 | 5 |
| 2-251 | 500 | 5 | 5 |
| 2-328 | 500 | 5 | 5 |
| 2-583 | 500 | 5 | 5 |
| 2-631 | 500 | 5 | 5 |
| 2-708 | 500 | 5 | 5 |
| 2-821 | 500 | 5 | 5 |
| 3-139 | 500 | 5 | 5 |
| 4-434 | 500 | 5 | 5 |
| 4-451 | 500 | 5 | 5 |
| 1-344 | 2000 | 5 | 5 |
| 1-981 | 2000 | 5 | 5 |
| 1-1689. | 2000 | 5 | 5 |
| 1-1720 | 2000 | 5 | 5 |
| 1-1721 | 2000 | 5 | 5 |

### Test Example 2: Foliar treatment on upland fields

Cylindrical plastic pots of 10 cm in diameter and 10 cm in depth were filled with soil, in which the seeds of barnyardgrass *(Echinochloa crus-galli),* entireleaf morningglory *(Ipomoea hederacea* var. *integriuscula),* and velvetleaf *(Abutilon theophrasti)* were sowed, and the test plants were grown in a greenhouse for 19 days. Each of the test compounds listed below was formulated into an emulsifiable concentrate according to Formulation Example 2, which was diluted with water containing a spreading agent to a prescribed concentration. The dilution was uniformly sprayed over the foliage of the test plants with a sprayer at a volume of 1000 liters per hectare. After the application, the test plants were grown in the greenhouse for 19 days, and the herbicidal activity was examined. The results are shown in Table 6.

**TABLE 6**

| Test compound | Application amount of active ingredient (g/ha) | Herbicidal activity | | |
|---|---|---|---|---|
| | | Barnyardgrass | Entireleaf morningglory | Velvetleaf |
| 1-369 | 32 | 5 | 5 | 5 |
| 1-420 | 32 | 5 | 5 | 5 |
| 1-439 | 32 | 4 | 5 | 5 |
| 1-482 | 32 | 5 | 5 | 5 |
| 1-486 | 32 | 4 | 5 | 5 |
| 1-487 | 32 | 5 | 5 | 5 |
| 1-491 | 32 | 5 | 5 | 5 |
| 1-495 | 32 | 5 | 5 | 5 |
| 1-496 | 32 | 5 | 5 | 5 |
| 1-499 | 32 | 5 | 5 | 5 |
| 1-503 | 32 | 5 | 5 | 5 |
| 1-576 | 32 | 5 | 5 | 5 |
| 1-577 | 32 | 5 | 5 | 5 |
| 1-579 | 32 | 5 | 5 | 5 |
| 1-581 | 32 | 5 | 5 | 5 |
| 1-584 | 32 | 5 | 5 | 5 |
| 1-585 | 32 | 5 | 5 | 5 |
| 1-625 | 32 | 5 | 5 | 5 |
| 1-641 | 32 | 5 | 5 | 5 |
| 1-642 | 32 | 5 | 5 | 5 |
| 1-1057 | 32 | 5 | 5 | 5 |
| 1-1076 | 32 | 4 | 5 | 5 |
| 1-1123 | 32 | 4 | 5 | 5 |
| 1-1124 | 32 | 5 | 5 | 5 |
| 1-1140 | 32 | 5 | 5 | 5 |
| 1-1141 | 32 | 4 | 5 | 5 |
| 1-1213 | 32 | 5 | 5 | |
| 1-1214 | 32 | 5 | 5 | 5 |
| 1-1221 | 32 | 5 | 5 | 5 |
| 1-1222 | 32 | 5 | 5 | 5 |
| 1-1226 | 32 | 5 | 5 | 5 |
| 1-1279 | 32 | 5 | 5 | 5 |
| 1-1422 | 32 | 5 | 5 | 5 |
| 1-1431 | 32 | 5 | 5 | 5 |
| 1-1441 | 32 | 5 | 5 | -5 |
| 1-1458 | 32 | 5 | 5 | 5 |
| 1-1540 | 32 | 5 | 5 | 5 |
| 1-1665 | 32 | 5 | 5 | 5 |
| 1-1670 | 32 | 5 | 5 | 5 |
| 1-1673 | 32 | 5 | 5 | 5 |
| 1-1678 | 32 | 5 | 5 | 5 |
| 1-1679 | 32 | 5 | 5 | 5 |
| 1-1680 | 32 | 5 | 5 | 5 |
| 1-1681 | 32 | 5 | 5 | 5 |
| 1-1682 | 32 | 5 | 5 | 5 |
| 1-1683 | 32 | 5 | 5 | 5 |
| 1-1701 | 32 | 5 | 5 | 5 |
| 1-1732 | 32 | 5 | 5 | 5 |
| 1-1780 | 32 | 5 | 5 | 5 |
| 1-1783 | 32 | 5 | 5 | 5 |
| 2-203 | 32 | 4 | 5 | 5 |
| 2-251 | 32 | 5 | 5 | 5 |
| 2-583 | 32 | 4 | 5 | 5 |
| 2-631 | 32 | 5 | 5 | 5 |
| 2-821 | 32 | 5 | 5 | 5 |
| 4-434 | 32 | 5 | 5 | 5 |

### Test Example 3: Soil surface treatment on upland fields

Cylindrical plastic pots of 10 cm in diameter and 10 cm in depth were filled with soil, in which the seeds of entireleaf morningglory *(Ipomoea hederacea* var. *integriuscula)* and velvetleaf *(Abutilon theophrasti)* were sowed. Each of the test compounds listed below was formulated into an emulsifiable concentrate according to Formulation Example 2, which was diluted with water to a prescribed concentration. The dilution was uniformly sprayed over the soil surface in the pots with a sprayer at a volume of 1000 liters per hectare. After the application, the test plants were grown in a greenhouse for 19 days, and the herbicidal activity was examined. The results are shown in Table 7.

**TABLE 7**

| Test compound | Application amount of active ingredient (g/ha) | Herbicidal activity | |
|---|---|---|---|
| | | Entireleaf morningglory | Velvetleaf |
| 1-347 | 500 | 5 | 5 |
| 1-332 | 500 | 5 | 5 |
| 1-335 | 500 | 5 | 5 |
| 1-338 | 500 | 5 | 5 |
| 1-353 | 500 | 5 | 5 |
| 1-367 | 500 | 5 | 5 |
| 1-369 | 500 | 5 | 5 |
| 1-391 | 500 | 5 | 5 |
| 1-392 | 500 | 5 | 5 |
| 1-398 | 500 | 5 | 5 |
| 1-420 | 500 | 5 | 5 |
| 1-439 | 500 | 5 | 5 |
| 1-449 | 500 | 5 | 5 |
| 1-456 | 500 | 5 | 5 |
| 1-474 | 500 | 5 | 5 |
| 1-475 | 500 | 5 | 5 |
| 1-476 | 500 | 5 | 5 |
| 1-482 | 500 | 5 | 5 |
| 1-486 | 500 | 5 | 5 |
| 1-487 | 500 | 5 | 5 |
| 1-491 | 500 | 5 | 5 |
| 1-495 | 500 | 5 | 5 |
| 1-496 | 500 | 5 | 5 |
| 1-497 | 500 | 5 | 5 |
| 1-498 | 500 | 5 | 5 |
| 1-499 | 500 | 5 | 5 |
| 1-500 | 500 | 5 | 5 |
| 1-501 | 500 | 5 | 5 |
| 1-503 | 500 | 5 | 5 |
| 1-504 | 500 | 5 | 5 |
| 1-511 | 500 | 5 | 5 |
| 1-576 | 500 | 5 | 5 |
| 1-577 | 500 | 5 | 5 |
| 1-579 | 500 | 5 | 5 |
| 1'-581 | 500 | 5 | |
| 1-584 | 500 | 5 | 5 |
| 1-585 | 500 | 5 | 5 |
| 1-587 | 500 | 5 | 5 |
| 1-621 | 500 | 5 | 5 |
| 1-625 | 500 | 5 | 5 |
| 1-641 | 500 | 5 | 5 |
| 1-642 | 500 | 5 | 5 |
| 1-1028 | 500 | 5 | 5 |
| 1-1029 | 500 | 5 | 5 |
| 1-1035 | 500 | 5 | 5 |
| 1-1057 | 500 | 5 | 5 |
| 1-1066 | 500 | 5 | 5 |
| 1-1076 | 500 | 5 | 5 |
| 1-1093 | 500 | 5 | 5 |
| 1-1113 | 500 | 5 | 5 |
| 1-1123 | 500 | 5 | 5 |
| 1-1124 | 500 | 5 | 5 |
| 1-1133 | 500 | 5 | 5 |
| 1-1140 | 500 | 5 | 5 |
| 1-1141 | 500 | 5 | 5 |
| 1-1213 | 500 | 5 | 5 |
| 1-1214 | 500 | 5 | 5 |
| 1-1221 | 500 | 5 | 5 |
| 1-1222. | 500 | 5 | 5 |
| 1-1226 | 500 | 5 | 5 |
| 1-1279 | 500 | 5 | 5 |
| 1-1422 | 500 | 5 | 5 |
| 1-1431 . | 500 | 5 | 5 |
| 1-1441 | 500 | 5 | 5 |
| 1-1451 | 500 | 5 | 5 |
| 1-1458 | 500 | 5 | 5 |
| 1-1540 | 500 | 5 | 5 |
| 1-1617 | 500 | 5 | 5 |
| 1-1622 | 500 | 5 | 5 |
| 1-1627 | 500 | 5 | 5 |
| 1-1638 | 500 | 5 | 5 |
| 1-1639 | 500 | 5 | 5 |
| 1-1641 | 500 | 5 | 5 |
| 1-1650 | 500 | 5 | 5 |
| 1-1655 | 500 | 5 | 5 |
| 1-1663 | 500 | 5 | 5 |
| 1-1665 | 500 | 5 | 5 |
| 1-1670 | 500 | 5 | 5 |
| 1-1673 | 500 | 5 | 5 |
| 1-1678 | 500 | 5 | 5 |
| 1-1679 | 500 | 5 | 5 |
| 1-1680 | 500 | 5 | 5 |
| 1-1681 | 500 | 5 | 5 |
| 1-1682 | 500 | 5 | 5 |
| 1-1683 | 500 | 5 | 5 |
| 1-1691 | 500 | 5 | 5 |
| 1-1780 | 500 | 5 | 5 |
| 1-1781 | 500 | 5 | 5 |
| 1-1782 | 500 | 5 | 5 |
| 1-1783 | 500 | 5 | 5 |
| 1-1785 | 500 | 5 | 5 |
| 2-203 | 500 | 5 | 5 |
| 2-251 | 500 | 5 | 5 |
| 2-328 | 500 | 5 | 5 |
| 2-583 | 500 | 5 | 5 |
| 2-631 | 500 | 5 | 5 |
| 2-708 | 500 | 5 | 5 |
| 2-821 | 500 | 5 | 5 |
| 4-434 | 500 | 5 | 5 |

### Test Example 4: Flooding treatment on paddy fields

Cylindrical plastic pots of 9 cm in diameter and 11 cm in depth were filled with soil, in which the seeds of barnyardgrass *(Echinochloa oryzicola)* were sowed. These pots were flooded to form a paddy field, and the test plants were grown in a greenhouse for 7 days. Each of the test compounds listed below was formulated into an emulsifiable concentrate according to Formulation Example 2, which was diluted with water to a prescribed concentration. The dilution was applied to the water surface in the pots at a volume of 50 liters per hectare. After the application, the test plants were grown in the greenhouse for 19 days, and the herbicidal activity was examined. The results are shown in Table 8.

**TABLE 8**

| Test compound | Application amount of active ingredient (g/ha) | Herbicidal activity |
|---|---|---|
| | | Barnyardgrass |
| 1-332 | 250 | 5 |
| 1-335 | 250 | 5 |
| 1-338 | 250 | 5 |
| 1-347 | 250 | 5 |
| 1-353 | 250 | 5 |
| 1-367 | 250 | 5 |
| 1-369 | 250 | 5 |
| 1-391 | 250 | 5 |
| 1-392 | 250 | 5 |
| 1-398 | 250 | 5 |
| 1-420 | 250 | 5 |
| 1-439 | 250 | 5 |
| 1-449 | 250 | 5 |
| 1-456 | 250 | 5 |
| 1-474 | 250 | 5 |
| 1-475 | 250 | 5 |
| 1-476 | 250 | 5 |
| 1-482 | 250 | 5 |
| 1-483 | 250 | 5 |
| 1-486 | 250 | 5 |
| 1-487 | 250 | 5 |
| 1-491 | 250 | 5 |
| 1-495 | 250 | 5 |
| 1-496 | 250 | 5 |
| 1-497 | 250 | 5 |
| 1.498 | 250 | 5 |
| 1-499 | 250 | 5 |
| 1-500 | 250 | 5 |
| 1-501 | 250 | 5 |
| 1-503 | 250 | 5 |
| 1-504 | 250 | 5 |
| 1-511 | 250 | 5 |
| 1-576 | 250 | 5 |
| 1-577 | 250 | 5 |
| 1-579 | 250 | 5 |
| 1-581 | 250 | 5 |
| 1-584 | 250 | 5 |
| 1-585 | 250 | 5 |
| 1-586 | 250 | 5 |
| 1-587 | 250 | 5 |
| 1-621 | 250 | 5 |
| 1-625 | 250 | 5 |
| 1-641 | 250 | 5 |
| 1-642 | 250 | 5 |
| 1-1025 | 250 | 5 |
| 1-1028 | 250 | 5 |
| 1-1029 | 250 | 5 |
| 1-1035 | 250 | 5 |
| 1-1057 | 250 | 5 |
| 1-1066 | 250 | 5 |
| 1-1076 | 250 | 5 |
| 1-1086 | 250 | 5 |
| 1-1093 | 250 | 5 |
| 1-1113 | 250 | 5 |
| 1-1123 | 250 | 5 |
| 1-1124 | 250 | 5 |
| 1-1133 | 250 | 5 |
| 1-1140 | 250. | 5 |
| 1-1141 | 250 | 5 |
| t-1213 | 250 | 5 |
| 1-1214 | 250 | 5 |
| 1-1221 | 250 | 5 |
| 1-1222 | 250 | 5 |
| 1-1226 | 250 | 5 |
| 1-1274 | 250 | 5 |
| 1-1278 | 250 | 5 |
| 1-1279 | 250 | 5 |
| 1-1422 | 250 | 5 |
| 1-1431 | 250 | 5 |
| 1-1441 | 250 | 5 |
| 1-1451 | 250 | 5 |
| 1-1458 | 250 | 5 |
| 1-1540 | 250 | 5 |
| 1-1617 | 250 | 5 |
| 1-1622 | 250 | 5 |
| 1-1627 | 250 | 5 |
| 1-1638 | 250 | 5 |
| 1-1639 | 250 | 5 |
| 1-1641 | 250 | 5 |
| 1-1650 | 250 | 5 |
| 1-1655 | 250 | 5 |
| 1-1663 | 250 | 5 |
| 1-1665 | 250 | 5 |
| 1-1670 | 250 | 5 |
| 1-1673 | 250 | 5 |
| 1-1678 | 250 | 5 |
| 1-1679 | 250 | 5 |
| 1-1680 | 250 | 5 |
| 1-1681 | 250 | 5 |
| 1-1682 | 250 | 5 |
| 1-1683 | 250 | 5 |
| 1-1687 | 250 | 5 |
| 1-1691 | 250 | 5 |
| 1-1701 | 250 | 5 |
| 1-1718 | 250 | 5 |
| 1-1719 | 250 | 5 |
| 1-1722 | 250 | 5 |
| 1-1732 | 250 | 5 |
| 1-1748 | 250 | 5 |
| 1-1780 | 250 | 5 |
| 1-1781 | 250 | 5 |
| 1-1782 | 250 | 5 |
| 1-1782 | 250 | 5 |
| 1-1783 | 250 | 5 |
| 1-1785 | 250 | 5 |
| 2-203 | 250 | 5 |
| 2-251 | 250 | 5 |
| 2-328 | 250 | 5 |
| 2-583 | 250 | 5 |
| 2-631 | 250 | 5 |
| 2-708 | 250 | 5 |
| 2-821 | 250 | 5 |
| 3-139 | 250 | 5 |
| 4-434 | 250 | 5 |
| 4-451 | 250 | 5 |
| 1-344 | 500 | 5 |
| 1-1690 | 500 | 5 |
| 1-1720 | 500 | 5 |
| 1-1721 | 500 | 5 |

### Test Example 5: Foliar treatment on upland fields

Plastic pots of 25 x 18 cm² in area and 7 cm in depth were filled with soil, in which the seeds of soybean *(Glycine max*), corn (*Zea mays),* entireleaf morningglory *(Ipomoea hederacea* var. *integriuscula),* common cocklebur (*Xanthium pensylvanicum),* common ragweed *(Ambrosia artemisiifolia)*, and common lambsquarters *(Chenopodium album)* were sowed, and the test plants were grown for 16 days. Each of the test compounds listed below was formulated into an emulsifiable concentrate according to Formulation Example 2, which was diluted with water to a prescribed concentration. The dilution was uniformly sprayed over the foliage of the test plants with a sprayer at a volume of 1000 liters per hectare. At this time, the unfavorable weeds and crop plants, although their growth state was different depending upon the weed species, were at the 1- to 4-leaf stage, and the plant height was 5 to 20 cm. After 18 days from the application, the herbicidal activity and phytotoxicity were examined. The results are shown in Table 9. This test was made in a greenhouse over the entire period.

**TABLE 9**

| Test compound | Application amount of active ingredient (g/ha) | Herbicidal activity and phytotoxicity | | | | | |
|---|---|---|---|---|---|---|---|
| | | Corn | Soy-bean | Common cocklebur | Entireleaf morning-glory | Common ragweed | Common lamb-squarters |
| 1-495 | 63 | 1 | 1 | 5 | 5 | 5 | 5 |
| 1-496 | 63 | 1 | 1 | 5 | 5 | 5 | 5 |
| 1-499 | 63 | 1 | 1 | 5 | 5 | 5 | 5 |
| 1-503 | 63 | 1 | 2 | 5 | 5 | 5 | 5 |
| 1-577 | 63 | 1 | 2 | 5 | 5 | 5 | 5 |

### Test Example 6: Foliar treatment on upland fields

Plastic pots of 16 x 11 cm² in are and 7 cm in depth were filled with soil, in which the seeds of wheat *(Triticum aestivum),* pale smartweed *(Polygonum lapathifolium),* catchweed bedstraw *(Galium aparine),* and common chickweed *(Stellaria media)* were sowed, and the test plants were grown for 29 days. Each of the test compounds listed below was formulated into an emulsifiable concentrate according to Formulation Example 2, which was diluted with water to a prescribed concentration. The dilution was uniformly sprayed over the foliage of the test plants with a sprayer at a volume of 1000 liters per hectare. At this time, the unfavorable weeds and crop plants, although their growth state was different depending upon the weed species, were at the 1- to 4-leaf stage, and the plant height was 5 to 15 cm. After 25 days from the application, the herbicidal activity and phytotoxicity were examined. The results are shown in Table 10. This test was made in a greenhouse over the entire period.

**TABLE 10**

| Test compound | Application amount of active ingredient (g/ha) | Herbicidal activity and phytotoxicity | | | |
|---|---|---|---|---|---|
| | | Wheat | Pale smart-weed | Catch-weed bedstraw | Common chick-weed |
| 1-439 | 63 | 1 | 5 | 5 | 5 |
| 1-482 | 63 | 1 | 5 | 5 | 5 |
| 1-486 | 63 | 1 | 5 | 5 | 5 |
| 1-496 | 63 | 0 | 4 | 5 | 4 |
| 1-1076 | 63 | 1 | 5 | 5 | 5 |
| 1-1123 | 63 | 1 | 5 | 5 | 5 |
| 1-1441 | 63 | 1 | 5 | 5 | 5 |

### Test Example 7: Soil surface treatment on upland fields

Plastic pots of 25 x 18 cm² in area and 7 cm in depth were filled with soil, in which the seeds of soybean *(Glycine max),* corn (*Zea mays),* common lambsquarters *(Chenopodium album),* slender amaranth *(Amaranthus gracilis),* and pale smartweed *(Polygonum lapathifolium)* were sowed. Each of the test compounds listed below was formulated into an emulsifiable concentrate according to Formulation Example 2, which was diluted with water to a prescribed concentration. The dilution was uniformly sprayed over the soil surface in the pots with a sprayer at a volume of 1000 liters per hectare. After the application, the test plants were grown in a greenhouse for 19 days, and the herbicidal activity and phytotoxicity were examined. The results are shown in Tablet 11.

**TABLE 11**

| Test compound | Application amount of active ingredient (g/ha) | Herbicidal activity and phytotoxicity | | | | |
|---|---|---|---|---|---|---|
| | | Soybean | Corn | Common lamb-squarters | Pale Slender amaranth | smart-weed |
| 1-511 | 63 | 1 | 0 | 5 | 5 | 5 |
| 1-642 | 63 | 1 | 0 | 5 | 5 | 5 |
| 1-1279 | 63 | 0 | 1 | 5 | 5 | 5 |
| 1-1691 | 63 | 0 | 1 | 5 | 5 | 5 |
| 2-203 | 63 | 2 | 1 | 5 | 5 | 5 |
| 2-631 | 63 | 1 | 3 | 5 | 5 | 5 |

## Claims

1. A compound of the formula: 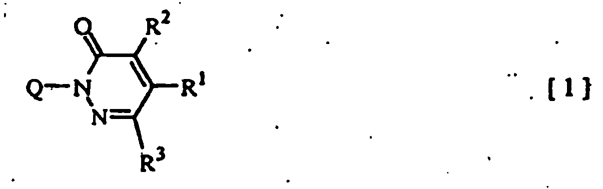 wherein R¹ is C₁-C₃ haloalkyl; R² and R³ are the same or different and are hydrogen, C₁-C₃ alkyl, C₁-C₃ haloalkyl, or C₁-C₃ alkoxy C₁-C₃ alkyl; and Q is [Q-1], [Q-2], [Q-3], or [Q-4] of the formula: 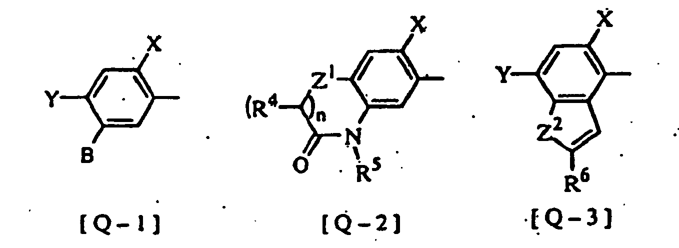 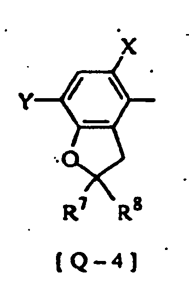 wherein X is hydrogen or halogen;
Y is halogen, nitro, cyano, or trifluoromethyl;
Z¹ is oxygen, sulfur, or NH;
Z² is oxygen or sulfur,
n is 0 or 1 :
B is hydrogen, halogen, nitro, cyano, chlorosulfonyl, OR¹⁰, SR¹⁰, SO₂-OR¹⁰, N(R¹¹)R¹², SO₂N(R¹¹)-R¹², NR¹¹(COR¹³), NR¹¹(SO₂R¹⁴), N(SO₂R¹⁴)-(SO₂R¹⁵), N(SO₂R¹⁴)(COR¹³), NHCOOR¹³, COOR¹⁰, CON(R¹¹)R¹², CSN(R¹¹)R¹², COR¹⁶, CR¹⁷=CR¹⁸COR¹⁶, CR¹⁷=CR¹⁸COOR¹³, CR¹⁷=CR¹⁸CON(R¹¹)R¹², CH₂CH-WCOOR¹³, CH₂CHWCON(R¹¹)R¹², CR¹⁷=NOR³³, CR¹⁷=NN(R¹¹)R¹², CR¹⁷(Z²-R³⁴)₂, OCO₂R¹⁹, or OCOR¹⁹;
R⁴ is hydrogen or C₁-C₃ alkyl;
R⁵ is hydrogen, C₁-C₆ alkyl, C₁-C₆ haloalkyl, C₃-C₈ cycloalkylalkyl, C₃-C₆ alkenyl, C₃-C₆ haloalkenyl, C₃-C₆ alkynyl, C₃-C₆ haloalkynyl, cyano C₁-C₆ alkyl, C₂-C₈ alkoxyalkyl, C₃-C₈ alkoxyalkoxyalkyl, carboxy C₁-C₆ alkyl, (C₁-C₆ alkoxy)-carbonyl C₁-C₆ alkyl, {(C₁-C₄ alkoxy) C₁-C₄ alkoxy}carbonyl C₁-C₆ alkyl, (C₃-C₈ cycloalkoxy)carbonyl C₁-C₆ alkyl, CH₂CON(R¹¹)R¹², CH₂COON(R¹¹)R¹², CH(C₁-C₄ alkyl)CON(R¹¹)R¹², CH(C₁-C₄ alkyl)COON(R¹¹)R¹², C₂-C₈ alkylthioalkyl, or hydroxy C₁-C₆ alkyl;
R⁶ is C₁-C₆ alkyl, C₁-C₆ haloalkyl, formyl, cyano, carboxyl, hydroxy C₁-C₆ alkyl, C₁-C₆ alkoxy C₁-C₆ alkyl, C₁-C₆ alkoxy C₁-C₆ alkoxy C₁-C₆ alkyl, (C₁-C₆ alkyl)carbonyloxy C₁-C₆ alkyl, (C₁-C₆ haloalkyl)carbonyloxy C₁-C₆ alkyl, (C₁-C₆ alkoxy)carbonyl, or (C₁-C₆ alkyl)carbonyl;
R⁷ is hydrogen or C₁-C₆ alkyl; and
R⁸ is hydrogen, C₁-C₆ alkyl, C₁-C₆ haloalkyl, hydroxy C₁-C₆ alkyl, C₂-C₈ alkoxyalkyl, C₃-C₁₀ alkoxyalkoxyalkyl, (C₁-C₅ alkyl)carbonyloxy C₁-C₆ alkyl, (C₁-C₆ haloalkyl)carbonyloxy C₁-C₆ alkyl, carboxyl, carboxy C₁-C₆ alkyl, (C₁-C₈ alkoxy)-carbonyl, (C₁-C₆ haloalkoxy)carbonyl, (C₃-C₁₀ cycloalkoxy)carbonyl, (C₃-C₈ alkenyloxy)carbonyl, (C₃-C₈ alkynyloxy)carbonyl, aminocarbonyl, (C₁-C₆ alkyl)aminocarbonyl, di(C₁-C₆ alkyl)aminocarbonyl, (C₁-C₆ alkyl)aminocarbonyloxy C₁-C₆ alkyl, or di(C₁-C₆ alkyl)aminocarbonyloxy C₁-C₆ alkyl;
wherein R¹⁰ is hydrogen, C₁-C₆ alkyl, C₁-C₆ haloalkyl, C₃-C₈ cycloalkyl, benzyl, C₃-C₆ alkenyl, C₃-C₆ haloalkenyl, C₃-C₆ alkynyl, C₃-C₆ haloalkynyl, cyano C₁-C₆ alkyl, C₂-C₈ alkoxyalkyl, C₂-C₈ alkylthioalkyl, carboxy C₁-C₆ alkyl, (C₁-C₈ alkoxy)carbonyl C₁-C₆ alkyl, (C₁-C₆ haloalkoxy)carbonyl C₁-C₆ alkyl, {(C₁-C₄ alkoxy) C₁-C₄ alkoxy}carbonyl C₁-C₆ alkyl, (C₃-C₈ cycloalkoxy)carbonyl C₁-C₆ alkyl, (C₁-C₆ alkyl)carbonyl C₁-C₆ alkyl, (C₁-C₆ haloalkyl)carbonyl C₁-C₆ alkyl, ((C₁-C₄ alkoxy) C₁-C₄ alkyl)carbonyl C₁-C₆ alkyl, (C₃-C₈ cycloalkyl)carbonyl C₁-C₆ alkyl, CH₂CON-(R¹¹)R¹², CH₂COON(R¹¹)R¹², CH(C₁-C₄ alkyl)CON(R¹¹)R¹², CH(C₁-C₄ alkyl)-COON(R¹¹)R¹², {(C₁-C₆ alkoxy)carbonyl C₁-C₆ alkyl}oxycarbonyl C₁-C₆ alkyl, or hydroxy C₁-C₆ alkyl ;
R¹¹ and R¹² are independently hydrogen, C₁-C₆ alkyl, C₁-C₆ haloalkyl, C₃-C₆ alkenyl, C₃-C₆ alkynyl, cyano C₁-C₆ alkyl, C₂-C₈ alkoxyalkyl. C₂-C₈ alkylthioalkyl, carboxy C₁-C₆ alkyl, (C₁-C₆ alkoxy)carbonyl C₁-C₆ alkyl, (C₃-C₈ cycloalkoxy)-carbonyl C₁-C₆ alkyl, {(C₁-C₄ alkoxy) C₁-C₄ alkoxy}carbonyl C₁-C₆ alkyl, or R¹¹ and R¹² are combined together to form tetramethylene, pentamethylene, or ethyleneoxyethylene;
R¹³ is hydrogen, C₁-C₆ alkyl, C₁-C₆ haloalkyl, C₃-C₈ cycloalkyl, or C₃-C₆ alkenyl;
R¹⁴ and R¹⁵ are independently C₁-C₆ alkyl, C₁-C₆ haloalkyl, or phenyl optionally substituted with methyl or nitro;
R¹⁶ is hydrogen, C₁-C₆ alkyl, C₁-C₆ haloalkyl, C₂-C₆ alkenyl, C₂-C₆ haloalkenyl, C₂-C₆ alkynyl, C₂-C₆ haloalkynyl, C₂-C₈ alkoxyalkyl, or hydroxy C₁-C₆ alkyl;
R¹⁷ and R¹⁸ are independently hydrogen or C₁-C₆ alkyl;
R¹⁹ is C₁-C₆ alkyl;
R³³ is hydrogen, C₁-C₆ alkyl, C₁-C₆ haloalkyl, C₃-C₈ cycloalkyl, C₃-C₆ alkenyl. C₃-C₆ haloalkenyl, C₃-C₆ alkynyl, C₃-C₆ haloalkynyl, cyano C₁-C₆ alkyl, or (C₁-C₆ alkoxy)carbonyl C₁-C₆ alkyl;
R³⁴ is C₁-C₆ alkyl, or two R³⁴'s are combined together to form (CH₂)₂ or (CH₂)₃; and
W is hydrogen, chlorine, or bromine.

2. A compound according to claim 1, wherein R¹ is trifluoromethyl.

3. A compound according to claim 1, wherein R² is hydrogen or C₁-C₃ alkyl, and R³ is hydrogen or C₁-C₃ alkyl.

4. A compound according to claim 1, wherein R¹ is trifluoromethyl, R² is hydrogen or C₁-C₃ alkyl, and R³ is hydrogen or C₁-C₃ alkyl,

5. A compound according to claim 1, 2, 3, or 4, wherein Q is [Q-1].

6. A compound according to claim 5, wherein B is OR¹⁰, SR¹⁰, N(R¹¹)R¹², NR¹¹(SO₂R¹⁴) or -COOR¹⁰.

7. A compound according to claim 5, wherein X is fluorine and Y is chlorine.

8. A compound according to claim 5, wherein X is fluorine, Y is chlorine, and B is OR¹⁰.

9. A compound according to claim 1, 2, 3, or 4 wherein Z¹ is oxygen, n is 1, and Q ist [Q-2].

10. A compound according to claim 1, which is 7-fluoro-6-(5-trifluoromethyl-3-pyridazinon-2-yl)-4-propargyl-2H-1,4-benzoxazin-3-one (compound 2-631),
7-fluoro-6-(4-methyl-5-trifluoromethyl-3-pyridazinon-2-yl)-4-propargyl,-2H-1,4-benzoxazin-3-one (compound 2-251),
6-(5-trifluoromethyl-3-pyridazinon-2-yl)-4-propargyl-2H-1,4-benzoxazin-3-one (compound 2-583), 6-(4-methyl-5-trifluoromethyl-3-pyridazinon-2-yl)-4-propargyl-2H-1,4-benzoxazin-3-one (compound 2-203),
2-(4-chloro-2-fluoro-5-isopropoxyphenyl)-4-methyl-5-trifluoromethylpyridazin-3-one (compound 1-476),
2-(4-chloro-2-fluoro-5-methoxyphenyl)-4-methyl-5-trifluoromethylpyridazin-3-one (compound 1-474),
2-(4-chloro-2-fluoro-5-ethoxyphenyl)-4-methyl-5-trifluoromethylpyridazin-3-one (compound 1-475),
2-(4-chloro-2-fluoro-5-propargyloxyphenyl)-4-methyl-5-trifluoromethylpyridazin-3-one (compound 1-486),
methyl 2-chloro-4-fluoro-5-(4-methyl-5-trifluoromethyl-3-pyridazinon-2-yl)phenoxyacetate (compound 1-495),
ethyl 2-chloro-4-fluoro-5-(4-methyl-5-trifluoromethyl-3-pyridazinon-2-yl)phenoxyacetate (compound 1-496),
propyl 2-chloro-4-fluoro-5-(4-methyl-5-trifluoromethyl-3-pyridazinon-2-yl)phenoxyacetate (compound 1-497),
isopropyl 2-chloro-4-fluoro-5-(4-methyl-5-trifluoromethyl-3-pyridazinon-2-yl)phenoxyacetate (compound 1-500),
butyl 2-chloro-4-fluoro-5-(4-methyl-5-trifluoromethyl-3-pyridazinon-2-yl)phenoxyacetate (compound 1-498),
pentyl 2-chloro-4-fluoro-5-(4-methyl-5-trifluoromethyl-3-pyridazinon-2-yl)phenoxyacetate (compound 1-499),
cyclopentyl 2-chloro-4-fluoro-5-(4-methyl-5-trifluoromethyl-3-pyridazinon-2-yl)phenoxyacetate (compound 1-501),
ethyl 2-{2-chloro-4-fluoro-5-(4-methyl-5-trifluoromethyl-3-pyridazinon-2-yl)phenoxy}propionate (compound 1-504),
methyl 2-{2-chloro-4-fluoro-5-(4-methyl-5-trifluoromethy-5-pyridazinon-2-yl)phenoxy}propionate (compound 1-503),
ethyl 2-chloro-4-fluoro-5-(4-methyl-5-triflupromethyl-3-pyridazinon-2-yl)phenylthioncetate (Compound 1-577),
methyl 2-chloro-4-fluoro-5-(4-methyl-5-trifluoromethyl-3-pyridazinon-2-yl)phenylthioacetate (compound 1-576),
ethyl 2-{2-chloro-4-fluoro-5-(4-methyl-5-trifluoromethyl-3-pyridazinon-2-yl)phenylthio}propionate (compound 1-585),
methyl 2-{2-chloro-4-fluoro-5-(4-methyl-5-trifluoromethyl-3-pyridazinon-2-yl)phenylthio}propionate (compound 1-584),
methyl 2-{2-chloro-4-fluoro-5-(5-trifluoromethyl-3-pyridazinon-2-yl)phenoxy}propionate (compound 1-1140),
ethyl 2-{2-chloro-4-fluoro-5-(5-trifluoromethyl-3-pyridazition-2-yl)phenoxy}propionate (compound 1-1141),
ethyl 2-chloro-4-fluoro-5-(5-trifluoromethyl-3-pyridazinon-2-yl)phenylthioacetate (compound 1-1214)
methyl 2-chloro-4-fluoro-5-(5-tnfluor6methyl-3-pyndazinon-2-yt)phenylthioacetate (compound 1-1213),
ethyl 2-{2-chloro-4-fluoro-5-(5-trifluoromethyl-3-pyridazinon-2-yl)phenylthio}propionate (compound 1-1222)
methyl 2-{2-chloro-4-fluoro-5-(5-trifluoromethyl-3-pyridazinon-2-yl)phenylthio}propionate (compound 1-1221),
isopropyl 2-(2-chloro-4-fluoro-5-(5-trifluoromethyl-3-pyridazinon-2-yl)phenylthio)propionate (compound 1-1226),
ethyl 2-{2-chloro-4-fluoro-5-(4-methyl-5-trifluoromethyl-3-pyridazinon-2-yl)phenylamino}propionate (compound 1-420),
ethyl 2-{2-chloro-4-fluoro-5-(5-trifluoromethyl-3-pyridazinon-2-yl)phenylamino} propionate (compound 1-1057),
N-{2-chloro-4-fluoro-5-(4-methyl-5-trifluoromethyl-3-pyridazinon-2-yl)phenyl}methanesulfonamide (compound 1-367),
N-{2-chloro-fluoro-5-(4-methyl-5-trifluoromethyl-3-pyridazinon-2-yl)phenyl}chloromethanesulfonamide (compound 1-369),
N-{2-chloro-5-(4-methyl-5-trifluoromethyl-3-pyridazinon-2-yl)phenyl}methanesulfonamide (compound 1-398),
methyl 2-chloro-4-fluoro-5-(4-methyl-3-trifluoromethyl-3-pyridazinon-2-yl)benzoate (compound 1-641),
ethyl 2-chloro-4-fluoro-5-(4-methyl-5-trifluoromethyl-3-pyridazinon-2-yl)benzoate (compound 1-642),
ethyl 2-chloro-5-(4-methyt-5-trifluoromethyl-3-pyridazinon-2-yl)benzoate (compound 1-621),
isopropyl 2-chloro-5-(4-methyl-5-trifluoromethyl-3-pyridazinon-2-yl)benzoate (compound 1-625), or
2-(4-chloro-2-fluoro-5-propargyloxyphenyl)-6-methyl-5-trifluoromethylpyridazin-3-one (compound 1-1441).

11. A herbicidal composition comprising a herbicidally effective amount of the compound according to claim 1, and an inert carrier or diluent.

12. A method for controlling unfavorable weeds which comprises applying a herbicidally effective amount of the compound according to claim 1 to an area where the unfavorable weeds grow or will grow.

13. Use of the compound according to claim 1 as a herbicide.

14. A compound of the formula: 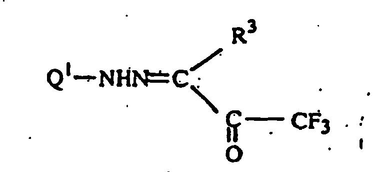 wherein R³ is as defined in claim 1, and Q¹ is [Q¹-1], [Q-2], [Q¹-3], or [Q-4] of the formula: 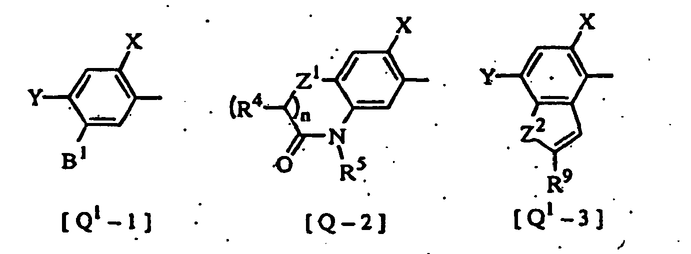 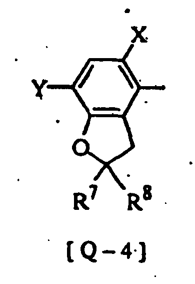 wherein X, Y, Z¹, Z², n, R⁴, R⁵, R⁷ and R⁸ are as defined in claim 1;
B¹ is hydrogen, halogen, nitro, cyano, OR²⁷, SR²⁷, SO₂OR²⁷, NR¹¹(R¹²), SO₂NR¹¹(R¹²), NR¹¹(COR¹³), NR¹¹(SO₂R¹⁴), N(SO₂R¹⁴)(SO₂R¹⁵), N(SO₂R¹⁴)-(COR¹³), NHCOOR¹³, COOR²⁷, CONR¹¹(R¹²), CSNR¹¹(R¹²), CR¹⁷=CR¹⁸COOR¹³, CR¹⁷=CR¹⁸CONR¹¹(R¹²), CH₂CHWCOOR¹³, CH₂CHWCONR¹¹(R¹²), CR¹⁷=NOR³³, CR¹⁷=NNR¹¹(R¹²), CR¹⁷(Z²R³⁴)₂, OCO₂R¹⁹, or OCOR¹⁹; and
R⁹ is C₁-C₆ alkyl, C₁-C₆ haloalkyl, cyano, carboxyl, hydroxy C₁-C₆ alkyl, C₁-C₆ alkoxy C₁-C₆ alkyl, C₁-C₆ alkoxy C₁-C₆ alkoxy C₁-C₆ alkyl, (C₁-C₆ alkyl) carbonyloxy C₁-C₆ alkyl, (C₁-C₆ haloalkyl)carbonyloxy C₁-C₆ alkyl, (C₁-C₆ alkoxy) carbonyl, or (C₁-C₆ alkyl)carbonyl;
wherein R²⁷ is hydrogen, C₁-C₆ alkyl, C₁-C₆ haloalkyl, C₃-C₈ cycloalkyl, benzyl, C₃-C₆ alkenyl, C₃-C₆ haloalkenyl, C₃-C₆ alkynyl, C₃-C₆ haloalkynyl, cyano C₁-C₆ alkyl, C₂-C₈ alkoxyalkyl, C₂-C₈ alkylthioalkyl, carboxy C₁-C₆ alkyl, (C₁-C₈ alkoxy)carbonyl C₁-C₆ alkyl, (C₁-C₆ haloalkoxy)carbonyl C₁-C₆ alkyl, {(C₁-C₄ alkoxy) C₁-C₄ alkoxy}carbonyl C₁-C₆ alkyl, (C₃-C₈ cycloalkoxy)carbonyl C₁-C₆ alkyl, CH₂CON(R¹¹)R¹², CH₂COON(R¹¹)R¹², CH(C₁-C₄ alkyl)CON(R¹¹)R¹², CH(C₁-C₄ alkyl) COON(R¹¹)R¹², {(C₁-C₆ alkoxy)carbonyl C₁-C₆ alkyl}oxycarbonyl C₁-C₆ alkyl, or hydroxy C₁-C₆ alkyl; and R¹¹, R¹², R¹³, R¹⁴, R¹⁵, R¹⁷, R¹⁸, R¹⁹, R³³, R³⁴, W and Z² are as defined in claim 1.

## Patentansprüche

1. Verbindung der Formel: 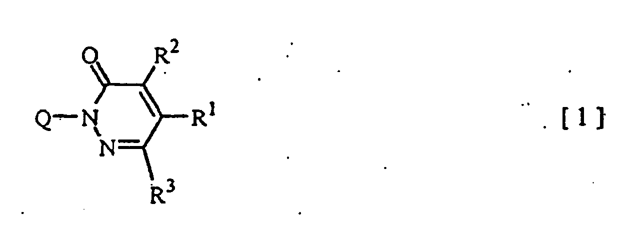 wobei R¹ C₁-C₃-Halogenalkyl ist; R² und R³ gleich oder verschieden sind und Wasserstoff, C₁-C₃-Alkyl, C₁-C₃-Halogenalkyl oder C₁-C₃-Alkoxy-C₁-C₃-alkyl sind; und Q [Q-1], [Q-2], [Q-3] oder [Q-4] der Formel: 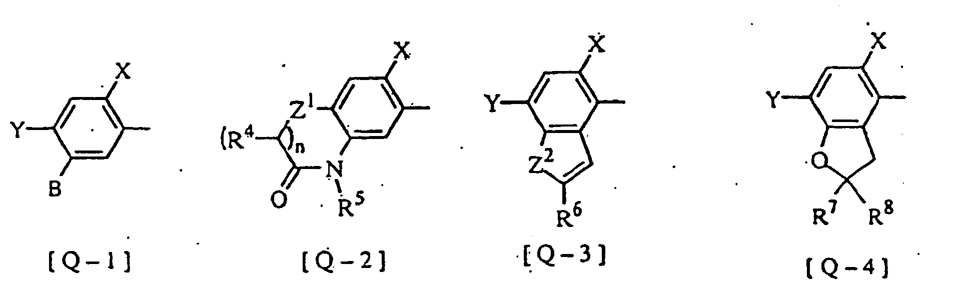 ist,
wobei X Wasserstoff oder Halogen ist;
Y Halogen, Nitro, Cyano oder Trifluormethyl ist;
Z¹ Sauerstoff, Schwefel oder NH ist;
Z² Sauerstoff oder Schwefel ist;
n 0 oder 1 ist;
B Wasserstoff, Halogen, Nitro, Cyano, Chlorsulfonyl, OR¹⁰, SR¹⁰, SO₂OR¹⁰, N(R¹¹)R¹², SO₂N(R¹¹)R¹², NR¹¹(COR¹³), NR¹¹(SO₂R¹⁴), N(SO₂R¹⁴)(SO₂R¹⁵), N(SO₂R¹⁴)(COR¹³), NHCOOR¹³, COOR¹⁰, CON(R¹¹)R¹², CSN(R¹¹)R¹², COR¹⁶, CR¹⁷=CR¹⁸COR¹⁶, CR¹⁷=CR¹⁸COOR¹³, CR¹⁸=CR¹⁸CON(R¹¹)R¹², CH₂CHWCOOR¹³, CH₂CHWCON(R¹¹)R¹², CR¹⁷=NOR³³, CR¹⁷=NN(R¹¹)R¹², CR¹⁷(Z²R³⁴)₂, OCO₂R¹⁹ oder OCOR¹⁹ ist;
R⁴ Wasserstoff oder C₁-C₃-Alkyl ist;
R⁵ Wasserstoff, C₁-C₆-Alkyl, C₁-C₆-Halogenalkyl, C₃-C₈-Cycloalkylalkyl, C₃-C₆-Alkenyl, C₃-C₆-Halogenalkenyl, C₃-C₆-Alkinyl, C₃-C₆-Halogenalkinyl, Cyano-C₁-C₆-alkyl, C₂-C₈-Alkoxyalkyl, C₃-C₈-Alkoxyalkoxyalkyl, Carboxy-C₁-C₆-alkyl, (C₁-C₆-Alkoxy)carbonyl-C₁-C₆-alkyl, {(C₁-C₄-Alkoxy)-C₁-C₄-alkoxy}carbonyl-C₁-C₆-alkyl, (C₃-C₈-Cycloalkoxy)carbonyl-C₁-C₆-alkyl, CH₂CON(R¹¹)R¹², CH₂COON(R¹¹)R¹², CH(C₁-C₄-Alkyl)CON(R¹¹)R¹², CH-(C₁-C₄-Alkyl)-COON(R¹¹)R¹², C₂-C₈-Alkylthioalkyl oder Hydroxy-C₁-C₆-alkyl ist;
R⁶ C₁-C₆-Alkyl, C₁-C₆-Halogenalkyl, Formyl, Cyano, Carboxyl, Hydroxy-C₁-C₆-alkyl, C₁-C₆-Alkoxy-C₁-C₆-alkyl, C₁-C₆-Alkoxy-C₁-C₆-alkoxy-C₁-C₆-alkyl, (C₁-C₆-Alkyl)-carbonyloxy-C₁-C₆-alkyl, (C₁-C₆-Halogenalkyl)carbonyloxy-C₁-C₆-alkyl, (C₁-C₆-Alkoxy)carbonyl oder (C₁-C₆-Alkyl)carbonyl ist;
R⁷ Wasserstoff oder C₁-C₆-Alkyl ist; und
R⁸ Wasserstoff, C₁-C₆-Alkyl, C₁-C₆-Halogenalkyl, Hydroxy-C₁-C₆-alkyl, C₂-C₈-Alkoxyalkyl, C₃-C₁₀-Alkoxyalkoxyalkyl, (C₁-C₅-Alkyl)carbonyloxy-C₁-C₆-alkyl, (C₁-C₆-Halogenalkyl)carbonyloxy-C₁-C₆-alkyl, Carboxyl, Carboxy-C₁-C₆-alkyl, (C₁-C₈-Alkoxy)carbonyl, (C₁-C₆-Halogenalkoxy)carbonyl, (C₃-C₁₀-Cycloalkoxy)carbonyl, (C₃-C₈-Alkenyloxy)carbonyl, (C₃-C₈-Alkinyloxy)carbonyl, Aminocarbonyl, (C₁-C₆-Alkyl)aminocarbonyl, Di-(C₁-C₆-alkyl)aminocarbonyl, (C₁-C₆-Alkyl)aminocarbonyloxy-C₁-C₆-alkyl oder Di-(C₁-C₆-alkyl)aminocarbonyloxy-C₁-C₆-alkyl ist;
wobei R¹⁰ Wasserstoff, C₁-C₆-Alkyl, C₁-C₆-Halogenalkyl, C₃-C₈-Cycloalkyl, Benzyl, C₃-C₆-Alkenyl, C₃-C₆-Halogenalkenyl, C₃-C₆-Alkinyl, C₃-C₆-Halogenalkinyl, Cyano-C₁-C₆-alkyl, C₂-C₈-Alkoxyalkyl, C₂-C₈-Alkylthioalkyl, Carboxy-C₁-C₆-alkyl, (C₁-C₈-Alkoxy)carbonyl-C₁-C₆-alkyl, (C₁-C₆-Halogenalkoxy)carbonyl-C₁-C₆-alkyl, {(C₁-C₄-Alkoxy)-C₁-C₄-alkoxy}carbonyl-C₁-C₆-alkyl, (C₃-C₈-Cycloalkoxy)carbonyl-C₁-C₆-alkyl, (C₁-C₆-Alkyl)carbonyl-C₁-C₆-alkyl, (C₁-C₆-Halogenalkyl)carbonyl-C₁-C₆-alkyl, {(C₁-C₄-Alkoxy)-C₁-C₄-alkyl}carbonyl-C₁-C₆-alkyl, (C₃-C₈-Cycloalkyl)carbonyl-C₁-C₆-alkyl, CH₂CON(R¹¹)R¹², CH₂COON(R¹¹)R¹², CH(C₁-C₄-Alkyl)CON(R¹¹)R¹², CH-(C₁-C₄-Alkyl)-COON(R¹¹)R¹², {(C₁-C₆-Alkoxy)carbonyl-C₁-C₆-alkyl}oxycarbonyl-C₁-C₆-alkyl oder Hydroxy-C₁-C₆-alkyl ist;
R¹¹ und R¹² unabhängig Wasserstoff, C₁-C₆-Alkyl, C₁-C₆-Halogenalkyl, C₃-C₆-Alkenyl, C₃-C₆-Alkinyl, Cyano-C₁-C₆-alkyl, C₂-C₈-Alkoxyalkyl, C₂-C₈-Alkylthioalkyl, Carboxy-C₁-C₆-alkyl, (C₁-C₆-Alkoxy)carbonyl-C₁-C₆-alkyl, (C₃-C₈-Cycloalkoxy)carbonyl-C₁-C₆-alkyl, {(C₁-C₄-Alkoxy)-C₁-C₄-alkoxy}carbonyl-C₁-C₆-alkyl sind, oder R¹¹ und R¹² zusammengeführt werden, um Tetramethylen, Pentamethylen oder Ethylenoxyethylen zu bilden,
R¹³ Wasserstoff, C₁-C₆-Alkyl, C₁-C₆-Halogenalkyl, C₃-C₈-Cycloalkyl oder C₃-C₆-Alkenyl ist;
R¹⁴ und R¹⁵ unabhängig C₁-C₆-Alkyl, C₁-C₆-Halogenalkyl oder Phenyl, gegebenenfalls substituiert mit Methyl oder Nitro, sind;
R¹⁶ Wasserstoff, C₁-C₆-Alkyl, C₁-C₆-Halogenalkyl, C₂-C₆-Alkenyl, C₂-C₆-Halogenalkenyl, C₂-C₆-Alkinyl, C₂-C₆-Halogenalkinyl, C₂-C₈-Alkoxyalkyl oder Hydroxy-C₁-C₆-alkyl ist;
R¹⁷ und R¹⁸ unabhängig Wasserstoff oder C₁-C₆-Alkyl sind;
R¹⁹ C₁-C₆-Alkyl ist;
R³³ Wasserstoff, C₁-C₆-Alkyl, C₁-C₆-Halogenalkyl, C₃-C₈-Cycloalkyl, C₃-C₆-Alkenyl, C₃-C₆-Halogenalkenyl, C₃-C₆-Alkinyl, C₃-C₆-Halogenalkinyl, Cyano-C₁-C₆-alkyl oder (C₁-C₆-Alkoxy)carbonyl-C₁-C₆-alkyl ist;
R³⁴ C₁-C₆-Alkyl ist oder zwei Reste R³⁴ zusammengeführt werden, um (CH₂)₂ oder (CH₂)₃ zu bilden; und
W Wasserstoff, Chlor oder Brom ist.

2. Verbindung gemäß Anspruch 1, wobei R¹ Trifluormethyl ist.

3. Verbindung gemäß Anspruch 1, wobei R² Wasserstoff oder C₁-C₃-Alkyl ist und R³ Wasserstoff oder C₁-C₃-Alkyl ist.

4. Verbindung gemäß Anspruch 1, wobei R¹ Trifluormethyl ist, R² Wasserstoff oder C₁-C₃-Alkyl ist, und R³ Wasserstoff oder C₁-C₃-Alkyl ist.

5. Verbindung gemäß Anspruch 1, 2, 3 oder 4, wobei Q [Q-1] ist.

6. Verbindung gemäß Anspruch 5, wobei B OR¹⁰, SR¹⁰, N(R¹¹)R¹², NR¹¹(SO₂R¹⁴) oder COOR¹⁰ ist.

7. Verbindung gemäß Anspruch 5, wobei X Fluor ist und Y Chlor ist.

8. Verbindung gemäß Anspruch 5, wobei X Fluor ist, Y Chlor ist und B OR¹⁰ ist.

9. Verbindung gemäß Anspruch 1, 2, 3 oder 4, wobei Z¹ Sauerstoff ist, n 1 ist und Q [Q-2] ist.

10. Verbindung gemäß Anspruch 1, nämlich
7-Fluor-6-(5-trifluormethyl-3-pyridazinon-2-yl)-4-propargyl-2H-1,4-benzoxazin-3-on (Verbindung 2-631);
7-Fluor-6-(4-methyl-5-trifluormethyl-3-pyridazinon-2-yl)-4-propargyl-2H-1,4-benzoxazin-3-on (Verbindung 2-251);
6-(5-Trifluormethyl-3-pyridazinon-2-yl)-4-propargyl-2H-1,4-benzoxazin-3-on (Verbindung 2-583),
6-(4-Methyl-5-trifluormethyl-3-pyridazinon-2-yl)-4-propargyl-2H-1,4-benzoxazin-3-on (Verbindung 2-203),
2-(4-Chlor-2-fluor-5-isopropoxyphenyl)-4-methyl-5-trifluormethylpyridazin-3-on (Verbindung 1-476),
2-(4-Chlor-2-fluor-5-methoxyphenyl)-4-methyl-5-trifluormethylpyridazin-3-on (Verbindung 1-474),
2-(4-Chlor-2-fluor-5-ethoxyphenyl)-4-methyl-5-trifluormethylpyridazin-3-on (Verbindung 1-475),
2-(4-Chlor-2-fluor-5-propargyloxyphenyl)-4-methyl-5-trifluormethylpyridazin-3-on (Verbindung 1-486),
Methyl-2-chlor-4-fluor-5-(4-methyl-5-trifluormethyl-3-pyridazinon-2-yl)phenoxyacetat (Verbindung 1-495),
Ethyl-2-chlor-4-fluor-5-(4-methyl-5-trifluormethyl-3-pyridazinon-2-yl)phenoxyacetat (Verbindung 1-496),
Propyl-2-chlor-4-fluor-5-(4-methyl-5-trifluormethyl-3-pyridazinon-2-yl)phenoxyacetat (Verbindung 1-497),
Isopropyl-2-chlor-4-fluor-5-(4-methyl-5-trifluormethyl-3-pyridazinon-2-yl)phenoxyacetat (Verbindung 1-500),
Butyl-2-chlor-4-fluor-5-(4-methyl-5-trifluormethyl-3-pyridazinon-2-yl)phenoxyacetat (Verbindung 1-498),
Pentyl-2-chlor-4-fluor-5-(4-methyl-5-trifluormethyl-3-pyridazinon-2-yl)phenoxyacetat (Verbindung 1-499),
Cyclopentyl-2-chlor-4-fluor-5-(4-methyl-5-trifluormethyl-3-pyridazinon-2-yl)phenoxyacetat (Verbindung 1-501),
Ethyl-2-{2-Chlor-4-fluor-5-(4-methyl-5-trifluormethyl-3-pyridazinon-2-yl)phenoxy}-propionat (Verbindung 1-504),
Methyl-2-{2-chlor-4-fluor-5-(4-methyl-5-trifluormethyl-3-pyridazinon-2-yl)phenoxy}-propionat (Verbindung 1-503),
Ethyl-2-chlor-4-fluor-5-(4-methyl-5-trifluormethyl-3-pyridazinon-2-yl)phenylthioacetat (Verbindung 1-577),
Methyl-2-chlor-4-fluor-5-(4-methyl-5-trifluormethyl-3-pyridazinon-2-yl)phenylthioacetat (Verbindung 1-576),
Ethyl-2-{2-chlor-4-fluor-5-(4-methyl-5-trifluormethyl-3-pyridazinon-2-yl)phenylthio}-propionat (Verbindung 1-585),
Methyl-2-{2-chlor-4-fluor-5-(4-methyl-5-trifluormethyl-3-pyridazinon-2-yl)phenylthio}-propionat (Verbindung 1-584),
Methyl-2- { 2-chlor-4-fluor-5-(5-trifluormethyl-3-pyridazinon-2-yl)phenoxy } propionat (Verbindung 1-1140),
Ethyl-2-{2-chlor-4-fluor-5-(5-trifluormethyl-3-pyridazinon-2-yl)phenoxy}propionat (Verbindung 1-1141),
Ethyl-2-chlor-4-fluor-5-(5-trifluormethyl-3-pyridazinon-2-yl)phenylthioacetat (Verbindung 1-1214),
Methyl-2-chlor-4-fluor-5-(5-trifluormethyl-3-pyridazinon-2-yl)phenylthioacetat (Verbindung 1-1213),
Ethyl-2- {2-Chlor-4-fluor-5-(5-trifluormethyl-3-pyridazinon-2-yl)phenylthio}propionat (Verbindung 1-1222),
Methyl-2- {2-chlor-4-fluor-5-(5-trifluormethyl-3-pyridazinon-2-yl)phenylthio} propionat (Verbindung 1-1221),
Isopropyl-2-{2-chlor-4-fluor-5-(5-trifluormethyl-3-pyridazinon-2-yl)phenylthio}-propionat (Verbindung 1-1226),
Ethyl-2-{2-chlor-4-fluor-5-(4-methyl-5-trifluormethyl-3-pyridazinon-2-yl)phenylamino}propionat (Verbindung 1-420),
Ethyl-2-{2-chlor-4-fluor-5-(5-trifluormethyl-3-pyridazinon-2-yl)phenylamino}propionat (Verbindung 1-1057),
N- {2-Chlor-4-fluor-5-(4-methyl-5-trifluormethyl-3-pyridazinon-2-yl)phenyl}methansulfonamid (Verbindung 1-367),
N-{2-Chlor-4-fluor-5-(4-methyl-5-trifluormethyl-3-pyridazinon-2-yl)phenyl}chlor-methansulfonamid (Verbindung 1-369),
N-{2-Chlor-5-(4-methyl-5-trifluormethyl-3-pyridazinon-2-yl)phenyl}methansulfonamid (Verbindung 1-398),
Methyl-2-chlor-4-fluor-5-(4-methyl-5-trifluormethyl-3-pyridazinon-2-yl)benzoat (Verbindung 1-641);
Ethyl-2-chlor-4-fluor-5-(4-methyl-5-trifluormethyl-3-pyridazinon-2-yl)benzoat (Verbindung 1-642),
Ethyl-2-chlor-5-(4-methyl-5-trifluormethyl-3-pyridazinon-2-yl)benzoat (Verbindung 1-621),
Isopropyl-2-chlor-5-(4-methyl-5-trifluormethyl-3-pyridazinon-2-yl)benzoat (Verbindung 1-625), oder
2-(4-Chlor-2-fluor-5-propargyloxyphenyl)-6-methyl-5-trifluormethylpyridazin-3-on (Verbindung 1-1441).

11. Herbizidzusammensetzung, umfassend eine herbizid wirksame Menge der Verbindung gemäß Anspruch 1 und einen inerten Träger oder ein Verdünnungsmittel.

12. Verfahren zur Bekämpfung von Unkraut, welches das Aufbringen einer herbizid wirksamen Menge der Verbindung gemäß Anspruch 1 auf eine Fläche, wo Unkraut wächst oder wachsen wird, umfasst.

13. Verwendung der Verbindung gemäß Anspruch 1 als ein Herbizid.

14. Verbindung der Formel: 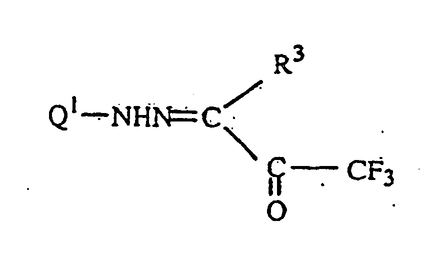 wobei R³ wie in Anspruch 1 definiert ist und Q¹ [Q¹-1], [Q-2], [Q¹-3] oder [Q-4] der Formel 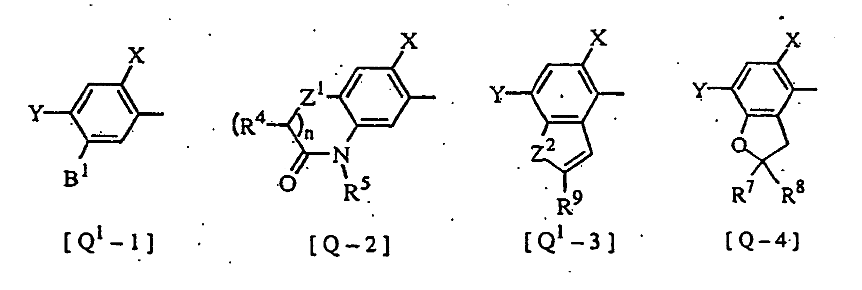 ist, wobei X, Y, Z¹, Z², n, R⁴, R⁵, R⁷ und R⁸ wie in Anspruch 1 definiert sind; B¹ Wasserstoff, Halogen, Nitro, Cyano, OR²⁷, SR²⁷, SO₂OR²⁷, NR¹¹(R¹²), SO₂NR¹¹(R¹²), NR¹¹(COR¹³), NR¹¹(SO₂R¹⁴), N(SO₂R¹⁴)(SO₂R¹⁵), N(SO_{2R}¹⁴)(COR¹³), NHCOOR¹³, COOR²⁷, CONR¹¹(R¹²), CSNR¹¹(R¹²), CR¹⁷=CR¹⁸COOR¹³, CR¹⁷=CR¹⁸CONR¹¹(R¹²), CH₂CHWCOOR¹³, CH₂CHWCONR¹¹(R¹²), CR¹⁷=NOR³³, CR¹⁷=NNR¹¹(R¹²), CR¹⁷(Z²R³⁴)₂, OCO₂R¹⁹ oder OCOR¹⁹ ist; und
R⁹ C₁-C₆-Alkyl, C₁-C₆-Halogenalkyl, Cyano, Carboxyl, Hydroxy-C₁-C₆-alkyl, C₁-C₆-Alkoxy-C₁-C₆-alkyl, C₁-C₆-Alkoxy-C₁-C₆-alkoxy-C₁-C₆-alkyl, (C₁-C₆-Alkyl)-carbonyloxy-C₁-C₆-alkyl, (C₁-C₆-Halogenalkyl)carbonyloxy-C₁-C₆-alkyl, (C₁-C₆-Alkoxy)carbonyl oder (C₁-C₆-Alkyl)carbonyl ist;
wobei R²⁷ Wasserstoff, C₁-C₆-Alkyl, C₁-C₆-Halogenalkyl, C₃-C₈-Cycloalkyl, Benzyl, C₃-C₆-Alkenyl, C₃-C₆-Halogenalkenyl, C₃-C₆-Alkinyl, C₃-C₆-Halogenalkinyl, Cyano-C₁-C₆-alkyl, C₂-C₈-Alkoxyalkyl, C₂-C₈-Alkylthioalkyl, Carboxy-C₁-C₆-alkyl, (C₁-C₈-Alkoxy)carbonyl-C₁-C₆-alkyl, (C₁-C₆-Halogenalkoxy)carbonyl-C₁-C₆-alkyl, {(C₁-C₄-Alkoxy)-C₁-C₄-alkoxy}carbonyl-C₁-C₆-alkyl, (C₃-C₈-Cycloalkoxy)carbonyl-C₁-C₆-alkyl, CH₂CON(R¹¹)R¹², CH₂COON(R¹¹)R¹², CH(C₁-C₄-Alkyl)CON(R¹¹)R¹², CH-(C₁-C₄-Alkyl)-COON(R¹¹)R¹², {(C₁-C₆-Alkoxy)carbonyl-C₁-C₆-alkyl}oxycarbonyl-C₁-C₆-alkyl oder Hydroxy-C₁-C₆-alkyl ist; und R¹¹, R¹², R¹³, R¹⁴, R¹⁵, R¹⁷, R¹⁸, R¹⁹, R³³, R³⁴, W und Z² wie in Anspruch 1 definiert sind.

## Revendications

1. Composé de formule : 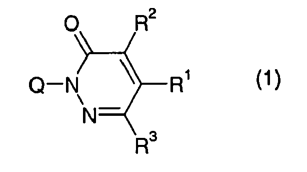 dans laquelle
- R¹ représente un groupe halogénoalkyle en C₁₋₃ ;
- R² et R³ représentent des entités identiques ou différentes et représentent chacun un atome d'hydrogène ou un groupe alkyle en C₁₋₃, halogénoalkyle en C₁₋₃ ou (alcoxy en C₁₋₃)-alkyle en C₁₋₃ ;
- et Q représente un groupe de formule [Q-1], [Q-2], [Q-3] ou [Q-4] : 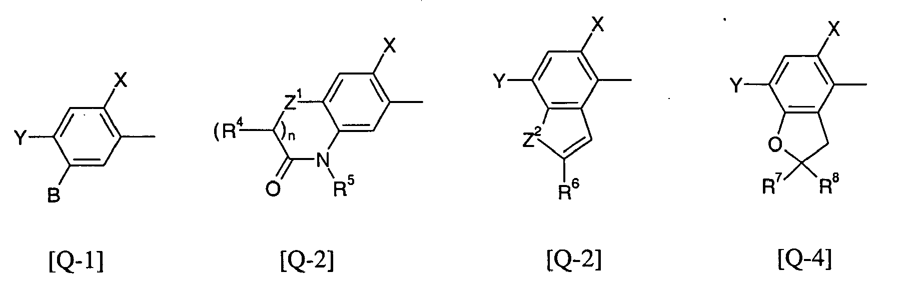
dans lesquelles formules
- X représente un atome d'hydrogène ou d'halogène ;
- Y représente un atome d'halogène ou un groupe nitro, cyano ou trifluorométhyle ;
- Z¹ représente un atome d'oxygène ou de soufre ou un chaînon de formule NH ;
- Z² représente un atome d'oxygène ou de soufre ;
- l'indice n vaut 0 ou 1 ;
- B représente un atome d'hydrogène ou d'halogène, un groupe nitro, cyano ou chlorosulfonyle, ou un groupe symbolisé par OR¹⁰, SR¹⁰, SO₂OR¹⁰, N(R¹¹)R¹², SO₂N(R¹¹)R¹², NR¹¹(COR¹³), NR¹¹(SO₂R¹⁴), N(SO₂R¹⁴)(SO₂R¹⁵), N(SO₂R¹⁴)(COR¹³), NHCOOR¹³, COOR¹⁰, CON(R¹¹)R¹², CSN(R¹¹)R¹², COR¹⁶, CR¹⁷=CR¹⁸COR¹⁶, CR¹⁷=CR¹⁸COOR¹³, CR¹⁷=CR¹⁸CON(R¹¹)R¹², CH₂CHWCOOR¹³, CH₂CHWCON(R¹¹)R¹², CR¹⁷=NOR³³, CR¹⁷=NN(R¹¹)R¹², CR¹⁷(Z²R³⁴)₂, OCO₂R¹⁹ ou OCOR¹⁹ ;
- R⁴ représente un atome d'hydrogène ou un groupe alkyle en C₁₋₃ ;
- R⁵ représente un atome d'hydrogène, un groupe alkyle en C₁₋₆, halogénoalkyle en C₁₋₆, (cycloalkyle en C₃₋₈)-alkyle, alcényle en C₃₋₆, halogénoalcényle en C₃₋₆, alcynyle en C₃₋₆, halogénoalcynyle en C₃₋₆, cyano-(alkyle en C₁₋₆), alcoxyalkyle en C₂₋₈, alcoxyalcoxyalkyle en C₃₋₈, carboxy-(alkyle en C₁₋₆), (alcoxy en C₁₋₆)-carbonyl-(alkyle en C₁₋₆), {(alcoxy en C₁₋₄)-alcoxy en C₁₋₄}-carbonyl-(alkyle en C₁₋₆), (cycloalcoxy en C₃₋₈)-carbonyl-(alkyle en C₁₋₆), ou encore un groupe symbolisé par CH₂CON(R¹¹)R¹², CH₂COON(R¹¹)R¹², CH(alkyle en C₁₋₄)CON(R¹¹)R¹² ou CH(alkyle en C₁₋₄)COON(R¹¹)R¹² ; alkylthioalkyle en C₂₋₈ ou hydroxyalkyle en C₁₋₆ ;
- R⁶ représente un groupe alkyle en C₁₋₆, halogénoalkyle en C₁₋₆, formyle, cyano, carboxyle, hydroxyalkyle en C₁₋₆, (alcoxy en C₁₋₆)-(alkyle en C₁₋₆), (alcoxy en C₁₋₆)-(alcoxy en C₁₋₆)-(alkyle en C₁₋₆), (alkyle en C₁₋₆)-carbonyloxy-(alkyle en C₁₋₆), (halogénoalkyle en C₁₋₆)-carbonyloxy-(alkyle en C₁₋₆), (alcoxy en C₁₋₆)-carbonyle ou (alkyle en C₁₋₆)-carbonyle ;
- R⁷ représente un atome d'hydrogène ou un groupe alkyle en C₁₋₆ ;
- et R⁸ représente un atome d'hydrogène, un groupe alkyle en C₁₋₆, halogénoalkyle en C₁₋₆, hydroxyalkyle en C₁₋₆, alcoxyalkyle en C₂₋₈, alcoxyalcoxyalkyle en C₃₋₁₀, (alkyle en C₁₋₅)-carbonyloxy-(alkyle en C₁₋₆), (halogénoalkyle en C₁₋₆)-carbonyloxy-(alkyle en C₁₋₆), carboxyle, carboxy-(alkyle en C₁₋₆), (alcoxy en C₁₋₈)-carbonyle, (halogénoalcoxy en C₁₋₆)-carbonyle, (cycloalcoxy en C₃₋₁₀)-carbonyle, (alcényloxy en C₃₋₈)-carbonyle, (alcynyloxy en C₃₋₈)-carbonyle, aminocarbonyle, (alkyle en C₁₋₆)-aminocarbonyle, di(alkyle en C₁₋₆)-aminocarbonyle, (alkyle en C₁₋₆)-aminocarbonyloxy-(alkyle en C₁₋₆) ou di(alkyle en C₁₋₆)-aminocarbonyloxy-(alkyle en C₁₋₆) ;
étant entendu que :
- R¹⁰ représente un atome d'hydrogène, un groupe alkyle en C₁₋₆, halogénoalkyle en C₁₋₆, cycloalkyle en C₃₋₈, benzyle, alcényle en C₃₋₆, halogénoalcényle en C₃₋₆, alcynyle en C₃₋₆, halogénoalcynyle en C₃₋₆, cyano-(alkyle en C₁₋₆), alcoxyalkyle en C₂₋₈, alkylthioalkyle en C₂₋₈, carboxy-(alkyle en C₁₋₆), (alcoxy en C₁₋₈)-carbonyl-(alkyle en C₁₋₆), (halogénoalcoxy en C₁₋₆)-carbonyl-(alkyle en C₁₋₆), {(alcoxy en C₁₋₄)-alcoxy en C₁₋₄)}-carbonyl-(alkyle en C₁₋₆), (cycloalcoxy en C₃₋₈)-carbonyl-(alkyle en C₁₋₆), (alkyle en C₁₋₆)-carbonyl-(alkyle en C₁₋₆), (halogénoalkyle en C₁₋₆) -carbonyl- (alkyle en C₁₋₆), {(alcoxy en C₁₋₄)-alkyle en C₁₋₄}-carbonyl-(alkyle en C₁₋₆), (cycloalkyle en C₃₋₈)-carbonyl-(alkyle en C₁₋₆), CH₂CON(R¹¹)R¹², CH₂COON(R¹¹)R¹², CH(alkyle en C₁₋₄)CON(R¹¹)R¹², CH(alkyle en C₁₋₄)COON(R¹¹)R¹² ; {(alcoxy en C₁₋₆)-carbonyl-alkyle en C₁₋₆}-oxycarbonyl-(alkyle en C₁₋₆), ou hydroxyalkyle en C₁₋₆ ;
- R¹¹ et R¹² représentent indépendamment un atome d'hydrogène, un groupe alkyle en C₁₋₆, halogénoalkyle en C₁₋₆, alcényle en C₃₋₆, alcynyle en C₃₋₆, cyano-(alkyle en C₁₋₆), alcoxyalkyle en C₂₋₈, alkylthioalkyle en C₂₋₈, carboxy-(alkyle en C₁₋₆), (alcoxy en C₁₋₆)-carbonyl-(alkyle en C₁₋₆), (cycloalcoxy en C₃₋₈)-carbonyl-(alkyle en C₁₋₆), {(alcoxy en C₁₋₄)-alcoxy en C₁₋₄}-carbonyl-(alkyle en C₁₋₆),
ou encore R¹¹ et R¹² représentent ensemble un groupe tétraméthylène, pentaméthylène ou éthylène-oxy-éthylène ;
- R¹³ représente un atome d'hydrogène, un groupe alkyle en C₁₋₆, halogénoalkyle en C₁₋₆, cycloalkyle en C₃₋₈ ou alcényle en C₃₋₆ ;
- R¹⁴ et R¹⁵ représentent indépendamment un groupe alkyle en C₁₋₆, halogénoalkyle en C₁₋₆, ou un groupe phényle, éventuellement substitué par méthyle ou nitro ;
- R¹⁶ représente un atome d'hydrogène, un groupe alkyle en C₁₋₆, halogénoalkyle en C₁₋₆, alcényle en C₂₋₆, halogénoalcényle en C₂₋₆, alcynyle en C₂₋₆, halogénoalcynyle en C₂₋₆, alcoxyalkyle en C₂₋₈ ou hydroxyalkyle en C₁₋₆;
- R¹⁷ et R¹⁸ représentent indépendamment un atome d'hydrogène ou un groupe alkyle en C₁₋₆ ;
- R¹⁹ représente un groupe alkyle en C₁₋₆ ;
- R³³ représente un atome d'hydrogène, un groupe alkyle en C₁₋₆, halogénoalkyle en C₁₋₆, cycloalkyle en C₃₋₈, alcényle en C₃₋₆, halogénoalcényle en C₃₋₆, alcynyle en C₃₋₆, halogénoalcynyle en C₃₋₆, cyano-(alkyle en C₁₋₆) ou (alcoxy en C₁₋₆)-carbonyl-(alkyle en C₁₋₆) ;
- R³⁴ représente un groupe alkyle en C₁₋₆, ou les deux symboles R³⁴ représentent ensemble un groupe de formule (CH₂)₂ ou (CH₂)₃ ;
- et W représente un atome d'hydrogène, de chlore ou de brome.

2. Composé conforme à la revendication 1, dans lequel R¹ représente un groupe trifluorométhyle.

3. Composé conforme à la revendication 1, dans lequel R² représente un atome d'hydrogène ou un groupe alkyle en C₁₋₃ et R³ représente un atome d'hydrogène ou un groupe alkyle en C₁₋₃.

4. Composé conforme à la revendication 1, dans lequel R¹ représente un groupe trifluorométhyle, R² représente un atome d'hydrogène ou un groupe alkyle en C₁₋₃ et R³ représente un atome d'hydrogène ou un groupe alkyle en C₁₋₃.

5. Composé conforme à la revendication 1, 2, 3 ou 4, dans lequel Q représente un groupe de formule [Q-1].

6. Composé conforme à la revendication 5, dans lequel B représente un groupe symbolisé par OR¹⁰, SR¹⁰, N(R¹¹)R¹², NR¹¹(SO₂R¹⁴) ou COOR¹⁰.

7. Composé conforme à la revendication 5, dans lequel X représente un atome de fluor et Y représente un atome de chlore.

8. Composé conforme à la revendication 5, dans lequel X représente un atome de fluor, Y représente un atome de chlore et B représente un groupe symbolisé par OR¹⁰.

9. Composé conforme à la revendication 1, 2, 3 ou 4, dans lequel Z¹ représente un atome d'oxygène, l'indice n vaut 1, et Q représente un groupe de formule [Q-2].

10. Composé conforme à la revendication 1, qui est l'un des suivants :
7-fluoro-6-(5-trifluorométhyl-pyridazin-3-one-2-yl)-4-propargyl-2H-1,4-benzoxazine-3-one (composé 2-631)
7-fluoro-6-(4-méthyl-5-trifluorométhyl-pyridazin-3-one-2-yl)-4-propargyl-2H-1,4-benzoxazine-3-one (composé 2-251)
6-(5-trifluorométhyl-pyridazin-3-one-2-yl)-4-propargyl-2H-1,4-benzoxazine-3-one (composé 2-583)
6-(4-méthyl-5-trifluorométhyl-pyridazin-3-one-2-yl)-4-propargyl-2H-1,4-benzoxazine-3-one (composé 2-203)
2-(4-chloro-2-fluoro-5-isopropoxy-phényl)-4-méthyl-5-trifluorométhylpyridazin-3-one (composé 1-476)
2-(4-chloro-2-fluoro-5-méthoxy-phényl)-4-méthyl-5-trifluorométhylpyridazin-3-one (composé 1-474)
2-(4-chloro-2-fluoro-5-éthoxy-phényl)-4-méthyl-5-trifluorométhylpyridazin-3-one (composé 1-475)
2-(4-chloro-2-fluoro-5-propargyloxy-phényl)-4-méthyl-5-trifluorométhylpyridazin-3-one (composé 1-486)
2-chloro-4-fluoro-5-(4-méthyl-5-trifluorométhyl-pyridazin-3-one-2-yl)-phénoxyacétate de méthyle (composé 1-495)
2-chloro-4-fluoro-5-(4-méthyl-5-trifluorométhyl-pyridazin-3-one-2-yl)-phénoxyacétate d'éthyle (composé 1-496)
2-chloro-4-fluoro-5-(4-méthyl-5-trifluorométhyl-pyridazin-3-one-2-yl)-phénoxyacétate de propyle (composé 1-497)
2-chloro-4-fluoro-5-(4-méthyl-5-trifluorométhyl-pyridazin-3-one-2-yl)-phénoxyacétate d'isopropyle (composé 1-500)
2-chloro-4-fluoro-5-(4-méthyl-5-trifluorométhyl-pyridazin-3-one-2-yl)-phénoxyacétate de butyle (composé 1-498)
2-chloro-4-fluoro-5-(4-méthyl-5-trifluorométhyl-pyridazin-3-one-2-yl)-phénoxyacétate de pentyle (composé 1-499)
2-chloro-4-fluoro-5-(4-méthyl-5-trifluorométhyl-pyridazin-3-one-2-yl)-phénoxyacétate de cyclopentyle (composé 1-501)
2-{2-chloro-4-fluoro-5-(4-méthyl-5-trifluorométhyl-pyridazin-3-one-2-yl)-phénoxy}propionate d'éthyle (composé 1-504)
2-{2-chloro-4-fluoro-5-(4-méthyl-5-trifluorométhyl-pyridazin-3-one-2-yl)-phénoxy}propionate de méthyle (composé 1-503)
2-chloro-4-fluoro-5-(4-méthyl-5-trifluorométhyl-pyridazin-3-one-2-yl)-phénylthioacétate d'éthyle (composé 1-577)
2-chloro-4-fluoro-5-(4-méthyl-5-trifluorométhyl-pyridazin-3-one-2-yl)-phénylthioacétate de méthyle (composé 1-576)
2-{2-chloro-4-fluoro-5-(4-méthyl-5-trifluorométhyl-pyridazin-3-one-2-yl)-phénylthio}propionate d'éthyle (composé 1-585)
2-{2-chloro-4-fluoro-5-(4-méthyl-5-trifluorométhyl-pyridazin-3-one-2-yl)-phénylthio}propionate de méthyle (composé 1-584)
2-{2-chloro-4-fluoro-5-(5-trifluorométhyl-pyridazin-3-one-2-yl)phénoxy}-propionate de méthyle (composé 1-1140)
2-{2-chloro-4-fluoro-5-(5-trifluorométhyl-pyridazin-3-one-2-yl)phénoxy}-propionate d'éthyle (composé 1-1141)
2-chloro-4-fluoro-5-(5-trifluorométhyl-pyridazin-3-one-2-yl)phénylthioacétate d'éthyle (composé 1-1214)
2-chloro-4-fluoro-5-(5-trifluorométhyl-pyridazin-3-one-2-yl)phénylthioacétate de méthyle (composé 1-1213)
2-{2-chloro-4-fluoro-5-(5-trifluorométhyl-pyridazin-3-one-2-yl)phénylthio}propionate d'éthyle (composé 1-1222)
2-{2-chloro-4-fluoro-5-(5-trifluorométhyl-pyridazin-3-one-2-yl)phénylthio}propionate de méthyle (composé 1-1221)
2-{2-chloro-4-fluoro-5-(5-trifluorométhyl-pyridazin-3-one-2-yl)phénylthio}propionate d'isopropyle (composé 1-1226)
2-{2-chloro-4-fluoro-5-(4-méthyl-5-trifluorométhyl-pyridazin-3-one-2-yl)-phénylamino}propionate d'éthyle (composé 1-420)
2-{2-chloro-4-fluoro-5-(5-trifluorométhyl-pyridazin-3-one-2-yl)phénylamino}propionate d'éthyle (composé 1-1057)
N-{2-chloro-4-fluoro-5-(4-méthyl-5-trifluorométhyl-pyridazin-3-one-2-yl)-phényl}-méthanesulfonamide (composé 1-367)
N-{2-chloro-4-fluoro-5-(4-méthyl-5-trifluorométhyl-pyridazin-3-one-2-yl)-phényl}-chlorométhanesulfonamide (composé 1-369)
N-{2-chloro-5-(4-méthyl-5-trifluorométhyl-pyridazin-3-one-2-yl)phényl}-méthanesulfonamide (composé 1-398)
2-chloro-4-fluoro-5-(4-méthyl-5-trifluorométhyl-pyridazin-3-one-2-yl)-benzoate de méthyle (composé 1-641)
2-chloro-4-fluoro-5-(4-méthyl-5-trifluorométhyl-pyridazin-3-one-2-yl)-benzoate d'éthyle (composé 1-642)
2-chloro-5-(4-méthyl-5-trifluorométhyl-pyridazin-3-one-2-yl)benzoate d'éthyle (composé 1-621)
2-chloro-5-(4-méthyl-5-trifluorométhyl-pyridazin-3-one-2-yl)benzoate d'isopropyle (composé 1-625), ou
2-(4-chloro-2-fluoro-5-propargyloxy-phényl)-6-méthyl-5-trifluorométhyl-pyridazin-3-one (composé 1-1441).

11. Composition herbicide comprenant un composé conforme à la revendication 1, en une quantité à effet herbicide, et un véhicule ou diluant inerte.

12. Procédé de lutte contre des mauvaises herbes, qui comporte le fait d'appliquer un composé conforme à la revendication 1, en une quantité à effet herbicide, sur une zone où de mauvaises herbes poussent ou pourraient pousser.

13. Emploi d'un composé conforme à la revendication 1 en qualité d'herbicide.

14. Composé de formule : 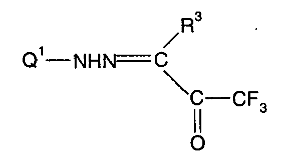 dans laquelle R³ a la signification indiquée dans la revendication 1, et Q¹ représente un groupe de formule [Q¹-1], [Q-2], [Q¹-3] ou [Q-4] : 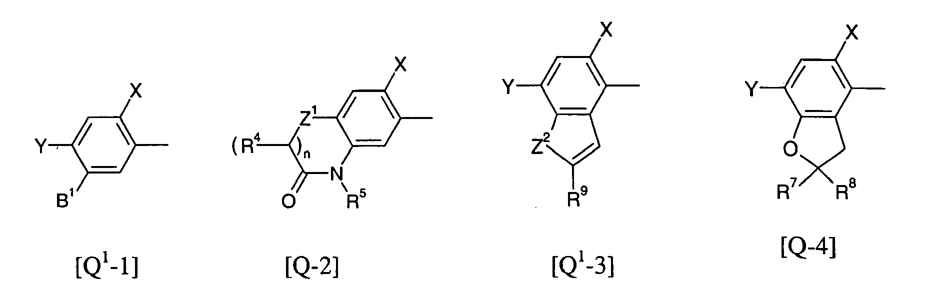 dans lesquelles formules
- X, Y, Z¹, Z², n, R⁴, R⁵, R⁷ et R⁸ ont les significations indiquées dans la revendication 1 ;
- B¹ représente un atome d'hydrogène ou d'halogène, un groupe nitro ou cyano, ou un groupe symbolisé par OR²⁷, SR²⁷, SO₂OR²⁷, N(R¹¹)R¹², SO₂N(R¹¹)R¹², NR¹¹(COR¹³), NR¹¹(SO₂R¹⁴), N(SO₂R¹⁴)(SO₂R¹⁵), N(SO₂R¹⁴)(COR¹³), NHCOOR¹³, COOR²⁷, CON(R¹¹)R¹², CSN(R¹¹)R¹², CR¹⁷=CR¹⁸COOR¹³, CR¹⁷=CR¹⁸CON(R¹¹)R¹², CH₂CHWCOOR¹³, CH₂CHWCON(R¹¹)R¹², CR¹⁷=NOR³³, CR¹⁷=NN(R¹¹)R¹², CR¹⁷(Z²R³⁴)₂, OCO₂R¹⁹ ou OCOR¹⁹;
- et R⁹ représente un groupe alkyle en C₁₋₆, halogénoalkyle en C₁₋₆, cyano, carboxyle, hydroxyalkyle en C₁₋₆, (alcoxy en C₁₋₆)-(alkyle en C₁₋₆), (alcoxy en C₁₋₆)-(alcoxy en C₁₋₆)-(alkyle en C₁₋₆), (alkyle en C₁₋₆)-carbonyloxy-(alkyle en C₁₋₆), (halogénoalkyle en C₁₋₆)-carbonyloxy-(alkyle en C₁₋₆), (alcoxy en C₁₋₆)-carbonyle ou (alkyle en C₁₋₆)-carbonyle ;
étant entendu que
- R²⁷ représente un atome d'hydrogène ou un groupe alkyle en C₁₋₆, halogénoalkyle en C₁₋₆, cycloalkyle en C₃₋₈, benzyle, alcényle en C₃₋₆, halogénoalcényle en C₃₋₆, alcynyle en C₃₋₆, halogénoalcynyle en C₃₋₆, cyano-(alkyle en C₁₋₆), alcoxyalkyle en C₂₋₈, alkylthioalkyle en C₂₋₈, carboxy-(alkyle en C₁₋₆), (alcoxy en C₁₋₈)-carbonyl-(alkyle en C₁₋₆), (halogénoalcoxy en C₁₋₆)-carbonyl-(alkyle en C₁₋₆), {(alcoxy en C₁₋₄)-alcoxy en C₁₋₄}-carbonyl-(alkyle en C₁₋₆), (cycloalcoxy en C₃₋₈)-carbonyl-(alkyle en C₁₋₆), CH₂CON(R¹¹)R¹², CH₂COON(R¹¹)R¹², CH(alkyle en C₁₋₄)CON(R¹¹)R¹², CH(alkyle en C₁₋₄)COON(R¹¹)R¹², {(alcoxy en C₁₋₆)-carbonyl-alkyle en C₁₋₆}-oxycarbonyl-(alkyle en C₁₋₆), ou hydroxyalkyle en C₁₋₆ ;
- et R¹¹, R¹², R¹³ R¹⁴, R¹⁵, R¹⁷, R¹⁸, R¹⁹ R³³, R³⁴, W et Z² ont les significa-tions indiquées dans la revendication 1.
